(19) Europäisches Patentamt
European Patent Office
Office européen des brevets

(11) EP 1 209 147 A1

(12) **EUROPEAN PATENT APPLICATION**
published in accordance with Art. 158(3) EPC

(43) Date of publication:
**29.05.2002 Bulletin 2002/22**

(21) Application number: **00949891.6**

(22) Date of filing: **26.07.2000**

(51) Int Cl.[7]: **C07C 233/51**, C07C 233/87, C07C 235/12, C07C 235/66, C07C 237/22, C07C 275/42, C07C 311/21, C07C 323/59, C07C 329/06, C07D 207/08, C07D 239/47

(86) International application number:
**PCT/JP00/04965**

(87) International publication number:
**WO 01/07400 (01.02.2001 Gazette 2001/05)**

(84) Designated Contracting States:
**AT BE CH CY DE DK ES FI FR GB GR IE IT LI LU MC NL PT SE**
Designated Extension States:
**AL LT LV MK RO SI**

(30) Priority: **26.07.1999 JP 21003799**
**29.11.1999 JP 33874799**

(71) Applicant: **TORAY INDUSTRIES, INC.**
**Tokyo 103-8666 (JP)**

(72) Inventors:
- **TAKAHASHI, Toshiya**
  **Fujisawa-shi, Kanagawa 251-0042 (JP)**
- **TAKANO, Yoshio**
  **Kamakura-shi, Kanagawa 248-0031 (JP)**
- **ISHIGAKI, Takeshi**
  **Kamakura-shi, Kanagawa 248-0031 (JP)**
- **FUNAHASHI, Miyuki**
  **Fujisawa-shi, Kanagawa 251-0037 (JP)**
- **KANEKO, Masayuki**
  **Kamakura-shi, Kanagawa 248-0034 (JP)**
- **KAINOH, Mie**
  **Fujisawa-shi, Kanagawa 251-0052 (JP)**

(74) Representative: **Kador & Partner**
**Corneliusstrasse 15**
**80469 München (DE)**

(54) **CARBOXYLIC ACID DERIVATIVES AND ADHESION MOLECULE INHIBITORS CONTAINING THE SAME AS THE ACTIVE INGREDIENT**

(57) Prevention or therapy of inflammatory diseases caused by invasion of leukocytes such as monocytes, lymphocytes and eosinophils, by providing a substance which inhibits cell adhesion via an adhesion molecule, especially adhesion molecule VLA-4, is disclosed. A group of carboxylic acid derivatives represented by, for example,

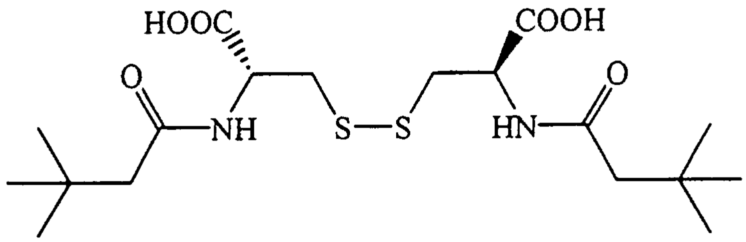

(140)

and adhesion molecule inhibitors comprising the same as an effective ingredient were provided.

EP 1 209 147 A1

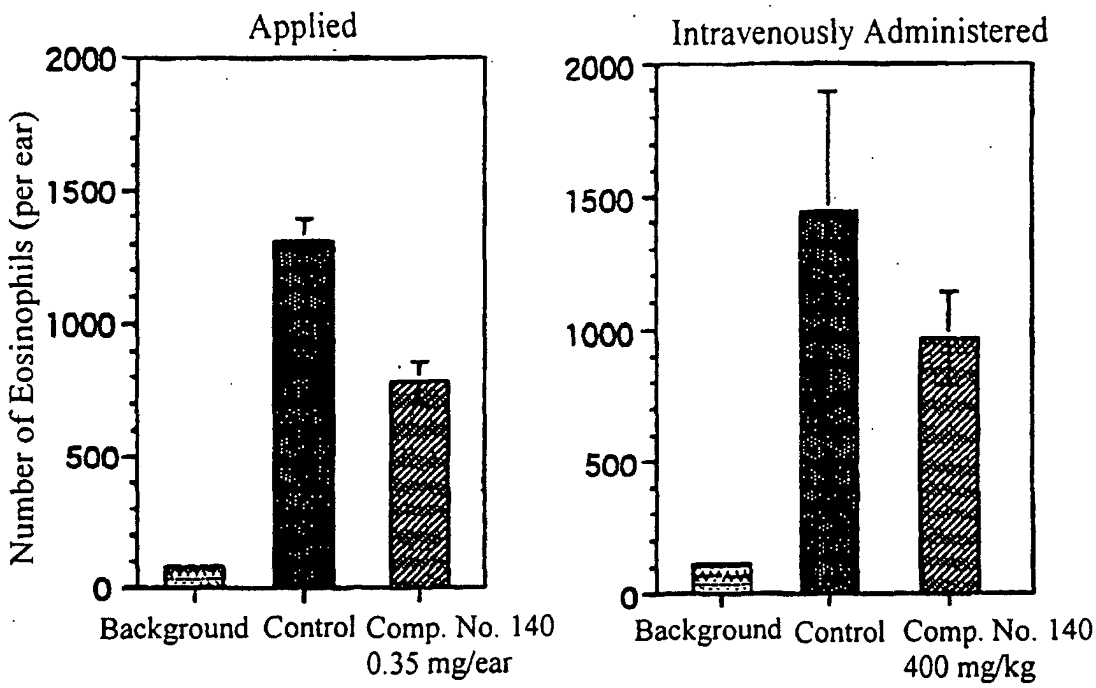
Applied
Intravenously Administered
Number of Eosinophils (per ear)
2000
1500
1000
500
0
Background
Control
Comp. No. 140
0.35 mg/ear
400 mg/kg

Fig. 1

## Description

### TECHNICAL FIELD

The present invention relates to novel carboxylic acid derivatives and pharmaceutically acceptable salts thereof, as well as to adhesion molecule Inhibitors, which contain the same as effective ingredients, especially to VLA-4 inhibitors.

### BACKGROUND ART

Adhesion molecules participate in adhesion between cells or between cells and intercellular substances, and participate in movement or activation of cells. Adhesion molecules are classified into a number of families such as integrin family and immunoglobulin super family. The adhesive molecules belonging to the integrin family are those expressed on leukocytes such as lymphocytes, monocytes, basophils and eosinophils, which have heterodimer structures in which an $\alpha$ chain and a $\beta$ chain are bound through non-covalent bond. They are classified into several subfamilies depending on the molecular species of the $\beta$ chain. VLA-4 (very late antigen-4), which is a member of the integrin family, is also called $\alpha4\beta1$ or CD49d/CD29 and participates in the interaction between leukocytes and vascular endothelial cells or between leukocytes and extracellular matrix, and in infiltration of leukocytes into inflammatory regions. As adhesion molecules which interact with VLA-4, fibronectin in the extracellular matrix and VCAM-1 (vascular cell adhesion molecule-1) existing on vascular endothelial cells are known.
The binding site on fibronectin, which binds to VLA-4, is located in the fibronectin fragment called CS-1. It has been reported that the minimum unit of amino acids, which is necessary for the binding, is the unit composed of three amino acid residues, that is, leucine-aspartic acid-valine.
Straight or circular inhibitor peptides based on the three amino acid residues leucine-aspartic acid-valine, which inhibit adhesion by VLA-4 have been reported (WO95/15973)
On the other hand, it is known that VCAM-1, the other adhesion molecule that interacts with VLA-4, is expressed on vascular endothelial cells, of which expression is increased by stimulation by cytokines such as IL-1, TNF-$\alpha$ and IL-4, and that it interacts with VLA-4 located on the cells such as lymphocytes, NK cells, monocytes and eosinophils. VLA-4 and VCAM-1 participate in the infiltration of leukocytes from blood vessels into inflammatory regions. In view of this, the interaction between VLA-4 and VCAM-1 in inflammation reaction is very important.
VCAM-1 belongs to, among the adhesion molecules, immunoglobulin super family and is known to include 7-Ig-like-domain VCAM-1 and 6-Ig-like-domain VCAM-1. From mutations of VCAM-1, it has been clarified that the binding sites on VCAM-1 at which VCAM-1 binds to VLA-4 are located on domain 1 and domain 4, and that in these domains, the amino acid sequence glutamine-isoleucine-aspartic acid-serine-proline in the CD loop is important for the binding to VLA-4 (for example, J.Cell Biol., 124, 601(1994). J.H.WANG et al. reported a circular peptide Cys*Gln-IleAspSerProCys*(wherein Cys*Cys* represents disulfide bond) based on a basic peptide glutamine-isoleucine-aspartic acid-serine-proline (Proc.Natl.Acad.Sci.USA, 92 , 5714 (1995).) A number of compounds which show VLA-4-inhibiting activity have also been reported (for example, US5,770,573, US5,821,231, WO99/6436). It has been reported that specific cystine derivatives have immunomodulating activities (WO91/18594, EP463514), mucolytic activities (Thorax, 40, 832(1985)), and effects for prophylaxis and therapy of restenosis (WO96/28149) However, it has not been found that cystine derivatives have inhibitory activities against adhesive molecules, especially VLA-4-inhibiting activity.
The fact that VLA-4 plays an important role in inflammatory reactions has been revealed by tests using anti-VLA-4 antibody in animal models such as models of contact hypersensitivity and delayed type hypersensitivity (mouse and rat), experimental autoimmune encephalomyelitis models (mouse and rat), nephrotic nephritis model (rat), passive cutaneous anaphylaxis model (guinea pig), immune complex-induced lung damage models (rat), spontaneous colitis model (monkey), asthma models (sheep) and adjuvant arthritis models.

### DISCLOSURE OF THE INVENTION

An object of the present invention is to discover a substance which inhibits cell adhesion via adhesion molecules, especially via adhesion molecule VLA-4, thereby providing an adhesion molecule inhibitor, especially a VLA-4 inhibitor, which enables prophylaxis and therapy of inflammatory diseases due to infiltration of monocytes, lymphocytes and eosinophils.
The present inventors intensively studied to discover that specific novel carboxylic acid derivatives and pharmaceutically acceptable salts thereof have activities to inhibit cell adhesion via adhesion molecules, especially the cell fusion via the adhesion molecule VLA-4, thereby completing the present invention.
That is, the present invention provides carboxylic acid derivatives of the Formula I:

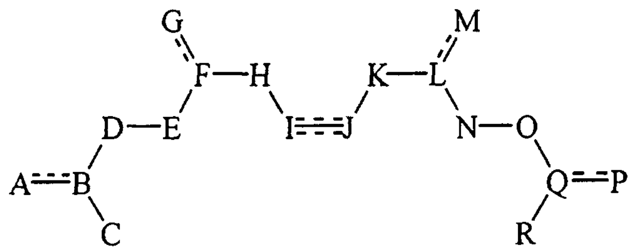

I

[In Formula I, A, C, P and R may or may not exist, and may be the same or different, in cases where A, C, P or R exist, they respectively represent (i) hydrogen, (ii) $C_1$-$C_6$ straight alkyl, (iii) $C_3$-$C_8$ branched alkyl, (iv) Formula II:

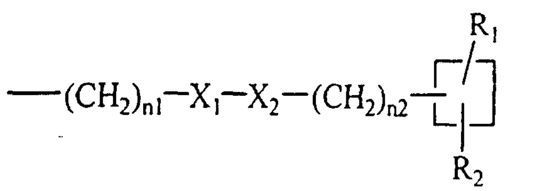

II

(wherein n1 and n2 represent numbers of 0 to 3, $X_1$ and $X_2$ may or may not exist, in cases where $X_1$ or $X_2$ exists, $X_1$ represents an oxygen atom, sulfur atom or nitrogen atom, $X_2$ represents carbonyl group or sulfonyl group; □represents cyclohexane ring, benzene ring, naphthalene ring, indole ring, imidazole ring, furan ring, thiophen ring, pyrrole ring or pyridine ring; $R_1$ and $R_2$ may or may not exist, and may be the same or different, in cases where $R_1$ or $R_2$ exist, they respectively represent $C_1$-$C_6$ straight alkyl, $C_3$-$C_8$ branched alkyl, hydroxyl, methoxy, chloro, bromo, fluoro, nitro, amino, Formula III:

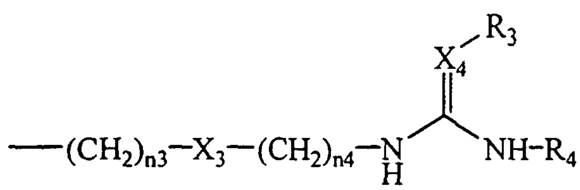

III

(wherein n3 and n4 represent numbers of 0 to 3; $X_3$ may or may not exist, in cases where $X_3$ exists, $X_3$ represents a nitrogen atom or oxygen atom; $X_4$ represents a carbon atom, oxygen atom, nitrogen atom or sulfur atom; in cases where $X_4$ is a carbon atom or nitrogen atom, $R_3$ exists and represents nitro, cyano, methylsulfonyl or phenylsulfonyl group; $R_4$ represents hydrogen, $C_1$-$C_6$ straight alkyl, $C_3$-$C_8$ branched alkyl, $C_6$-$C_{10}$ non-substituted aryl or $C_6$-$C_{10}$ aryl substituted with 0 to 2 hydroxyl groups, methoxy groups, nitro groups, amino groups, chloro, bromo and/or fluoro) or Formula IV:

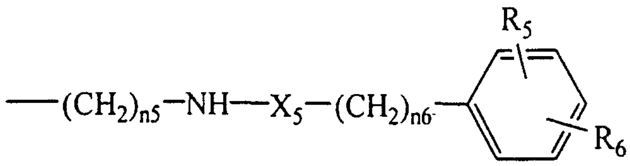

IV

(wherein n5 and n6 represent numbers of 0 to 3; $X_5$ represents carbonyl group or sulfonyl group; $R_5$ and $R_6$ represent hydrogen, methyl, methoxy, chloro, bromo, fluoro, nitro or amino groups)

B and Q may or may not exist, and may be the same or different, in cases where B or Q exist, they represent (i) nitrogen atom, (ii) carbon atom, (iii) $C_6$-$C_{10}$ aryl or cyclohexyl each of which is bound through 0 to 3 methylene groups, each of which is substituted with 0 to 3 methyl groups, ethyl groups, hydroxyl groups, methoxy groups, chloro, bromo, fluoro, nitro groups, tetrazole groups or amino groups, (iv) Formula V:

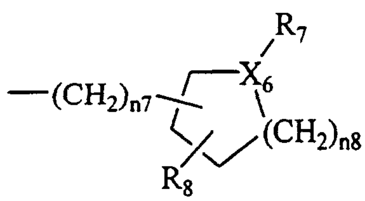

V

(wherein n7 represents number of 0 to 3; n8 represents number of 1 to 3; $X_6$ represents a carbon atom, nitrogen atom, oxygen atom or sulfur atom; in cases where $X_6$ is a carbon atom or nitrogen atom, $R_7$ exists and represents $C_1$-$C_6$ straight alkyl, $C_1$-$C_6$ straight acyl, $C_3$-$C_8$ branched alkyl, $C_3$-$C_8$ branched acyl, $C_6$-$C_{10}$ aryl or benzoyl group bound through 0 to 3 methylene groups, which aryl or benzoyl group is substituted with 0 to 2 hydroxyl groups, methoxy groups, chloro, bromo, fluoro, nitro groups or amino groups; $R_8$ represents Formula VI:

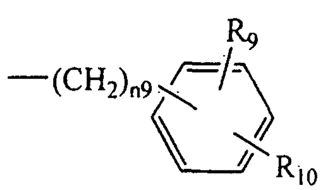

VI

(wherein n9 represents number of 0 to 3; $-(CH_2)_{n9}-$ is bound to an arbitrary position on the benzene ring; $R_9$ and $R_{10}$ may or may not exist, and may be the same or different, in cases where $R_9$ or $R_{10}$ exist, $R_9$ and $R_{10}$ are bound at ortho-, meta- or para-position to $-(CH_2)_{n9}-$, and represent $C_1$-$C_6$ straight alkyl, $C_3$-$C_8$ branched alkyl, hydroxy, methoxy, nitro, amino, chloro, bromo, fluoro, Formula III:

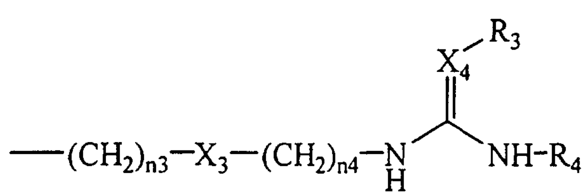

III

(wherein n3, n4, $X_3$, $X_4$, $R_3$ and $R_4$ represent the same meanings as described above), or Formula IV:

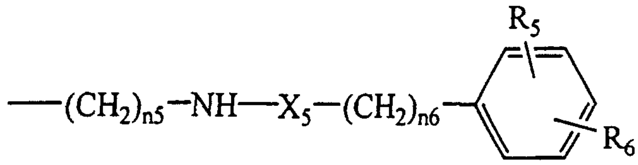

IV

(wherein n5, n6, $X_5$, $R_5$ and $R_6$ represent the same meanings as described above)
(v) Formula VII

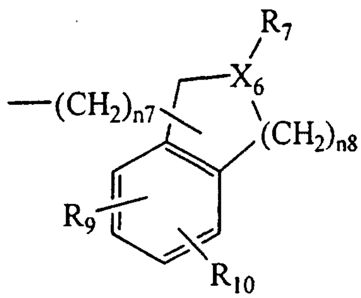

VII

(wherein n7, n8, $X_6$, $R_7$, $R_9$ and $R_{10}$ represent the same meanings described above);

D and O may or may not exist, and may be the same or different, in cases where D or O exist, D and O represent carbonyl group, sulfonyl group, $C_1$-$C_6$ methylene chain, nitrogen atom, oxygen atom or sulfor atom;

E and N may or may not exist, and may be the same or different, in cases where E or N exist, E and N represent $C_1$-$C_6$ methylene chain, nitrogen atom, oxygen atom or sulfur atom;

F and L may or may not exist, and may be the same or different, in cases where F or L exist, F and L represent carbon atom or nitrogen atom;

G and M may or may not exist, and may be the same or different, in cases where G or M exist, G and M respectively represent (i) Formula VIII:

$$-(CH_2)_{n9}COOR_{11} \qquad \text{VIII}$$

(wherein n9 represents number of 0 to 3; $R_{11}$ represents hydrogen, or $C_1$-$C_6$ straight alkyl);

(ii) hydrogen; (iii) hydroxyl group; (iv) amino group; (v) F or L are carbon atoms and the bonds between F and G, or between L and M are double bonds, they respectively represents an oxygen atom; (vi) E, F and G, or M, L and N cooperatively form Formula IX:

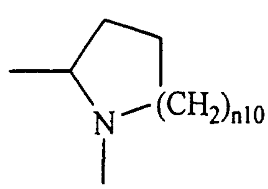

IX

(wherein n10 represents number of 1 to 3); or
(vii) tetrazole group;

H and K may or may not exist, and may be the same or different, in cases where H or K exist, they represent Formula X:

$$-(CH_2)_{n11}-X_7-(CH_2)_{n12}-$$

X

(wherein n11 and n12 represent numbers of 0 to 3; $X_7$ may or may not exist, in cases where $X_7$ exists, it represents a nitrogen atom, oxygen atom or sulfur atom);

I and J may or may not exist, and may be the same or different, in cases where I or J exist, they respectively represent (i) carbon atom; (ii) nitrogen atom; (iii) sulfur atom (with the proviso that the compounds represented by Formula I wherein both I and J are sulfur atoms, both D and O are carbonyl groups, both E and N are nitrogen atoms, all of F, L, K and H are carbon atoms, both G and M are carboxyl groups or ester groups, none of A, B, Q and P exists, and C and R respectively are hydrogen, $C_1$-$C_6$ straight alkyl, $C_3$-$C_8$ branched alkyl or those represented by Formula II wherein none of $X_1$, $X_2$, $R_1$ and $R_2$ exists, or $X_1$ is an oxygen atom and $X_2$ does not exist, □ is benzene ring, n1 = 1 to 3 and n2 = 0, are excluded); (iv) oxygen atom; (v) Formula XI (with the proviso that the compounds represented by Formula I wherein none of A, C, P, R, K, L and M exist, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom, G is a carboxyl group or an ester group, and H is methylene, are excluded)

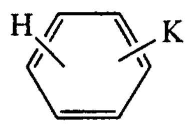

XI

(wherein H and K represent the same meanings as described above and are bound to the benzene ring at ortho-, meta- or para- positions)
(vi) Formula XII (with the proviso that the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom G is a carboxyl group or an ester group, and H is methylene, are excluded),

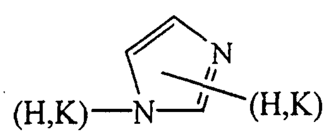

XII

(wherein H and K represent the same meanings as described above, one of H and K is bound to the nitrogen atom at the 1-position, and the other is bound to 2-, 4- or 5-position of the imidazole ring);
(vii) Formula XIII (with the proviso that the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom G is a carboxyl group or an ester group, and H is methylene, are excluded)

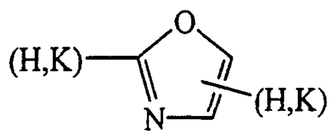

XIII

(wherein H and K represent the same meanings as described above, one of H and K is bound to the 2-position and the other is bound to the 4- or 5-position of the oxazole ring);

(viii) Formula xiv

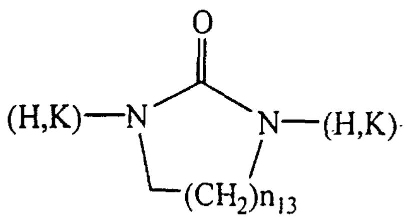

xiv

(wherein n13 represents number of 1 to 3; H and K represent the same meanings as described above with the proviso that one of H and K is bound to one of the nitrogen atoms and the other is bound to the other nitrogen atom); or

(ix) Formula xv:

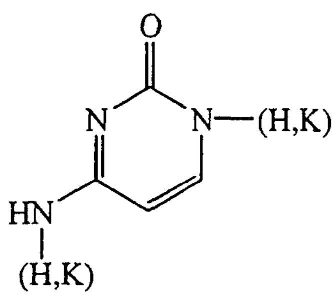

xv

(wherein H and K represent the same meanings as described above with the proviso that one of H and K is bound to one of the nitrogen atoms and the other is bound to the other nitrogen atom);

——— represents single bond or double bond; and

≡ represents single bond, double bond or triple bond]

and pharmaceutically acceptable salts thereof.

The present invention also provides pharmaceuticals comprising the compounds represented by the above-described Formula I as effective ingredients. Examples of the pharmaceuticals include, as will be hereinafter described in detail, therapeutic agents for inflammatory dieseases exploiting the adhesion molecules-inhibiting actions of the compounds.

The present invention also provides adhesion molecule inhibitors, especially VLA-4 inhibitors comprising carboxylic acid derivatives of the Formula I:

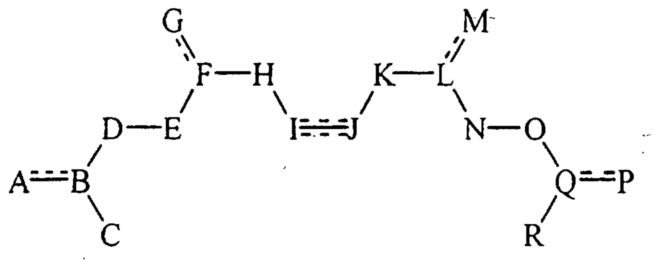

I

[In Formula I, A, C, P and R may or may not exist, and may be the same or different, in cases where A, C, P or R exist, they respectively represent (i) hydrogen, (ii) $C_1$-$C_6$ straight alkyl, (iii) $C_3$-$C_8$ branched alkyl, (iv) Formula II:

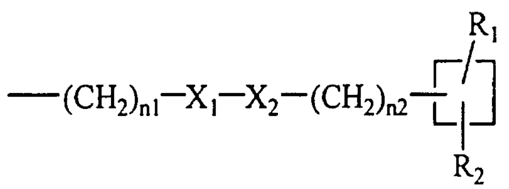

II

(wherein n1 and n2 represent numbers of 0 to 3, $X_1$ and $X_2$ may or may not exist, in cases where $X_1$ or $X_2$ exists, $X_1$ represents an oxygen atom, sulfur atom or nitrogen atom, $X_2$ represents carbonyl group or sulfonyl group; □ represents cyclohexane ring, benzene ring, naphthalene ring, indole ring, imidazole ring, furan ring, thiophen ring, pyrrole ring or pyridine ring; $R_1$ and $R_2$ may or may not exist, and may be the same or different, in cases where $R_1$ or $R_2$ exist, they respectively represent $C_1$-$C_6$ straight alkyl, $C_3$-$C_8$ branched alkyl, hydroxyl, methoxy, chloro, bromo, fluoro, nitro, amino, Formula III:

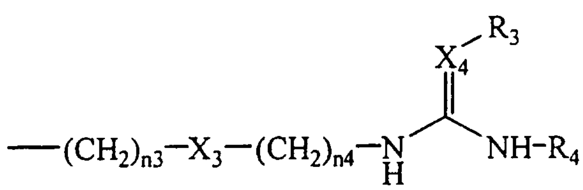

III

(wherein n3 and n4 represent numbers of 0 to 3; $X_3$ may or may not exist, in cases where $X_3$ exists, $X_3$ represents a nitrogen atom or oxygen atom; $X_4$ represents a carbon atom, oxygen atom, nitrogen atom or sulfur atom; in cases where $X_4$ is a carbon atom or nitrogen atom, $R_3$ exists and represents nitro, cyano, methylsulfonyl or phenylsulfonyl group; $R_4$ represents hydrogen, $C_1$-$C_6$ straight alkyl, $C_3$-$C_8$ branched alkyl, $C_6$-$C_{10}$ non-substituted aryl or $C_6$-$C_{10}$ aryl substituted with 0 to 2 hydroxyl groups, methoxy groups, nitro groups, amino groups, chloro, bromo and/or fluoro) or Formula IV:

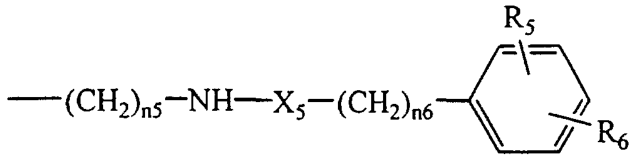

IV

(wherein n5 and n6 represent numbers of 0 to 3; $X_5$ represents carbonyl group or sulfonyl group; $R_5$ and $R_6$ represent hydrogen, methyl, methoxy, chloro, bromo, fluoro, nitro or amino groups)

B and Q may or may not exist, and may be the same or different, in cases where B or Q exist, they represent (i) nitrogen atom, (ii) carbon atom, (iii) $C_6$-$C_{10}$ aryl or cyclohexyl each of which is substituted with 0 to 2 hydroxyl groups, methoxy groups, chloro, bromo, fluoro, nitro, amino, or $C_6$-$C_{10}$ aryl or cyclohexyl each of which is bound through 1 to 3 methylene groups, each of which is substituted with 0 to 2 methoxy groups, chloro, bromo, fluoro, nitro, tetrazole or amino groups, (iv) Formula V:

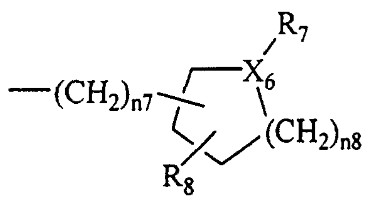

V

(wherein n7 represents number of 0 to 3; n8 represents number of 1 to 3; $X_6$ represents a carbon atom, nitrogen atom, oxygen atom or sulfur atom; in cases where $X_6$ is a carbon atom or nitrogen atom, $R_7$ exists and represents $C_1$-$C_6$ straight alkyl, $C_1$-$C_6$ straight acyl, $C_3$-$C_8$ branched alkyl, $C_3$-$C_8$ branched acyl, $C_6$-$C_{10}$ aryl or benzoyl group bound through 0 to 3 methylene groups, which aryl or benzoyl group is substituted with 0 to 2 hydroxyl groups, methoxy groups, chloro, bromo, fluoro, nitro groups and/or amino groups; $R_8$ represents Formula VI:

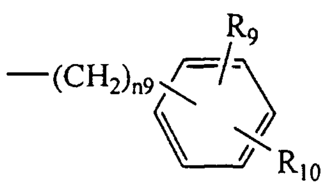

VI

(wherein n9 represents number of 0 to 3; $-(CH_2)_{n9}-$ is bound to an arbitrary position on the benzene ring; $R_9$ and $R_{10}$ may or may not exist, and may be the same or different, in cases where $R_9$ or $R_{10}$ exist, $R_9$ and $R_{10}$ are bound at ortho-, meta- or para-position to $-(CH_2)_{n9}-$, and represent $C_1$-$C_6$ straight alkyl, $C_3$-$C_8$ branched alkyl, hydroxy, methoxy, nitro, amino, chloro, bromo, fluoro, Formula III:

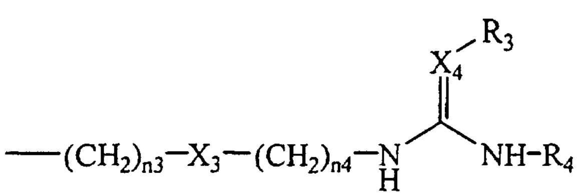

III

(wherein n3, n4, $X_3$, $X_4$, $R_3$ and $R_4$ represent the same meanings as described above), or Formula IV:

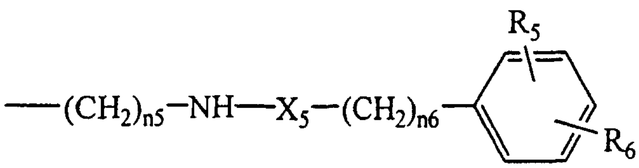

IV

(wherein n5, n6, $X_5$, $R_5$ and $R_6$ represent the same meanings as described above) (v) Formula VII

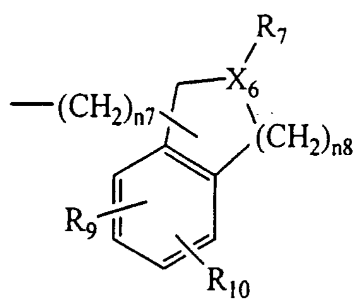

VII

(wherein n7, n8, $X_6$, $R_7$, $R_9$ and $R_{10}$ represent the same meanings as described above);

D and O may or may not exist, and may be the same or different, in cases where D or O exist, D and O represent carbonyl group, sulfonyl group, $C_1$-$C_6$ methylene chain, nitrogen atom, oxygen atom or sulfor atom;

E and N may or may not exist, and may be the same or different, in cases where E or N exist, E and N represent $C_1$-$C_6$ methylene chain, nitrogen atom, oxygen atom or sulfur atom;

F and L may or may not exist, and may be the same or different, in cases where F or L exist, F and L represent carbon atom or nitrogen atom;

G and M may or may not exist, and may be the same or different, in cases where G or M exist, G and M respectively represent (i) Formula VIII:

$$-(CH_2)_{n9}COOR_{11} \qquad \text{VIII}$$

(wherein n9 represents number of 0 to 3; $R_{11}$ represents hydrogen or $C_1$-$C_6$ straight alkyl);

(ii) hydrogen; (iii) hydroxyl group; (iv) amino group; (v) F or L are carbon atoms and the bonds between F and G, or between L and M are double bonds, they respectively represents an oxygen atom; (vi) E, F and G, or M, L and N cooperatively form Formula IX:

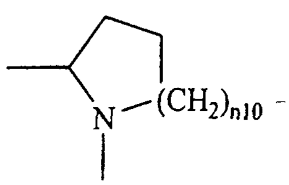

IX

(wherein n10 represents number of 1 to 3); or

(vii) tetrazole group;

H and K may or may not exist, and may be the same or different, in cases wherein H or K exist, they represent Formula X:

$$-(CH_2)_{n11}-X_7-(CH_2)_{n12}-$$

X

(wherein n11 and n12 represent numbers of 0 to 3; $X_7$ may or may not exist, in cases where $X_7$ exists, it represents a nitrogen atom, oxygen atom or sulfur atom);

I and J may or may not exist, and may be the same or different, in cases where I or J exist, they respectively represent (i) carbon atom; (ii) nitrogen atom; (iii) sulfur atom; (iv) oxygen atom; (v) Formula XI (with the proviso that the compounds represented by Formula I wherein none of A, C, P, R, K, L and M exist, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom, G is a carboxyl group or an ester group, and H is methylene, are excluded)

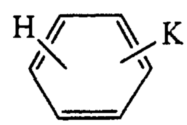

XI

(wherein H and K represent the same meanings as described above and are bound to the benzene ring at ortho-, meta- or para- positions)

(vi) Formula XII (with the proviso that the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom, G is a carboxyl group or an ester group, and H is methylene, are excluded)

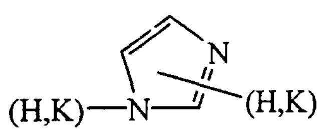

XII

(wherein H and K represent the same meanings as described above, one of H and K is bound to the nitrogen atom at the 1-position, and the other is bound to 2-, 4- or 5-position of the imidazole ring);

(vii) Formula XIII (with the proviso that the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom G is a carboxyl group or an ester group, and H is methylene, are excluded)

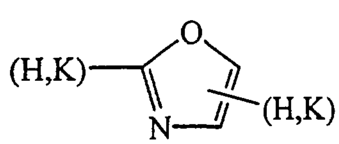

XIII

(wherein H and K represent the same meanings as described above, one of H and K is bound to the 2-position and other is bound to the 4- or 5-position of the oxazole ring);
(viii) Formula xiv

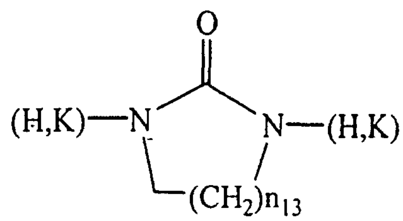

xiv

(wherein n13 represents number of 1 to 3; H and K represent the same meanings as described above with the proviso that one of H and K is bound to one of the nitrogen atoms and the other is bound to the other nitrogen atom); or
(ix) Formula xv:

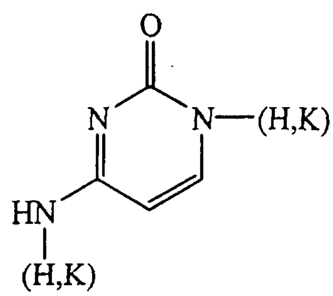

xv

(wherein H and K represent the same meanings as described above with the proviso that one of H and K is bound to one of the nitrogen atoms and the other is bound to the other nitrogen atom);
- - - - - — — — represents single bond or double bond; and
- - - - -

represents single bond, double bond or triple bond]
and pharmaceutically acceptable salts thereof. These adhesion molecule inhibitors make it possible to prevent or treat inflammatory diseases, especially allergic diseases.

## BRIEF DESCRIPTION OF THE DRAWINGS

Fig. 1 shows that Compound No. 140 inhibits the antigen-induced accumulation of eosinophils to auricles in mouse auricular edema models;
Fig. 2 shows that Compound No. 140 inhibits the antigen-induced accumulation of eosinophils to lungs in mouse asthma models; and
Fig. 3 shows that Compound No. 140 inhibits the antigen-induced increase in breathing resistance in guinea pig rhinitis models.

## BEST MODE FOR CARRYING OUT THE INVENTION

As mentioned above, the carboxylic acid derivatives according to the present invention are represented by

the above-described Formula I. In Formula I, A, C, P and R may or may not exist, and may be the same or different, in cases where A, C, P or R exist, they respectively represent (i) hydrogen, (ii) a $C_1$-$C_6$ straight alkyl group, that is, methyl, ethyl, n-propyl, n-butyl, n-pentyl or n-hexyl; (iii) a $C_3$-$C_8$ branched alkyl group such as 1-methylethyl, 2-methylethyl, 1,1-dimethylethyl, 1,2-dimethylethyl, 2,2-dimethylethyl, 1-methylpropyl, 2-methylpropyl, 3-methylpropyl, 1,1-dimethylpropyl, 2,2-dimethylpropyl, 3,3-dimethylpropyl, 1,2-dimethylpropyl, 1,3-dimethylpropyl, 1-methylbutyl, 2-methylbutyl, 3-methylbutyl, 4-methylbutyl, 1,1-dimethylbutyl, 2,2-dimethylbutyl, 3,3-dimethylbutyl, 4,4-dimethylbutyl, 1,2-dimethylbutyl, 1,3-dimethylbutyl, 1,4-dimethylbutyl, 2,3-dimethylbutyl, 2,4-dimethylbutyl, 1-methylpentyl, 2-methylpentyl, 3-methylpentyl, 4-methylpentyl, 5-methylpentyl, 1,1-dimethylpentyl, 2,2-dimethylpentyl, 3,3-dimethylpentyl, 4,4-dimethylpentyl, 5,5-dimethylpentyl, 1,2-dimethylpentyl, 1,3-dimethylpentyl, 1,4-dimethylpentyl, 1,5-dimethylpentyl, 2,3-dimethylpentyl, 2,4-dimethylpentyl, 2,5-dimethylpentyl, 3,4-dimethylpentyl, 3,5-dimethylpentyl, 4,5-dimethylpentyl, 1-methylhexyl, 2-methylhexyl, 3-methylhexyt, 4-methylhexyl, 5-methylhexyl, 6-methylhexyl, 1,1-dimethylhexyl, 2,2-dimethylhexyl, 3,3-dimethylhexyl, 4,4-dimethylhexyl, 5,5-dimethylhexyl, 6,6-dimethylhexyl, 1,2-dimethylhexyl, 1,3-dimethylhexyl, 1,4-dimethylhexyl, 1,5-dimethylhexyl, 1,6-dimethylhexyl, 2,3-dimethylhexyl, 2,4-dimethylhexyl, 2,5-dimethylhexyl, 2,6-dimethylhexyl, 3,4-dimethylhexyl, 3,5-dimethylhexyl, 3,6-dimethylhexyl, 4,5-dimethylhexyl or 5,6-dimethylhexyl group; (iv) Formula II:

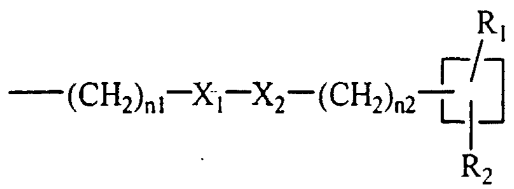

II

(wherein n1 and n2 represent numbers of 0 to 3, $X_1$ and $X_2$ may or may not exist, in cases where $X_1$ or $X_2$ exists, $X_1$ represents an oxygen atom, sulfur atom or nitrogen atom, $X_2$ represents carbonyl group or sulfonyl group; D represents cyclohexane ring, benzene ring, indole ring, imidazole ring, furan ring, thiophen ring, pyrrole ring or pyridine ring; $R_1$ and $R_2$ may or may not exist, and may be the same or different, in cases where $R_1$ or $R_2$ exist, they respectively represent a $C_1$-$C_6$ straight alkyl group, that is, methyl, ethyl, n-propyl, n-butyl, n-pentyl or n-hexyl; a $C_3$-$C_8$ branched alkyl group such as 1-methylethyl, 2-methylethyl, 1,1-dimethylethyl, 1,2-dimethylethyl, 2,2-dimethylethyl, 1-methylpropyl, 2-methylpropyl, 3-methylpropyl, 1,1-dimethylpropyl, 2,2-dimethylpropyl, 3,3-dimethylpropyl, 1,2-dimethylpropyl, 1,3-dimethylpropyl, 1-methylbutyl, 2-methylbutyl, 3-methylbutyl, 4-methylbutyl, 1,1-dimethylbutyl, 2,2-dimethylbutyl, 3,3-dimethylbutyl, 4,4-dimethylbutyl, 1,2-dimethylbutyl, 1,3-dimethylbutyl, 1,4-dimethylbutyl, 2,3-dimethylbutyl, 2,4-dimethylbutyl, 1-methylpentyl, 2-methylpentyl, 3-methylpentyl, 4-methylpentyl, 5-methylpentyl, 1,1-dimethylpentyl, 2,2-dimethylpentyl, 3,3-dimethylpentyl, 4,4-dimethylpentyl, 5,5-dimethylpentyl, 1,2-dimethylpentyl, 1,3-dimethylpentyl, 1,4-dimethylpentyl, 1,5-dimethylpentyl, 2,3-dimethylpentyl, 2,4-dimethylpentyl, 2,5-dimethylpentyl, 3,4-dimethylpentyl, 3,5-dimethylpentyl, 4,5-dimethylpentyl, 1-methylhexyl, 2-methylhexyl, 3-methylhexyl, 4-methylhexyl, 5-methylhexyl, 6-methylhexyl, 1,1-dimethylhexyl, 2,2-dimethylhexyl, 3,3-dimethylhexyl, 4,4-dimethylhexyl, 5,5-dimethylhexyl, 6,6-dimethylhexyl, 1,2-dimethylhexyl, 1,3-dimethylhexyl, 1,4-dimethylhexyl, 1,5-dimethylhexyl, 1,6-dimethylhexyl, 2,3-dimethylhexyl, 2,4-dimethylhexyl, 2,5-dimethylhexyl, 2,6-dimethylhexyl, 3,4-dimethylhexyl, 3,5-dimethylhexyl, 3,6-dimethylhexyl, 4,5-dimethylhexyl or 5,6-dimethylhexyl group; hydroxyl group, methoxy group, chloro, bromo, fluoro, nitro group or amino group; Formula III:

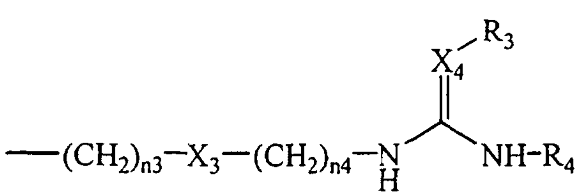

III

(wherein n3 and n4 represent numbers of 0 to 3; $X_3$ may or may not exist, in cases where $X_3$ exists, $X_3$ represents a nitrogen atom or oxygen atom; $X_4$ represents a carbon atom, oxygen atom, nitrogen atom or sulfur atom; in cases

where $X_4$ is a carbon atom or nitrogen atom, $R_3$ exists and represents nitro, cyano, methylsulfonyl or phenylsulfonyl group; $R_4$ represents hydrogen, a $C_1$-$C_6$ straight alkyl group, that is, methyl, ethyl, n-propyl, n-butyl, n-pentyl or n-hexyl; a $C_3$-$C_8$ branched alkyl group such as 1-methylethyl, 2-methylethyl, 1,1-dimethylethyl, 1,2-dimethylethyl, 2,2-dimethylethyl, 1-methylpropyl, 2-methylpropyl, 3-methylpropyl, 1,1-dimethylpropyl, 2,2-dimethylpropyl, 3,3-dimethylpropyl, 1,2-dimethylpropyl, 1,3-dimethylpropyl, 1-methylbutyl, 2-methylbutyl, 3-methylbutyl, 4-methylbutyl, 1,1-dimethylbutyl, 2,2-dimethylbutyl, 3,3-dimethylbutyl, 4,4-dimethylbutyl, 1,2-dimethylbutyl, 1,3-dimethylbutyl, 1,4-dimethylbutyl, 2,3-dimethylbutyl, 2,4-dimethylbutyl, 1-methylpentyl, 2-methylpentyl, 3-methylpentyl, 4-methylpentyl, 5-methylpentyl, 1,1-dimethylpentyl, 2,2-dimethylpentyl, 3,3-dimethylpentyl, 4,4-dimethylpentyl, 5,5-dimethylpentyl, 1,2-dimethylpentyl, 1,3-dimethylpentyl, 1,4-dimethylpentyl, 1,5-dimethylpentyl, 2,3-dimethylpentyl, 2,4-dimethylpentyl, 2,5-dimethylpentyl, 3,4-dimethylpentyl, 3,5-dimethylpentyl, 4,5-dimethylpentyl, 1-methylhexyl, 2-methylhexyl, 3-methylhexyl, 4-methylhexyl, 5-methylhexyl, 6-methylhexyl, 1,1-dimethylhexyl, 2,2-dimethylhexyl, 3,3-dimethylhexyl, 4,4-dimethylhexyl, 5,5-dimethylhexyl, 6,6-dimethylhexyl, 1,2-dimethylhexyl, 1,3-dimethylhexyl, 1,4-dimethylhexyl, 1,5-dimethylhexyl, 1,6-dimethylhexyl, 2,3-dimethylhexyl, 2,4-dimethylhexyl, 2,5-dimethylhexyl, 2,6-dimethylhexyl, 3,4-dimethylhexyl, 3,5-dimethylhexyl, 3,6-dimethylhexyl, 4,5-dimethylhexyl or 5,6-dimethylhexyl group; substituted or non-substituted $C_6$-$C_{10}$ aryl group such as phenyl, benzyl, phenylethyl, phenylpropyl, phenylbutyl, 2-methylphenyl, 2-methylbenzyl, 2-(2-methylphenyl)ethyl, 3-(2-(methylphenyl)propyl, 3-methylphenyl, 3-methylbenzyl, 2-(3-methylphenyl)ethyl, 4-(3-methylphenyl)propyl, 4-methylphenyl, 4-methylbenzyl, 2-(4-methylphenyl)ethyl, 3-(4-methylphenyl)propyl, 2-propylphenyl, 2-propylbenzyl, 3-propylphenyl, 3-propylbenzyl, 2,3-dimethylphenyl, 2,3-dimethylbenzyl, (2,3-dimethyl-phenyl)ethyl, 3,4-dimethyl-phenyl, 3,4-dimethyl-benzyl, (3,4-dimethyl-phenyl)ethyl, 2,4-dimethyl-phenyl, 2,4-dimethyl-benzyl or (2,4-dimethyl-phenyl)ethyl; or aryl group substituted with 0 to 2 hydroxyl groups, methoxy groups, nitro groups, amino groups, chloro, bromo or fluoro, such as 4-hydroxyphenyl, 4-methoxyphenyl, 4-chlorophenyl, 4-nitrophenyl, 4-aminophenyl, 4-bromophenyl, 4-fluorophenyl, 2-hydroxyphenyl, 2-chlorophenyl, 2-nitrophenyl, 2-aminophenyl, 2-bromophenyl, 2-fluorophenyl, 2,4-dihydroxyphenyl, 2,4-dimetehoxyphenyl, 2,4-dichlorophenyl, 2,4-dinitrophenyl, 2,4-diaminophenyl, 2,4-dibromophenyl or 2,4-difluorophenyl; or Formula IV:

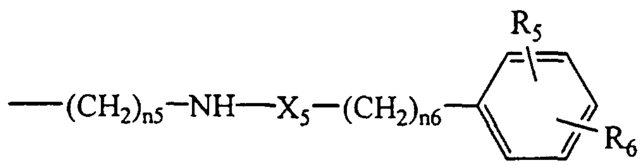

IV

(wherein n5 and n6 represent numbers of 0 to 3; $X_5$ represents carbonyl group or sulfonyl group; $R_5$ and $R_6$ represent hydroxyl group, chloro, bromo, fluoro, nitro or amino groups).

B and Q may or may not exist, and may be the same or different, in cases where B or Q exist, they represent (i) nitrogen atom, (ii) carbon atom, (iii) aryl or cyclohexyl group each of which is substituted with 0 to 2 hydroxyl groups, methoxy groups, chloro, bromo, fluoro, nitro groups, or amino group, such as 4-hydroxyphenyl, 4-methoxyphenyl, 4-chlorophenyl, 4-nitrophenyl, 4-aminophenyl, 4-bromophenyl, 4-fluorophenyl, 2-hydroxyphenyl, 2-chlorophenyl, 2-nitrophenyl, 2-aminophenyl, 2-bromophenyl, 2-fluorophenyl, 2,4-dihydroxyphenyl, 2,4-dimethoxyphenyl, 2,4-dichclorophenyl, 2,4-dinitrophenyl, 2,4-diaminophenyl, 2,4-dibromophenyl, 2,4-difluorophenyl, 2,6-dimethylphenyl, 2,6-diehylphenyl, 2,6-dihydroxyphenyl, 2,6-dimethoxyphenyl, 2,6-dichlorophenyl, 2,6-dinitrophenyl, 2,6-diaminophenyl, 2,6-dibromophenyl, 2,6-difluorophenyl, 4-hydroxycyclohexyl, 4-chlorocyclohexyl, 4-nitrocyclohexyl, 4-aminocyclohexyl, 4-bromocyclohexyl, 4-fluorocyclohexyl, 2-hydroxycyclohexyl, 2-chlorocyclohexyl, 2-nitrocyclohexyl, 2-aminocyclohexyl, 2-bromocyclohexyl, 2-fluorocyclohexyl, 2,4-dihydroxycyclohexyl, 2,4-dichlorocyclohexyl, 2,4-dinitrocyclohexyl, 2,4-diaminocyclohexyl, 2,4-dibromocyclohexyl or 2,4-difluorocyclohexyl group; (iv) Formula V:

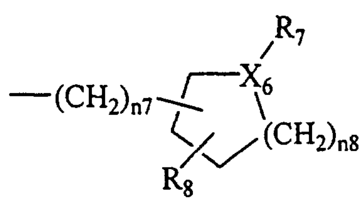

V

(wherein n7 represents number of 0 to 3; n8 represents number of 1 to 3; $X_6$ represents a carbon atom, nitrogen atom, oxygen atom or sulfur atom; in cases where $X_6$ is a carbon atom or nitrogen atom, $R_7$ exists and represents a $C_1$-$C_6$ straight alkyl group, that is, methyl, ethyl, n-propyl, n-butyl, n-pentyl or n-hexyl; $C_1$-$C_6$ straight acyl, that is, acetoxy, propionyl, butyryl, valeryl or n-hexanoyl; a $C_3$-$C_8$ branched alkyl group such as 1-methylethyl, 2-methylethyl, 1,1-dimethylethyl, 1,2-dimethylethyl, 2,2-dimethylethyl, 1-methylpropyl, 2-methylpropyl, 3-methylpropyl, 1,1-dimethylpropyl, 2,2-dimethylpropyl, 3,3-dimethylpropyl, 1,2-dimethylpropyl, 1,3-dimethylpropyl, 1-methylbutyl, 2-methylbutyl, 3-methylbutyl, 4-methylbutyl, 1,1-dimethylbutyl, 2,2-dimethylbutyl, 3,3-dimethylbutyl, 4,4-dimethylbutyl, 1,2-dimethylbutyl, 1,3-dimethylbutyl, 1,4-dimethylbutyl, 2,3-dimethylbutyl, 2,4-dimethylbutyl, 1-methylpentyl, 2-methylpentyl, 3-methylpentyl, 4-methylpentyl, 5-methylpentyl, 1,1-dimethylpentyl, 2,2-dimethylpentyl, 3,3-dimethylpentyl, 4,4-dimethylpentyl, 5,5-dimethylpentyl, 1,2-dimethylpentyl, 1,3-dimethylpentyl, 1,4-dimethylpentyl, 1,5-dimethylpentyl, 2,3-dimethylpentyl, 2,4-dimethylpentyl, 2,5-dimethylpentyl, 3,4-dimethylpentyl, 3,5-dimethylpentyl, 4,5-dimethylpentyl, 1-methylhexyl, 2-methylhexyl, 3-methylhexyl, 4-methylhexyl, 5-methylhexyl, 6-methylhexyl, 1,1-dimethylhexyl, 2,2-dimethylhexyl, 3,3-dimethylhexyl, 4,4-dimethylhexyl, 5,5-dimethylhexyl, 6,6-dimethylhexyl, 1,2-dimethylhexyl, 1,3-dimethylhexyl, 1,4-dimethylhexyl, 1,5-dimethylhexyl, 1,6-dimethylhexyl, 2,3-dimethylhexyl, 2,4-dimethylhexyl, 2,5-dimethylhexyl, 2,6-dimethylhexyl, 3,4-dimethylhexyl, 3,5-dimethylhexyl, 3,6-dimethylhexyl, 4,5-dimethylhexyl or 5,6-dimethylhexyl group; $C_3$-$C_8$ branched acyl group such as 1-methylethanoyl, 2-methylethanoyl, 1,1-dimethylethanoyl, 1,2-dimethylethanoyl, 2,2-dimethylethanoyl, 1-methylpropanoyl, 2-methylpropanoyl, 3-methylpropanoyl, 1,1-dimethylpropanoyl, 2,2-dimethylpropanoyl, 3,3-dimethylpropanoyl, 1,2-dimethylpropanoyl, 1,3-dimethylpropanoyl, 1-methylbutanoyl, 2-methylbutanoyl, 3-methylbutanoyl, 4-methylbutanoyl, 1,1-dimethylbutanoyl, 2,2-dimethylbutanoyl, 3,3-dimethylbutanoyl, 4,4-dimethylbutanoyl, 1,2-dimethylbutanoyl, 1,3-dimethylbutanoyl, 1,4-dimethylbutanoyl, 2,3-dimethylbutanoyl, 2,4-dimethylbutanoyl, 1-methylpentanoyl, 2-methylpentanoyl, 3-methylpentanoyl, 4-methylpentanoyl, 5-methylpentanoyl, 1,1-dimethylpentanoyl, 2,2-dimethylpentanoyl, 3,3-dimethylpentanoyl, 4,4-dimethylpentanoyl, 5,5-dimethylpentanoyl, 1,2-dimethylpentanoyl, 1,3-dimethylpentanoyl, 1,4-dimethylpentanoyl, 1,5-dimethylpentanoyl, 2,3-dimethylpentanoyl, 2,4-dimethylpentanoyl, 2,5-dimethylpentanoyl, 3,4-dimethylpentanoyl, 3,5-dimethylpentanoyl, 4,5-dimethylpentanoyl, 1-methylhexanoyl, 2-methylhexanoyl, 3-methylhexanoyl, 4-methylhexanoyl, 5-methylhexanoyl, 6-methylhexanoyl, 1,1-dimethylhexanoyl, 2,2-dimethylhexanoyl, 3,3-dimethylhexanoyl, 4,4-dimethylhexanoyl, 5,5-dimethylhexanoyl, 6,6-dimethylhexanoyl, 1,2-dimethylhexanoyl, 1,3-dimethylhexanoyl, 1,4-dimethylhexanoyl, 1,5-dimethylhexanoyl, 1,6-dimethylhexanoyl, 2,3-dimethylhexanoyl, 2,4-dimethylhexanoyl, 2,5-dimethylhexanoyl, 2,6-dimethylhexanoyl, 3,4-dimethylhexanoyl, 3,5-dimethylhexanoyl, 3,6-dimethylhexanoyl, 4,5-dimethylhexanoyl or 5,6-dimethylhexanoyl group; an aryl or benzoyl group bound through 1 to 3 methylene groups, which aryl or benzoyl group is substituted with 0 to 2 hydroxyl groups, methoxy groups, chloro, bromo, fluoro, nitro groups or amino groups, such as 4-hydroxybenzyl, 4-methoxyphenyl, 4-chlorobenzyl, 4-nitrobenzyl, 4-aminobenzyl, 4-bromobenzyl, 4-fluorobenzyl, 2-hydroxybenzyl, 2-chlorobenzyl, 2-nitrobenzyl, 2-aminobenzyl, 2-bromobenzyl, 2-fluorobenzyl, 2,4-dihydroxybenzyl, 2,4-dimethoxyphenyl, 2,4-dichlorobenzyl, 2,4-dinitrobenzyl, 2,4-diaminobenzyl, 2,4-dibromobenzyl, 2,4-difluorobenzyl, (4-hydroxyphenyl)ethyl, (4-chlorophenyl)ethyl, (4-nitrophenyl)ethyl, (4-aminophenyl)ethyl, (4-bromophenyl)ethyl, (4-fluorophenyl)ethyl, (2-hydroxyphenyl)ethyl, (2-chlorophenyl)ethyl, (2-nitrophenyl)ethyl, (2-aminophenyl)ethyl, (2-bromophenyl)ethyl, (2-fluorophenyl)ethyl, (2,4-dihydroxyphenyl)ethyl, (2,4-dichlorophenyl) ethyl, (2,4-dinitrophenyl)ethyl, (2,4-diaminophenyl)ethyl, (2,4-dibromophenyl)ethyl, (2,4-difluorophenyl)ethyl, 4-hydroxybenzoyl, 4-methoxybenzoyl, 4-chlorobenzoyl, 4-nitrobenzoyl, 4-aminobenzoyl, 4-bromobenzoyl, 4-fluorobenzoyl, 2-hydroxybenzoyl, 2-chlorobenzoyl, 2-nitrobenzoyl, 2-aminobenzoyl, 2-bromobenzoyl, 2-fluorobenzoyl, 2,4-dihydroxybenzoyl, 2,4-dimethoxybenzoyl, 2,4-dichlorobenzoyl, 2,4-dinitrobenzoyl, 2,4-diaminobenzoyl, 2,4-dibromobenzoyl or 2,4-difluorobenzoyl group; $R_8$ represents Formula VI:

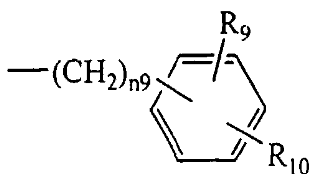

VI

(wherein n9 represents number of 0 to 3; $-(CH_2)_{n9}-$ is bound to an arbitrary position on the benzene ring; $R_9$ and $R_{10}$ may or may not exist, and may be the same or different, in cases where $R_9$ or $R_{10}$ exist, $R_9$ and $R_{10}$ are bound at ortho-, meta- or para-position to $-(CH_2)_{n9}-$, and represent a $C_1$-$C_6$ straight alkyl group, that is, methyl, ethyl, n-propyl, n-butyl, n-pentyl or n-hexyl; a $C_3$-$C_8$ branched alkyl group such as 1-methylethyl, 2-methylethyl, 1,1-dimethylethyl, 1,2-dimethylethyl, 2,2-dimethylethyl, 1-methylpropyl, 2-methylpropyl, 3-methylpropyl, 1,1-dimethylpropyl, 2,2-dimethylpropyl,3,3-dimethylpropyl, 1,2-dimethylpropyl, 1,3-dimethylpropyl, 1-methylbutyl, 2-methylbutyl, 3-methylbutyl, 4-methylbutyl, 1,1-dimethylbutyl, 2,2-dimethylbutyl, 3,3-dimethylbutyl, 4,4-dimethylbutyl, 1,2-dimethylbutyl, 1,3-dimethylbutyl, 1,4-dimethylbutyl, 2,3-dimethylbutyl, 2,4-dimethylbutyl, 1-methylpentyl, 2-methylpentyl, 3-methylpentyl, 4-methylpentyl, 5-methylpentyl, 1,1-dimethylpentyl, 2,2-dimethylpentyl, 3,3-dimethylpentyl, 4,4-dimethylpentyl, 5,5-dimethylpentyl, 1,2-dimethylpentyl, 1,3-dimethylpentyl, 1,4-dimethylpentyl, 1,5-dimethylpentyl, 2,3-dimethylpentyl, 2,4-dimethylpentyl, 2,5-dimethylpentyl, 3,4-dimethylpentyl, 3,5-dimethylpentyl, 4,5-dimethylpentyl, 1-methylhexyl, 2-methylhexyl, 3-methylhexyl, 4-methylhexyl, 5-methylhexyl, 6-methylhexyl, 1,1-dimethylhexyl, 2,2-dimethylhexyl, 3,3-dimethylhexyl, 4,4-dimethylhexyl, 5,5-dimethylhexyl, 6,6-dimethylhexyl, 1,2-dimethylhexyl, 1,3-dimethylhexyl, 1,4-dimethylhexyl, 1,5-dimethylhexyl, 1,6-dimethylhexyl, 2,3-dimethylhexyl, 2,4-dimethylhexyl, 2,5-dimethylhexyl, 2,6-dimethylhexyl, 3,4-dimethylhexyl, 3,5-dimethylhexyl, 3,6-dimethylhexyl, 4,5-dimethylhexyl or 5,6-dimethylhexyl group; hydroxy, nitro, amino, chloro, bromo, fluoro, Formula III:

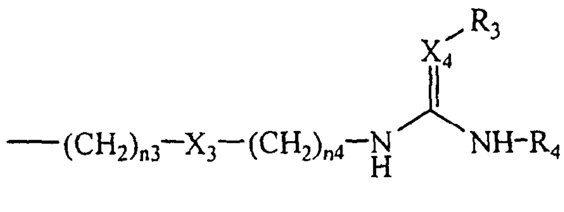

III

(wherein n3, n4, $X_3$, $X_4$, $R_3$ and $R_4$ represent the same meanings as described above), or Formula IV:

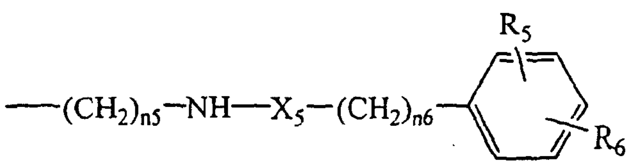

IV

(wherein n5, n6, $X_5$, $R_5$ and $R_6$ represent the same meanings as described above)
(v) Formula VII

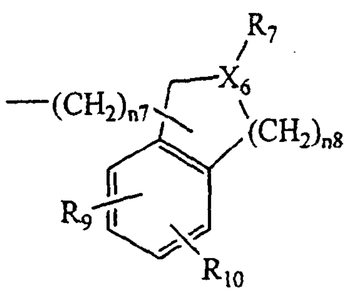

VII

(wherein n7, n8, $X_6$, $R_7$, $R_9$ and $R_{10}$ represent the same meanings described above);

D and O may or may not exist, and may be the same or different, in cases where D or O exist, D and O represent carbonyl group, sulfonyl group, a $C_1$-$C_6$ methylene chain, that is, methylene, ethylene, trimethylene, tetramethylene, pentamethylene or hexamethylene, nitrogen atom, oxygen atom or sulfur atom; E and N may or may not exist, and may be the same or different, in cases where E or N exist, E and N represent a $C_1$-$C_6$ methylene chain, that is, methylene, ethylene, trimethylene, tetramethylene, pentamethylene or hexamethylene, nitrogen atom, oxygen atom or sulfur atom; F and L may or may not exist, and may be the same or different, in cases where F or L exist, F and L represent carbon atom or nitrogen atom; G and M may or may not exist, and may be the same or different, in cases where G or

M exist, G and M respectively represent (i) Formula VIII:

$$—(CH_2)_{n9}COOR_{11} \qquad \text{VIII}$$

(wherein n9 represents number of 0 to 3; $R_{11}$ represents hydrogen, or a $C_1$-$C_6$ straight alkyl, that is, methyl, ethyl, n-propyl, n-butyl, n-pentyl or n-hexyl; (ii) hydrogen; (iii) hydroxyl group; (iv) amino group; (v) F or L are carbon atoms and the bonds between F and G, or between L and M are double bonds, they respectively represents an oxygen atom; (vi) E, F and G, or M, L and N cooperatively form Formula IX:

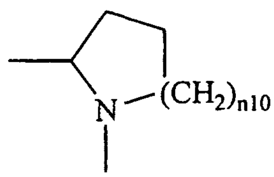

IX

(wherein n10 represents number of 1 to 3); H and K may or may not exist, and may be the same or different, in cases where H or K exist, they represent Formula X:

$$—(CH_2)_{n11}\text{-}X_7\text{-}(CH_2)_{n12}— \qquad \text{X}$$

(wherein n11 and n12 represent numbers of 0 to 3; $X_7$ may or may not exist, in cases where $X_7$ exists, it represents a nitrogen atom, oxygen atom or sulfur atom); I and J may or may not exist, and may be the same or different, in cases where I or J exist, they respectively represent (i) carbon atom; (ii) nitrogen atom; (iii) sulfur atom (with the proviso that the compounds represented by Formula I wherein both I and J are sulfur atoms, both D and O are carbonyl groups, both E and N are nitrogen atoms, all of F, L, K and H are carbon atoms, both G and M are carboxyl groups or ester groups, and A, B and C, or P, Q and N cooperatively form $C_1$-$C_6$ straight alkyl or $C_3$-$C_8$ branched alkyl or represented by Formula II wherein none of $X_1$, $X_2$, $R_1$ and $R_2$ exists or $X_1$ is oxygen atom and none of $X_2$, $R_1$ and $R_2$ exists, are excluded); (iv) oxygen atom; (v) Formula XI:

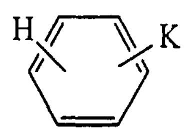

XI

(wherein H and K represent the same meanings as described above and are bound to the benzene ring at ortho-, meta- or para- positions)
(vi) Formula XII

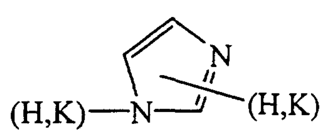

XII

(wherein H and K represent the same meanings as described above, one of H and K is bound to the nitrogen atom at

the 1-position, and the other is bound to 2-, 4- or 5-position of the imidazole ring);
(vii) Formula XIII:

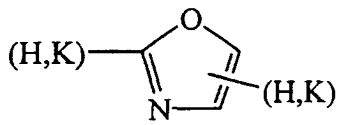

XIII

(wherein H and K represent the same meanings as described above, one of H and K is bound to the 2-position and the other is bound to the 4- or 5-position of the oxazole ring);
(viii) Formula xiv

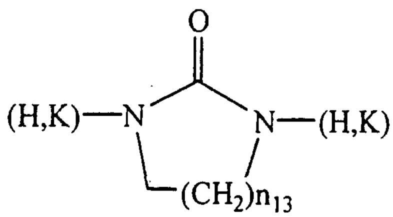

xiv

(wherein n13 represents number of 1 to 3; H and K represent the same meanings as described above with the proviso that one of H and K is bound to one of the nitrogen atoms and the other is bound to the other nitrogen atom); or
(ix) Formula xv:

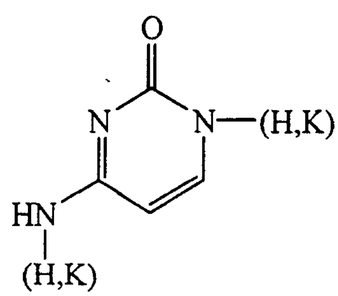

xv

(wherein H and K represent the same meanings as described above with the proviso that one of H and K is bound to one of the nitrogen atoms and the other is bound to the other nitrogen atom);
— — — represents single bond or double bond; and
represents single bond, double bond or triple bond].

Preferred are the carboxylic acid derivatives and pharmaceutically acceptable salts thereof according to claim 1, wherein A, C, P, R, H and K represent the same meanings as described above; I and J may or may not exist, and may be the same or different, in cases where I or J exist, they respectively represent (i) carbon atom; (ii) sulfur atom (with the proviso that the compounds represented by Formula I wherein both I and J are sulfur atoms, both D and O are carbonyl groups, both E and N are nitrogen atoms, all of F, L, K and H are carbon atoms, both G and M are carboxyl groups or ester groups, none of A, B, Q and P exists, and C and R respectively are hydrogen, $C_1$-$C_6$ straight alkyl, $C_3$-$C_8$ branched alkyl or those represented by Formula II wherein none of $X_1$, $X_2$, $R_1$ and $R_2$ exists, or $X_1$ is an oxygen atom and $X_2$ does not exist, □ is benzene ring, n1 = 1 to 3 and n2 = 0, are excluded); (iii) nitrogen atom; (iv) oxygen

atom; B and Q may or may not exist, and may be the same or different, in cases where B or Q exist, they represent carbon atom, $C_6$-$C_{10}$ aryl group bound through 0 to 2 methylene groups, which aryl group is substituted with 0 to 3 methyl groups, ethyl groups, hydroxyl groups, methoxy groups, nitro groups, amino groups, chloro, bromo, fluoro or tetrazole groups, Formula V or Formula VII; D and O may or may not exist, and may be the same or different, in cases where D or O exist, D and O represent carbonyl group, sulfonyl group, $C_1$-$C_6$ methylene chain; E and N may or may not exist, and may be the same or different, in cases where E or N exist, E and N represent $C_1$-$C_3$ methylene chain, nitrogen atom, oxygen atom, carbon atom or sulfur atom; F and L may or may not exist, and may be the same or different, in cases where F or L exist, F and L represent carbon atom; G and M may or may not exist, and may be the same or different, in cases where G or M exist, G and M respectively represent hydrogen, tetrazole group or $-(CH_2)_{n9}COOH$, E, F and G, or M, L and N may or may not cooperatively form Formula IX; in cases where B, Q, I or J are carbon atoms, the bonds between A and B, between Q and P, or between I and J are single bonds or double bonds; the definitions other than mentioned above being the same as those recited in claim 1. More preferred are the carboxylic acid derivatives and pharmaceutically acceptable salts thereof according to claim 1, wherein in Formula I, A, C, P, R, H and K are those described above; I and J may or may not exist, and may be the same or different, in cases where I or J exist, they respectively represent Formula XI (with the proviso that the compounds represented by Formula I wherein none of A, C, P, R, K, L and M exist, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom, G is a carboxyl group or an ester group, and H is methylene, are excluded); (vi) Formula xiv or (vii) Formula xv; B and Q may or may not exist, and may be the same or different, in cases where B or Q exist, they represent carbon atom, $C_6$-$C_{10}$ aryl group bound through 0 to 2 methylene groups, which aryl group is substituted with 0 to 3 methyl groups, ethyl groups, hydroxyl groups, methoxy groups, nitro groups, amino groups, chloro, bromo, fluoro or tetrazole groups, Formula V or Formula VII; D and O may or may not exist, and may be the same or different, in cases where D or O exist, D and O represent carbonyl group, sulfonyl group, $C_1$-$C_6$ methylene chain; E and N may or may not exist, and may be the same or different, in cases where E or N exist, E and N represent $C_1$-$C_3$ methylene chain, nitrogen atom, oxygen atom, carbon atom or sulfur atom; F and L may or may not exist, and may be the same or different, in cases where F or L exist, F and L represent carbon atom; G and M may or may not exist, and may be the same or different, in cases where G or M exist, G and M respectively represent hydrogen, tetrazole group or $-(CH_2)_{n9}COOH$, in cases where B, Q, I or J are carbon atoms, the bonds between A and B, between Q and P, or between I and J are single bonds or double bonds; the definitions other than mentioned above being the same as those recited in claim 1.

In the definitions of each substituents, the phrase that a certain group does "not exist" or "no group" exists means that the group is "not shown in the structural formula", and includes the both cases where atom(s) actually does (do) not exist and hydrogen atom(s) exist(s). For example, in Formula I, in case where B is a carbon atom and C does not exist, since C bound to B is not shown in the structural formula, one or two hydrogen atoms exist as B. On the other hand, in Formula I, in case where J does not exist, I and K are directly bound. Those skilled in the art can easily and definately understand what a specific structural formula means.

In the present invention, "aryl group" is preferably a $C_6$-$C_{10}$ aryl group, and "$C_6$-$C_{10}$ aryl group" is preferably phenyl group or naphthyl group.

Specific examples of the compounds represented by Formula I include

4-(2(R)-carboxy-2(R)-(2(R,S)-(4-hydroxyphenyl)-4-methylpentanoylamino)ethylthio)-2(S)-(2(R,S)-(4-hydroxyphenyl)-4-methylpentanoylamino) butyric acid,

4-(2(R)-carboxy-2(R)-(3,3-dimethylbutanoylamino)ethylthio)-2(S)-(2(R,S)-(4-hydroxyphenyl)-4-methylpentanoylamino) butyric acid,

3-(3-(2(R,S)-(4-hydroxyphenyl)-4-methylpentanoylamino)propylthio)-2(R)-(3,3-dimethylbutanoylamino)propionic acid,

3-(3-(2(S)-(4-hydroxyphenyl)-4-methylpentanoylamino)propylthio)-2(R)-(3,3-dimethylbutanoylamino)propionic acid,

3-(3-(2(R)-(4-hydroxyphenyl)-4-methylpentanoylamino)propylthio)-2(R)-(3,3-dimethylbutanoylamino)propionic acid,

3-(3-(2(R,S)-(4-((1-amino-2-aza-2-cyanovinyl)amino)phenyl)-4-methylpentanoylamino)propylthio-2R)-(3,3-dimethylbutanoylamino)propionic acid,

4-(2(R)-carboxy-2-(R-(2(R,S)-(4-hydroxyphenyl)-4-methylpentanoylamino)ethylthio-2(S)-(3,3-dimethylbutanoylamino) butyric acid,

4-(2-(2(R,S)-(4-acetyloxyphenyl)-4-methylpentanoylamino)ethoxy-2(S)-(3,3-dimethylbutanoylamino) butyric acid,

3-((2(R)-carboxy-2-(R)-(3,3-dimethylbutanoylamino)ethyl)disulfanyl)-2(R)-(3,3-dimethyl-2(S)-phenylmethoxy)butanoylamino)propionic acid,

3-((2(R)-carboxy-2-(R)-(3,3-dimethylbutanoylamino)ethyl)disulfanyl)-2(R)-(4-methyl-2(S)-phenylmethoxy)pentanoylamino)propionic acid,

3-((2(R)-carboxy-2-(R)-(3,3-dimethylbutanoylamino)ethyl)disulfanyl)-2(R)-(3,3-dimethyl-2(R,S)-benzylbutanoylamino)propionic acid,

3-((2(R)-carboxy-2-(R)-(3,3-dimethylbutanoylamino)ethyl)disulfanyl)-2(R)-(2(R,S)-(*tert*-butyl)-5-phenylpentanoylamino)propionic acid,
3-((2(R)-carboxy-2-(R)-(3,3-dimethylbutanoylamino)ethyl)disulfanyl)-2(R)-(3,3-dimethylbutanoylamino)ethyl)disulfanyl-2(R)-(3,3-dimethyl-2(S)-((4-methoxyphenyl)methoxy)butanoylamino)propionic acid,
3-((2(R)-carboxy-2-(R)-((tert-butoxy)carbonylamino)ethyl)disulfanyl)-2(R)-(4-methyl-2(S)-((4-methoxyphenyl)methoxy)pentanoylamino)propionic acid,
3-((2(R)-carboxy-2-(R)-((tert-butoxy)carbonylamino)ethyl)disulfanyl)-2(R)-(2(S)-((4-hydroxyphenyl)methoxy)-4-methylpentanoylamino)propionic acid,
3 -((2(R)-carboxy-2-(R)-(2(S)-((3 -hydroxyphenyl)methoxy-4-methylpentanoylamino)ethyl)disulfanyl)-2(R) -(3,3-dimethylbutanoylamino) propionic acid,
3-((2(R)-carboxy-2-(R)-(2(S)-((2-hydroxyphenyl)methoxy-4-methylpentanoylamino)ethyl)disulfanyl)-2(R)-(3,3-dimethylbutanoylamino) propionic acid,
3-((2(R)-carboxy-2-(R)-(2(R,S)-(4-hydroxyphenyl)-4-methylpentanoylamino)ethyl)disulfanyl)-2(R)-(3,3-dimethylbutanoylamino) propionic acid,
3-((2(R)-carboxy-2-(R)-(2(R,S)-(3-(4-hydroxyphenyl)propyl)-4-methylpentanoylamino)ethyl)disulfanyl)-2(R) -(3,3-dimethylbutanoylamino) propionic acid,
3-((2(R)-carboxy-2-(3-(methylbutanoylamino)ethyl)disulfanyl)-2(R)-(3,3-dimethylbutanoylamino)propionic acid,
3-((2(R)-(2(R,S)-(2-(4-acetyloxyphenyl)ethyl)-4-methylpentanoylamino-2(R)-(methoxycarbonyl)ethyl)disulfanyl)-2(R) -(3,3-dimethylbutanoylamino)propionic acid,
3-((2(R)-carboxy-2(R)-(2(R,S)-(2-(3-hydroxyphenyl)ethyl)-4-methylpentanoylamino)ethyl)disulfanyl)-2(R) -(3,3-dimethylbutanoylamino) propionic acid,
3-((2(R)-carbonyl-2(R)-(4-methyl-2(R,S)-(4-methoxyphenyl)pentanoylamino)ethyl)disulfanyl)-2(R)-(3,3-dimethylbutanoylamino) propionic acid,
3-((2(R)-((1 -acetyl-3(R,S)-(4-hydroxyphenyl)pyrrolidin-2(R,S)-yl)carbonylamino)-2(R)-carbonylethyl)disulfanyl)-2(R) -(3,3-dimethylbutanoylamino)propionic acid,
3-((2-carboxy-2-(3,3-dimethylbutanoylamino)ethyl)disulfanyl)-2-(4-methyl-2-(2-(4-((phenylamino)carbonylamino)phenyl)acetylamino)pentanoylamino)propionic acid,
3-((2(S)-carboxy-2(S)-((tert-butoxy)carbonylamino)ethyl)disulfanyl)-2(S)-(4-methyl-2(S)-(phenylmethoxy)pentanoylamino)propionic acid,
3-(4-(cyclohexylmethoxy)phenyl)-2(S)-(2(R,S)-(tert-butyl)-5-phenylpentanoylamino) propionic acid,
3-(4-(cyclohexylmethoxy)phenyl)-2(S)-(2(R,S)-cyclohexyl-5-phenylpentanoylamino) propionic acid,
3-(4-(cyclohexylmethoxy)phenyl)-2(S)-(3,3-dimethyl-2(S)-(2-phenylacetylamino)butanoylamino)propionic acid,
3-(4-(cyclohexylmethoxy)phenyl)-2(S)-((2-(2-phenylethyl)phenyl)carbonylamino) propionic acid,
3-(4-(cyclohexylmethoxy)phenyl)-2(S)-(3,3-dimethyl-2(S)-(phenylmethoxy)butanoylamino)propionic acid,
2(S)-(3,3-dimethyl-2(S)-(phenylmethoxy)butanoylamino)-3-(4-(2,2-dimethylpropoxy)phenyl)propionic acid,
2(S)-(4-methyl-2(S)-(phenylmethoxy)pentanoylamino)-3-(4-(2,2-dimethylpropoxy)phenyl)propionic acid,
2(S)-(2(S)-((2-naphthyl)methoxy)-3,3-dimethylbutanoylamino)-3-(4-(2-methylpropoxy) phenyl)propionic acid,
2(S)-(3,3-dimethyl-2(S)-(2-naphthylmethoxy)butanoylamino)-3-(4-(2-methylpropoxy) phenyl)propionic acid,
2(S)-(2(S)-((4-chlorophenyl)methoxy)-4-methylpentanoylamino)-3-(4-(2-methylpropoxy) phenyl)propionic acid,
2-(4-methyl-2-(2-(4-((phenylamino)carbonylamino)phenyl)acetylamino)pentanoylamino)-3-(4-(2-methylpropoxy) phenyl)propionic acid,
3-(4-(2-carboxyethyl)phenyl)-2-(2-(4-hydroxyphenyl)-4-methylpentanoylamino) propionic acid,
3-(4-(2(S)-carboxyethyl)phenyl)-2-(4-methyl-2(R,S)-(4-((phenylsulfonyl)amino)phenyl)pentanoylamino)propionic acid,
3-(4-(2(S)-carbonylethyl)phenyl)-2(S)-(4-methyl-2(R,S)-(4-(phenylcarbonylamino)phenyl) pentanoylamino)propionic acid, 3-(4-(2-(N-(1-(4-hydroxyphenyl)-3-methylbutyl)carbamoyl)ethyl)phenyl)-2-(3,3-dimethylbutanoylamino)propionic acid,
3-(4-(2-(N-(1-(4-hydroxyphenyl)-3-methylbutyl)carbamoyl)phenyl)-2-(2-(4-hydroxyphenyl)-4-methylpentanoylamino) propionic acid,
3-((2-carboxy-2-(4-methyl-2-(2-(4-((N-phenylcarbamoyl)amino)phenyl)acetylamino)pentanoylamino)ethyl)disulfanyl)-2-(3,3-dimethylbutanoylamino)propionic acid,
3-((2-carboxy-2-(4-methyl-2-(2-(4-((N-(4-methylphenyl)carbamoyl)amino)phenyl)acetylamino)pentanoylamino)ethyl) disulfan yl)-2-(3,3-dimethylbutanoylamino)propionic acid,
3-((2-carboxy-2-(4-methyl-2-(2-(4-((N-(4-nitrophenyl)carbamoyl)amino)phenyl)acetylamino)pentanoylamino)ethyl)disulfanyl) -2-(3,3-dimethylbutanoylamino)propionic acid,
3-((2-carboxy-2-(4-methyl-2-(2-(4-((N-(4-methoxylphenyl)carbamoyl)amino)phenyl)acetylamino)pentanoylamino) ethyl)disulf anyl)-2-(3,3-dimethylbutanoylamino)propionic acid,
3-((2-carboxy-2-(2-(2-(4-((N-(4-hydroxyphenyl)carbamoyl)amino)phenyl)acetylamino)-4-methylpentanoylamino)ethyl)disulfanyl)-2-(3,3-dimethylbutanoylamino)propionic acid,
3-((2-carboxy-2-(2-(2-(4-((N-(4-chlorophenyl)carbamoyl)amino)phenyl)acetylamino)-4-methylpentanoylamino)ethyl) disulfanyl)-2-(3,3-dimethylbutanoylamino)propionic acid,
3-((2-carboxy-2-(4-methyl-2-(2-(4-(((phenylamino)thioxomethyl)amino)phenyl)acetylamino)pentanoylamino)ethyl)-disulfanyl)-2-(3,3-dimethylbutanoylamino)propionic acid,
4-(2-carboxy-2-(4-methyl-2-(2-(4-((N-phenylcarbamoyl)amino)phenyl)acetylamino)pentanoylamino)ethylthio)-2-(3,3-dimethylbutanoylamino)butanoic acid,
4-(2-carboxy-2-(4-methyl-2-(2-(4-((N-(4-methylphenyl)carbamoyl)amino)phenyl)acetylamino)pentanoylamino)ethylthio)-2-(3,3-dimethylbutanoylamino)butanoic acid,
4-(2-carboxy-2-(4-methyl-2-(2-(4-((N-(4-nitrophenyl)carbamoyl)amino)phenyl)acetylamino)pentanoylamino)ethylthio)-2-(3,3-dimethylbutanoylamino)butanoic acid,
4-(2-carboxy-2-(4-methyl-2-(2-(4-((N-(4-methoxyphenyl)carbamoyl)amino)phenyl)acetylamino)pentanoylamino) ethylthio)-2-(3,3-dimethylbutanoylamino)butanoic acid,
4-(2-carboxy-2-(2-(2-(4-((N-(4-hydroxyphenyl)carbamoyl)amino)phenyl)acetylamino)-4-pentanoylamino)ethylthio)-2-(3,3-dimethylbutanoylamino)butanoic acid,
4-(2-carboxy-2-(2-(2-(4-((N-(4-chlorophenyl)carbamoyl)amino)phenyl)acetylamino)-4-pentanoylamino)ethylthio)-2-(-3,3-dimethylbutanoylamino)butanoic acid,
4-(2-carboxy-2-(4-methyl-2-(2-(4-(((phenylamino)thioxomethyl)amino)phenyl)acetylamino)pentanoylamino)ethylthio) -2-(3,3-dimethylbutanoylamino)butanoic acid,
1-(3,3-dimethylbutanoylamino)-6-(4-methyl-2-(2-(4-((N-phenylcarbamoyl)amino)phenyl)acetylamino)pentanoylamino)hexane-1,6-dicarboxylic acid,
1-(3,3-dimethylbutanoylamino)-6-(4-methyl-2-(2-(4-((N-(4-methylphenyl)carbamoyl)amino)phenyl)acetylamino)pentanoylamino)hexane-1,6-dicarboxylic acid,
1-(3,3-dimethylbutanoylamino)-6-(4-methyl-2-(2-(4-((N-(4-nitrophenyl)carbamoyl)amino)phenyl)acetylamino)pentanoylamino)hexane-1,6-dicarboxylic acid,
1-(3,3-dimethylbutanoylamino)-6-(4-methyl-2-(2-(4-((N-(4-methoxyphenyl)carbamoyl)amino)phenyl)acetylamino) pentanoylamino)hexane-1,6-dicarboxylic acid,
6-(2-(2-(4-((N-(4-hydroxyphenyl)carbamoyl)amino)phenyl)acetylamino)-4-methylpentanoylamino)-1-(3,3-dimethylbutanoylamino)hexane-1,6-dicarboxylic acid,
6-(2-(2-(4-((N-(4-chlorophenyl)carbamoyl)amino)phenyl)acetylamino)-4-methylpentanoylamino)-1-(3,3-dimethylbutanoylamino)hexane-1,6-dicarboxylic acid,
1-(3,3-dimethylbutanoylamino)-6-(4-methyl-2-(2-(4-(((phenylamino)thioxomethyl)amino)phenyl)acetylamino)pentanoylamino)hexane-1,6-dicarboxylic acid,
4-(2-carboxy-2-(3,3-dimethylbutanoylamino)ethoxy)-2-(4-methyl-2-(2-(4-((N-phenylcarbamoyl)amino)phenyl) acetylamino)pentanoylamino)butanoic acid,
4-(2-carboxy-2-(3,3-dimethylbutanoylamino)ethoxy)-2-(4-methyl-2-(2-(4-((N-(4-methylphenyl)carbamoyl)amino)phenyl)acetylamino)pentanoylamino)butanoic acid,
4-(2-carboxy-2-(3,3-dimethylbutanoylamino)ethoxy)-2-(4-methyl-2-(2-(4-((N-(4-nitrophenyl)carbamoyl)amino)phenyl) acetylamino)pentanoylamino)butanoic acid,
4-(2-carboxy-2-(3,3-dimethylbutanoylamino)ethoxy)-2-(4-methyl-2-(2-(4-((N-(4-methoxyphenyl)carbamoyl)amino) phenyl)acetylamino)pentanoylamino)butanoic acid,
4-(2-carboxy)-2-(3,3-dimethylbutanoylamino)ethoxy)-2-(2-(2-(4-((N-(4-hydroxyphenyl)carbamoyl)amino)phenyl) acetylamino)-4-methylpentanoylamino) butanoic acid,
4-(2-carboxy)-2-(3,3-dimethylbutanoylamino)ethoxy)-2-(2-(2-(4-((N-(4-chlorophenyl)carbamoyl)amino)phenyl) acetylamino)-4-methylpentanoylamino) butanoic acid,
4-(2-carboxy-2-(3,3-dimethylbutanoylamino)ethoxy)-2-(4-methyl-2-(2-(4-(((phenylamino)thioxomethyl)amino)phenyl) acetylamino)pentanoylamino)butanoic acid,
4-((2-carboxy-2-(3,3-dimethylbutanoylamino)ethyl)amino)-2-(4-methyl-2-(2-(4-((N-phenylcarbamoyl)amino)phenyl) acetylamino)pentanoylamino)butanoic acid,
4-((2-carboxy-2-(3,3-dimethylbutanoylamino)ethyl)amino)-2-(4-methyl-2-(2-(4-((N-(4-methylphenyl)carbamoyl)amino)phenyl)acetylamino)pentanoylamino)butanoic acid,
4-((2-carboxy-2-(3,3-dimethylbutanoylamino)ethyl)amino)-2-(4-methyl-2-(2-(4-((N-(4-nitrophenyl)carbamoyl)amino) phenyl)acetylamino)pentanoylamino)butanoic acid,
4-((2-carboxy-2-(3,3-dimethylbutanoylamino)ethyl)amino)-2-(4-methyl-2-(2-(4-((N-(4-methoxyphenyl)carbamoyl)amino)phenyl)acetylamino)pentanoylamino)butanoic acid,
4-((2-carboxy-2-(3,3-dimethylbutanoylamino)ethyl)amino)-2-(2-(2-(4-((N-(4-hydroxyphenyl)carbamoyl)amino)phenyl) acetylamino)-4-methylpentanoylamino) butanoic acid,
4-((2-carboxy-2-(3,3-dimethylbutanoylamino)ethyl)amino)-2-(2-(2-(4-((N-(4-chlorophenyl)carbamoyl)amino)phenyl)acetylamino)-4-methylpentanoylamino) butanoic acid,
4-((2-carboxy-2-(3,3-dimethylbutanoylamino)ethyl)amino)-2-(4-methyl-2-(2-(4-(((phenylamino)thioxomethyl)amino)phenyl)acetylamino)pentanoylamino)butanoic acid,
6-(2-(2-(4-((2-aza-2-cyano-1-(phenylamino)vinyl)amino)phenyl)acetylamino)-4-methylpentanoylamino)-1-(3,3-dimethylbutanoylamino)hexane-1,6-dicarboxylic acid, 6-(2-(2-(4-((2-aza-2-cyano-1-((4-methylphenyl)amino)vinyl)amino)phenyl)acetylamino)-4-methylpentanoylamino)-1-(3,3-dimethylbutanoylamino)hexane-1,6-dicarboxylic acid,
6-(2-(2-(4-((2-aza-2-cyano-1-((4-nitrophenyl)amino)vinyl)amino)phenyl)acetylamino)-4-methylpentanoylamino)-1-(3,3-dimethylbutanoylamino)hexane-1,6-dicarboxylic acid,
6-(2-(2-(4-((2-aza-2-cyano-1-((4-methoxyphenyl)amino)vinyl)amino)phenyl)acetylamino)-4-methylpentanoylamino)-1-(3,3-dimethylbutanoylamino)hexane-1,6-dicarboxylic acid,
6-(2-(2-(4-((2-aza-2-cyano-1-((4-hydroxyphenyl)amino)vinyl)amino)phenyl)acetylamino)-4-methylpentanoylamino)-1-(3,3-dimethylbutanoylamino)hexane-1,6-dicarboxylic acid,
6-(2-(2-(4-((2-aza-2-cyano-1-((4-chlorophenyl)amino)vinyl)amino)phenyl)acetylamino)-4-methylpentanoylamino)-1-(3,3-dimethylbutanoylamino)hexane-1,6-dicarboxylic acid,
4-((2-carboxy-2-(4-methyl-2-(2-(4-((2-nitro-1-(phenylamino)vinyl)amino)phenyl)acetylamino)pentanoylamino)ethyl)amino)-2-(3,3-dimethylbutanoylamino)butanoic acid,
4-((2-carboxy-2-(4-methyl-2-(2-(4-((1-((4-methylphenyl)amino)-2-nitrovinyl)amino)phenyl)acetylamino)pentanoylamino)ethyl)amino)-2-(3,3-dimethylbutanoylamino)butanoic acid,
4-((2-carboxy-2-(4-methyl-2-(2-(4-((2-nitro-1-((4-nitrophenyl)amino)vinyl)amino)phenyl)acetylamino)pentanoylamino)ethyl)amino)-2-(3,3-dimethylbutanoylamino)butanoic acid,
4-((2-carboxy-2-(4-methyl-2-(2-(4-((1-((4-methoxyphenyl)amino)-2-nitrovinyl)amino)phenyl)acetylamino)pentanoylamino)ethyl)amino)-2-(3,3-dimethylbutanoylamino)butanoic acid,
4-((2-carboxy-2-(2-(2-(4-((1-((4-hydroxyphenyl)amino)-2-nitrovinyl)amino)phenyl)acetylamino)-4-methylpentanoylamino)ethyl)amino)-2-(3,3-dimethylbutanoylamino)butanoic acid,
4-((2-carboxy-2-(2-(2-(4-((1-((4-chlorophenyl)amino)-2-nitrovinyl)amino)phenyl)acetylamino)-4-methylpentanoylamino)ethyl)amino)-2-(3,3-dimethylbutanoylamino)butanoic acid,
1 -(3,3-dimethylbutanoylamino)-6-(4-methyl-2-(2-(4-((2-nitro-1-(phenylamino)vinyl)amino)phenyl)acetylamino)pentanoylamino)hexane-1,6-dicarboxylic acid,
1-(3,3-dimethylbutanoylamino)-6-(4-methyl-2-(2-(4-((1-((4-methylphenyl)amino)-2-nitrovinyl)amino)phenyl)acetylamino)pentanoylamino)hexane-1,6-dicarboxylic acid,
1-(3,3-dimethylbutanoylamino)-6-(4-methyl-2-(2-(4-((2-nitro-1-((4-nitrophenyl)amino)vinyl)amino)phenyl)acetylamino)pentanoylamino)hexane-1,6-dicarboxylic acid,
1-(3,3-dimethylbutanoylamino)-6-(4-methyl-2-(2-(4-((1-((4-methoxyphenyl)amino)-2-nitrovinyl)amino)phenyl)acetylamino)pentanoylamino)hexane-1,6-dicarboxylic acid,
6-(2-(2-(4-((1-((4-hydroxyphenyl)amino)-2-nitrovinyl)amino)phenyl)acetylamino)-4-methylpentanoylamino)-1-(3,3-dimethylbutanoylamino)hexane-1,6-dicarboxylic acid,
6-(2-(2-(4-((1-((4-chlorophenyl)amino)-2-nitrovinyl)amino)phenyl)acetylamino)-4-methylpentanoylamino)-1-(3,3-dimethylbutanoylamino)hexane-1,6-dicarboxylic acid,
4-((2-carboxy-2-(4-methyl-2-(2-(4-((N-phenyicarbamoyi)amino)phenyl)acetylamino)pentanoylamino)ethoxy)-2-(3,3-dimethylbutanoylamino)butanoic acid,
4-((2-carboxy-2-(4-methyl-2-(2-(4-((N-(4-methylphenyl)carbamoyl)amino)phenyl)acetylamino)pentanoylamino)ethoxy)-2-(3,3-dimethylbutanoylamino)butanoic acid,
4-((2-carboxy-2-(4-methyl-2-(2-(4-((N-(4-nitrophenyl)carbamoyl)amino)phenyl)acetylamino)pentanoylamino)ethoxy)-2-(3,3-dimethylbutanoylamino)butanoic acid,
4-((2-carboxy-2-(4-methyl-2-(2-(4-((N-(4-methoxyphenyl)carbamoyl)amino)phenyl)acetylamino)pentanoylamino)ethoxy)-2-(3,3-dimethylbutanoylamino)butanoic acid,
4-((2-carboxy-2-(2-(2-(4-((N-(4-hydroxyphenyl)carbamoyl)amino)phenyl)acetylamino)-4-methylpentanoylamino)ethoxy)-2-(3,3-dimethylbutanoylamino)butanoic acid,
4-((2-carboxy-2-(2-(2-(4-((N-(4-chlorophenyl)carbamoyl)amino)phenyl)acetylamino)-4-methylpentanoylamino)ethoxy)-2-(3,3-dimethylbutanoylamino)butanoic acid,
4-((2-carboxy-2-(4-methyl-2-(2-(4-(((phenylamino)thioxomethyl)amino)phenyl)acetylamino)pentanoylamino)ethoxy)-2-(3,3-dimethylbutanoylamino)butanoic acid,
4-((2-carboxy-2-(4-methyl-2-(2-(4-((N-phenylcarbamoyl)amino)phenyl)acetylamino)pentanoylamino)ethyl)amino)-2-(3,3-dimethylbutanoylamino)butanoic acid,
4-((2-carboxy-2-(4-methyl-2-(2-(4-((N-(4-methylphenyl)carbamoyl)amino)phenyl)acetylamino)pentanoylamino)ethyl)amino)-2-(3,3-dimethylbutanoylamino)butanoic acid,
4-((2-carboxy-2-(4-methyl-2-(2-(4-((N-(4-nitrophenyl)carbamoyl)amino)phenyl)acetylamino)pentanoylamino)ethyl)amino)-2-(3,3-dimethylbutanoylamino)butanoic acid,

4-((2-carboxy-2-(4-methyl-2-(2-(4-((N-(4-methoxyphenyl)carbamoyl)amino)phenyl)acetylamino)pentanoylamino)ethyl)amino) -2-(3,3-dimethylbutanoylamino)butanoic acid,
4-((2-carboxy-2-(2-(2-(4-((N-(4-hydroxyphenyl)carbamoyl)amino)phenyl)acetylamino)-4-methylpentanoylamino)ethyl)amino)-2-(3,3-dimethylbutanoylamino)butanoic acid,
4-((2-carboxy-2-(2-(2-(4-((N-(4-chlorophenyl)carbamoyl)amino)phenyl)acetylamino)-4-methylpentanoylamino)ethyl)amino)-2-(3,3-dimethylbutanoylamino)butanoic acid,
4-((2-carboxy-2-(4-methyl-2-(2-(4-(((phenylamino)thioxomethyl)amino)phenyl)acetylamino)pentanoylamino)ethyl)-amino)-2-(3,3-dimethylbutanoylamino)butanoic acid,
4-(2-carboxy-2-(3,3-dimethylbutanoylamino)ethylthio)-2-(4-methyl-2-(2-(4-((N-phenylcarbamoyl)amino)phenyl)acetylamino)pentanoylamino)butanoic acid,
4-(2-carboxy-2-(3,3-dimethylbutanoylamino)ethylthio)-2-(4-methyl-2-(2-(4-((N-(4-methylphenyl)carbamoyl)amino)phenyl)acetylamino)pentanoylamino)butanoic acid,
4-(2-carboxy-2-(3,3-dimethylbutanoylamino)ethylthio)-2-(4-methyl-2-(2-(4-((N-(4-nitrophenyl)carbamoyl)amino)phenyl)acetylamino)pentanoylamino)butanoic acid,
4-(2-carboxy-2-(3,3-dimethylbutanoylamino)ethylthio)-2-(4-methyl-2-(2-(4-((N-(4-methoxyphenyl)carbamoyl)amino)phenyl)acetylamino)pentanoylamino)butanoic acid,
4-(2-carboxy-2-(3,3-dimethylbutanoylamino)ethylthio)-2-(2-(2-(4-((N-(4-hydroxyphenyl)carbamoyl)amino)phenyl)acetylamino)-4-methylpentanoylamino) butanoic acid,
4-(2-carboxy-2-(3,3-dimethylbutanoylammo)ethylthio)-2-(2-(2-(4-((N-(4-chlorphenyl)carbamoyl)amino)phenyl)acetylamino)-4-methylpentanoylamino) butanoic acid,
4-(2-carboxy-2-(3,3-dimethylbutanoylamino)ethylthio)-2-(4-methyl-2-(2-(4-(((phenylamino)thioxomethyl)amino)phenyl)acetylamino)pentanoylamino)butanoic acid,
2-(2-(2-(4-((2-aza-2-cyano-1-(phenylamino)vinyl)amino)phenyl)acetylamino)-4-methylpentanoylamino)-4-((2-carboxy-2-(3,3-dimethylbutanoylamino)ethyl)amino) butanoic acid,
2-(2-(2-(4-((2-aza-2-cyano-1-((4-methylphenyl)amino)vinyl)amino)phenyl)acetylamino)-4-methylpentanoylamino)-4-((2-carboxy-2-(3,3-dimethylbutanoylamino)ethyl)amino)butanoic acid,
2-(2-(2-(4-((2-aza-2-cyano-1-((4-nitrophenyl)amino)vinyl)amino)phenyl)acetylamino)-4-methylpentanoylamino)-4-((2-carboxy-2-(3,3-dimethylbutanoylamino)ethyl)amino)butanoic acid,
2-(2-(2-(4-((2-aza-2-cyano-1-((4-methoxyphenyl)amino)vinyl)amino)phenyl)acetylamino)-4-methylpentanoylamino)-4-((2-carboxy-2-(3,3-dimethylbutanoylamino)ethyl)amino)butanoic acid,
2-(2-(2-(4-((2-aza-2-cyano-1-((4-hydroxyphenyl)amino)vinyl)amino)phenyl)acetylamino)-4-methylpentanoylamino)-4-((2-carboxy-2-(3,3-dimethylbutanoylamino)ethyl)amino)butanoic acid,
2-(2-(2-(4-((2-aza-2-cyano-1-((4-chlorophenyl)amino)vinyl)amino)phenyl)acetylamino)-4-methylpentanoylamino)-4-((2-carboxy-2-(3,3-dimethylbutanoylamino)ethyl)amino)butanoic acid,
4-((2-carboxy-2-(3,3-dimethylbutanoylamino)ethyl)amino)-2-(4-methyl-2-(2-(4-((2-nitro-1-(phenylamino)vinyl)amino)phenyl)acetylamino)pentanoylamino)butanoic acid,
4-((2-carboxy-2-(3,3-dimethylbutanoylamino)ethyl)amino)-2-(4-methyl-2-(2-(4-((1-((4-methylphenyl)amino)-2-nitrovinyl)amino)phenyl)acetylamino)pentanoylamino) butanoic acid,
4-((2-carboxy-2-(3,3-dimethylbutanoylamino)ethyl)amino)-2-(4-methyl-2-(2-(4-((2-nitro-1-((4-nitrophenyl)amino)vinyl)amino)phenyl)acetylamino)pentanoylamino) butanoic acid,
4-((2-carboxy-2-(3,3-dimethylbutanoylamino)ethyl)amino)-2-(4-methyl-2-(2-(4-((1-((4-methoxyphenyl)amino)-2-nitrovinyl)amino)phenyl)acetylamino)pentanoylamino) butanoic acid,
4-((2-carboxy-2-(3,3-dimethylbutanoylamino)ethyl)amino)-2-(2-(2-(4-((1-((4-hydroxyphenyl)amino)-2-nitrovinyl)amino)phenyl)acetylamino)-4-methylpentanoylamino)butanoic acid,
4-((2-carboxy-2-(3,3-dimethylbutanoylamino)ethyl)amino)-2-(2-(2-(4-((1-((4-chlorophenyl)amino)-2-nitrovinyl)amino)phenyl)acetylamino)-4-methylpentanoylamino)butanoic acid,
4-(2-carboxy-2-(3,3-dimethylbutanoylamino)ethoxy)-2-(4-methyl-2-(2-(4-((2-nitro-1-(phenylamino)vinyl)amino)phenyl)acetylamino)pentanoylamino)butanoic acid, 4-(2-carboxy-2-(3,3-dimethylbutanoylamino)ethoxy)-2-(4-methyl-2-(2-(4-((1-((4-methylphenyl)amino)-2-nitrovinyl)amino)phenyl)acetylamino)pentanoylamino) butanoic acid,
4-(2-carboxy-2-(3,3-dimethylbutanoylamino)ethoxy)-2-(4-methyl-2-(2-(4-((2-nitro-1-((4-nitrophenyl)amino)vinyl)amino)phenyl)acetylamino)pentanoylamino)butanoic acid,
4-(2-carboxy-2-(3,3-dimethylbutanoylamino)ethoxy)-2-(4-methyl-2-(2-(4-((1-((4-methoxyphenyl)amino)-2-nitrovinyl)amino)phenyl)acetylamino)pentanoylamino) butanoic acid,
4-(2-carboxy-2-(3,3-dimethylbutanoylamino)ethoxy)-2-(2-(2-(4-((1-(4-hydroxyphenyl)amino)-2-nitrovinyl)amino)phenyl)acetylamino)-4-methylpentanoylamino)butanoic acid,
4-(2-carboxy-2-(3,3-dimethylbutanoylamino)ethoxy)-2-(2-(2-(4-((1-(4-chlorophenyl)amino)-2-nitrovinyl)amino)phenyl)acetylamino)-4-methylpentanoylamino)butanoic acid,
1-(2-(2-(4-((2-aza-2-cyano-1-(phenylamino)vinyl)amino)phenyl)acetylamino)-4-methylpentanoylamino)-6-(3,3-dimethylbutanoylamino)hex-3-ene-1,6-dicarboxylic acid,
1-(2-(2-(4-((2-aza-2-cyano-1-((4-methylphenyl)amino)vinyl)amino)phenyl)acetylamino)-4-methylpentanoylamino)-6-(3,3-dimethylbutanoylamino)hex-3-ene-1,6-dicarboxylic acid,
1-(2-(2-(4-{(2-aza-2-cyano-1-((4-nitrophenyl)amino)vinyl)amino)phenyl)acetylamino)-4-methylpentanoylamino)-6-(3,3-dimethylbutanoylamino)hex-3-ene-1,6-dicarboxylic acid,
1-(2-(2-(4-((2-aza-2-cyano-1-((4-methoxyphenyl)amino)vinyl)amino)phenyl)acetylamino)-4-methylpentanoylamino)-6-(3,3-dimethylbutanoylarnino)hex-3-ene-1,6-dicarboxylic acid,
1-(2-(2-(4-((2-aza-2-cyano-1-((4-hydroxyphenyl)amino)vinyl)amino)phenyl)acetylamino)-4-methylpentanoylamino)-6-(3,3-dimethylbutanoylamino)hex-3-ene-1,6-dicarboxylic acid,
1-(2-(2-(4-((2-aza-2-cyano-1-((4-chlorophenyl)amino)vinyl)amino)phenyl)acetylamino)-4-methylpentanoylamino)-6-(3,3-dimethylbutanoylamino)hex-3-ene-1,6-dicarboxylic acid,
1-(3,3-dimethylbutanoylamino)-6-(4-methyl-2-(2-(4-((2-nitro-1-(phenylamino)vinyl)amino)phenyl)acetylamino)pentanoylamino)hex-3-ene-1,6-dicarboxylic acid,
1-(3,3-dimethylbutanoylamino)-6-(4-methyl-2-(2-(4-((1-((4-methylphenyl)amino)-2-nitrovinyl)amino)phenyl)acetylamino)pentanoylamino)hex-3-ene-1,6-dicarboxylic acid,
1-(3,3-dimethylbutanoylamino)-6-(4-methyl-2-(2-(4-((2-nitro-1-((4-nitrophenyl)amino)vinyl)amino)phenyl)acetylamino)pentanoylamino)hex-3-ene-1,6-dicarboxylic acid,
1-(3,3-dimethylbutanoylamino)-6-(4-methyl-2-(2-(4-((1-((4-methoxyphenyl)amino)-2-nitrovinyl)amino)phenyl) acetylamino)pentanoylamino)hex-3-ene-1,6-dicarboxylic acid,
6-(2-(2-(4-((1-((4-hydroxyphenyl)amino)-2-nitrovinyl)amino)phenyl)acetylamino)-4-methylpentanoylamino)-1-(3,3-dimethylbutanoylamino)hex-3-ene-1,6-dicarboxylic acid,
6-(2-(2-(4-((1-((4-chlorophenyl)amino)-2-nitrovinyl)amino)phenyl)acetylamino)-4-methylpentanoylamino)-1-(3,3-dimethylbutanoylamino)hex-3-ene-1,6-dicarboxylic acid,
6-(3,3-dimethylbutanoylamino)-1-(4-methyl-2-(2-(4-((N-phenylcarbamoyl)amino)phenyl)acetylamino)pentanoylamino)hex-3-ene-1,6-dicarboxylic acid,
6-(3,3-dimethylbutanoylamino)-1-(4-methyl-2-(2-(4-((N-(4-methylphenyl)carbamoyl)amino)phenyl)acetylamino)pentanoylamino)hex-3-ene-1,6-dicarboxylic acid,
6-(3,3-dimethylbutanoylamino)-1-(4-methyl-2-(2-(4-((N-(4-nitrophenyl)carbamoyl)amino)phenyl)acetylamino)pentanoylamino)hex-3-ene-1,6-dicarboxylic acid,
6-(3,3-dimethylbutanoylamino)-1-(4-methyl-2-(2-(4-((N-(4-methoxyphenyl)carbamoyl)amino)phenyl)acetylamino) pentanoylamino)hex-3-ene-1,6-dicarboxylic acid,
1-(2-(2-(4-((N-(4-hydroxyphenyl)carbamoyl)amino)phenyl)acetylamino)-4-methylpentanoylamino)-6-(3,3-dimethylbutanoylamino)hex-3-ene-1,6-dicarboxylic acid,
1-(2-(2-(4-((N-(4-chlorophenyl)carbamoyl)amino)phenyl)acetylamino)-4-methylpentanoylamino)-6-(3,3-dimethylbutanoylamino)hex-3-ene-1,6-dicarboxylic acid,
6-(3,3-dimethylbutanoylamino)-1-(4-methyl-2-(2-(4-(((phenylamino)thioxomethyl)amino)phenyl)acetylamino)pentanoylamino)hex-3-ene-1,6-dicarboxylic acid,
3-(2-(2-(2-(2-(4-((2-aza-2-cyano-1-(phenylamino)vinyl)amino)phenyl)acetylamino)-4-methylpentanoylamino)-2-carboxyethyl)phenyl)-2-(3,3-dimethylbutanoylamino) propanoic acid,
3-(2-(2-(2-(2-(4-((2-aza-2-cyano-1-((4-methylphenyl)amino)vinyl)amino)phenyl)acetylamino)-4-methylpentanoylamino)-2-carboxyethyl)phenyl)-2-(3,3 -dimethylbutanoylamino)propanoic acid,
3-(2-(2-(2-(2-(4-((2-aza-2-cyano-1-((4-nitrophenyl)amino)vinyl)amino)phenyl)acetylamino)-4-methylpentanoylamino)-2-carboxyethyl)phenyl)-2-(3,3-dimethylbutanoylamino)propanoic acid,
3-(2-(2-(2-(2-(4-((2-aza-2-cyano-1-((4-methoxyphenyl)amino)vinyl)amino)phenyl)acetylamino)-4-methylpentanoylamino)-2-carboxyethyl)phenyl)-2-(3,3-dimethylbutanoylamino)propanoic acid,
3-(2-(2-(2-(2-(4-((2-aza-2-cyano-1-((4-hydroxyphenyl)amino)vinyl)amino)phenyl)acetylamino)-4-methylpentanoylamino)-2-carboxyethyl)phenyl)-2-(3,3-dimethylbutanoylamino)propanoic acid,
3-(2-(2-(2-(2-(4-((2-aza-2-cyano-1-((4-chlorophenyl)amino)vinyl)amino)phenyl)acetylamino)-4-methylpentanoylamino)-2-carboxyethyl)phenyl)-2-(3,3-dimethylbutanoylamino)propanoic acid,
3-(2-(2-carboxy-2-(4-methyl-2-(2-(4-((2-nitro-1-(phenylamino)vinyl)amino)phenyl)acetylamino)pentanoylamino)ethyl) phenyl)-2-(3,3-dimethylbutanoylarnino)propanoic acid,
3-(2-(2-carboxy-2-(4-methyl-2-(2-(4-((1-((4-methylphenyl)amino)-2-nitrovinyl)amino)phenyl)acetylamino)pentanoylamino)ethyl)phenyl)-2-(3,3-dimethylbutanoylamino)propanoic acid,
3-(2-(2-carboxy-2-(4-methyl-2-(2-(4-((2-nitro-1-((4-nitrophenyl)amino)vinyl)amino)phenyl)acetylamino)pentanoylamino)ethyl)phenyl)-2-(3,3-dimethylbutanoylamino)propanoic acid,
3-(2-(2-carboxy-2-(4-methyl-2-(2-(4-((1-((4-methoxyphenyl)amino)-2-nitrovinyl)amino)phenyl)acetylamino)pentanoylamino)ethyl)phenyl)-2-(3,3-dimethylbutanoylamino)propanoic acid,
3-(2-(2-carboxy-2-(2-(2-(4-((1-((4-hydroxyphenyl)amino)-2-nitrovinyl)amino)phenyl)acetylamino)-4-methylpentanoylamino)ethyl)phenyl)-2-(3,3-dimethylbutanoylamino)propanoic acid,
3-(2-(2-carboxy-2-(2-(2-(4-((1-((4-chlorophenyl)amino)-2-nitrovinyl)amino)phenyl)acetylamino)-4-methylpentanoylamino)ethyl)phenyl)-2-(3,3-dimethylbutanoylamino)propanoic acid,
3-(2-(2-carboxy-2-(4-methyl-2-(2-(4-((N-phenylcarbamoyl)amino)phenyl)acetylamino)pentanoylamino)ethyl)phenyl)-2-(3,3-dimethylbutanoylamino)propanoic acid,
3-(2-(2-carboxy-2-(4-methyl-2-(2-(4-((N-(4-methylphenyl)carbamoyl)amino)phenyl)acetylamino)pentanoylamino) ethyl)phenyl)-2-(3,3-dimethylbutanoylamino)propanoic acid,
3-(2-(2-carboxy-2-(4-methyl-2-(2-(4-((N-(4-nitrophenyl)carbamoyl)amino)phenyl)acetylamino)pentanoylamino)ethyl) phenyl)-2-(3,3-dimethylbutanoylamino)propanoic acid,
3-(2-(2-carboxy-2-(4-methyl-2-(2-(4-((N-(4-methoxyphenyl)carbamoyl)amino)phenyl)acetylamino)pentanoylamino) ethyl)-phenyl)-2-(3,3-dimethylbutanoylamino)propanoic acid,
3-(2-(2-carboxy-2-(2-(2-(4-((N-(4-hydroxyphenyl)carbamoyl)amino)phenyl)acetylamino)-4-methylpentanoylamino) ethyl)phenyl)-2-(3,3-dimethylbutanoylamino)propanoic acid,
3-(2-(2-carboxy-2-(2-(2-(4-((N-(4-chlorophenyl)carbamoyl)amino)phenyl)acetylamino)-4-methylpentanoylamino) ethyl)phenyl)-2-(3,3-dimethylbutanoylamino)propanoic acid,
3-(2-(2-carboxy-2-(4-methyl-2-(2-(4-(((phenylamino)thioxomethyl)amino)phenyl)acetylamino)pentanoylamino)ethyl)-phenyl)-2-(3,3-dimethylbutanoylamino)propanoic acid,
4-(2-carboxy-2-(4-methyl-2-(4-((N-phenylcarbamoyl)amino)phenyl)pentanoylamino)ethylthio)-2-(3,3-dimethylbutanoylamino)butanoic acid,
4-(2-carboxy-2-(4-methyl-2-(4-((N-(4-methylphenyl)carbamoyl)amino)phenyl)pentanoylamino)ethylthio)-2-(3,3-dimethylbutanoylamino)butanoic acid,
4-(2-carboxy-2-(4-methyl-2-(4-((N-(4-nitrophenyl)carbamoyl)amino)phenyl)pentanoylamino)ethylthio)-2-(3,3-dimethylbutanoylamino)butanoic acid,
4-(2-carboxy-2-(4-methyl-2-(4-((N-(4-methoxyphenyl)carbamoyl)amino)phenyl)pentanoylamino)ethylthio)-2-(3,3-dimethylbutanoylamino)butanoic acid,
4-(2-carboxy-2-(2-(4-((N-(4-hydroxyphenyl)carbamoyl)amino)phenyl)-4-methylpentanoylamino)ethylthio)-2-(3,3-dimethylbutanoylamino)butanoic acid,
4-(2-carboxy-2-(2-(4-((N-(4-nitrophenyl)carbamoyl)amino)phenyl)-4-methylpentanoylamino)ethylthio)-2-(3,3-dimethylbutanoylamino)butanoic acid,
1-(3,3 -dimethylbutanoylamino)-6-(4-methyl-2-(4-((N-phenylcarbamoyl)amino)phenyl)pentanoylamino)hexane-1,6-dicarboxylic acid,
1-(3,3-dimethylbutanoylamino)-6-(4-methyl-2-(4-((N-(4-methylphenyl)carbamoyl)amino)phenyl)pentanoylamino)hexane-1,6-dicarboxylic acid,
1-(3,3-dimethylbutanoylamino)-6-(4-methyl-2-(4-((N-(4-nitrophenyl)carbamoyl)amino)phenyl)pentanoylamino)hexane-1,6-dicarboxylic acid,
1-(3,3-dimethylbutanoylamino)-6-(4-methyl-2-(4-((N-(4-methoxyphenyl)carbamoyl)amino)phenyl)pentanoylamino) hexane-1,6-dicarboxylic acid,
1-(3,3-dimethylbutanoylamino)-6-(2-(4-((N-(4-hydroxyphenyl)carbamoyl)amino)phenyl)-4-methylpentanoylamino) hexane-1,6-dicarboxylic acid,
1-(3,3-dimethylbutanoylamino)-6-(2-(4-((N-(4-chlorophenyl)carbamoyl)amino)phenyl)-4-methylpentanoylamino)hexane-1,6-dicarboxylic acid,
1-(3,3-dimethylbutanoylamino)-6-(4-methyl-2-(4-(((phenylamino)thioxomethyl)amino)phenyl)pentanoylamino)hexane-1,6-dicarboxylic acid,
4-(2-carboxy-2-(3,3-dimethylbutanoylamino)ethylthio)-2-(4-methyl-2-(4-((N-phenylcarbamoyl)amino)phenyl)pentanoylamino)butanoic acid,
4-(2-carboxy-2-(3,3-dimethylbutanoylamino)ethylthio)-2-(4-methyl-2-(4-((N-(4-methylphenyl)carbamoyl)amino)phenyl)pentanoylamino)butanoic acid,
4-(2-carboxy-2-(3,3-dimethylbutanoylamino)ethylthio)-2-(4-methyl-2-(4-((N-(4-nitrophenyl)carbamoyl)amino)phenyl) pentanoylamino)butanoic acid,
4-(2-carboxy-2-(3,3-dimethylbutanoylamino)ethylthio)-2-(4-methyl-2-(4-((N-(4-methoxyphenyl)carbamoyl)amino)phenyl)pentanoylamino)butanoic acid,
4-(2-carboxy-2-(3,3-dimethylbutanoylamino)ethylthio)-2-(2-(4-((N-(4-hydroxyphenyl)carbamoyl)amino)phenyl)-4-methylpentanoylamino)butanoic acid,
4-(2-carboxy-2-(3,3-dimethylbutanoylamino)ethylthio)-2-(2-(4-((N-(4-chlorophenyl)carbamoyl)amino)phenyl)-4-methylpentanoylamino)butanoic acid,
4-(2-carboxy-2-(3,3-dimethylbutanoylamino)ethylthio)-2-(4-methyl-2-(4-(((phenylamino)thioxomethyl)amino)phenyl) pentanoylamino)butanoic acid,
4-(2-(2-(4-((2-aza-2-cyano-1-(phenylamino)vinyl)amino)phenyl)-4-methylpentanoylamino)-2-carboxyethoxy)-2-(3,3-dimethylbutanoylamino)butanoic acid,
4-(2-(2-(4-((2-aza-2-cyano-1-((4-methylphenyl)amino)vinyl)amino)phenyl)-4-methylpentanoylamino)-2-carboxyethoxy)-2-(3,3-dimethylbutanoylamino)butanoic acid,
4-(2-(2-(4-((2-aza-2-cyano-1-((4-nitrophenyl)amino)vinyl)amino)phenyl)-4-methylpentanoylamino)-2-carboxyethoxy)-2-(3,3-dimethylbutanoylamino)butanoic acid,
4-(2-(2-(4-((2-aza-2-cyano-1-((4-methoxyphenyl)amino)vinyl)amino)phenyl)-4-methylpentanoylamino)-2-carboxyethoxy)-2-(3,3-dimethylbutanoylamino)butanoic acid,
4-(2-(2-(4-((2-aza-2-cyano-1-((4-hydroxyphenyl)amino)vinyl)amino)phenyl)-4-methylpentanoylamino)-2-carboxyethoxy)-2-(3,3-dimethylbutanoylamino)butanoic acid,
4-(2-(2-(4-((2-aza-2-cyano-1-((4-chlorophenyl)amino)vinyl)amino)phenyl)-4-methylpentanoylamino)-2-carboxyethoxy)-2-(3,3-dimethylbutanoylamino)butanoic acid,
2-(2-(4-((2-aza-2-cyano-1-(phenylamino)vinyl)amino)phenyl)-4-methylpentanoylamino)-4-((2-carboxy-2-(3,3-dimethylbutanoylamino)ethyl)amino) butanoic acid,
2-(2-(4-((2-aza-2-cyano-1-((4-methylphenyl)amino)vinyl)amino)phenyl)-4-methylpentanoylamino)-4-((2-carboxy-2-(3,3-dimethylbutanoylamino)ethyl)amino) butanoic acid,
2-(2-(4-((2-aza-2-cyano-1-((4-nitrophenyl)amino)vinyl)amino)phenyl)-4-methylpentanoylamino)-4-((2-carboxy-2-(3,3-dimethylbutanoylamino)ethyl)amino) butanoic acid,
2-(2-(4-((2-aza-2-cyano-1-((4-methoxyphenyl)amino)vinyl)amino)phenyl)-4-methylpentanoylamino)-4-((2-carboxy-2-(3,3-dimethylbutanoylamino)ethyl)amino) butanoic acid,
2-(2-(4-((2-aza-2-cyano-1-((4-hydroxyphenyl)amino)vinyl)amino)phenyl)-4-methylpentanoylamino)-4-((2-carboxy-2-(3,3-dimethylbutanoylamino)ethyl)amino) butanoic acid,
2-(2-(4-((2-aza-2-cyano-1-((4-chlorophenyl)amino)vinyl)amino)phenyl)-4-methylpentanoylamino)-4-((2-carboxy-2-(3,3-dimethylbutanoylamino)ethyl)amino) butanoic acid,
2-(2-(4-((2-aza-2-cyano-1-(phenylamino)vinyl)amino)phenyl)-4-methylpentanoylamino)-4-(2-carboxy-2-(3,3-dimethylbutanoylamino)ethoxy) butanoic acid,
2-(2-(4-((2-aza-2-cyano-1-((4-methylphenyl)amino)vinyl)amino)phenyl)-4-methylpentanoylamino)-4-(2-carboxy-2-(3,3-dimethylbutanoylamino)ethoxy) butanoic acid,
2-(2-(4-((2-aza-2-cyano-1-((4-nitrophenyl)amino)vinyl)amino)phenyl)-4-methylpentanoylamino)-4-(2-carboxy-2-(3,3-dimethylbutanoylamino)ethoxy) butanoic acid,
2-(2-(4-((2-aza-2-cyano-1-((4-methoxyphenyl)amino)vinyl)amino)phenyl)-4-methylpentanoylamino)-4-(2-carboxy-2-(3,3-dimethylbutanoylamino)ethoxy) butanoic acid,
2-(2-(4-((2-aza-2-cyano-1-((4-hydroxyphenyl)amino)vinyl)amino)phenyl)-4-methylpentanoylamino)-4-(2-carboxy-2-(3,3-dimethylbutanoylamino)ethoxy) butanoic acid,
2-(2-(4-((2-aza-2-cyano-1-((4-chlorophenyl)amino)vinyl)amino)phenyl)-4-methylpentanoylamino)-4-(2-carboxy-2-(3,3-dimethylbutanoylamino)ethoxy) butanoic acid,
4-((2-carboxy-2-(4-methyl-2-(4-((N-phenylcarbamoyl)amino)phenyl)pentanoylamino)ethyl)amino)-2-(3,3-dimethylbutanoylamino) butanoic acid,
4-((2-carboxy-2-(4-methyl-2-(4-((N-(4-methylphenyl)carbamoyl)amino)phenyl)pentanoylamino)ethyl)amino)-2-(3,3-dimethylbutanoylamino) butanoic acid,
4-((2-carboxy-2-(4-methyl-2-(4-((N-(4-nitrophenyl)carbamoyl)amino)phenyl)pentanoylamino)ethyl)amino)-2-(3,3-dimethylbutanoylamino) butanoic acid,
4-((2-carboxy-2-(4-methyl-2-(4-((N-(4-methoxyphenyl)carbamoyl)amino)phenyl)pentanoylamino)ethyl)amino)-2-(3,3-dimethylbutanoylamino)butanoic acid,
4-((2-carboxy-2-(2-(4-((N-(4-hydroxyphenyl)carbamoyl)amino)phenyl)-4-methylpentanoylamino)ethyl)amino)-2-(3,3-dimethylbutanoylamino)butanoic acid,
4-((2-carboxy-2-(2-(4-((N-(4-chlorophenyl)carbamoyl)amino)phenyl)-4-methylpentanoylamino)ethyl)amino)-2-(3,3-dimethylbutanoylamino)butanoic acid, 4-((2-carboxy-2-(4-methyl-2-(4-(((phenylamino)thioxomethyl)amino) phenyl)pentanoylamino)ethyl)amino)-2-(3,3-dimethylbutanoylamino)butanoic acid,
4-((2-carboxy-2-(3,3-dimethylbutanoylamino)ethyl)amino)-2-(4-methyl-2-(4-methyl-2-(4-((N-phenylcarbamoyl)amino) phenyl)pentanoylamino)butanoic acid,
4-((2-carboxy-2-(3,3-dimethylbutanoylamino)ethyl)amino)-2-(4-methyl-2-(4-methyl-2-(4-((N-(4-methylphenyl)carbamoyl)amino)phenyl)pentanoylamino)butanoic acid,
4-((2-carboxy-2-(3,3-dimethylbutanoylamino)ethyl)amino)-2-(4-methyl-2-(4-methyl-2-(4-((N-(4-nitrophenyl)carbamoyl)amino)phenyl)pentanoylamino)butanoic acid,
4-((2-carboxy-2-(3,3-dimethylbutanoylamino)ethyl)amino)-2-(4-methyl-2-(4-methyl-2-(4-((N-(4-methoxyphenyl)carbamoyl)amino)phenyl)pentanoylamino)butanoic acid,
4-((2-carboxy-2-(3,3-dimethylbutanoylamino)ethyl)amino)-2-(2-(4-((N-(4-hydroxyphenyl)carbamoyl)amino)phenyl)-4-methylpentanoylamino)butanoic acid,

4-((2-carboxy-2-(3,3-dimethylbutanoylamino)ethyl)amino)-2-(2-(4-((N-(4-chlorophenyl)carbamoyl)amino)phenyl)-4-methylpentanoylamino)butanoic acid,
4-((2-carboxy-2-(3,3-dimethylbutanoylamino)ethyl)amino)-2-(4-methyl-2-(4-methyl-2-(4-(((phenylamino)thioxomethyl)amino)phenyl)pentanoylamino)butanoic acid,
4-(2-carboxy-2-(3,3-dimethylbutanoylamino)ethoxy)-2-(4-methyl-2-(4-((N-phenylcarbamoyl)amino)phenyl)pentanoylamino)butanoic acid,
4-(2-carboxy-2-(3,3-dimethylbutanoylamino)ethoxy)-2-(4-methyl-2-(4-((N-(4-methylphenyl)carbamoyl)amino)phenyl) pentanoylamino)butanoic acid,
4-(2-carboxy-2-(3,3-dimethylbutanoylamino)ethoxy)-2-(4-methyl-2-(4-((N-(4-nitrophenyl)carbamoyl)amino)phenyl) pentanoylamino)butanoic acid,
4-(2-carboxy-2-(3,3-dimethylbutanoylamino)ethoxy)-2-(4-methyl-2-(4-((N-(4-methoxyphenyl)carbamoyl)amino)phenyl)pentanoylamino)butanoic acid,
4-(2-carboxy-2-(3,3-dimethylbutanoylamino)ethoxy)-2-(2-(4-((N-(4-hydroxyphenyl)carbamoyl)amino)phenyl)-4-methylpentanoylamino)butanoic acid,
4-(2-carboxy-2-(3,3-dimethylbutanoylamino)ethoxy)-2-(2-(4-((N-(4-chlorophenyl)carbamoyl)amino)phenyl)-4-methylpentanoylamino)butanoic acid,
4-(2-carboxy-2-(3,3-dimethylbutanoylamino)ethoxy)-2-(4-methyl-2-(4-(((phenylamino)thioxomethyl)amino)phenyl) pentanoylamino)butanoic acid, 6-(2-(4-((2-aza-2-cyano-1-(phenylamino)vinyl)amino)phenyl)-4-methylpentanoylamino)-1-(3,3-dimethylbutanoylamino)hexane-1,6-dicarboxylic acid, 6-(2-(4-((2-aza-2-cyano-1-((4-methylphenyl)amino) vinyl)amino)phenyl)-4-methylpentanoylamino)-1-(3,3-dimethylbutanoylamino)hexane-1,6-dicarboxylic acid, 6-(2-(4-((2-aza-2-cyano-1-((4-nitrophenyl)amino)vinyl)amino)phenyl)-4-methylpentanoylamino)-1-(3,3-dimethylbutanoylamino)hexane-1,6-dicarboxylic acid, 6-(2-(4-((2-aza-2-cyano-1-((4-methoxyphenyl)amino)vinyl)amino)phenyl)-4-methylpentanoylamino)-1-(3,3-dimethylbutanoylamino)hexane-1,6-dicarboxylic acid, 6-(2-(4-((2-aza-2-cyano-1-((4-hydroxyphenyl)amino)vinyl)amino)phenyl)-4-methylpentanoylamino)-1-(3,3 -dimethylbutanoylamino)hexane-1,6-dicarboxylic acid, 6-(2-(4-((2-aza-2-cyano-1-((4-chlorophenyl)amino)vinyl)amino)phenyl)-4-methylpentanoylamino)-1-(3,3-dimethylbutanoylamino)hexane-1,6-dicarboxylic acid, 4-((2-(2-(4-((2-aza-2-cyano-1-(phenylamino)vinyl)amino)phenyl)-4-methylpentanoylamino)-2-carboxyethyl)amino)-2-(3,3 -dimethylbutanoylamino) butanoic acid,
4-((2-(2-(4-((2-aza-2-cyano-1-((4-methylphenyl)amino)vinyl)amino)phenyl)-4-methylpentanoylamino)-2-carboxyethyl)amino)-2-(3,3-dimethylbutanoylamino) butanoic acid,
4-((2-(2-(4-((2-aza-2-cyano-1-((4-nitrophenyl)amino)vinyl)amino)phenyl)-4-methylpentanoylamino)-2-carboxyethyl) amino)-2-(3,3-dimethylbutanoylamino) butanoic acid,
4-((2-(2-(4-((2-aza-2-cyano-1-((4-methoxyphenyl)amino)vinyl)amino)phenyl)-4-methylpentanoylamino)-2-carboxyethyl)amino)-2-(3,3-dimethylbutanoylamino) butanoic acid,
4-((2-(2-(4-((2-aza-2-cyano-1-((4-hydroxyphenyl)amino)vinyl)amino)phenyl)-4-methylpentanoylamino)-2-carboxyethyl)amino)-2-(3,3-dimethylbutanoylamino) butanoic acid,
4-((2-(2-(4-((2-aza-2-cyano-1-((4-chlorophenyl)amino)vinyl)amino)phenyl)-4-methylpentanoylamino)-2-carboxyethyl) amino)-2-(3,3-dimethylbutanoylamino) butanoic acid,
2-(2-(4-((2-aza-2-cyano-1-(phenylamino)vinyl)amino)phenyl)-4-methylpentanoylamino)-4-(2-carboxy-2-(3,3-dimethylbutanoylamino)ethylthio) butanoic acid,
2-(2-(4-((2-aza-2-cyano-1-((4-methylphenyl)amino)vinyl)amino)phenyl)-4-methylpentanoylamino)-4-(2-carboxy-2-(3,3 -dimethylbutanoylamino)ethylthio) butanoic acid,
2-(2-(4-((2-aza-2-cyano-1-((4-nitrophenyl)amino)vinyl)amino)phenyl)-4-methylpentanoylamino)-4-(2-carboxy-2-(3,3-dimethylbutanoylamino)ethylthio) butanoic acid,
2-(2-(4-((2-aza-2-cyano-1-((4-methoxyphenyl)amino)vinyl)amino)phenyl)-4-methylpentanoylamino)-4-(2-carboxy-2-(3,3-dimethylbutanoylamino)ethylthio) butanoic acid,
2-(2-(4-((2-aza-2-cyano-1-((4-hydroxyphenyl)amino)vinyl)amino)phenyl)-4-methylpentanoylamino)-4-(2-carboxy-2-(3,3-dimethylbutanoylamino)ethylthio) butanoic acid,
2-(2-(4-((2-aza-2-cyano-1-((4-chlorophenyl)amino)vinyl)amino)phenyl)-4-methylpentanoylamino)-4-(2-carboxy-2-(3,3-dimethylbutanoylamino)ethylthio) butanoic acid,
1-(2-(4-((2-aza-2-cyano-1-(phenylamino)vinyl)amino)phenyl)-4-methylpentanoylamino)-6-(3,3-dimethylbutanoylamino)hex-3-ene-1,6-dicarboxylic acid,
1-(2-(4-((2-aza-2-cyano-1-((4-methylphenyl)amino)vinyl)amino)phenyl)-4-methylpentanoylamino)-6-(3,3-dimethylbutanoylamino)hex-3-ene-1,6-dicarboxylic acid,
1-(2-(4-((2-aza-2-cyano-1-((4-nitrophenyl)amino)vinyl)amino)phenyl)-4-methylpentanoylamino)-6-(3,3-dimethylbutanoylamino)hex-3-ene-1,6-dicarboxylic acid,
1-(2-(4-((2-aza-2-cyano-1-((4-methoxyphenyl)amino)vinyl)amino)phenyl)-4-methylpentanoylamino)-6-(3,3-dimethylbutanoylamino)hex-3-ene-1,6-dicarboxylic acid,
1-(2-(4-((2-aza-2-cyano-1-((4-hydroxyphenyl)amino)vinyl)amino)phenyl)-4-methylpentanoylamino)-6-(3,3-dimethylbutanoylamino)hex-3-ene-1,6-dicarboxylic acid,
1-(2-(4-((2-aza-2-cyano-1-((4-chlorophenyl)amino)vinyl)amino)phenyl)-4-methylpentanoylamino)-6-(3,3-dimethylbutanoylamino)hex-3-ene-1,6-dicarboxylic acid,
3-(2-(2-(2-(4-((2-aza-2-cyano-1-(phenylamino)vinyl)amino)phenyl)-4-methylpentanoylamino)-2-carboxyethyl)phenyl)-2-(3,3-dimethylbutanoylamino)propanoic acid,
3-(2-(2-(2-(4-((2-aza-2-cyano-1-((4-methylphenyl)amino)vinyl)amino)phenyl)-4-methylpentanoylamino)-2-carboxyethyl)phenyl)-2-(3,3-dimethylbutanoylamino) propanoic acid,
3-(2-(2-(2-(4-((2-aza-2-cyano-1-((4-nitrophenyl)amino)vinyl)amino)phenyl)-4-methylpentanoylamino)-2-carboxyethyl) phenyl)-2-(3,3-dimethylbutanoylamino) propanoic acid,
3-(2-(2-(2-(4-((2-aza-2-cyano-1-((4-methoxyphenyl)amino)vinyl)amino)phenyl)-4-methylpentanoylamino)-2-carboxyethyl)phenyl)-2-(3,3-dimethylbutanoylamino) propanoic acid,
3-(2-(2-(2-(4-((2-aza-2-cyano-1-((4-hydroxyphenyl)amino)vinyl)amino)phenyl)-4-methylpentanoylamino)-2-carboxyethyl)phenyl)-2-(3,3-dimethylbutanoylamino) propanoic acid,
3-(2-(2-(2-(4-((2-aza-2-cyano-1-((4-chlorophenyl)amino)vinyl)amino)phenyl)-4-methylpentanoylamino)-2-carboxyethyl)phenyl)-2-(3,3-dimethylbutanoylamino) propanoic acid,
3-(2-(2-carboxy-2-(3,3-dimethylbutanoylamino)ethyl)phenyl)-2-(4-methyl-2-(4-((N-phenylcarbamoyl)amino)phenyl) pentanoylamino)propanoic acid,
3-(2-(2-carboxy-2-(3,3-dimethylbutanoylamino)ethyl)phenyl)-2-(4-methyl-2-(4-((N-(4-methylphenyl)carbamoyl)amino)phenyl)pentanoylamino)propanoic acid,
3-(2-(2-carboxy-2-(3,3-dimethylbutanoylamino)ethyl)phenyl)-2-(4-methyl-2-(4-((N-(4-nitrophenyl)carbamoyl)amino) phenyl)pentanoylamino)propanoic acid,
3-(2-(2-carboxy-2-(3,3-dimethylbutanoylamino)ethyl)phenyl)-2-(4-methyl-2-(4-((N-(4-methoxyphenyl)carbamoyl)amino)phenyl)pentanoylamino)propanoic acid,
3-(2-(2-carboxy-2-(3,3-dimethylbutanoylamino)ethyl)phenyl)-2-(4-methyl-2-(4-((N-(4-hydroxyphenyl)carbamoyl)amino)phenyl)pentanoylamino)propanoic acid,
3-(2-(2-carboxy-2-(3,3-dimethylbutanoylamino)ethyl)phenyl)-2-(4-methyl-2-(4-((N-(4-chlorophenyl)carbamoyl)amino) phenyl)pentanoylamino)propanoic acid,
3-(2-(2-carboxy-2-(3,3-dimethylbutanoylamino)ethyl)phenyl)-2-(4-methyl-2-(4-(((phenylamino)thioxomethyl)amino) phenyl)pentanoylamino)propanoic acid,
6-(3,3-dimethylbutanoylamino)-1-(4-methyl-2-(4-((2-nitro-1-(phenylamino)vinyl)amino)phenyl)pentanoylamino)hex-3-ene-1,6-dicarboxylic acid,
6-(3,3-dimethylbutanoylamino)-1-(4-methyl-2-(4-((1-(4-methylphenyl)amino)-2-nitrovinyl)amino)phenyl)pentanoylamino)hex-3-ene-1,6-dicarboxylic acid,
6-(3,3-dimethylbutanoylamino)-1-(4-methyl-2-(4-((2-nitro-1-(4-nitrophenyl)amino)vinyl)amino)phenyl)pentanoylamino)hex-3-ene-1,6-dicarboxylic acid,
6-(3,3-dimethylbutanoylamino)-1-(4-methyl-2-(4-((1-((4-methoxyphenyl)amino)-2-nitrovinyl)amino)phenyl)pentanoylamino)hex-3-ene-1,6-dicarboxylic acid,
1-(2-(4-((1-(4-hydroxyphenyl)amino)-2-nitrovinyl)amino)phenyl)-4-methylpentanoylamino)-6-(3,3-dimethylbutanoylamino)hex-3-ene-1,6-dicarboxylic acid,
1-(2-(4-((1-(4-chlorophenyl)amino)-2-nitrovinyl)amino)phenyl)-4-methylpentanoylamino)-6-(3,3-dimethylbutanoylamino)hex-3-ene-1,6-dicarboxylic acid,
3-(2-(2-carboxy-2-(4-methyl-2-(4-((2-nitro-1-(phenylamino)vinyl)amino)phenyl)pentanoylamino)ethyl)phenyl)-2-(3,3-dimethylbutanoylamino)propanoic acid,
3-(2-(2-carboxy-2-(4-methyl-2-(4-((1-(4-methylphenyl)amino)-2-nitrovinyl)amino)phenyl)pentanoylamino)ethyl)phenyl)-2-(3,3-dimethylbutanoylamino)propanoic acid,
3-(2-(2-carboxy-2-(4-methyl-2-(4-((2-nitro-1-((4-nitrophenyl)amino)vinyl)amino)phenyl)pentanoylamino)ethyl)phenyl)-2-(3,3-dimethylbutanoylamino)propanoic acid,
3-(2-(2-carboxy-2-(4-methyl-2-(4-((1-(4-methoxyphenyl)amino)-2-nitrovinyl)amino)phenyl)pentanoylamino)ethyl)phenyl)-2-(3,3-dimethylbutanoylamino)propanoic acid,
3-(2-(2-carboxy-2-(2-(4-((1-((4-hydroxyphenyl)amino)-2-nitrovinyl)amino)phenyl)-4-methylpentanoylamino)ethyl)phenyl)-2-(3,3-dimethylbutanoylamino)propanoic acid,
3-(2-(2-carboxy-2-(2-(4-((1-((4-chlorophenyl)amino)-2-nitrovinyl)amino)phenyl)-4-methylpentanoylamino)ethyl)phenyl)-2-(3,3-dimethylbutanoylamino)propanoic acid,
3-(2-(2-carboxy-2-(2-phenylacetylamino)ethyl)phenyl)-2-(4-methyl-2-(4-((N-phenylcarbamoyl)amino)phenyl)pentanoylamino)propanoic acid,
3-(2-(2-carboxy-2-(2-phenylacetylamino)ethyl)phenyl)-2-(4-methyl-2-(4-((N-(4-methylphenyl)carbamoyl)amino)phenyl)pentanoylamino)propanoic acid,
3-(2-(2-carboxy-2-(2-phenylacetylamino)ethyl)phenyl)-2-(4-methyl-2-(4-((N-(4-nitrophenyl)carbamoyl)amino)phenyl)pentanoylamino)propanoic acid,
3-(2-(2-carboxy-2-(2-phenylacetylamino)ethyl)phenyl)-2-(4-methyl-2-(4-((N-(4-methoxyphenyl)carbamoyl)amino) phenyl)pentanoylamino)propanoic acid,
3-(2-(2-carboxy-2-(2-phenylacetylamino)ethyl)phenyl)-2-(2-(4-((N-(4-hydroxyphenyl)carbamoyl)amino)phenyl)-4-methylpentanoylamino)propanoic acid,
3-(2-(2-carboxy-2-(2-phenylacetylamino)ethyl)phenyl)-2-(2-(4-((N-(4-chlorophenyl)carbamoyl)amino)phenyl)-4-methylpentanoylamino)propanoic acid,
3-(2-(2-carboxy-2-(2-phenylacetylamino)ethyl)phenyl)-2-(4-methyl-2-(4-(((phenylamino)thioxomethyl)amino)phenyl) pentanoylamino)propanoic acid,
6-(3,3 -dimethylbutanoylamino)-1-((1-methyl-4-(4-((phenylamino)carbonylamino)phenyl)pyrrolidin-2-yl)carbonylamino)hex-3-ene-1,6-dicarboxylic acid,
6-(3,3-dimethylbutanoylamino)-1-((1-methyl-4-(4-(((4-methylphenyl)amino)carbonylamino)phenyl)pyrrolidin-2-yl)carbonylamino)hex-3-ene-1,6-dicarboxylic acid,
6-(3,3-dimethylbutanoylamino)-1-((1-methyl-4-(4-(((4-nitrophenyl)amino)carbonylamino)phenyl)pyrrolidin-2-yl)carbonylamino)hex-3-ene-1,6-dicarboxylic acid,
6-(3,3-dimethylbutanoylamino)--((1-methyl-4-(4-(((4-methoxyphenyl)amino)carbonylamino)phenyl)pyrrolidin-2-yl)carbonylamino)hex-3-ene-1,6-dicarboxylic acid,
1-((4-(4-(((4-hydroxyphenyl)amino)carbonylamino)phenyl)-1-methylpyrrolidin-2-yl)carbonylamino)-6-(3,3-dimethylbutanoylamino)hex-3-ene-1,6-dicarboxylic acid,
1-((4-(4-(((4-chlorophenyl)amino)carbonylamino)phenyl)-1-methylpyrrolidin-2-yl)carbonylamino)-6-(3,3-dimethylbutanoylamino)hex-3-ene-1,6-dicarboxylic acid,
6-(3,3-dimethylbutanoylamino)-1-((1-methyl-4-(4-(((phenylamino)thioxomethyl)amino)phenyl)pyrrolidin-2-yl)carbonylamino)hex-3-ene-1,6-dicarboxylic acid,
6-(3,3-dimethylbutanoylamino)-1-((1-methyl-4-(4-((phenylamino)carbonylamino)phenyl)pyrrolidin-2-yl)carbonylamino)hexane-1,6-dicarboxylic acid,
6-(3,3-dimethylbutanoylamino)-1-((1-methyl-4-(4-(((4-methylphenyl)amino)carbonylamino)phenyl)pyrrolidin-2-yl)carbonylamino)hexane-1,6-dicarboxylic acid,
6-(3,3-dimethylbutanoylamino)-1-((1-methyl-4-(4-(((4-nitrophenyl)amino)carbonylamino)phenyl)pyrrolidin-2-yl)carbonylamino)hexane-1,6-dicarboxylic acid,
6-(3,3-dimethylbutanoylamino)-1-((1-methyl-4-(4-(((4-methoxyphenyl)amino)carbonylamino)phenyl)pyrrolidin-2-yl) carbonylamino)hexane-1,6-dicarboxylic acid,
1-((4-(4-(((4-hydroxyphenyl)amino)carbonylamino)phenyl)-1-methylpyrrolidin-2-yl)carbonylamino)-6-(3,3-dimethylbutanoylamino)hexane-1,6-dicarboxylic acid,
1-((4-(4-(((4-chlorophenyl)amino)carbonylamino)phenyl)-1-methylpyrrolidin-2-yl)carbonylamino)-6-(3,3-dimethylbutanoylamino)hexane-1,6-dicarboxylic acid,
6-(3,3-dimethylbutanoylamino)-1-((1-methyl-4-(4-(((phenylamino)thioxomethyl)amino)phenyl)pyrrolidin-2-yl)carbonylamino)hexane-1,6-dicarboxylic acid,
1-(4-methyl-2-(4-((N-phenylcarbamoyl)amino)phenyl)pentanoylamino)-6-(2-phenylacetylamino)hex-3-ene-1,6-dicarboxylic acid,
1-(4-methyl-2-(4-((N-(4-methylphenyl)carbamoyl)amino)phenyl)pentanoylamino)-6-(2-phenylacetylamino)hex-3-ene-1,6-dicarboxylic acid,
1-(4-methyl-2-(4-((N-(4-nitrophenyl)carbamoyl)amino)phenyl)pentanoylamino)-6-(2-phenylacetylamino)hex-3-ene-1,6-dicarboxylic acid,
1-(4-methyl-2-(4-((N-(4-methoxyphenyl)carbamoyl)amino)phenyl)pentanoylamino)-6-(2-phenylacetylamino)hex-3-ene-1,6-dicarboxylic acid,
1-(2-(4-((N-(4-hydroxyphenyl)carbamoyl)amino)phenyl)-4-methylpentanoylamino)-6-(2-phenylacetylamino)hex-3-ene-1,6-dicarboxylic acid,
1-(2-(4-((N-(4-chlorophenyl)carbamoyl)amino)phenyl)-4-methylpentanoylamino)-6-(2-phenylacetylamino)hex-3-ene-1,6-dicarboxylic acid,
1-(4-methyl-2-(4-((phenylamino)thioxomethyl)amino)phenyl)pentanoylamino)-6-(2-phenylacetylamino)hex-3-ene-1,6-dicarboxylic acid,
1-((1-acetyl-4-(4-((phenylamino)carbonylamino)phenyl)pyrrolidin-2-yl)carbonylamino)-6-(3,3-dimethylbutanoylamino) hex-3-ene-1,6-dicarboxylic acid,
1-((1-acetyl-4-(4-(((4-methylphenyl)amino)carbonylamino)phenyl)pyrrolidin-2-yl)carbonylamino)-6-(3,3-dimethylbutanoylamino)hex-3-ene-1,6-dicarboxylic acid,
1-((1-acetyl-4-(4-(((4-nitrophenyl)amino)carbonylamino)phenyl)pyrrolidin-2-yl)carbonylamino)-6-(3,3-dimethylbutanoylamino)hex-3-ene-1,6-dicarboxylic acid,
1-(((1-acetyl-4-(4-(((4-methoxyphenyl)amino)carbonylamino)phenyl)pyrrolidin-2-yl)carbonylamino)-6-(3,3-dimethylbutanoylamino)hex-3-ene-1,6-dicarboxylic acid,
1-((1-acetyl-4-(4-(((4-hydroxyphenyl)amino)carbonylamino)phenyl)pyrrolidin-2-yl)carbonylamino)-6-(3,3-dimethylbutanoylamino)hex-3-ene-1,6-dicarboxylic acid,
1-((1-acetyl-4-(4-(((4-chlorophenyl)amino)carbonylamino)phenyl)pyrrolidin-2-yl)carbonylamino)-6-(3,3-dimethylbutanoylamino)hex-3-ene-1,6-dicarboxylic acid,
1-((1-acetyl-4-(4-(((phenylamino)thioxomethyl)amino)phenyl)pyrrolidin-2-yl)carbonylamino)-6-(3,3-dimethylbutanoylamino)hex-3-ene-1,6-dicarboxylic acid,
4-(2-carboxy-2-(3,3-dimethylbutanoylamino)ethylthio)-2-((-methyl-4-(4-((phenylamino)carbonylamino)phenyl)pyrrolidin-2-yl)carbonylamino)butanoic acid,
4-(2-carboxy-2-(3,3-dimethylbutanoylamino)ethylthio)-2-((1-methyl-4-(4-(((4-methylphenyl)amino)carbonylamino) phenyl)pyrrolidin-2-yl)carbonylamino) butanoic acid,
4-(2-carboxy-2-(3,3-dimethylbutanoylamino)ethylthio)-2-((1-methyl-4-(4-(((4-nitrophenyl)amino)carbonylamino)phenyl)pyrrolidin-2-yl)carbonylamino)butanoic acid,
4-(2-carboxy-2-(3,3-dimethylbutanoylamino)ethylthio)-2-((1-methyl-4-(4-(((4-methoxyphenyl)amino)carbonylamino) phenyl)pyrrolidin-2-yl)carbonylamino) butanoic acid,
4-(2-carboxy-2-(3,3-dimethylbutanoylamino)ethylthio)-2-((4-(4- (((4-hydroxyphenyl)amino)carbonylamino)phenyl)-1-methylpyrrolidin-2-yl)carbonylamino)butanoic acid,
4-(2-carboxy-2-(3,3-dimethylbutanoylamino)ethylthio)-2-((4-(4- (((4-chlorophenyl)amino)carbonylamino)phenyl)-1-methylpyrrolidin-2-yl)carbonylamino)butanoic acid,
4-(2-carboxy-2-(3,3-dimethylbutanoylamino)ethylthio)-2-((1-methyl-4-(4-(((phenylamino)thioxomethyl)amino)phenyl) pyrrolidin-2-yl)carbonylamino) butanoic acid,
2-((1-acetyl-4-(4-((phenylamino)carbonylamino)phenyl)pyrrolidin)-2-yl)carbonylamino)-4-(2-carboxy-2-(3,3-dimethylbutanoylamino)ethylthio)butanoic acid,
2-((1-acetyl-4-(4-(((4-methylphenyl)amino)carbonylamino)phenyl)pyrrolidin-2-yl)carbonylamino)-4-(2-carboxy-2-(3,3-dimethylbutanoylamino)ethylthio)butanoic acid,
2-((1-acetyl-4-(4-(((4-nitrophenyl)amino)carbonylamino)phenyl)pyrrolidin-2-yl)carbonylamino)-4-(2-carboxy-2-(3,3-dimethylbutanoylamino)ethylthio)butanoic acid,
2-((1-acetyl-4-(4-(((4-methoxyphenyl)amino)carbony(amino)phenyl)pyrrolidin-2-yl)carbonylamino)-4-(2-carboxy-2-(3,3-dimethylbutanoylamino)ethylthio)butanoic acid,
2-((1-acetyl-4-(4-(((4-hydroxyphenyl)amino)carbonylamino)phenyl)pyrrolidin-2-yl)carbonylamino)-4-(2-carboxy-2-(3,3-dimethylbutanoylamino)ethylthio)butanoic acid,
2-((1-acetyl-4-(4-(((4-chlorophenyl)amino)carbonylamino)phenyl)pyrrolidin-2-yl)carbonylamino)-4-(2-carboxy-2-(3,3-dimethylbutanoylamino)ethylthio)butanoic acid,
2-((1-acetyl-4-(4-(((phenylamino)thioxomethyl)amino)phenyl)pyrrolidin-2-yl)carbonylamino)-4-(2-carboxy-2-(3,3-dimethylbutanoylamino)ethylthio)butanoic acid,
4-(2-carboxy-2-(3,3-dimethylbutanoylamino)ethylthio)-2-((2-methyl-6-((2-methyl-6-((2-nitro-1-(phenylamino)vinyl) amino)-1,3,4-trihydroisoquinolyl)carbonylamino) butanoic acid,
4-(2-carboxy-2-(3,3-dimethylbutanoylamino)ethylthio)-2-((2-methyl-6-((1-((4-methylphenyl)amino)-2-nitrovinyl)amino)-1,3,4-trihydroisoquinolyl)carbonylamino) butanoic acid,
4-(2-carboxy-2-(3,3-dimethylbutanoylamino)ethylthio)-2-((2-methyl-6-((2-nitro-1-((4-nitrophenyl)amino)vinyl)amino)-1,3,4-trihydroisoquinolyl)carbonylamino) butanoic acid,
4-(2-carboxy-2-(3,3-dimethylbutanoylamino)ethylthio)-2-((2-methyl-6-((1-((4-methoxyphenyl)amino)-2-nitrovinyl)amino)-1,3,4-trihydroisoquinolyl)carbonylamino)butanoic acid,
4-(2-carboxy-2-(3,3-dimethylbutanoylamino)ethylthio)-2-((6-((1-((4-hydroxyphenyl)amino)-2-nitrovinyl)amino)-2-methyl-1,3,4-trihydroisoquinolyl)carbonylamino)butanoic acid,
4-(2-carboxy-2-(3,3-dimethylbutanoylamino)ethylthio)-2-((6-((1-((4-chlorophenyl)amino)-2-nitrovinyl)amino)-2-methyl-1,3,4-trihydroisoquinolyl)carbonylamino)butanoic acid,
4-(2-carboxy-2-(3,3-dimethylbutanoylamino)ethylthio)-2-((2-methyl-6-((phenylamino)carbonylamino)-1,3,4-trihydroisoquinolyl)carbonylamino)butanoic acid,
4-(2-carboxy-2-(3,3-dimethylbutanoylamino)ethylthio)-2-((2-methyl-6-(((4-methylphenyl)amino)carbonylamino)-1,3,4-trihydroisoquinolyl)carbonylamino) butanoic acid,
4-(2-carboxy-2-(3,3-dimethylbutanoylamino)ethylthio)-2-((2-methyl-6-(((4-nitrophenyl)amino)carbonylamino)-1,3,4-trihydroisoquinolyl)carbonylamino) butanoic acid,
4-(2-carboxy-2-(3,3-dimethylbutanoylamino)ethylthio)-2-((2-methyl-6-(((4-methoxyphenyl)amino)carbonylamino)-1,3,4-trihydroisoquinolyl)carbonylamino) butanoic acid,
4-(2-carboxy-2-(3,3-dimethylbutanoylamino)ethylthio)-2-((6-(((4-hydroxyphenyl)amino)carbonylamino)-2-methyl-1,3,4-trihydroisoquinolyl)carbonylamino)butanoic acid,
4-(2-carboxy-2-(3,3-dimethylbutanoylamino)ethylthio)-2-((6-(((4-chlorophenyl)amino)carbonylamino)-2-methyl-1,3,4-trihydroisoquinolyl)carbonylamino)butanoic acid,
4-(2-carboxy-2-(3,3-dimethylbutanoylamino)ethylthio)-2-((2-methyl-6-(((phenylamino)thioxomethyl)amino)-1,3,4-trihydroisoquinolyl)carbonylamino) butanoic acid,
2-((6-((2-aza-2-cyano-1-(phenylamino)vinyl)amino)-2-methyl-1,3,4-trinitroisoquinolyl)carbonylamino)-4-(2-carboxy-2-(3,3-dimethylbutanoylamino)ethylthio)butanoic acid,
2-((6-((2-aza-2-cyano-1-((4-methylphenyl)amino)vinyl)amino)-2-methyl-1,3,4-trinitroisoquinolyl)carbonylamino)-4-(2-carboxy-2-(3,3-dimethylbutanoylamino)ethylthio)butanoic acid,
2-((6-((2-aza-2-cyano-1-((4-nitrophenyl)amino)vinyl)amino)-2-methyl-1,3,4-trinitroisoquinolyl)carbonylamino)-4-(2-carboxy-2-(3,3-dimethylbutanoylamino)ethylthio)butanoic acid,
2-((6-((2-aza-2-cyano-1-((4-methoxyphenyl)amino)vinyl)amino)-2-methyl-1,3,4-trinitroisoquinolyl)carbonylamino)-4-(2-carboxy-2-(3,3-dimethylbutanoylamino)ethylthio)butanoic acid,
2-((6-((2-aza-2-cyano-1-((4-hydroxyphenyl)amino)vinyl)amino)-2-methyl-1,3,4-trinitroisoquinolyl)carbonylamino)-4-(2-carboxy-2-(3,3-dimethylbutanoylamino)ethylthio)butanoic acid,
2-((6-((2-aza-2-cyano-1-((4-chlorophenyl)amino)vinyl)amino)-2-methyl-1,3,4-trinitroisoquinolyl)carbonylamino)-4-(2-carboxy-2-(3,3-dimethylbutanoylamino)ethylthio)butanoic acid,
2-((2-acetyl-5 -((2-nitro-1-(phenylamino)vinyl)amino)isoindolinyl)carbonylamino)-4-(2-carboxy-2-(3,3-dimethylbutanoylamino)ethylthio)butanoic acid,
2-((2-acetyl-5-((1-((4-methylphenyl)amino)-2-nitrovinyl)amino)isoindolinyl)carbonylamino)-4-(2-carboxy-2-(3,3-dimethylbutanoylamino)ethylthio)butanoic acid,
2-((2-acetyl-5-((2-nitro-1-((4-nitrophenyl)amino)vinyl)amino)isoindolinyl)carbonylamino)-4-(2-carboxy-2-(3,3-dimethylbutanoylamino)ethylthio)butanoic acid,
2-((2-acetyl-5-((1-((4-methoxyphenyl)amino)-2-nitrovinyl)amino)isoindolinyl)carbonylamino)-4-(2-carboxy-2-(3,3-dimethylbutanoylamino)ethylthio)butanoic acid,
2-((2-acetyl-5-((1-((4-hydroxyphenyl)amino)-2-nitrovinyl)amino)isoindolinyl)carbonylamino)-4-(2-carboxy-2-(3,3-dimethylbutanoylamino)ethylthio)butanoic acid,
2-((2-acetyl-5-((1-((4-chlorophenyl)amino)-2-nitrovinyl)amino)isoindolinyl)carbonylamino)-4-(2-carboxy-2-(3,3-dimethylbutanoylamino)ethylthio)butanoic acid,
4-(2-((2-acetyl-5-((2-aza-2-cyano-1-(phenylamino)vmyl)amino)isomdolinyl)carbonylamino)-2-carboxyethylthio)-2-(3,3-dimethylbutanoylamino)butanoic acid,
4-(2-((2-acetyl-5-((2-aza-2-cyano-1-((4-methylphenyl)amino)vinyl)amino)isoindolinyl)carbonylamino)-2-carboxyethylthio)-2-(3,3-dimethylbutanoylamino)butanoic acid,
4-(2-((2-acetyl-5-((2-aza-2-cyano-1-((4-nitrophenyl)amino)vinyl)amino)isoindolinyl)carbonylamino)-2-carboxyethylthio)-2-(3,3-dimethylbutanoylamino)butanoic acid,
4-(2-((2-acetyl-5-((2-aza-2-cyano-1-((4-methoxyphenyl)amino)vinyl)amino)isoindolinyl)carbonylamino)-2-carboxyethylthio)-2-(3,3-dimethylbutanoylamino)butanoic acid,
4-(2-((2-acetyl-5-((2-aza-2-cyano-1-((4-hydroxyphenyl)amino)vinyl)amino)isoindolinyl)carbonylamino)-2-carboxyethylthio)-2-(3,3-dimethylbutanoylamino)butanoic acid,
4-(2-((2-acetyl-5-((2-aza-2-cyano-1-((4-chlorophenyl)amino)vinyl)amino)isoindolinyl)carbonylamino)-2-carboxyethylthio)-2-(3,3-dimethylbutanoylamino)butanoic acid,
4-(2-carboxy-2-((5-hydroxy-2-methyl-1,3,4-trihydroisoquinolyl)carbonylamino)ethoxy)-2-(3,3-dimethylbutanoylamino) butanoic acid,
4-(2-carboxy-2-((6-hydroxy-2-methyl-1,3,4-trihydroisoquinolyl)carbonylamino)ethoxy)-2-(3,3-dimethylbutanoylamino) butanoic acid,
4-(2-carboxy-2-((7-hydroxy-2-methyl-1,3,4-trihydroisoquinolyl)carbonylamino)ethoxy)-2-(3,3-dimethylbutanoylamino) butanoic acid,
4-(2-carboxy-2-((2-methyl-5-methoxy-1,3,4-trihydroisoquinolyl)carbonylamino)ethoxy)-2-(3,3-dimethylbutanoylamino)butanoic acid,
4-(2-carboxy-2-((2-methyl-6-methoxy-1,3,4-trihydroisoquinolyl)carbonylamino)ethoxy)-2-(3,3-dimethylbutanoylamino)butanoic acid,
4-(2-carboxy-2-((2-methyl-7-methoxy-1,3,4-trihydroisoquinolyl)carbonylamino)ethoxy)-2-(3,3-dimethylbutanoylamino)butanoic acid,
1-((2-acetyl-5-((2-nitro-1-(phenylamino)vinyl)amino)isoindolinyl)carbonylamino)-6-(3,3-dimethylbutanoylamino)hex-3-ene-1,6-dicarboxylic acid,
1-((2-acetyl-5-((1-((4-methylphenyl)amino)-2-nitrovinyl)amino)isoindolinyl)carbonylamino)-6-(3,3-dimethylbutanoylamino)hex-3-ene-1,6-dicarboxylic acid,
1-((2-acetyl-5-((1-((4-nitrophenyl)amino)-2-nitrovinyl)amino)isoindolinyl)carbonylamino)-6-(3,3 dimethylbutanoylamino)hex-3-ene-1,6-dicarboxylic acid,
1-((2-acetyl-5-((1-((4-methoxyphenyl)amino)-2-nitrovinyl)amino)isoindolinyl)carbonylamino)-6-(3,3-dimethylbutanoylamino)hex-3-ene-1,6-dicarboxylic acid,
1-((2-acetyl-5-((1-((4-hydroxyphenyl)amino)-2-nitrovinyl)amino)isoindolinyl)carbonylamino)-6-(3,3-dimethylbutanoylamino)hex-3-ene-1,6-dicarboxylic acid,
1-((2-acetyl-5-((1-((4-chlorophenyl)amino)-2-nitrovinyl)amino)isoindolinyl)carbonylamino)-6-(3,3-dimethylbutanoylamino)hex-3-ene-1,6-dicarboxylic acid,
1-((2-acetyl-5-((2-aza-2-cyano-1-(phenylamino)vinyl)amino)isoindolinyl)carbonylamino)-6-(3,3-dimethylbutanoylamino)hexane-1,6-dicarboxylic acid,
1-((2-acetyl-5-((2-aza-2-cyano-1-((4-methylphenyl)amino)vinyl)amino)isoindolinyl)carbonylamino)-6-(3,3-dimethylbutanoylarnino)hexane-1,6-dicarboxylic acid,
1-((2-acetyl-5-((2-aza-2-cyano-1-((4-nitrophenyl)amino)vinyl)amino)isoindolinyl)carbonylamino)-6-(3,3-dimethylbutanoylamino)hexane-1,6-dicarboxylic acid,
1-((2-acetyl-5-((2-aza-2-cyano-1-((4-methoxyphenyl)amino)vinyl)amino)isoindolinyl)carbonylamino)-6-(3,3-dimethylbutanoylamino)hexane-1,6-dicarboxylic acid,
1-((2-acetyl-5-((2-aza-2-cyano-1-((4-hydroxyphenyl)amino)vinyl)amino)isoindolinyl)carbonylamino)-6-(3,3-dimethylbutanoylamino)hexane-1,6-dicarboxylic acid,
1-((2-acetyl-5-((2-aza-2-cyano-1-((4-chlorophenyl)amino)vinyl)amino)isoindolinyl)carbonylamino)-6-(3,3-dimethylbutanoylamino)hexane-1,6-dicarboxylic acid,
1-((5-hydroxy-2-methyl-1,3,4-trihydroisoquinolyl)carbonylamino)-6-(3,3-dimethylbutanoylamino)hexane-1,6-dicarboxylic acid,
1-((6-hydroxy-2-methyl-1,3,4-trihydroisoquinolyl)carbonylamino)-6-(3,3-dimethylbutanoylamino)hexane-1,6-dicarboxylic acid,
1-((7-hydroxy-2-methyl-1,3,4-trihydroisoquinolyl)carbonylamino)-6-(3,3-dimethylbutanoylamino)hexane-1,6-dicarboxylic acid,
1-((5-methoxy-2-methyl-1,3,4-trihydroisoquinolyl)carbonylamino)-6-(3,3-dimethylbutanoylamino)hexane-1,6-dicarboxylic acid,
1-((6-methoxy-2-methyl-1,3,4-trihydroisoquinolyl)carbonylamino)-6-(3,3-dimethylbutanoylamino)hexane-1,6-dicarboxylic acid,
1-((7-methoxy-2-methyl-1,3,4-trihydroisoquinolyl)carbonylamino)-6-(3,3-dimethylbutanoylamino)hexane-1,6-dicarboxylic acid,
3-(2-(2-((2-acetyl-5-((2-nitro-1-(phenylamino)vinyl)amino)isoindolinyl)carbonylamino)-2-carboxyethyl)phenyl)-2-(3,3-dimethylbutanoylamino)propanoic acid,
3-(2-(2-((2-acetyl-5-((1-((4-methylphenyl)amino)-2-nitrovinyl)amino)isoindolinyl)carbonylamino)-2-carboxyethyl)phenyl)-2-(3,3-dimethylbutanoylamino)propanoic acid,
3-(2-(2-((2-acetyl-5-((1-((4-nitrophenyl)amino)-2-nitrovinyl)amino)isoindolinyl)carbonylamino)-2-carboxyethyl)phenyl)-2-(3,3-dimethylbutanoylamino)propanoic acid,
3-(2-(2-((2-acetyl-5-((1-((4-methoxyphenyl)amino)-2-nitrovinyl)amino)isoindolinyl)carbonylamino)-2-carboxyethyl) phenyl)-2-(3,3-dimethylbutanoylamino)propanoic acid,
3-(2-(2-((2-acetyl-5-((1-((4-hydroxyphenyl)amino)-2-nitrovinyl)amino)isoindolinyl)carbonylarnino)-2-carboxyethyl) phenyl)-2-(3,3-dimethylbutanoylarnino)propanoic acid,
3-(2-(2-((2-acetyl-5-((1-((4-chlorophenyl)amino)-2-nitrovinyl)amino)isoindolinyl)carbonylamino)-2-carboxyethyl)phenyl)-2-(3,3-dimethylbutanoylamino)propanoic acid,
3-(2-(2-((2-acetyl-5-((2-aza-2-cyano-1-(phenylamino)vinyl)amino)isoindolinyl)carbonylamino)-2-carboxyethyl)phenyl)-2-(3,3-dimethylbutanoylamino)propanoic acid,
3-(2-(2-((2-acetyl-5-((2-aza-2-cyano-1-((4-methylphenyl)amino)vinyl)amino)isoindolinyl)carbonylamino)-2-carboxyethyl)phenyl)-2-(3,3-dimethylbutanoylamino)propanoic acid,
3-(2-(2-((2-acetyl-5-((2-aza-2-cyano-1-((4-nitrophenyl)amino)vinyl)amino)isoindolinyl)carbonylamino)-2-carboxyethyl)phenyl)-2-(3,3-dimethylbutanoylamino)propanoic acid,
3-(2-(2-((2-acetyl-5-((2-aza-2-cyano-1-((4-methoxyphenyl)amino)vinyl)amino)isoindolinyl)carbonylamino)-2-carboxyethyl)phenyl)-2-(3,3-dimethylbutanoylamino)propanoic acid,
3-(2-(2-((2-acetyl-5-((2-aza-2-cyano-1-((4-hydroxyphenyl)amino)vinyl)amino)isoindolinyl)carbonylamino)-2-carboxyethyl)phenyl))-2-(3,3-dimethylbutanoylamino)propanoic acid,
3-(2-(2-((2-acetyl-5-((2-aza-2-cyano-1-((4-chlorophenyl)amino)vinyl)amino)isoindolinyl)carbonylamino)-2-carboxyethyl)phenyl)-2-(3,3-dimethylbutanoylamino)propanoic acid,
3-(2-(2-carboxy-2-((5-hydroxy-2-methyl-1,3,4-trihydroisoquinolyl)carbonylamino)ethyl)phenyl)-2-(3,3-dimethylbutanoylamino) propanoic acid,
3-(2-(2-carboxy-2-((6-hydroxy-2-methyl-1,3,4-trihydroisoquinolyl)carbonylamino)ethyl)phenyl)-2-(3,3-dimethylbutanoylamino) propanoic acid,
3-(2-(2-carboxy-2-((7-hydroxy-2-methyl-1,3,4-trihydroisoquinolyl)carbonylamino)ethyl)phenyl)-2-(3,3-dimethylbutanoylamino) propanoic acid,
3-(2-(2-carboxy-2-((5-methoxy-2-methyl-1,3,4-trihydroisoquinolyl)carbonylamino)ethyl)phenyl)-2-(3,3-dimethylbutanoylamino) propanoic acid,
3-(2-(2-carboxy-2-((6-methoxy-2-methyl-1,3,4-trihydroisoquinolyl)carbonylamino)ethyl)phenyl)-2-(3,3-dimethylbutanoylamino) propanoic acid,
3-(2-(2-carboxy-2-((7-methoxy-2-methyl-1,3,4-trihydroisoquinolyl)carbonylamino)ethyl)phenyl)-2-(3,3-dimethylbutanoylamino) propanoic acid,
3-(2-(2-((2-acetyl-4-hydroxyisoindolinyl)carbonylamino)-2-carboxyethyl)phenyl)-2-(3,3-dimethylbutanoylamino)propanoic acid,
3-(2-(2-((2-acetyl-5-hydroxyisoindolinyl)carbonylamino)-2-carboxyethyl)phenyl)-2-(3,3-dimethylbutanoylamino)propanoic acid,
3-(2-(2-((2-acetyl-6-hydroxyisoindolinyl)carbonylamino)-2-carboxyethyl)phenyl)-2-(3,3-dimethylbutanoylamino)propanoic acid,
3-(2-(2-((2-acetyl-4-methoxyisoindolinyl)carbonylamino)-2-carboxyethyl)phenyl)-2-(3,3-dimethylbutanoylamino)propanoic acid,
3-(2-(2-((2-acetyl-5-methoxyisoindolinyl)carbonylamino)-2-carboxyethyl)phenyl)-2-(3,3-dimethylbutanoylamino)propanoic acid,
3-(2-(2-((2-acetyl-6-methoxyisoindolinyl)carbonylamino)-2-carboxyethyl)phenyl)-2-(3,3-dimethylbutanoylamino)propanoic acid,
3-(2-(2-((2-acetyl-5-((phenylamino)carbonylamino)isoindolinyl)carbonylamino)-2-carboxyethyl)phenyl)-2-(3,3-dimethylbutanoylamino)propanoic acid,
3-(2-(2-((2-acetyl-5-(((4-methylphenyl)amino)carbonylamino)isoindolinyl)carbonylamino)-2-carboxyethyl)phenyl)-2-(3,3-dimethylbutanoylamino)propanoic acid,
3-(2-(2-((2-acetyl-5-(((4-nitrophenyl)amino)carbonylamino)isoindolinyl)carbonylamino)-2-carboxyethyl)phenyl)-2-(3,3-dimethylbutanoylamino)propanoic acid,
3-(2-(2-((2-acetyl-5-(((4-methoxyphenyl)amino)carbonylamino)isoindolinyl)carbonylamino)-2-carboxyethyl}phenyl)-2-(3,3-dimethylbutanoylamino)propanoic acid,
3-(2-(2-((2-acetyl-5-(((4-hydroxyphenyl)amino)carbonylamino)isoindolinyl)carbonylamino)-2-carboxyethyl)phenyl)-2-(3,3-dimethylbutanoylamino)propanoic acid,
3-(2-(2-((2-acetyl-5-(((4-chlorophenyl)amino)carbonylamino)isoindolinyl)carbonylamino)-2-carboxyethyl)phenyl)-2-(3,3-dimethylbutanoylamino)propanoic acid,
3-(2-(2-((2-acetyl-5-(((phenylamino)thioxomethyl)amino)isoindolinyl)carbonylamino)-2-carboxyethyl)phenyl)-2-(3,3-dimethylbutanoylamino)propanoic acid,
3-(2-(2-((2-acetyl-5-(2-phenylacetylamino)isoindolinyl)carbonylamino)-2-carboxyethyl)phenyl)-2-(3,3-dimethylbutanoylamino)propanoic acid,
3-(2-(2-((2-acetyl-5-(2-(4-methylphenyl)acetylamino)isoindolinyl)carbonylamino)-2-carboxyethyl)phenyl)-2-(3,3-dimethylbutanoylamino)propanoic acid,
3-(2-(2-((2-acetyl-5-(2-(4-nitrophenyl)acetylamino)isoindolinyl)carbonylamino)-2-carboxyethyl)phenyl)-2-(3,3-dimethylbutanoylamino)propanoic acid,
3-(2-(2-((2-acetyl-5-(2-(4-methoxyphenyl)acetylamino)isoindolinyl)carbonylamino)-2-carboxyethyl)phenyl)-2-(3,3-dimethylbutanoylamino)propanoic acid,
3-(2-(2-((2-acetyl-5-(2-(4-hydroxyphenyl)acetylamino)isoindolinyl)carbonylamino)-2-carboxyethyl)phenyl)-2-(3,3-dimethylbutanoylamino)propanoic acid,
3-(2-(2-((2-acetyl-5-(2-(4-chlorophenyl)acetylamino)isoindolinyl)carbonylamino)-2-carboxyethyl)phenyl)-2-(3,3-dimethylbutanoylamino)propanoic acid,
3-(2-(2-((2-acetyl-5-((2-phenyl-1-thioxoethyl)amino)isoindolinyl)carbonylamino)-2-carboxyethyl)phenyl)-2-(3,3-dimethylbutanoylamino)propanoic acid,
3-(2-(2-((1-acetyl-4-(4-((phenylamino)carbonylamino)phenyl)pyrrolidin-2-yl)carbonylamino)-2-carboxyethyl)phenyl)-2-(3,3-dimethylbutanoylamino)propanoic acid,
3-(2-(2-((1-acetyl-4-(4-(((4-methylphenyl)amino)carbonylamino)phenyl)pyrrolidin-2-yl)carbonylamino)-2-carboxyethyl)phenyl)-2-(3,3-dimethyibutanoylamino) propanoic acid,
3-(2-(2-((1-acetyl-4-(4-(((4-nitrophenyl)amino)carbonylamino)phenyl)pyrrolidin-2-yl)carbonylamino)-2-carboxyethyl) phenyl)-2-(3,3-dimethylbutanoylamino)propanoic acid,
3-(2-(2-((1-acetyl-4-(4-(((4-methoxyphenyl)amino)carbonylamino)phenyl)pyrrolidin-2-yl)carbonylamino)-2-carboxyethyl)phenyl)-2-(3,3-dimethylbutanoylamino)propanoic acid, 3-(2-(2-((1-acetyl-4-(4-(((4-hydroxyphenyl)amino)carbonylamino)phenyl)pyrrolidin-2-yl)carbonylamino)-2-carboxyethyl)phenyl)-2-(3,3-dimethylbutanoylamino)propanoic acid,
3-(2-(2-((1-acetyl-4-(4-(((4-chlorophenyl)amino)carbonylamino)phenyl)pyrrolidin-2-yl)carbonylamino)-2-carboxyethyl) phenyl)-2-(3,3-dimethylbutanoylamino) propanoic acid,

3-(2-(2-((1-acetyl-4-(4-(((phenylamino)thioxomethyl)amino)phenyl)pyrrolidin-2-yl)carbonylamino)-2-carboxyethyl) phenyl)-2-(3,3-dimethylbutanoylamino)propanoic acid,
4-(2-carboxy-2-((1-methyl-4-(4-((phenylamino)carbonylamino)phenyl)pyrrolidin-2-yl)carbonylamino)ethoxy)-2-(3,3-dimethylbutanoylamino)butanoic acid,
4-(2-carboxy-2-((1-methyl-4-(4-(((4-methylphenyl)amino)carbonylamino)phenyl)pyrrolidin-2-yl)carbonylamino) ethoxy)-2-(3,3-dimethylbutanoylamino)butanoic acid,
4-(2-carboxy-2-((1-methyl-4-(4-(((4-nitrophenyl)amino)carbonylamino)phenyl)pyrrolidin-2-yl)carbonylamino)ethoxy)-2-(3,3-dimethylbutanoylamino)butanoic acid,
4-(2-carboxy-2-((1-methyl-4-(4-(((4-methoxyphenyl)amino)carbonylamino)phenyl)pyrrolidin-2-yl)carbonylamino) ethoxy)-2-(3,3-dimethylbutanoylamino)butanoic acid,
4-(2-carboxy-2-((4-(4-(((4-hydroxyphenyl)amino)carbonylamino)phenyl)-1-methylpyrrolidin-2-yl)carbonylamino) ethoxy)-2-(3,3-dimethylbutanoylamino) butanoic acid,
4-(2-carboxy-2-((4-(4-(((4-chlorophenyl)amino)carbonylamino)phenyl)-1-methylpyrrolidin-2-yl)carbonylamino) ethoxy)-2-(3,3-dimethylbutanoylamino) butanoic acid,
4-(2-carboxy-2-((1-methyl-4-(4-(((phenylamino)thioxomethyl)amino)phenyl)pyrrolidin-2-yl)carbonylamino)ethoxy)-2-(3,3-dimethylbutanoylamino)butanoic acid,
4-(2-((1-acetyl-4-(4-(phenylamino)carbonylamino)phenyl)pyrrolidin-2-yl)carbonylamino)-2-carboxyethoxy)-2-(3,3-dimethylbutanoylamino)butanoic acid,
4-(2-((-1-acetyl-4-(4-(((4-methylphenyl)amino)carbonylamino)phenyl)pyrrolidin-2-yl)carbonylamino)-2-carboxyethoxy)-2-(3,3-dimethylbutanoylamino)butanoic acid,
4-(2-((1-acetyl-4-(4-(((4-nitrophenyl)amino)carbonylamino)phenyl)pyrrolidin-2-yl)carbonylamino)-2-carboxyethoxy)-2-(3,3-dimethylbutanoylamino)butanoic acid,
4-(2-((1-acetyl-4-(4-(((4-methoxyphenyl)amino)carbonylamino)phenyl)pyrrolidin-2-yl)carbonylamino)-2-carboxyethoxy)-2-(3,3-dimethylbutanoylamino)butanoic acid,
4-(2-((-1-acetyl-4-(4-(((4-hydroxyphenyl)amino)carbonylamino)phenyl)pyrrolidin-2-yl)carbonylamino)-2-carboxyethoxy)-2-(3,3-dimethylbutanoylamino)butanoic acid,
4-(2-((1-acetyl-4-(4-(((4-chlorophenyl)amino)carbonylamino)phenyl)pyrrolidin-2-yl)carbonylamino)-2-carboxyethoxy)-2-(3,3-dimethylbutanoylamino)butanoic acid,
4-(2-((1-acetyl-4-(4-(((phenylamino)thioxomethyl)amino)phenyl)pyrrolidin-2-yl)carbonylamino)-2-carboxyethoxy)-2-(3,3-dimethylbutanoylamino)butanoic acid,
6-(2-(2-(4-((2-aza-2-cyano-1-(phenylamino)vinyl)amino)phenyl )acetylamino)-4-methylpentanoylamino)-1-(3,3-dimethylbutanoylamino)hex-3-yne-1,6-dicarboxylic acid,
6-(2-(2-(4-((2-aza-2-cyano-1-((4-methylphenyl)amino)vinyl)amino)phenyl)acetylamino)-4-methylpentanoylamino)-1-(3,3-dimethylbutanoylamino)hex-3-yne-1,6-dicarboxylic acid,
6-(2-(2-(4-((2-aza-2-cyano-1-((4-nitrophenyl)amino)vinyl)amino)phenyl)acetylamino)-4-methylpentanoylamino)-1-(3,3-dimethylbutanoylamino)hex-3-yne-1,6-dicarboxylic acid,
6-(2-(2-(4-((2-aza-2-cyano-1-((4-methoxyphenyl)amino)vinyl)amino)phenyl)acetylamino)-4-methylpentanoylamino)-1-(3,3-dimethylbutanoylamino)hex-3-yne-1,6-dicarboxylic acid,
6-(2-(2-(4-((2-aza-2-cyano-1-((4-hydroxyphenyl)amino)vinyl)amino)phenyl)acetylamino)-4-methylpentanoylamino)-1-(3,3-dimethylbutanoylamino)hex-3-yne-1,6-dicarboxylic acid,
6-(2-(2-(4-((2-aza-2-cyano-1-((4-nitrophenyl)amino)vinyl)amino)phenyl)acetylamino)-4-methylpentanoylamino)-1-(3,3-dimethylbutanoylamino)hex-3-yne-1,6-dicarboxylic acid,
1-(3,3-dimethylbutanoylamino)-6-(4-methyl-2-(2-(4-((2-nitro-1-(phenylamino)vinyl)amino)phenyl)acetylamino)pentanoylamino)hex-3-yne-1,6-dicarboxylic acid,
1-(3,3-dimethylbutanoylamino)-6-(4-methyl-2-(2-(4-((1-((4-methylphenyl)amino)-2-nitrovinyl)amino)phenyl)acetylamino)pentanoylamino)hex-3-yne-1,6-dicarboxylic acid,
1-(3,3-dimethylbutanoylamino)-6-(4-methyl-2-(2-(4-((2-nitro-1-((4-nitrophenyl)amino)vinyl)amino)phenyl)acetylamino)pentanoylamino)hex-3-yne-1,6-dicarboxylic acid,
1-(3,3-dimethylbutanoylamino)-6-(4-methyl-2-(2-(4-((1-((4-methoxyphenyl)amino)-2-nitrovinyl)amino)phenyl) acetylamino)pentanoylamino)hex-3-yne-1,6-dicarboxylic acid,
6-(2-(2-(4-((1-((4-hydroxyphenyl)amino)-2-nitrovinyl)amino)phenyl)acetylamino)-4-methylpentanoylamino)-1-(3,3-dimethylbutanoylamino)hex-3-yne-1,6-dicarboxylic acid,
6-(2-(2-(4-((1-((4-chlorophenyl)amino)-2-nitrovinyl)amino)phenyl)acetylamino)-4-methylpentanoylamino)-1-(3,3-dimethylbutanoylamino)hex-3-yne-1,6-dicarboxylic acid,
1-(3,3-dimethylbutanoylamino)-6-(4-methyl-2-(2-(4-((phenylamino)carbonylamino)phenyl)acetylamino)pentanoylamino)hex-3-yne-1,6-dicarboxylic acid,
1-(3,3 -dimethylbutanoylamino)-6-(4-methyl-2-(2-(4-(((4-methylphenyl)amino)carbonylamino)phenyl)acetylamino) pentanoylamino)hex-3-yne-1,6-dicarboxylic acid,

1-(3,3-dimethylbutanoylamino)-6-(4-methyl-2-(2-(4-(((4-nitrophenyl)amino)carbonylamino)phenyl)acetylamino)pentanoylamino)hex-3-yne- 1,6-dicarboxylic acid,
1-(3,3-dimethylbutanoylamino)-6-(4-methyl-2-(2-(4-(((4-methoxyphenyl)amino)carbonylamino)phenyl)acetylamino) pentanoylamino)hex-3-yne-1,6-dicarboxylic acid,
6-(2-(2-(4-(((4-hydroxyphenyl)amino)carbonylamino)phenyl)acetylamino)-4-methylpentanoylamino)-1-(3,3-dimethylbutanoylamino)hex-3 -yne-1,6-dicarboxylic acid,
6-(2-(2-(4-(((4-chlorophenyl)amino)carbonylamino)phenyl)acetylamino)-4-methylpentanoylamino)-1-(3,3-dimethylbutanoylamino)hex-3-yne-1,6-dicarboxylic acid,
1-(3,3-dimethylbutanoylamino)-6-(4-methyl-2-(2-(4-(((phenylamino)thioxomethyl)amino)phenyl)acetylamino)pentanoylamino)hex-3-yne-1,6-dicarboxylic acid,
3-(4-(2-(2-(2-(4-((2-aza-2-cyano-1-(phenylamino)vinyl)amino)phenyl)acetylamino)-4-methylpentanoylamino)-2-carboxyethyl)piperazinyl)-2-(3,3-dimethylbutanoylamino)propanoic acid,
3-(4-(2-(2-(2-(4-((2-aza-2-cyano-1-((4-methylphenyl)amino)vinyl)amino)phenyl)acetylamino)-4-methylpentanoylamino)-2-carboxyethyl)piperazinyl)-2-(3,3-dimethylbutanoylamino)propanoic acid,
3-(4-(2-(2-(2-(4-((2-aza-2-cyano-1-((4-nitrophenyl)amino)vinyl)amino)phenyl)acetylamino)-4-methylpentanoylamino)-2-carboxyethyl)piperazinyl)-2-(3,3-dimethylbutanoylamino)propanoic acid,
3-(4-(2-(2-(2-(4-((2-aza-2-cyano-1-((4-methoxyphenyl)amino)vinyl)amino)phenyl)acetylamino)-4-methylpentanoylamino)-2-carboxyethyl)piperazinyl)-2-(3,3-dimethylbutanoylamino)propanoic acid,
3-(4-(2-(2-(2-(4-((2-aza-2-cyano-1-((4-hydroxyphenyl)amino)vinyl)amino)phenyl)acetylamino)-4-methylpentanoylamino)-2-carboxyethyl)piperazinyl)-2-(3,3-dimethylbutanoylamino)propanoic acid,
3-(4-(2-(2-(2-(4-((2-aza-2-cyano-1-((4-chlorophenyl)amino)vinyl)amino)phenyl)acetylamino)-4-methylpentanoylamino)-2-carboxyethyl)piperazinyl)-2-(3,3-dimethylbutanoylamino)propanoic acid,
3-(4-(2-carboxy-2-(4-methyl-2-(2-(4-((2-nitro-1-(phenylamino)vinyl)amino)phenyl)acetylamino)pentanoylamino)ethyl) piperazinyl)-2-(3,3-dimethylbutanoylamino)propanoic acid,
3-(4-(2-carboxy-2-(4-methyl-2-(2-(4-((1-((4-methylphenyl)amino)-2-nitrovinyl)amino)phenyl)acetylamino)pentanoylamino)ethyl)piperazinyl)-2-(3,3-dimethylbutanoylamino)propanoic acid,
3-(4-(2-carboxy-2-(4-methyl-2-(2-(4-((2-nitro-1-((4-nitrophenyl)amino)vinyl)amino)phenyl)acetylamino)pentanoylamino)ethyl)-piperazinyl)-2-(3,3-dimethylbutanoylamino)propanoic acid,
3-(4-(2-carboxy-2-(4-methyl-2-(2-(4-((1-((4-methoxyphenyl)amino)-2-nitrovinyl)amino)phenyl)acetylamino)pentanoylamino)ethyl)piperazinyl)-2-(3,3-dimethylbutanoylamino)propanoic acid,
3-(4-(2-carboxy-2-(2-(2-(4-((1-((4-hydroxyphenyl)amino)-2-nitrovinyl)amino)phenyl)acetylamino)-4-methylpentanoylamino)ethyl)piperazinyl)-2-(3,3-dimethylbutanoylamino)propanoic acid,
3-(4-(2-carboxy-2-(2-(2-(4-((-((4-chlorophenyl)amino)-2-nitrovinyl)amino)phenyl)acetylamino)-4-methylpentanoylamino)ethyl)piperazinyl)-2-(3,3-dimethylbutanoylamino)propanoic acid,
3-(4-(2-carboxy-2-(4-methyl-2-(2-(4-((phenylamino)carbonylamino)phenyl)acetylamino)pentanoylamino)ethyl)piperaziny 1)-2-(3,3-dimethylbutanoylamino)propanoic acid,
3-(4-(2-carboxy-2-(4-methyl-2-(2-(4-(((4-methylphenyl)amino)carbonylamino)phenyl)acetylamino)pentanoylamino) ethyl)-piperazinyl)-2-(3,3-dimethylbutanoylamino)propanoic acid,
3-(4-(2-carboxy-2-(4-methyl-2-(2-(4-(((4-nitrophenyl)amino)carbonylamino)phenyl)acetylamino)pentanoylamino) ethyl)-piperazinyl)-2-(3,3-dimethylbutanoylamino)propanoic acid,
3-(4-(2-carboxy-2-(4-methyl-2-(2-(4-(((4-methoxyphenyl)amino)carbonylamino)phenyl)acetylamino)pentanoylamino) ethyl)-piperazinyl)-2-(3,3-dimethylbutanoylamino)propanoic acid,
3-(4-(2-carboxy-2-(2-(2-(4-(((4-hydroxyphenyl)amino)carbonylamino)phenyl)acetylamino)-4-methylpentanoylamino) ethyl)piperazinyl)-2-(3,3-dimethylbutanoylamino)propanoic acid,
3-(4-(2-carboxy-2-(2-(2-(4-(((4-chlorophenyl)amino)carbonylamino)phenyl)acetylamino)-4-methylpentanoylamino) ethyl)piperazinyl)-2-(3,3-dimethylbutanoylamino)propanoic acid,
3-(4-(2-carboxy-2-(4-methyl-2-(2-(4-(((phenylamino)thioxomethyl)amino)phenyl)acetylamino)pentanoylamino)ethyl) piperazinyl)-2-(3,3-dimethylbutanoylamino)propanoic acid,
3-(4-(2-carboxyethyl)phenyl)-2-(4-methyl-2-(4-((phenylamino)carbonylamino)phenyl)pentanoylamino)propanoic acid,
3-(4-(2-carboxyethyl)phenyl)-2-(4-methyl-2-(4-(((4-methylphenyl)amino)carbonylamino)phenyl)pentanoylamino)propanoic acid,
3-(4-(2-carboxyethyl)phenyl)-2-(4-methyl-2-(4-(((4-nitrophenyl)amino)carbonylamino)phenyl)pentanoylamino)propanoic acid,
3-(4-(2-carboxyethyl)phenyl)-2-(4-methyl-2-(4-(((4-methoxyphenyl)amino)carbonylamino)phenyl)pentanoylamino) propanoic acid,
3-(4-(2-carboxyethyl)phenyl)-2-(2-(4-(((4-hydroxyphenyl)amino)carbonylamino)phenyl)-4-methylpentanoylamino) propanoic acid,
3-(4-(2-carboxyethyl)phenyl)-2-(2-(4-(((4-chlorophenyl)amino)carbonylamino)phenyl)-4-methylpentanoylamino)propanoic acid,
3-(4-(2-carboxyethyl)phenyl)-2-(4-methyl-2-(4-(((phenylamino)thioxomethyl)amino)phenyl)pentanoylamino)propanoic acid,
2-(2-(4-((2-aza-2-cyano-1-(phenylamino)vinyl)amino)phenyl)-4-methylpentanoylamino)-3-(4-(2-carboxyethyl)phenyl) propanoic acid,
2-(2-(4-((2-aza-2-cyano-1-((4-methylphenyl)amino)vinyl)amino)phenyl)-4-methylpentanoylamino)-3-(4-(2-carboxyethyl)phenyl)propanoic acid,
2-(2-(4-((2-aza-2-cyano-1-((4-nitrophenyl)amino)vinyl)amino)phenyl)-4-methylpentanoylamino)-3-(4-(2-carboxyethyl) phenyl)propanoic acid,
2-(2-(4-((2-aza-2-cyano-1-((4-methoxyphenyl)amino)vinyl)amino)phenyl)-4-methylpentanoylamino)-3-(4-(2-carboxyethyl)phenyl)propanoic acid,
2-(2-(4-((2-aza-2-cyano-1-((4-hydroxyphenyl)amino)vinyl)amino)phenyl)-4-methylpentanoylamino)-3-(4-(2-carboxyethyl)phenyl)propanoic acid,
2-(2-(4-((2-aza-2-cyano-1-((4-chlorophenyl)amino)vinyl)amino)phenyl)-4-methylpentanoylamino)-3-(4-(2-carboxyethyl)phenyl)propanoic acid,
3-(4-(2-carboxyethyl)phenyl)-2-(4-methyl-2-(4-((2-nitro-1-(phenylamino)vinyl)amino)phenyl)pentanoylamino)propanoic acid,
3-(4-(2-carboxyethyl)phenyl)-2-(4-methyl-2-(4-((1-((4-methylphenyl)amino)-2-nitrovinyl)amino)phenyl)pentanoylamino)propanoic acid,
3-(4-(2-carboxyethyl)phenyl)-2-(4-methyl-2-(4-((2-nitro-1-((4-nitrophenyl)amino)vinyl)amino)phenyl)pentanoylamino) propanoic acid,
3-(4-(2-carboxyethyl)phenyl)-2-(4-methyl-2-(4-((1-((4-methoxyphenyl)amino)-2-nitrovinyl)amino)phenyl)pentanoylamino)propanoic acid,
3-(4-(2-carboxyethyl)phenyl)-2-(2-{4-((1-((4-hydroxyphenyl)amino)-2-nitrovinyl)amino)phenyl)-4-methylpentanoylamino)propanoic acid,
3-(4-(2-carboxyethyl)phenyl)-2-(2-(4-((1-((4-chlorophenyl)amino)-2-nitrovinyl)amino)phenyl)-4-methylpentanoylamino)propanoic acid,
3-(4-(2-carboxyethyl)phenyl)-2-(4-methyl-2-(2-(4-((phenylamino)carbonylamino)phenyl)acetylamino)pentanoylamino)propanoic acid,
3-(4-(2-carboxyethyl)phenyl)-2-(4-methyl-2-(2-(4-(((4-methylphenyl)amino)carbonylamino)phenyl)acetylamino)pentanoylamino)propanoic acid,
3-(4-(2-carboxyethyl)phenyl)-2-(4-methyl-2-(2-(4-(((4-nitrophenyl)amino)carbonylamino)phenyl)acetylamino)pentanoylamino)propanoic acid,
3-(4-(2-carboxyethyl)phenyl)-2-(4-methyl-2-(2-(4-(((4-methoxyphenyl)amino)carbonylamino)phenyl)acetylamino) pentanoylamino) propanoic acid,
3-(4-(2-carboxyethyl)phenyl)-2-(2-(4-(((4-hydroxyphenyl)amino)carbonylamino)phenyl)acetylamino)-4-methylpentanoylamino)propanoic acid,
3-(4-(2-carboxyethyl)phenyl)-2-(2-(4-(((4-chlorophenyl)amino)carbonylamino)phenyl)acetylamino)-4-methylpentanoylamino) propanoic acid,
3-(4-(2-carboxyethyl)phenyl)-2-(4-methyl-2-(2-(4-(((phenylamino)thioxomethyl)amino)phenyl)acetylamino)pentanoylamino)propanoic acid,
2-(2-(2-(4-((2-aza-2-cyano-1-(phenylamino)vinyl)amino)phenyl)acetylamino)-4-methylpentanoylamino)-3-(4-(2-carboxyethyl)phenyl)propanoic acid,
2-(2-(2-(4-((2-aza-2-cyano-1-((4-methylphenyl)amino)vinyl)amino)phenyl)acetylamino)-4-methy]pentanoylamino)-3-(4-(2-carboxyethyl)phenyl)propanoic acid,
(2-(2-(4-((2-aza-2-cyano-1-((4-nitrophenyl)amino)vinyl)amino)phenyl)acetylamino)-4-methylpentanoylamino)-3-(4-(2-carboxyethyl)phenyl)propanoic acid,
2-(2-(2-(4-((2-aza-2-cyano-1-((4-methoxyphenyl)amino)vinyl)amino)phenyl)acetylamino)-4-methylpentanoylamino)-3-(4-(2-carboxyethyl)phenyl)propanoic acid,
2-(2-(2-(4-((2-aza-2-cyano-1-((4-hydroxyphenyl)amino)vinyl)amino)phenyl)acetylamino)-4-methylpentanoylamino)-3-(4-(2-carboxyethyl)phenyl)propanoic acid,
2-(2-(2-(4-((2-aza-2-cyano-1-((4-chlorophenyl)amino)vinyl)amino)phenyl)acetylamino)-4-methylpentanoylamino)-3-(4-(2-carboxyethyl)phenyl)propanoic acid,
3-(4-(2-carboxyethyl)phenyl)-2-(4-methyl-2-(2-(4-((2-nitro-1-(phenylamino)vinyl)amino)phenyl)acetylamino)pentanoylamino)propanoic acid,
3-(4-(2-carboxyethyl)phenyl)-2-(4-methyl-2-(2-(4-((1-((4-methylphenyl)amino)-2-nitrovinyl)amino)phenyl)acetylamino)pentanoylamino)propanoic acid,
3-(4-(2-carboxyethyl)phenyl)-2-(4-methyl-2-(2-(4-((2-nitro-1-((4-nitrophenyl)amino)vinyl)amino)phenyl)acetylamino)pentanoylamino)propanoic acid,
3-(4-(2-carboxyethyl)phenyl)-2-(4-methyl-2-(2-(4-(( 1-((4-methoxyphenyl)amino)-2-nitrovinyl)amino)phenyl)acetylamino)pentanoylamino)propanoic acid,
3-(4-(2-carboxyethyl)phenyl)-2-(2-(2-(4-((1-((4-hydroxyphenyl)amino)-2-nitrovinyl)amino)phenyl)acetylamino)-4-methylpentanoylamino)propanoic acid,
3-(4-(2-carboxyethyl)phenyl)-2-(2-(2-(4-((1-((4-chlorophenyl)amino)-2-nitrovinyl)amino)phenyl)acetylamino)-4-methylpentanoylamino)propanoic acid,
3 -(4-(2-carboxyethyl)phenyl)-2-((1-methyl-4-(4-((phenylamino)carbonylamino)phenyl)pyrrolidin-2-yl)carbonylamino) propanoic acid,
3-(4-(2-carboxyethyl)phenyl)-2-((1-methyl-4-(4-(((4-methylphenyl)amino)carbonylamino)phenyl)pyrrolidin-2-yl)carbonylamino) propanoic acid,
3-(4-(2-carboxyethyl)phenyl)-2-((1-methyl-4-(4-(((4-nitrophenyl)amino)carbonylamino)phenyl)pyrrolidin-2-yl)carbonylamino)propanoic acid,
3-(4-(2-carboxyethyl)phenyl)-2-((1-methyl-4-(4-(((4-methoxyphenyl)amino)carbonylamino)phenyl)pyrrolidin-2-yl)carbonylamino) propanoic acid,
3-(4-(2-carboxyethyl)phenyl)-2-((4-(4-(((4-hydroxyphenyl)amino)carbonylamino)phenyl)-1-methylpyrrolidin-2-yl)carbonylamino)propanoic acid,
3-(4-(2-carboxyethyl)phenyl)-2-((4-(4-(((4-chlorophenyl)amino)carbonylamino)phenyl)-1-methylpyrrolidin-2-yl)carbonylamino)propanoic acid,
3-(4-(2-carboxyethyl)phenyl)-2-((1-methyl-4-(4-(((phenylamino)thioxomethyl)amino)phenyl)pyrrolidin-2-yl)carbonylamino) propanoic acid,
2-((1-acetyl-4-(4-((phenylamino)carbonylamino)phenyl)pyrrolidin-2-yl)carbonylamino)-3-(4-(2-carboxyethyl)phenyl) propanoic acid,
2-((1-acetyl-4-(4-(((4-methylphenyl)amino)carbonylamino)phenyl)pyrrolidin-2-yl)carbonylamino)-3-(4-(2-carboxyethyl)phenyl)propanoic acid,
2-((1-acetyl-4-(4-(((4-nitrophenyl)amino)carbonylamino)phenyl)pyrrolidin-2-yl)carbonylamino)-3-(4-(2-carboxyethyl) phenyl)propanoic acid,
2-((1-acetyl-4-(4-(((4-methoxyphenyl)amino)carbonylamino)phenyl)pyrrolidin-2-yl)carbonylamino)-3-(4-(2-carboxyethyl)phenyl)propanoic acid,
2-((1-acetyl-4-(4-(((4-hydroxyphenyl)amino)carbonylamino)phenyl)pyrrolidin-2-yl)carbonylamino)-3-(4-(2-carboxyethyl)phenyl)propanoic acid,
2-((1-acetyl-4-(4-(((4-chlorophenyl)amino)carbonylamino)phenyl)pyrrolidin-2-yl)carbonylamino)-3-(4-(2-carboxyethyl) phenyl)propanoic acid,
2-((1-acetylA-(4-((phenylamino)thioxomethyl)amino)phenyl)pyrrolidin-2-yl)carbonylamino)-3-(4-(2-carboxyethyl)phenyl)propanoic acid,
3-(4-(2-carboxyethyl)phenyl)-2-((2-methyl-6-((phenylamino)carbonylamino)-1,3,4-trihydroisoquinolyl)carbonylamino) propanoic acid,
3-(4-(2-carboxyethyl)phenyl)-2-((2-methyl-6-(((4-methylphenyl)amino)carbonylamino)-1,3,4-trihydroisoquinolyl)carbonylamino) propanoic acid,
3-(4-(2-carboxyethyl)phenyl)-2-((2-methyl-6-(((4-nitrophenyl)amino)carbonylamino)-1,3,4-trihydroisoquinolyl)carbonylamino) propanoic acid,
3-(4-(2-carboxyethyl)phenyl)-2-((2-methyl-6-(((4-methoxyphenyl)amino)carbonylamino)-1,3,4-trihydroisoquinolyl)carbonylamino) propanoic acid,
3-(4-(2-carboxyethyl)phenyl)-2-((6-(((4-hydroxyphenyl)amino)carbonylamino)-2-methyl-1,3,4-trihydroisoquinolyl)carbonylamino)propanoic acid,
3-(4-(2-carboxyethyl)phenyl)-2-((6-(((4-chlorophenyl)amino)carbonylamino)-2-methyl-1,3,4-trihydroisoquinolyl)carbonylamino)propanoic acid,
3-(4-(2-carboxyethyl)phenyl)-2-((2-methyl-6-(((phenylamino)thioxomethyl)amino)-1,3,4-trihydroisoquinolyl)carbonylamino)propanoic acid,
2-((2-acetyl-6-((phenylamino)carbonylamino)-1,3,4-trihydroisoquinolyl)carbonylamino)-3-(4-(2-carboxyethyl)phenyl) propanoic acid,
2-((2-acetyl-6-(((4-methylphenyl)amino)carbonylamino)-1,3,4-trihydroisoquinolyl)carbonylamino)-3-(4-(2-carboxyethyl)phenyl)propanoic acid,
2-((2-acetyl-6-(((4-nitrophenyl)amino)carbonylamino)-1,3,4-trihydroisoquinolyl)carbonylamino)-3-(4-(2-carboxyethyl) phenyl)propanoic acid,
2-((2-acetyl-6-(((4-methoxyphenyl)amino)carbonylamino)-1,3,4-trihydroisoquinolyl)carbonylamino)-3-(4-(2-carboxyethyl)phenyl)propanoic acid,
2-((2-acetyl-6-(((4-hydroxyphenyl)amino)carbonylamino)-1,3,4-trihydroisoquinolyl)carbonylamino)-3-(4-(2-carboxyethyl)phenyl)propanoic acid,
2-((2-acetyl-6-(((4-chlorophenyl)amino)carbonylamino)-1,3,4-trihydroisoquinolyl)carbonylamino)-3-(4-(2-carboxyethyl)phenyl)propanoic acid,
2-((2-acetyl-6-(((phenylamino)thioxomethyl)amino)-1,3,4-trihydroisoquinolyl)carbonylamino)-3-(4-(2-carboxyethyl) phenyl)propanoic acid,
2-((6-((2-aza-2-cyano-1-(phenylamino)vinyl)amino)-2-methyl-1,3,4,-trihydroisoquinolyl)carbonylamino)-3-(4-(2-carboxyethyl)phenyl)propanoic acid,
2-((6-((2-aza-2-cyano-1-((4-methylphenyl)amino)vinyl)amino)-2-methyl-1,3,4,-trihydroisoquinolyl)carbonylamino)-3-(4-(2-carboxyethyl)phenyl)propanoic acid,
2-((6-((2-aza-2-cyano-1-((4-nitrophenyl)amino)vinyl)amino)-2-methyl-1,3,4,-trihydroisoquinolyl)carbonylamino)-3-(4-(2-carboxyethyl)phenyl)propanoic acid,
2-((6-((2-aza-2-cyano-1-((4-methoxyphenyl)amino)vinyl)amino)-2-methyl-1,3,4,-trihydroisoquinolyl)carbonylamino)-3-(4-(2-carboxyethyl)phenyl)propanoic acid,
2-((6-((2-aza-2-cyano-1-((4-hydroxyphenyl)amino)vinyl)amino)-2-methyl-1,3,4,-trihydroisoquinolyl)carbonylamino)-3-(4-(2-carboxyethyl)phenyl)propanoic acid,
2-((6-((2-aza-2-cyano-1-((4-chlorophenyl)amino)vinyl)amino)-2-methyl-1,3,4,-trihydroisoquinolyl)carbonylamino)-3-(4-(2-carboxyethyl)phenyl)propanoic acid,
2-((2-acetyl-6-((2-aza-2-cyano-1-(phenylamino)vinyl)amino)-1,3,4-trihydroisoquinolyl)carbonylamino)-3-(4-(2-carboxyethyl)phenyl)propanoic acid,
2-((2-acetyl-6-((2-aza-2-cyano-1-((4-methylphenyl)amino)vinyl)amino)-1,3,4-trihydroisoquinolyl)carbonylamino)-3-(4-(2-carboxyethyl)phenyl)propanoic acid,
2-((2-acetyl-6-((2-aza-2-cyano-1-((4-nitrophenyl)amino)vinyl)amino)-1,3,4-trihydroisoquinolyl)carbonylamino)-3-(4-(2-carboxyethyl)phenyl)propanoic acid,
2-((2-acetyl-6-((2-aza-2-cyano-1-((4-methoxyphenyl)amino)vinyl)amino)-1,3,4-trihydroisoquinolyl)carbonylamino)-3-(4-(2-carboxyethyl)phenyl)propanoic acid,
2-((2-acetyl-6-((2-aza-2-cyano-1-((4-hydroxyphenyl)amino)vinyl)amino)-1,3,4-trihydroisoquinolyl)carbonylamino)-3-(4-(2-carboxyethyl)phenyl)propanoic acid,
2-((2-acetyl-6-((2-aza-2-cyano-1-((4-chlorophenyl)amino)vinyl)amino)-1,3,4-trihydroisoquinolyl)carbonylamino)-3-(4-(2-carboxyethyl)phenyl)propanoic acid,
3-(4-(2-carboxyethyl)phenyl)-2-((2-methyl-6-((2-nitro-1-(phenylamino)vinyl)amino)-1,3,4-trihydroisoquinolyl)carbonylamino)propanoic acid,
3-(4-(2-carboxyethyl)phenyl)-2-((2-methyl-6-((1-((4-methylphenyl)amino)-2-nitrovinyl)amino)-1,3,4-trihydroisoquinolyl)carbonylamino)propanoic acid,
(4-(2-carboxyethyl)phenyl)-2-((2-methyl-6-((2-nitro-1-((4-nitrophenyl)amino)vinyl)amino)-1,3,4-trihydroisoquinolyl) carbonylamino)propanoic acid,
3-(4-(2-carboxyethyl)phenyl)-2-((2-methyl-6-((1-((4-methoxyphenyl)amino)-2-nitrovinyl)amino)-1,3,4-trihydroisoquinolyl)carbonylamino)propanoic acid,
3-(4-(2-carboxyethyl)phenyl)-2-((6-((1-((4-hydroxyphenyl)amino)-2-nitrovinyl)amino)-2-methyl-1,3,4-trihydroisoquinolyl)carbonylamino)propanoic acid,
3-(4-(2-carboxyethyl)phenyl)-2-((6-((1-((4-chlorophenylamino)-2-nitrovinyl)amino)-2-methyl-1,3,4-trihydroisoquinolyl) carbonylamino)propanoic acid,
2-((2-acetyl-6-((2-nitro-1-(phenylamino)vinyl)amino)-1,3,4-trihydroisoquinolyl)carbonylamino)-3-(4-(2-carboxyethyl) phenyl)propanoic acid,
2-((2-acetyl-6-((1-((4-methylphenyl)amino)-2-nitrovinyl)amino)-1,3,4-trihydroisoquinolyl)carbonylamino)-3-(4-(2-carboxyethyl)phenyl)propanoic acid,
2-((2-acetyl-6-((2-nitro-1-(4-nitrophenyl)amino)vinyl)amino)-1,3,4-trihydroisoquinolyl)carbonylamino)-3-(4-(2-carboxyethyl)phenyl)propanoic acid,
2-((2-acetyl-6-((1-((4-hydroxyphenyl)amino)-2-nitrovinyl)amino)-1,3,4-trihydroisoquinolyl)carbonylamino)-3-(4-(2-carboxyethyl)phenyl)propanoic acid,
2-((2-acetyl-6-((1-((4-chlorophenyl)amino)-2-nitrovinyl)amino)-1,3,4-trihydroisoquinolyl)carbonylamino)-3-(4-(2-carboxyethyl)phenyl)propanoic acid,
3-(4-(2-carboxyethyl)phenyl)-2-((2-methyl-5-((phenylamino)carbonylamino)isoindolinyl)carbonylamino)propanoic acid,
3-(4-(2-carboxyethyl)phenyl)-2-((2-methyl-5-(((4-methylphenyl)amino)carbonylamino)isoindolinyl)carbonylamino)propanoic acid,
3-(4-(2-carboxyethyl)phenyl)-2-((2-methyl-5-(((4-nitrophenyl)amino)carbonylamino)isoindolinyl)carbonylamino)propanoic acid,
3-(4-(2-carboxyethyl)phenyl)-2-((2-methyl-5-(((4-methoxyphenyl)amino)carbonylamino)isoindolinyl)carbonylamino)propanoic acid,
3-(4-(2-carboxyethyl)phenyl)-2-((5-(((4-hydroxyphenyl)amino)carbonylamino)-2-methylisoindolinyl)carbonylamino) propanoic acid,
3-(4-(2-carboxyethyl)phenyl)-2-((5-(((4-chlorophenyl)amino)carbonylamino)-2-methylisoindolinyl)carbonylamino)propanoic acid,
3-(4-(2-carboxyethyl)phenyl)-2-((2-methyl-5-(((phenylamino)thioxomethyl)amino)isoindolinyl)carbonylamino)propanoic acid,
2-((2-acetyl-5-((phenylamino)carbonylamino)isoindolinyl)carbonylamino)-3-(4-(2-carboxyethyl)phenyl)propanoic acid,
2-((2-acetyl-5-(((4-methylphenyl)amino)carbonylamino)isoindolinyl)carbonylamino)-3-(4-(2-carboxyethyl)phenyl)propanoic acid,
2-((2-acetyl-5-(((4-nitrophenyl)amino)carbonylamino)isoindolinyl)carbonylamino)-3-(4-(2-carboxyethyl)phenyl)propanoic acid,
2-((2-acetyl-5-(((4-methoxyphenyl)amino)carbonylamino)isoindolinyl)carbonylamino)-3-(4-(2-carboxyethyl)phenyl) propanoic acid,
2-((2-acetyl-5-(((4-hydroxyphenyl)amino)carbonylamino)isoindolinyl)carbonylamino)-3-(4-(2-carboxyethyl)phenyl) propanoic acid,
2-((2-acetyl-5-(((4-chlorophenyl)amino)carbonylamino)isoindolinyl)carbonylamino)-3-(4-(2-carboxyethyl)phenyl)propanoic acid,
2((2-acetyl-5-(((phenylamino)thioxomethyl)amino)isoindolinyl)carbonylamino)-3-(4-(2-carboxyethyl)phenyl)propanoic acid,
2-((5-((2-aza-2-cyano-1-(phenylamino)vinyl)amino)-2-methyl-isoindolinyl)carbonylamino)-3-(4-(2-carboxyethyl)phenyl)propanoic acid,
2-((5-((2-aza-2-cyano-1-((4-methylphenyl)amino)vinyl)amino)-2-methyl-isoindolinyl)carbonylamino)-3-(4-(2-carboxyethyl)phenyl)propanoic acid,
2-((5-((2-aza-2-cyano-1-((4-nitrophenyl)amino)vinyl)amino)-2-methyl-isoindolinyl)carbonylamino)-3-(4-(2-carboxyethyl)phenyl)propanoic acid,
2-((5-((2-aza-2-cyano-1-((4-methoxyphenyl)amino)vinyl)amino)-2-methyl-isoindolinyl)carbonylamino)-3-(4-(2-carboxyethyl)phenyl)propanoic acid,
2-((5-((2-aza-2-cyano-1-((4-hydroxyphenyl)amino)vinyl)amino)-2-methyl-isoindolinyl)carbonylamino)-3-(4-(2-carboxyethyl)phenyl)propanoic acid,
2-((5-((2-aza-2-cyano-1-((4-chlorophenyl)amino)vinyl)amino)-2-methyl-isoindolinyl)carbonylamino)-3-(4-(2-carboxyethyl)phenyl)propanoic acid,
2-((2-acetyl-5-((2-aza-2-cyano-1-(phenylamino)vinyl)amino)isoindolinyl)carbonylamino)-3-(4-(2-carboxyethyl)phenyl) propanoic acid,
2-((2-acetyl-5-((2-aza-2-cyano-1-((4-methylphenyl)amino)vinyl)amino)isoindolinyl)carbonylamino)-3-(4-(2-carboxyethyl)phenyl)propanoic acid,
2-((2-acetyl-5-((2-aza-2-cyano-1-((4-nitrophenyl)amino)vinyl)amino)isoindolinyl)carbonylamino)-3-(4-(2-carboxyethyl)phenyl)propanoic acid,
2-((2-acetyl-5-((2-aza-2-cyano-1-((4-methoxyphenyl)amino)vinyl)amino)isoindolinyl)carbonylamino)-3-(4-(2-carboxyethyl)phenyl)propanoic acid,
2-((2-acetyl-5-((2-aza-2-cyano-1-((4-hydroxyphenyl)amino)vinyl)amino)isoindolinyl)carbonylamino)-3-(4-(2-carboxyethyl)phenyl)propanoic acid,
2-((2-acetyl-5-((2-aza-2-cyano-1-((4-chlorophenyl)amino)vinyl)amino)isoindolinyl)carbonylamino)-3-(4-(2-carboxyethyl)phenyl)propanoic acid,
3-(4-(2-carboxyethyl)phenyl)-2-((2-methyl-5-((2-nitro-1-(phenylamino)vinyl)amino)isoindolinyl)carbonylamino)propanoic acid,
3-(4-(2-carboxyethyl)phenyl)-2-((2-methyl-5-((1-((4-methylphenyl)amino)-2-nitrovinyl)amino)isoindolinyl)carbonylamino)propanoic acid,
3-(4-(2-carboxyethyl)phenyl)-2-((2-methyl-5-((2-nitro-1-((4-nitrophenyl)amino)vinyl)amino)isoindolinyl)carbonylamino)propanoic acid,
3-(4-(2-carboxyethyl)phenyl)-2-((2-methyl-5-((1-((4-methoxyphenyl)amino)-2-nitrovinyl)amino)isoindolinyl)carbonylamino)propanoic acid,
3-(4-(2-carboxyethyl)phenyl)-2-((5-((1-((4-hydroxyphenyl)amino)-2-nitrovinyl)amino)-2-methylisoindolinyl)carbonylamino)propanoic acid,
3-(4-(2-carboxyethyl)phenyl)-2-((5-((1-((4-chlorophenyl)amino)-2-nitrovinyl)amino)-2-methylisoindolinyl)carbonylamino)propanoic acid,
2-((2-acetyl-5-((2-nitro-1-(phenylamino)vinyl)amino)isoindolinyl)carbonylamino)-3-(4-(2-carboxyethyl)phenyl)propanoic acid,
2-((2-acetyl-5-((1-((4-methylphenyl)amino)-2-nitrovinyl)amino)isoindolinyl)carbonylamino)-3-(4-(2-carboxyethyl)phenyl) propanoic acid,
2-((2-acetyl-5-((2-nitro-1-(4-nitrophenyl)amino)vinyl)amino)isoindolinyl)carbonylamino)-3-(4-(2-carboxyethyl)phenyl) propanoic acid,
2-((2-acetyl-5-((2-nitro-1-(4(methoxyphenyl)amino)vinyl)amino)isoindolinyl)carbonylamino)-3-(4-(2-carboxyethyl) phenyl)propanoic acid,
2-((2-acetyl-5-(( 1-((4-hydroxyphenyl)amino)-2-nitrovinyl)amino)isoindolinyl)carbonylamino)-3-(4-(2-carboxyethyl) phenyl) propanoic acid,
2-((2-acetyl-5-((1-((4-chlorophenyl)amino)-2-nitrovinyl)amino)isoindolinyl)carbonylamino)-3-(4-(2-carboxyethyl)phenyl) propanoic acid,
2-((2,6-dichlorophenyl)carbonylamino)-3-(4-((2,6-dichlorophenyl)amino)-2-oxo-1,3-diazinyl)propanoic acid,
2-((2,6-dichlorophenyl)carbonylamino)-3-(4-((2,6-dimethoxyphenyl)amino)-2-oxo-1,3-diazinyl)propanoic acid,
2-((2,6-dichlorophenyl)carbonylamino)-3- (4-( ((2,6-dichlorophenyl)methyl)amino)-2-oxo-1,3-diazinyl)propanoic acid,
2-((2,6-dichlorophenyl)carbonylamino)-3- (4-( ((2,6-dimethoxyphenyl)methyl)amino)-2-oxo-1,3-diazinyl)propanoic acid,
2-((2,6-dichlorophenyl)carbonylamino)-3-(4-((2,6-dichlorophenyl)carbonylamino)-2-oxo-1,3-diazinyl)propanoic acid,
2-((2,6-dichlorophenyl)carbonylamino)-3- (4- ((2,6-dimethoxyphenyl)carbonylamino)-2-oxo-1,3-diazinyl)propanoic acid,
2-((2,6-dichlorophenyl)carbonylamino)-3- (3- ((2,6-dichlorophenyl)-2-oxo-1,3-diazaperhydroinyl)propanoic acid,
2-((2,6-dichlorophenyl)carbonylamino)-3-(3-(2,6-dimethoxyphenyl)-2-oxo-1,3-diazaperhydroinyl)propanoic acid,
2-((2,6-dichlorophenyl)carbonylamino)-3- (3-(2,6-dichlorophenyl)-2-oxoimidazolizinyl)propanoic acid,
2-((2,6-dichlorophenyl)carbonylamino)-3-(3-(2,6-dimethoxyphenyl)-2-oxoimidazolizinyl)propanoic acid,
2-((2,6-dichlorophenyl)carbonylamino)-4- (3-(2,6-dichlorophenyl)-2-oxoimidazolizinyl)butanoic acid,
2-((2,6-dichlorophenyl)carbonylamino)-4- (3-(2,6-dimethoxyphenyl)-2-oxoimidazolizinyl)butanoic acid,
3-(3-(2-carboxy-2-((2,6-dichlorophenyl)carbonylamino)ethyl)-2-oxoimidazolizinyl)-2-((2,6-dichlophenyl)carbonylamino)propanoic acid,
3-(3-(2-carboxy-2-((2,6-dichlorophenyl)carbonylamino)ethyl)-2-oxoimidazolizinyl)-2-((2,6-dimethoxyphenyl)carbonylamino)propanoic acid,
4-(2-carboxy-2-((2,6-dichlorophenyl)carbonylamino)ethylthio)-2-((2,6-dichlorophenyl)carbonylamino)butanoic acid,
2-((2,6-chlorophenyl)carbonylamino)-5-(4-((2-chlrophenyl)piperazinyl) pentanoic acid,
2-((2,6-dichlorophenyl)carbonylamino)-5-(4-((2,6-dichlrophenyl)piperazinyl) pentanoic acid,
2,5-di((2,6-dichlorophenyl)carbonylamino)pentanoic acid,
2-((2,6-dichlorophenyl)carbonylamino)-5- ((2,6-dimethoxyphenyl)carbonylamino) pentanoic acid,
2,6-di((2,6-dichlorophenyl)carbonylamino)hexanoic acid,
2-((2,6-dichlorophenyl)carbonylamino)-6-((2,6-dimethoxyphenyl)carbonylamino) hexanoic acid,
3-(4-(2-carboxy-2-((2,6-dichlorophenyl)carbonylamino)ethyl)phenyl)-2-((2,6-dichlorophenyl)carbonylamino)propanoic acid,
3-(4-(2-carboxy-2-((2,6-dimethoxyphenyl)carbonylamino)ethyl)phenyl)-2-((2,6-dichlorophenyl)carbonylamino)propanoic acid,
N-(4-(2H-2,3,4,5-tetraazolyl)-4-((2,6-dichlorophenyl)carbonylamino)butyl)(2,6-dimethoxyphenyl)formamide,
5-((2-(2H-2,3,4,5-tetraazolyl)phenyl)carbonylamino)-2-((2,6-dichlorophenyl)carbonylamino) pantanoic acid,
5-((6-(2H-2,3,4,5-tetraazolyl)-2-methoxyphenyl)carbonylamino)-2-((2,6-dichlorophenyl)carbonylamino) pentanoic acid,
3-(4-((2-(2H-2,3,4,5-tetraazolyl)phenyl)carbonylamino)-2-oxo-1,3-diazinyl)-2-((2,6-dichlorophenyl)carbonylamino) propanoic acid,
2-((2-(2H-2,3,4,5-tetraazolyl)phenyl)carbonylamino)-3-(3-(2-carboxy-2-((2,6-dichlorophenyl)carbonylamino)ethyl)-2-oxoimidazolizinyl)propanoic acid,
3-(3-(2-(2H-2,3,4,5-tetraazolyl)phenyl)-2-oxoimidazolyzinyl)-2-((2,6-dichlorophenyl)carbonylamino)propanoic acid,
2-((2,6-dichlorophenyl)carbonylamino)-3-(4-((2,6-dimethylphenyl)carbonylamino)-2-oxohydropyrimidinyl)propanoic acid,
2-((2,6-difluorophenyl)carbonylamino)-3-(4-((2,6-dichlorophenyl)carbonylamino)-2-oxohydropyrimidinyl)propanoic acid,
2-((2,6-difluorophenyl)carbonylamino)-3-(4-((2,6-dimethylphenyl)carbonylamino)-2-oxohydropyrimidinyl)propanoic acid,
2-((2,6-difluorophenyl)carbonylamino)-3 -(4-((2,6-dimethoxylphenyl)carbonylamino)-2-oxohydropyrimidinyl)propanoic acid,
2-((2,6-dibromophenyl)carbonylamino)-3-(4-((2,6-dichlorophenyl)carbonylamino)-2-oxohydropyrimidinyl)propanoic

acid,
2-((2,6-dibromophenyl)carbonylamino)-3-(4-((2,6-dimethylphenyl)carbonylamino)-2-oxohydropyrimidinyl)propanoic acid,
2-((2,6-dibromophenyl)carbonylamino)-3-(4-((2,6-dimetoxylphenyl)carbonylamino)-2-oxohydropyrimidinyl)propanoic acid,
2-((2-methyl-5-nitrophenyl)carbonylamino)-3-(4-((2,6-dichlorophenyl)carbonylamino)-2-oxohydropyrimidinyl)propanoic acid,
2-((2-methyl-5-nitrophenyl)carbonylamino)-3-(4-((2,6-dimethylphenyl)carbonylamino)-2-oxohydropyrimidinyl)propanoic acid,
2-((2-methyl-5-nitrophenyl)carbonylamino)-3-(4-((2,6-dimethoxyphenyl)carbonylamino)-2-oxohydropyrimidinyl)propanoic acid,
2-((2-bromo-6-methylphenyl)carbonylamino)-3-(4-((2,6-dichlorophenyl)carbonylamino)-2-oxohydropyrimidinyl)propanoic acid,
2-((2-bromo-6-methylphenyl)carbonylamino)-3-(4-((2,6-dimethylphenyl)carbonylamino)-2-oxohydropyrimidinyl)propanoic acid,
2-((2-bromo-6-methylphenyl)carbonylamino)-3-(4-((2,6-dimethoxylphenyl)carbonylamino)-2-oxohydropyrimidinyl) propanoic acid,
2-((2,6-dimethylphenyl)carbonylamino)-3-(4-((2,6-dichlorophenyl)carbonylamino)-2-oxohydropyrimidinyl)propanoic acid,
2-((2,6-dimethylphenyl)carbonylamino)-3-(4-((2,6-dimethoxyphenyl)carbonylamino)-2-oxohydropyrimidinyl)propanoic acid,
2-((2,6-dimethylphenyl)carbonylamino)-3-(4-((2,6-dimethyphenyl)carbonylamino)-2-oxohydropyrimidinyl)propanoic acid,
2-((2,6-dimethoxyphenyl)carbonylamino)-3-(4-((2,6-dichlorophenyl)carbonylamino)-2-oxohydropyrimidinyl)propanoic acid,
2-((2,6-dimethoxyphenyl)carbonylamino)-3-(4-((2,6-dimethoxyphenyl)carbonylamino)-2-oxohydropyrimidinyl)propanoic acid,
2-((2,6-dimethoxyphenyl)carbonylamino)-3-(4-((2,6-dimethylphenyl)carbonylamino)-2-oxohydropyrimidinyl)propanoic acid,
3-(4-((2,6-dichlorophenyl)carbonylamino)-2-oxohydropyrimidinyl)-2-((4-chlorophenyl)carbonylamino)propanoic acid,
3-(4-((2,6-dichlorophenyl)carbonylamino)-2-oxohydropyrimidinyl)-2-((2-chlorophenyl)carbonylamino)propanoic acid,
3-(4-((2,6-dichlorophenyl}carbonylamino)-2-oxohydropyrimidinyl)-2-((3-chlorophenyl)carbonylamino)propanoic acid,
3-(4-((2,6-dichlorophenyl)carbonylamino)-2-oxohydropyrimidinyl)-2-((4-bromophenyl)carbonylamino)propanoic acid,
3-(4-((2,6-dichlorophenyl)carbonylamino)-2-oxohydropyrimidinyl)-2-((2-bromophenyl)carbonylamino)propanoic acid,
3-(4-((2,6-dichlorophenyl)carbonylamino)-2-oxohydropyrimidinyl)-2-((3-bromophenyl)carbonylamino)propanoic acid,
3-(4-((2,6-dichlorophenyl)carbonylamino)-2-oxohydropyrimidinyl)-2-((4-methylphenyl)carbonylamino)propanoic acid,
3-(4-((2,6-dichlorophenyl)carbonylamino)-2-oxohydropyrimidinyl)-2-((2-methylphenyl)carbonylamino)propanoic acid,
3-(4-((2,6-dichlorophenyl)carbonylamino)-2-oxohydropyrimidinyl)-2-((3-methylphenyl)carbonylamino)propanoic acid,
3-(4-((2,6-dichlorophenyl)carbonylamino)-2-oxohydropyrimidinyl)-2-((4-methoxyphenyl)carbonylamino)propanoic acid,
3-(4-((2,6-dichlorophenyl)carbonylamino)-2-oxohydropyrimidinyl)-2-((2-methoxyphenyl)carbonylamino)propanoic acid,
3-(4-((2,6-dichlorophenyl)carbonylamino)-2-oxohydropyrimidinyl)-2-((3-methoxyphenyl)carbonylamino)propanoic acid,
3-(4-((2,6-dimethylphenyl)carbonylamino)-2-oxohydropyrimidinyl)-2-((4-chlorophenyl)carbonylamino)propanoic acid,
3-(4-((2,6-dimethylphenyl)carbonylamino)-2-oxohydropyrimidinyl)-2-((2-chlorophenyl)carbonylamino)propanoic acid,
3-(4-((2,6-dimethylphenyl)carbonylamino)-2-oxohydropyrimidinyl)-2-((3-chlorophenyl)carbonylamino)propanoic acid,
3-(4-((2,6-dimethylphenyl)carbonylamino)-2-oxohydropyrimidinyl)-2-((4-bromophenyl)carbonylamino)propanoic acid,
3-(4-((2,6-dimethylphenyl)carbonylamino)-2-oxohydropyrimidinyl)-2-((2-bromophenyl)carbonylamino)propanoic acid,
3-(4-((2,6-dimethylphenyl)carbonylamino)-2-oxohydropyrimidinyl)-2-((3-bromophenyl)carbonylamino)propanoic acid,
3-(4-((2,6-dimethylphenyl)carbonylamino)-2-oxohydropyrimidinyl)-2-((4-methylphenyl)carbonylamino)propanoic acid,
3-(4-((2,6-dimethylphenyl)carbonylamino)-2-oxohydropyrimidinyl)-2-((2-methylphenyl)carbonylamino)propanoic acid,
3-(4-((2,6-dimethylphenyl)carbonylamino)-2-oxohydropyrimidinyl)-2-((3-methylphenyl)carbonylamino)propanoic acid,
3-(4-((2,6-dimethylphenyl)carbonylamino)-2-oxohydropyrimidinyl)-2-((4-methoxyphenyl)carbonylamino)propanoic acid,
3-(4-((2,6-dimethylphenyl)carbonylamino)-2-oxohydropyrimidinyl)-2-((2-methoxyphenyl)carbonylamino)propanoic acid,
3-(4-((2,6-dimethylphenyl)carbonylamino)-2-oxohydropyrimidinyl)-2-((3-methoxyphenyl)carbonylamino)propanoic acid,
3-(4-((2,6-dimethoxyphenyl)carbonylamino)-2-oxohydropyrimidinyl)-2-((4-chlorophenyl)carbonylamino)proparioic acid,
3-(4-((2,6-dimethoxyphenyl)carbonylamino)-2-oxohydropyrimidinyl)-2-((2-chlorophenyl)carbonylamino)propanoic acid,
3-(4-((2,6-dimethoxyphenyl)carbonylamino)-2-oxohydropyrimidinyl)-2-((3-chlorophenyl)carbonylamino)propanoic acid,
3-(4-((2,6-dimethoxyphenyl)carbonylamino)-2-oxohydropyrimidinyl)-2-((4-bromophenyl)carbonylamino)propanoic acid,
3-(4-((2,6-dimethoxyphenyl)carbonylamino)-2-oxohydropyrimidinyl)-2-((2-bromophenyl)carbonylamino)propanoic acid,
3-(4-((2,6-dimethoxyphenyl)carbonylamino)-2-oxohydropyrimidinyl)-2-((3-bromophenyl)carbonylamino)propanoic acid,
3-(4-((2,6-dimethoxyphenyl)carbonylamino)-2-oxohydropyrimidinyl)-2-((4-methylphenyl)carbonylamino)propanoic acid,
3-(4-((2,6-dimethoxyphenyl)carbonylamino)-2-oxohydropyrimidinyl)-2-((2-methylphenyl)carbonylamino)propanoic acid,
3-(4-((2,6-dimethoxyphenyl)carbonylamino)-2-oxohydropyrimidinyl)-2-((3-methylphenyl)carbonylamino)propanoic acid,
3-(4-((2,6-dimethoxyphenyl)carbonylamino)-2-oxohydropyrimidinyl)-2-((4-methoxyphenyl)carbonylamino)propanoic acid,
3-(4-((2,6-dimethoxyphenyl)carbonylamino)-2-oxohydropyrimidinyl)-2-((2-methoxyphenyl)carbonylamino)propanoic acid, and
3-(4-((2,6-dimethoxyphenyl)carbonylamino)-2-oxohydropyrimidinyl)-2-((3-methoxyphenyl)carbonylamino)propanoic acid.

The processes for producing the compounds represented by Formula I (hereinafter, "the compounds represented by Formula I", for example, may be referred to as simply "Formula I" for short) will now be described. However, the processes for producing each compound is not restricted thereto. Further, in the various production processes, the reaction conditions may appropriately be selected from those described below.

Among the compounds represented by Formula I, those wherein B, F, J, L and Q are carbon atoms, H and K are methylene groups, G and M are carboxyl groups, D and O are carbonyl groups, E and N are nitrogen atoms and I is sulfor atom, that is, the compounds of the Formula XIV:

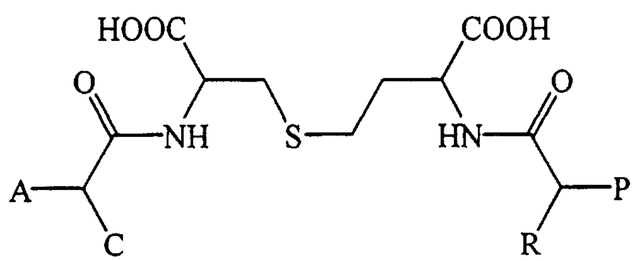

XIV

(wherein A, C, P and R represent the same meanings as described above) may be produced by reacting a compound of the Formula XV:

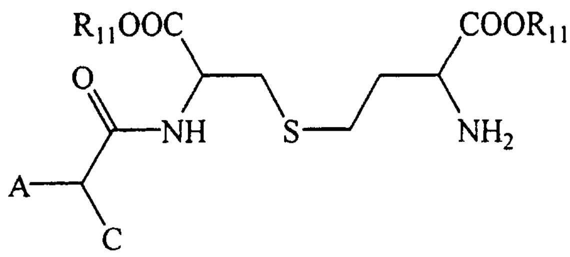

XV

(wherein A and C represent the same meanings as described above, and $R_{11}$ is linear alkyl) with P(R)COCl or P(R)COOH (wherein P and R represent the same meanings as described above) in the presence of a tertiary amine such as triethylamine or diisopropylamine, and then hydrolyzing the ester group with an aqueous sodium hydroxide solution or the like in an alcoholic solvent such as methanol. The reaction between Formula XV and P(R)COCl or P(R)COOH may be carried out, for example, in a soluvent such as tetrahydrofuran, dimethylformamide, chloroform or the like, usually at a temperature of about 0°C to room temperature, for about 1 hour to 24 hours, although the reaction conditions are not restricted thereto. Although the mixing ratio between Formula XV to P(R)COCl or P(R) COOH is not restricted, the mixing ratio may usually be about 1:1 to 1:2 by mole. The amount of the tertiary amine to be added is not restricted, and usually about 1 to 4 equivalents. In cases where P(R)COOH is used, a condensing agent such as dicyclohexylcarbodiimide (DCC), benzotriazole-1-tris(dicyclopentylamino)phosphonium hexafluorophosphide salt (PyBOP) or diphenylphosphorylazide (DPPA) is usually used. The amount of such a condensing agent is not restricted, and usually about 1 to 3 equivalents. Formula XV may be produced by reacting Formula XVI:

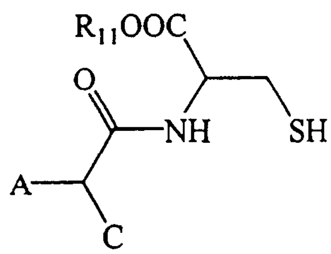

XVI

(wherein A and C represent the same meanings as described above. $R_{11}$ is linear alkyl) with Formula XVII:

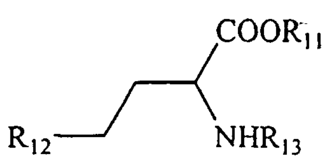

XVII

(wherein $R_{11}$ is linear alkyl; $R_{12}$ is a leaving group such as chloro, bromo, mesyl or tosyl; and $R_{13}$ is Boc or Trt group) in an ether solvent such as tetrahydrofuran using a base such as sodium t-butoxide, and then deprotecting $R_{13}$. The reaction between Formula XVI and Formula XVII may be carried out at a temperature usually from about -20°C to 40°C for about 30 minutes to 6 hours, although the reaction conditions are not restricted thereto. The mixing ratio of Formula XVI to XVII is not restricted and may usually be 1:1 to 1:3 by mole. The amount of the base is not restricted and may be about 1 to 2 equivalents. The compounds represented by Formula XVI may be produced by the process described below. In the present specification, in reaction schemes "step" is indicated as "Step".

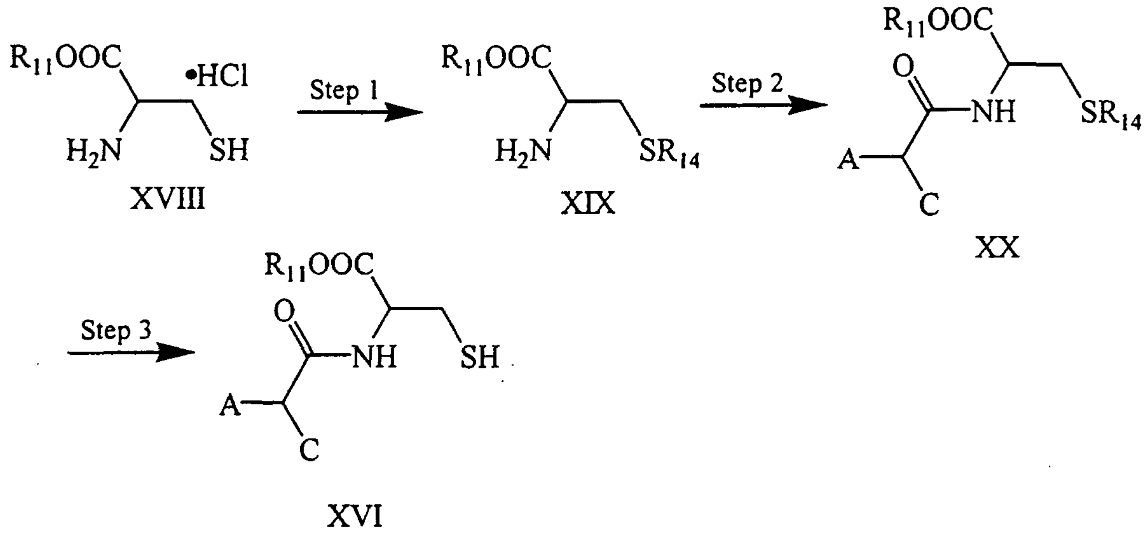


XVIII XIX XX XVI

Step 1 is the step of protecting the thiol group in the cystein ester hydrochloride XVIII. As the protective group $R_{14}$ of the thiol, trityl group, benzyloxycarbonyl group or the like are usually employed. In cases where $R_{14}$ is trityl group, Formula XVIII is reacted with 2 to 4 equivalents of trityl chloride in a polar solvent such as tetrahydrofuran or 1,4-dioxane at a temperature between room temperature and refluxing temperature, and then the generated N,S-ditritylated compound is treated with 50% acetic acid at a temperature between ice temperature to room temperature. The reaction time may be appropriately selected depending on the reaction temperature, and usually about 1 to 10 hours.

This step may also be attained by reacting Formula XVIII with 1 to 3 equivalents of triphenylmethanol in the presence of a Lewis acid such as $BF_3$-$OEt_2$. The reaction time may be selected depending on the reaction temperature, and usually about 1 to 24 hours. In cases where $R_{14}$ is benzyloxycarbonyl group, the step may be carried out by reacting Formula XVIII with 1 to 2 equivalents of benzyloxycarbonyl chloride in a mixed solvent of an ether solvent such as ether, tetrahydrofuran or 1,4-dioxane and aqueous sodium hydrogen carbonate solution at a temperature from ice temperature to room temperature. The reaction time may appropriately be selected depending on the reaction temperature, and usually about 1 to 10 hours.

Step 2 is the step of forming an amide bond. Formation of the amide bond may be accomplished by reacting Formula XIX with A(C)COCl or A(C)COOH (wherein A and C represent the same meanings as described above) in the presence of 1 to 3 equivalents of a tertiary amine such as triethylamine or diisopropylethylamine. In cases where A(C)COOH is used, a condensing agent such as dicyclohexylcarbodiimide (DCC), benzotriazole-1-tris(dicyclopentylamino)phosphonium hexafluorophosphide salt (PyBOP) or diphenylphosphorylazide (DPPA) is usually used. Although the reaction temperature is usually selected from ice temperature to room temperature, the reaction may be carried out at another temperature. The reaction time may appropriately be selected depending on the reaction temperature, and usually about 1 to 24 hours.

Step 3 is a step of deprotecting the protective group of the thiol group. In cases where the protective group is trityl group or benzyloxycarbonyl group, the deprotection may be attained by treating Formula XX with hydrochloric acid, trifluoroacetic acid, hydrobromic acid-acetic acid or the like in an amount of usually 1 to 10 equivalents at a temperature between ice temperature and room temperature. The reaction time may appropriately be selected depending on the reaction temperature, and usually about 30 minutes to 6 hours.

The compound represented by Formula XVII may be produced by the process shown below.

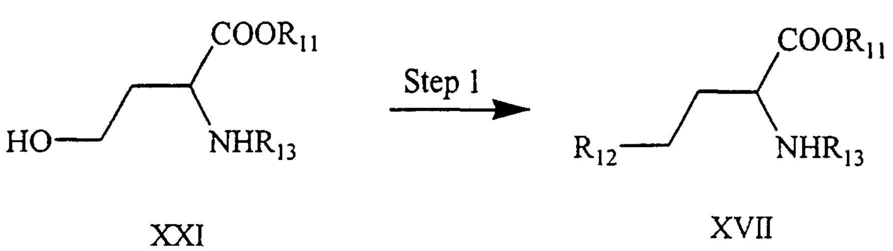


XXI XVII

(wherein $R_{11}$ and $R_{13}$ represent the same meanings as described above, and $R_{11}$ is linear alkyl)

Step 1 is the step of converting the hydroxyl group of the compound to a leaving group such as chloro, bromo, mesyl or tosyl group. To convert the hydroxyl group to chloro, a chlorinating reagent such as thionyl chloride, concentrated hydrochloric acid or tetrachloromethane-triphenylphosphine is used, but other chlorinating agents may also be employed. To convert the hydroxyl group to bromo, a brominating agent such as thionyl bromide, hydrobromic acid or tetrabromomethane-triphenylphosphine is used, but other brominating agents may also be employed. To convert the hydroxyl group to tosyl group or mesyl group, the product is treated with methanesulfonyl chloride or p-toluenesulfonyl chloride, respectively, under basic condition in the presence of pyridine, trimethylamine or the like. This reaction is usually carried out at -20°C to room temperature, and preferably at ice temperature. As the solvent, halogen-containing solvent such as methylene chloride or chloroform is used, but other solvents such as pyridine may also be employed. The mixing ratio of Formula XXI to the chlorinating agent, brominating agent or the reagent such as sulfonyl chloride in the reaction mixture at the initiation of the reaction is not restricted and 1:1 to 1:3 by mole may usually be suited. The reaction time may appropriately be selected depending on the reaction temperature, and usually about 1 to 24 hours.

Among the compounds represented by Formula I, those wherein B, F, J, L and Q are carbon atoms, H and K are methylene groups, G is carboxyl group, D and O are carbonyl groups, E and N are nitrogen atoms, I is sulfur atom and M is hydrogen, that is, the compound of the Formula XXII:

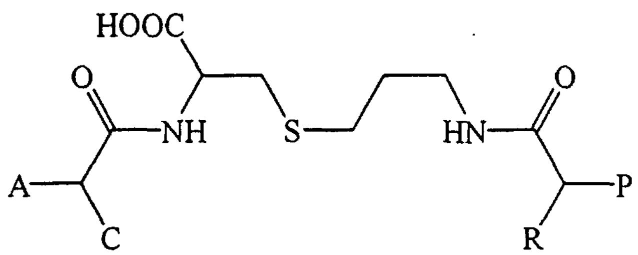

XXII

(wherein A, C, P and R represent the same meanings as described above) may be produced by reacting a compound of Formula XXIII:

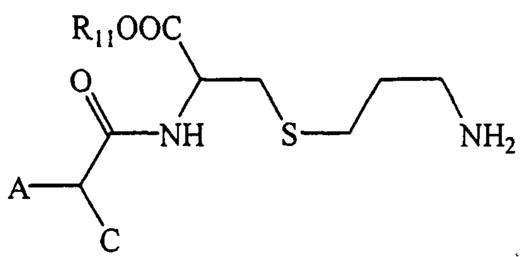

XXIII

(wherein A and C represent the same meanings described above, and $R_{11}$ is linear alkyl) with P(R)COCl or P(R)COOH (wherein P and R represent the same meanings as described above) in the presence of a tertiary amine such as triethylamine or diisopropylamine, and then hydrolyzing the ester group with an aqueous sodium hydroxide solution or the like in an alcoholic solvent such as methanol. In cases where P(R)COOH is used, a condensing agent such as dicyclohexylcarbodiimide (DCC), benzotriazole-1-tris(dicyclopentylamino)phosphonium hexafluorophosphide salt (PyBOP) or diphenylphosphorylazide (DPPA) is usually used. This reaction may be carried out under the similar reaction conditions of the reaction between Formula XV and P(R)COCl or P(R)COOH (see the 151st paragraph). Formula XXIII may be produced by reacting Formula XVI and Formula XXIV:

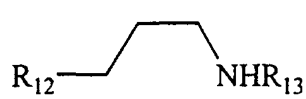

XXIV

(wherein $R_{12}$ and $R_{13}$ represent the same meanings as described above)
in an ether solvent such as tetrahydrofuran using a base such as potassium t-butoxide, and then deprotecting $R_{13}$. This reaction may be carried out under the similar reaction conditions of the reaction between Formula XVII and the base (see 155th paragraph). The compounds represented by Formula XXIV may be produced by the following process:

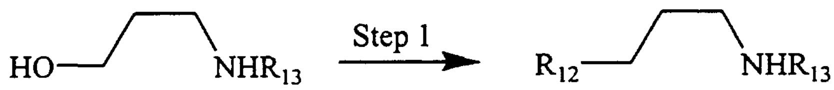


XXV XXIV

(wherein $R_{12}$ and $R_{13}$ represent the same meanings as described above).

Step 1 may be carried out in the same manner as in Step 1 of the process for producing Formula XVII.

Among the compounds represented by Formula I, those wherein B, F, I, L and Q are carbon atoms, H and K are methylene groups, G and M are carboxyl groups, D and O are carbonyl groups, E and N are nitrogen atoms and J is sulfur atom, that is, the compounds of Formula XXVI:

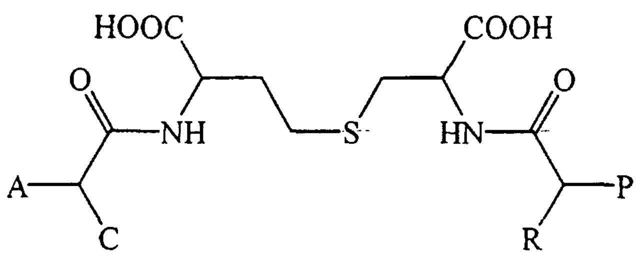

XXVI

(wherein A, C, P and R represent the same meanings as described above) may be produced by reacting Formula XXVII:

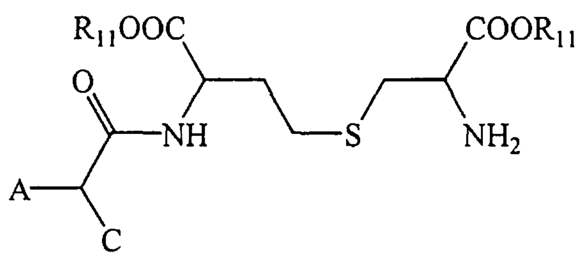

XXVII

(wherein A and C represent the same meanings as described above, and $R_{11}$ is linear alkyl)
with P(R)COCl or P(R)COOH (P and R represent the same meanings as described above) in the presence of a tertiary amine such as triethylamine or diisopropylamine, and then hydrolyzing the ester group with an aqueous sodium hydroxide solution or the like in an alcoholic solvent such as methanol. In cases where P(R)COOH is used, a condensing agent such as dicyclohexylcarbodiimide (DCC), benzotriazole-1-tris(dicyclopentylamino)phosphonium hexafluorophosphide salt (PyBOP) or diphenylphosphorylazide (DPPA) is usually used. This reaction may be carried out under the similar reaction conditions of the reaction between Formula XV and P(R)COCl or P(R)COOH (see the 151st paragraph). Formula XXVII may be produced by reacting Formula XXVIII:

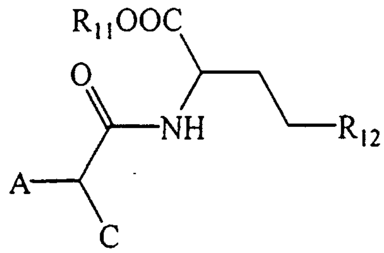

XXVIII

(wherein A, C and $R_{12}$ represent the same meanings as described above, and $R_{11}$ is linear alkyl) with Formula XXIX:

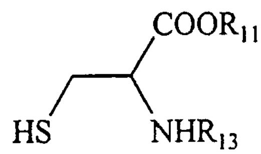

XXIX

(wherein $R_{13}$ represent the same meanings as described above, and $R_{11}$ is linear alkyl) in an ether solvent such as tetrahydrofuran using a base such as potassium t-butoxide, and then deprotecting $R_{13}$. This reaction may be carried out under the similar reaction conditions of the reaction between Formula XVII and the base (see 155th paragraph). The compounds represented by Formula XXVIII may be produced by the process shown below.

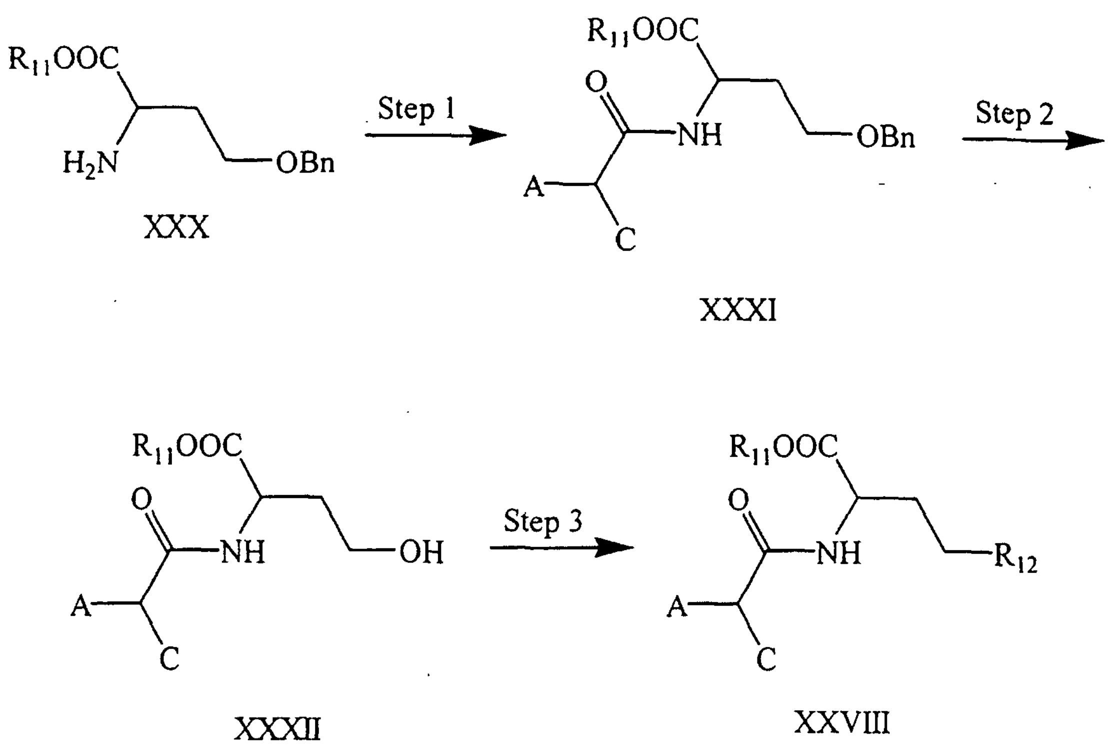


XXX XXXI XXXII XXVIII

(wherein A, C and $R_{12}$ represent the same meanings as described above, and $R_{11}$ is linear alkyl)

Step 1 may be accomplished by reacting Formula XXX with A(C)COCl (wherein A and C represent the same

meanings as described above) in the presence of a tertiary amine such as triethylamine in an ether solvent such as tetrahydrofuran or in a halogen-containing solvent such as chloroform, or by reacting Formula XXX with A(C)COOH in the presence of a tertiary amine such as triethylamine using a condensing agent such as dicyclohexylcarbodiimide (DCC), benzotriazole-1-tris(dicyclopentylamino)phosphonium hexafluorophosphide salt (PyBOP) or diphenylphosphorylazide (DPPA) in a polar solvent such as dimethylformamide. Although the reaction temperature is usually selected from ice temperature to room temperature, the reaction may be carried out at another temperature. The mixing ratio of Formula XXX:A(C)COCl or A(C)COOH: condensing agent: amine at the initiation of the reaction is not restricted and about 1:1:1:2 to 1:1:2:4 by mole is usually appropriate. The reaction time may appropriately be selected depending on the reaction temperature, and usually about 1 to 24 hours.

Step 2 may be generally attained by hydrogenating Formula XXXI in an alcoholic solvent such as methanol or ethanol, or in a polar solvent such as ethyl acetate, THF or dioxane using a palladium catalyst such as palladium/carbon or palladium hydroxide, or platinum catalyst such as platinum dioxide. The reaction temperature is not restricted, and usually about 10 to 30°C is appropriate. The reaction time may appropriately be selected depending on the reaction temperature, and usually about 2 to 40 hours.

Step 3 is carried out as in Step 1 of the process for producing Formula XVII.

Among the compounds of the Formula I, those wherein B, F, I, L and Q are carbon atoms, H and K are methylene groups, G is carboxyl group, M is hydrogen, D and O are carbonyl groups, E and N are nitrogen atoms, and J is oxygen atom, that is, the compounds of the Formula XXXIII:

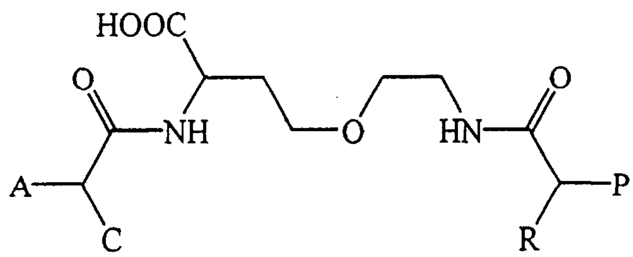

XXXIII

(wherein A, C, P and R represent the same meanings as described above) may be produced by reacting Formula XXXIV:

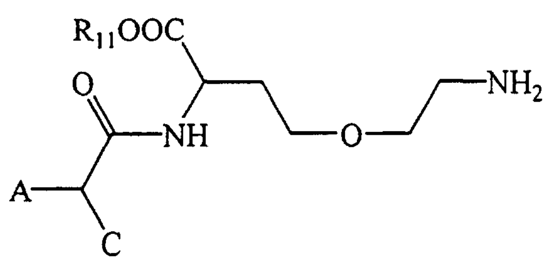

XXXIV

(wherein A and C represent the same meanings as described above, and $R_{11}$ is linear alkyl group)
with P(R)COCl or P(R)COOH (P and R represent the same meanings as described above) in the presence of a tertiary amine such as triethylamine or diisopropylamine, and then hydrolyzing the ester group with an aqueous sodium hydroxide solution or the like in an alcoholic solvent such as methanol. In cases where P(R)COOH is used, a condensing agent such as dicyclohexylcarbodiimide (DCC), benzotriazole-1-tris(dicyclopentylamino)phosphonium hexafluorophosphide salt (PyBOP) or diphenylphosphorylazide (DPPA) is usually used. This reaction may be carried out under the similar reaction conditions of the reaction between Formula XV and P(R)COCl or P(R)COOH (see the 151st paragraph). Formula XXXIV may be produced by the process shown below.

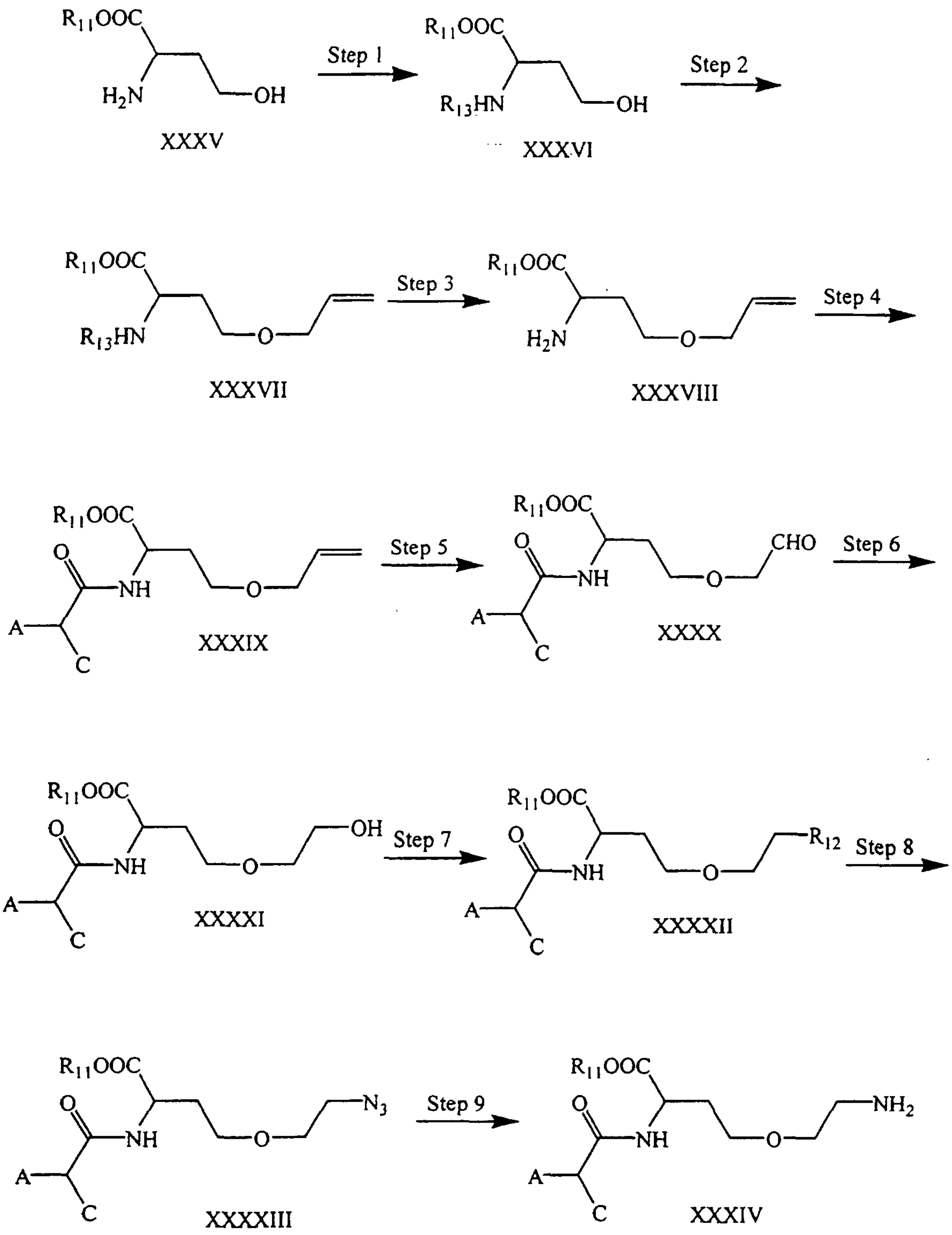


XXXV XXXVI

XXXVII XXXVIII

XXXIX XXXX

XXXXI XXXXII

XXXXIII XXXIV

(wherein A, C, $R_{12}$ and $R_{13}$ represent the same meanings as described above, and $R_{11}$ is alkyl group)

Step 1 is the step of protecting amino group. In general, trityl group or Boc group is employed as the protective group. In case of trityl group, the protection may be attained by reacting Formula XXXV with 1 to 3 equivalents of trityl chloride in the presence of a tertiary amine such as pyridine and triethylamine. As the reaction solvent, a halogen-containing solvent such as chloroform may also be employed. Although the reaction temperature is usually selected from ice temperature to room temperature, the reaction may be carried out at another temperature. The reaction time may appropriately be selected depending on the reaction temperature, and usually about 1 to 24 hours. In case of Boc group, the protection may be attained by reacting Formula XXXV with 1 to 3 equivalents of t-butyloxycarbonyl chloride or di-t-butyldicarbonate in the presence of aqueous sodium hydroxide solution or a tertiary amine such as pyridine or

triethylamine. As the reaction solvent, an ether solvent such as tetrahydrofuran or 1,4-dioxane is used. Although the reaction temperature is usually selected from ice temperature to room temperature, the reaction may be carried out at another temperature. The reaction time may appropriately be selected depending on the reaction temperature, and usually about 1 to 24 hours.

Step2 is the step of producing Formula XXXVII by reacting Formula XXXVI with allyl bromide in an ether solvent such as tetrahydrofuran or dimethoxyethane using a base such as sodium hydride or potassium t-butoxide. The reaction may also be carried out by conducting the reaction in a mixture of aqueous sodium hydroxide solution and a halogen-containing solvent such as dichloromethane using an phase-transfer catalyst such as tetra-n-butylammonium chloride or n-butyltrimethylammonium chloride. Although the reaction temperature is usually selected from ice temperature to room temperature, the reaction may be carried out at another temperature. The reaction time may appropriately be selected depending on the reaction temperature, and usually about 1 to 24 hours.

Step 3 is the step of deprotecting the protective group. In cases where the protective group is trityl group or Boc group, this may be attained by using trifluoroacetic acid, hydrochloric acid, hydrobromic acid or the like. As the reaction solvent, a halogen-containing solvent such as chloroform may also be employed. Although the reaction temperature is usually selected from ice temperature to room temperature, the reaction may be carried out at another temperature. The reaction time may appropriately be selected depending on the reaction temperature, and usually about 1 to 24 hours.

Step 4 may be carried out as in Step 2 of the process for producing Formula XVI.

Step 5 may be generally attained by using 1 to 3 equivalents of sodium periodate in a mixed solvent of an ether solvent such as tetrahydrofuran or 1,4-dioxane and water, in the presence of a catalytic amount of osmium tetroxide. Although the reaction temperature is usually selected from ice temperature to room temperature, the reaction may be carried out at another temperature. The reaction time may appropriately be selected depending on the reaction temperature, and usually about 1 to 24 hours. The step may also be accomplished by ozonolysis reaction. Ozonolysis reaction is carried out using an alcoholic solvent such as ethanol, at a temperature usually -78°C to -40°C, although the reaction may be carried out at another temperature. The reaction time may appropriately be selected depending on the reaction temperature, and usually about 30 minutes to 5 hours.

Step 6 is the step of reducing the aldehyde of the Formula XXXX to an alcohol. In general, as the reducing agent, sodium borohydride is used, but other reducing agents may also be employed. In case of using sodium borohydride, as the reaction solvent, usually, an alcoholic solvent such as methanol or ethanol is used. The mixing ratio of Formula XXXX to the sodium borohydride at the initiation of the reaction is not restricted, and about 1:0.5 to 1:2 by mole may usually be suited. Although the reaction temperature is usually selected from ice temperature to room temperature, the reaction may be carried out at another temperature. The reaction time may appropriately be selected depending on the reaction temperature, and usually about 30 minutes to 3 hours.

Step 7 is carried out as in Step 1 of the process for producing Formula XVII.

Step 8 may be attained by reacting Formula XXXXII with sodium azide in a polar solvent such as dimethylformamide. The mixing ratio of to sodium borohydride at the beginning of the reaction is not restricted, and a mixing ratio of about 1:1 to 1:2 by mole is appropriate. Although the reaction temperature is usually selected from room temperature to 100°C, sufficient reaction rate may be attained even at 50°C. The reaction time may appropriately be selected depending on the reaction temperature, and usually about 1 to 24 hours.

Step 9 is carried out as in Step 2 of the process for producing Formula XXVIII.

Among the compounds of the Formula I, those wherein B, F, I, L and Q are carbon atoms, H and K are methylene groups, G and M are carboxyl groups, D and O are carbonyl groups, E and N are nitrogen atoms, and I and J are sulfur atoms, that is, the compounds of the Formula XXXXIV:

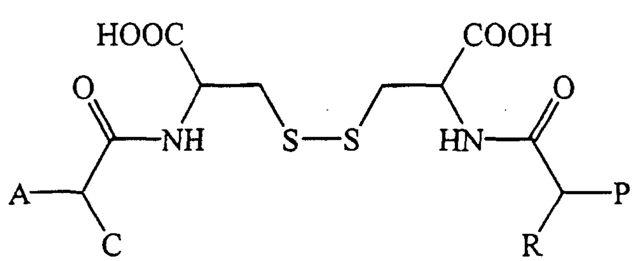

XXXXIV

(wherein A, C, P and R represent the same meanings as described above)
may be produced by the following process:

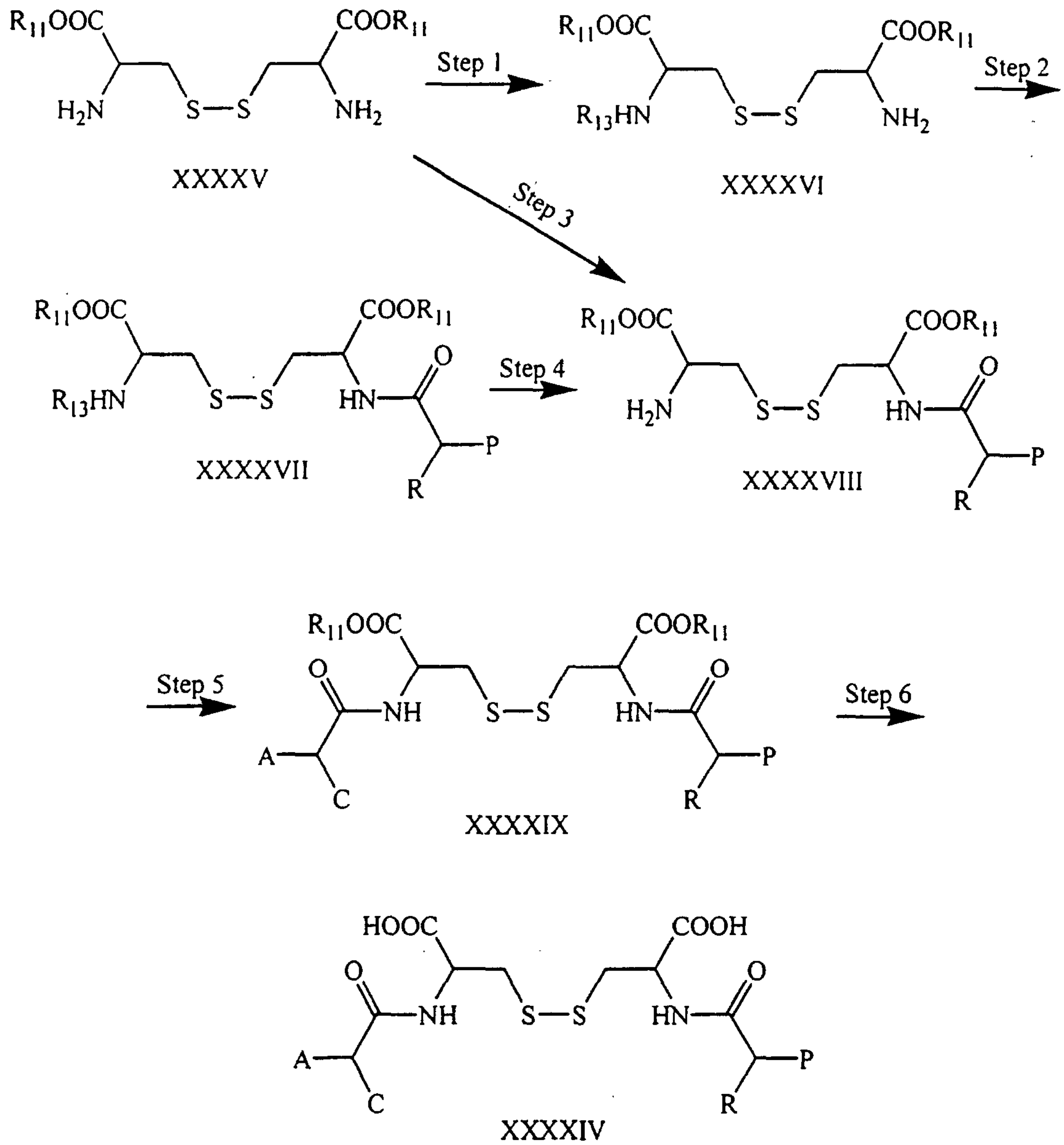


XXXXV

XXXXVI

XXXXVII

XXXXVIII

XXXXIX

XXXXIV

(wherein A, C, P, R and $R_{13}$ represent the same meanings as described above, and $R_{11}$ is linear alkyl)

Step 1 is carried out as in Step 1 of the process for producing Formula XXXIV.

Steps 2 and 3 are carried out as in Step 2 of the process for producing Formula XVI using R(P)COCl or R(P)COOH.

Step 4 is carried out as in Step 3 of the process for producing Formula XXXIV.

Step 5 is carried out as in Step 2 of the process for producing Formula XVI.

Step 6 may be carried out by using a base such as aqueous sodium hydroxide solution in an alcoholic solvent such as methanol. The mixing ratio of Formula XXXXIX and the aqueous sodium hydroxide solution at the beginning of the reaction is not restricted, and usually about 1:1 to 1:5 by mole is appropriate. Although the reaction temperature is usually selected from room temperature to 50°C, the reaction may be carried out at another temperature. The reaction time may appropriately be selected depending on the reaction temperature or the like, and usually about 1 to 24 hours. The step may also be carried out by using a tin reagent such as $n\text{-}Bu_2SnO$ or $(n\text{-}Bu_3Sn)_2O$ in a nonpolar solvent such as toluene. The mixing ratio of Formula XXXXIX and the tin reagent at the beginning of the reaction is not restricted, and usually about 1:1 to 1:20 by mole is appropriate. Although the reaction temperature is usually selected from 50°C to 150°C, the reaction may be carried out at another temperature. The reaction time may appropriately be selected depending on the reaction temperature or the like, and usually about 3 to 24 hours.

Among the compounds of the Formula I, those wherein B, F, I, L and Q are carbon atoms, H and K are methylene groups, G and M are carboxyl groups, D and O are carbonyl groups, E and N are nitrogen atoms, and J is

a nitrogen atom, that is, the compounds of the Formula XXXXX:

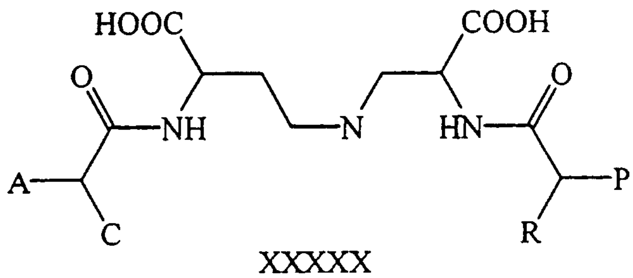

XXXXX

(wherein A, C, P and R represent the same meanings as described above) may be produced by reacting Formula XXXXXI:

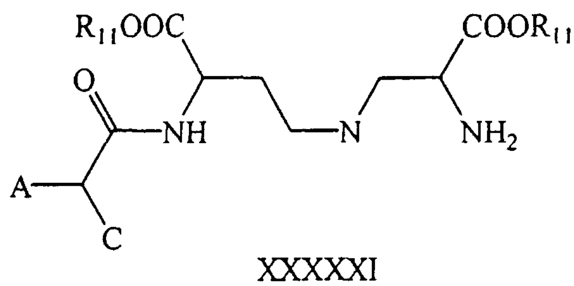

XXXXXI

(wherein A and C represent the same meanings as described above, and $R_{11}$ is alkyl) with P(R)COCl or P(R)COOH (wherein P and R represent the same meanings as described above) in the presence of a tertiary amine such as triethylamine or diisopropylamine, and then hydrolyzing the ester group with aqueous sodium hydroxide solution or the like in an alcoholic solvent such as methanol. In cases where P(R)COOH is used, a condensing agent such as dicyclohexylcarbodiimide (DCC), benzotriazole-1-tris(dicyclopentylamino)phosphonium hexafluorophosphide salt (PyBOP) or diphenylphosphorylazide (DPPA) is usually used. This reaction may be carried out under the similar reaction conditions of the reaction between Formula XV and P(R)COCl or P(R)COOH (see the 151st paragraph). Formula XXXXXI may be produced by reacting Formula XXXXXII:

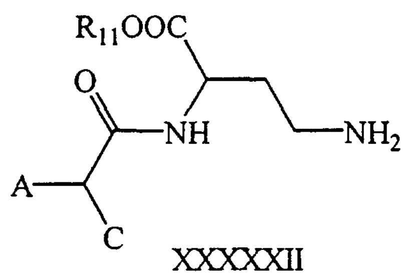

XXXXXII

(wherein A and C represent the same meanings as described above, and $R_{11}$ is alkyl) with Formula XXXXXIII:

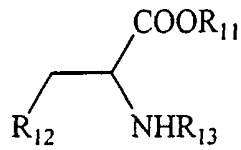

XXXXXIII

(wherein $R_{12}$ and $R_{13}$ represent the same meanings as described above, and $R_{11}$ is linear alkyl) in the presence of a base such as potassium carbonate or triethylamine in a polar solvent such as dimethylformamide, and then deprotecting $R_{13}$. This reaction may be carried out under the similar conditions of the reaction between Formula XVII and the base (see 155th paragraph). The compounds represented by Formula XXXXXII may be produced by the process shown below.

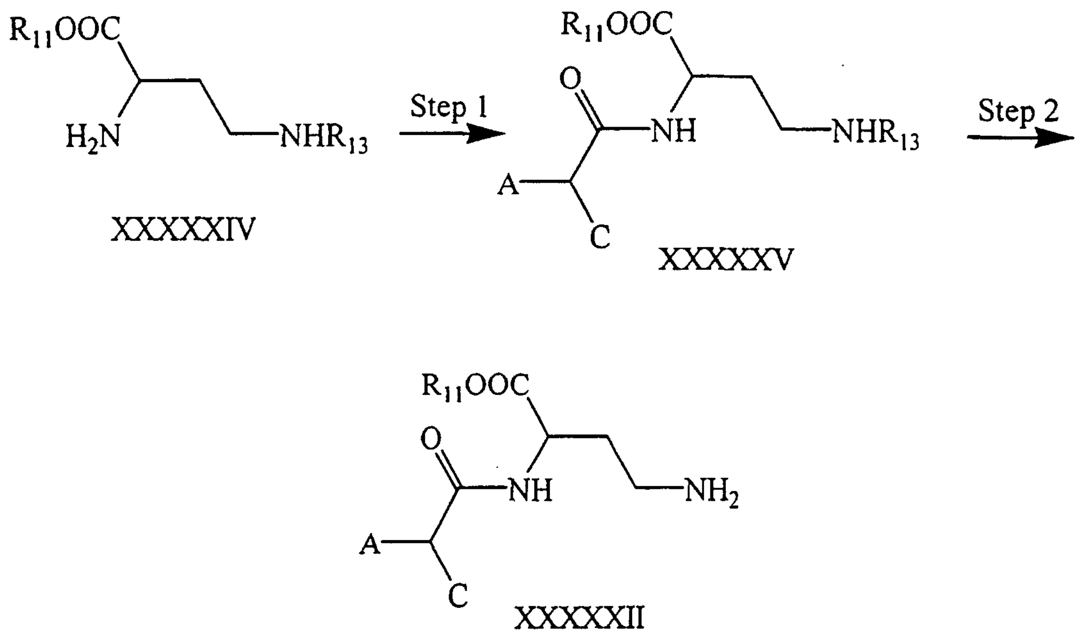

XXXXXIV XXXXXV XXXXXII

(wherein A, C and $R_{13}$ represent the same meanings as described above, and $R_{11}$ is linear alkyl)

Step 1 is carried out as in Step 2 of the process for producing Formula XVI.

Step 2 is carried out as in Step 3 of the process for producing Formula XXXIV.

Formula XXXXXIII may be produced by the following step:

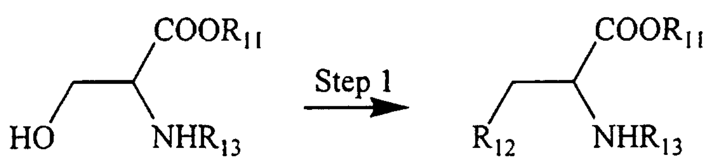


XXXXXVI XXXXXIII

(wherein $R_{13}$ represents the same meanings as described above, and $R_{11}$ is linear alkyl)

Step 1 is carried out as in Step 1 of the process for producing Formula XVII.

Among the compounds of the Formula I, those wherein B, F, J, L and Q are carbon atoms, H and K are methylene groups, G and M are carboxyl groups, D and O are carbonyl groups, E and N are nitrogen atoms, and I is a nitrogen atom, that is, the compounds of the Formula XXXXXVII:

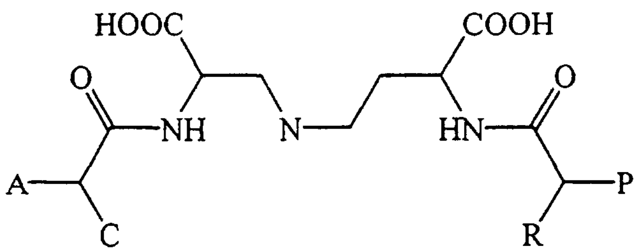

XXXXXVII

(wherein A, C, P and R represent the same meanings as described above) may be produced by reacting Formula XXXXXVIII:

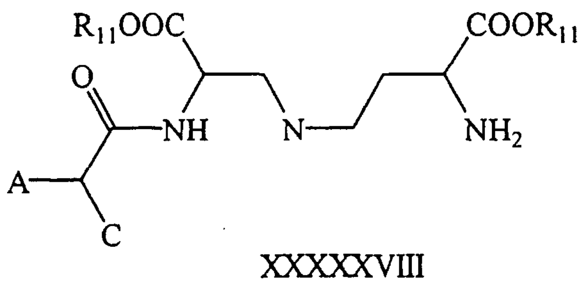

XXXXXVIII

(wherein A and C represent the same meanings as described above, and $R_{11}$ is alkyl) with P(R)COCl or P(R)COOH (wherein P and R represent the same meanings as described above) in the presence of a tertiary amine such as triethylamine or diisopropylamine, and then hydrolyzing the ester group with aqueous sodium hydroxide solution or the like in an alcoholic solvent such as methanol. In cases where P(R)COOH is used, a condensing agent such as dicyclohexylcarbodiimide (DCC), benzotriazole-1-tris(dicyclopentylamino)phosphonium hexafluorophosphide salt (PyBOP) or diphenylphosphorylazide (DPPA) is usually used. This reaction may be carried out under the similar reaction conditions of the reaction between Formula XV and P(R)COCl or P(R)COOH (see the 151st paragraph). Formula XXXXXVIII may be produced by reacting Formula XXXXXIX:

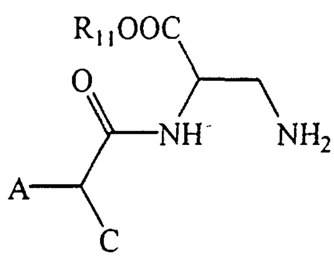

XXXXXIX

(wherein A and C represent the same meanings as described above, and $R_{11}$ is linear alkyl)
in the presence of a base such as potassium carbonate or triethylamine in a polar solvent such as dimethylformamide, and then deprotecting $R_{13}$. This reaction may be carried out under the similar conditions of the reaction between Formula XVII and the base (see 155th paragraph). The compounds represented by Formula XXXXXIX may be produced by the process shown below.

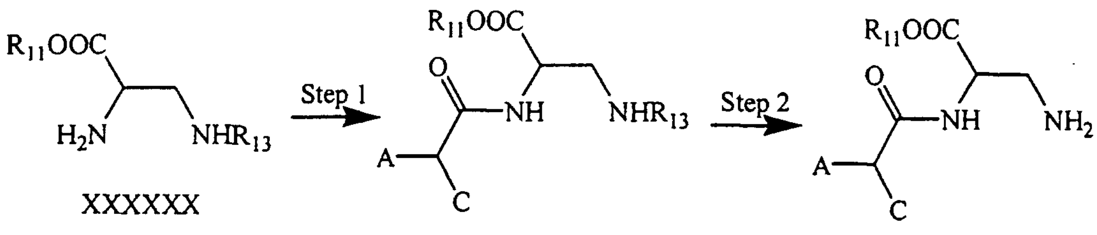


XXXXXX XXXXXXI XXXXXIX

(wherein $R_{13}$ represents the same meaning as described above, and $R_{11}$ is linear alkyl)

Step 1 may be carried out as in Step 2 of the process for producing Formula XVI.

Step 2 may be carried out as in Step 3 of the process for producing Formula XXXIV.

Among the compounds of the Formula I, those wherein A and P are the same, C and R are the same, B, F, I, J, L and Q are carbon atoms, H and K are methylene groups, G and M are carboxyl groups, D and O are carbonyl groups, and E and N are nitrogen atoms, that is, the compounds of the Formula XXXXXXII:

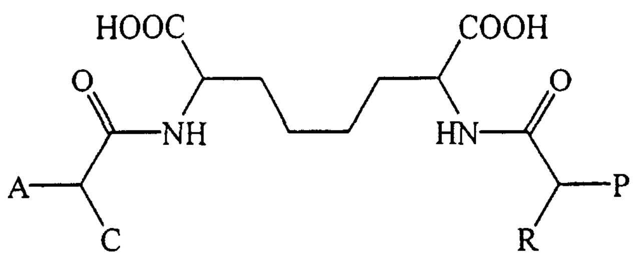

XXXXXXXII

(wherein A, C, P and R represent the same meanings as described above),
the compounds of the Formula I wherein A and P are the same, C and R are the same, B, F, I, J, L and Q are carbon atoms, H and K are methylene groups, G and M are carboxyl groups, D and O are carbonyl groups, E and N are nitrogen atoms, and the bond between I and J is double bond, that is, the compounds of the Formula XXXXXXIII:

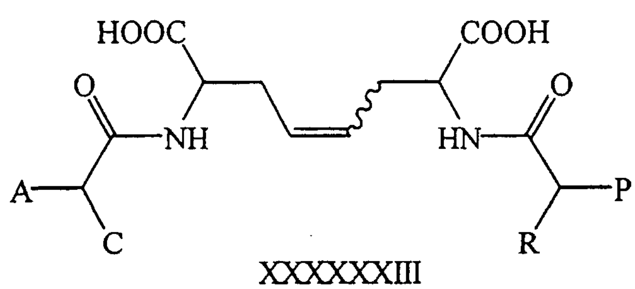

XXXXXXXIII

(wherein A, C, P and R represent the same meanings as described above, represents *cis* or *trans*),
and the compounds of the Formula I wherein J does not exist, A and P are the same, C and R are the same, B, F, L and Q are carbon atoms, H and K are methylene groups, G and M are carboxyl groups, D and O are carbonyl groups, E and N are nitrogen atoms, I is benzene ring, that is, the compounds of the Formula XXXXXXIV:

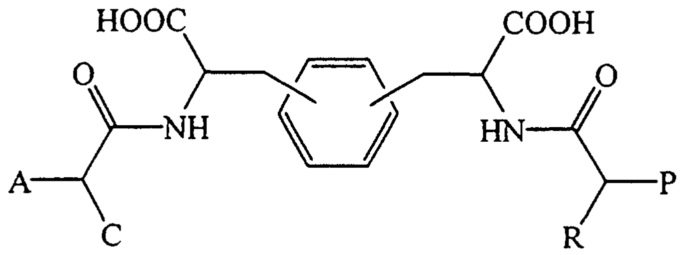

XXXXXXIV

(wherein A, C, P and R represent the same meanings as described above, and the position of substitution may be any of ortho, meta and para).
may be produced by the following process, respectively:

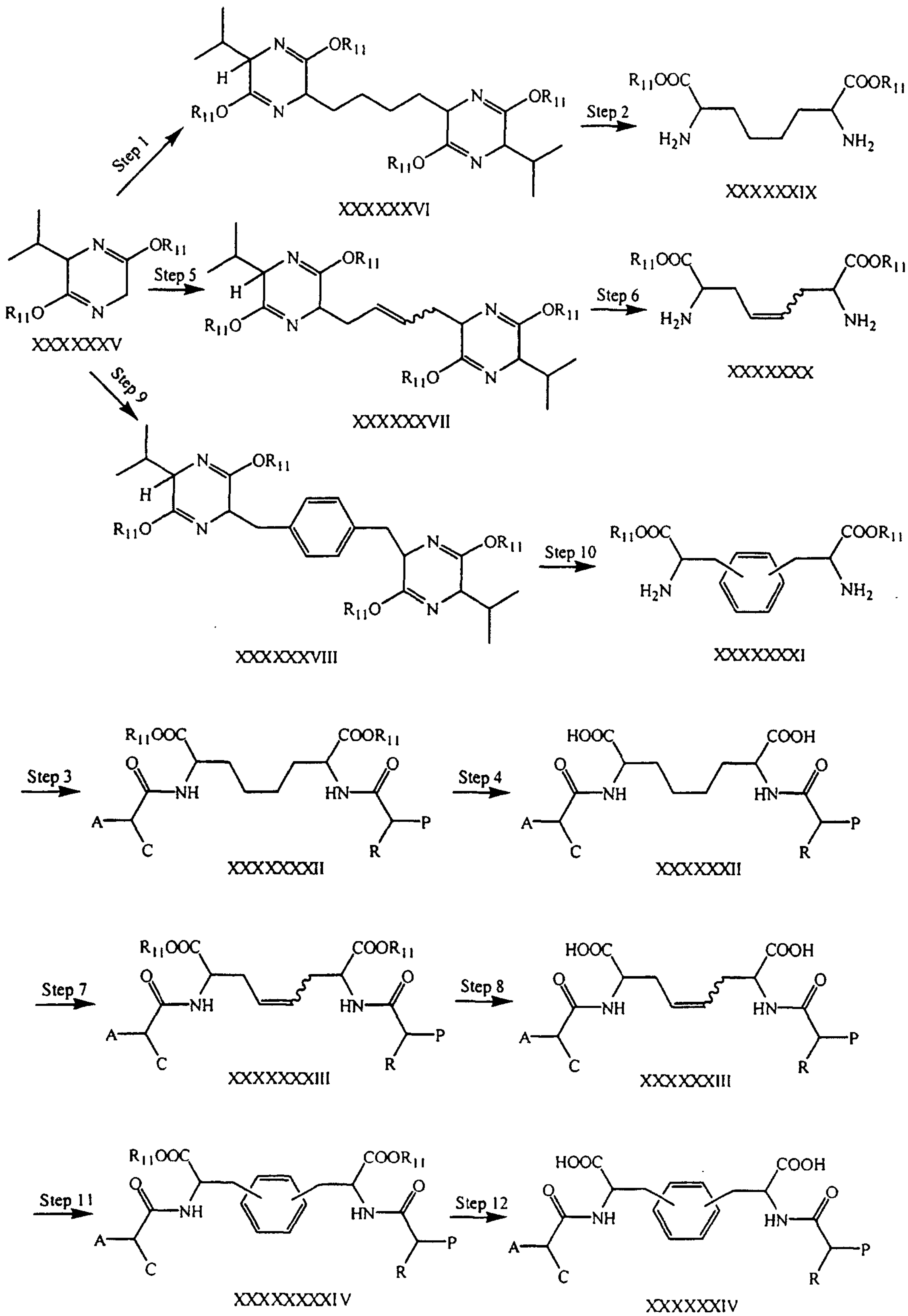


XXXXXXVI XXXXXXIX XXXXXXV XXXXXXVII XXXXXXX XXXXXXVIII XXXXXXXI XXXXXXXII XXXXXXII XXXXXXXIII XXXXXXIII XXXXXXXIV XXXXXXIV

(wherein A, C, P and R represent the same meanings as described above, and $R_{11}$ is linear alkyl).

Steps 1, 5 and 9 may be carried out by reacting Formula XXXXXXV with 1,4-dibromobutane, 1,4-dibromo-2-butene, and $\alpha,\alpha'$-dibromoxylene, respectively, in an ether solvent such as tetrahydrofuran or dimethoxymethane at -78°C to -20°C using an alkyllithium such as n-butyllithium. The mixing ratio of Formula XXXXXXV to 1,4-dibromobutane, 1,4-dibromo-2-butene, or to α,α'-dibromoxylene at the beginning of the reaction is not restricted, and a molar ratio of Formula XXXXXXV: 1,4-dibromobutane, 1,4-dibromo-2-butene or α,α'-dibromoxylene of about 1:2 to 1:4 is appropriate. The reaction time is appropriately selected depending on the reaction temperature or the like, and may usually be about 1 to 8 hours.

Steps 2, 6 and 10 are the steps for hydrolyzing the bislactarn ether by reacting it with an acid such as dilute hydrochloric acid in an ether solvent such as tetrahydrofuran or dimethoxyethane at 0°C to 40°C. The mixing ratio of Formula XXXXXXVI, XXXXXXVII or XXXXXXVIII to the dilute hydrochloric acid is not restricted, and a molar ratio of Formula XXXXXXVI, XXXXXXVII or XXXXXXVIII:hydrochloric acid of about 1:2 to 1:4 is appropriate. The reaction time is appropriately selected depending on the reaction temperature or the like, and may usually be about 5 to 40 hours.

Steps 3, 7 and 11 may be carried out as in Step 2 of the process for producing Formula XVI.

Steps 4, 8 and 12 may be carried out by using a base such as aqueous sodium hydroxide solution in an alcoholic solvent such as methanol. The mixing ratio of Formula XXXXXXXII, XXXXXXXIII or XXXXXXXIV to the aqueous sodium hydroxide solution is not restricted, and a molar ratio of Formula XXXXXXXII, XXXXXXXIII or XXXXXXXIV: aqueous sodium hydroxide solution of about 1:1 to 1:5 is appropriate. Although the reaction temperature is usually selected from room temperature to 50°C, the reaction may be carried out at another temperature. The reaction time may appropriately be selected depending on the reaction temperature or the like, and usually about 1 to 24 hours.

Among the compounds of the Formula I, those wherein F, G, H and J do not exist, B, L and Q are carbon atoms, K and D are methylene groups, M is carboxyl group, O is carbonyl group, and E is oxygen atom, N is nitrogen atom, and I is benzene ring, that is, the compounds of the Formula XXXXXXXV:

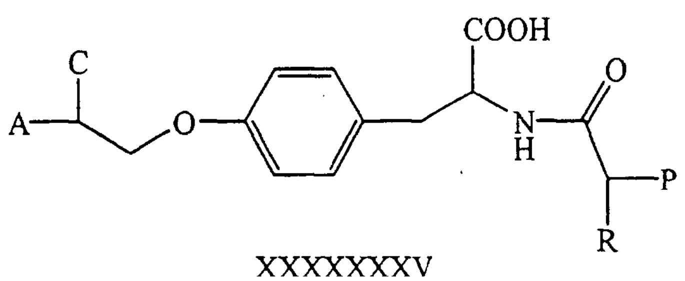

XXXXXXXV

(wherein A, B, C, P and R represent the same meanings as described above) may be produced by the following process:

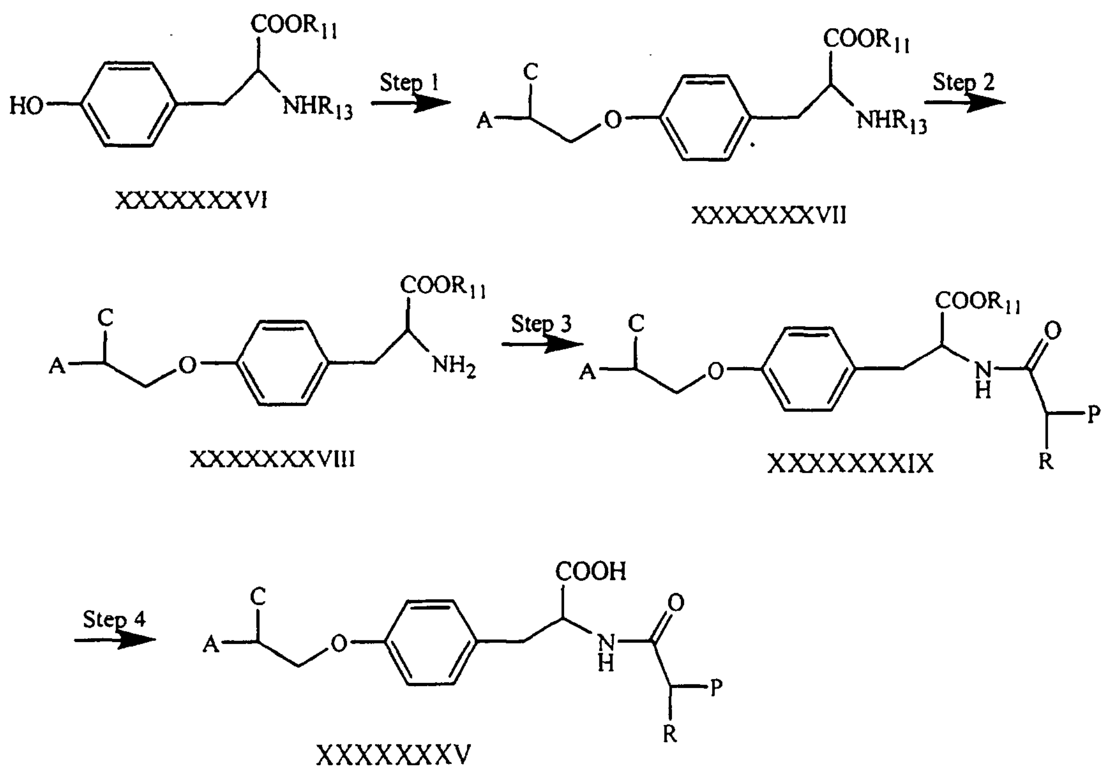


XXXXXXXVI

XXXXXXXVII

XXXXXXXVIII

XXXXXXXIX

XXXXXXXV

(wherein A, C, P, R and $R_{13}$ represent the same meanings as described above, and $R_{11}$ is linear alkyl).

Step 1 is the step for reacting Formula XXXXXXXV with $A(B)CCH_2R_{12}$ (wherein A, B and $R_{12}$ represent the same meanings as described above) in an ether solvent such as tetrahydrofuran or dimethoxymethane. As the base, sodium hydride, potassium t-butoxide or the like is usually employed. The mixing ratio of Formula XXXXXXXVI to $A(B)CCH_2R_{12}$ is not restricted, and a molar ratio of about 1:1 to 1:2 may usually be appropriate. The reaction temperature is not restricted and usually about 0°C to 50°C is appropriate. The reaction time may appropriately be selected depending on the reaction temperature or the like, and usually about 1 to 24 hours.

Step 2 is carried out as in Step 3 of the process for producing Formula XXXIV.

Step 3 is carried out as in Step 2 of the process for producing Formula XVI using R(P)COCl or R(P)COOH.

Step 4 is carried out as in Step 4 of the process for producing Formula XXXXXXII.

Among the compounds of the Formula I, those wherein A, B, C, D, E and J do not exist, K and H are methylene groups, F, L and Q are carbon atoms, G and M are carboxyl groups, O is carbonyl group, N is nitrogen atom, and I is benzene ring, that is, the compounds of the Formula XXXXXXXX:

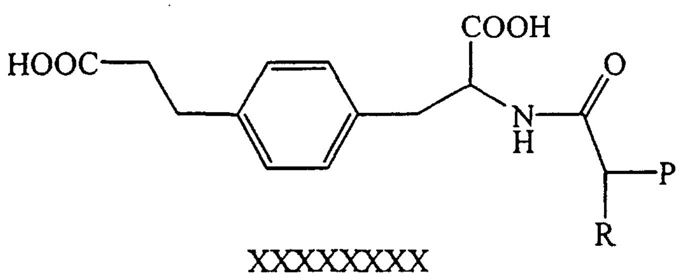

XXXXXXXX

may be produced by the following process:

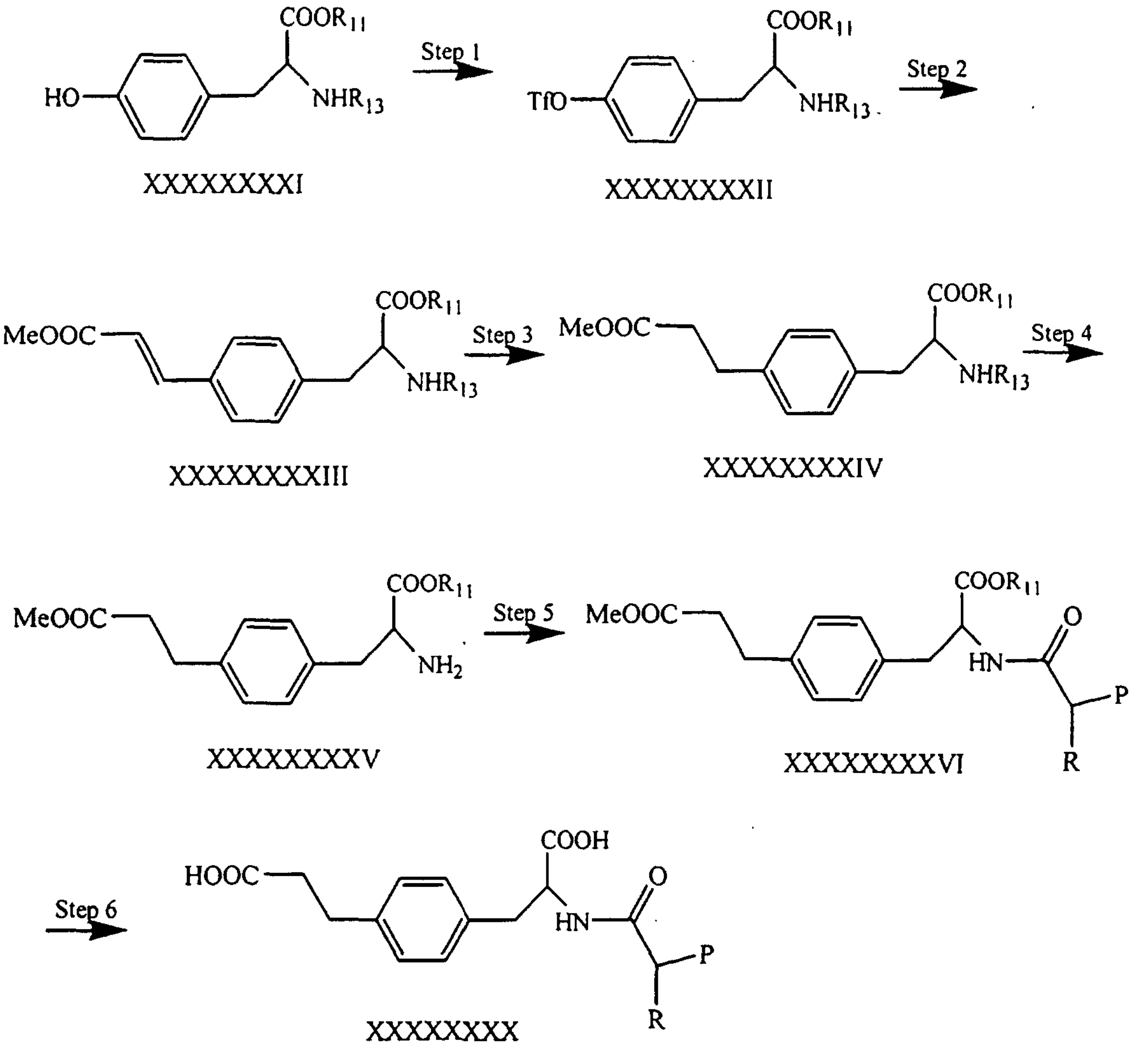


XXXXXXXXXI

XXXXXXXXXII

XXXXXXXXXIII

XXXXXXXXXIV

XXXXXXXXXV

XXXXXXXXXVI

XXXXXXXXX

(wherein $R_{13}$ represent the same meaning as described above, and $R_{11}$ is linear alkyl).

Step 1 is the step of converting a hydroxyl group to triflate group in a halogen-containing solvent such as dichloromethane or chloroform. As the reagent for converting hydroxyl group to triflate,, usually, trifluoromethane sulfonic anhydride, N-phenyltrifluoromethanesulfoimide or the like is employed. As the base, a tertiary amine such as triethylamine is used. The mixing ratio of Formula XXXXXXXXI:trifluoromethanesulfonic anhydride or N-phenyltrifluoromethanesulfoimide:base is not restricted, and a molar ratio of about 1:1:1 to 1:2:4 is appropriate. The reaction temperature is not restricted, and usually about 0°C to room temperature is appropriate. The reaction time may appropriately be selected depending on the reaction temperature or the like, and usually about 1 to 24 hours.

Step 2 is the step for reacting Formula XXXXXXXXII with methyl acrylate in a polar solvent such as dimethylformamide in the presence of a palladium catalyst such as palladium acetate or palladium chloride. In some cases, an inorganic salt such as lithium chloride is used as an additive. In some cases, a phosphine compound such as P(o-Tol)$_3$ is used as a ligand of palladium. As a base, a tertiary amine such as triethylamine is used. In cases where this step is carried out by using palladium acetate, lithium chloride and P(o-Tol)$_3$, a mixing ratio of Formula XXXXXXXXII: methyl acrylate: palladium acetate: lithium chloride: P(o-Tol)$_3$: base at the beginning of the reaction of about 1:1:0.1: 1:1:2 to 1:3:0.5:5:3:10 is usually appropriate. The reaction temperature is not restricted, and usually room temperature to about 120°C is appropriate. The reaction time may appropriately be selected depending on the reaction temperature, and may usually be about 2 to 30 hours.

Step 3 may be carried out as in Step 2 of the process for producing Formula XXVIII.

Step 4 may be carried out as in Step 3 of the process for producing Formula XXXIV.

Step 5 may be carried out as in Step 2 of the process for producing Formula XXXXIV.

Step 6 may be carried out as in Step 4 of the process for producing Formula XXXXXXII.

Among the compounds of the Formula I, those wherein Q and J do not exist, K is ethylene, H is methylene,

B, F, L and Q are carbon atoms, G is carboxyl group, M is oxygen atom, D is carbonyl group, E and N are nitrogen atoms, I is benzene ring, and the bond between L and M is double bond, that is, the compounds of the Formula XXXXXXXXXVII:

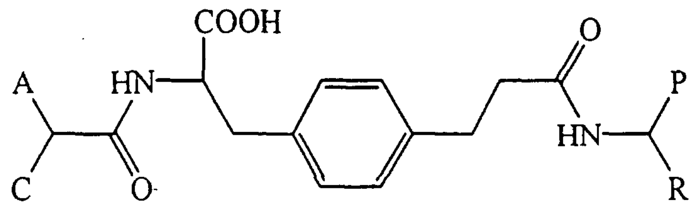

XXXXXXXXXVII

(wherein A, C, P and R represent the same meanings as described above) may be produced by the following process:

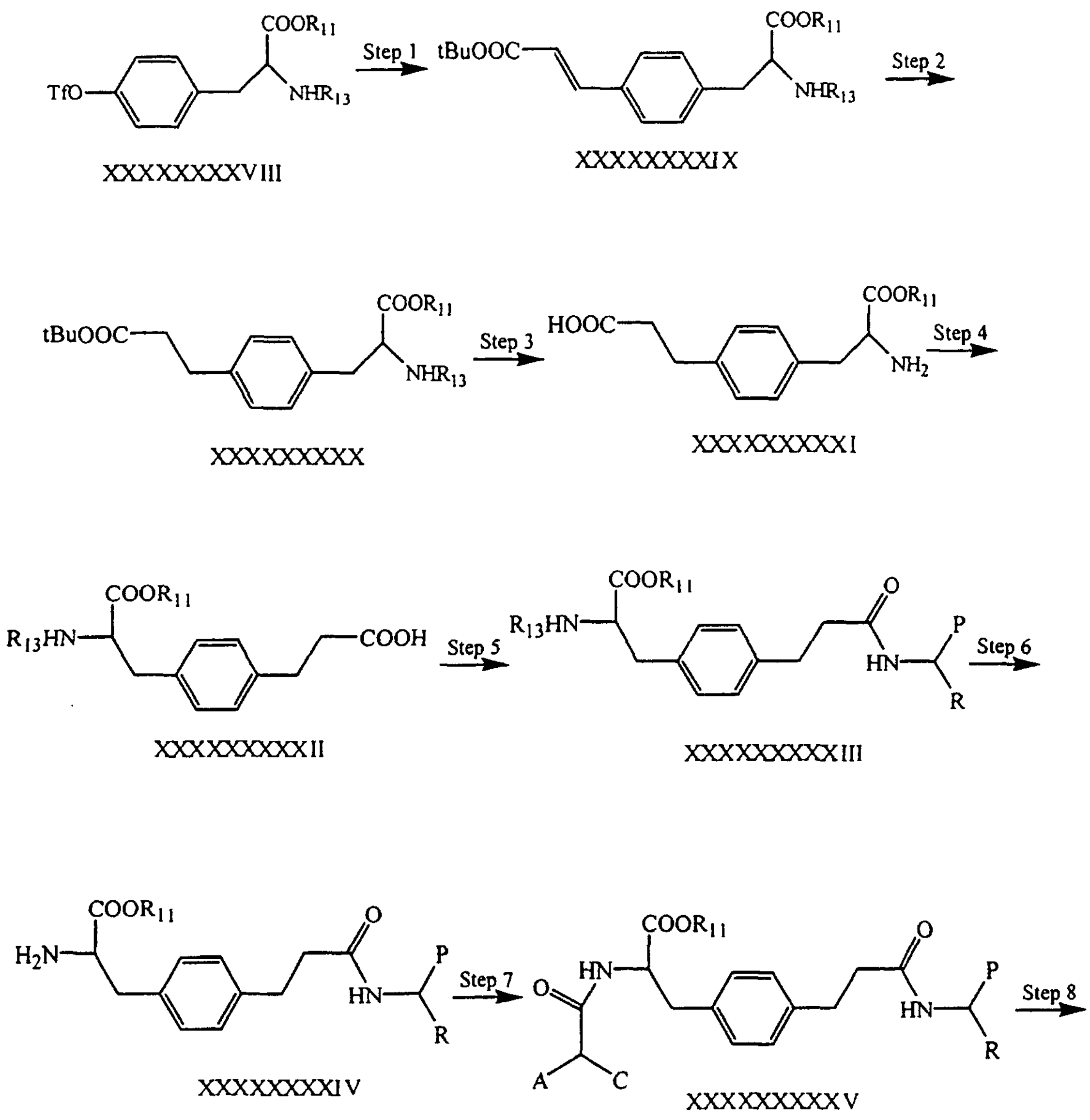


XXXXXXXXXVIII XXXXXXXXXIX XXXXXXXXXX XXXXXXXXXXI XXXXXXXXXXII XXXXXXXXXXIII XXXXXXXXXXIV XXXXXXXXXXV

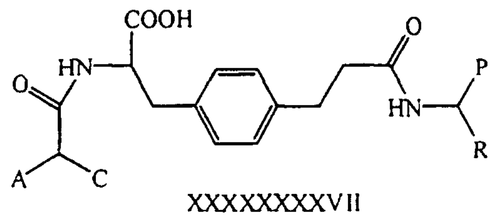

XXXXXXXXVII

(wherein A, C, P, R and $R_{13}$ represent the same meanings as described above, and $R_{11}$ is linear alkyl).

Step 1 may be carried out as in Step 2 of the process for producing Formula XXXXXXXX except that t-butyl acrylate is used in place of methyl acrylate.

Step 2 may be carried out as in Step 2 of the process for producing Formula XXVIII.

Step 3 may be carried out as in Step 3 of the process for producing Formula XXXIV.

Step 4 may be carried out as in Step 1 of the process for producing Formula XXXIV.

Step 5 may be carried out as in Step 2 of the process for producing Formula XXXXIV.

Step 6 may be carried out as in Step 3 of the process for producing Formula XXXIV.

Step 7 may be carried out as in Step 2 of the process for producing Formula XVI.

Step 8 may be carried out as in Step 4 of the process for producing Formula XXXXXXII.

Among the compounds of the Formula I, those wherein I and J do not exist, H is $-CH_2-S-$, K is methylene, B, F, L and Q are carbon atoms, M is carboxyl group, D and O are carbonyl groups, E and N are nitrogen atoms, E, F and G form piperidine ring, that is, the compounds of the Formula XXXXXXXXXVI:

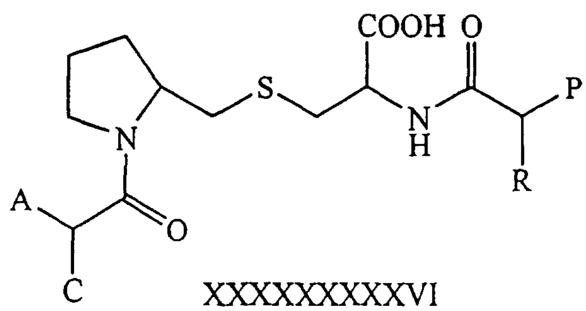

XXXXXXXXXVI

(wherein A, C, P and R represent the same meanings as described above)
may be produced by reacting Formula XXXXXXXXXVII:

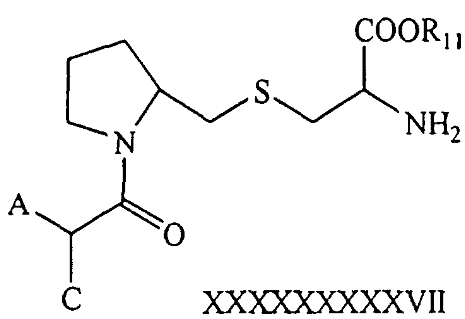

XXXXXXXXXVII

(wherein A and C represent the same meanings as described above, and $R_{12}$ is linear alkyl)
with P(R)COCl or P(R)COOH (wherein P and R represent the same meanings as described above) in the presence of a tertiary amine such as triethylamine or diisopropylamine, and then hydrolyzing the ester group with aqueous sodium hydroxide solution or the like in an alcoholic solvent such as methanol. In cases where P(R)COOH is used, a condensing agent such as dicyclohexylcarbodiimide (DCC), benzotriazole-1-tris(dicyclopentylamino)phosphonium hexafluorophosphide salt (PyBOP) or diphenylphosphorylazide (DPPA) is usually used. This reaction may be carried out under the similar reaction conditions of the reaction between Formula XV and P(R)COCl or P(R)COOH (see the 151st paragraph). Formula XXXXXXXXXVII may be produced by reacting Formula XXXXXXXXXVIII

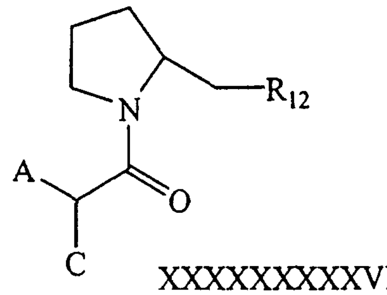

XXXXXXXXXVIII

(wherein A, C and $R_{12}$ represent the same meanings as described above) with Formula XXIX in an ether solvent such as tetrahydrofuran in the presence of a base such as potassium t-butoxide, and then deprotecting $R_{13}$. This reaction may be carried out under the similar conditions of the reaction between Formula XVII and the base (see 155th paragraph). Formula XXXXXXXXXVIII may be produced by the following process:

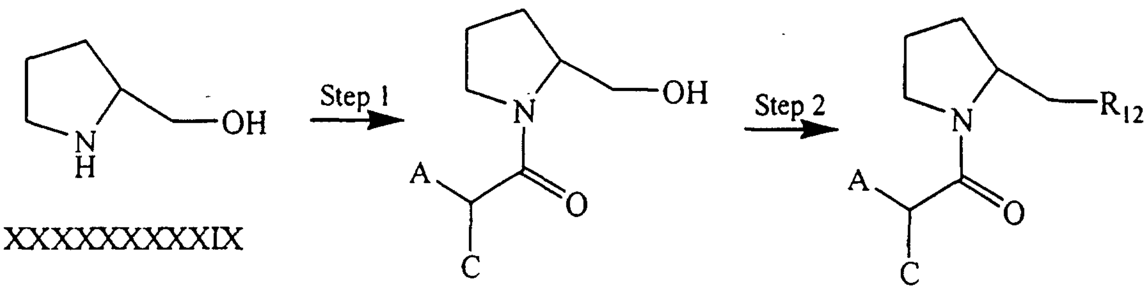


XXXXXXXXXIX XXXXXXXXXX XXXXXXXXXVIII

(wherein A, C and $R_{12}$ represent the same meanings as described above)

Step 1 is carried out as in Step 2 of the process for producing Formula XVI.

Step 2 is carried out as in Step 1 of the process for producing Formula XVII.

Among the compounds of the Formula I, those wherein D and O are carbonyl groups, E and N are nitrogen atoms, F and L are carbon atoms, G and M are carboxyl groups, H and K are methylene groups, I is benzene ring, and A, C, P, R and J do not exist, that is, the compounds of the Formula XXXXXXXXXIX:

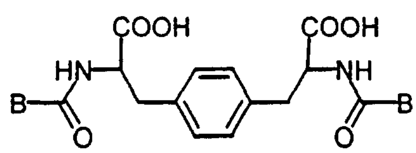

XXXXXXXXXIX

(wherein B represents the same meaning as described above) may be produced by hydrolyzing Formula XXXXXXXXXX:

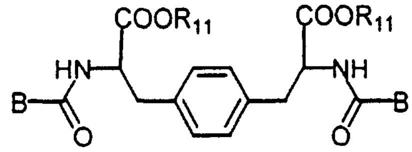

XXXXXXXXXX

(wherein B represents the same meanings as described above, and $R_{11}$ represents linear alkyl) with a base such as aqueous sodium hydroxide solution in a solvent such as methanol. The hydrolysis with the base such as aqueous sodium hydroxide solution may usually be carried out at a temperature of about 0°C to room temperature for about 1 hour to 24 hours, and the amount of the base may usually be about 1 to 4 equivalents, although the reaction conditions are not restricted to these.

Formula XXXXXXXXXX may be produced by the following process:

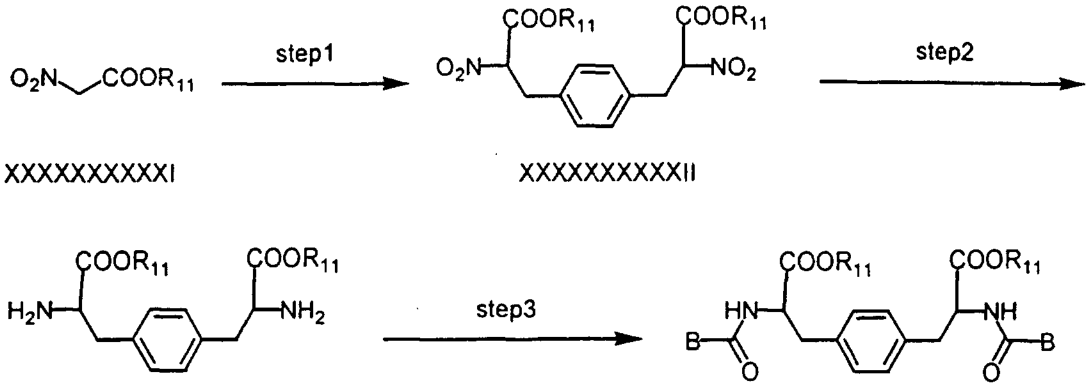


XXXXXXXXXXXI XXXXXXXXXXXII XXXXXXXXXXXIII XXXXXXXXXX

(wherein $R_{11}$ is linear alkyl)

Step 1 may be attained by reacting alkyl 2-nitroacetate XXXXXXXXXXXI with 1,4-bis(iodomethyl)benzene in a solvent such as tetrahydrofuran in the presence of about 0.05 to 0.2 equivalents of 18-crown-6-ether, after treating Formula XXXXXXXXXXXI with a base such as potassium t-butoxide. The mixing ratio of alkyl 2-nitroacetate XXXXXXXXXXXI and 1,4-bis(iodomethyl)benzene is not restricted, and usually about 1:0.5 to 1:1. The reaction temperature is not restricted and usually room temperature to reflux. The equivalents of the based used is not restricted and usually about 1 to 2 equivalents.

Step 2 may be carried out as in Step 2 of the process for producing Formula XXVIII.

Step 3 may be carried out as in Step 1 of the process for producing Formula XXVIII using B-COOH or B-C(O)Cl as the substrate.

Among the compounds of the Formula I, those wherein D and O are carbonyl groups, E and N are nitrogen atoms, F and L are carbon atoms, G and M are carboxyl groups, H and K are $-(CH_2)_{n11}-(CH_2)_{n12}-$, and A, C, P, R, I and J do not exist, that is, Formula XXXXXXXXXXXIV:

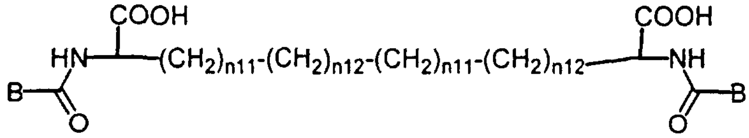

XXXXXXXXXXXIV

(wherein B, n11 and n12 represent the same meanings as described above) may be produced by hydrolyzing Formula XXXXXXXXXXXV:

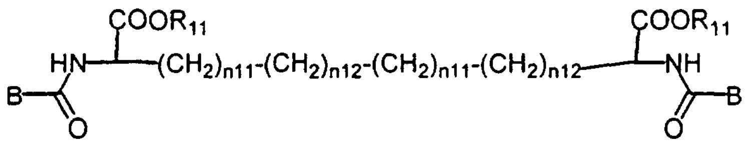

XXXXXXXXXXXV

(wherein $R_{11}$ is linear alkyl)
with a base such as aqueous sodium hydroxide solution in a solvent such as methanol. The hydrolysis with the base such as aqueous sodium hydroxide solution may usually be carried out at a temperature of about 0°C to room temperature for about 1 to 24 hours, and the amount of the base may usually be about 1 to 4 equivalents, although the reaction conditions are not restricted to these.

Formula XXXXXXXXXXXV may be produced by the following process:

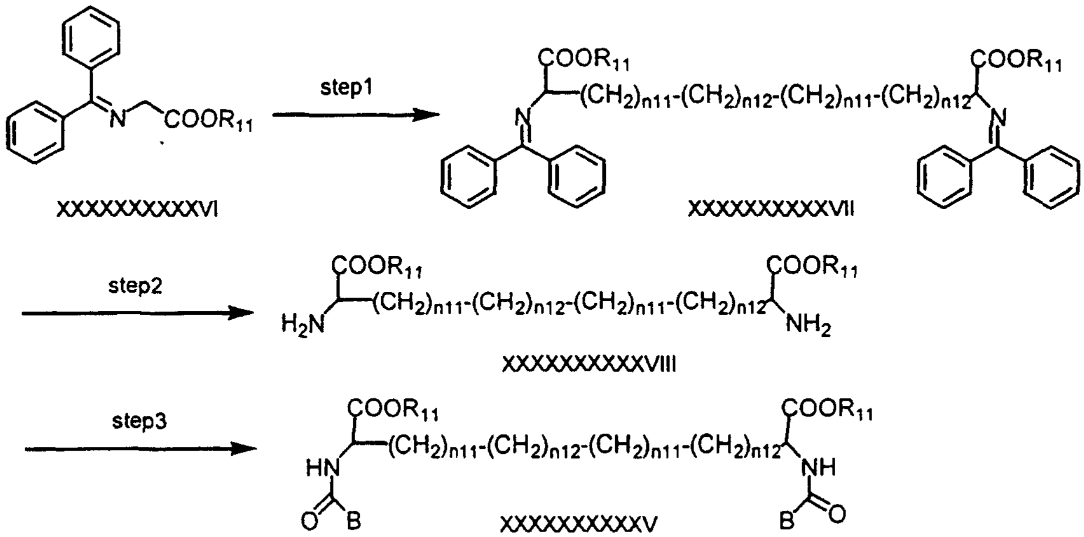


XXXXXXXXXXXVI

XXXXXXXXXXXVII

XXXXXXXXXXXVIII

XXXXXXXXXXXV

(wherein $R_{11}$ is linear alkyl)

Step 1 may be attained by reacting Formula XXXXXXXXXXXVI with a diiodoalkane such as 1,4-diiodobutane or 1,4-diiodopentane, after treating Formula XXXXXXXXXXXVI with a base such as cesium hydroxide, in a solvent such as dichloromethane and usually in the presence of about 0.05 to 0.4 equivalents of n-butyltriethylammonium salt. The mixing ratio of Formula XXXXXXXXXXXVI and diiodoalkane is not restricted, and usually about 1:0.5 to 1:1. The reaction temperature is not restricted and usually about 0°C to 50°C. The reaction time is not restricted and usually about 1 to 15 hours. The equivalents of the based used is not restricted and usually about 1 to 2 equivalents.

Step 2 may be attained by treating Formula XXXXXXXXXXXVII with 2 to 50 equivalents of an acid such as hydrochloric acid in a solvent such as dimethoxyethane or diethylether at about 0°C to 40°C. The reaction time is not restricted and usually about 4 to 40 hours.

Step 3 may be carried out as in Step 1 of the process for producing Formula XXVIII using B-COOH or B-C(O)Cl as the substrate.

In cases where the novel carboxylic acid derivative used in the present invention has one or more asymmetric carbon atoms, there may be racemate, diastereomers and each optical isomer. In the present invention, all of these may be employed.

Examples of the pharmaceutically acceptable salts of the compounds represented by Formula I include inorganic salts such as ammonium salt, alkaline metal salts (e.g., sodium salt and potassium salt), and alkaline earth metal salts (e.g., calcium salt and magnesium salt), and organic salts such as dicyclohexylamine salt and N-methyl-D-glucamine.

The adhesion-inhibition activity of the compound of the present invention against VLA-4 may be examined by using an adhesion assay system in which the adhesion between VLA-4-expressing cells such as Ramos cells and Jurkat cells, and fibronectin or a fragment thereof, e.g., a peptide containing CS-1 sequence (Gly Pro Glu Ile Leu Asp Val Pro Ser Thr) (hereinafter referred to as "CS-1 peptide") immobilized on an immunoplate is measured. Alternatively, a binding assay system in which the binding of the VLA-4 protein to the fibronectin or a fragment thereof such as CS-1 peptide immobilized on an immunoplate is measured may be employed. In the present invention, the inhibition assay of the compound is preferably measured by a binding assay system which measures the binding between a chimeric protein of VLA-4 and immunoglobulin (VLA-4-IgG chimeric protein) and CS-1 peptide (Japanese patent application No. H9-234544), although the method of measuring the inhibition activity is not restricted to this method. The term "VLA-4-IgG chimeric protein" herein means the heterodimer composite molecule in which a chimeric protein between α4 of VLA-4 and immunoglobulin (hereinafter referred to as "VLAα4·IgG chimeric protein), and a chimeric protein between β1 of VLA-4 and immunoglobulin (hereinafter referred to as "VLAβ1·IgG chimeric protein) are associated. As the immunoglobulin, although heavy chain or light chain of IgG, IgM or the like may be employed, in the present invention, $IgG_1$ heavy chain is used. When testing the inhibition activity of the compound, it is preferred to preliminarily mix the VLA-4-IgG chimeric protein and the test compound.

The compound of the present invention has an activity to inhibit adhesion of VLA-4 and inhibit the accumulation of leukocytes to inflammation sites, so that it may be used as a therapeutic drug for treating inflammatory diseases, especially chronic inflammatory diseases. The term "inflammatory diseases" herein means, for example, allergic inflammatory diseases such as bronchial asthma, atopic dermatosis, allergic rhinitis; hepatitis; nephritis; autoimmune diseases such as chronic rheumatoid arthritis and multiple sclerosis; rejection reaction after plantation of organs; type I diabetes; Crohn's disease and the like. In addition, the compound may be used for prevention of postoperative restenosis and as a therapeutic drug for arterial sclerosis.

The effects of the compounds obtained by the above-described methods are examplified by inflammation models of various animals. In the present invention, the effects of the compounds against accumulation of leukocytes to inflammatory tissues-and expression of allergic symptoms are exemplified by using peritonitis model, auricular edema model, bronchial asthma model, and rhinitis model, but the models are not restricted to these. To induce inflammation in these models, ascaris extract, ragweed pollen, dinitrofluorobenzene, oxazolone, egg white albumin or the like is used as an antigen. These models are known to present symptoms similar to allergic inflammation in human and to be useful as drug effect-evaluation models which reflect the IgE-mast cell dependent type I allergy reaction and cell-mediated immunological type IV allergy reaction (e.g., "Handbook of Experimental Immunology", vol 2, Miller,S.D andJenkins,M.K. (1986) Blackwell Scientific Publications, Oxford). In human, the diseases related to type I allergy reaction include bronchial asthma, atopic dermatitis, allergic rhinitis and the like, the diseases related to type IV allergy reaction include contact dermatitis, atopic dermatitis and the like.

When using the compound of the present invention as a therapeutic drug of the above-mentioned diseases, the compounds represented by Formula I and the base addition salts thereof may be administered as they are as powder, or as medical compositions in the form of an appropriate formulation orally or parenterally (e.g., percutaneous, intravenous, intrarectal administration or the like).

Examples of the formulations for administration include tablets, powders, balls, capsules, granules, syrups, liquids, injection solutions, emulsions, suspensions and suppositories. These formulations may be prepared by known methods, and include various carriers which are conventionally used in the field of formulation of medicines. Examples of these carriers include, for solid formulations, vehicles, lubricants, binders and disintegrators; for liquid formulations, solvents, solubilizers, suspending agents and soothing agents. Additives such as preservatives, antioxidants, coloring agents, sweetners, adsorbing agents and wetting agents may be used.

Examples of the vehicles include lactose, saccharose, D-mannitol, starch, sucrose, corn starch, crystalline cellulose and light anhydrous silicic acid. Examples of the lubricants include magnesium stearate, calcium stearate, talc and colloidal silica. Examples of the binders include crystalline cellulose, saccharose, D-mannitol, dextrin, hydroxypropyl cellulose, hydroxypropylmethyl cellulose, polyvinylpyrrolidone, starch, sucrose, gelatin, methyl cellulose and sodium carboxymethyl cellulose. Examples of the disintegrators include starch, carboxymethyl cellulose, potassium carboxymethyl cellulose, croscarmellose sodium, sodium carboxymethyl starch and L-hydroxypropyl cellulose. Examples of the solvents include water for injection, alcohol, propylene glycol, Macrogol, sesame oil and corn oil. Examples of the solubilizers include polyethylene glycol, propylene glycol, D-mannitol, benzyl benzoate, ethanol, cholesterol, triethanolamine, sodium carbonate and sodium citrate. Examples of the suspending agents include surfactants such as stearyl triethanolamine, sodium lauryl sulfate, lauryl aminopropionate, lecithin, benzalkonium chloride, benzethonium chloride and glycerin monostearate; and hydrophilic macromolecules such as polyvinyl alcohol, polyvinyl pyrrolidone, methyl cellulose, hydroxymethyl cellulose, hydroxyethyl cellulose and hydroxypropyl cellulose. Examples of the isotonic agents include glucose, sodium chloride, D-sorbitol and D-mannitol. Examples of the buffering agents include phosphoric acid salts, acetic acid salts, carbonic acid salts and citric acid salts. Examples of the soothing agents include benzyl alcohol. Examples of the antiseptics include paraoxy benzoic acid esters, chlorobutanol, benzyl alcohol, phenethyl alcohol, dehydroacetic acid and sorbic acid. Examples of the antioxidants include sulfurous acid salts and ascorbic acid.

The effective dose and the number of administration of the compound of Formula I or the pharmaceutically acceptable salt thereof vary depending on the formulation, and age, body weight, state or severity of the symptom of the patient, but usually 1 to 1000 mg, preferably 1 to 300 mg of the compound or the salt thereof may be administered

to an adult per day in one time or in several times.

Unless undesirable interactions with the compound of Formula I or the pharmaceutically acceptable salt thereof occur, the formulation may include one or more of other therapeutically effective components. Examples of such therapeutic components include steroid drugs, nonsteroidal anti-inflammatory drugs, lipoxygenase inhibitors, leucotriene antagonists, bronchodilators, thromboxane synthesis inhibitors, thromboxane antagonists, histamine antagonists, histamine liberation inhibitors, platelet activating factor (PAF) antagonists, serotonin antagonists, adenosine receptor antagonists, adrenergic β-receptor antagonists, immunosuppressive agents and immunomodulators.

The effects of the present invention will now be described concretely by way of examples.

Example 1

Methyl 2(R)-amino-3-(benzyloxycarbonylthio)propionate (1)

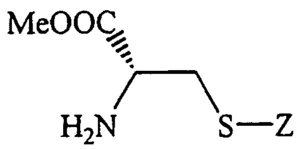

(1)

In 15 ml of 1N aqueous sodium hydrogen carbonate solution, 2.0 g (11.65 mmol) of L-cystein hydrochroride was dissolved and 15 ml of ether was added thereto. Under cooling in ice, 1.67 ml (11.65 mmol) of benzyloxycarbonyl chloride (Z-Cl) was added and the resulting mixture was stirred for 2 hours, followed by stirring at room temperature for 2 hours. To the reaction solution, 70 ml of sodium hydrogen carbonate solution was added and the resulting mixture was extracted with ethyl acetate (100 ml x 2). The organic layers were combined and washed with saturated brine (120 ml). The resulting mixture was dried over anhydrous magnesium sulfate and concentrated. The residue was purified by column chromatography (silica gel 100g; hexane:ethyl acetate = 4:1→2:1) to obtain 2.33 g (yield 74%) of methyl 2 (R)-amino-3-(benzyloxycarbonylthio)propionate (1) as colorless oil.

LR-MS (m/z) :269($M^+$)

$^1$H-NMR (300MHz, $CDCl_3$,δppm): 7.38 (5H, m), 5.68 (1H, bd), 5.13 (2H, s), 4.70 (1H, m), 3.79 (3H, s), 3.04 (2H, m), 1.42 (1H, t, J=8.8Hz)

Example 2

Methyl 2(R)-(4-methyl-2(R,S)-(4-methoxyphenyl)pentanoylamino)-3-(benzyloxycarbonylthio)propionate (2)

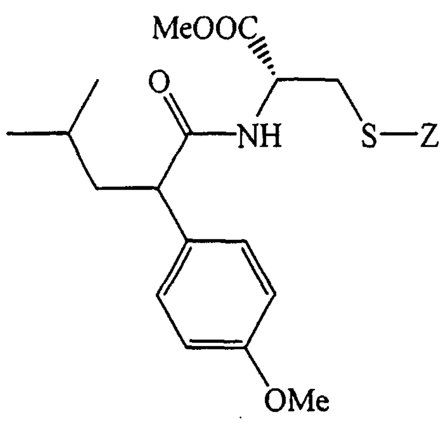

(2)

To a solution containing 539 mg (2.0 mmol) of methyl 2(R)-amino-3-(benzyloxycarbonylthio)propionate (1), 445 mg (2.0 mmol) of 2-(4-methoxyphenyl)-4-methylvaleric acid and 1.35 g (2.6 mmol) of PyBOP in DMF (4 ml), 0.33 ml (3.0 mmol) of N-methylmorpholine was added and the mixture was stirred overnight at room temperature. To the

reaction solution, 1N hydrochloric acid was added and the mixture was extracted with ethyl acetate (50 ml x 2). The organic layers were combined and washed with aqueous sodium hydrogen carbonate solution (70 ml) and with saturated brine (70 ml). The resulting mixture was dried over anhydrous sodium sulfate and concentrated. The residue was purified by column chromatography (silica gel 50g; hexane:ethyl acetate = 6:1→4:1→2:1) to obtain 892 mg (yield 94%) of methyl 2(R)-(4-methyl-2(R,S)-(4-methoxyphenyl)pentanoylamino)-3-(benzyloxycarbonylthio)propionate (2) as colorless oil.
LR-MS (m/z) :473($M^+$)
$^1$H-NMR (300MHz,$CDCl_3$, δ ppm): 0.86 (3H, d, J = 6.3Hz), 0.88 (3H, d, J = 6.3Hz), 1.43 (1H, m), 1.67 (1H, m), 1.92 (1H, m), 3.23-3.40 (2H, m), 3.65 (1.5H, s), 3.69 (1.5H, s), 3.77 (1H, m), 3.77 (3H, s), 4.57 (1H, m), 5.04-5.13 (2H, m), 5.35 (0.5H, d, J = 7.8Hz), 5.42 (0.5H, d, J = 7.8Hz), 6.81-6.91 (2H, m), 7.16-7.22 (2H, m), 7.32-7.35 (5H, m).

Example 3

Methyl 2(R)-(4-methyl-2(R,S)-(4-methoxyphenyl)pentanoylamino)-3-(sulfanyl)propionate (3)

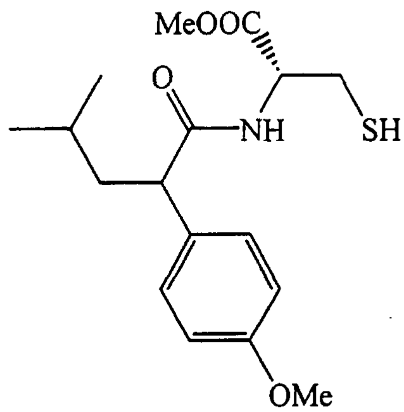

(3)

To 690 mg (1.46 mmol) of methyl 2(R)-(4-methyl-2(R,S)-(4-methoxyphenyl)pentanoylamino)-3-(benzyloxycarbonylthio)propionate (2), 5 ml of 30% HBr-acetic acid was added under cooling in ice and the mixture was stirred at room temperature for 30 minutes. To the reaction solution, aqueous sodium hydrogen carbonate solution was added and the resulting mixture was extracted with ethyl acetate (50 ml x 2). The organic layers were combined and washed with saturated brine (80 ml). The resulting mixture was dried over anhydrous sodium sulfate and concentrated. The residue was purified by column chromatography (silica gel 50g; hexane:ethyl acetate = 6:1→3:1) to obtain 420 mg (yield 85%) of methyl 2(R)-(4-methyl-2(R,S)-(4-methoxyphenyl)pentanoylamino)-3-(sulfanyl)propionate (3) as colorless oil.
LR-MS (m/z) :339($M^+$)
$^1$H-NMR (300MHz, $CDCl_3$, δppm): 0.88 (3H, d, J = 2.7Hz), 0.90 (3H, d, J = 2.7Hz), 1.44 (1H, m), 1.70 (1H, m), 1.98 (1H, m), 2.82-3.07 (2H, m), 3.48 (0.5H, t, J = 7.7Hz), 3.49 (0.5H, t, J = 7.7Hz), 3.73 (1.5H, s), 3.76 (1.5H, s), 3.80 (3H, s), 4.81 (1H, m), 6.27 (0.5H, d, J = 7.1Hz), 6.34 (0.5H, d, J = 7.1Hz), 6.86-6.91 (2H, m), 7.22-7.27 (2H, m).

Example 4

Methyl (S)-4-(methylsulfonyloxy)-2-((tert-butoxy)carbonylamino) butyrate (4)

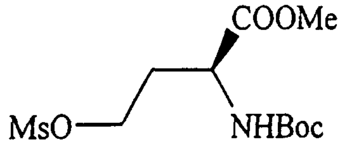

(4)

In 5 ml of dichloromethane, 213 mg of methyl (S)-4-hydroxy-2-((tert-butoxy)carbonylamino) butyrate was dissolved, and 0.4ml(2.87mmol) of triethylamine and 0.095ml(1.22mmol) of methanesulfonyl chloride were added under cooling the mixture in ice, followed by stirring the mixture at room temperature for 2 hours. To the reaction solution, water and 1N hydrochloric acid were added and the resulting mixture was extracted with ethyl acetate. The organic layers were combined and washed with saturated sodium hydrogen carbonate solution and saturated brine. The resulting mixture was dried over anhydrous sodium sulfate and concentrated under reduced pressure. The residue was purified by column chromatography (hexane:ethyl acetate = 2:1- 1:1) to obtain 272 mg (yield 96%) of methyl (S)-4-(methylsulfonyloxy)-2-((tert-butoxy)carbonylamino) butyrate (4) as colorless oil.
LR-MS (m/z): 311($M^+$)
$^1$H-NMR (300MHz,$CDCl_3$, δppm,): 1.44 (9H, s), 2.06 (1H, m), 2.33 (1H, m), 3.02 (3H, s), 3.77 (3H, s), 4.24-4.43 (2H, m), 4.45 (1H, m), 5.21 (1H, d, J = 7.4Hz).

Example 5

Methyl 2(S)-((tert-butoxy)carbonylamino)-4-(2(R)-(4-methyl-2(R,S)-(4-methoxyphenyl)pentanoylamino)-2(R) -(methoxycarbonyl)ethylthio)butyrate (5)

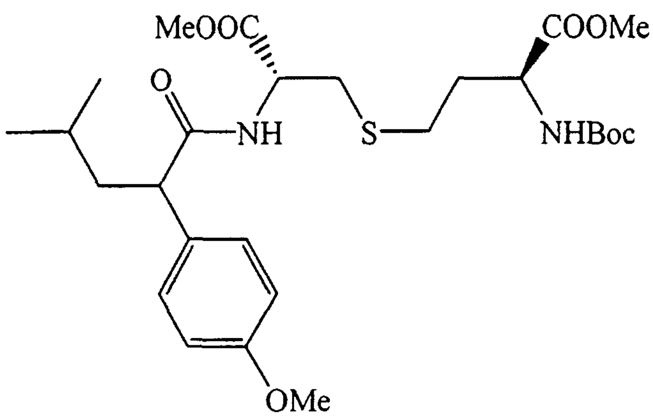

(5)

Under argon atmosphere, 123 mg (1.1 mmol) of t-BuOK was added to a solution (10 ml) of 407 mg (1.2 mmol) of methyl 2(R)-(4-methyl-2(R,S)-(4-methoxyphenyl)pentanoylamino)-3-(sulfanyl)propionate (3) in anhydrous THF while cooling the mixture in ice, followed by stirring the mixture for 30 minutes. To the reaction solution, a solution (5 ml) of methyl (S)-4-(methylsulfonyloxy)-2-((tert-butoxy)carbonylamino) butyrate (4) in anhydrous THF was added and the mixture was stirred at room temperature for 1 hour. To the reaction solution, 1N hydrochloric acid was added and the mixture was extracted with ethyl acetate (50 ml x 2). The organic layers were combined and washed with saturated brine (70 ml). The resulting product was dried over anhydrous sodium sulfate and concentrated. The residue was purified by column chromatography (silica gel 50 g; hexane:ethyl acetate = 3:1→1:1) to obtain 360 mg (yield 65%) of methyl 2(S)-((tert-butoxy)carbonylamino)-4-(2(R)-(4-methyl-2(R,S)-(4-methoxyphenyl)pentanoylamino-2(R)-(methoxycarbonyl)ethylthio)butyrate (5) as colorless oil.
LR-MS (m/z): 554($M^+$)
$^1$H-NMR (300MHz,$CDCl_3$, δppm): 0.87-0.91 (6H, m), 1.26 (1H, m), 1.41-1.46 (9H, m), 1.64-1.80 (2H, m), 1.82-2.02 (2H, m), 2.36 (1H, m), 2.49 (1H, m), 2.87 (1H, m), 2.94 (1H, m), 3.49 (1H, m), 3.70-3.76 (6H, m), 3.80 (3H, s), 4.33 (1H, m), 4.74 (1H, m), 5.13 (1H, m), 6.32 (1H, m), 6.83-6.90 (2H, m), 7.19-7.27 (2H, m).

Example 6

Methyl 2(S)-amino-4-(2(R)-(4-methyl-2(R,S)-(4-methoxyphenyl)pentanoylamino-2(R)-(methoxycarbonyl)ethylthio) butyrate (6)

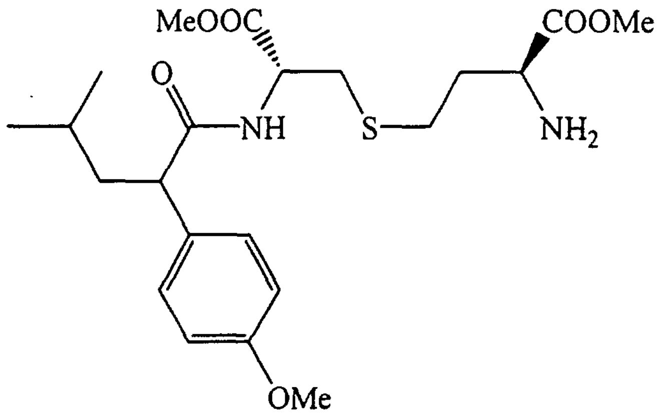

(6)

To a solution of 200 mg (0.36 mmol) of methyl 2(S)-((tert-butoxy)carbonylamino)-4-(2(R)-(4-methyl-2(R,S) -(4-methoxyphenyl)pentanoylamino)-2(R)-(methoxycarbonyl)ethylthio)butyrate (5) in dichloromethane (2 ml), 1 ml of trifluoroacetic acid was added and the mixture was stirred at room temperature for 1 hour. After concentrating the mixture, the residue was subjected to azeotropic distillation with toluene twice to obtain methyl 2(S)-amino-4-(2(R) -(4-methyl-2(R,S)-(4-methoxyphenyl)pentanoylamino-2(R)-(methoxycarbonyl)ethylthio) butyrate (6). The obtained (6) was used in the next reaction without purification.

Example 7

Methyl 2(S)-(4-methyl-2(R,S)-(4-methoxyphenyl)pentanoylamino-4-(2(R)-(4-methyl-2(R,S)-(4-methoxyphenyl) pentanoylamino)-2(R)-(methoxycarbonyl)ethylthio) butyrate (7)

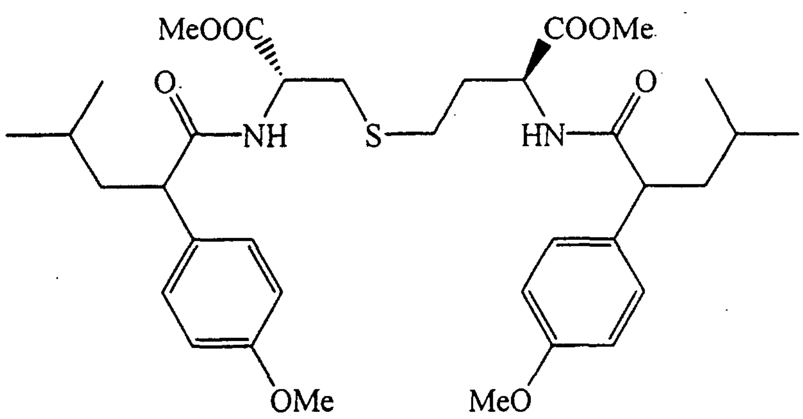

(7)

To methyl 2(S)-amino-4-(2(R)-(4-methyl-2(R,S)-(4-methoxyphenyl)pentanoylamino-2(R)-(methoxycarbonyl) ethylthio) butyrate (6), a solution (4 ml) of 95.6 mg (0.43 mmol) of 2-(4-methoxyphenyl)-4-methyl valeric acid in dichloromethane, 190 mg (0.43 mmol) of BOP reagent and 0.25 ml (1.44 mmol) of i-$Pr_2$NEt were added, and the mixture was stirred at room temperature overnight. To the reaction solution, 1N hydrochloric acid was added and the mixture was extracted with ethyl acetate (50 ml x 2). The organic layers were combined and washed with saturated brine (70 ml). The resulting product was dried over anhydrous sodium sulfate and concentrated. The residue was purified by column chromatography (silica gel 20 g; hexane:ethyl acetate = 2:1→2:3) to obtain 197 mg (yield 83%) of methyl 2(S)-(4-methyl-2(R,S)-(4-methoxyphenyl)pentanoylamino-4-(2(R)-(4-methyl-2(R,S)-(4-methoxyphenyl)pentanoylamino)-2(R)-(methoxycarbonyl)ethylthio) butyrate (7) as colorless oil.
LR-MS (m/z) :658($M^+$)
$^1$H-NMR (300MHz,$CDCl_3$, δppm): 0.88-0.91 (12H, m), 1.42-1.44 (2H, m), 1.65-1.75 (2H, m), 1.91-2.00 (4H, m), 2.24 (1H, m), 2.67-2.89 (3H, m), 3.45-3.50 (2H, m), 3.64-3.69 (6H, m), 3.71-3.79 (6H, m), 4.54-4.73 (2H, m), 6.18-6.34 (2H, m), 6.81-6.92 (4H, m), 7.17-7.28 (4H, m).

Example 8

4-(2(R)-carboxy-2(R)-(2(R,S)-(4-hydroxyphenyl)-4-methylpentanoylamino)ethylthio)-2(S)-(2(R,S)-(4-hydroxyphenyl)-4-methylpentanoylamino) butyric acid (8)

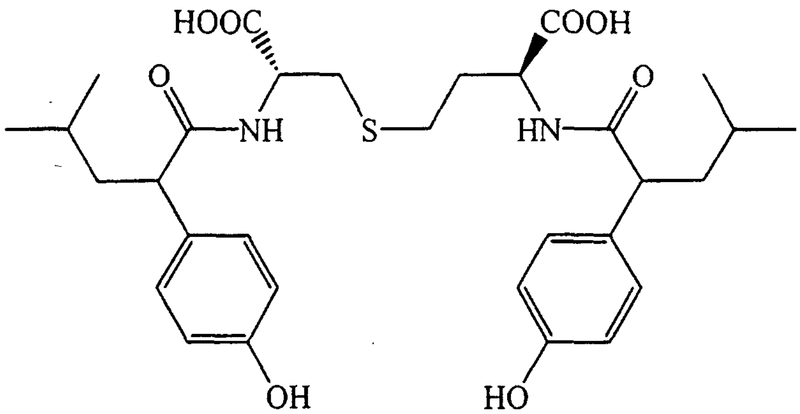

(8)

To a solution of 83 mg (0.13 mmol) of methyl 2(S)-(4-methyl-2(R,S)-(4-methoxyphenyl)pentanoylamino-4-(2(R)-(4-methyl-2(R,S)-(4-methoxyphenyl)pentanoylamino)-2(R)-(methoxycarbonyl)ethylthio) butyrate (7) in methanol (2 ml), 0.4 ml of 1N aqueous sodium hydroxide solution was added and the mixture was stirred at room temperature for 3 hours. To the reaction solution, 1N hydrochloric acid was added and the mixture was extracted with ethyl acetate (50 ml x 2). The organic layers were combined and washed with saturated brine (70 ml). The resulting product was dried over anhydrous sodium sulfate and concentrated. The residue was dissolved in anhydrous dichloromethane (2 ml) and the solution was cooled to -78°C. To the reaction solution, boron tribromide (1.0M in $CH_2Cl_2$)0.75 ml (0.60 mmol) was added and the mixture was stirred at room temperature for 3 hours. To the reaction solution, water was added and the mixture was extracted with ethyl acetate (50 ml x 2). The organic layers were combined and washed with saturated brine (70 ml). The resulting product was dried over anhydrous sodium sulfate and concentrated. The residue was purified by medium-pressure liquid chromatography (Yamazen Ultrapack DIOL-40B; hexane:ethyl acetate = 50:50→ 20:80) to obtain 10 mg (yield 13%) of 4-(2(R)-carboxy-2(R)-(2(R,S)-(4-hydroxyphenyl)-4-methylpentanoylamino)ethylthio)-2(S)-(2(R,S)-(4-hydroxyphenyl)-4-methylpentanoylamino) butyric acid (8) as colorless oil.
LR-MS(m/z): 603$(M+H)^+$
$^1$H-NMR (300MHz,$CD_3OD$, δppm); 0.89-0.93 (12H, m), 1.45-1.56 (4H, m), 1.89-1.98 (3H, m), 2.15-2.21 (2H, m), 2.33-2.58 (3H, m), 3.56-3.63 (2H, m), 4.44-4.51 (2H, m), 6.68-6.72 (4H, m), 7.13-7.18 (4H, m).

Example 9

Methyl 2(R)-(3,3-dimethylbutanoylamino)-3-(benzyloxycarbonylthio) propionate (9)

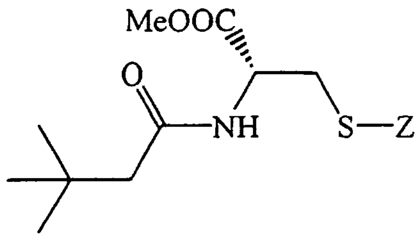

(9)

Under cooling in ice, methyl 2(R)-amino-3-(benzyloxycarbonylthio)propionate (1) (1.5 g, 5.57 mmol) was dissolved in chloroform (2 ml), and 2 ml of pyridine and tert-butylacetyl chloride (825mg, 6.13mmol, 1.1eq) were added, followed by stirring the resulting mixture at room temperature for 2 hours. To the reaction solution, water and 6N hydrochloric acid were added to attain a pH of 1, and the resulting mixture was extracted with ethyl acetate. The organic layers were combined and washed with saturated sodium hydrogen carbonate solution and saturated brine. The resulting mixture was dried over anhydrous magnesium sulfate and concentrated under reduced pressure. The residue was purified by silica gel column chromatography to obtain 1.67 g (yield 82%) of methyl 2(R)-(3,3-dimethylbutanoylamino)-3-(benzyloxycarbonylthio) propionate (9).
LR-MS(m/z): 367($M^+$)
$^1$H-NMR (300MHz, $CDCl_3$, δppm): 7.37-7.31 (5H, m), 5.51 (1H, bd, J=7.4Hz), 5.11 (2H, s), 4.60 (1H, m), 3.76 (3H, s), 3.35 (2H, d, J=5.5Hz), 2.43 (2H, s), 1.59 (1H, s), 1.00 (9H, s)

Example 10

Methyl 2(R)-(3,3-dimethylbutanoylamino)-3-sulfanylpropionate (10)

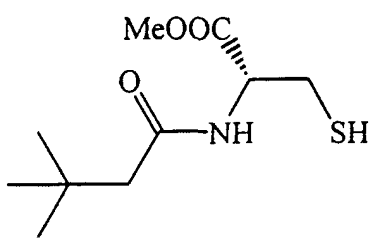

(10)

In 10 ml of TFA, methyl 2(R)-(3,3-dimethylbutanoylamino)-3-(benzyloxycarbonylthio) propionate (9) (1.6g, 4.35mmol) was dissolved and the mixture was heated at 100°C. After concentrating the reaction solution under reduced pressure, water was added and the resulting mixture was extracted with ethyl acetate. The organic layers were combined and washed with saturated sodium hydrogen carbonate solution and saturated brine. The resulting mixture was dried over anhydrous magnesium sulfate and concentrated under reduced pressure. The residue was purified by silica gel column chromatography to obtain 713 mg of methyl 2(R)-(3,3-dimethylbutanoylamino)-3-sulfanylpropionate (10).
LR-MS(m/z): 233($M^+$)
$^1$H-NMR (300MHz,$CDCl_3$, δppm): 6.24 (1H, bs), 4.93 (1H, m), 3.80 (3H, m), 3.02 (2H, m), 2.18 (2H, s), 1.32 (1H, t), 1.03 (9H, s)

Example 11

Methyl 4-(2(R)-(3,3-dimethylbutanoylamino)-2(RR)-(methoxycarbonyl)ethylthio)-2(S)-((tert-butoxy)carbonylamino) butyrate (11)

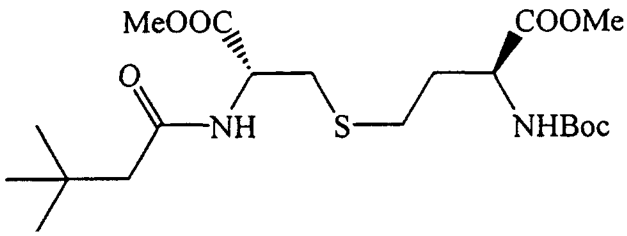

(11)

In 2 ml of THF, 107mg (459 μmol) of methyl 2(R)-(3,3-dimethylbutanoylamino)-3-sulfanylpropionate (10) was dissolved and 5mg (0.45mmol) of potassium t-butoxide was added thereto, followed by stirring the mixture at room temperature for 30 minutes. To the reaction solution, 150 mg (481 μmol) of (S)-4-(methylsulfonyloxy)-2-((tert-butoxy) carbonylamino) butyrate (4) dissolved in 0.4 ml of THF was added, and the mixture was stirred at room temperature for 1 hour. Water was added to the reaction solution and the resulting mixture was extracted with ethyl acetate. The organic layers were combined and washed with saturated brine. The obtained product was dried over anhydrous sodium sulfate and concentrated. The residue was purified by column chromatography (hexane:ethyl acetate = 2:1-1:1) to obtain 167 mg (yield 81%) of methyl 4-(2(R)-(3,3-dimethylbutanoylamino)-2(RR)-(methoxycarbonyl)ethylthio)-2(S) -((tert-butoxy)carbonylamino) butyrate (11) as colorless oil.
LR-MS (m/z) :448($M^+$)
$^1$H-NMR (300MHz, $CDCl_3$, δppm) : 1.05, 1.06 (9H, s), 1.44, 1.45 (9H, s), 1.89 (1H, m), 2.09 (1H, m), 2.13, 2.14 (2H, s), 2.53-2.61 (2H, m), 2.92-3.04 (2H, m), 3.76, 3.77, 3.78 (6H, s), 4.41 (1H, m), 4.80 (1H, m), 5.18 (1H, m), 6.29 (1H, m).
$[\alpha]^{18}_D$ : +14.8° (c=1.670, $CHCl_3$)

Example 12

Methyl 2(S)-amino-4-(2(R)-(3,3-dimethylbutanoylamino)-2(R)-(methoxycarbonyl)ethylthio) butyrate (12)

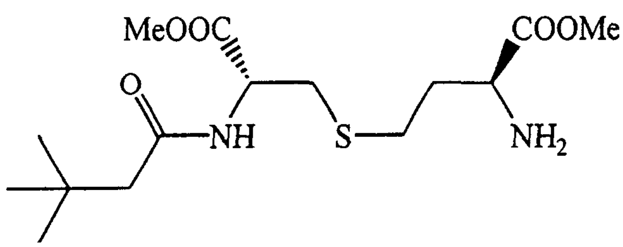

(12)

In 3 ml of dichloromethane, 157mg (350mmol) of methyl 4-(2(R)-(3,3-dimethylbutanoylamino)-2(RR)-(methoxycarbonyl)ethylthio-2(S)-((tert-butoxy)carbonylamino) butyrate (11) was dissolved and 1.5 ml of trifluoroacetic acid was added, followed by stirring the mixture at room temperature for 15 minutes. The reaction solution was concentrated under reduced pressure to obtain methyl 2(S)-amino-4-(2(R)-(3,3-dimethylbutanoylamino)-2(R)-(methoxycarbonyl) ethylthio) butyrate (12) as crude product. The product (12) was subjected to azeotropic distillation with toluene and then used in the next reaction without purification.

Example 13

Methyl 2(S)-(2(R,S)-(4-acetyloxyphenyl)-4-methylpentanoylamino)-4-(2(R)-(3,3-dimethylbutanoylamino)-2(R)-(methoxycarbonyl)ethylthio) butyrate (13)

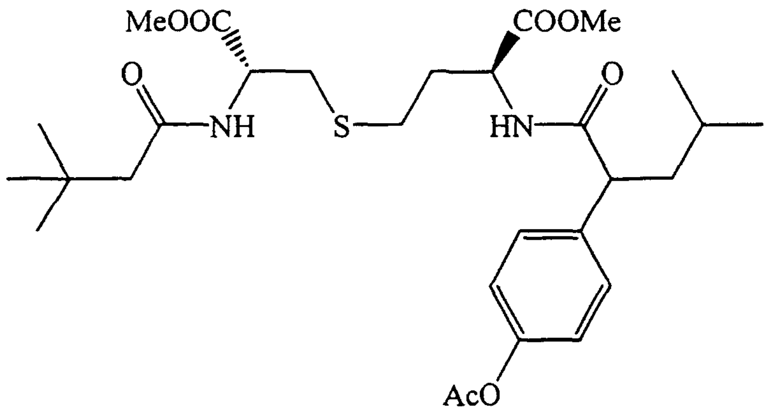

(13)

In 3 ml of dichloromethane, 90mg (0.36mmmol) of 2-(4-acetoxyphenyl)-4-methyl valeric acid was dissolved and 180 mg (407 μmol) of BOP reagent was added, followed by stirring the mixture at room temperature for 1 hour. To the reaction solution, methyl 2(S)-amino-4-(2(R)-(3,3-dimethylbutanoylamino)-2(R)-(methoxycarbonyl)ethylthio) butyrate (12) and 250 μl (1.43 mmol) of diisopropylethylamine were added and the resulting mixture was stirred at room temperature for 1 hour. Water was added to the reaction solution and the mixture was extracted with ethyl acetate. The organic layers were combined and washed with 1N hydrochloric acid, saturated sodium hydrogen carbonate solution and saturated brine. The resulting mixture was dried over anhydrous sodium sulfate and concentrated under reduced pressure. The residue was purified by column chromatography (hexane: ethyl acetate = 2:1 - 3:2 - 1:1 - 1:2) to obtain 191 mg of methyl 2(S)-(2(R,S)-(4-acetyloxyphenyl)-4-methylpentanoylamino)-4-(2(R)-(3,3-dimethylbutanoylamino)-2(R)-(methoxycarbonyl)ethylthio) butyrate (13) as colorless oil (yield 94%).
LR-MS (m/z): 580($M^+$)

Example 14

4-(2(R)-carboxy-2(R)-(3,3-dimethylbutanoylamino)ethylthio)-2(S)-(2(R,S)-(4-hydroxyphenyl)-4-methylpentanoylamino) butyric acid (14)

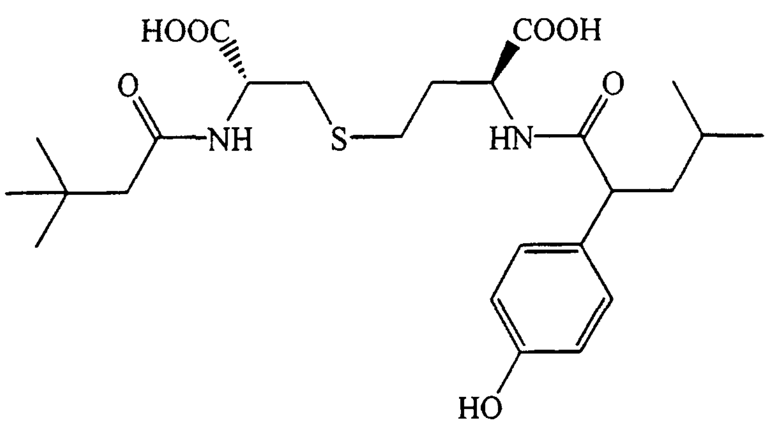

(14)

In 4 ml of methanol, 172 mg (296 μmol) of methyl 2(S)-(2(R,S)-(4-acetyloxyphenyl)-4-methylpentanoylamino)-4-(2(R)-(3,3-dimethylbutanoylamino)-2(R)-(methoxycarbonyl)ethylthio) butyrate (13) was dissolved, and 1.3ml

(1.17mmol) of 1N aqueous sodium hydroxide solution was added, followed by stirring the mixture at room temperature for 10 hours. To the reaction solution, 1N hydrochloric acid was added and the mixture was extracted with ethyl acetate. The organic layers were combined and washed with saturated brine. The resulting mixture was dried over anhydrous sodium sulfate and concentrated under reduced pressure. The residue was purified by diol Lobar column (hexane: ethyl acetate = 1:2 - ethyl acetate 100%) to obtain 138 mg of 4-(2(R)-carboxy-2(R)-(3,3-dimethylbutanoylamino)ethylthio)-2(S)-(2(R,S)-(4-hydroxyphenyl)-4-methylpentanoylamino) butyric acid (14) (yield 93%).
LR-MS(m/z):511$(M+H)^+$
$^1$H-NMR (300MHz, $CDCl_3$, δppm): 0.85-0.98 (6H, m), 1.04 (9H, s), 1.35-1.54 (1H, m), 1.58-1.74 (1H, m), 1.74-2.09 (3H, m), 2.14 (2H, s), 2.20-2.38 (1H, m), 2.38-2.56 (1H, m), 2.72-3.03 (2H, m), 3.43-3.48 (1H, m), 4.45-4.74 (2H, m), 6.42-6.67 (2H, m), 6.78-6.82 (2H, m), 7.11-7.15 (2H, m), 0.84-0.89 (6H, m), 1.04 (9H, s), 1.12-1.28 (1H, m), 1.35-1.48 (1H, m), 1.55-1.70 (5H, m), 2.13, 2.14 (2H, s), 2.19-2.31 (1H, m), 2.50 (2H, t, J = 7.0Hz), 3.13-3.31 (4H, m), 4.74-4.90 (2H, m), 6.63-6.72 (2H, m), 6.76 (2H, dd, J = 3.3, 8.2Hz), 6.99 (2H, d, J = 8.2Hz).
HR-MS: calcd. for $C_{25}H_{39}N_2O_7S$ 511.2478 found. 511.2479

Example 15

N-(3-(methylsulfonyloxy)propyl)(tert-butoxy)formamide (15)

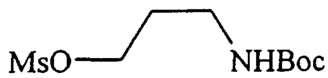

(15)

In 2 ml of dichloromethane, N-(3-hydroxypropyl)(tert-butoxy)formamide (200 mg, 1.14 mmol) was dissolved, and triethylamine (0.32 ml, 2.28 mmol) and methanesulfonyl chloride (0.12ml, 1.72mmol) were added, followed by stirring the mixture at room temperature for 1 hour. Water was added to the reaction solution and the mixture was extracted with ethyl acetate. The organic layers were combined and washed with saturated sodium hydrogen carbonate solution and saturated brine. The resulting mixture was dried over anhydrous magnesium sulfate and concentrated under reduced pressure. The residue was purified by silica gel column chromatography to obtain 230 mg (yield 80%) of N-(3-(methylsulfonyloxy)propyl)(tert-butoxy)formamide (15).
LR-MS(m/z):253$(M^+)$
$^1$H-NMR(300MHz, $CDCl_3$, δppm): 4.75 (1H, bs), 4.32 (2H, t), 3.26 (2H, m), 3.03 (3H, s), 1.95 (2H, m), 1.44 (9H, s)

Example 16

Methyl 2(R)-(3,3-dimethylbutanoylamino)-3-(3-((tert-butoxy)carbonylamino)propylthio)propionate (16)

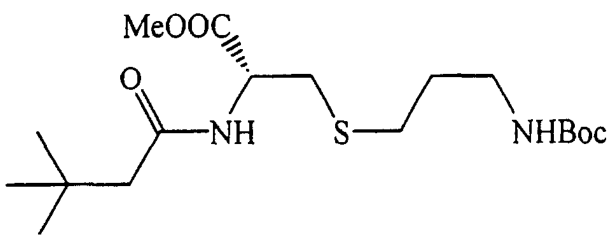

(16)

To a solution of methyl 2(R)-(3,3-dimethylbutanoylamino)-3-sulfanylpropionate (10) (161mg, 0.69mmol) in THF (3 ml), potassium tert-butoxide (71mg, 0.64mmol) was added and the mixture was stirred for 15 minutes. To the solution, N-(3-(methylsulfonyloxy)propyl)(tert-butoxy)formamide (15) (135mg, 0.53mmol) dissolved in 2 ml of THF was added and the mixture was stirred for 30 minutes. Water and 1N hydrochloric acid were added to the reaction solution and the resulting mixture was extracted with ethyl acetate. The organic layers were combined and washed with saturated brine. The resulting mixture was dried over anhydrous magnesium sulfate and concentrated under reduced pressure. The residue was purified by column chromatography to obtain 257 mg (yield 85%) of methyl 2(R)-(3,3-dimethylbutanoylamino)-3-(3-((tert-butoxy)carbonylamino)propylthio)propionate (16).
LR-MS (m/z):391$(M+H)^+$

IR(KBr): 3324, 2954, 1742, 1693, 1656, 1527, 1437, 1366, 1251, 1173 $cm^{-1}$
$^1H$-NMR (300MHz,$CDCl_3$, δppm): 6.21 (1H, bd), 4.82 (1H, m), 4.75 (1H, bs), 3.77 (3H, s), 3.19 (2H, m), 3.00 (2H, m), 2.58 (2H, m), 2.14 (2H, s), 1.78 (2H, m), 1.44 (9H, s), 1.06 (9H, s)

Example 17

Methyl 2(R)-(3,3-dimethylbutanoylamino)-3-(3-aminopropylthio)propionate (17)

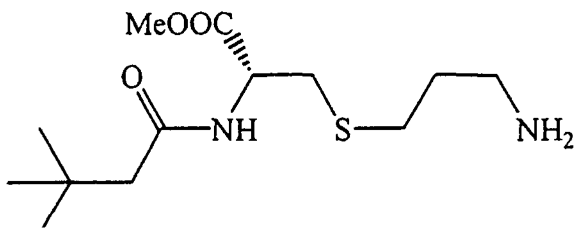

(17)

In 5 ml of dichloromethane, methyl 2(R)-(3,3-dimethylbutanoylamino)-3-(3-((tert-butoxy)carbonylamino)propylthio)propionate (16) (250 mg, 0.64 mmol) was dissolved and 1 ml of TFA was added thereto, followed by stirring the mixture at room temperature for 1 and half hours. The reaction solution was concentrated under reduced pressure to obtain methyl 2(R)-(3,3-dimethylbutanoylamino)-3-(3-aminopropylthio)propionate (17). The product (17) was used in the next reaction without purification.

Example 18

Methyl 3-(3-(2(R,S)-(4-acetyloxyphenyl)-4-methylpentanoylamino)propylthio)-2(R)-(3,3-dimethylbutanoylamino) propionate (18)

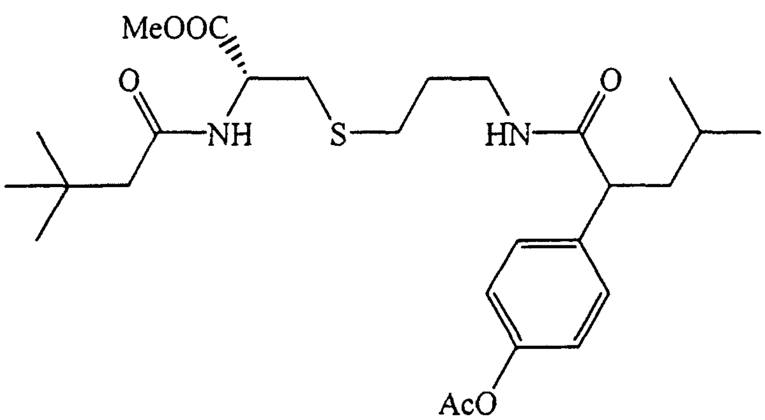

(18)

To a solution of 2-(4-acetoxyphenyl)-4-methyl valeric acid (192 mg, 0.77 mmol) in dichloromethane, BOP reagent (340 mg, 0.77 mmol) was added and the mixture was stirred at room temperature for 10 minutes. Then methyl 2(R)-(3,3-dimethylbutanoylamino)-3-(3-aminopropylthio)propionate (17) dissolved in 3 ml of dichloromethane was added and then diisopropylethylamine (331 mg, 2.56 mmol) was added to the mixture, followed by stirring the mixture for 2 hours. Water was added to the reaction solution and the mixture was extracted with ethyl acetate. Organic layers were combined and washed with 1N hydrochloric acid, saturated sodium hydrogen carbonate solution and saturated brine. The resulting mixture was dried over anhydrous magnesium sulfate and concentrated under reduced pressure. The residue was purified by silica gel column chromatography to obtain 253 mg (71%) of methyl 3-(3-(2(R,S)-(4-acetyloxyphenyl)-4-methylpentanoylamino)propylthio)-2(R)-(3,3-dimethylbutanoylamino)propionate (18).
LR-MS(m/z): 523 $(M+H)^+$
IR(neat): 3301, 3070, 2954, 1747, 1651, 1538, 1437, 1369, 1203, 1018 $cm^{-1}$

$^1$H-NMR (300MHz,$CDCl_3$ δppm): 7.35 (2H, d, J=8.5Hz), 7.03 (2H, d, J=8.5Hz), 6.20 (2H, m), 4.78 (1H, m), 3.76 (3H, s), 3.49 (1H, t, J=7.7Hz), 3.42-3.14 (2H, m), 2.98-2.72 (2H, m), 2.55-2.43 (2H, m), 2.30 (3H, s), 2.14 (2H, s), 2.00 (1H, m), 1.77-1.59 (4H, m), 1.45 (1H, m), 1.06 (9H, s), 0.90 (6H, m)

Example 19

3-(3-(2(R,S)-(4-hydroxyphenyl)-4-(methylpentanoylamino)propylthio-2(R)-(3,3-dimethylbutanoylamino)propionic acid (19)

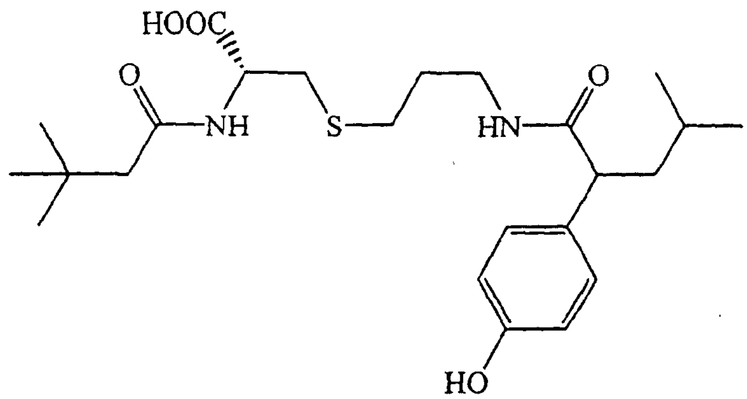

(19)

To a solution of methyl 3-(3-(2(R,S)-(4-acetyloxyphenyl)-4-methylpentanoylamino)propylthio)-2(R)-(3,3-dimethylbutanoylamino)propionate (18) (240 mg, 0.43 mmol) in methanol (2 ml), aqueous 1N sodium hydroxide solution (0.15ml, 1.30 mmol, 3.0eq) was added and the mixture was stirred at room temperature for 4 hours. To the reaction solution, 1N hydrochloric acid was added to attain a pH of 1, and the resulting mixture was extracted with ethyl acetate. Organic layers were combined and washed with saturated brine. The resulting mixture was dried over anhydrous magnesium sulfate and concentrated under reduced pressure. The residue was purified by diol Lobar column chromatography to obtain 164 mg (82%) of 3-(3-(2(R,S)-(4-hydroxyphenyl)-4-(methylpentanoylamino)propylthio-2(R)-(3,3-dimethylbutanoylamino)propionic acid (19).
LR-MS(m/z):467$(M+H)^+$
IR(KBr): 3321, 296, 1726, 1648, 1514, 1448, 1368, 1235, 838 $cm^{-1}$
$^1$H-NMR (300MHz,$CDCl_3$, δppm): 7.13 (2H, d, J=7.7Hz), 6.80 (2H, d, J=8.5Hz), 6.49 (1H, d, J=7.7Hz), 6.33 (1H, m), 4.70 (1H, m), 3.40 (1H, t, J=8.0Hz), 3.38-3.12 (2H, m), 3.04-2.78 (2H, m), 2.60-2.43 (2H, m), 2.13 (2H, s), 2.02-1.88 (2H, m), 1.71-1.65 (2H, m), 1.45 (1H, m), 1.05 (9H, s), 1.90-1.86 (6H, m)

Example 20

Methyl 2(R)-(3,3-dimethylbutanoylamino)-3-(3-(4-methyl-2(S)-(4-methoxyphenyl)pentanoylamino)propylthio) propionate (20)

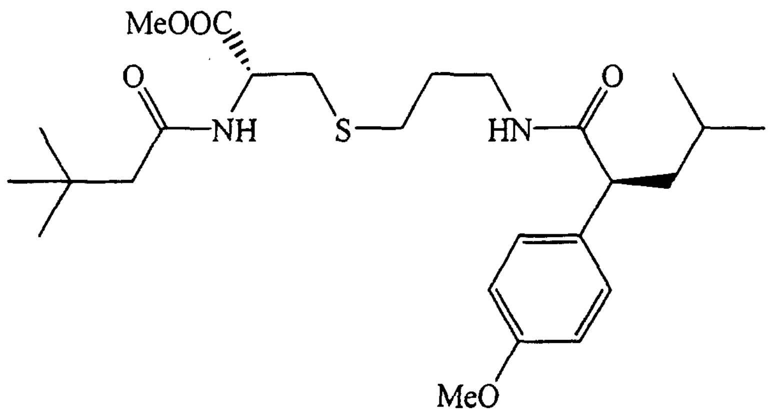

(20)

Under argon atmosphere, to a solution of 209.1 mg (0.72 mmol) of methyl 2(R)-(3,3-(dimethylbutanoylamino)-3-(3-aminopropylthio)propionate (17) and 123.2 mg (0.554 mmol) of (S)-2-(4-acetoxyphenyl)-4-methyl valeric acid in dichloromethane (4 ml), 274.4 mg (0.62 mmol) of BOP reagent and 0.4ml (2.3 mmol) of diisopropylethylamine were added while cooling the mixture in ice, and the mixture was stirred overnight at room temperature. To the reaction solution, 4 ml of 0.1N hydrochloric acid and 1 ml of water were added and the mixture was extracted with ethyl acetate (3 x 12 ml). Organic layers were combined and washed with saturated brine. The resulting mixture was dried over anhydrous sodium sulfate and concentrated under reduced pressure. The obtained oily product was purified by column chromatography (silica gel; chloroform/methanol (50/1)) to obtain 270.3 mg of methyl 2(R)-(3,3-dimethylbutanoylamino)-3-(3-(4-methyl-2(S)-(4-methoxyphenyl)pentanoylamino)propylthio)propionate (20) as colorless oil (yield 99%).
LR-MS(m/z):495$(M+H)^+$
IR (neat):3301, 2955, 2868, 2243, 1746, 1647, 1611, 1510, 1465, 1437, 1366, 1249, 1178, 1036, 910, 835, 793 $cm^{-1}$.
$^1$H-NMR (300MHz,$CDCl_3$, δppm):7.23 (2H, d), 6.85 (2H, d), 6.20 (1H, br), 5.90 (1H, br), 4.82-4.70 (1H, m), 3.79 (3H, s), 3.75 (3H, s), 3.42 (1H, t), 3.32 (1H, ddd), 3.25-3.10 (1H, m), 3.0-2.85 (1H, m), 2.76 (1H, dd), 2.50-2.38 (2H, m), 2.13 (2H, s), 2.04-1.88 (1H, m), 1.75-1.55 (3H, m), 1.46-1.37 (1H, m), 1.05 (9H, s), 0.90 (6H, d).

Example 21

2(R)-(3,3-dimethylbutanoylamino)-3-(3-(4-methyl-2(S)-(4-methoxyphenyl)pentanoylamino)propylthio)propionic acid (21)

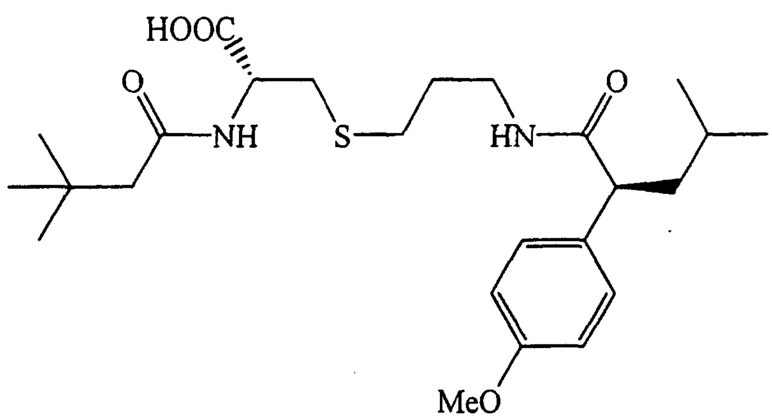

(21)

Under argon atmosphere, to a solution of 270.3 mg (0.546 mmol) of methyl 2(R)-(3,3-dimemylbutanoylamino)-3-(3-(4-methyl-2(S)-(4-methoxyphenyl)pentanoylamino)propylthio)propionate (20) in 5 ml of methanol, 2 ml of aqueous 1N sodium hydroxide solution was added and the mixture was stirred at room temperature for 1.5 hours. To the reaction solution, 2.2 ml of 1N hydrochloric acid and 5 ml of water were added and the mixture was extracted with ethyl acetate (3 x 12 ml). Organic layers were combined and washed with 6 ml of saturated brine. The resulting mixture was dried over anhydrous sodium sulfate and concentrated under reduced pressure. The obtained oily product was purified by column chromatography (silica gel; chloroform/methanol (10/1)) to obtain 225.4 mg of 2(R)-(3,3-dimethylbutanoylamino)-3-(3-(4-methyl-2(S)-(4-methoxyphenyl)pentanoylamino)propylthio)propionic acid (21) as colorless oil (yield 86%).
LR-MS(m/z):481(M+H)$^+$
IR (neat): 3301, 2955, 1719, 1646, 1510, 1466, 1367, 1249, 1179, 1036, 835, 536cm$^{-1}$.
$^1$H-NMR (300MHz,CDCl$_3$, δppm):7.20 (2H, d), 6.85 (2H, d), 6.63 (1H, br), 5.92 (1H, br), 4.78-4.62 (1H, m), 3.79 (3H, s), 3.50-3.40 (2H, m), 3.18-2.90 (2H, m), 2.89-2.80 (1H, m), 2.55-1.85 (5H, m), 1.74-1.58 (3H, m), 1.42-1.35 (1H, m), 1.04 (9H, s), 0.85 (3H, d), 0.84 (3H, d).

Example 22

3-(3-(2(S)-(4-hydroxyphenyl)-4-methylpentanoylamino)propylthio)-2(R)-(3,3-dimethylbutanoylamino)propionic acid (22)

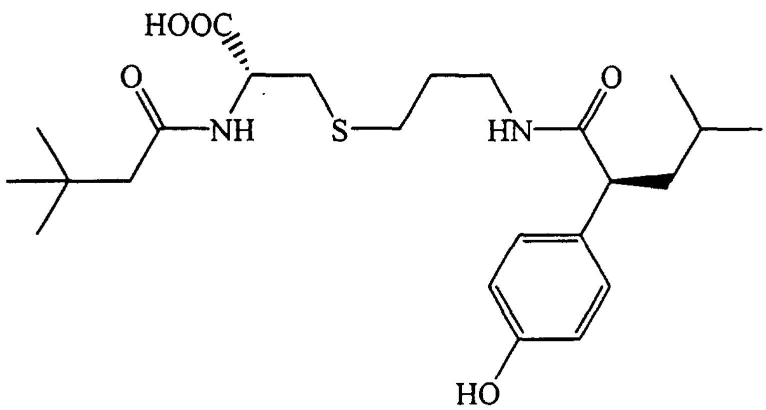

(22)

Under argon atmosphere, to a solution of 225.4 mg (0.469 mmol) of 2(R)-(3,3-dimethylbutanoylamino)-3-(3-(4-methyl-2(S)-(4-methoxyphenyl)pentanoylamino)propylthio)propionic acid (21) in 5 ml of dichloromethane, 3.4 ml of 1N boran tribromide solution in dichloromethane was added and the mixture was stirred for 5 hours while cooling the mixture in ice. To the reaction solution, 5 ml of water was added and the mixture was extracted with ethyl acetate (3 x 12 ml). Organic layers were combined and washed with 6 ml of saturated brine. The resulting mixture was dried over anhydrous sodium sulfate and concentrated under reduced pressure. The obtained oily product was purified by column chromatography (DIOL, hexane:ethyl acetate (1/5)) to obtain 75.4 mg of 3-(3-(2(S)-(4-hydroxyphenyl)-4-methylpentanoylamino)propylthio)-2(R)-(3,3-dimethylbutanoylamino)propionic acid (22) as colorless oil (yield 34%).
LR-MS(m/z):467(M+H)$^+$
IR (neat):3313, 2956, 2869, 1735, 1647,1512,1447,1368, 1233, 837, 532 cm$^{-1}$.
$^1$H-NMR (300MHz,CD,OD, δ ppm):8.05 (1H, m), 7.14 (2H, d), 6.70 (2H, d), 4.58-4.52 (1H, m), 3.46 (1H, t), 3.25-3.13 (3H, m), 2.97 (1H, ddd), 2.76 (1H, ddd), 2.51-2.45 (2H, m), 2.13 (2H, s), 1.93-1.86 (1H, m), 1.75-1.67 (2H, m), 1.56-1.39 (1H, m), 1.03 (9H, s), 0.91 (3H, d), 0.90 (3H, d).
HR-MS:$C_{22}H_{39}N_2O_5S$ Calcd.: 467.2580, Found: 467.2617

Example 23

Methyl 2(R)-(3,3-dimethylbutanoylamino)-3-(3-(4-methyl-2(R)-(4-methoxyphenyl)pentanoylamino)propylthio) propionate (23)

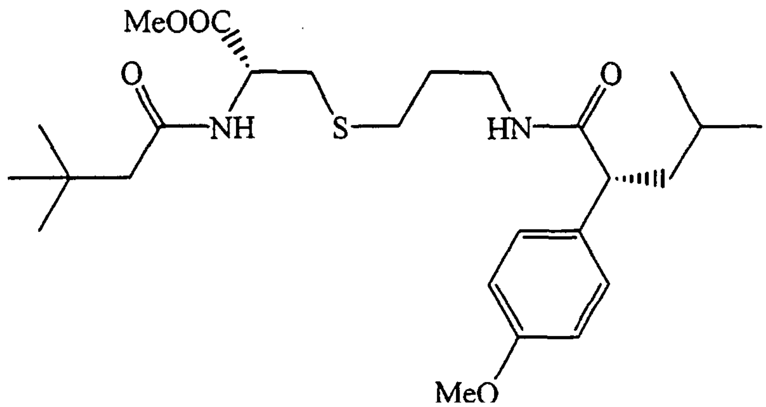

(23)

Under argon atmosphere, to a solution of 212.0 mg (0.73 mmol) of methyl 2(R)-(3,3-(dimethylbutanoylamino)-3-(3-aminopropylthio)propionate (17) in dichloromethane (5 ml), 139.0 mg (0.625 mmol) of (R)-2-(4-acetoxyphenyl)-4-methyl valeric acid, 316.3 mg (0.715 mmol) of BOP reagent and 0.45ml (2.58 mmol) of diisopropylethylamine were added while cooling the mixture in ice, and the mixture was stirred overnight at room temperature. To the reaction solution, 4 ml of 0.1N hydrochloric acid and 1 ml of water were added and the mixture was extracted with ethyl acetate (3 x 12 ml). Organic layers were combined and washed with saturated brine. The resulting mixture was dried over anhydrous sodium sulfate and concentrated under reduced pressure. The obtained oily product was purified by column chromatography (NH-coated silica gel, hexane/ethyl acetate (1/1)) to obtain 301.1 mg of methyl 2(R)-(3,3-dimethylbutanoylamino)-3-(3-(4-methyl-2(R)-(4-methoxyphenyl)pentanoylamino)propylthio)propionate (23) as colorless oil (yield 97%).
LR-MS(m/z):495$(M+H)^+$
IR (neat):3295, 2953, 2867, 1747, 1645, 1610, 1542, 1509, 1465, 1437, 1366, 1248, 1178, 1035, 835 $cm^{-1}$.
$^1$H-NMR (300MHz,$CDCl_3$, δppm):7.23 (2H, d, J = 8.5 Hz), 6.85 (2H, d, J = 8.5 Hz), 6.18 (1H, br), 5.93 (1H, br), 4.82-4.72 (1H, m), 3.79 (3H, s), 3.75 (3H, s), 3.42 (1H, t, J = 7.7 Hz), 3.32 (1H, ddd, J = 13.7, 6.6, 6.3 Hz), 3.17 (1H, ddd, J = 13.7, 6.6, 6.3 Hz), 2.93 (1H, ddd, J = 14.0, 6.9, 4.9 Hz), 2.80 (1H, dt, J = 14.0, 6.9 Hz), 2.53-2.40 (2H, m), 2.13 (2H, s), 2.04-1.90 (1H, m), 1.78-1.64 (3H, m), 1.46-1.37 (1H, m), 1.05 (9H, s), 0.89 (3H, d, J = 6.6 Hz), 0.88 (3H, d, J = 6.6 Hz).

Example 24

2(R)-(3,3-dimethylbutanoylamino)-3-(3-(4-methyl-2(R)-(4-methoxyphenyl)pentanoylamino)propylthio)propionic acid (24)

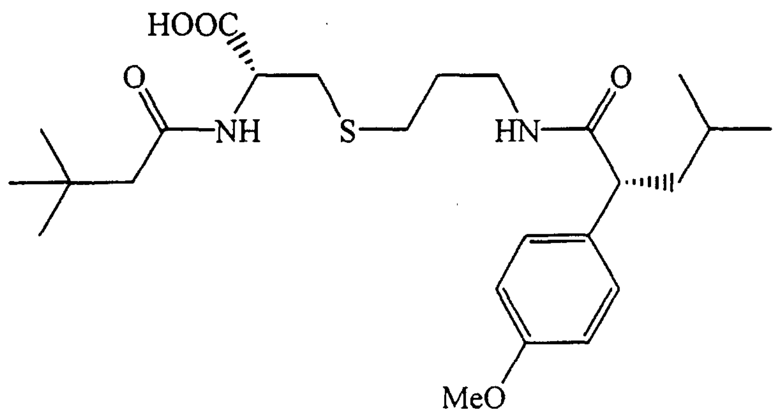

(24)

Under argon atmosphere, to a solution of 289.6 mg (0.585 mmol) of methyl 2(R)-(3,3-dimethylbutanoylamino)-3-(3-(4-methyl-2(R)-(4-methoxyphenyl)pentanoylamino)propylthio)propionate (23) in 5.5 ml of methanol, 2 ml of aqueous 1N sodium hydroxide solution was added, and the mixture was stirred at room temperature for 1.5 hours. To the reaction solution, 2.2 ml of 1N hydrochloric acid and 5 ml of water were added and the mixture was extracted with ethyl acetate (3 x 12 ml). Organic layers were combined and washed with 6 ml of saturated brine. The resulting mixture was dried over anhydrous sodium sulfate and concentrated under reduced pressure. The obtained oily product was purified by column chromatography (DIOL, hexane/ethyl acetate (1/1.5)) to obtain 262.8 mg of 2(R)-(3,3-dimethylbutanoylammo)-3-(3-(4-methyl-2(R)-(4-methoxyphenyl)pentanoylamino)propylthio)propionic acid (24) as colorless oil (yield 93%).
LR-MS(m/z):481(M+H)$^+$
IR (neat):3301, 2955, 2868, 1736, 1647, 1510, 1466, 1367, 1249, 1179, 1036, 835, 792, 754 cm$^{-1}$.
$^1$H-NMR (300MHz,$CDCl_3$, δppm):7.20 (2H, d, J = 8.8 Hz), 6.87 (2H, d, J = 8.8 Hz), 6.57 (1H, br), 5.92 (1H, br), 4.78-4.71 (1H, m), 3.79 (3H, s), 3.50-3.40 (2H, m), 3.15-3.09 (2H, m), 2.87 (1H, ddd, J = 14.0, 8.0, 5.8 Hz), 2.51-2.43 (2H, m), 2.15 (2H, s), 2.00-1.89 (3H, m), 1.74-1.58 (3H, m), 1.42-1.35 (1H, m), 1.04 (9H, s), 0.85 (3H, d, J = 6.3 Hz), 0.84 (3H, d, J = 6.3 Hz).

Example 25

3-(3-(2(R)-(4-hydroxyphenyl)-4-methylpentanoylamino)propylthio)-2(R)-(3,3-dimethylbutanoylamino)propionic acid (25)

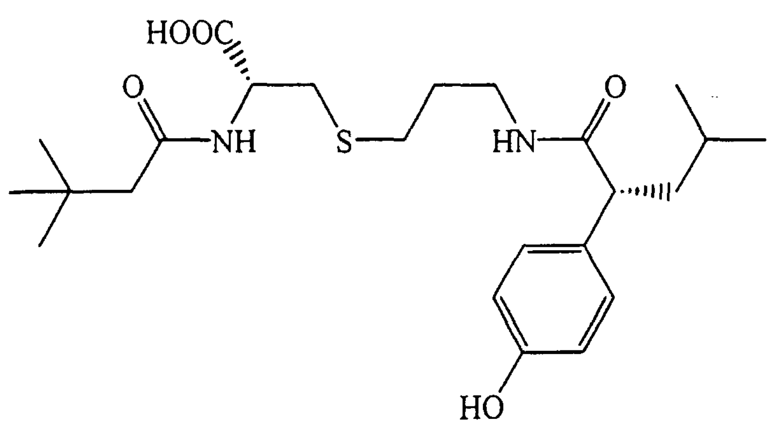

(25)

Under argon atmosphere, to a solution of 254.4 mg (0.529 mmol) of 2(R)-(3,3-dimethylbutanoylamino)-3-(3-(4-methyl-2(R)-(4-methoxyphenyl)pentanoylamino)propylthio)propionic acid (24) in 6 ml of dichloromethane, 3.4 ml of 1M boran tribromide in dichloromethane was added and the mixture was stirred for 5 hours while cooling the mixture in ice. To the reaction solution, 5 ml of water was added and the mixture was extracted with ethyl acetate (3 x 12 ml). Organic layers were combined and washed with 6 ml of saturated brine. The resulting mixture was dried over anhydrous sodium sulfate and concentrated under reduced pressure. The obtained oily product was purified by column chromatography (DIOL, hexane/ethyl acetate (1/5)) to obtain 162.5 mg of 3-(3-(2(R)-(4-hydroxyphenyl)-4-methylpentanoylamino)propylthio)-2(R)-(3,3-dimethylbutanoylamino)propionic acid (25) as colorless oil (yield 66%).
LR-MS(m/z):467(M+H)$^+$
IR (neat):3314, 2956, 1720, 1647, 1512, 1448, 1367, 1234, 837 $cm^{-1}$.
$^1$H-NMR (300MHz,CD,OD, δ ppm):8.14 (1H, br), 8.05 (1H, m), 7.14 (2H, d, J = 8.6 Hz), 6.70 (2H, d, J = 8.6 Hz), 4.58-4.52 (1H, m), 3.46 (1H, dd, J = 8.2, 6.9 Hz), 3.25-3.13 (3H, m), 2.97 (1H, ddd, J = 13.7, 6.9, 4.4 Hz), 2.76 (1H, ddd, J = 13.7, 8.5, 2.3 Hz), 2.51-2.45 (2H, m), 2.13 (2H, s), 1.93-1.86 (1H, m), 1.75-1.67 (2H, m), 1.56-1.39 (1H, m). 1.03 (9H, s), 0.91 (3H, d, J = 6.3 Hz), 0.90 (3H, d, J = 6.3 Hz). HR-MS:$C_{22}H_{39}N_2O_5S$ Calcd.: 467.2580, Found: 467.2611.

Example 26

Methyl 3-(3-(2(R,S)-(4-((1-amino-2-aza-2-cyanovinyl)amino)phenyl)-4-methylpentanoylamino)propylthio)-2(R)-(3,3-dimethylbutanoylamino)propionate (26)

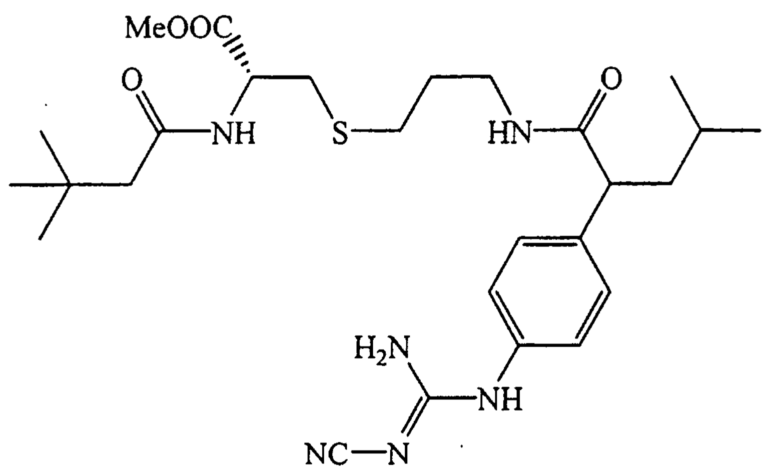

(26)

Under argon atmosphere, to 72.6 mg (0.25 mmol) of 2(R)-(3,3-(dimethylbutanoylamino)-3-(3-aminopropylthio)propionate (17) in DMF (2 ml), 63.1 mg (0.23 mmol) of methyl 3-(3-(2(R,S)-(4-((1-amino-2-aza-2-cyanovinyl)amino)phenyl)-4-methylpentanoylamino)propylthio)-2(R)-(3,3-dimethylbutanoylamino)propionate, 114.2 mg (0.258 mmol) of BOP reagent and 0.18ml (1.03 mmol) of diisopropylamine were added while cooling the mixture in ice, and the mixture was stirred overnight at room temperature. To the reaction solution, 4 ml of 0.1N hydrochloric acid and 1 ml of water were added, and the mixture was extracted with chloroform (3 x 12 ml). Organic layers were combined and washed with saturated brine. The resulting mixture was dried over anhydrous magnesium sulfate and concentrated under reduced pressure. The obtained oily product was purified by column chromatography (silica gel, chloroform/methanol (30/1)) to obtain 110.0 mg of methyl 3-(3-(2(R,S)-(4-((1-amino-2-aza-2-cyanovinyl)amino)phenyl)-4-methylpentanoylamino)propylthio)-2(R)-(3,3-dimethylbutanoylamino)propionate (26) (diastereomer ratio: about 1:1) as colorless solid (yield 87%).
LR-MS(m/z):547(M+H)$^+$
IR (neat):3308, 2954, 2868, 2182, 1744, 1647, 1544, 1511, 1435, 1366, 1215, 838, 754$cm^{-1}$.
$^1$H-NMR (300MHz,$CDCl_3$, δppm):7.84 (1H, br), 7.31 (2H, d, J = 7.3 Hz), 7.09 (2H, d, J = 7.3 Hz), 6.79 (1H, br), 6.68 (1H, br), 6.23 (1H, dd, J = 7.9, 4.0 Hz), 5.82-5.78 (2H, br), 4.76-4.68 (1H, m), 3.79 (3H, s), 3.76-3.06 (4H, m), 2.91-2.85 (1H, m), 2.72-2.61 (1H, m), 2.55-2.41 (2H, m), 2.17 (2H, s), 1.99-1.90 (1H, m), 1.74-1.63 (1H, m), 1.53-1.40 (1H, m), 1.07 (9H, s), 0.93-0.88 (6H, m).

Example 27

3-(3-(2(R,S)-(4-((1-amino-2-aza-2-cyanovinyl)amino)phenyl)-4-methylpentanoylamino)propylthio)-2R)-(3,3-dimethylbutanoylamino)propionic acid (27)

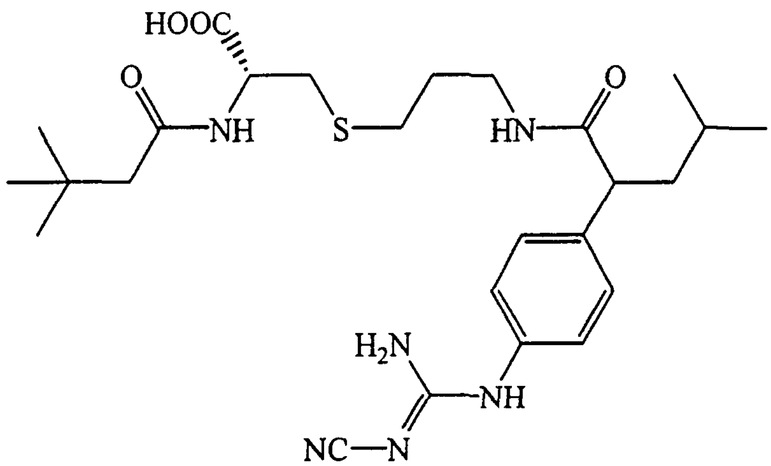

(27)

Under argon atmosphere, to a solution of 48.0 mg (0.0878 mmol) of methyl 3-(3-(2(R,S)-(4-((1-amino-2-aza-2-cyanovinyl)amino)phenyl)-4-methylpentanoylamino)propylthio)-2(R)-(3,3-dimethylbutanoylamino)propionate (26) in 2.0 ml of methanol, 0.3 ml of aqueous 1N sodium hydroxide solution was added and the mixture was stirred at room temperature for 1.5 hours. To the reaction solution, 1.1 ml of 1N hydrochloric acid and 5 ml of water were added and the mixture was extracted with ethyl acetate (3 x 12 ml). Organic layers were combined and washed with 6 ml of saturated brine. The resulting mixture was dried over anhydrous magnesium sulfate and concentrated under reduced pressure. The obtained oily product was purified by column chromatography (DIOL, hexane/ethyl acetate (1/2)-(0/1)) to obtain 37.2 mg of3-(3-(2(R,S)-(4-((1-amino-2-aza-2-cyanovinyl)amino)phenyl)-4-methylpentanoylamino)propylthio)-2R)-(3,3-dimethylbutanoylamino)propionic acid (27)(diastereomer ratio: about 1:1) as colorless oil (yield 80%).
LR-MS(m/z):533$(M+H)^+$
IR (neat): 3325, 2956, 2183, 1719, 1646, 1543, 1511, 1437, 1260 $cm^{-1}$.
$^1$H-NMR (300MHz,$CD_3OD$, δppm):8.17-8.09 (1H, m), 7.34 (2H, d, J = 8.7 Hz), 7.27 (2H, d, J = 8.7Hz), 4.54-4.51 (1H, m), 3.56 (1H, dd, J = 6.9, 6.6 Hz), 3.25-3.14 (1H, m), 3.00 (1H, dd, J = 13.7, 4.7 Hz), 2.77 (1H, dd, J = 13.7, 8.8 Hz), 2.51-2.45 (2H, m), 2.13 (2H, s), 1.94 (1H, ddd, J = 13.7, 6.9, 6.6 Hz), 1.76-1.67 (2H, m), 1.55 (1H, ddd, J = 13.7, 6.9, 6.6 Hz), 1.48-1.41 (1H, m), 1.03 (9H, s), 0.92 (3H, d, J = 6.3 Hz), 0.91 (3H, d, J = 6.3 Hz).

Example 28

Methyl (S)-2-amino-4-(phenylmethoxy)butyrate (28)

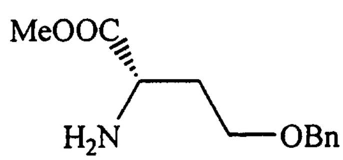

(28)

In 5 ml of methanol, 545 mg (1.76 mmol) of (S)-2-((tert-butoxy)carbonylamino)-4-(phenylmethoxy)butyric acid was dissolved, and about 4 ml of trimethylsilyldiazomethane solution in hexane was added while cooling the mixture in ice. The reaction solution was concentrated under reduced pressure and the residue was dissolved in 3 ml of dichloromethane. To the mixture, 3 ml of trifluoroacetic acid was added and the resulting mixture was stirred at room temperature for 1 hour. The reaction solution was concentrated under reduced pressure to obtain methyl (S)-2-amino-4-(phenylmethoxy)butyrate (28) as a crude product. The product (28) was subjected to azeotropic distillation with toluene and used in the next reaction without purification.

Example 29

Methyl (S)-2-(3,3-dimethylbutanoylamino)-4-(phenylmethoxy)butyrate (29)

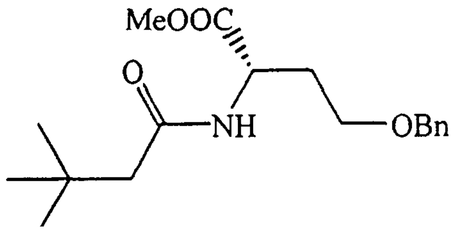

(29)

In 5 ml of dichloromethane, 230 mg (1.98 mmol) of t-butylacetic acid was dissolved and 937 mg (2.12 mmol) of BOP reagent was added thereto, followed by stirring the mixture at room temperature for 2 hours. To the reaction solution, Compound 28 dissolved in 2 ml of dichloromethane and 1.25ml (7.17mmol) of diisopropylethylamine were added and the mixture was stirred at room temperature for 1 hour. Water was added to the reaction solution and the mixture was extracted with ethyl acetate. Organic layers were combined and washed with 1N hydrochloric acid, saturated sodium hydrogen carbonate solution and saturated brine. The resulting mixture was dried over anhydrous sodium sulfate and concentrated under reduced pressure. The residue was purified by column chromatography (hexane:ethyl acetate = 3:1 - 2:1) to obtain 537 mg of methyl (S)-2-(3,3-dimethylbutanoylamino)-4-(phenylmethoxy)butyrate (29) as colorless oil (yield 95%).
LR-MS(m/z):321 $(M)^+$
$^1$H-NMR (300MHz,$CDCl_3$, δppm): 0.99 (9H, s), 2.00 (2H, s), 2.10-2.16 (2H, m), 3.51-3.64 (2H, m), 3.69 (3H, s), 4.44 (1H, d, J = 11.5Hz), 4.49 (1H, d, J = 11.5Hz), 4.69 (1H, dt, J = 5.2, 7.4Hz), 6.53 (1H, d, J = 7.4Hz), 7.28-7.40 (5H, m).

Example 30

Methyl (S)-4-hydroxy-2-(3,3-dimethylbutanoylamino)butyrate (30)

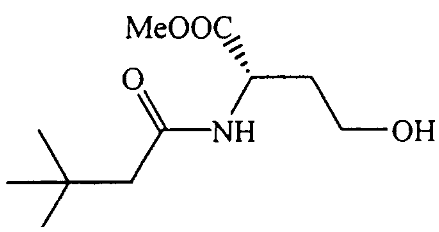

(30)

Under argon atmosphere, 508 mg of methyl (S)-2-(3,3-dimethylbutanoylamino)-4-(phenylmethoxy)butyrate (29) was dissolved in 10 ml of methanol and 58 mg of 10% palladium carbon was added, followed by hydrogen replacement. After stirring the reaction solution at room temperature for 16 hours, palladium/carbon was removed by filtration and the filtrate was concentrated under reduced pressure. The residue was purified by column chromatography (hexane:ethyl acetate = 1:1 - 1:2) to quantitatively obtain 376 mg of methyl (S)-4-hydroxy-2-(3,3-dimethylbutanoylamino)butyrate (30) as colorless oil.
LR-MS(m/z):231$(M^+)$
$^1$H-NMR (300MHz,$CDCl_3$, δppm):1.02(s, 9H), 1.60(m, 1H), 2.14(s, 2H), 2.18(m, 1H), 3.00(brs, 1H), 3.52-3.74(m, 2H), 3.75(s, 3H), 4.74(m, 1H), 6.36(brd, 1H).

Example 31

Methyl (S)-4-(methylsulfonyloxy)-2-(3,3-dimethylbutanoylamino)butyrate (31)

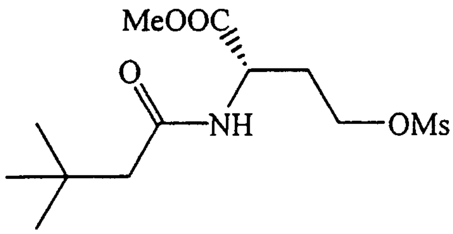

(31)

Under cooling in ice, 355 mg (1.53 mmol) of methyl (S)-4-hydroxy-2-(3,3-dimethylbutanoylamino)butyrate (30) was dissolved in 5 ml of dichloromethane, and 650 μl (4.66 mmol) of triethylamine and 160 μl (2.07 mmol) of methanesulfonyl chloride were added thereto, followed by stirring the resulting mixture at room temperature for 21 hours. Water was added to the reaction solution and the mixture was extracted with ethyl acetate. Organic layers were combined and washed with 1N hydrochloric acid, saturated sodium hydrogen carbonate solution and saturated brine. The resulting mixture was dried over anhydrous sodium sulfate and concentrated under reduced pressure. The residue was purified by column chromatography (hexane:ethyl acetate = 2:1 - 1:1 - 1:2) to obtain 225 mg of methyl (S)-4-(methylsulfonyloxy)-2-(3,3-dimethylbutanoylamino)butyrate (31) as colorless oil (yield 47%).
LR-MS(m/z):309($M^+$)
$^1$H-NMR (300MHz,$CDCl_3$, δppm):1.02(s, 9H), 2.12(s, 2H), 2.14-2.42(m, 2H), 3.02(s, 3H), 3.76(s, 3H), 4.22-4.35(m, 2H), 4.70(m, 1H), 6.18(brd, 1H).

Example 32

Methyl (R)-2-((tert-butoxy)carbonylamino)-3-sulfanylpropionate (32)

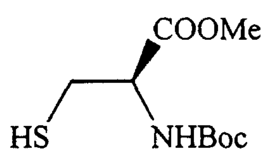

(32)

In a mixed solvent consisting of 10 ml of THF and 10ml (2.85M, 28.5 mmol) of aqueous 1N sodium hydroxide solution, 1.12g (9.24 mmol) of L-cystein was dissolved, and 4.38g (20.1 mmol) of di-t-butyldicarbonate was added, followed by stirring the resulting mixture at room temperature for 2 hours. Then 20 ml of THF and 2.33g (10.2 mmol) of di-t-butyldicarbonate were added, followed by stirring the resulting mixture at room temperature for 2 hours. Water and ether were added to the reaction solution, and aqueous layer was separated. The aqueous layer was acidified with 6N hydrochloric acid and then extracted with ethyl acetate. Organic layers were combined and washed with saturated brine. The resulting mixture was dried over anhydrous sodium sulfate and concentrated under reduced pressure. The residue was dissolved in 15 ml of methanol, and about 20 ml of trimethylsilyldiazomethane was added while cooling the mixture in ice. The reaction solution was concentrated under reduced pressure and the residue was purified by column chromatography (hexane:ethyl acetate = 5:1 - 3:1 - 2:1) to obtain 2.68 g of methyl (R)-2-((tert-butoxy)carbonylamino)-3-sulfanylpropionate (32) as colorless oil (yield 99%).
LR-MS(m/z):235($M^+$)

Example 33

Methyl 2(S)-(3,3-dimethylbutanoylamino)-4-(2(R)-((tert-butoxy)carbonylamino)-2(R)-(methoxycarbonyl)ethylthio) butyrate (33)

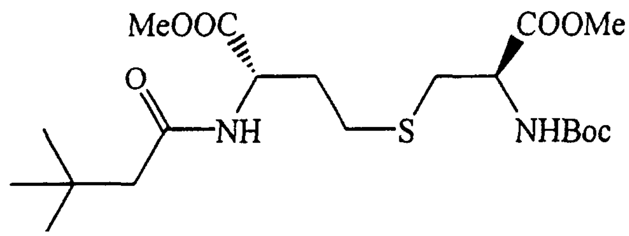

(33)

In 5 ml of THF, 142 mg (0.6 mmol) of methyl (R)-2-((tert-butoxy)carbonylamino)-3-sulfanylpropionate (32) was dissolved and 77 mg (0.69 mmol) of potassium t-butoxide was added, followed by stirring the mixture at room temperature for 20 minutes. Then 203 mg (0.66 mmol) of methyl (S)-4-(methylsulfonyloxy)-2-(3,3-dimethylbutanoylamino)butyrate (31) dissolved in 2 ml of THF was added and the mixture was stirred at room temperature for 1.5 hours. Water was added to the reaction solution and the mixture was extracted with ethyl acetate. Organic layers were combined and washed with saturated brine. The resulting mixture was dried over anhydrous sodium sulfate and concentrated under reduced pressure. The residue was purified by column chromatography (hexane:ethyl acetate = 3:1 - 2:1 - 1:1) to obtain 47 mg of methyl 2(S)-(3,3-dimethylbutanoylamino)-4-(2(R)-((tert-butoxy)carbonylamino)-2(R)-(methoxycarbonyl)ethylthio)butyrate (33) as colorless oil (yield 17%).
LR-MS(m/z):448($M^+$)
$^1$H-NMR (300MHz,$CDCl_3$, δppm): 1.05 (9H, s), 1.45, 1.46 (9H, s), 1.98 (1H, m), 2.12, 2.13 (2H, s), 2.13 (1H, m), 2.54-2.60 (2H, m), 2.86-3.01 (2H, m), 3.75, 3.76, 3.77 (6H, s), 4.52 (1H, m), 4.67 (1H, m), 5.36 (1H, d, J = 7.4Hz), 6.18 (1H, m).
$[\alpha]^{18}_D$ :+31° (c=0.940, $CHCl_3$)

Example 34

Methyl 4-(2(R)-amino-2(R)-(methoxycarbonyl)ethylthio)-2(S)-(3,3-dimethylbutanoylamino)butyrate (34)

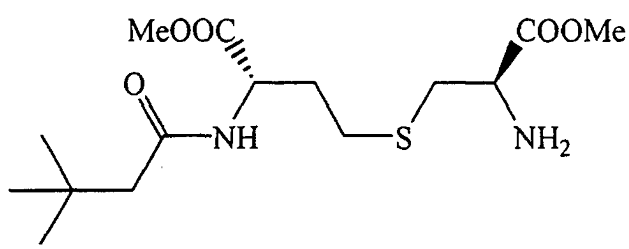

(34)

In 2 ml of dichloromethane, 44 mg (98 mmol) of methyl 2(S)-(3,3-dimethylbutanoylamino)-4-(2(R)-((tert-butoxy)carbonylamino)-2(R)-(methoxycarbonyl)ethylthio)butyrate (33) was dissolved and 1 ml of trifluoroacetic acid was added, followed by stirring the mixture at room temperature for 1 hour. The reaction solution was concentrated under reduced pressure to obtain methyl 4-(2(R)-amino-2(R)-(methoxycarbonyl)ethylthio)-2(S)-(3,3-dimethylbutanoylamino) butyrate (34) as a crude product. The product (34) was subjected to azeotropic distillation with toluene and used in the next reaction without purification.

Example 35

Methyl 4-(2(R)-(2(R,S)-(4-acetyloxyphenyl)-4-methylpentanoylamino)-2(R)-(methoxycarbonyl)ethylthio)-2(S)-(3,3-dimethylbutanoylamino)butyrate (35)

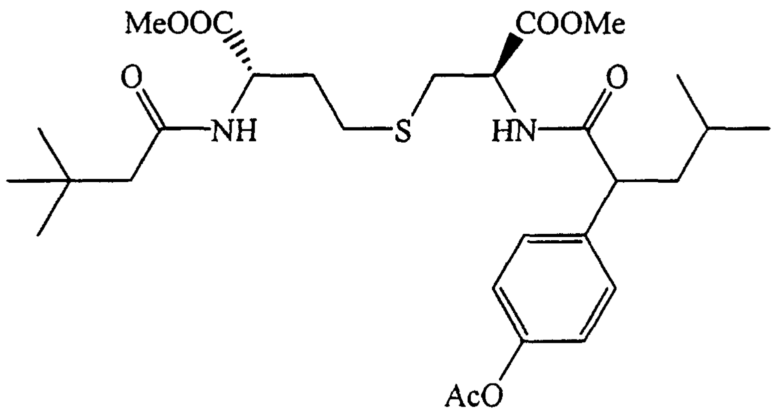

(35)

In 1 ml of dichloromethane, 26 mg (0.1mmol) of 2-(4-acetoxyphenyl)-4-methyl valeric acid was dissolved and 51 mg (0.12 mmol) of BOP reagent was added, followed by stirring the mixture at room temperature for 1 hour. To the reaction solution, methyl 4-(2(R)-amino-2(R)-(methoxycarbonyl)ethylthio)-2(S)-(3,3-dimethylbutanoylamino)butyrate (34) dissolved in 0.5 ml of dichloromethane and 0.7ml (4.01 mmol) of diisopropylethylamine were added and the resulting mixture was stirred at room temperature for 30 minutes. Water was added to the reaction solution and the mixture was extracted with ethyl acetate. Organic layers were combined and washed with 1N hydrochloric acid, aqueous saturated sodium hydrogen solution and saturated brine. The resulting mixture was dried over anhydrous sodium sulfate and concentrated under reduced pressure. The residue was purified by column chromatography (hexane:ethyl acetate = 1:1) to obtain 36 mg of methyl 4-(2(R)-(2(R,S)-(4-acetyloxyphenyl)-4-methylpentanoylamino)-2(R)-(methoxycarbonyl)ethylthio)-2(S)-(3,3-dimethylbutanoylamino)butyrate (35) as colorless oil (yield 63%).
LR-MS(m/z):580($M^+$)
$^1$H-NMR (300MHz,$CDCl_3$, δppm):0.84-0.92 (6H, m), 1.03, 1.04, 1.05 (9H, s), 1.46 (1H, m), 1.60-1.80 (2H, m), 1.82-2.02 (2H, m), 2.10, 2.11, 2.13, 2.14 (2H, s), 2.25 (1H, m), 2.29, 2.30, 2.31 (3H, s), 2.46 (1H, m), 2.76-2.98 (2H, m), 3.55 (1H, m), 3.69, 3.70, 3.73, 3.75 (6H, s), 4.60 (1H, m), 4.72 (1H, m), 6.33 (1H, m), 6.46 (1H, m), 7.01-7.08 (2H, m), 7.33-7.37 (2H, m).

Example 36

4-(2(R)-carboxy-2(R-(2(R,S)-(4-hydroxyphenyl)-4-methylpentanoylamino)ethylthio)-2(S) -(3,3-dimethylbutanoylamino)butyric acid (36)

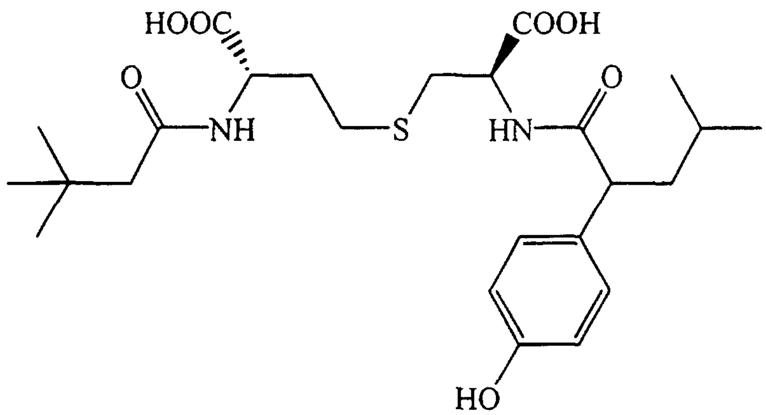

(36)

In 1 ml of methanol, 30 mg (0.05 mmol) of methyl 4-(2(R)-(2(R,S)-(4-acetyloxyphenyl)-4-methylpentanoylamino)-2(R)-(methoxycarbonyl)ethylthio)-2(S)-(3,3-dimethylbutanoylamino)butyrate (35) was dissolved, and 0.35ml (0.35 mmol) of aqueous 1N sodium hydroxide solution was added, followed by stirring the mixture at room temperature for 1.5 hours. To the reaction solution, 1N hydrochloric acid was added and the mixture was extracted with ethyl acetate. Organic layers were combined and washed with saturated brine. The resulting mixture was dried over anhydrous sodium sulfate and concentrated under reduced pressure. The residue was purified by diol Lobar column chromatography (hexane:ethyl acetate = 1:2 to ethyl acetate 100%) to obtain 17 mg of 4-(2(R)-carboxy-2(R-(2(R,S)-(4-hydroxyphenyl)-4-methylpentanoylamino)ethylthio)-2(S)-(3,3-dimethylbutanoylamino)butyric acid (36) as amorphous product (yield 64%).
LR-MS(m/z): 511 $(M+H)^+$
IR(KBr):3300~2500, 3321, 2957, 1726, 1234cm$^{-1}$
$^1$H-NMR (300MHz, $CDCl_3$, δppm):0.86-0.89 (6H, m), 1.04 (9H, s), 1.38-1.52 (1H, m), 1.63-1.79 (1H, m), 1.86-1.98 (2H, m), 2.12, 2.13 (2H, s), 2.18-2.48 (1H, m), 2.74-2.85 (1H, m), 2.87-3.00 (2H, m), 3.41-3.52 (2H, m), 4.41-4.60 (1H, m), 4.62-4.73 (1H, m), 6.40-6.58 (2H, m), 6.79-6.86 (2H, m), 7.12-7.18 (2H, m).
HR-MS: $C_{25}H_{39}N_2O_7S$ Calcd.: 511.2478, Found: 511.2508

Example 37

Methyl 2(S)-((tert-butoxy)carbonylamino)-4-propyl-2-enyloxybutyrate(37)

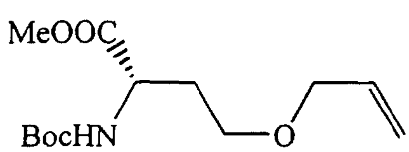

(37)

In 7.5 ml of THF, 227.3 mg (1.908 mmol) of L-homoserine was suspended, and 763.2 mg (19.08 mmol) of sodium hydroxide was dissolved in water (7.5 ml) under cooling in ice. To the mixture, 1.75ml (7.63 mmol) of di-tert-butyl dicarbonate was added dropwise and the resulting mixture was stirred overnight at room temperature. Then additional 1.75 ml of di-tert-butyl dicarbonate was added and the mixture was stirred for 3 hours, followed by addition of 30 ml of water. The aqueous layer was washed 3 times with ether and 6N hydrochloric acid was added to the aqueous layer to adjust its pH to 2-3, followed by extraction 3 times with ethyl acetate. Organic layers were combined and

washed with saturated brine. The resulting mixture was dried over anhydrous sodium sulfate and concentrated under reduced pressure. The residue was dissolved in 6 ml of methanol and 4 ml of trimethylsilyldiazomethane (2N solution in hexane) was added, followed by concentrating the resulting mixture.

The residue was dissolved in 6 ml of dichloromethane, and 566.9 mg of benzyltrimethylammonium chloride, 0.33ml (3.82 mmol) of allyl bromide and 305.3 mg of aqueous sodium hydroxide solution (40 wt%) were added, followed by stirring the mixture overnight at room temperature. After adding 10 ml of water to the reaction solution, the aqueous layer was saturated with sodium chloride and then extracted with ethyl acetate. Organic layers were combined and washed with saturated brine. The resulting mixture was dried over anhydrous sodium sulfate and concentrated. The residue was purified by column chromatography (silica gel; hexane/ethyl acetate = 3/1) to obtain 207.1 mg (0.76 mmol, yield 40%) of methyl 2(S)-((tert-butoxy)carbonylamino)-4-propyl-2-enyloxybutyrate (37) as colorless oil.
LR-MS(m/z):274 $(M+1)^+$
IR(neat):3366, 2978, 1745, 1716, 1508, 1366, 1163, 1105cm$^{-1}$
$^1$H-NMR (300MHz,$CDCl_3$, δppm):5.93-5.80 (1H, m), 5.45-5.43 (1H, brm), 5.25 (1H, ddd, J=1.6, 3.3, 17.3Hz), 5.16 (1H, ddd, J=1.1, 3.0, 10.2Hz), 4.40 (1H, dd, J=6.6, 12.4Hz), 3.93 (2H, ddd, J=1.4, 2.5, 5.5Hz), 3.72 (3H, s), 3.55-3.43 (2H, m), 2.13-1.96 (2H, m), 1.43 (9H, s)

Example 38

Methyl 2(S)-(3,3-dimethylbutanoylamino)-4-propyl-2-enyloxybutyrate (38)

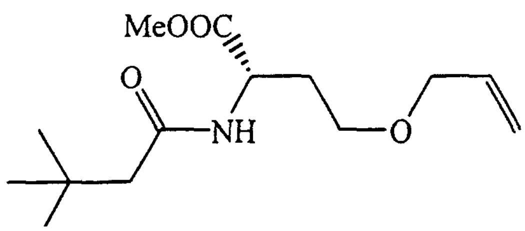

(38)

In 10 ml of dichloromethane, 193.0 mg (0.71 mmol) of methyl 2(S)-((tert-butoxy)carbonylamino)-4-propyl-2-enyloxybutyrate (37) was dissolved and 1.63ml (21.18 mmol) of trifluoroacetic acid was added dropwise while cooling the mixture in ice, followed by stirring the resulting mixture for 2 hours. Another 1.63 ml of trifluoroacetic was then added and the mixture was stirred at room temperature for 30 minutes, followed by concentrating the mixture. The residue was dissolved in 7 ml of anhydrous dichloromethane under argon atmosphere, and 0.39ml (2.82 mmol) of triethylamine and 0.196ml (1.41 mmol) of tert-butylacetyl chloride were added while cooling the mixture in ice, followed by stirring the mixture at 0°C for 2 hours. Saturated aqueous sodium hydrogen carbonate solution was added to the reaction solution and the resulting mixture was extracted 3 times with dichloromethane. Organic layers were combined and washed with saturated brine. The resulting mixture was dried over anhydrous sodium sulfate and concentrated. The residue was purified by column chromatography (silica gel; hexane/ethyl acetate = 3/1) to obtain 186.8 mg (0.688 mmol) of methyl 2(S)-(3,3-dimethylbutanoylamino)-4-propyl-2-enyloxybutyrate (38) as colorless oil (98%).
LR-MS(m/z):271$(M)^+$
IR(neat):3308, 2953, 2868, 1747, 1649, 1538, 1437, 1366, 1200, 1156, 1102cm$^{-1}$
$^1$H-NMR (300MHz,$CDCl_3$, δppm):6.55 (1H, brd, J=7.4Hz), 5.87 (1H, tdd, J=5.8, 10.2, 21.7Hz), 5.25 (1H, ddd, J=1.6, 3.3, 17.3Hz), 5.18 (1H, ddd, J=1.4, 3.0, 10.4Hz), 4.68-4.62 (1H, m), 3.94-3.91 (2H, m), 3.72 (3H, s), 3.57-3.45 (2H, m), 2.11-2.07 (4H, m), 1.03 (9H, s)

Example 39

Methyl 2(S)-(3,3-dimethylbutanoylamino)-4-(2-(methylsulfonyloxy)ethoxy)butyrate (39)

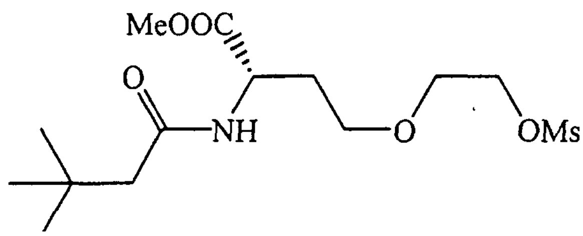

(39)

In 8 ml of THF/water (3/1) mixed solvent, 172.9 mg (0.64 mmol) of methyl 2(S)-(3,3-dimethylbutanoylamino)-4-propyl-2-enyloxybutyrate (38) was dissolved, and 408.7 mg (1.91 mmol) of sodium periodate and 0.64ml (0.06 mmol) of 0.1N osmium tetroxide solution in THF were added thereto, followed by stirring the mixture at room temperature for 2.5 hours. To the reaction solution, 10 ml of water was added and the mixture was extracted 3 times with ethyl acetate. Organic layers were combined and washed with saturated brine. The resulting mixture was dried over anhydrous sodium sulfate and concentrated. The residue was dissolved in 7 ml of methanol and 96.4 mg (2.55 mmol) of sodium borohydride was added, followed by stirring the resulting mixture at room temperature. To the reaction solution, 15 ml of water was added and the mixture was extracted 3 times with ethyl acetate. Organic layers were combined and washed with saturated brine. The resulting mixture was dried over anhydrous sodium sulfate and concentrated to obtain 132.4 mg of yellow oil.

This oily product was dissolved in 5 ml of anhydrous pyridine under argon atmosphere and 0.11ml (1.40 mmol) of methanesulfonyl chloride was added thereto under cooling in ice, followed by stirring the resulting mixture overnight at room temperature. To the reaction solution, 25 ml of water was added and the mixture was extracted 3 times with ethyl acetate. Organic layers were combined and washed with saturated brine, saturated aqueous cupric sulfate solution and with saturated brine. The resulting mixture was dried over anhydrous sodium sulfate and concentrated. The residue was purified by column chromatography (silica gel; hexane/ethyl acetate = 1/2) to obtain 93.1 mg (0.26 mmol) of methyl 2(S)-(3,3-dimethylbutanoylamino)-4-(2-(methylsulfonyloxy)ethoxy)butyrate (39) as colorless oil (yield 41%).
LR-MS(m/z):353 $(M)^+$
IR(neat):3601, 3392, 3311, 3023, 2955, 2871, 1742, 1651, 1528, 1352, 1174, 1130, 921cm$^{-1}$
$^1$H-NMR (300MHz,$CDCl_3$, δppm):6.41 (1H, brd, J=7.1Hz), 4.67 (1H, dd, J=5.5; 11.8Hz), 4.35-4.30 (2H, brm), 3.71-3.70 (3H, m), 3.66-3.63 (2H, m), 3.61-3.47 (2H, m), 3.05 (3H, d, J=0.5Hz), 2.16-2.01 (4H, m), 1.01 (9H, d, J=1.9Hz)

Example 40

Methyl 4-(2-(2(R,S)-(4-acetyloxyphenyl)-4-methylpentanoylamino)ethoxy)-2(S)-(3,3-dimethylbutanoylamino)butyrate (40)

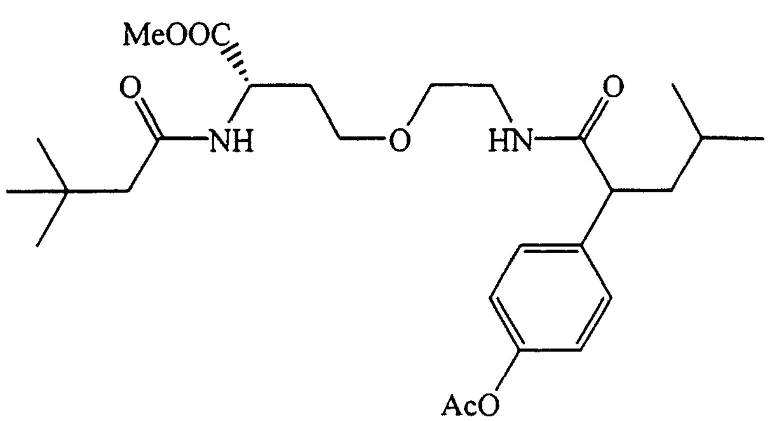

(40)

In 3 ml of DMF, 89.2 mg (0.25 mmol) of methyl 2(S)-(3,3-dimethylbutanoylamino)-4-(2-(methylsulfonyloxy) ethoxy)butyrate (39) was dissolved and 246.1 mg (3.79 mmol) of sodium azide was added thereto, followed by stirring the resulting mixture at 50°C for 3 hours. To the reaction solution, 20 ml of water was added and the mixture was extracted 3 times with ethyl acetate. Organic layers were combined and washed with water and saturated brine. The resulting mixture was dried over anhydrous sodium sulfate and concentrated. The residue was dissolved in 2 ml of methanol and 32 mg of 10% palladium/carbon was added to carry out hydrogen replacement, followed by stirring the resulting mixture at room temperature for 3 hours. The 10% palladium/carbon was removed by filtration through Celite, and the filtrate was concentrated to obtain colorless oil.

The obtained oily product was dissolved in 3 ml of anhydrous dichloromethane under argon atmosphere, and 98.9 mg (0.4 mmol) of 2-(p-acetoxy)phenyl-2-isobutylacetic acid, 205.6 mg (0.4 mmol) of pyBOP and 0.21ml (1.18 mmol) of diisopropylethylamine were added, followed by stirring the resulting mixture at room temperature for 2.5 hours. To the reaction solution, 15 ml of water was added and the mixture was extracted 3 times with dichloromethane. Organic layers were combined and washed with saturated brine. The resulting mixture was dried over anhydrous sodium sulfate, filtered and concentrated. The residue was purified by thin layer chromatography (hexane/ethyl acetate = 1/2) to obtain 66.1 mg (0.13 mmol) of methyl 4-(2-(2(R,S)-(4-acetyloxyphenyl)-4-methylpentanoylamino)ethoxy)-2(S)-(3,3-dimethylbutanoylamino)butyrate (40) as colorless oil (yield 52%).
LR-MS(m/z):506 $(M)^+$
IR(neat):3305, 2955, 2869, 1746, 1649, 1540, 1505, 1368, 1203, 1126cm$^{-1}$
$^1$H-NMR (300MHz, δppm,$CDCl_3$): 7.40 (2H, dd, J=6.6, 8.5Hz), 6.98 (2H, d, J=8.5Hz), 6.84 (1H, brs), 6.24 (1H, brt, J=9.3Hz), 4.84-7.76 (1H, m), 3.72-3.33 (10H, m), 2.25 (3H, s), 2.21-1.84 (5H, m), 1.62 (1H, dt, J=7.1, 13.2Hz), 1.48-1.39 (1H, m), 1.03 (9H, d, J=5.2Hz), 0.90-0.86 (6H, m)

Example 41

4-(2-(2(R,S)-(4-acetyloxyphenyl)-4-methylpentanoylamino)ethoxy)-2(S)-(3,3-dimethylbutanoylamino)butyric acid (41)

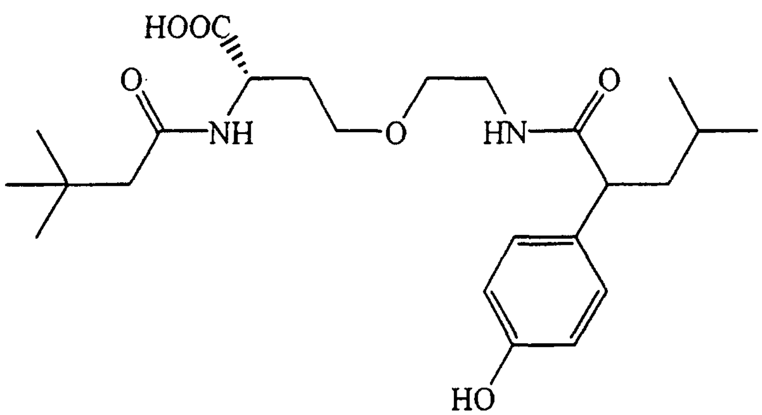

(41)

In 2 ml of methanol, methyl 4-(2-(2(R,S)-(4-acetyloxyphenyl)-4-methylpentanoylamino)ethoxy)-2(S) -(3,3-dimethylbutanoylamino)butyrate (40) was dissolved, and 0.34 ml of 1N aqueous sodium hydroxide solution was added thereto, followed by stirring the mixture at room temperature for 3.5 hours. After adding 5 ml of water to the reaction solution, the pH of the mixture was adjusted to 2 with 0.1N hydrochloric acid, and the resulting mixture was extracted 3 times with ethyl acetate. Organic layers were combined and washed with saturated brine. The resulting mixture was dried over anhydrous sodium sulfate and concentrated. The residue was purified by column chromatography (medium-pressure; diol column; cyclohexane/ethyl acetate = 25/75) to obtain 35.3 mg (0.078 mmol) of 4-(2-(2 (R,S)-(4-acetyloxyphenyl)-4-methylpentanoylamino)ethoxy)-2(S)-(3,3-dimethylbutanoylamino)butyric acid (41) as colorless oil (69%).
LR-MS(m/e):450 $(M^+)$
IR(meat):3308, 2957, 1725,1645, 1514, 1449, 1367, 1234, 1155, 1126cm$^{-1}$ $^1$H-NMR (300MHz, $CD_3OD$, δppm):7.17 (2H, d, J=7.7Hz), 6.70 (2H, d, J=8.5Hz), 4.58-4.56 (1H, m), 3.56-3.19 (7H, m), 2.13-2.07 (3H, m), 1.88-1.84 (2H, m), 1.58-1.42 (2H, m), 1.04 (9H, s), 0.92-0.89 (6H, m)
HR-MS:$C_{24}H_{38}N_2O_6$ Calcd.: 450.2730, Found: 450.2722

Example 42

Methyl 2(R)-amino-3-((2(R)-(3,3-dimethylbutanoylamino)-2(R)-(methoxycarbonyl)ethyl)disulfanyl)propionate (42)

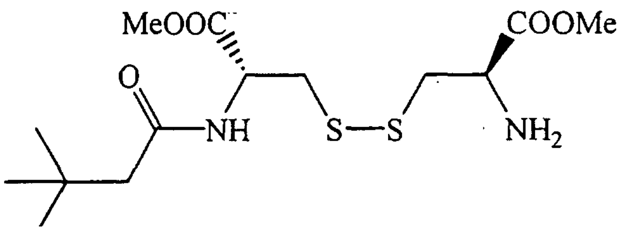

(42)

In 20 ml of dichloromethane, 1.51g(5.63 mmol) of L-cystine methyl ester was dissolved and 1.1ml (6.31 mmol) of diisopropylethylamine was added, followed by cooling the resulting mixture to -30°C. To the reaction solution, 0.79ml (5.68 mmol) of t-butylacetyl chloride was added dropwise and the temperature of the mixture was raised to 0°C. After stirring the mixture at 0°C for another 1 hour, water was added to the reaction solution and the mixture was extracted with ethyl acetate. Organic layers were combined and washed with saturated brine. The resulting mixture was dried over anhydrous sodium sulfate and concentrated under reduced pressure. The residue was purified by column chromatography (hexane:ethyl acetate = 1:1 - 1:2 - ethyl acetate 100% - 5% methanol/chloroform) to obtain 767 mg of methyl 2(R)-amino-3-((2(R)-(3,3-dimethylbutanoylamino)-2(R)-(methoxycarbonyl)ethyl)disulfanyl)propionate (42) as colorless oil (yield 37%).
LR-MS(m/z):367 ($M^+$)
$^1$H-NMR (300MHz,$CDCl_3$, δppm):1.06(s, 9H), 2.14(s, 2H), 2.93(dd, J=7.2, 13.8Hz, 1H), 3.11(dd, J=4.8, 13.8Hz, 1H), 3.19(dd, J=5.5, 14.1Hz, 1H), 3.25(dd, J=4.9, 14.1Hz, 1H), 3.76(s, 3H), 3.76-3.82(m, 1H), 3.78(s, 3H), 4.91(m, 1H), 6.33(brd, J=7.1Hz, 1H).

Example 43

Methyl 3-((2(R)-(3,3-dimethylbutanoylamino)-2(R)-(methoxycarbonyl)ethyl)disulfanyl)-2(R)-(3,3-dimethyl-2(S) -(phenylmethoxy)butanoylamino)propionate (43)

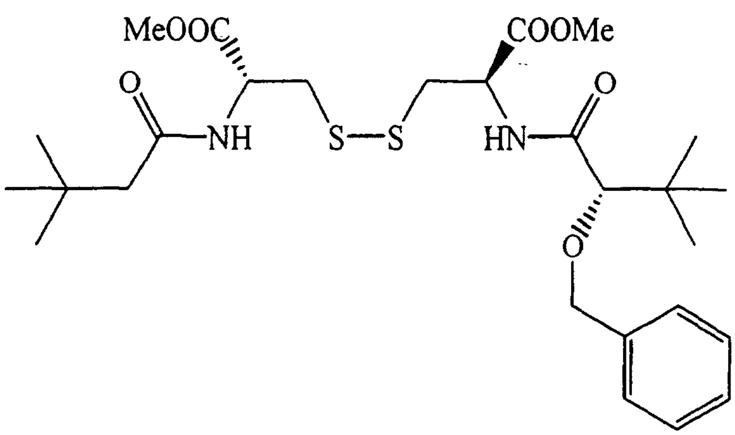

(43)

In 1.5 ml of DMF, 100 mg (0.27 mmol) of methyl 2(R)-amino-3-((2(R)-(3,3-dimethylbutanoylamino)-2(R)-(methoxycarbonyl)ethyl)disulfanyl)propionate (42) and 62 mg (0.28 mmol) of (S)-3,3-dimethyl-2-(phenylmethoxy)butyric acid were dissolved, and 160 mg (0.31 mmol) of PyBOP and 0.04ml (0.37 mmol) of N-methylmorpholine were added thereto, followed by stirring the mixture at room temperature for 1 hour. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 5:1 - 3:1 - 2:1 - 1:1) to obtain 125 mg of methyl 3-((2(R)-(3,3-dimethylbutanoylamino)-2(R)-(methoxycarbonyl)ethyl)disulfanyl)-2(R)-(3,3-dimethyl-2(S)-(phenylmethoxy)butanoylamino)propionate (43) as colorless oil (yield 80%).
LR-MS(m/z):571($M^+$)
$^1$H-NMR (300MHz,$CDCl_3$, δppm): 0.99(s, 9H), 1.04(s, 9H), 2.12(s, 2H), 3.15-3.20(m, 4H), 3.49(s, 1H), 3.74(s, 3H), 3.76(s, 3H), 4.40(d, J=11.0Hz, 1H), 4.69(d, J=11.0Hz, 1H), 4.81-4.94(m, 2H), 6.27(brd, J=7.7Hz, 1H), 7.24(brd, J=8.0Hz, 1H), 7.31-7.39(m, 5H).

Example 44

3-((2(R)-carboxy-2(R)-(3,3-dimethylbutanoylamino)ethyl)disulfanyl)-2(R)-(3,3-dimethyl-2(S)-(phenylmethoxy)butanoylamino)propionic acid (44)

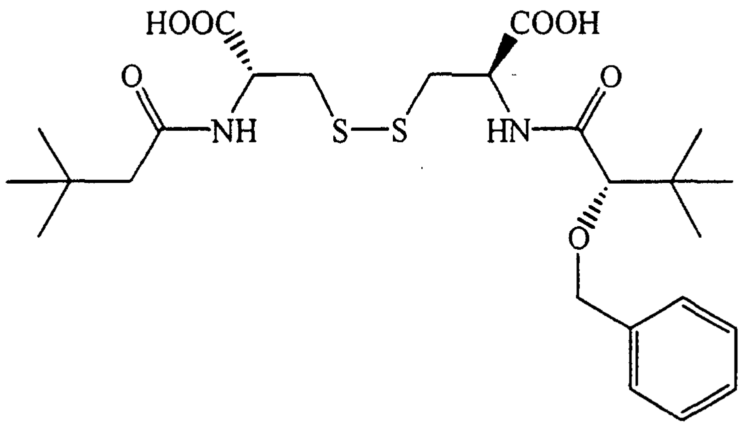

(44)

In 7 ml of toluene, 115 mg (0.2 mmol) of methyl 3-((2(R)-(3,3-dimethylbutanoylamino)-2(R)-(methoxycarbonyl)ethyl)disulfanyl)-2(R)-(3,3-dimethyl-2(S)-(phenylmethoxy)butanoylamino)propionate (43) was dissolved, and 502 mg (2.01 mmol) of dibutyltin oxide was added thereto, followed by stirring the mixture at 100°C for 17 hours. To the reaction solution, 10 ml of aqueous 10% potassium fluoride solution and 15 ml of ethyl acetate were added and the mixture was vigorously stirred for 30 minutes. The organic layer was washed with 1N hydrochloric acid and saturated brine. The resulting mixture was dried over anhydrous sodium sulfate and concentrated under reduced pressure. The residue was purified by diol Lobar column (hexane:ethyl acetate = 1:1 - 1:2) to obtain 44 mg of 3-((2(R)-carboxy-2(R)-(3,3-dimethylbutanoylamino)ethyl)disulfanyl)-2(R)-(3,3-dimethyl-2(S)-(phenylmethoxy)butanoylamino)propionic acid (44) as an amorphous product (yield 40%).
LR-MS(m/z):543($M^+$)
IR(KBr):3300~2500, 2957, 1735, 1233$cm^{-1}$
$^1$H-NMR (300MHz, $CDCl_3$, δppm) : 1.00(s, 9H), 1.04(s, 9H), 2.18(s, 2H), 3.05-3.19(m, 2H), 3.27-3.33(m, 2H), 3.55(s, 1H), 4.38(d, J=11.3Hz, 1H), 4.68(d, J=11.3Hz, 1H), 5.03-5.07(m, 2H), 6.66(brd, J=7.1Hz, 1H), 7.29-7.43(m, 6H).
HR-MS:$C_{25}H_{39}N_2O_7S_2$ Calcd. : 543.2200, Found: 543.2194
$[\alpha]^{25}_D$:-187.81° (c=0.55, MeOH)

Example 45

Methyl 3-((2(R)-(3,3-dimethylbutanoylamino)-2(R)-(methoxycarbonyl)ethyl)disulfanyl)-2(R)-(4-methyl-2(S)-(phenylmethoxy)pentanoylamino)propionate (45)

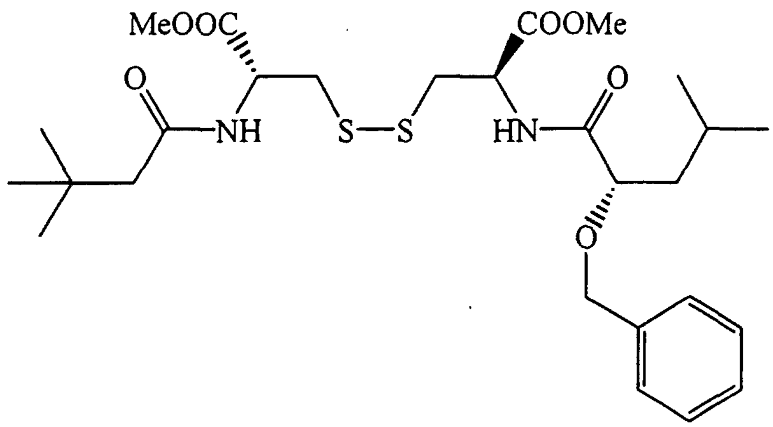

(45)

In 1.5 ml of DMF, 101 mg (0.28 mmol) of methyl 2(R)-amino-3-((2(R)-(3,3-dimethylbutanoylamino)-2(R)-(methoxycarbonyl)ethyl)disulfanyl)propionate (42) and 62 mg (0.28 mmol) of (S)-4-methyl-2-(phenylmethoxy)valeric acid were dissolved, and 160 mg (0.31 mmol) of PyBOP and 0.04ml (0.37 mmol) of N-methylmorpholine were added to the solution, followed by stirring the resulting mixture at room temperature for 3 hours. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 5:1 - 3:1 - 2:1) to obtain 70 mg of methyl 3-((2(R)-(3,3-dimethylbutanoylamino)-2(R)-(methoxycarbonyl)ethyl)disulfanyl)-2(R)-(4-methyl-2(S)-(phenylmethoxy)pentanoylamino)propionate (45) as colorless oil (yield 45%).
LR-MS(m/z):571($M^+$)
$^1$H-NMR (300MHz, $CDCl_3$, δppm) : 0.85 (3H, d, J = 6.6Hz), 0.93 (3H, d, J = 6.6Hz), 1.05 (9H, s), 1.54 (1H, m), 1.66 (1H, m), 1.85 (1H, m), 2.15 (2H, s), 3.16-3.21 (4H, m), 3.75 (3H, s), 3.79 (3H, s), 3.91 (1H, dd, J = 3.8, 9.3Hz), 4.30 (1H, m), 4.68 (1H, m), 4.83-4.95 (2H, m), 6.31 (1H, d, J = 7.4Hz), 7.30-7.41 (6H, m).
$[\alpha]^{22}_D$:+31.9° (c=1.406, $CHCl_3$)

Example 46

3-((2(R)-carboxy-2(R)-(3,3-dimethylbutanoylamino)ethyl)disulfanyl)-2(R)-(4-methyl-2(S)-(phenylmethoxy) pentanoylamino)propionic acid (46)

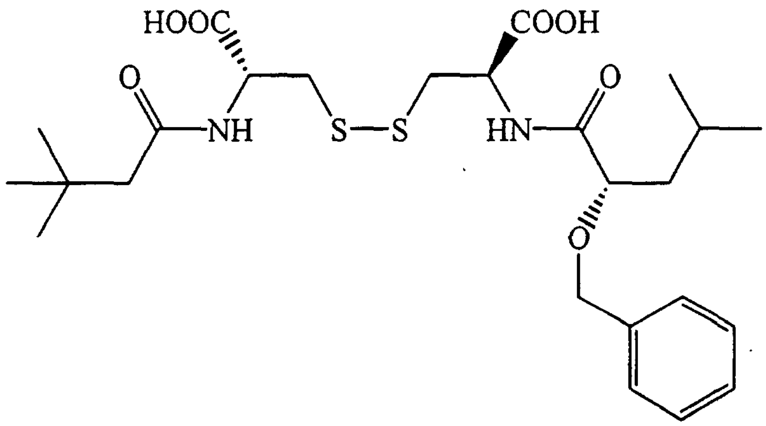

(46)

In 4 ml of toluene, 67 mg (0.12 mmol) of methyl 3-((2(R)-(3,3-dimethylbutanoylamino)-2(R)-(methoxycarbonyl) ethyl)disulfanyl)-2(R)-(4-methyl-2(S)-(phenylmethoxy)pentanoylamino)propionate (45) was dissolved and 300 mg (1.20 mmol) of dibutyltin oxide was added, followed by stirring the mixture at 100°C for 13 hours. To the reaction solution, 10 ml of 10% aqueous potassium fluoride solution and 15 ml of ethyl acetate were added and the mixture was vigorously stirred for 1 hour. The organic layer was washed with 1N hydrochloric acid and saturated brine, and dried over anhydrous sodium sulfate, followed by concentrating the mixture under reduced pressure. The residue was purified by diol Lobar column chromatography (hexane:ethyl acetate = 1:1 - 1:2) to obtain 36 mg of 3-((2(R)-carboxy-2(R)-(3,3-dimethylbutanoylamino)ethyl)disulfanyl)-2(R)-(4-methyl-2(S)-(phenylmethoxy)pentanoylamino)propionic acid (46) as amorphous product (yield 57%).
LR-MS(m/z):543($M^+$)
IR(KBr):3300-2500, 2957, 1736, 1232$cm^{-1}$
$^1$H-NMR (300MHz, $CDCl_3$, δppm) : 0.82(d, J=6.3Hz, 3H), 0.91(d, J=6.6Hz, 3H), 1.03(s, 9H), 1.48-1.69(m, 2H), 1.79-1.85(m, 1H), 2.17(s, 2H), 3.11-3.22(m, 2H), 3.27-3.32(m, 2H), 3.94(dd, J=3.8, 9.3Hz, 1H), 4.43(d, J=11.0Hz, 1H), 4.68(d, J=11.0Hz, 1H), 4.93-5.00(m, 2H), 6.69(brd, J=6.6Hz, 1H), 7.29-7.39(m, 5H), 7.57(d, J=8.5Hz, 1H).
HR-MS:$C_{25}H_{39}N_2O_7S_2$ Calcd.: 543.2200, Found: 543.2191
$[\alpha]^{25}_D$:-134.79° (c=0.434, MeOH)

Example 47

Methyl 3-((2(R)-(3,3-dimethylbutanoylamino)-2(R)-(methoxycarbonyl)ethyl)disulfanyl)-2(R)-(3,3-dimethyl-2(R,S)-benzylbutanoylamino)propionate (47)

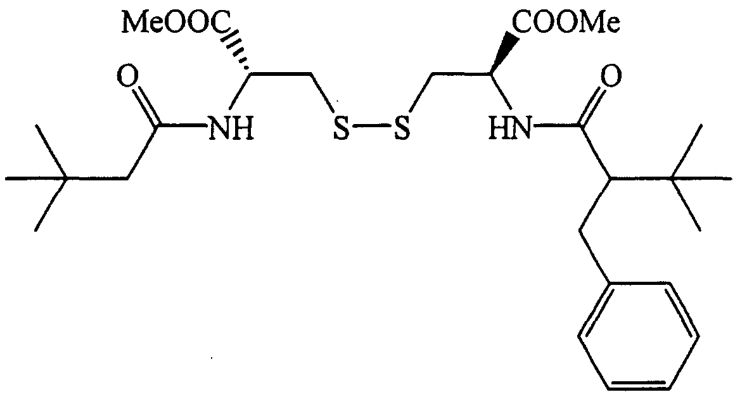

(47)

In 2 ml of DMF, 140 mg (0.38 mmol) of methyl 2(R)-amino-3-((2(R)-(3,3-dimethylbutanoylamino)-2(R)-(methoxycarbonyl)ethyl)disulfanyl)propionate (42) and 90 mg (0.38 mmol) of 10 were dissolved, and 0.09ml (0.42 mmol) of DPPA and 0.11ml (0.79 mmol) of triethylamine were added thereto while cooling the mixture in ice, followed by stirring the mixture at room temperature for 4.5 hours. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 2:1 - 3:2) to obtain 124 mg of methyl 3-((2(R)-(3,3-dimethylbutanoylamino)-2(R)-(methoxycarbonyl)ethyl)disulfanyl)-2(R)-(3,3-dimethyl-2(R,S)-benzylbutanoylamino)propionate (47) as colorless oil (yield 56%).
LR-MS(m/z):571($M^+$)
$^1$H-NMR (300MHz, $CDCl_3$, δppm) : 0.98(s, 4.5H), 0.99(s, 4.5H), 1.06(s, 9H), 1.78-1.82(m, 1H), 2.14(s, 2H), 2.30-2.41 (m, 1H), 2.72-3.22(m, 5H), 3.65(s, 1.5H), 3.68(s, 1.5H), 3.76(s, 3H), 4.50-4.89(m, 2H), 5.12(brs, 0.5H), 5.52(brs, 0.5H), 6.47-6.54(m, 1H), 7.11-7.25(m, 5H).

Example 48

3-((2(R)-carboxy-2(R)-(3,3-dimethylbutanoylamino)ethyl)disulfanyl)-2(R)-(3,3-dimethyl-2(R,S)-benzylbutanoylamino) propionic acid (48)

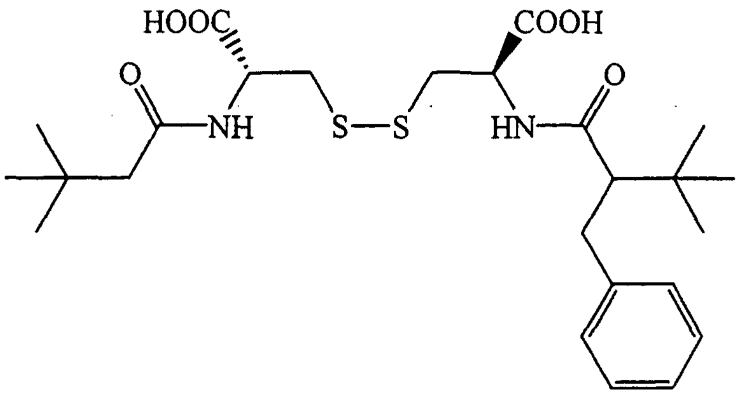

(48)

In 2 ml of toluene, 75 mg (0.13 mmol) of methyl 3-((2(R)-(3,3-dimethylbutanoylamino)-2(R)-(methoxycarbonyl) ethyl)disulfanyl)-2(R)-(3,3-dimethyl-2(R,S)-benzylbutanoylamino)propionate (47) was dissolved, and 320 mg (1.29

mmol) of dibutyltin oxide was added, followed by stirring the mixture at 100°C for 12 hours. To the reaction solution, 10 ml of 10% aqueous potassium fluoride solution and 15 ml of ethyl acetate were added and the mixture was vigorously stirred for 1.5 hours. The organic layer was washed with 1N hydrochloric acid and saturated brine, and dried over anhydrous sodium sulfate, followed by concentrating the mixture under reduced pressure. The residue was purified by diol Lobar column chromatography (hexane:ethyl acetate = 1:1 - 1:2) to obtain 36 mg of 3-((2(R)-carboxy-2(R)-(3,3-dimethylbutanoylamino)ethyl)disulfanyl)-2(R)-(3,3-dimethyl-2(R,S)-benzylbutanoylamino)propionic acid (48) as amorphous product (yield 50%).
LR-MS(m/z):527$(M+H)^+$
IR(KBr):3300~2500, 2958, 1720, 1234$cm^{-1}$
$^1$H-NMR (300MHz, $CDCl_3$, δppm) : 0.98-1.04(m, 18H), 2.13-2.16(m, 3H), 2.32-2.42(m, 1H), 2.90-3.00(m, 3H), 3.14-3.26(m, 2H),3.64-3.70(m, 1H), 4.37-4.43(m, 1H), 4.61-4.88(m, 2H), 7.08-7.23(m, 5H).

Example 49

Synthesis of methyl 3-((2(R)-(3,3-dimethylbutanoylamino)-2(R)-(methoxycarbonyl)ethyl)disulfanyl)-2(R)-(2(R,S)-(tert-butyl)-5-phenylpentanoylamino)propionate (49)

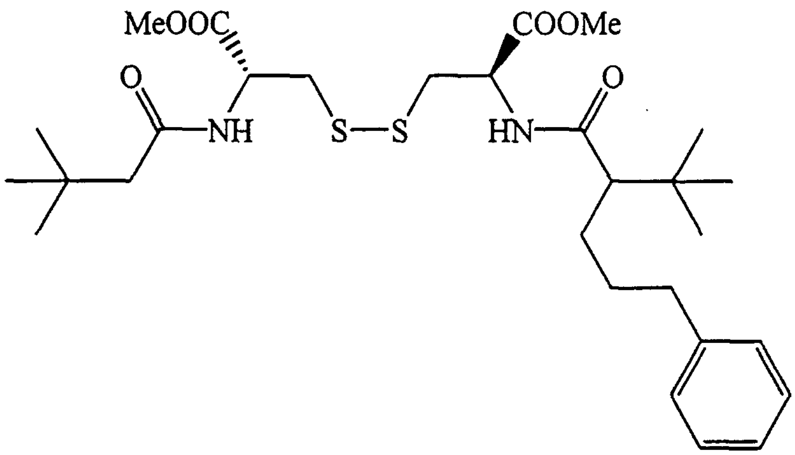

(49)

In 2 ml of DMF, 151 mg (0.37 mmol) of methyl 2(R)-amino-3-((2(R)-(3,3-dimethylbutanoylamino)-2(R)-(methoxycarbonyl)ethyl)disulfanyl)propionate (42) hydrochloric acid salt and 80 mg (0.39 mmol) of 2-(tert-butyl)-5-phenylvaleric acid were dissolved, and 0.1 ml (0.46 mmol) of DPPA and 0.16ml (1.15 mmol) of triethylamine were added thereto while cooling the mixture in ice, followed by stirring the mixture at room temperature for 8 hours. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 2:1 - 3:2) to obtain 156 mg of methyl 3-((2(R)-(3,3-dimethylbutanoylamino)-2(R)-(methoxycarbonyl)ethyl)disulfanyl)-2(R)-(2(R,S)-(tert-butyl)-5-phenylpentanoylamino)propionate (49) as colorless oil (yield 75%).
LR-MS(m/z):582$(M^+)$
$^1$H-NMR (300MHz, $CDCl_3$, δppm) : 0.88, 0.89(s, 9H), 1.04, 1.05(s, 9H), 1.61-1.89(m, 4H), 2.10, 2.14(s, 2H), 2.48-2.60 (m, 1H), 2.65-2.74(m, 1H), 3.03-3.27(m, 3H), 3.39-3.63(m, 2H), 3.67, 3.71(s, 3H), 3.76(s, 3H), 4.75-4.87(m, 2H), 5.65-5.75(br, 1H), 6.40-6.48(br, 1H), 7.13-7.28(m, 5H).

Example 50

3-((2(R)-carboxy-2(R)-(3,3-dimethylbutanoylamino)ethyl)disulfanyl)-2(R)-(2(R,S)-(tert-butyl)-5-phenylpentanoylamino)propionic acid (50)

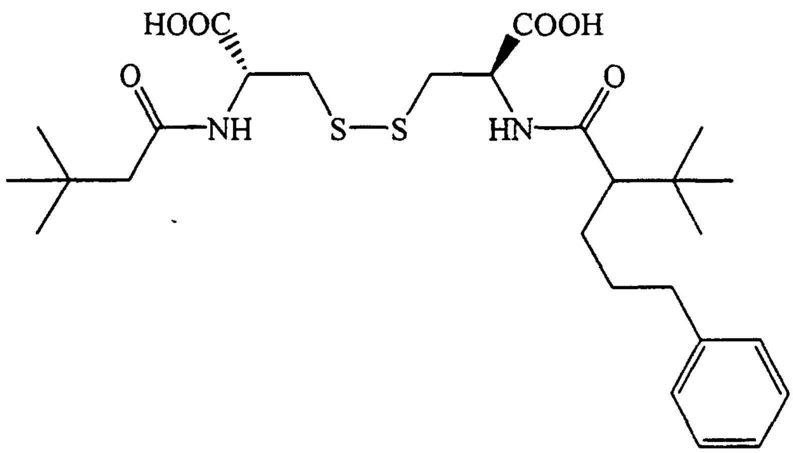

(50)

In 3 ml of toluene, 142 mg (0.26 mmol) of methyl 3-((2(R)-(3,3-dimethylbutanoylamino)-2(R)-(methoxycarbonyl)ethyl)disulfanyl)-2(R)-(2(R,S)-(tert-butyl)-5-phenylpentanoylamino)propionate (49) was dissolved, and 638 mg (1.29 mmol) of dibutyltin oxide was added, followed by stirring the mixture at 100°C for 21 hours. To the reaction solution, 10 ml of 10% aqueous potassium fluoride solution and 15 ml of ethyl acetate were added and the mixture was vigorously stirred for 1 hour. The organic layer was washed with 1N hydrochloric acid and saturated brine, and dried over anhydrous sodium sulfate, followed by concentrating the mixture under reduced pressure. The residue was purified by diol Lobar column chromatography (hexane:ethyl acetate = 1:1 - 1:2) to obtain 25 mg of 3-((2(R)-carboxy-2(R)-(3,3-dimethylbutanoylamino)ethyl)disulfanyl)-2(R)-(2(R,S)-(tert-butyl)-5-phenylpentanoylamino)propionic acid (50) as amorphous product (yield 19%).
LR-MS(m/z):555$(M+H)^+$
IR(KBr):3300-2500, 2958, 1720, 1234$cm^{-1}$
$^1$H-NMR (300MHz, $CDCl_3$, δppm) : 0.86(s, 9H), 1.03(s, 9H), 1.60-1.73(m, 4H), 2.13(s, 2H), 2.49-2.58(m, 1H), 2.63-2.72 (m, 1H), 2.88-3.28(m, 4H), 3.46(brd, J=11.5Hz, 1H), 4.60-4.64(m, 1H), 4.67-4.72(m, 1H), 7.08-7.25(m, 5H).

Example 51

Synthesis of methyl 3-((2(R)-(3,3-dimethylbutanoylamino)-2(R)-(methoxycarbonyl)ethyl)disulfanyl)-2(R)-(3,3-dimethyl-2(S)-((4-methoxyphenyl)methoxy)butanoylamino)propionate (51)

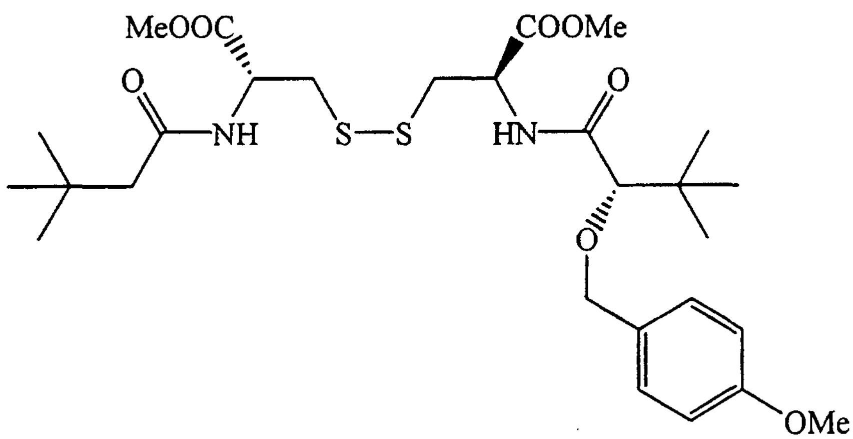

(51)

In 1.5 ml of DMF, 61 mg (242 μmol) of methyl 2(R)-amino-3-((2(R)-(3,3-dimethylbutanoylamino)-2(R)-(methoxycarbonyl)ethyl)disulfanyl)propionate (42) hydrochloric acid salt and 97 mg (0.24 mmol) of(S)-3,3-dimethyl-2-((4-methoxyphenyl)methoxy)butyric acid were dissolved, and 155 mg (298 μmol) of PyBOP and 40 μl (372 μmol) of N-methylmorpholine were added, followed by stirring the mixture at room temperature for 30 minutes. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 2:1- 3:2) to obtain 117 mg of methyl 3-((2(R)-(3,3-dimethylbutanoylamino)-2(R)-(methoxycarbonyl)ethyl)disulfanyl)-2(R)-(3,3-dimethyl-2(S)-((4-methoxyphenyl)methoxy)butanoylamino)propionate (51) as colorless oil (yield 81%).
LR-MS(m/z):601($M^+$)
$^1$H-NMR (300MHz, $CDCl_3$, δppm) : 0.98(s, 9H), 1.05(s, 9H), 2.13(s, 2H), 3.16-3.22(m, 4H), 3.47(s, 1H), 3.76(s, 3H), 3.79(s, 3H), 3.82(s, 3H), 4.33(d, J=10.7Hz, 1H), 4.62(d, J=10.7Hz, 1H), 4.82-4.96(m, 2H), 6.28(d, J=7.7Hz, 1H), 6.87-6.92(m, 2H), 7.26(d, J=8.1Hz, 1H), 7.30-7.33(m, 2H).

Example 52

3-((2(R)-carboxy-2(R)-(3,3-dimethylbutanoylamino)ethyl)disulfanyl)-2(R)-(3,3-dimethyl-2(S)-((4-methoxyphenyl)methoxy)butanoylamino)propionic acid (52)

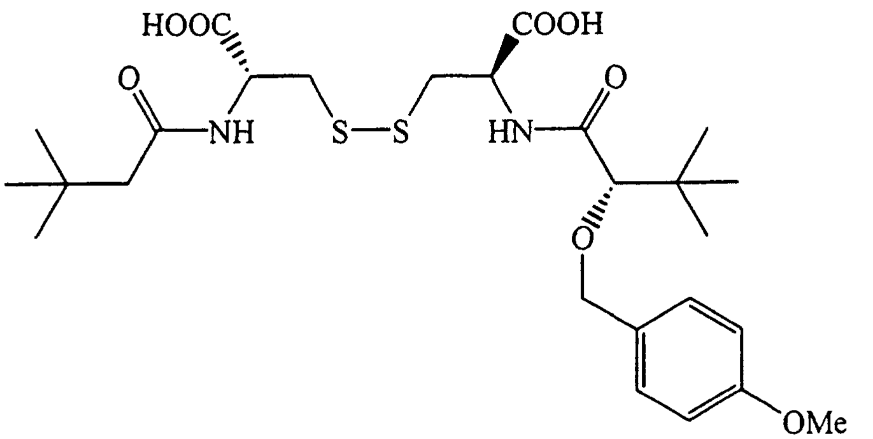

(52)

In 7 ml of toluene, 99 mg (0.17 mmol) of methyl 3-((2(R)-(3,3-dimethylbutanoylamino)-2(R)-(methoxycarbonyl)ethyl)disulfanyl)-2(R)-(3,3-dimethyl-2(S)-((4-methoxyphenyl)methoxy)butanoylamino)propionate (51) was dissolved, and 0.9ml (1.77 mmol) of di-tributyltin oxide was added, followed by stirring the mixture at 100°C for 5 hours: The reaction solution was concentrated, and 12 ml of 10% aqueous potassium fluoride solution and 15 ml of ethyl acetate were added to the residue, followed by vigorous stirring of the resulting mixture for 30 minutes. The organic layer was separated and dried over anhydrous sodium sulfate, followed by concentrating the mixture under reduced pressure. Hexane and saturated aqueous sodium hydrogen carbonate solution were added to the residue, and the aqueous layer, after separation, was acidified with 1N hydrochloric acid and the resulting mixture was extracted with ethyl acetate. Organic layers were combined and washed with saturated brine. The resulting mixture was dried over anhydrous sodium sulfate and concentrated under reduced pressure. The residue was purified by diol Lobar column chromatography (hexane:ethyl acetate = 2:1 - 1:1 - 1:2 - ethyl acetate 100%) to obtain 34 mg of 3-((2(R)-carboxy-2(R)-(3,3-dimethylbutanoylamino)ethyl)disulfanyl)-2(R)-(3,3-dimethyl-2(S)-((4-methoxyphenyl)methoxy)butanoylamino)propionic acid (52) as amorphous product (yield 36%).
LR-MS(m/z):573$(M+H)^+$
$^1$H-NMR (300MHz, $CDCl_3$, δppm) : 0.98(s, 9H), 1.04(s, 9H), 2.18(s, 2H), 3.06-3.20(m, 2H), 3.27-3.34(m, 2H), 3.52(s, 1H), 3.79(s, 3H), 4.31(d, J=10.7Hz, 1H), 4.60(d, J=10.7Hz, 1H), 4.98-5.10(m, 2H), 6.68(d, J=7.7Hz, 1H), 6.84-6.90(m, 2H), 7.26-7.31(m, 2H), 7.43(d, J=8.5Hz, 1H).

Example 53

Methyl 3-((2(R)-((tert-butoxy)carbonylamino)-2(R)-(methoxycarbonyl)ethyl)disulfanyl)-2(R)-(4-methyl-2(S)-((4-methoxyphenyl)methoxy)pentanoylamino)propionate (53)

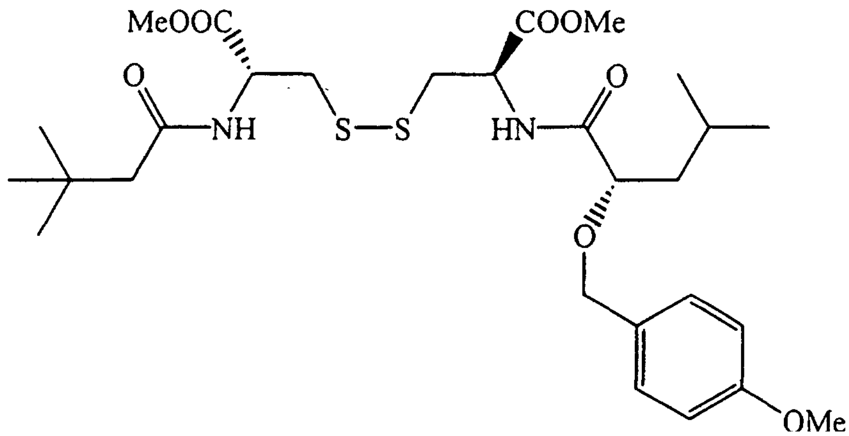

(53)

In 3 ml of DMF, 127 mg (0.32 mmol) of methyl 2(R)-amino-3-((2(R)-(3,3-dimethylbutanoylamino)-2(R)-(methoxycarbonyl)ethyl)disulfanyl)propionate (42) hydrochloric acid salt and 80 mg (0.32 mmol) of (S)-4-methyl-2-((4-methoxyphenyl)methoxy)valeric acid were dissolved, and 220 mg (0.42 mmol) of PyBOP and 0.06ml (0.5 mmol) of N-methylmorpholine were added thereto, followed by stirring the resulting mixture at room temperature for 6 hours. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 2:1 - 3:2) to obtain 138 mg of methyl 3-((2(R)-((tert-butoxy)carbonylamino)-2(R)-(methoxycarbonyl)ethyl)disulfanyl)-2(R)-(4-methyl-2(S)-((4-methoxyphenyl)methoxy) pentanoylamino)propionate (53) as colorless oil (yield 73%).
LR-MS(m/z):600($M^+$)
$^1$H-NMR (300MHz, $CDCl_3$, δppm) : 0.84 (3H, d, J = 6.6Hz), 0.92 (3H, d, J = 6.6Hz), 1.05 (9H, s), 1.52 (1H, m), 1.64 (1H, m), 1.82 (1H, m), 2.13, 2.14 (2H, s), 3.11-3.27 (4H, m), 3.75, 3.76, 3.77, 3.80 (6H, s), 3.82 (3H, s), 3.89, 3.90 (1H, dd, J = 3.6, 9.3Hz), 4.40 (1H, m), 4.60 (1H, m), 4.83-4.95 (2H, m), 6.32, 6.35 (1H, d, J = 7.4Hz), 6.88-6.92 (2H, m), 7.27-7.39 (3H, m).
$[\alpha]^{26}_D$:+45.6° (c=2.760, $CHCl_3$)

Example 54

Synthesis of 3-((2(R)-carboxy-2(R)-((tert-butoxy)carbonylamino)ethyl)disulfanyl)-2(R)-(4-methyl-2(S)-((4-methoxyphenyl)methoxy)pentanoylamino)propionic acid (54)

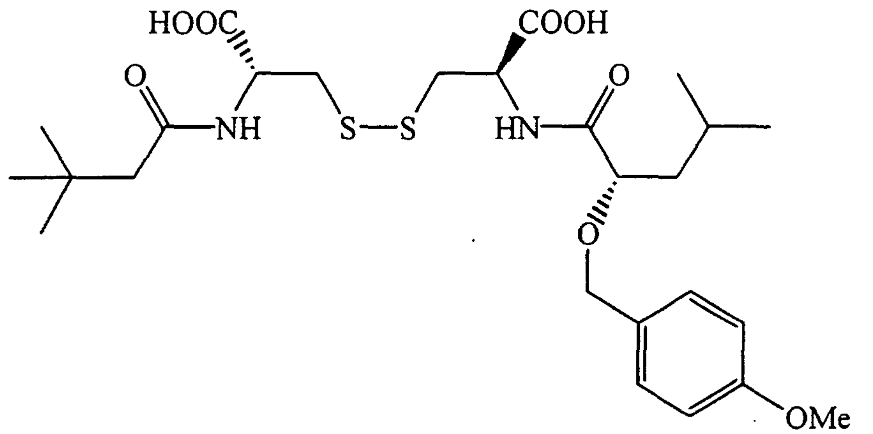

(54)

In 5 ml of toluene, 133 mg (0.17 mmol) of methyl 3-((2(R)-((tert-butoxy)carbonylamino)-2(R)-(methoxycarbonyl)ethyl)disulfanyl)-2(R)-(4-methyl-2(S)-((4-methoxyphenyl)methoxy)pentanoylamino)propionate (53) was dissolved, and 1.10 ml (2.16 mmol) of di-tributyltin oxide was added, followed by stirring the mixture at 100°C for 5.5 hours. The reaction solution was concentrated under reduced pressure, and toluene and saturated aqueous sodium hydrogen carbonate solution were added to the residue. The aqueous layer, after separation, was acidified with 6N hydrochloric acid under cooling in ice, and the resulting mixture was extracted with ethyl acetate. Organic layers were combined and washed with saturated brine. The resulting mixture was dried over anhydrous sodium sulfate and concentrated under reduced pressure. The residue was dissolved in acetonitrile/water = 1/1 and the resulting solution was made to pass through a C18 disposable column, followed by freeze-drying the eluted solution. The residue was purified by diol Lobar column (hexane:ethyl acetate = 1:1 - 1:2 - 1:3) to obtain 63 mg of 3-((2(R)-carboxy-2(R)-((tert-butoxy)carbonylamino)ethyl)disulfanyl)-2(R)-(4-methyl-2(S)-((4-methoxyphenyl)methoxy)pentanoylamino)propionic acid (54) as amorphous product (yield 50%).
LR-MS(m/z):573$(M+H)^+$
IR(KBr):3300-2500, 2957, 1734, 1250$cm^{-1}$
$^1$H-NMR (300MHz, $CDCl_3$, δppm) : 0.79-0.83 (3H, m), 0.88-0.91 (3H, m), 1.03 (9H, s), 1.51 (1H, m), 1.60 (1H, m), 1.79 (1H, m), 2.17 (2H, s), 3.10-3.21 (2H, m), 3.26-3.38 (2H, m), 3.79, 3.80 (3H, s), 3.92 (1H, m), 4.38 (1H, m), 4.60 (1H, m), 4.86-5.02 (2H, m), 6.75 (1H, m), 6.88 (2H, dd, J = 1.9, 8.5Hz), 7.25-7.31 (2H, m), 7.56 (1H, m).
$[\alpha]^{25}_D$:-113.80° (c=0.478, MeOH)

Example 55

Methyl 2(R)-(2(S)-((4-acetoxyphenyl)methoxy)-4-methylpentanoylamino)-3-((2(R)-((tert-butoxy)carbonylamino)-2(R)-(methoxycarbonyl)ethyl)disulfanyl)propionate (55)

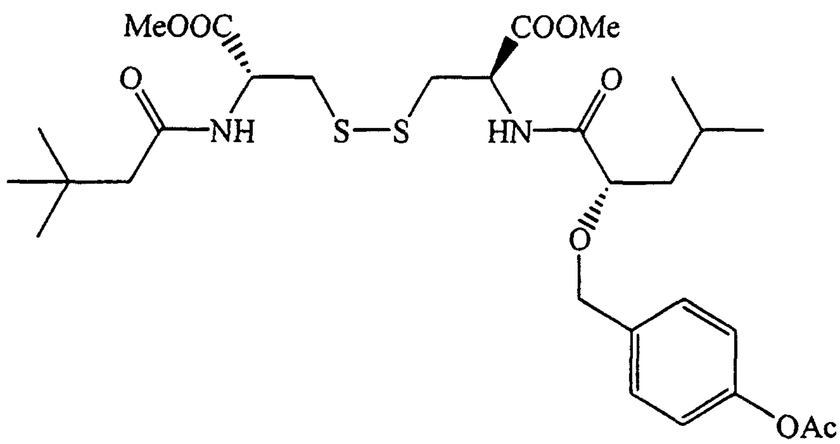

(55)

In 3 ml of DMF, 119 mg (0.3 mmol) of methyl 2(R)-amino-3-((2(R)-(3,3-dimethylbutanoylamino)-2(R)-(methoxycarbonyl)ethyl)disulfanyl)propionate (42) hydrochloric acid salt and 83 mg (0.3 mmol) of (S)-2-((4-acetoxyphenyl) methoxy-4-methylvaleric acid were dissolved, and 200 mg (0.3 mmol) of PyBOP and 0.05ml (0.46 mmol) of N-methylmorpholine were added thereto, followed by stirring the resulting mixture at room temperature for 1 hour. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 2:1 - 3:2) to obtain 103 mg of methyl 2(R)-(2(S)-((4-acetoxyphenyl)methoxy)-4-methylpentanoylamino)-3-((2(R)-((tert-butoxy)carbonylamino)-2(R)-(methoxycarbonyl)ethyl)disulfanyl)propionate (55) as colorless oil (yield 56%).
LR-MS(m/z):628($M^+$)
$^1$H-NMR (300MHz, $CDCl_3$, δppm) : 0.87 (3H, d, J = 6.6Hz), 0.93 (3H, d, J = 6.6Hz), 1.05 (9H, s), 1.56 (1H, m), 1.66 (1H, m), 1.84 (1H, m), 2.13 (2H, s), 2.31 (3H, s), 3.06-3.26 (4H, m), 3.75, 3.76, 3.77, 3.78 (6H, s), 3.92 (1H, m), 4.47 (1H, m), 4.66 (1H, m), 4.83-4.95 (2H, m), 6.34, 6.37 (1H, d, J = 7.4Hz), 7.09 (2H, dd, J = 2.2, 8.5Hz), 7.35-7.42 (3H, m).

Example 56

Synthesis of 3-((2(R)-carboxy-2(R)-((tert-butoxy)carbonylamino)ethyl)disulfanyl)-2(R)-(2(S)-((4-hydroxyphenyl) methoxy)-4-methylpentanoylamino)propionic acid (56)

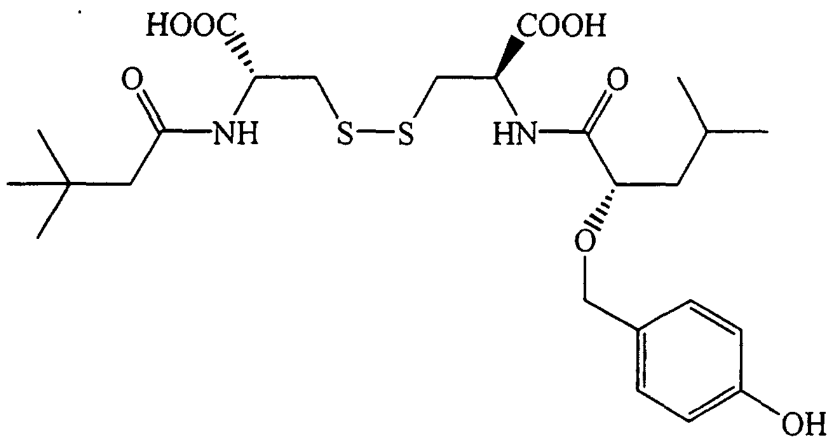

(56)

In 5 ml of toluene, 93 mg (0.15 mmol) of methyl 2(R)-(2(S)-((4-acetoxyphenyl)methoxy)-4-methylpentanoylamino)-3-((2(R)-((tert-butoxy)carbonylamino)=2(R)-(methoxycarbonyl)ethyl)disulfanyl)propionate was dissolved, and 0.8 ml (1.57 mmol) of di-tributyltin oxide was added, followed by stirring the mixture at 100°C for 15 hours. The reaction solution was concentrated under reduced pressure, and hexane and saturated aqueous sodium hydrogen carbonate solution were added to the residue. The aqueous layer, after separation, was acidified with 6N hydrochloric acid under cooling in ice, and the resulting mixture was extracted with ethyl acetate. Organic layers were combined and washed with saturated brine. The resulting mixture was dried over anhydrous sodium sulfate and concentrated under reduced pressure. The residue was dissolved in acetonitrile/water = 1/1 and the resulting solution was made to pass through a C18 disposable column, followed by freeze-drying the eluted solution. The residue was purified by diol Lobar column (hexane:ethyl acetate = 1:1 - 1:2 - 1:3) to obtain 58 mg of 3-((2(R)-carboxy-2(R)-((tert-butoxy)carbonylamino)ethyl)disulfanyl)-2(R)-(2(S)-((4-hydroxyphenyl)methoxy)-4-methylpentanoylamino)propionic acid (56) as amorphous product (yield 70%).

LR-MS(m/z):559$(M+H)^+$

IR(KBr):3300-2500, 2957, 1731, 1233$cm^{-1}$

$^1$H-NMR (300MHz, $CDCl_3$, δ ppm): 0.84-0.99 (6H, m), 1.03 (9H, s), 1.47-1.62 (2H, m), 1.81 (1H, m), 2.15 (2H, s), 3.11-3.35 (4H, m), 3.88, 3.90 (1H, dd, J = 3.9, 9.3Hz), 4.41 (1H, m), 4.53 (1H, m), 4.80-4.87 (2H, m), 6.76 (1H, m), 6.81-6.85 (2H, m), 7.19-7.25 (2H, m), 7.51 (1H, m).

$[\alpha]^{25}_D$:-241.37° (c=0.058, MeOH)

Example 57

Methyl 3-((2(R)-(2(S)-((3-acetoxyphenyl)methoxy)-4-methylpentanoylamino)-2(R)-(methoxycarbonyl)ethyl) disulfanyl)-2(R)-(3,3-dimethylbutanoylamino)propionate (57)

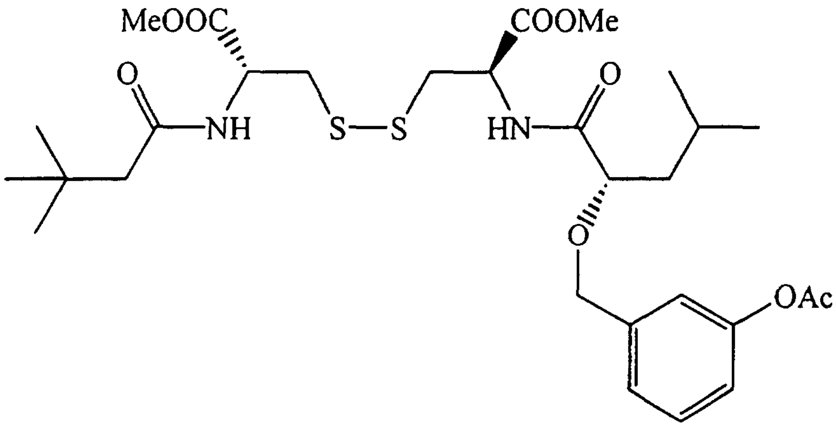

(57)

In 3 ml of DMF, 125 mg (310 μmol) of methyl 2(R)-amino-3-((2(R)-(3,3-dimethylbutanoylamino)-2(R)-(methoxycarbonyl)ethyl)disulfanyl)propionate (42) hydrochloric acid salt and 84 mg (0.3 mmol) of (S)-2-((3-acetoxyphenyl) methoxy)-4-methylvaleric acid were dissolved, and 215 mg (0.41 mmol) of PyBOP and 0.05ml (0.46 mmol) of N-methylmorpholine were added thereto, followed by stirring the resulting mixture at room temperature for 5.5 hours. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 2:1 - 3:2) to obtain 143 mg of methyl 3-((2(R)-(2(S) -((3-acetoxyphenyl)methoxy)-4-methylpentanoylamino)-2(R)-(methoxycarbonyl)ethyl)disulfanyl)-2(R)-(3,3-dimethylbutanoylamino)propionate (57) as colorless oil (yield 76%).
LR-MS(m/z):628($M^+$)
$^1$H-NMR (300MHz, $CDCl_3$, δppm) : 0.87, 0.87(d, J=6.6Hz, 3H), 0.94(d, J=6.6Hz, 3H), 1.04(s, 9H), 1.50-1.74(m, 2H), 1.81-1.91(m, 1H), 2.12(s, 2H), 2.31, 2.32(s, 3H), 3.05-3.25(m, 4H), 3.75(s, 3H), 3.77, 3.78(s, 3H), 3.89-3.95(m, 1H), 4.47, 4.50(d, J=11.3Hz, 1H), 4.66, 4.69(d, J=11.3Hz, 1H), 4.80-4.94(m, 2H), 6.35, 6.41(d, J=7.5Hz, 1H), 7.03-7.07(m, 1H), 7.10-7.14(m, 1H), 7.22-7.27(m, 1H), 7.33-7.45(m, 2H).

Example 58

3-((2(R)-carboxy-2(R)-(2(S)-((3-hydroxyphenyl)methoxy)-4-methylpentanoylamino)ethyl)disulfanyl)-2(R)-(3,3-dimethylbutanoylamino)propionic acid (58)

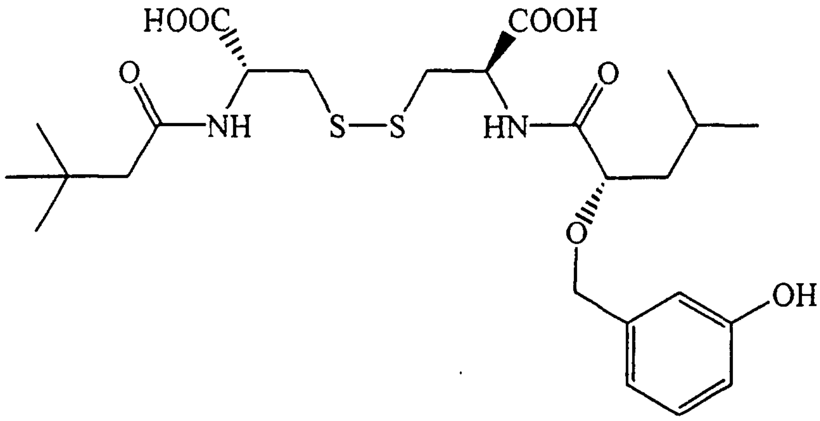

(58)

In 5 ml of toluene, 115 mg (0.18 mmol) of methyl 3-((2(R)-(2(S)-((3-acetoxyphenyl)methoxy)-4-methylpentanoylamino)-2(R)-(methoxycarbonyl)ethyl)disulfanyl)-2(R)-(3,3-dimethylbutanoylamino)propionate (57) was dissolved, and 1 ml (1.96 mmol) of di-tributyltin oxide was added thereto, followed by stirring the mixture at 100°C for 19 hours. The reaction solution was concentrated under reduced pressure, and hexane and saturated aqueous sodium hydrogen carbonate solution were added to the residue. The aqueous layer, after separation, was acidified with 6N hydrochloric acid under cooling in ice, and the resulting mixture was extracted with ethyl acetate. Organic layers were combined and washed with saturated brine. The resulting mixture was dried over anhydrous sodium sulfate and concentrated under reduced pressure. The residue was dissolved in acetonitrile/water = 1/1 and the resulting solution was made to pass through a C18 disposable column, followed by freeze-drying the eluted solution. The residue was purified by diol Lobar column (hexane:ethyl acetate = 1:1 - 1:2 - 1:5) to obtain 92 mg of 3-((2(R)-carboxy-2(R)-(2(S)-((3-hydroxyphenyl)methoxy)-4-methylpentanoylamino)ethyl)disulfanyl)-2(R)-(3,3-dimethylbutanoylamino)propionic acid (58) as amorphous product (yield 90%).
LR-MS(m/z):559$(M+H)^+$
IR(KBr):3300-2500, 2957, 1723, 1230$cm^{-1}$
$^1H$-NMR (300MHz, $CDCl_3$, δppm) : 0.86-0.94(m, 6H), 1.04(s, 9H), 1.50-1.70(m, 2H), 1.80-1.93(m, 1H), 2.14, 2.15(s, 2H), 3.05-3.35(m, 4H), 3.88-3.92(m, 1H), 4.41(d, J=10.8Hz, 1H), 4.57, 4.59(d, J=10.8Hz, 1H), 4.76-4.93(m, 2H), 6.63, 6.65(d, J=7.4Hz, 1H), 6.79-6.98(m, 3H), 7.16-7.25(m, 1H), 7.45(dd, J=7.9, 16.7Hz, 1H).
$[\alpha]^{25}_D$:-130.36° (c=0.662, MeOH)

Example 59

Methyl 3-((2(R)-(2(S)-((2-acetoxyphenyl)methoxy)-4-methylpentanoylamino)-2(R)-(methoxycarbonyl)ethyl) disulfanyl)-2(R)-(3,3-dimethylbutanoylamino)propionate (59)

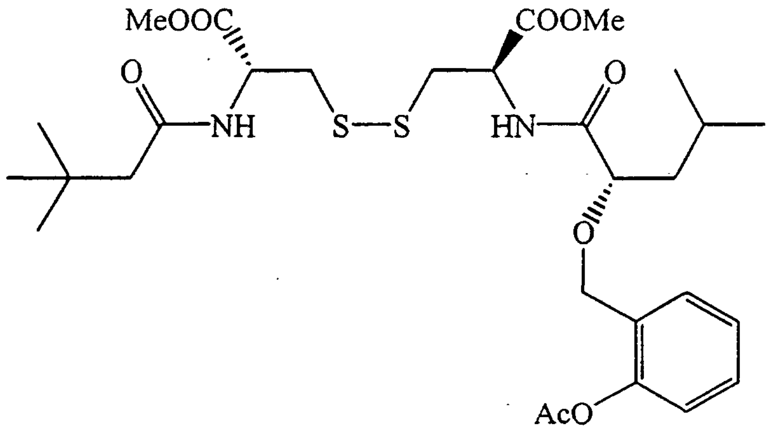

(59)

In 3 ml of DMF, 145 mg (0.36 mmol) of methyl 2(R)-amino-3-((2(R)-(3,3-dimethylbutanoylamino)-2(R)-(methoxycarbonyl)ethyl)disulfanyl)propionate (42) hydrochloric acid salt and 98 mg (0.35 mmol) of (S)-2-((2-acetoxyphenyl) methoxy)-4-methylvaleric acid were dissolved, and 240 mg (0.46 mol) of PyBOP and 0.06ml (0.55 mmol) of N-methylmorpholine were added thereto, followed by stirring the resulting mixture at room temperature for 3.5 hours. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 2:1 - 3:2) and by thin layer chromatography (hexane: ethyl acetate = 1:1) to obtain 163 mg of methyl 3-((2(R)-(2(S)-((2-acetoxyphenyl)methoxy)-4-methylpentanoylamino)-2(R)-(methoxycarbonyl)ethyl)disulfanyl)-2(R)-(3,3-dimethylbutanoylamino)propionate (59) as colorless oil (yield 74%).
LR-MS(m/z):628($M^+$)
$^1$H-NMR (300MHz, $CDCl_3$, δppm) : 0.87(d, J=6.6Hz, 3H), 0.93(d, J=6.6Hz, 3H), 1.05(s, 9H), 1.50-1.72(m, 2H), 1.77-1.89(m, 1H), 2.13(s, 2H), 2.32(s, 3H), 3.14-3.22(m, 4H), 3.75(s, 3H), 3.76(s, 3H), 3.91(dd, J=4.1, 9.1Hz, 1H), 4.48(d, J=11.5Hz, 1H), 4.60(d, J=11.5Hz, 1H), 4.79-4.91(m, 2H), 6.32(d, J=7.7Hz, 1H), 7.07-7.11(m, 1H), 7.25-7.39 (m, 3H), 7.44-7.54(m, 1H).

Example 60

3-((2(R)-carboxy-2(R)-(2(S)-((2-hydroxyphenyl)methoxy)-4-methylpentanoylamino)ethyl)disulfanyl)-2(R)-(3,3-dimethylbutanoylamino)propionic acid (60)

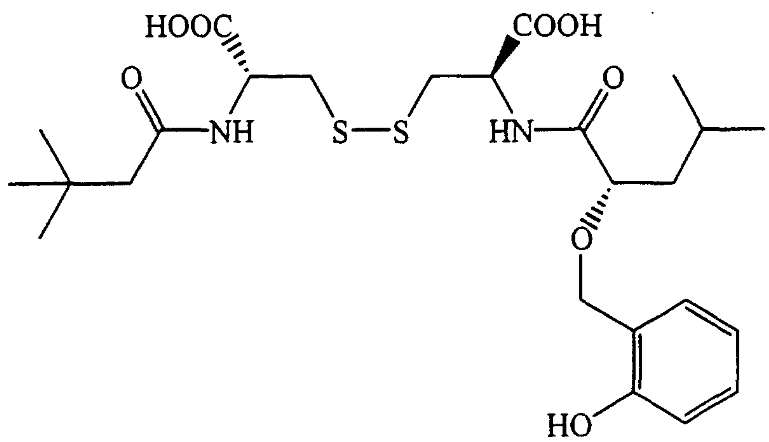

(60)

In 5 ml of toluene, 134 mg (0.21 mmol) of methyl 3-((2(R)-(2(S)-((2-acetoxyphenyl)methoxy)-4-methylpentanoylamino)-2(R)-(methoxycarbonyl)ethyl)disulfanyl)-2(R)-(3,3-dimethylbutanoylamino)propionate (59) was dissolved, and 1.10 ml (2.16 mmol) of di-tributyltin oxide was added thereto, followed by stirring the mixture at 100°C for 16 hours. The reaction solution was concentrated under reduced pressure, and hexane and saturated aqueous sodium hydrogen carbonate solution were added to the residue. The aqueous layer, after separation, was acidified with 6N hydrochloric acid under cooling in ice, and the resulting mixture was extracted with ethyl acetate. Organic layers were combined and washed with saturated brine. The resulting mixture was dried over anhydrous sodium sulfate and concentrated under reduced pressure. The residue was dissolved in acetonitrile/water = 1/1 and the resulting solution was made to pass through a C18 disposable column, followed by freeze-drying the eluted solution. The residue was purified by diol Lobar column (hexane:ethyl acetate = 1:2 - 1:3 - 1:5) to obtain 79 mg of 3-((2(R)-carboxy-2(R)-(2(S)-((2-hydroxyphenyl)methoxy)-4-methylpentanoylamino)ethyl)disulfanyl)-2(R)-(3,3-dimethylbutanoylamino)propionic acid (60) as amorphous product (yield 66%).
LR-MS(m/z):559(M+H)$^+$
IR(KBr):3300-2500, 2957, 1728, 1235cm$^{-1}$
$^1$H-NMR (300MHz, $CDCl_3$, δppm) : 1.03 (9H, s), 1.58 1.70 (2H, m), 1.83-1.92 (m, 6H), 1.84 (m, 1H), 2.13 (s, 2H), 3.00-3.42 (m, 4H), 3.94 (m, 1H), 4.34 (m, 1H), 4.76-4.88 (m, 3H), 6.64 (d, 1H), 6.80-7.22 (m, 4H), 7.95 (d, 1H).
$[\alpha]^{25}_D$:-154.49° (c=0.60, MeOH)

Example 61

Methyl 3-((2(R)-(2(R,S)-(4-acetoxyphenyl)-4-methylpentanoylamino)-2(R)-(methoxycarbonyl)ethyl)disulfanyl)-2(R) -(3,3-dimethylbutanoylamino)propionate (61)

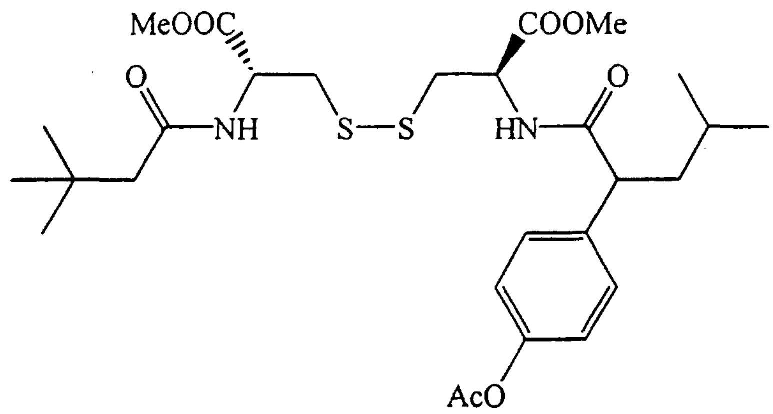

(61)

In 3 ml of DMF, 150 mg (0.37 mmol) of methyl 2(R)-amino-3-((2(R)-(3,3-dimethylbutanoylamino)-2(R)-(methoxycarbonyl)ethyl)disulfanyl)propionate (42) hydrochloric acid salt and 92 mg (0.37 mmol) of 2-(4-acetoxyphenyl)-4-methylvaleric acid were dissolved, and 249 mg (0.48 mmol) of PyBOP and 0.07 ml (0.59 mmol) of N-methylmorpholine were added thereto, followed by stirring the resulting mixture at room temperature for 1 hour. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 2:1- 3:2) to obtain 173 mg of methyl 3-((2(R)-(2(R,S)-(4-acetoxyphenyl)-4-methylpentanoylamino)-2(R)-(methoxycarbonyl)ethyl)disulfanyl)-2(R)-(3,3-dimethylbutanoylamino)propionate (61) as colorless oil (yield 79%).
LR-MS(m/z):598($M^+$)
$^1$H-NMR (300MHz, $CDCl_3$, δppm) : 0.88-0.92 (6H, m), 1.05, 1.06 (9H, s), 1.46 (1H, m), 1.69 (1H, m), 1.98 (1H, m), 2.12, 2.14 (2H, s), 2.30 (3H, s), 2.82-3.18 (4H, m), 3.55 (1H, t), 3.70, 3.73, 3.75, 3.76 (6H, s), 4.72-4.87 (2H, m), 6.38 (1H, m), 6.52 (1H, m), 7.00-7.08 (2H, m), 7.32-7.37 (2H, m).

Example 62

3-((2(R)-carboxy-2(R)-(2(R,S)-(4-hydroxyphenyl)-4-methylpentanoylamino)ethyl)disulfanyl)-2(R) -(3,3-dimethylbutanoylamino)propionic acid (62)

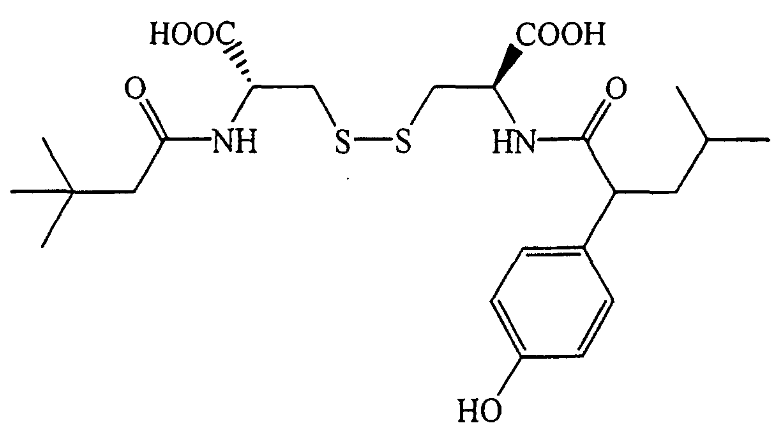

(62)

In 5 ml of toluene, 150 mg (0.25mmol) of methyl 3-((2(R)-(2(R,S)-(4-acetoxyphenyl)-4-methylpentanoylamino)-2(R)-(methoxycarbonyl)ethyl)disulfanyl)-2(R)-(3,3-dimethylbutanoylamino)propionate (61) was dissolved, and 1.3 ml (2.55 mmol) of di-tributyltin oxide was added, followed by stirring the mixture at 100°C for 12 hours. The reaction solution was concentrated under reduced pressure, and hexane and saturated aqueous sodium hydrogen carbonate solution were added to the residue. The aqueous layer, after separation, was acidified with 6N hydrochloric acid under cooling in ice, and the resulting mixture was extracted with ethyl acetate. Organic layers were combined and washed with saturated brine. The resulting mixture was dried over anhydrous sodium sulfate and concentrated under reduced pressure. The residue was dissolved in acetonitrile/water = 1/1 and the resulting solution was made to pass through a C18 disposable column, followed by freeze-drying the eluted solution. The residue was purified by diol Lobar column (hexane:ethyl acetate = 1:1 - 1:2 - 1:5) to obtain 103 mg of 3-((2(R)-carboxy-2(R)-(2(R,S)-(4-hydroxyphenyl)-4-methylpentanoylamino)ethyl)disulfanyl)-2(R)-(3,3-dimethylbutanoylamino)propionic acid (62) as amorphous product (yield 78%).
LR-MS(m/z):529(M+H)$^+$
IR(KBr):3300-2500, 2957, 1729, 1234cm$^{-1}$
$^1$H-NMR (300MHz, $CDCl_3$, δppm) : 0.78-0.88 (6H, m), 1.01 (9H, s), 1.39 (1H, m), 1.64 (1H, m), 1.88 (1H, m), 2.08 (1H, s), 2.10 (1H, s), 2.92-3.20 (4H, m), 3.43 (1H, m), 4.62-4.78 (2H, m), 6.53-6.67 (2H, m), 6.76 (2H, d), 7.06-7.13 (2H, m).
HR-MS: $C_{24}H_{37}N_2O_7S_2$ Calcd.:529.2042 Found: 529.1997

Example 63

Synthesis of methyl 3-((2(R)-(2(R,S)-(3-(4-acetoxyphenyl)propyl)-4-methylpentanoylamino)-2(R)-(methoxycarbonyl) ethyl)disulfanyl)-2(R)-(3,3-dimethylbutanoylamino)propionate (63)

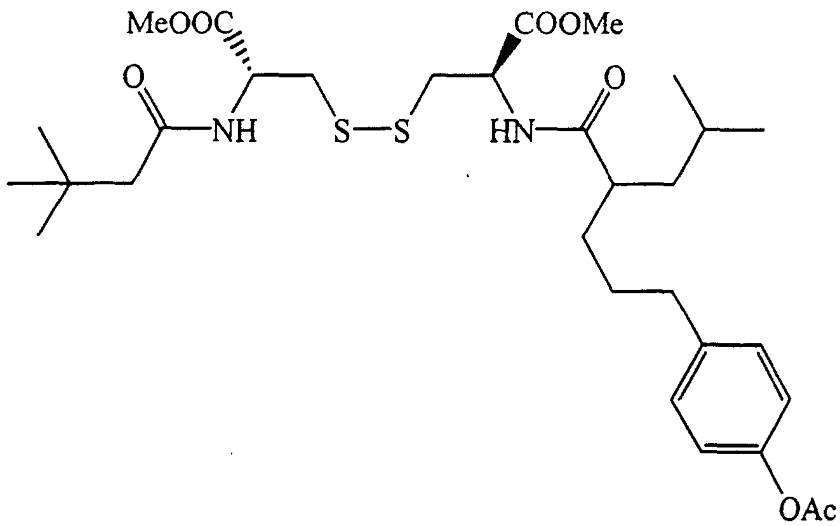

(63)

In 3 ml of DMF, 140 mg (0.35mmol) of methyl 2(R)-amino-3-((2(R)-(3,3-dimethylbutanoylamino)-2(R)-(methoxycarbonyl)ethyl)disulfanyl)propionate (42) hydrochloric acid salt and 101 mg (0.35mmol) of 2-(3-(4-acetoxyphenyl)-propyl)-4-methylvaleric acid were dissolved, and 240 mg (0.46 mmol) of PyBOP and 0.06 ml (0.55 mmol) of N-methylmorpholine were added thereto, followed by stirring the resulting mixture at room temperature for 3 hours. To the reaction solution, 1N hydrochloric acid was added and the mixture was extracted with ethyl acetate. Organic layers were combined and washed with saturated aqueous sodium hydrogen carbonate solution and saturated brine. The resulting mixture was dried over anhydrous sodium sulfate and concentrated under reduced pressure. The residue was purified by column chromatography (hexane:ethyl acetate = 2:1- 3:2) to obtain 130 mg of methyl 3-((2(R)-(2(R,S) -(3-(4-acetoxyphenyl)propyl)-4-methylpentanoylamino)-2(R)-(methoxycarbonyl)ethyl)disulfanyl)-2(R)-(3,3-dimethylbutanoylamino)propionate (63) as colorless oil (yield 59%).
LR-MS:m/z 640 (M$^+$).

Example 64

3-((2(R)-carboxy-2(R)-(2(R,S)-(3-(4-hydroxyphenyl)propyl)-4-methylpentanoylamino)ethyl)disulfanyl)-2(R)-(3,3-dimethylbutanoylamino)propionic acid (64)

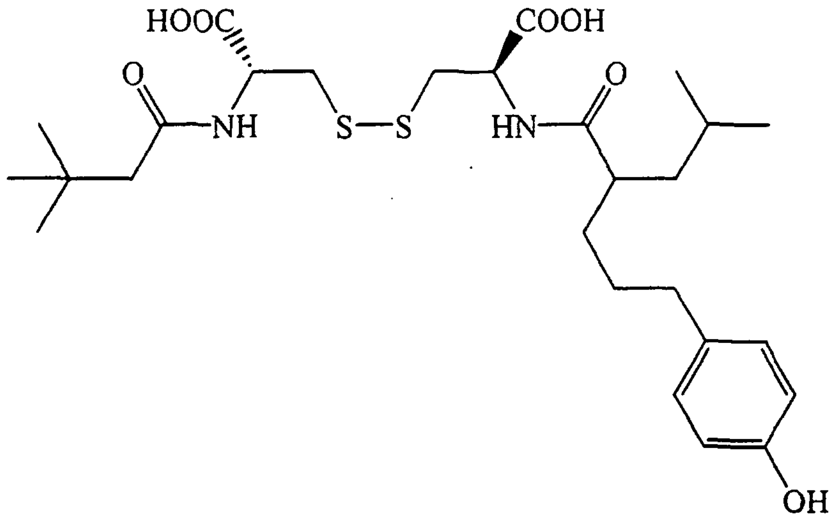

(64)

In 5 ml of toluene, 115 mg (0.18mmol) of methyl 3-((2(R)-(2(R,S)-(3-(4-acetoxyphenyl)propyl)-4-methylpentanoylamino)-2(R)-(methoxycarbonyl)ethyl)disulfanyl)-2(R)-(3,3-dimethylbutanoylamino)propionate (63) was dissolved, and 1 ml (1.96 mmol) of di-tributyltin oxide was added, followed by stirring the mixture at 100°C for 38 hours. The reaction solution was concentrated under reduced pressure, and hexane and saturated aqueous sodium hydrogen carbonate solution were added to the residue. The aqueous layer, after separation, was acidified with 6N hydrochloric acid under cooling in ice, and the resulting mixture was extracted with ethyl acetate. Organic layers were combined and washed with saturated brine. The resulting mixture was dried over anhydrous sodium sulfate and concentrated under reduced pressure. The residue was dissolved in acetonitrile/water = 1/1 and the resulting solution was made to pass through a C18 disposable column, followed by freeze-drying the eluted solution. The residue was purified by diol Lobar column (hexane:ethyl acetate = 1:1 - 1:2 - 1:5) to obtain 92 mg of 3-((2(R)-carboxy-2(R)-(2(R,S)-(3-(4-hydroxyphenyl)propyl)-4-methylpentanoylamino)ethyl)disulfanyl)-2(R)-(3,3-dimethylbutanoylamino)propionic acid (64) as amorphous product (yield 90%).
LR-MS(m/z): 571$(M+H)^+$
IR(KBr):3300~2500, 2953, 1729, 1234$cm^{-1}$
$^1$H-NMR (300MHz, $CDCl_3$, δppm) :0.84-0.89 (6H, m), 1.04 (9H, s), 1.12-1.28 (1H, m), 1.35-1.48 (1H, m), 1.55-1.70 (5H, m), 2.13, 2.14 (2H, s), 2.19-2.31 (1H, m), 2.50 (2H, t, J = 7.0Hz), 3.13-3.31 (4H, m), 4.74-4.90 (2H, m), 6.63-6.72 (2H, m), 6.76 (2H, dd, J = 3.3, 8.2Hz), 6.99 (2H, d, J = 8.2Hz).

Example 65

Methyl 2(R)-(3,3-dimethylbutanoylamino)-3-((2(R)-methylbutanoylamino)-2-(methoxycarbonyl)ethyl)disulfanyl) propionate (65)

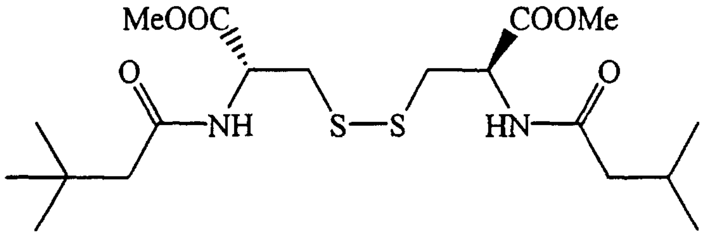

(65)

In 3 ml of dichloromethane, methyl 2(R)-amino-3-((2(R)-(3,3-dimethylbutanoylamino)-2(R)-(methoxycarbonyl)ethyl)disulfanyl)propionate (42) hydrochloric acid salt (200 mg, 0.50 mmol) was suspended, and triethylamine (152 mg) was added, followed by stirring the mixture in ice for 10 minutes. Isovaleric chloride (72 mg, 0.60 mmol) was added to the reaction solution and the mixture was stirred for 10 minutes while cooling the mixture in ice, followed by stirring the mixture at room temperature for 30 minutes. After adding 1N hydrochloric acid, the mixture was extracted with ethyl acetate. Organic layers were combined and washed with saturated aqueous sodium hydrogen carbonate solution and saturated brine. The resulting mixture was dried over anhydrous magnesium sulfate and concentrated under reduced pressure. The residue was purified by silica gel column chromatography to obtain 210 mg of methyl 2(R)-(3,3-dimethylbutanoylamino)-3-((2(R)-methylbutanoylamino)-2-(methoxycarbonyl)ethyl)disulfanyl)propionate (65) (yield: 93%).
LR-MS (m/z): 451 ((M+H)$^+$
IR(KBr): 3331, 2956, 1746, 1651, 1537, 1215, 756 cm$^{-1}$
$^1$H-NMR (300MHz, $CDCl_3$, δppm): 6.38 (2H, dd, J=15.38, 7.14 Hz), 4.86 (2H, m), 3.77 (6H, s), 3.14 (4H, m), 2.14 (5H, s), 1.06 (9H, s), 0.98 (3H, s), 0.96 (s, J=6.0Hz)

Example 66

3-((2(R)-carboxy-2-(3-methylbutanoylamino)ethyl)disulfanyl)-2(R)-(3,3-dimethylbutanoylamino)propionic acid (66)

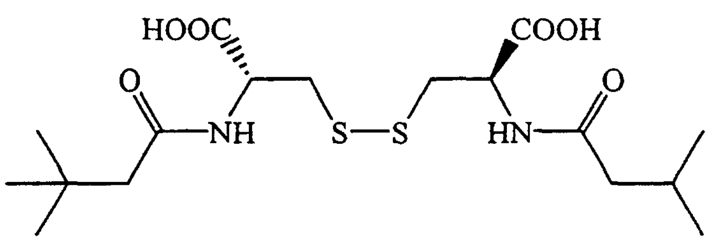

(66)

In 10 ml of toluene, methyl 2(R)-(3,3-dimethylbutanoylamino)-3-((2(R)-methylbutanoylamino)-2-(methoxycarbonyl)ethyl)disulfanyl)propionate (65) (186 mg, 0.41 mmol) was dissolved, and dibutyltin oxide (1.03 g, 4.12 mmol) was added thereto, followed by stirring the resulting mixture at 100°C for 17 hours. The reaction solution was concentrated, and n-hexane and saturated aqueous sodium hydrogen carbonate solution were added. To the aqueous layer, after separation, 6N hydrochloric acid was added and the resulting mixture was extracted with ethyl acetate. Organic layers were combined and washed with saturated brine. The resulting mixture was dried over anhydrous sodium sulfate and concentrated. The residue was recrystallized from ethyl acetate to obtain 88 mg of 3-((2(R)-carboxy-2-(3-methylbutanoylamino)ethyl)disulfanyl)-2(R)-(3,3-dimethylbutanoylamino)propionic acid (66) (51%).
m.p.: 202°C
LR-MS (m/z): 421 (M-H)$^+$
IR(KBr): 3333, 2957, 1719, 1610, 1552, 1221 cm$^{-1}$
$^1$H-NMR (300MHz, $CDCl_3$, δppm) : 6.63 (2H, dd, J=23.6, 7.7Hz), 4.83 (2H, m), 3.34 (4H, m), 2.14 (5H, s), 1.74 (2H,

s), 1.05 (9H, s), 0.96 (6H, d, J=6.0Hz)

| Elementary Analysis: $C_{17}H_{30}N_2O_6S_2$ | | | | |
|---|---|---|---|---|
| Calcd. | C; 48.32 | H;7.16 | N; 6.63 | S; 15.17 |
| Found | 48.23 | 7.17 | 6.68 | 15.57 |

Example 67

Methyl 3-((2(R)-(2(R,S)-(2-(4-acetyloxyphenyl)ethyl)-4-methylpentanoylamino)-2(R)-(methoxycarbonyl) ethyldisulfanyl)-2(R)-(3,3-dimethylbutanoylamino)propionate (67)

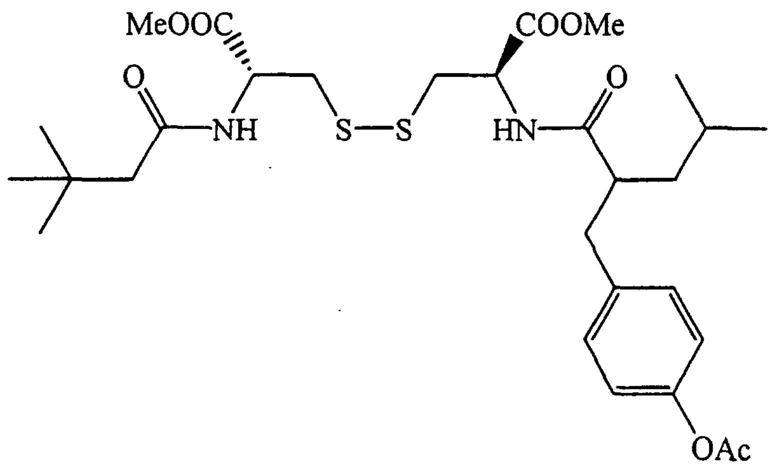

(67)

In 6 ml of DMF, 2-((4-acetyloxyphenyl)methyl)-4-methylvaleric acid (230 mg, 0.87 mmol) was dissolved, and methyl 2(R)-amino-3-((2(R)-(3,3-dimethylbutanoylamino)-2(R)-(methoxycarbonyl)ethyl)disulfanyl)propionate (42) hydrochloric acid salt (356 mg, 0.88 mmol), PyBOP (589 mg, 1.13 mmol) and N-methylmorpholine (141 mg, 1.39 mmol) were added thereto, followed by stirring the resulting mixture at room temperature. To the reaction solution, water was added and the mixture was extracted with ethyl acetate. Organic layers were combined and washed with saturated brine. The resulting mixture was dried over anhydrous magnesium sulfate and concentrated under reduced pressure. The residue was purified by silica gel column chromatography to obtain 405 mg of methyl-3-((2(R)-(2(R,S)-(2-(4-acetyloxyphenyl)ethyl)-4-methylpentanoylamino)-2(R)-(methoxycarbonyl)ethyldisulfanyl)-2(R)-(3,3-dimethylbutanoylamino)propionate (67) (76%).
LR-MS(m/z):613$(M+H)^+$
IR(neat): 3309, 2954, 1747, 1653, 1509, 1436, 1368, 1199 $cm^{-1}$
$^1$H-NMR (300MHz, $CDCl_3$, δppm) 7.18 (2H, d, J=7.7Hz), 6.96 (2H, d), 6.50 (1H, d, J=7.7Hz), 6.35 (1H, d, J=7.7Hz), 6.25 (2H, d, 7.7 Hz), 4.85-4.72 (2H, m), 3.79 (3H, s), 3.70 (3H, d, J=7.7Hz), 3.20-3.00 (3H, m), 2.90 (1H, m), 2.75 (1H, m), 2.54 (1H, m), 2.28 (3H, d, J=3.0Hz), 2.14 (2H, s), 1.30 (1H, m), 1.06 (9H, s), 0.91 (6H, m)

Example 68

3-((2(R)-(2(R,S)-(2-(4-acetyloxyphenyl)ethyl)-4-methylpentanoylamino)-2(R)-(methoxycarbonyl)ethyldisulfanyl)-2(R)-(3,3-dimethylbutanoylamino)propionic acid (68)

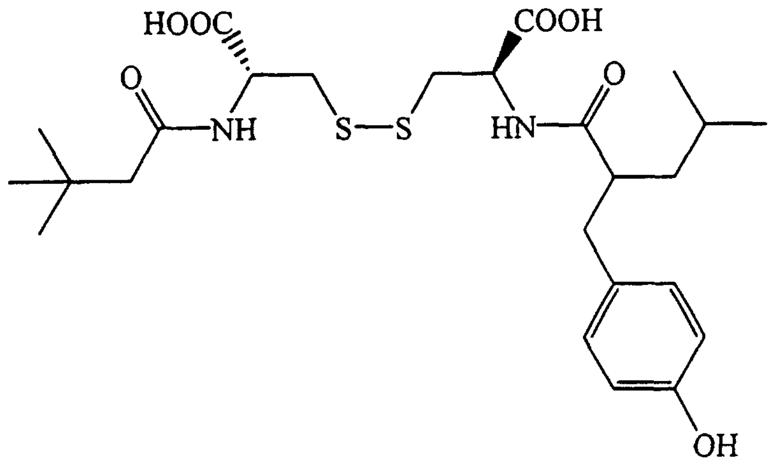

(68)

Methyl 3-((2(R)-(2(R,S)-(2-(4-acetyloxyphenyl)ethyl)-4-methylpentanoylamino)-2(R)-(methoxycarbonyl)ethyldisulfanyl)-2(R)-(3,3-dimethylbutanoylamino)propionate (67) (165 mg, 0.27 mmol) was dissolved in toluene, and 1.4 ml of bis-tributyltin oxide was added thereto, followed by stirring the mixture at 100°C for 12 hours. To the reaction solution, n-hexane and saturated aqueous sodium hydrogen carbonate solution were added, and the aqueous layer, after separation, was acidified with 6N hydrochloric acid to attain a pH of 1, followed by extraction with ethyl acetate. Organic layers were combined and washed with saturated brine. The resulting mixture was dried over anhydrous magnesium sulfate and concentrated under reduced pressure. The residue was made to pass through a C18 column (water:$CH_3CN$ = 1:1), followed by freeze-drying the eluted solution. The residue was purified by diol column chromatography to obtain 104 mg of 3-((2(R)-(2(R,S)-(2-(4-acetyloxyphenyl)ethyl)-4-methylpentanoylamino)-2(R)-(methoxycarbonyl)ethyldisulfanyl)-2(R)-(3,3-dimethylbutanoylamino)propionic acid (68) (71%).
LR-MS (m/z): 543 $(M+H)^+$
IR(KBr): 3758, 3416, 2957, 1650, 1517, 1231, 829 $cm^{-1}$
$^1$H-NMR (300MHz, $CD_3OD$, δppm): 7.00 (2H, m), 6.69 (2H, m), 4.70 (1H, m), 3.25 (2H, m), 3.14-2.46 (9H,m), 2.14 (2H, m), 1.62 (2H, m), 1.38-1.05 (3H, m), 1.03 (9H, s), 0.87 (6H, m)

Example 69

Methyl 3-((2(R)-(2(R,S)-(2-(3-acetyloxyphenyl)ethyl)-4-methylpentanoylamino)-2(R)-(methoxycarbonyl)ethyl) disulfanyl)-2(R)-(3,3-dimethlbutanoylamino)propionate (69)

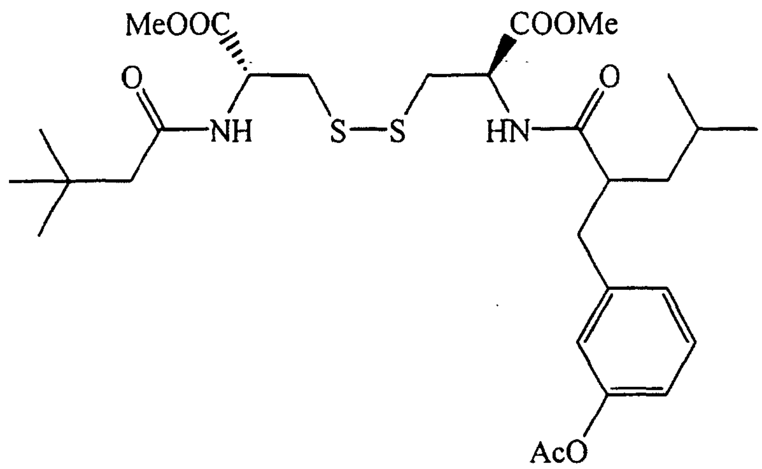

(69)

In 10 ml of DMF, 2-((3-acetyloxy)methyl)-4-methylvaleric acid (490 mg, 1.85 mmol) was dissolved, and methyl 2(R)-amino-3-((2(R)-(3,3-dimethylbutanoylamino)-2(R)-(methoxycarbonyl)ethyl)disulfanyl)propionate (42) hydrochloric acid salt (754 mg, 1.87 mmol), PyBOP (1.25 g, 2.41 mmol) and N-methylmorpholine (300 mg, 2.96 mmol) were added thereto, followed by stirring the resulting mixture at room temperature. To the reaction solution, water was added and the mixture was extracted with ethyl acetate. Organic layers were combined and washed with saturated brine. The resulting mixture was dried over anhydrous magnesium sulfate and concentrated under reduced pressure. The residue was purified by silica gel column chromatography to obtain 505 mg of methyl 3-((2(R)-(2(R,S)-(2-(3-acetyloxyphenyl) ethyl)-4-methylpentanoylamino)-2(R)-(methoxycarbonyl)ethyl)disulfanyl)-2(R)-(3,3-dimethlbutanoylamino)propionate (69) (yield: 45%).
LR-MS(m/z):613(M+H)$^+$

Example 70

3-((2(R)-carboxy-2(R)-(2(R,S)-(2-(3-hydroxyphenyl)ethyl)-4-methylpentanoylamino)ethyl)disulfanyl)-2(R) -(3,3-dimethylbutanoylamino)propionic acid (70)

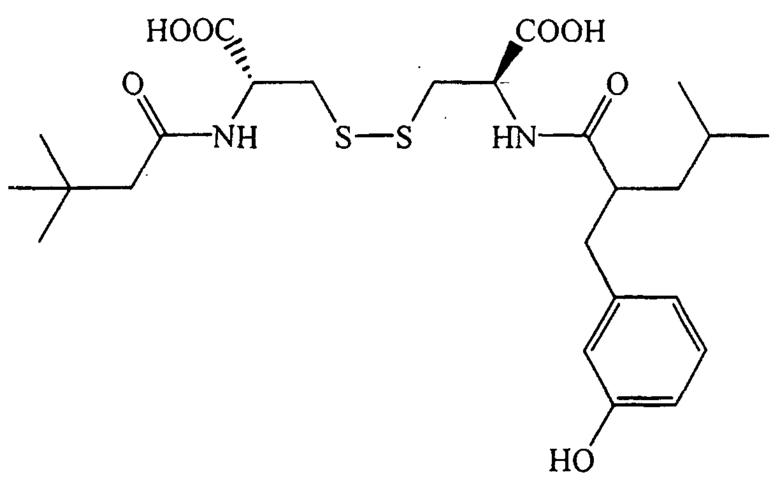

(70)

In toluene, methyl 3-((2(R)-(2(R,S)-(2-(3-acetyloxyphenyl)ethyl)-4-methylpentanoylamino)-2(R)-(methoxycarbonyl)ethyl)disulfanyl)-2(R)-(3,3-dimethlbutanoylamino)propionate (69) (155 mg, 0.25 mmol) was dissolved, and 1.3 ml of bis-tributyltin oxide was added thereto, followed by stirring the mixture at 100°C for 12 hours. To the reaction solution, n-hexane and saturated aqueous sodium hydrogen carbonate solution were added, and the aqueous layer, after separation, was acidified with 6N hydrochloric acid to attain a pH of 1, followed by extraction with ethyl acetate. Organic layers were combined and washed with saturated brine. The resulting mixture was dried over anhydrous magnesium sulfate and concentrated under reduced pressure. The residue was made to pass through a C18 column (water:$CH_3CN$ = 1:1), followed by freeze-drying the eluted solution. The residue was purified by diol column chromatography to obtain 75 mg of 3-((2(R)-carboxy-2(R)-(2(R,S)-(2-(3-hydroxyphenyl)ethyl)-4-methylpentanoylamino)ethyl) disulfanyl)-2(R)-(3,3-dimethylbutanoylamino)propionic acid (70) (yield 56%).
LR-MS (m/z):543 $(M+H)^+$
IR(KBr): 3320, 2957, 1649, 1540, 1233, 1009, 947, 785, 697 $cm^{-1}$
$^1$H-NMR (300MHz, $CD_3OD$, δppm): 7.05 (1H, m), 6.61 (4H, m), 4.69 (3H, m), 3.23 (2H, m), 3.09-2.55 (6H,m), 2.14 (3H, m), 1.61 (3H, m), 1.20 (1H, m), 1.04 (9H, s), 0.86 (6H, m)

Example 71

Methyl 2(R)-(3,3-dimethylbutanoylamino)-3-((2(R)-(4-methyl-2(R,S)-(4-methoxyphenyl)pentanoylamino)-2(R) -(methoxycarbonyl)ethyl)disulfanyl)propionate (71)

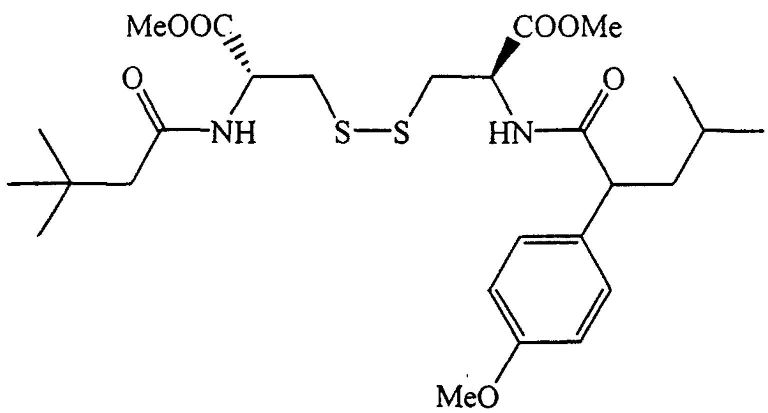

(71)

In 3 ml of DMF, 4-methyl-2-(4-methoxy)phenylvaleric acid (100 mg, 0.45 mmol) was dissolved, and methyl 2 (R)-amino-3-((2(R)-(3,3-dimethylbutanoylamino)-2(R)-(methoxycarbonyl)ethyl)disulfanyl)propionate (42) hydrochloric acid salt (183 mg, 0.45 mmol), PyBOP (304 mg, 0.59 mmol) and N-methylmorpholine (72 mg, 0.72 mmol) were added thereto, followed by stirring the resulting mixture at room temperature. To the reaction solution, water was added and the mixture was extracted with ethyl acetate. Organic layers were combined, washed with saturated brine, dried over anhydrous magnesium sulfate and concentrated under reduced pressure. The residue was purified by silica gel column chromatography to obtain 196 mg of methyl 2(R)-(3,3-dimethylbutanoylamino)-3-((2(R)-(4-methyl-2(R,S)-(4-methoxyphenyl)pentanoylamino)-2(R)-(methoxycarbonyl)ethyl)disulfanyl)propionate (71) (yield: 77%).
LR-MS (m/z):571 $(M+H)^+$
$^1$H-NMR (300MHz, $CDCl_3$, δppm) :7.22 (2H, d, J=7.2Hz), 6.85 (2H, d, J=7.2Hz), 6.32 (2H, m), 4.80 (3H, m), 3.79 (3H, s), 3.75 (3H, d), 3.70 (1H, s), 3.48 (2H, m), 3.18-3.00 (4H, m), 2.13 (2H, d, J=4.4Hz), 1.93 (1H, m), 1.74 (1H, m), 1.42 (1H, m), 1.05 (9H, s), 0.90 (6H, m)

Example 72

3-((2(R)-carbonyl-2(R)-(4-methyl-2(R,S)-(4-methoxyphenyl)pentanoylamino)ethyl)disulfanyl)-2(R)-(3,3-dimethylbutanoylamino)propionic acid (72)

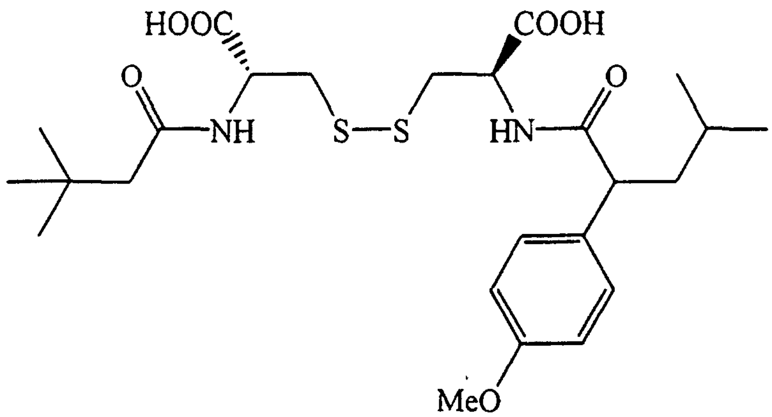

(72)

In 4 ml of toluene, methyl 2(R)-(3,3-dimethylbutanoylamino)-3-((2(R)-(4-methyl-2(R,S)-(4-methoxyphenyl)pentanoylamino)-2(R)-(methoxycarbonyl)ethyl)disulfanyl)propionate (71) (135 mg, 0.24 mmol) was dissolved, and 0.3 ml of bis-tributyltin oxide was added thereto, followed by stirring the mixture at 100°C for 12 hours. To the reaction solution, n-hexane and saturated aqueous sodium hydrogen carbonate solution were added, and the aqueous layer, after separation, was acidified with 6N hydrochloric acid to attain a pH of 1, followed by extraction with ethyl acetate. Organic layers were combined, washed with saturated brine, dried over anhydrous magnesium sulfate and concentrated under reduced pressure. The residue was made to pass through a C18 column (water:$CH_3CN$ = 1:1), and the eluted solution was freeze-dried. The residue was purified by diol column chromatography to obtain 76 mg of 3-((2(R)-carbonyl-2(R)-(4-methyl-2(R,S)-(4-methoxyphenyl)pentanoylamino)ethyl)disulfanyl)-2(R)-(3,3-dimethylbutanoylamino)propionic acid (72) (yield 59%).
LR-MS (m/z): 543 $(M+H)^+$
$^1$H-NMR (300MHz, $CDCl_3$, δppm) :8.21 (2H, bs), 7.24 (2H, d, J=8.8Hz), 6.85 (2H, d, J=8.8Hz), 6.74 (2H, m), 4.83 (2H, m), 3.78 (3H, s), 3.55 (1H, t, J=7.4Hz), 3.25-3.00 (4H, m), 2.16 (2H, s), 1.93 (1H, m), 1.71 (1H, m), 1.42 (1H, m), 1.02 (9H, s), 0.90 (6H, m)

Example 73

Methyl 3-((2(R)-((1-acetyl-3(R,S)-(4-acetoxyphenyl)pyrrolidin-2(R,S)-yl)carbonylamino)-2(R)-(methoxycarbonyl) ethyl)disulfanyl)-2(R)-(3,3-dimethylbutanoylamino)propionate (73)

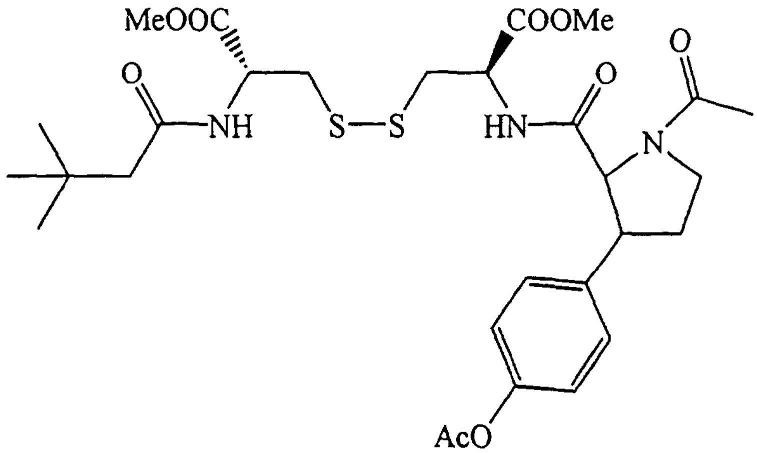

(73)

In 4 ml of DMF, 1-acetyl-3-(4-acetyloxyphenyl)pyrrolidine-2-carboxylic acid (95 mg, 0.33 mmol) was dissolved, and hydrochloric acid salt of Compound 42 (133 mg, 0.33 mmol), PyBOP (220 mg, 0.42 mmol) and N-methylmorpholine (53 mg, 0.52 mmol) were added to thereto, followed by stirring the mixture at room temperature for 3 hours. To the reaction solution, water was added and the mixture was extracted with ethyl acetate. Organic layers were combined, washed with saturated brine, dried over anhydrous magnesium sulfate and concentrated under reduced pressure. The residue was purified by silica gel column chromatography to obtain 95 mg of methyl 3-((2(R)-((1-acetyl-3(R,S)-(4-acetoxyphenyl)pyrrolidin-2(R,S)-yl)carbonylamino)-2(R)-(methoxycarbonyl)ethyl)disulfanyl)-2(R)-(3,3-dimethylbutanoylamino)propionate (73) (yield: 45%).
LR-MS (m/z):640$(M+H)^+$

Example 74

3-((2(R)-((1-acetyl-3(R,S)-(4-hydroxyphenyl)pyrrolidin-2(R,S)-yl)carbonylamino)-2(R)-carbonylethyl)disulfanyl)-2(R) -(3,3-dimethylbutanoylamino)propionic acid (74)

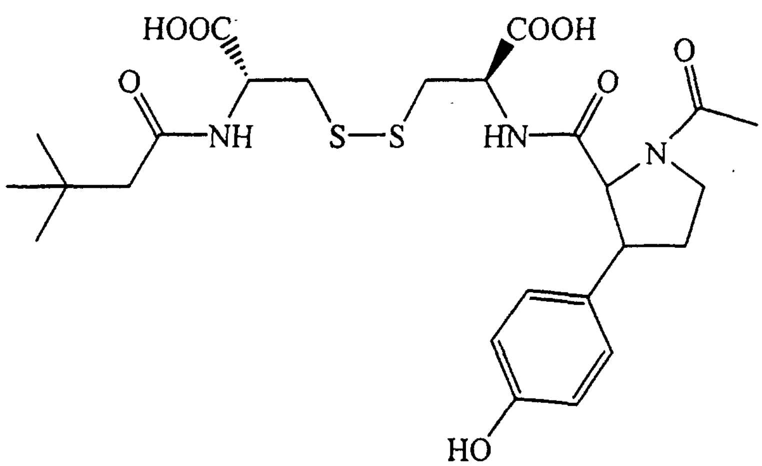

(74)

In 2 ml of toluene, methyl 3-((2(R)-((1-acetyl-3(R,S)-(4-acetoxyphenyl)pyrrolidin-2(R,S)-yl)carbonylamino)-2 (R)-(methoxycarbonyl)ethyl)disulfanyl)-2(R)-(3,3-dimethylbutanoylamino)propionate (73) (40 mg, 0.06 mmol) was dissolved, and 0.3 ml of bis-tributyltin oxide was added thereto, followed by stirring the mixture at 100°C for 12 hours. To the reaction solution, n-hexane and saturated aqueous sodium hydrogen carbonate solution were added, and the aqueous layer, after separation, was acidified with 6N hydrochloric acid to attain a pH of 1, followed by extraction with ethyl acetate. Organic layers were combined, washed with saturated brine, dried over anhydrous magnesium sulfate and concentrated under reduced pressure. The residue was made to pass through a C18 column (water:$CH_3CN$ = 1: 1) to remove bis-tributyltin oxide, and purified by diol column chromatography to obtain 18.8 mg of 3-((2(R)-((1-acetyl-3(R,S)-(4-hydroxyphenyl)pyrrolidin-2(R,S)-yl)carbonylamino)-2(R)-carbonylethyl)disulfanyl)-2(R)-(3,3-dimethylbutanoylamino)propionic acid (74) (yield 55%).
LR-MS (m/z): 640 $(M+H)^+$
$^1$H-NMR (300MHz, $CDCl_3$, δppm) 7.11 (2H, d, J=9.0Hz, 6.86 (2H, d, J=9.0Hz), 4.29 (2H, q, J=7.5Hz), 3.89 (3H, m), 3.81 (3H, s), 3.75 (2H, m), 2.61 (1H, m), 2.26 (3H, m), 2.15 (2H, m), 1.86-1.55 (6H, m), 1.12 (9H, s)

<u>Example 75</u>

Methyl 3-((2-(3,3-dimethylbutanoylamino)-2-(methoxycarbonyl)ethyl)disulfanyl)-2-(4-methyl-2-((tert-butoxy)carbonylamino)pentanoylamino)propionate (75)

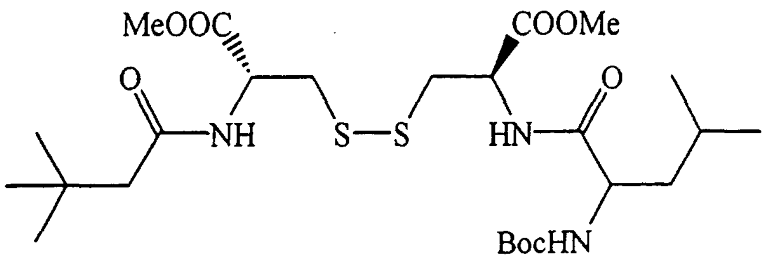

(75)

Boc-leucine hydrate (97 mg, 0.36 mmol) was dissolved in 3 ml of dichloromethane, and BOP (171 mg, 0.38 mmol), methyl 2(R)-amino-3-((2(R)-(3,3-dimethylbutanoylamino)-2(R)-(methoxycarbonyl)ethyl)disulfanyl)propionate (42) (130 mg, 0.32 mmol), diisopropylethylamine (0.25 ml, 1.37 mmol) were added to the solution, followed by stirring the mixture at room temperature for 1 and half hours. To the reaction solution, water was added and the mixture was extracted with ethyl acetate. Organic layers were combined, washed with saturated aqueous sodium hydrogen carbonate solution and saturated brine, dried over anhydrous magnesium sulfate and concentrated under reduced pressure. The residue was purified by silica gel column chromatography to obtain 176 mg of methyl 3-((2-(3,3-dimethylbutanoylamino)-2-(methoxycarbonyl)ethyl)disulfanyl)-2-(4-methyl-2-((tert-butoxy)carbonylamino)pentanoylamino)propionate (75) (yield: 94%).
LR-MS(m/z):580$(M+H)^+$
IR(neat): 3313, 2956, 1746, 1660, 1523, 1438, 1366, 1247, 1171 $cm^{-1}$
$^1$H-NMR (300MHz, $CDCl_3$, δppm): 0.95(6H, m), 1.05(9H,s), 1.62(9H,s), 1.71(1H,m), 1.73(2H,m), 2.05(2H,s), 3.18(4H, m), 3.77(6H,s), 4.18(1H,m), 4.85(2H,m), 5.09(1H,br), 6.38(1H,br), 7.05(1H,brs)
$[\alpha]^{20}_D$:-152.40° (c=0.35, MeOH)

Example 76

Methyl 3-((2-(3,3-dimethylbutanoylamino)-2-(methoxycarbonyl)ethyl)disulfanyl)-2-((2-amino-4-methylpentanoyl) amino)propionate (76)

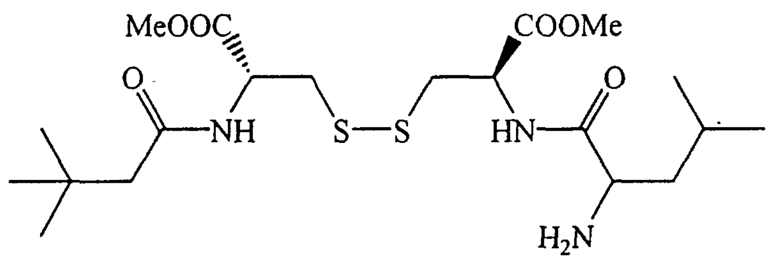

(76)

In 2 ml of dichloromethane, 176 mg (0.304 mmol) of methyl 3-((2-(3,3-dimethylbutanoylamino)-2-(methoxycarbonyl)ethyl)disulfanyl)-2-(4-methyl-2-((tert-butoxy)carbonylamino)pentanoylamino)propionate (75) was dissolved, and 2 ml of TFA was added thereto, followed by stirring the mixture at room temperature for 1 hour. The reaction solution was concentrated under reduced pressure to obtain methyl 3-((2-(3,3-dimethylbutanoylamino)-2-(methoxycarbonyl)ethyl)disulfanyl)-2-((2-amino-4-methylpentanoyl)amino)propionate (76). The product (76) was used in the next reaction without purification.

Example 77

Methyl 3-((2-(3,3-dimethylbutanoylamino)-2-(methoxycarbonyl)ethyl)disulfanyl)-2-(4-methyl-2-(2-(4-((phenylamino) carbonylamino)phenyl)acetylamino)pentanoylamino)propionate (77)

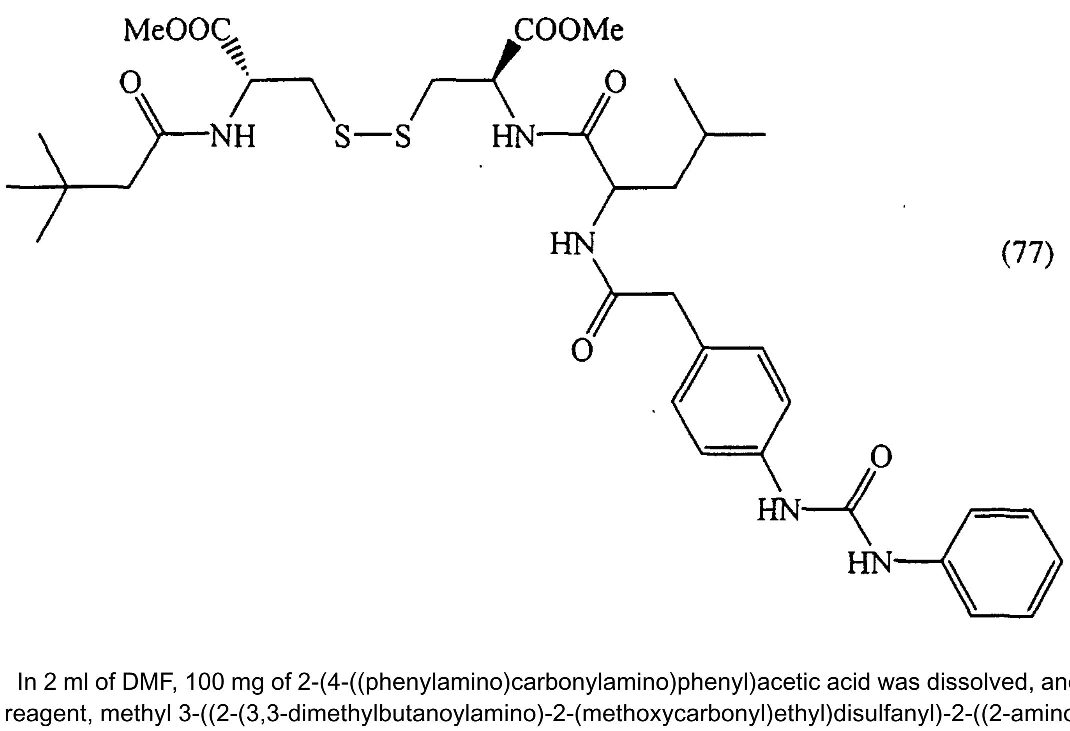

(77)

In 2 ml of DMF, 100 mg of 2-(4-((phenylamino)carbonylamino)phenyl)acetic acid was dissolved, and 160 mg of BOP reagent, methyl 3-((2-(3,3-dimethylbutanoylamino)-2-(methoxycarbonyl)ethyl)disulfanyl)-2-((2-amino-4-methylpentanoyl)amino)propionate (76) and 0.21 ml of diisopropylethylamine were added thereto, followed by stirring the mixture at room temperature for 15 hours. Water was added to the reaction solution and the mixture was extracted

with chloroform. Organic layers were combined, washed with 1N hydrochloric acid and saturated aqueous sodium hydrogen carbonate solution, dried over anhydrous magnesium sulfate and concentrated under reduced pressure. The residue was purified by silica gel column chromatography to obtain 130 mg of methyl 3-((2-(3,3-dimethylbutanoylamino)-2-(methoxycarbonyl)ethyl)disulfanyl)-2-(4-methyl-2-(2-(4-((phenylamino)carbonylamino)phenyl)acetylamino)pentanoylamino)propionate (77) (yield: 59%).
m.p.: 159.5°C
LR-MS(m/z):732$(M+H)^+$
IR(KBr): 3290, 2955, 1742, 1644, 1598, 1545, 1442, 1314, 1234, 753, 694 $cm^{-1}$
$^1$H-NMR (300MHz, $CDCl_3$, δppm) :0.70, 0.75 (6H, d), 0.84 (9H, s), 1.25-1.52 (3H, m), 1.93 (2H, s), 2.67-3.00 (4H, m), 3.33 (2H, s), 3.54 (3H, s), 3.57 (3H, s), 4.27 (1H, m), 4.52-4.61 (2H, m), 6.78-7.26 (14H, m).
$[\alpha]^{20}_D$:-104.86° (c=0.42, MeOH)

Example 78

3-((2-carboxy-2-(3,3-dimethylbutanoylamino)ethyl)disulfanyl)-2-(4-methyl-2-(2-(4-((phenylamino)carbonylamino) phenyl)acetylamino)pentanoylamino)propionic acid (78)

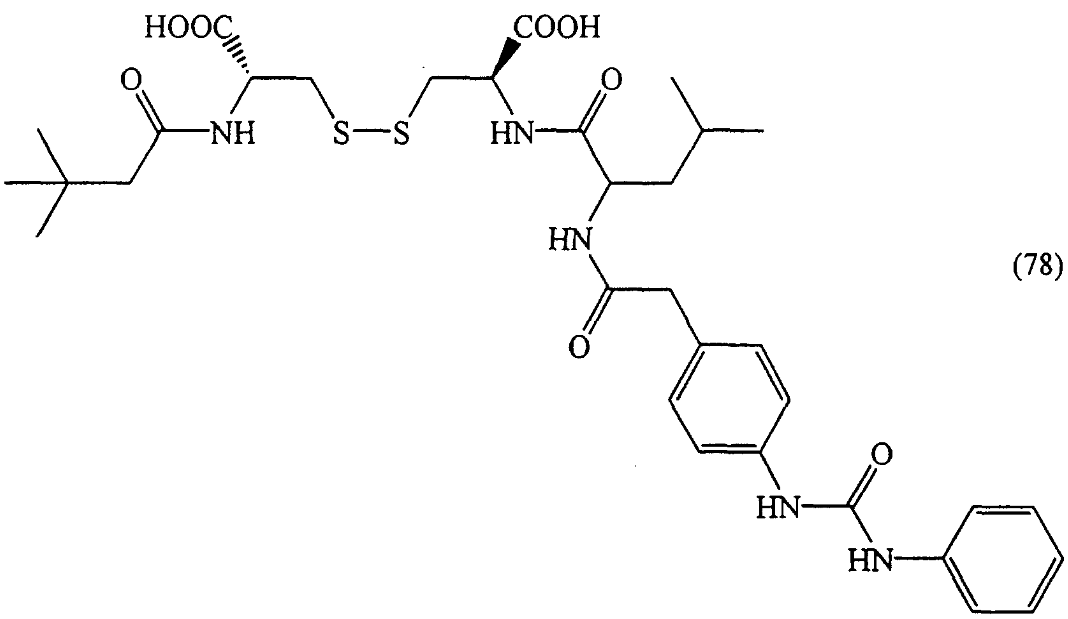

(78)

In 4 ml of toluene, methyl 3-((2-(3,3-dimethylbutanoylamino)-2-(methoxycarbonyl)ethyl)disulfanyl)-2-(4-methyl-2-(2-(4-((phenylamino)carbonylamino)phenyl)acetylamino)pentanoylamino)propionate (77) (30 mg, 0.04 mmol) was dissolved, and 0.2 ml of bis-tributyltin oxide was added thereto, followed by stirring the mixture at 100°C for 12 hours. To the reaction solution, n-hexane and saturated aqueous sodium hydrogen carbonate solution were added, and the aqueous layer, after separation, was acidified with 6N hydrochloric acid to attain a pH of 1, followed by extraction with ethyl acetate. Organic layers were combined, washed with saturated brine, dried over anhydrous magnesium sulfate and concentrated under reduced pressure. The residue was made to pass through a C18 column (water:$CH_3CN$ = 1:1) to remove bis-tributyltin oxide, and purified by diol column chromatography to obtain 16 mg of 3-((2-carboxy-2-(3,3-dimethylbutanoylamino)ethyl)disulfanyl)-2-(4-methyl-2-(2-(4-((phenylamino)carbonylamino)phenyl)acetylamino)pentanoylamino)propionic acid (78) (yield 54%).
LR-MS (m/z):704$(M+H)^+$
$^1$H-NMR (300MHz, $CDCl_3$, δppm) :0.74, 0.77 (6H, d), 0.88 (9H, s), 1.33-1.58 (3H, m), 1.98 (2H, s), 2.78-3.18 (4H, m), 3.21 (2H, s), 4.34 (1H, m), 4.48-4.63 (2H, m), 6.83-7.36 (14H, m).

Example 79

Methyl 2(S)-amino-3-((2(S)-((tert-butoxy)carbonylamino)-2(S)-(methoxycarbonyl)ethyl)disulfanyl)propionate (79)

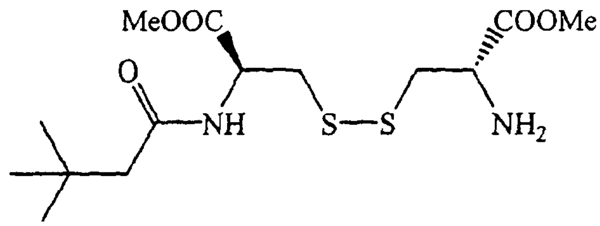

(79)

By the method similar to the method for synthesizing methyl 2(R)-amino-3-((2(R)-(3,3-dimethylbutanoylarnino)-2(R)-(methoxycarbonyl)ethyl)disulfanyl)propionate (42), methyl 2(S)-amino-3-((2(S)-((tert-butoxy)carbonylamino)-2(S)-(methoxycarbonyl)ethyl)disulfanyl)propionate (79) was obtained as colorless oil from D-cystine methyl ester.
LR-MS(m/z):366($M^+$)
$^1$H-NMR (300MHz, $CDCl_3$, δppm):1.03(s, 9H), 2.11(s, 2H), 2.91(dd, J=7.2, 13.8Hz, 1H), 3.08(dd, J=4.8, 13.8Hz, 1H), 3.18-3.21(m, 2H), 3.73(s, 3H), 3.75(s, 3H), 4.85-4.92(m, 1H), 6.34(brd, J=7.4Hz, 1H).

Example 80

Methyl 3-((2(S)-((tert-butoxy)carbonylamino)-2(S)-(methoxycarbonyl)ethyl)disulfanyl)-2(S)-(4-methyl-2(S)-(phenylmethoxy)pentanoylamino)propionate (80)

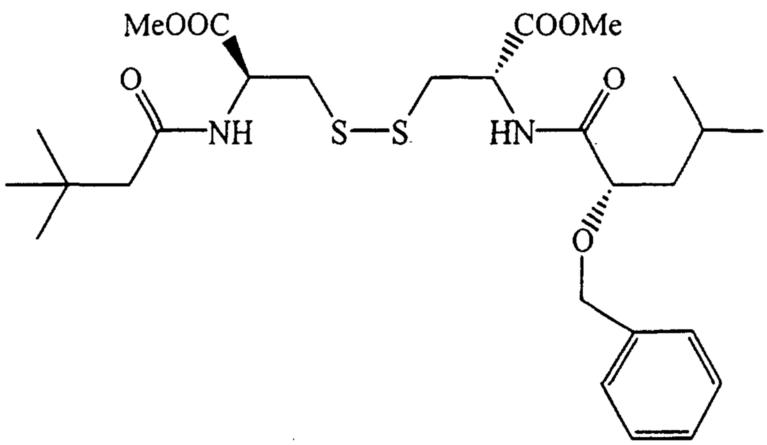

(80)

In 2 ml of DMF, 73 mg (199 μmol) of methyl 2(S)-amino-3-((2(S)-((tert-butoxy)carbonylamino)-2(S)-(methoxycarbonyl)ethyl)disulfanyl)propionate (79) and 50 mg (0.22mmol) of (S)-4-methyl-2-(phenylmethoxy)valeric acid were dissolved, and 135 mg (0.26mmol) of PyBOP and 0.03ml (0.29mmol) of N-methylmorpholine were added thereto, followed by stirring the mixture at room temperature for 15 hours. To the reaction solution, 1N hydrochloric acid was added and the mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 2:1- 3:2) to obtain 105 mg of methyl 3-((2(S)-((tert-butoxy)carbonylamino)-2(S)-(methoxycarbonyl)ethyl)disulfanyl)-2(S)-(4-methyl-2(S)-(phenylmethoxy)pentanoylamino)propionate (80) as colorless oil (yield: 92%).
LR-MS(m/z):570($M^+$)
$^1$H-NMR (300MHz, $CDCl_3$, δppm) : 0.85(d, J=6.6Hz, 3H), 0.92(d, J=6.9Hz, 3H), 1.05(s, 9H), 1.50-1.72(m, 2H), 1.81-1.90(m, 1H), 2.13(s, 2H), 3.10-3.26(m, 4H), 3.76(s, 3H), 3.77(s, 3H), 3.92(dd, J=3.8, 9.3Hz, 1H), 4.48(d, J=11.4Hz,

1H), 4.67(d, J=11.4Hz, 1H), 4.83-4.91(m, 2H), 6.34(brd, J=7.4Hz, 1H), 7.29-7.41(m, 6H).

Example 81

3-((2(S)-carboxy-2(S)-((tert-butoxy)carbonylamino)ethyl)disulfanyl)-2(S)-(4-methyl-2(S)-(phenylmethoxy) pentanoylamino)propionic acid (81)

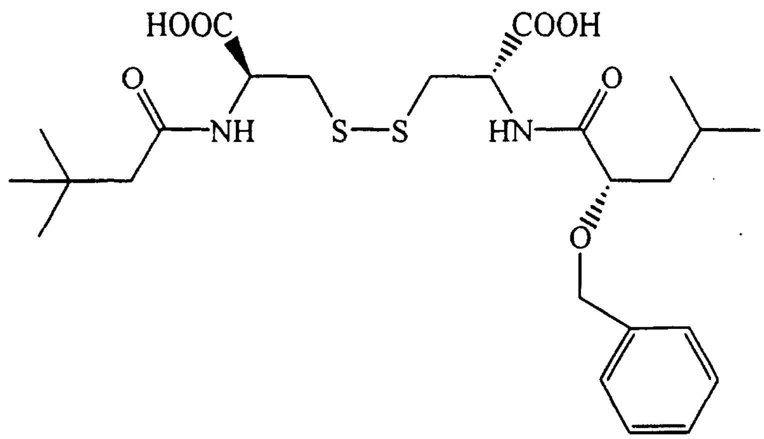

(81)

In 3 ml of toluene, 88 mg (0.15mmol) of methyl 3-((2(S)-((tert-butoxy)carbonylamino)-2(S)-(methoxycarbonyl) ethyl)disulfanyl)-2(S)-(4-methyl-2(S)-(phenylmethoxy)pentanoylamino)propionate (80) was dissolved, and 0.8ml (1.77 mmol) of di-tributyltin oxide was added, followed by stirring the mixture at 100°C for 23 hours. The reaction solution was concentrated, and 20 ml of 10% aqueous potassium fluoride solution and 25 ml of ethyl acetate were added to the residue, followed by vigorously stirring the resulting mixture for 1 hour. The aqueous layer, after separation, was acidified with 1N hydrochloric acid and the mixture was extracted with ethyl acetate. Organic layers were combined and dried over anhydrous sodium sulfate, followed by concentrating the mixture under reduced pressure. The residue was purified by diol column chromatography (hexane: ethyl acetate = 1:1 - 1:2) to obtain 47 mg of 3-((2(S)-carboxy-2 (S)-((tert-butoxy)carbonylamino)ethyl)disulfanyl)-2(S)-(4-methyl-2(S)-(phenylmethoxy)pentanoylamino)propionic acid (81) as an amorphous product (yield: 56%).
LR-MS(m/z):543$(M+H)^+$
IR(KBr):3300-2500, 2957, 1734, 1233$cm^{-1}$
$^1$H-NMR (300MHz, $CDCl_3$, δppm):0.83 (3H, d, J = 6.6Hz), 0.91 (3H, d, J = 6.6Hz), 1.04 (9H, s), 1.48-1.59 (1H, m), 1.60-1.74 (1H, m), 1.77-1.96 (1H, m), 2.13 (2H, s), 3.14-3.42 (4H, m), 3.88-3.92 (1H, m), 4.45 (1H, d, J = 11.3Hz), 4.70 (1H, d, J = 11.3Hz), 4.76-4.94 (2H, m), 6.53-6.63 (1H, brd), 7.23-7.38 (6H, m).
$[\alpha]^{25}_D$:+144.17° (c=0.29, MeOH)

Example 82

Methyl (S)-3-(4-(cyclohexylmethoxy)phenyl)-2-((tert-butoxy)carbonylamino)propionate (82)

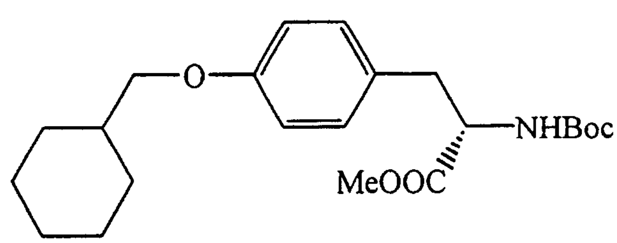

(82)

In 500 ml of dehydrated THF, 31.06 g of N-Boc-L-tyrosine methyl ester was dissolved, and 5.94 g (110mmol)

of sodium methoxide was added at 0°C under stirring, followed by stirring the mixture at room temperature for 30 minutes. To the reaction solution, 90.0 ml (517 mmol) of HMPA was added and the mixture was stirred at room temperature for 2 hours. Then 17.0 ml (121 mmol) of cyclohexylmethyl bromide was added and the mixture was stirred at 50°C for 44 hours. Water was added to the reaction solution and the mixture was extracted with ethyl acetate. Organic layers were combined, washed twice with saturated brine, dried over anhydrous sodium sulfate and concentrated under reduced pressure. The residue was purified by column chromatography (hexane: ethyl acetate = 9:1 - 5:1 - 3:1 - 2:1) to obtain 10.84 g of methyl (S)-3-(4-(cyclohexylmethoxy)phenyl)-2-((tert-butoxy)carbonylamino)propionate (82).
LR-MS(m/z):391($M^+$)
$^1$H-NMR(300MHz, $CDCl_3$, δppm):0.97-1.88(11H, m), 1.42(9H, s), 2.95-3.08(2H, m), 3.71(3H, s), 3.72(2H, d, J=4.5Hz), 4.49-4.57(1H, m), 4.95(1H, d, J=9.0Hz), 6.81(2H, d, J=8.5Hz), 7.01(2H, d, J=8.5Hz)

Example 83

Methyl (S)-2-amino-3-(4-(cyclohexylmethoxy)phenyl)propionate (83)

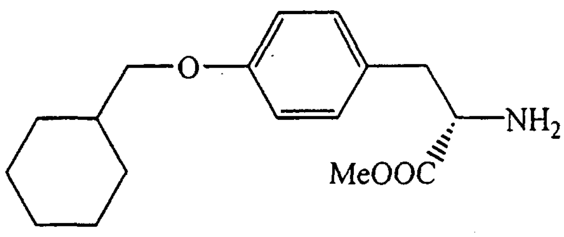

(83).

In 200 ml of dichloromethane, 10.68 g (27.28 mmol) of methyl (S)-3-(4-(cyclohexylmethoxy)phenyl)-2-((tert-butoxy)carbonylamino)propionate (82) was dissolved, and 11.5 ml (1.45mol) of trifluoroacetic acid was added, followed by stirring the mixture at room temperature for 22 hours. Saturated aqueous sodium hydrogen carbonate solution was added to the reaction solution and the mixture was extracted with ethyl acetate. Organic layers were combined, washed with saturated brine, dried over anhydrous sodium sulfate and concentrated under reduced pressure to obtain 7.61 g of methyl (S)-2-amino-3-(4-(cyclohexylmethoxy)phenyl)propionate (83) (yield: 95%). The product (83) was used in the next reaction without purification.
LR-MS(m/z):291($M^+$)
$^1$H-NMR(300MHz, $CDCl_3$, δppm): 1.01-1.88(11H, m), 2.82(1H, dd, J=8.0, 14.0Hz), 3.04(1H, dd, J=5.0, 14.0Hz), 3.722 (2H, d, J=6.5Hz), 3.723(3H, s), 6.83(2H, d, J=8.5Hz), 7.08(2H, d, J=8.5Hz)

Example 84

Synthesis of methyl 3-(4-(cyclohexylmethoxy)phenyl)-2(S)-(2(R,S)-(tert-butyl)-5-phenylpentanoylamino)propionate (84)

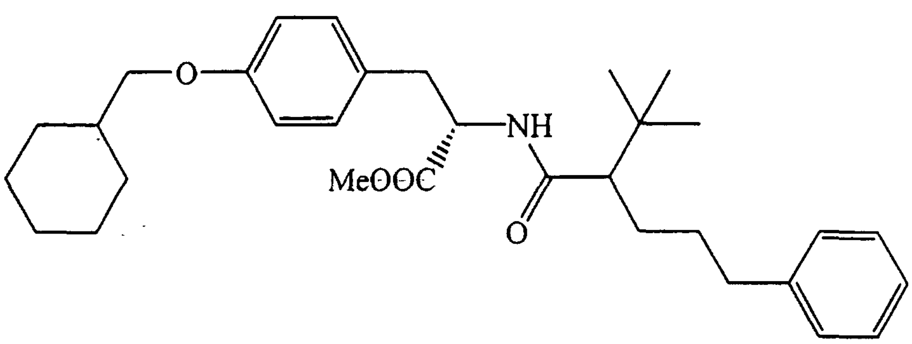

(84)

In 0.5 ml of DMF dehydrated with molecular sieves, 55 mg (0.19mmol) of methyl (S)-2-amino-3-(4-(cyclohexylmethoxy)phenyl)propionate (83) and 40 mg (0.17mmol) of 2-(tert-butyl)-5-phenylvaleric acid were dissolved, and 0.04ml (0.19mmol) of DPPA and 0.05ml (0.36mmol) of triethylamine were added at 0°C, followed by stirring the mixture at room temperature for 14 hours. To the reaction solution, 1N hydrochloric acid was added and the mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 9:1 - 5:1 - 3:1) to obtain 86 mg of methyl 3-(4-(cyclohexylmethoxy)phenyl)-2(S)-(2(R,S)-(tert-butyl)-5-phenylpentanoylamino)propionate (84) (yield: 99%).
LR-MS(m/z):507($M^+$)
$^1$H-NMR(300MHz, $CDCl_3$, δppm) : 0.84 and 0.85(9H, each s), 0.98-1.87(11H, m), 2.50-2.72(2H, m), 2.98-3.05(2H, m), 3.66-3.70(2H, m), 3.68 and 3.70(3H, each s), 3.95-4.02(1H, m), 4.57-4.62(1H, m), 4.67-4.76(1H, m), 6.71-6.77(2H, m), 6.94-6.99(2H, m), 7.15-7.29(5H, m)

Example 85

3-(4-(cyclohexylmethoxyphenyl)phenyl)-2(S)-(2(R,S)-(tert-butyl)-5-phenylpentanoylamino)propionic acid (85)

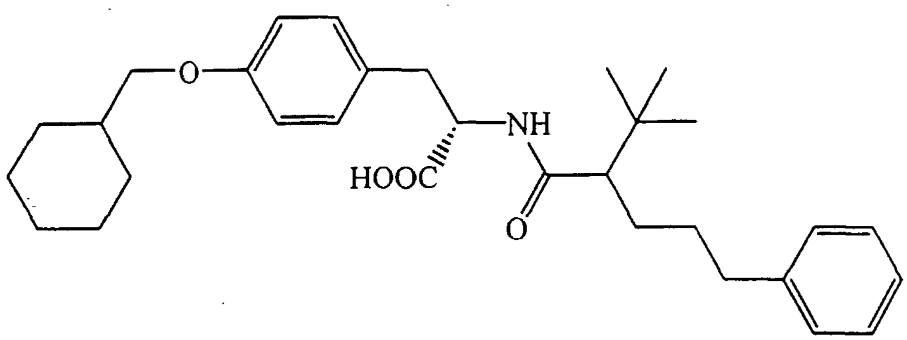

(85)

In 2 ml of methanol, 80 mg (0.16mmol) of methyl 3-(4-(cyclohexylmethoxy)phenyl)-2(S)-(2(R,S)-(tert-butyl)-5-phenylpentanoylamino)propionate (84) was dissolved, and 0.57ml (0.57mmol) of 1N aqueous sodium hydroxide solution was added, followed by stirring the mixture at room temperature for 20 hours. Water was added to the reaction solution and ether was added. The aqueous layer, after separation, was acidified with 1N hydrochloric acid and the mixture was extracted with ethyl acetate. Organic layers were combined, washed with saturated brine, dried over anhydrous sodium sulfate and concentrated under reduced pressure. The residue was purified by column chromatography (diol column, hexane: ethyl acetate = 2:1 - 1:1) to obtain 40 mg of 3-(4-(cyclohexylmethoxyphenyl)phenyl)-2(S)-(2(R,S)-(tert-butyl)-5-phenylpentanoylamino)propionic acid (85) (yield: 52%).
LR-MS(m/z):494$(M+H)^+$

Example 86

Methyl 3-(4-(cyclohexylmethoxy)phenyl)-2(S)-(2(R,S)-cyclohexyl-5-phenylpentanoylamino)propionate (86)

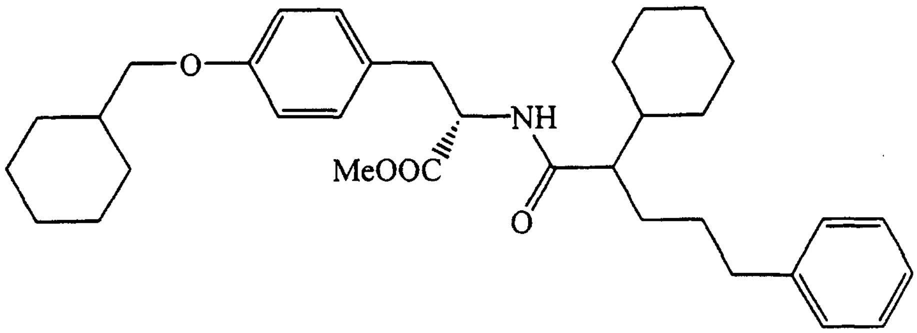

(86)

In 1 ml of DMF, 87 mg (333 μmol) of 2-cyclohexyl-5-phenylvaleric acid and 100 mg (0.34mmol) of methyl (S)-2-amino-3-(4-(cyclohexylmethoxy)phenyl)propionate (83) were dissolved, and 0.09ml (0.42mmol) of DPPA and 0.12ml (0.86mmol) of triethylamine were added at 0°C, followed by stirring the mixture at room temperature for 14 hours. To the reaction solution, 1N hydrochloric acid was added and the mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 9:1 - 7:1 - 5:1 - 3:1) to obtain 134 mg of methyl 3-(4-(cyclohexylmethoxy)phenyl)-2(S) -(2(R,S)-cyclohexyl-5-phenylpentanoylamino)propionate (86) (yield: 75%).
LR-MS(m/z):533($M^+$)
$^1$H-NMR(300MHz, $CDCl_3$, δppm) : 0.86-1.36, 1.47-1.87(m, 26H), 2.51-2.67(m, 2H), 3.01(dd, J=5.8, 14.8Hz, 1H), 3.08 (dd, J=5.5, 14.8Hz, 1H), 3.67-3.71(m, 5H), 4.03(brd, J=9.3Hz, 1H), 4.59-4.62(m, 1H), 4.69-4.75(m, 1H), 6.73-6.78(m, 2H), 6.95-7.00(m, 2H), 7.14-7.18(m, 3H), 7.23-7.30(m, 2H).

Example 87

3-(4-(cyclohexylmethoxy)phenyl)-2(S)-(2(R,S)-cyclohexyl-5-phenylpentanoylamino)propionic acid (87)

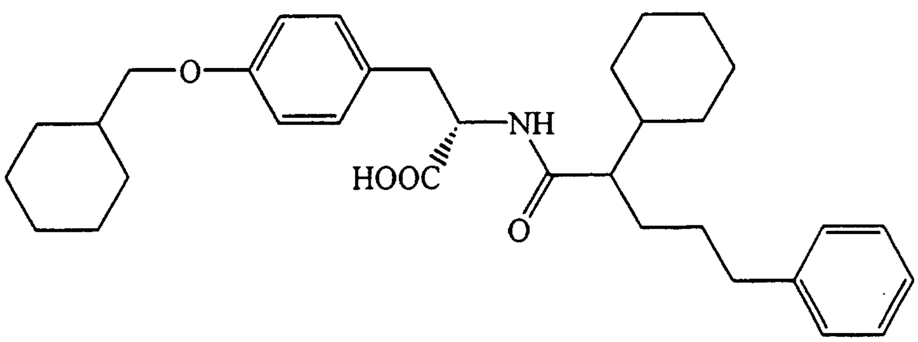

(87)

In 3 ml of methanol, 97 mg (0.16mmol) of methyl 3-(4-(cyclohexylmethoxy)phenyl)-2(S)-(2(R,S)-cyclohexyl-5-phenylpentanoylamino)propionate (86) was dissolved, and 0.4ml (0.37mmol) of 1N aqueous sodium hydroxide solution was added, followed by stirring the mixture at room temperature for-16 hours. To the reaction solution, 1N hydrochloric acid was added and the mixture was extracted with ethyl acetate. Organic layers were combined, washed with saturated brine, dried over anhydrous sodium sulfate and concentrated under reduced pressure. The residue was purified by column chromatography (diol column, hexane: ethyl acetate = 2:1 -1:1) to obtain 22 mg of 3-(4-(cyclohexylmethoxy)phenyl)-2(S)-(2(R,S)-cyclohexyl-5-phenylpentanoylamino)propionic acid (87) (yield: 23%).

LR-MS(m/z):520(M+H)+
$^1$H-NMR(300MHz, $CDCl_3$, δppm) : 0.76-1.35, 1.40-1.87(m, 26H), 2.49-2.66(m, 2H), 2.99-3.07(m, 1H), 3.10-3.18(m, 1H), 3.64-3.69(m, 2H), 4.49(brs, 1H), 4.90(brs, 1H), 6.73-6.77(m, 2H), 7.04-7.28(m, 7H).

Example 88

Methyl 3-(4-(cyclohexylmethoxy)phenyl)-2(S)-(2(S)-((fluoren-9-ylmethoxy)carbonylamino)-3,3-dimethylbutanoylamino)propionate (88)

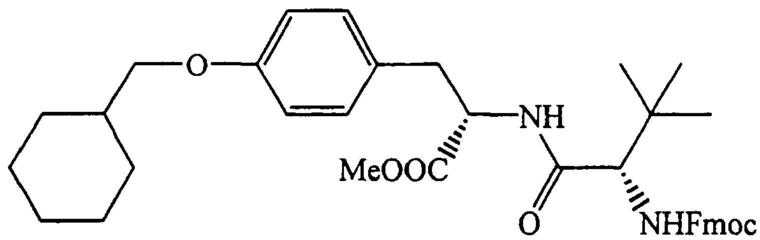

(88)

In 1.5 ml of DMF, 87 mg (0.33mmol) of 2-cyclohexyl-5-phenylvaleric acid, 214 mg (734 μmol) of methyl (S)-2-amino-3-(4-(cyclohexylmethoxy)phenyl)propionate (83) and 260 mg (0.74mmol) of N-Fmoc-L-leucine were dissolved, and 468 mg (0.9mmol) of PyBOP and 0.11ml (1mmol) of N-methylmorpholine were added, followed by stirring the mixture at room temperature for 1 hour. The reaction solution was acidified with 1N hydrochloric acid and the mixture was extracted with ethyl acetate. Organic layers were combined, washed with water, saturated aqueous sodium hydrogen carbonate solution and saturated brine, dried over anhydrous sodium sulfate and concentrated under reduced pressure. The residue was purified by column chromatography (hexane: ethyl acetate = 9:1 - 5:1 - 3:1) to obtain 440 mg of methyl 3-(4-(cyclohexylmethoxy)phenyl)-2(S)-(2(S)-((fluoren-9-ylmethoxy)carbonylamino)-3,3-dimethylbutanoylamino)propionate (88) (yield: 95%).
LR-MS(m/z):627(M+H)+
$^1$H-NMR(300MHz, $CDCl_3$, δppm): 0.78-1.82(12H, m), 0.99(9H, s), 3.05(2H, d, J=6.0Hz), 3.62-3.73(2H, m), 3.73(3H, s), 3.91(1H, d, J=9.5Hz), 4.21-4.46(3H, m), 4.79-4.85(1H, m), 5.56(1H, d, J=9.5Hz), 6.05(1H, d, J=7.5Hz), 6.76(2H, d, J=8.5Hz), 6.96(2H, d, J=8.5Hz), 7.28-7.78(8H, m)

Example 89

Synthesis of methyl 2(S)-(2(S)-amino-3,3-dimethylbutanoylamino)3-(4-(cyclohexylmethoxy)phenyl)propionate (89)

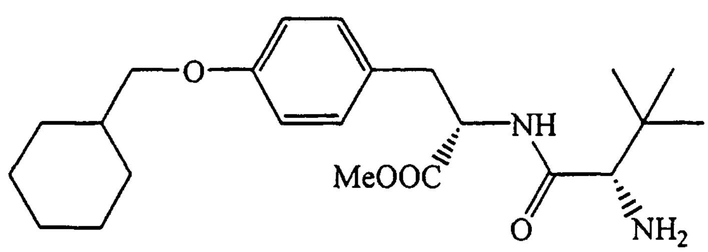

(89)

In 2 ml of DMF, 440 mg (0.7mmol) of methyl 3-(4-(cyclohexylmethoxy)phenyl)-2(S)-(2(S)-((fluoren-9-ylmethoxy)carbonylamino)-3,3-dimethylbutanoylamino)propionate (88) was dissolved and 0.1ml (1.01 mmol) of piperidine was added, followed by stirring the mixture at room temperature for 20 minutes. Water was added to the reaction solution and the mixture was extracted with ethyl acetate. Organic layers were combined, washed with saturated brine, dried over anhydrous sodium sulfate and concentrated under reduced pressure. The residue was purified by column chromatography (hexane: ethyl acetate = 2:1 - 1:1 - ethyl acetate) to obtain 218 mg of methyl 2(S)-(2(S)-amino-3,3-dimethylbutanoylamino)3-(4-(cyclohexylmethoxy)phenyl)propionate (89) (yield: 76%).
LR-MS(m/z):404(M+)

$^1H$-NMR(300MHz, $CDCl_3$, δppm) : 0.92(9H, s), 0.96-1.88(11H, m), 2.97-3.09(3H, m), 3.714(2H, d, J=6.5Hz), 3.715 (3H, s), 4.79-4.86(1H, m), 6.80(2H, d, J=8.5Hz), 7.03(2H, d, J=8.5Hz), 7.10(1H, d, J=7.5Hz)

Example 90

Methyl 3-(4-(cyclohexyimethoxy)phenyl)-2(S)-(3,3-dimethyl-2(S)-(2-phenylacetylamino)butanoylamino)propionate (90)

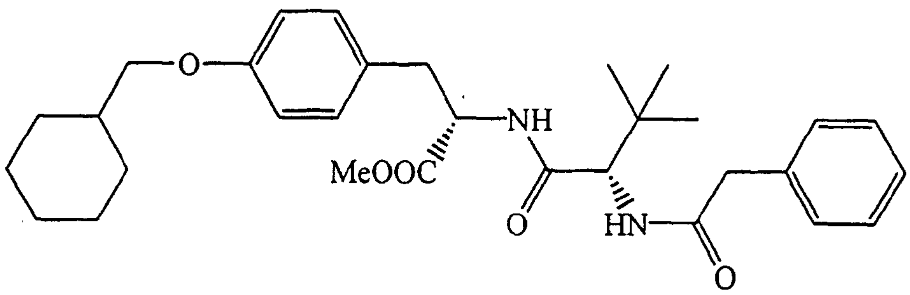

(90)

In 2 ml of pyridine, 185 mg (0.46mmol) of methyl 2(S)-(2(S)-amino-3,3-dimethylbutanoylamino)3-(4-(cyclohexylmethoxy)phenyl)propionate (89) was dissolved, and 0.07ml (0.53mmol) of benzoyl chloride was added, followed by stirring the mixture at room temperature for 10 minutes. To the reaction solution, 1N hydrochloric acid was added and the mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 5:1 - 4:1 - 3:1 - 2:1 -1:1) to obtain 166 mg of methyl 3-(4-(cyclohexylmethoxy)phenyl)-2(S)-(3,3-dimethyl-2(S)-(2-phenylacetylamino)butanoylamino)propionate (90) (yield: 69%).
LR-MS(m/z):522($M^+$)
$^1H$-NMR(300MHz, $CDCl_3$, δppm) : 0.84(9H, s), 0.97-1.87(11H, m), 2.95-3.06(2H, m), 3.55(1H, d, J=15.5Hz), 3.63(1H, d, J=15.5Hz), 3.706(3H, s), 3.708(2H, d, J=6.0Hz), 4.19(1H, d, J=9.0Hz), 4.72-4.79(1H, m), 6.06(1H, d, J=9.0Hz), 6.12 (1H, d, J=7.5Hz), 6.79(2H, d, J=8.5Hz), 6.96(2H, d, J=8.5Hz), 7.28-7.38(5H, m)

Example 91

3-(4-(cyclohexylmethoxy)phenyl)-2(S)-(3,3-dimethyl-2(S)-(2-phenylacetylamino)butanoylamino)propionic acid (91)

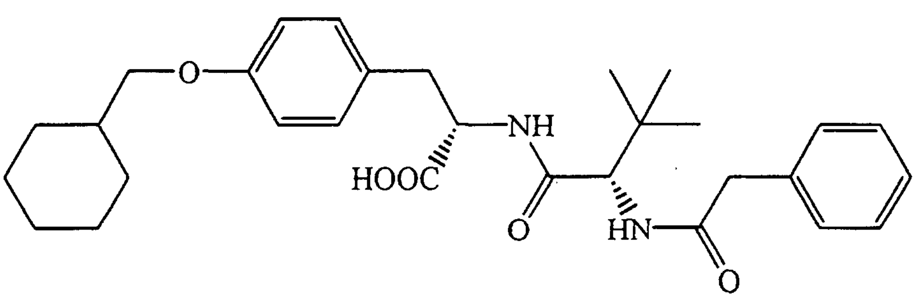

(91)

In 4 ml of methanol, 133 mg (0.25mmol) of methyl 3-(4-(cyclohexylmethoxy)phenyl)-2(S)-(3,3-dimethyl-2(S) -(2-phenylacetylamino)butanoylamino)propionate (90) was dissolved, and 0.35ml (0.29mmol) of 1N aqueous sodium hydroxide solution was added, followed by stirring the mixture at room temperature for 15 hours. Water and ether were added to the reaction solution, and the aqueous layer, after separation, was acidified with 1N hydrochloric acid, followed by extraction of the mixture with ethyl acetate. Organic layers were combined, washed with saturated brine, dried over anhydrous sodium sulfate and concentrated under reduced pressure. The residue was purified by column chromatography (diol column, hexane: ethyl acetate = 2:1 - 1:1 - 1:2) to obtain 25 mg of 3-(4-(cyclohexylmethoxy)phenyl)-2(S)-(3,3-dimethyl-2(S)-(2-phenylacetylamino)butanoylamino)propionic acid (91) (yield: 20%).
LR-MS(m/z):509$(M+H)^+$
$^1$H-NMR(300MHz, $CDCl_3$, δppm) : 0.81(9H, s), 0.95-1.82(11H, m), 2.75(1H, dd, J=6.5, 14.0Hz), 3.07(1H, dd, J=5.0, 14.0Hz), 3.50-3.64(3H, m), 4.41(1H, d, J=9.5Hz),4.73-4.80(1H, m), 6.50(1H, brs), 6.69(2H, d, J=8.5Hz), 6.80(1H, d, J=8.0Hz), 6.95(2H, d, J=8.5Hz), 7.25-7.37(5H, m)

Example 92

Methyl 3-(4-(cyclohexylmethoxy)phenyl)-2(S)-(3,3-dimethyl-2(R,S)-benzylbutanoylamino)propionate (92)

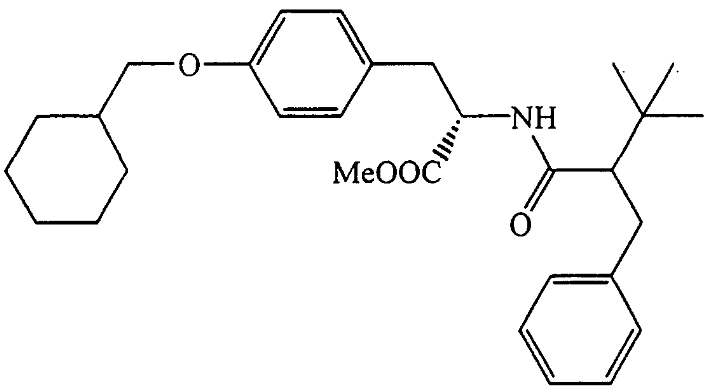

(92)

In 1 ml of DMF, 87 mg (0.33mmol) of 2-cyclohexyl-5-phenylvaleric acid, 107 mg (0.37mmol) of methyl (S)-2-amino-3-(4-(cyclohexylmethoxy)phenyl)propionate (83) and 76 mg (0.37mmol) of 3,3-dimethyl-2-valeric acid were dissolved, and 0.095ml (0.44mmol) of DPPA and 0.13ml (0.93mmol) of triethylamine were added at 0°C, followed by stirring the mixture at room temperature for 31 hours. To the reaction solution, 1N hydrochloric acid was added and the mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 9:1 - 5:1 - 3:1) to obtain 145 mg of methyl 3-(4-(cyclohexylmethoxy)phenyl)-2(S)-(3,3-dimethyl-2(R,S)-benzylbutanoylamino)propionate (92) (yield: 82%).
LR-MS(m/z):479$(M^+)$
$^1$H-NMR(300MHz, $CDCl_3$, δppm) : 0.98(s, 9H), 0.96-1.08, 1.16-1.37, 1.68-1.88(m, 11H), 2.30-2.39(m, 1H), 2.78-2.88 (m, 2H), 3.00-3.11(m, 1H), 3.63, 3.65(s, 3H), 3.64(d, J=5.5Hz, 2H), 4.02-4.18(m, 1H), 4.43-4.59(m, 1H), 6.64-6.77(m, 2H), 6.83-6.89(m, 1H), 7.16-7.32(m, 7H).

Example 93

3-(4-(cyclohexylmethoxy)phenyl)-2(S)-(3,3-dimethyl-2(R,S)-benzylbutanoylamino)propionic acid (93)

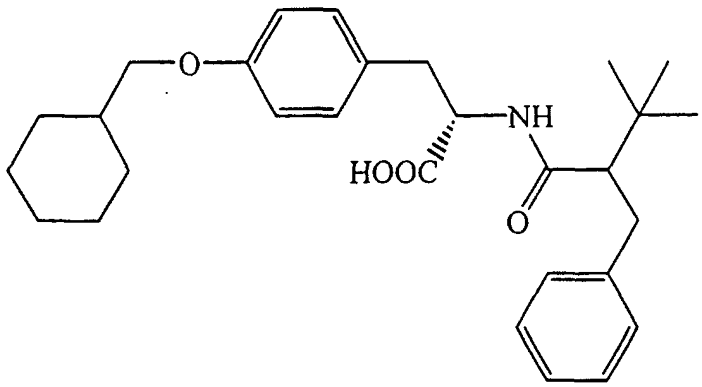

(93)

In 2 ml of methanol, 135 mg (0.28mmol) of 3-(4-(cyclohexylmethoxy)phenyl)-2(S)-(3,3-dimethyl-2(S)-(2-phenylacetylamino)butanoylamino)propionic acid (91) was dissolved, and 0.31ml (0.29mmol) of 1N aqueous sodium hydroxide solution was added, followed by stirring the mixture at room temperature for 9 hours. To the reaction solution, 1N hydrochloric acid was added to acidify the solution, and the mixture was extracted with ethyl acetate. Organic layers were combined, washed with saturated brine, dried over anhydrous sodium sulfate and concentrated under reduced pressure. The residue was purified by column chromatography (diol column, hexane: ethyl acetate = 2:1 - 1:1 - 1:2) to obtain 79 mg of 3-(4-(cyclohexylmethoxy)phenyl)-2(S)-(3,3-dimethyl-2(R,S)-benzylbutanoylamino)propionic acid (93) (yield: 56%).
LR-MS(m/z):466(M+H)$^+$
$^1$H-NMR(300MHz, $CDCl_3$, δppm) : 0.81(9H, s), 0.95-1.82(11H, m), 2.75(1H, dd, J=6.5, 14.0Hz), 3.07(1H, dd, J=5.0, 14.0Hz), 3.50-3.64(3H, m), 4.41(1H, d, J=9.5Hz),4.73-4.80(1H, m), 6.50(1H, brs), 6.69(2H, d, J=8.5Hz), 6.80(1H, d, J=8.0Hz), 6.95(2H, d, J=8.5Hz), 7.25-7.37(5H, m)

Example 94

Methyl 3-(4-(cyclohexylmethoxy)phenyl)-2(S)-((2-(2-phenylethyl)phenyl)carbonylamino)propionate (94)

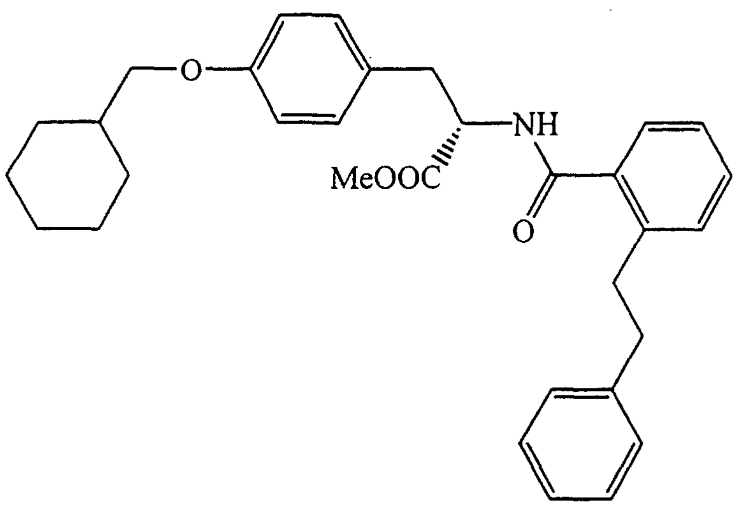

(94)

In 1 ml of DMF, 87 mg (0.33mmol) of 2-cyclohexyl-5-phenylvaleric acid, 99 mg (0.34mmol) of methyl (S)-2-amino-3-(4-(cyclohexylmethoxy)phenyl)propionate (83) and 77 mg (0.34mmol) of 4-phenethyl benzoic acid were dissolved, and 0.09ml (0.42mmol) of DPPA and 0.12ml (0.86mmol) of triethylamine were added at 0°C, followed by stirring the mixture at room temperature for 21 hours. To the reaction solution, 1N hydrochloric acid was added to acidify the solution, and the mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 5:1 - 4:1 - 2:1) to obtain 80 mg of methyl 3-(4-(cyclohexylmethoxy)phenyl)-2(S)-((2-(2-phenylethyl)phenyl)carbonylarnino)propionate (94) (yield: 48%).
LR-MS(m/z):499($M^+$)
$^1$H-NMR(300MHz, $CDCl_3$, δppm) : 0.95-1.07(m, 2H), 1.15-1.35(m, 3H), 1.63-1.86(m, 6H), 2.84-2.90(m, 2H), 3.03-3.09 (m, 2H), 3.11(dd, J=6.0, 14.0Hz, 1H), 3.23(dd, J=5.6, 14.0Hz, 1H), 3.66(d, J=6.3Hz, 2H), 3.75(s, 3H), 4.99-5.06(m, 1H), 6.14(d, J=7.7Hz, 1H), 6.76-6.81(m, 2H), 7.01-7.06(m, 2H), 7.15-7.35(m, 5H).

Example 95

3-(4-(cyclohexylmethoxy)phenyl)-2(S)-((2-(2-phenylethyl)phenyl)carbonylamino)propionic acid (95)

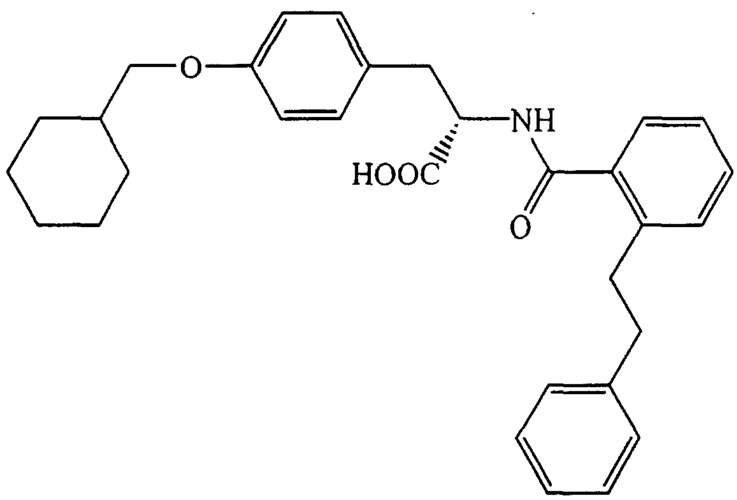

(95)

In 1 ml of THF, 60 mg (0.12mmol) of methyl 3-(4-(cyclohexylmethoxy)phenyl)-2(S)-((2-(2-phenylethyl)phenyl) carbonylamino)propionate (94) was dissolved, and 0.13ml (0.12mmol) of 1N aqueous sodium hydroxide solution was added, followed by stirring the mixture at room temperature for 5 hours. To the reaction solution, 1N hydrochloric acid was added to acidify the solution, and the mixture was extracted with ethyl acetate. Organic layers were combined, washed with saturated brine, dried over anhydrous sodium sulfate and concentrated under reduced pressure. The residue was recrystallized from THF/ether to obtain 40 mg of 3-(4-(cyclohexylmethoxy)phenyl)-2(S)-((2-(2-phenylethyl) phenyl)carbonylamino)propionic acid (95) (yield: 66%).
LR-MS(m/z):486$(M+H)^+$
IR(KBr):3300-2500, 3293, 2922, 2855, 1711, 1249$cm^{-1}$
$^1$H-NMR(300MHz, $CDCl_3$, δppm) : 0.95-1.07(m, 2H), 1.16-1.35(m, 3H), 1.73-1.86(m, 6H), 2.85-2.90(m, 2H), 3.01-3.13 (m, 2H), 3.15(dd, J=6.3, 14.0Hz, 1H), 3.31(dd, J=5.2, 14.0Hz, 1H), 3.66(d, J=6.3Hz, 2H), 4.94-5.00(m, 1H), 6.30(d, J=7.7Hz, 1H), 6.75-6.78(m, 2H), 7.10-7.33(m, 7H).

Example 96

Methyl 3-(4-(cyclohexylmethoxy)phenyl)-2(S)-(3,3-dimethyl-2(S)-(phenylmethoxy)butanoylamino)propionate (96)

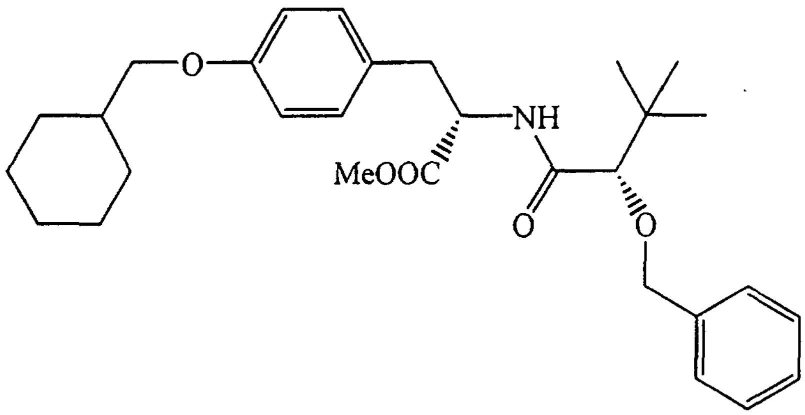

(96)

In 1 ml of DMF, 53 mg (182 μmol) of methyl (S)-2-amino-3-(4-(cyclohexylmethoxy)phenyl)propionate (83) and 41 mg (0.18mmol) of (S)-3,3-dimethyl-2-(phenylmethoxy)butyric acid were dissolved, and 105 mg (0.2mmol) of PyBOP and 0.025ml (0.23mmol) of N-methylmorpholine were added, followed by stirring the mixture at room temperature for 1 hour. To the reaction solution, 1N hydrochloric acid was added to acidify the solution, and the mixture was extracted with ethyl acetate. Organic layers were combined, washed with saturated brine, dried over anhydrous sodium sulfate and concentrated under reduced pressure. The residue was purified by column chromatography (hexane: ethyl acetate = 9:1 - 5:1 - 3:1) to obtain 79 mg of methyl 3-(4-(cyclohexylmethoxy)phenyl)-2(S)-(3,3-dimethyl-2(S)-(phenylmethoxy) butanoylamino)propionate (96) (yield: 88%).
LR-MS(m/z):495($M^+$)
$^1$H-NMR(300MHz, $CDCl_3$, δppm) : 0.89, 0.98(s, 9H), 1.02-1.18, 1.21-1.32, 1.71-1.88(m, 11H), 2.92-3.20(m, 2H), 3.41, 3.43(s, 1H), 3.63, 3.69(d, J=6.3Hz, 2H), 3.73, 3.74(s, 3H), 4.14, 4.30(d, J=10.8Hz, 1H), 4.29, 4.60(d, J=10.8Hz, 1H), 4.86-4.93(m, 1H), 6.67-6.86(m, 3H), 7.00-7.03(m, 2H), 7.16-7.34(m, 5H).
$[\alpha]^{20}_D$: -51.0° (c=0.592, $CHCl_3$)

Example 97

Synthesis of 3-(4-(cyclohexylmethoxy)phenyl)-2(S)-(3,3-dimethyl-2(S)-(phenylmethoxy)butanoylamino)propionic acid (97)

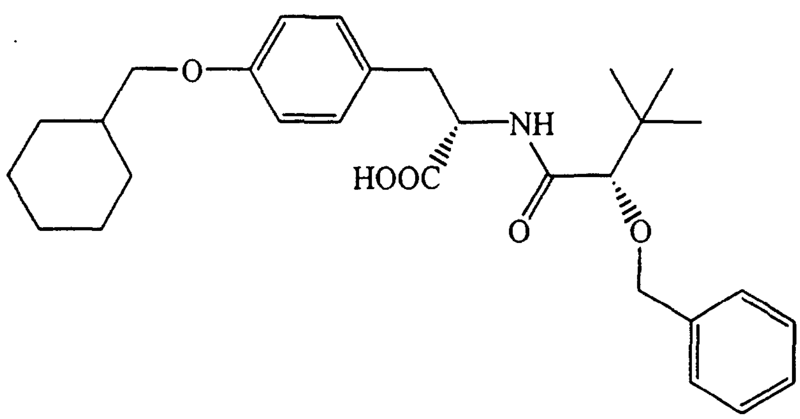

(97)

In 1 ml of methanol, 43 mg (0.09mmol) of methyl 3-(4-(cyclohexylmethoxy)phenyl)-2(S)-(3,3-dimethyl-2(S)-(phenylmethoxy)butanoylamino)propionate (96) was dissolved, and 0.1ml (0.1mmol) of 1N aqueous sodium hydroxide solution was added, followed by stirring the mixture at room temperature for 9 hours. To the reaction solution, 1N hydrochloric acid was added to acidify the solution, and the mixture was extracted with ethyl acetate. Organic layers were combined, washed with saturated brine, dried over anhydrous sodium sulfate and concentrated under reduced pressure. The residue was purified by column chromatography (diol column, hexane: ethyl acetate = 2:1) to obtain 36 mg of 3-(4-(cyclohexylmethoxy)phenyl)-2(S)-(3,3-dimethyl-2(S)-(phenylmethoxy)butanoylamino)propionic acid (97) (yield: 87%).
LR-MS(m/z):482$(M+H)^+$
IR(KBr):3300-2500, 2926, 2853, 1736, 1246 $cm^{-1}$
$^1$H-NMR(300MHz, $CDCl_3$, δppm) : 0.86, 0.96(s, 9H), 1.01-1.17, 1.23-1.36, 1.65-1.87(m, 11H), 2.96-3.28(m, 2H), 3.43, 3.46(s, 1H), 3.62, 3.68(d, J=6.3Hz, 2H), 4.14, 4.28(d, J=11.0Hz, 1H), 4.23, 4.53(d, J=11.0Hz, 1H), 4.84-4.91(m, 1H), 6.68, 6.78(d, J=8.5Hz, 2H), 6.88(d, J=8.2Hz, 1H), 7.01-7.33(m, 7H).
$[\alpha]^{25}_D$:-106.85° (c=0.39, MeOH)

Example 98

Methyl (S)-2-amino-3-(4-(2,2-dimethylpropoxy)phenyl)propionate (98)

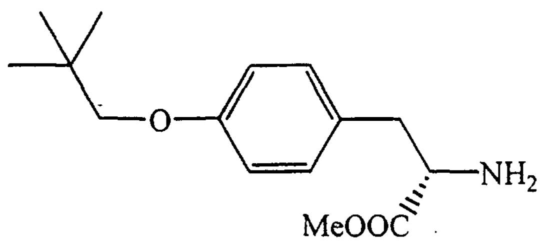

(98)

To 0.82 g of N-Boc-L-tyrosine methyl ester, 0.35 g of potassium tert-butoxide was added, and the mixture was dissolved in 14 ml of DMSO. To the mixture, 0.40 g of 1-bromo-3,3-dimethylpropane was added and the resulting mixture was stirred at room temperature for 1 hour and then at 80°C for 19 hours. Water was added to the reaction solution and the mixture was extracted with ethyl acetate. The organic layer was washed with saturated brine and dried over anhydrous sodium sulfate, followed by concentrating the mixture. The residue was purified by silica gel column chromatography (hexane: ethyl acetate = 7:1) to obtain 192 mg of methyl (S)-3-(4-(2,2-diemthylpropoxy)phenyl)-2-((tert-butoxy)carbonylamino)propionate.

Then 0.17 g of methyl (S)-3-(4-(2,2-dimethylpropoxy)phenyl)-2-((tert-butoxy)carbonylamino)propionate was dissolved in 5 ml of methylene chloride, and 10.5 ml of trifluoroacetic acid was added thereto, followed by stirring the mixture for 8 hours. Sodium hydrogen carbonate powder and saturated aqueous sodium hydrogen carbonate solution were added to the reaction solution, and the mixture was extracted with ethyl acetate. Organic layers were combined, washed with saturated brine, dried over sodium sulfate and concentrated to obtain methyl (S)-2-amino-3-(4-(2,2-dimethylpropoxy)phenyl)propionate (98). The product (98) was used in the next reaction without purification.

Example 99

Methyl 2(S)-(3,3-dimethyl-2(S)-(phenylmethoxy)butanoylamino)-3-(4-(2,2-dimethylpropoxy)phenyl)propionate (99)

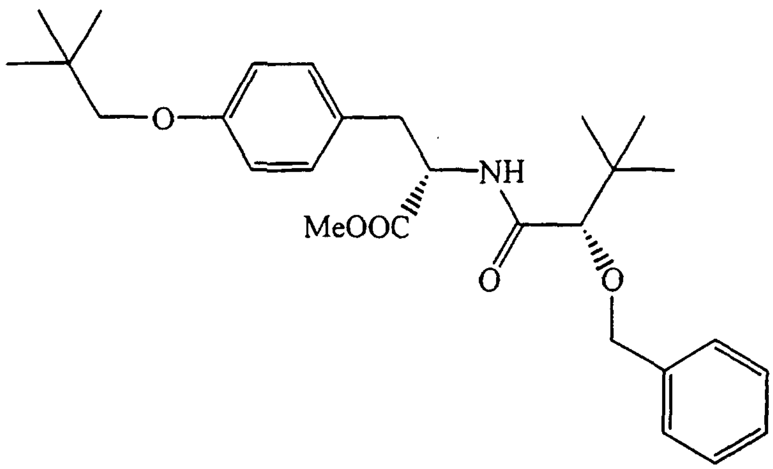

(99)

In 1 ml of DMF, 57 mg (0.22mmo1) of methyl (S)-2-amino-3-(4-(2,2-dimethylpropoxy)phenyl)propionate (98) and 49 mg (0.22mmol) of (S)-3,3-dimethyl-2-(phenylmethoxy)butyric acid were dissolved, and 125 mg (0.24mmol) of PyBOP and 0.03ml (0.27mmol) of N-methylmorpholine were added, followed by stirring the mixture at room temperature for 1.5 hours. To the reaction solution, 1N hydrochloric acid was added to acidify the solution, and the mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 9:1 - 5:1) to obtain 64 mg of methyl 2 (S)-(3,3-dimethyl-2(S)-(phenylmethoxy)butanoylamino)-3-(4-(2,2-dimethylpropoxy)phenyl)propionate (99) (yield: 64%).

LR-MS(m/z):469($M^+$)

$^1$H-NMR(300MHz, $CDCl_3$, δppm) : 0.90, 0.98, 1.01, 1.03(s, 18H), 2.93-3.20(m, 2H), 3.41, 3.44(s, 1H), 3.47, 3.53(s, 2H), 3.72, 3.75(s, 3H), 4.15(d, J=12.0Hz, 0.5H), 4.29(d, J=11.0Hz, 0.5H), 4.30(d, J=12.0Hz, 0.5H), 4.60(d, J=11.0Hz, 0.5H), 4.87-4.94(m, 1H), 6.67-7.37(m, 10H).

$[\alpha]^{20}_D$: -46.9° (c=1.286, $CHCl_3$)

Example 100

2(S)-(3,3-dimethyl-2(S)-(phenylmethoxy)butanoylamino)-3-(4-(2,2-dimethylpropoxy)phenyl)propionic acid (100)

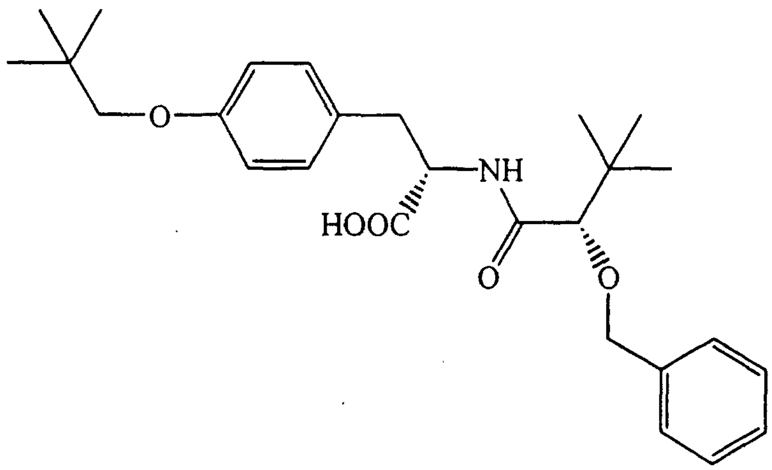

(100)

In 1 ml of methanol, 62 mg (0.13mmol) of methyl 2(S)-(3,3-dimethyl-2(S)-(phenylmethoxy)butanoylamino)-3-(4-(2,2-dimethylpropoxy)phenyl)propionate (99) was dissolved, and 0.17ml (0.17mmol) of 1N aqueous sodium hydroxide solution was added, followed by stirring the mixture at room temperature for 16 hours. To the reaction solution, 1N hydrochloric acid was added to acidify the solution, and the mixture was extracted with ethyl acetate. Organic layers were combined, washed with saturated brine, dried over anhydrous sodium sulfate and concentrated under reduced pressure. The residue was purified by column chromatography (diol column, hexane: ethyl acetate = 2:1) to obtain 51 mg of 2(S)-(3,3-dimethyl-2(S)-(phenylmethoxy)butanoylamino)-3-(4-(2,2-dimethylpropoxy)phenyl)propionic acid (100) (yield: 87%).
LR-MS(m/z):455($M^+$)
IR(KBr):3300-2500, 2956, 1737, 1246$cm^{-1}$
$^1$H-NMR(300MHz, $CDCl_3$, δ ppm) : 0.88, 0.97, 1.00, 1.03(s, 18H), 2.97-3.29(m, 2H), 3.44, 3.47(s, 1H), 3.46, 3.53(s, 2H), 4.12, 4.26(d, J=12.6Hz, 1H), 4.28, 4.54(d, 3=11.0Hz, 1H), 4.86-4.92(m, 1H), 6.68-7.36(m, 10H).
$[\alpha]^{25}_D$:-80.23° (c=0.34, MeOH)

Example 101

Methyl 2(S)-(4-methyl-2(S)-(phenylmethoxy)pentanoylamino)-3-(4-(2,2-dimethylpropoxy)phenyl)propionate (101)

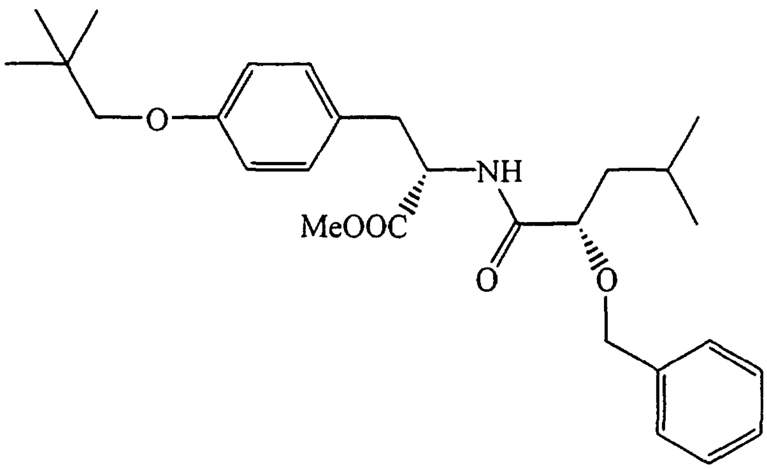

(101)

In 1 ml of DMF, 31 mg (116 μmol) of methyl (S)-2-amino-3-(4-(2,2-dimethylpropoxy)phenyl)propionate (98) and 26 mg (0.12mmol) of (S)-4-methyl-2-(phenylmethoxy)valeric acid were dissolved, and 66 mg (0.13mmol) of PyBOP and 0.016ml (0.15mmol) of N-methylmorpholine were added, followed by stirring the mixture at room temperature for 2 hours. To the reaction solution, 1N hydrochloric acid was added to acidify the solution, and the mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 9:1 - 5:1) to obtain 42 mg of methyl 2(S)-(4-methyl-2(S)-(phenylmethoxy)pentanoylamino)-3-(4-(2,2-dimethylpropoxy)phenyl)propionate (101) (yield: 77%).
LR-MS(m/z):469($M^+$)
$^1$H-NMR(300MHz, $CDCl_3$, δppm) : 0.81, 0.82, 0.90 (6H, d, J = 6.6Hz), 1.01, 1.03 (9H, s), 1.47 (1H, m), 1.61 (1H, m), 1.78 (1H, m), 2.99-3.19 (2H, m), 3.45-3.53 (2H, m), 3.74, 3.75 (3H, s), 3.83 (1H, m), 4.25, 4.34 (1H, d, J = 11.3Hz), 4.38,4.58 (1H, d, J = 11.3Hz), 4.88 (1H, m), 6.70-6.81 (2H, m), 6.90 (1H, d, J = 8.2Hz), 6.96-7.02 (2H, m), 7.16-7.20 (2H, m), 7.27-7.37 (3H, m).
$[\alpha]^{17}_D$:-30.1° (c=0.836, $CHCl_3$)

Example 102

2(S)-(4-methyl-2(S)-(phenylmethoxy)pentanoylamino)-3-(4-(2,2-dimethylpropoxy)phenyl)propionic acid (102)

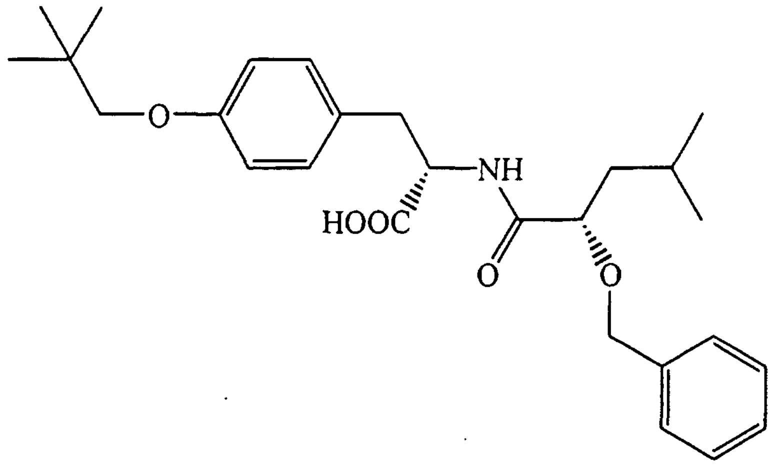

(102)

In 1 ml of methanol, 39 mg (0.09mmol) of methyl 2(S)-(4-methyl-2(S)-(phenylmethoxy)pentanoylamino)-3-(4-(2,2-dimethylpropoxy)phenyl)propionate (101) was dissolved, and 0.12ml (0.11mmol) of 1N aqueous sodium hydroxide solution was added, followed by stirring the mixture at room temperature for 23 hours. To the reaction solution, 1N hydrochloric acid was added to acidify the solution, and the mixture was extracted with ethyl acetate. Organic layers were combined, washed with saturated brine, dried over anhydrous sodium sulfate and concentrated under reduced pressure. The residue was purified by column chromatography (diol Lobar column, hexane: ethyl acetate = 2:1) to obtain 37 mg of 2(S)-(4-methyl-2(S)-(phenylmethoxy)pentanoylamino)-3-(4-(2,2-dimethylpropoxy)phenyl)propionic acid (102) (yield: 80%).
LR-MS(m/z):455($M^+$)
IR(KBr):3300-2500, 2956, 1736, 1245, 849$cm^{-1}$
$^1$H-NMR(300MHz, $CDCl_3$, δppm) : 0.98(9H,s), 3.72(1H,s), 4.82-4.88(1H,.m), 5.16-5.20(1H, m), 7.42-7.59(4H, m), 7.80-7.90(2H, m), 8.14(1H, d, J=8.0Hz),0.80, 0.81, 0.88, 0.89 (6H, d, J = 6.6Hz), 1.00, 1.02 (9H, s), 1.45 (1H, m), 1.59 (1H, m), 1.76 (1H, m), 3.02-3.28 (2H, m), 3.45-3.53 (2H, m), 3.85 (1H, m), 4.21, 4.34 (1H, d, J = 11.0Hz), 4.34, 4.53 (1H, d, J = 11.0Hz), 4.86 (1H, m), 6.73-6.82 (2H, m), 6.95 (1H, d, J = 8.0Hz), 6.99-7.06 (2H, m), 7.14-7.23 (2H, m), 7.27-7.36 (3H, m).
$[\alpha]^{25}_D$:-26-08° (c=0.23, MeOH)

Example 103

Methyl (S)-3-(4-(2-methylpropoxy)phenyl)-2-((tert-butoxy)carbonylamino)propionate (103)

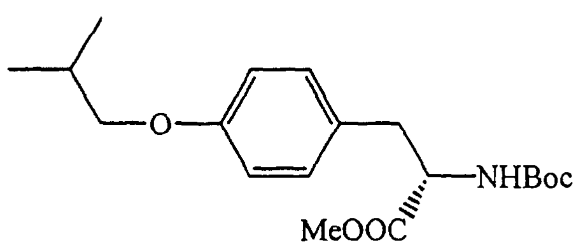

(103)

To 1.0 g of N-Boc-L-tyrosine methyl ester, 0.42 g of potassium tert-butoxide was added, and the mixture was dissolved in 35 ml of DMSO. To the mixture, 0.41 g of isobutyl bromide was added and the resulting mixture was stirred at room temperature for 1 hour and then at 80°C for 30 hours. Water was added to the reaction solution and the mixture

was once extracted with ethyl acetate. The organic layer was washed with saturated brine and dried over anhydrous sodium sulfate, followed by concentrating the mixture. The residue was purified by silica gel column chromatography (hexane: ethyl acetate = 5:1) to obtain 317 mg of methyl (S)-3-(4-(2-methylpropoxy)phenyl)-2-((tert-butoxy)carbonylamino)propionate (103) (yield: 17%).

LR-MS(m/z):351($M^+$)

IR(neat):3370, 2962, 1742, 1707, 1516, 1369, 1299, 1241, 1224, 1180, 1033, 830, 801, 667, 555, 536, 507, 491$cm^{-1}$

$^1$H-NMR(300MHz, $CDCl_3$, δppm) : 7.02(2H, d, J=8.7Hz), 6.81(2H, d, J=8.7Hz), 4.95(1H, br d, J=8.7Hz), 5.24(1H, br q, J=8.7Hz), 3.71(3H, s), 3.68(2H, d, J=6.6Hz), 3.0-3.1(2H, m), 2.0-2.1(1H, m), 1.42(9H, s), 1.01(6H, d, J=6.3Hz)

$[\alpha]^{27}_D$: +2.88(c=1.018,$CHCl_3$)

Example 104

Methyl (S)-2-amino-3-(4-(2-methylpropoxy)phenyl)propionate (104)

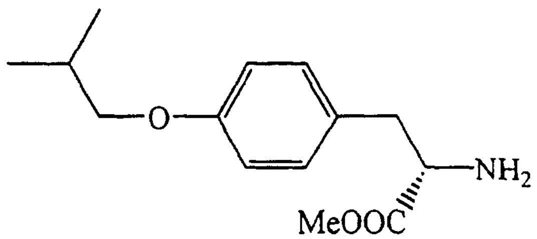

(104)

In 10 ml of methylene chloride, 250 mg of methyl (S)-3-(4-(2-methylpropoxy)phenyl)-2-((tert-butoxy)carbonylamino)propionate (103) was dissolved and 1.38 ml of trifluoroacetic acid was added, followed by stirring the mixture at room temperature for 6.5 hours. Saturated aqueous sodium hydrogen carbonate solution was added to the reaction solution and the mixture was extracted with ethyl acetate. Organic layers were combined, washed with saturated brine, dried over anhydrous sodium sulfate and concentrated to obtain 186 mg of methyl (S)-2-amino-3-(4-(2-methylpropoxy) phenyl)propionate (104). The product (104) was used in the next reaction without purification.

Example 105

Methyl 2(S)-(2(S)-((2-naphthyl)methoxy)-3,3-dimethylbutanoylamino)-3-(4-(2-methylpropoxy)phenyl)propionate (105)

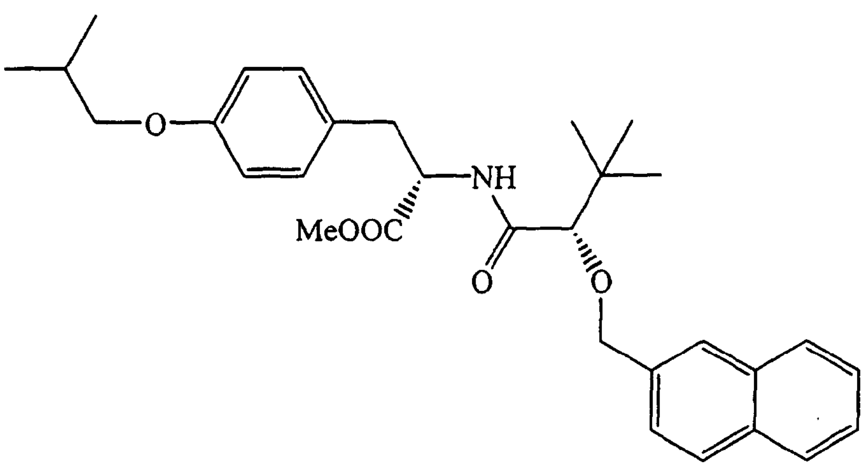

(105)

In 1 ml of DMF, 34 mg (0.14mmol) of methyl (S)-2-amino-3-(4-(2-methylpropoxy)phenyl)propionate (104) and 38 mg (0.14mmol) of 2(S)-((2-naphthyl)methoxy)-3,3-dimethylbutyric acid were dissolved, and 80 mg (0.15mmol) of PyBOP and 0.02ml (0.18mmol) of N-methylmorpholine were added, followed by stirring the mixture at room temperature for 30 minutes. To the reaction solution, 1N hydrochloric acid was added to acidify the solution, and the mixture was

extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 9:1 - 5:1) to obtain 60 mg of methyl 2 (S)-(2(S)-((2-naphthyl)methoxy)-3,3-dimethylbutanoylamino)-3-(4-(2-methylpropoxy)phenyl)propionate (105) (yield: 88%).
LR-MS(m/z):505($M^+$)
$^1$H-NMR(300MHz, $CDCl_3$, δppm) : 0.90, 0.92, 0.95 (9H, s), 0.98-1.01 (6H, m), 2.00 (1H, m), 2.95-3.21 (2H, m), 3.45, 3.61 (2H, d, J = 6.6Hz), 3.48, 3.49 (1H, s), 3.73, 3.75 (3H, s), 4.30,4.46 (1H, d, J = 11.8Hz), 4.47, 4.78 (1H, d, J = 11.8Hz), 4.92 (1H, m), 6.61-6.83 (2H, m), 6.88 (1H, d, J = 8.2Hz), 6.96-7.05 (2H, m), 7.29-7.51 (3H, m), 7.74-7.85 (4H, m).
$[\alpha]^{17}_D$: -51.8° (c=1.202, $CHCl_3$)

Example 106

2(S)-(2(S)-((2-naphthyl)methoxy)-3,3 -dimethylbutanoylamino)-3 -(4-(2-methylpropoxy)phenyl)propionic acid (106)

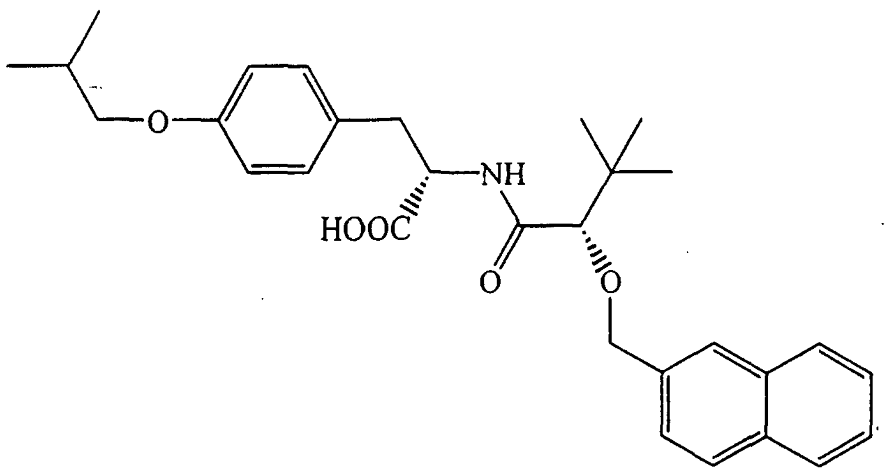

(106)

In 1 ml of methanol, 57 mg (0.11mmol) of methyl 2(S)-(2(S)-((2-naphthyl)methoxy)-3,3-dimethylbutanoylamino)-3-(4-(2-methylpropoxy)phenyl)propionate (105) was dissolved, and 0.14ml (0.13mmol) of 1N aqueous sodium hydroxide solution was added, followed by stirring the mixture at room temperature for 22 hours. To the reaction solution, 1N hydrochloric acid was added to acidify the solution, and the mixture was extracted with ethyl acetate. Organic layers were combined, washed with saturated brine, dried over anhydrous sodium sulfate and concentrated under reduced pressure. The residue was purified by column chromatography (diol column, hexane: ethyl acetate = 2:1) to obtain 46 mg of 2(S)-(2(S)-((2-naphthyl)methoxy)-3,3-dimethylbutanoylamino)-3-(4-(2-methylpropoxy)phenyl)propionic acid (106) (yield: 84%).
LR-MS(m/z):491($M^+$)
IR(KBr):3300-2500, 2958, 1737, 1246, 819$cm^{-1}$
$^1$H-NMR(300MHz, $CDCl_3$, δppm) : 1.05(9H, s), 3.68(1H, s), 4.59(1H, d, J=11.5Hz), 4.86(1H, d, J=11.5Hz), 7.48-7.51 (3H, m), 7.79-7.87(4H, m), 0.87, 0.91, 0.93 (9H, s), 0.97-0.99 (6H, m), 1.99 (1H, m), 2.98-3.29 (2H, m), 3.44, 3.45, 3.59 (2H, d, J = 6.9Hz), 3.50, 3.51 (1H, s), 4.26, 4.41 (1H, d, J = 11.8Hz), 4.42, 4.71 (1H, d, J = 11.8Hz), 4.92 (1H, m), 6.62-6.79 (2H, m), 6.86, 6.94 (1H, d, J = 8.5Hz), 7.01-7.08 (2H, m), 7.27-7.66 (3H, m), 7.73-7.85 (4H, m).
$[\alpha]^{25}_D$:-99.03° (c=0.41, MeOH)

Example 107

Methyl 2(S)-(3,3-dimethyl-2(S)-(2-naphthylmethoxy)butanoylamino)-3-(4-(2-methylpropoxy)phenyl)propionate (107)

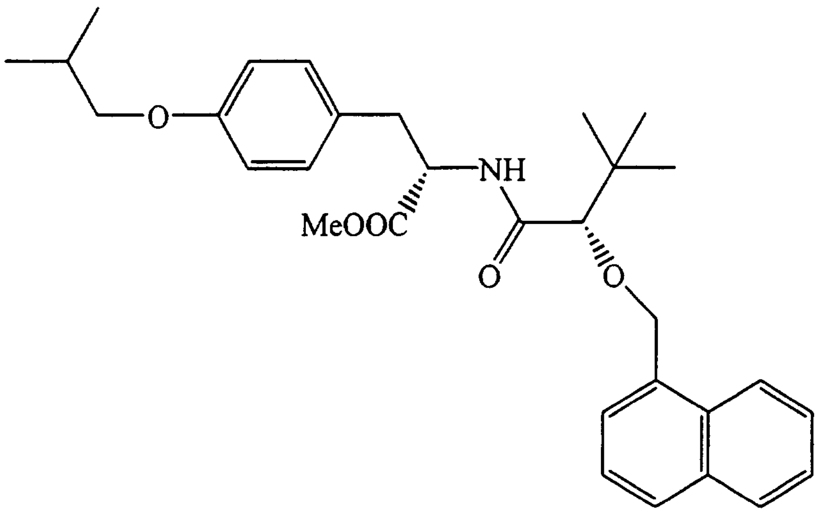

(107)

In 1 ml of DMF, 31 mg (0.12mmol) of methyl (S)-2-amino-3-(4-(2-methylpropoxy)phenyl)propionate (104) and 35 mg (0.13mmol) of 3,3-dimethyl-2-(S)-(naphthylmethoxy)butyric acid were dissolved, and 73mg (0.14mmol) of PyBOP and 0.016ml (0.15mmol) of N-methylmorpholine were added, followed by stirring the mixture at room temperature for 2 hours. To the reaction solution, 1N hydrochloric acid was added to acidify the solution, and the mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 9:1 - 5:1) to obtain 50 mg of methyl 2 (S)-(3,3-dimethyl-2(S)-(2-naphthylmethoxy)butanoylamino)-3-(4-(2-methylpropoxy)phenyl)propionate (107) (yield: 80%).

LR-MS(m/z):505($M^+$)

$^1$H-NMR(300MHz, $CDCl_3$, δppm): 0.85, 0.90, 0.93, 0.94 (9H, s), 0.96-1.02 (6H, m), 2.01 (1H, m), 2.85-3.11 (2H, m), 3.45, 3.02 (2H, d, J = 6.9Hz), 3.54, 3.56 (1H, s), 3.72, 3.73, 3.75 (3H, s), 4.64, 4.79 (1H, d, J = 11.2Hz), 4.82, 5.06 (1H, d, J = 11.2Hz), 4.89 (1H, m), 6.54-7.04 (5H, m), 7.32-8.08 (7H, m).

$[\alpha]^{16}_D$:-537.° (c=1.002, $CHCl_3$)

Example 108

2(S)-(3,3-dimethyl-2(S)-(2-naphthylmethoxy)butanoylamino)-3-(4-(2-methylpropoxy)phenyl)propionic acid (108)

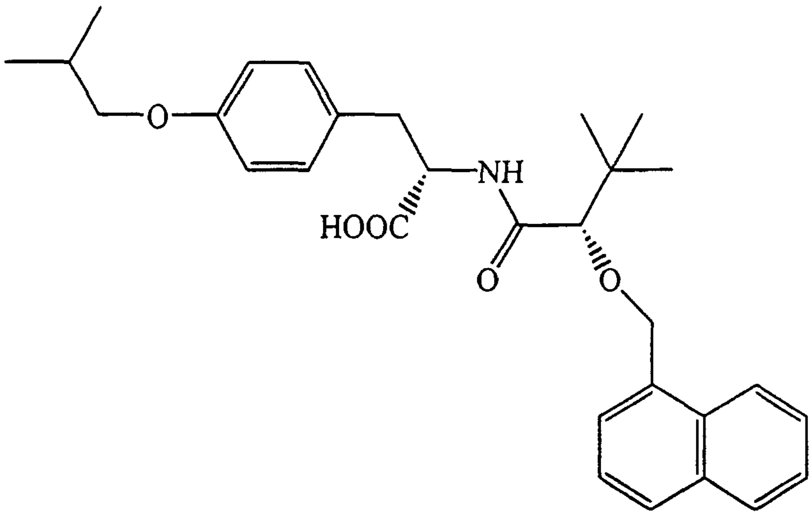

(108)

In 1 ml of methanol, 47 mg (0.09mmol) of methyl 2(S)-(3,3-dimethyl-2(S)-(2-naphthylmethoxy)butanoylamino)-3-(4-(2-methylpropoxy)phenyl)propionate (107) was dissolved, and 0.13ml (0.13mmol) of 1N aqueous sodium hydroxide solution was added, followed by stirring the mixture at room temperature for 18 hours. To the reaction solution, 1N hydrochloric acid was added to acidify the solution, and the mixture was extracted with ethyl acetate. Organic layers were combined, washed with saturated brine, dried over anhydrous sodium sulfate and concentrated under reduced pressure. The residue was purified by column chromatography (diol column, hexane: ethyl acetate = 2:1) to obtain 37 mg of 2(S)-(3,3-dimethyl-2(S)-(2-naphthylmethoxy)butanoylamino)-3-(4-(2-methylpropoxy)phenyl)propionic acid (108) (yield: 81%).
LR-MS(m/z):491($M^+$)
IR(KBr):3300-2500, 2958, 1737, 1245, 778$cm^{-1}$
$^1$H-NMR(300MHz, $CDCl_3$, δppm) : 0.72-0.93 (9H, m), 0.97-1.01 (6H, m), 1.99 (1H, m), 2.92 (1H, m), 3.15 (1H, m), 3.37-3.61 (3H, m), 4.58-4.98 (3H, m), 6.54-7.08 (5H, m), 7.29-8.06 (7H, m).
$[\alpha]^{25}_D$:-62.58° (c=0.29, MeOH)

Example 109

Methyl 2(S)-(2(S)-((4-chlorophenyl)methoxy)-4-methylpentanoylamino)-3-(4-(2-methylpropoxy)phenyl)propionate (109)

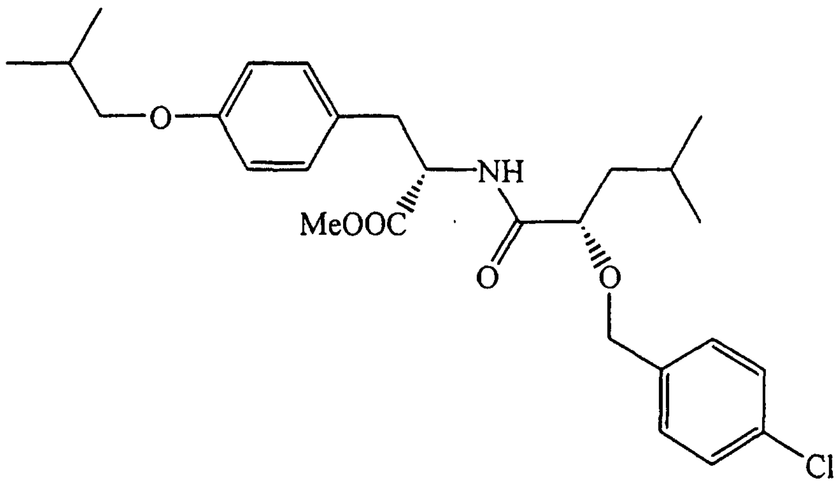

(109)

In 1 ml of DMF, 50 mg (200 μmol) of methyl (S)-2-amino-3-(4-(2-methylpropoxy)phenyl)propionate (104) and 51 mg (0.2mmol) of 2(S)-((4-chlorophenyl)methoxy)-4-methylvaleric acid were dissolved, and 115 mg (0.22mmol) of PyBOP and 0.03ml (0.25mmol) of N-methylmorpholine were added, followed by stirring the mixture at room temperature for 2.5 hours. To the reaction solution, 1N hydrochloric acid was added to acidify the solution, and the mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (hexane: ethyl acetate = 9:1 - 5:1) to obtain 88 mg of methyl 2 (S)-(2(S)-((4-chlorophenyl)methoxy)-4-methylpentanoylamino)-3-(4-(2-methylpropoxy)phenyl)propionate (109) (yield: 90%).
LR-MS(m/z):489($M^+$)
$^1$H-NMR(300MHz. $CDCl_3$, δppm) : 0.90, 0.97 (9H, s), 0.99-1.04 (6H, m), 2.06 (1H, m), 2.93-3.23 (2H, m), 3.39, 3.41 (1H, s), 3.59, 3.60, 3.67 (2H, d, J = 6.6Hz), 3.73, 3.76 (3H, s), 4.09, 4.25 (1H, d, J = 11.0Hz), 4.22, 4.56 (1H, d, J = 11.0Hz), 4.90 (1H, m), 6.67-6.82 (3H, m), 6.95-7.16 (4H, m), 7.28 (1H, m).
$[\alpha]^{17}_D$:-51.0° (c=1.770, $CHCl_3$)

Example 110

2(S)-(2(S)-((4-chlorophenyl)methoxy)-4-methylpentanoylamino)-3-(4-(2-methylpropoxy)phenyl)propionic acid (110)

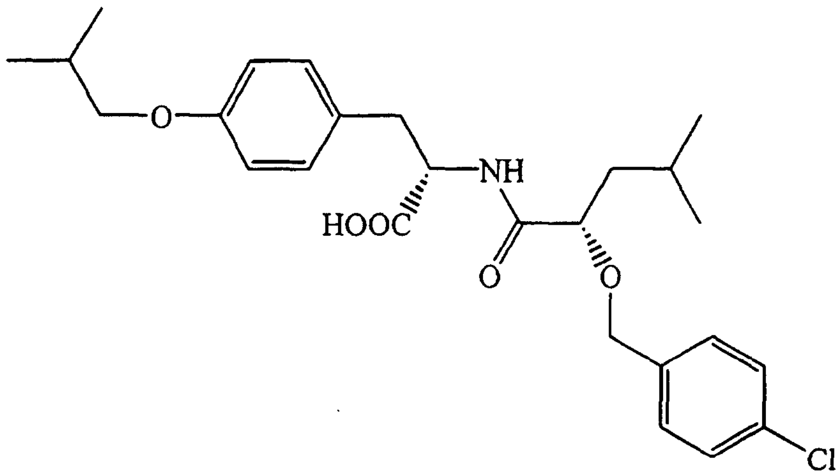

(110)

In 1 ml of methanol, 83 mg (0.17mmol) of methyl 2(S)-(2(S)-((4-chlorophenyl)methoxy)-4-methylpentanoylamino)-3-(4-(2-methylpropoxy)phenyl)propionate (109) was dissolved, and 0.2ml (0.19mmol) of 1N aqueous sodium hydroxide solution was added, followed by stirring the mixture at room temperature for 22 hours. To the reaction solution, 1N hydrochloric acid was added to acidify the solution, and the mixture was extracted with ethyl acetate. Organic layers were combined, washed with saturated brine, dried over anhydrous sodium sulfate and concentrated under reduced pressure. The residue was purified by column chromatography (diol column, hexane: ethyl acetate = 2:1) to obtain 73 mg of 2(S)-(2(S)-((4-chlorophenyl)methoxy)-4-methylpentanoylamino)-3-(4-(2-methylpropoxy)phenyl)propionic acid (110) (yield: 91%).
LR-MS(m/z):475($M^+$)
$^1$H-NMR(300MHz, $CDCl_3$, δppm) : 0.87, 0.95, 0.96 (9H, s), 0.98-1.03 (6H, m), 2.08 (1H, m), 2.96-3.31 (2H, m), 3.41, 3.44 (1H, s), 3.58, 3.59, 3.66 (2H, d, J = 6.6Hz), 4.05, 4.17 (1H, d, J = 11.0Hz), 4.21, 4.49 (1H, d, J = 11.0Hz), 4.89 (1H, m), 6.68-6.85 (3H, m), 6.91-7.08 (4H, m), 7.25 (1H, m).

Example 111

Methyl 2-(4-methyl-2-((tert-butoxy)carbonylamino)pentanoylamino-3-(4-(2-methylpropoxy)phenyl)propionate (111)

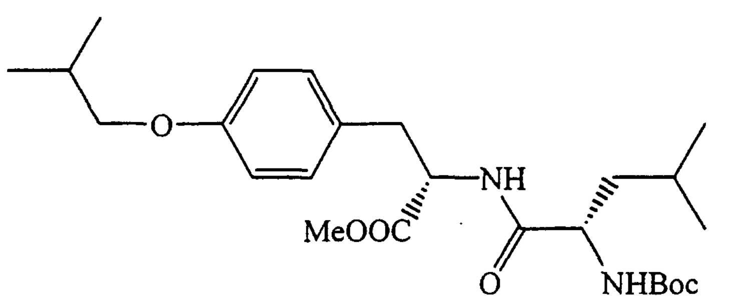

(111)

In 3 ml of dichloromethane, 210 mg (0.60 mmol) of methyl (S)-3-(4-(2-methylpropoxy)phenyl)-2-((tert-butoxy)carbonylamino)propionate (103) was dissolved, and 3 ml of TFA was added thereto, followed by stirring the mixture at room temperature for 1 hour. The reaction solution was concentrated under reduced pressure to obtain methyl (S)-2-amino-3-(4-(2-methylpropoxy)phenyl)propionate (104). This compound was dissolved in 5 ml of dichloromethane, and 180 mg of N-Boc-leucine, 317 mg of BOP reagent and 0.42 ml of diisopropylethylamine were added thereto,

followed by stirring the resulting mixture at room temperature for 1 and half hours. Water was added to the reaction solution and the mixture was extracted with ethyl acetate. Organic layers were combined, washed with 1N hydrochloric acid and saturated aqueous sodium hydrogen carbonate solution, dried over anhydrous magnesium sulfate and concentrated under reduced pressure. The residue was purified by silica gel column chromatography to quantitatively obtain 280 mg of methyl 2-(4-methyl-2-((tert-butoxy)carbonylamino)pentanoylamino-3-(4-(2-methylpropoxy)phenyl) propionate (111).

LR-MS(m/z):464($M^+$)

$^1$H-NMR(300MHz, $CDCl_3$, δppm) 0.72-0.94 (6H, m), 1.00 (6H, d), 1.28 (1H, m), 2.50 (1H, m), 2.93-3.12 (2H, m), 3.64 (2H, d), 3.70 (3H, s), 4.11 (1H, m), 4.67 (1H, m), 6.78, 6.97 (4H, d), 7.14 (1H, d).

Example 112

Methyl 2-(2-amino-4-methylpentanoylamino-3-(4-(2-methylpropoxy)phenyl)propionate (112)

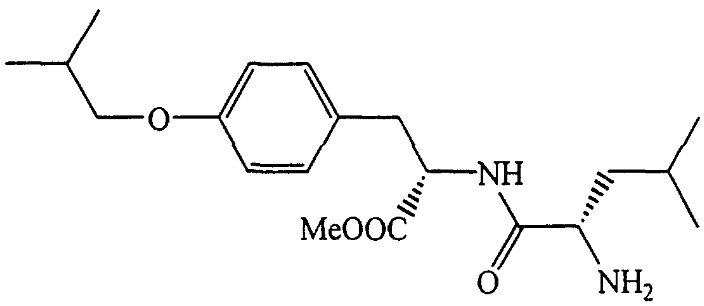

(112)

In 5 ml of dichloromethane, 280 mg (0.60 mmol) of methyl 2-(4-methyl-2-((tert-butoxy)carbonylamino)pentanoylamino-3-(4-(2-methylpropoxy)phenyl)propionate (111) was dissolved, and 5 ml of TFA was added thereto, followed by stirring the mixture at room temperature for 1 hour. The reaction solution was concentrated under reduced pressure to obtain methyl 2-(2-amino-4-methylpentanoylamino-3-(4-(2-methylpropoxy)phenyl)propionate (112). The product (112) was used in the next reaction without purification.

Example 113

Methyl 2-(4-methyl-2-(2-(4-((phenylamino)carbonylamino)phenyl)acetylamino)pentanoylamino)-3-(4-(2-methylpropoxy)phenyl)propionate (113)

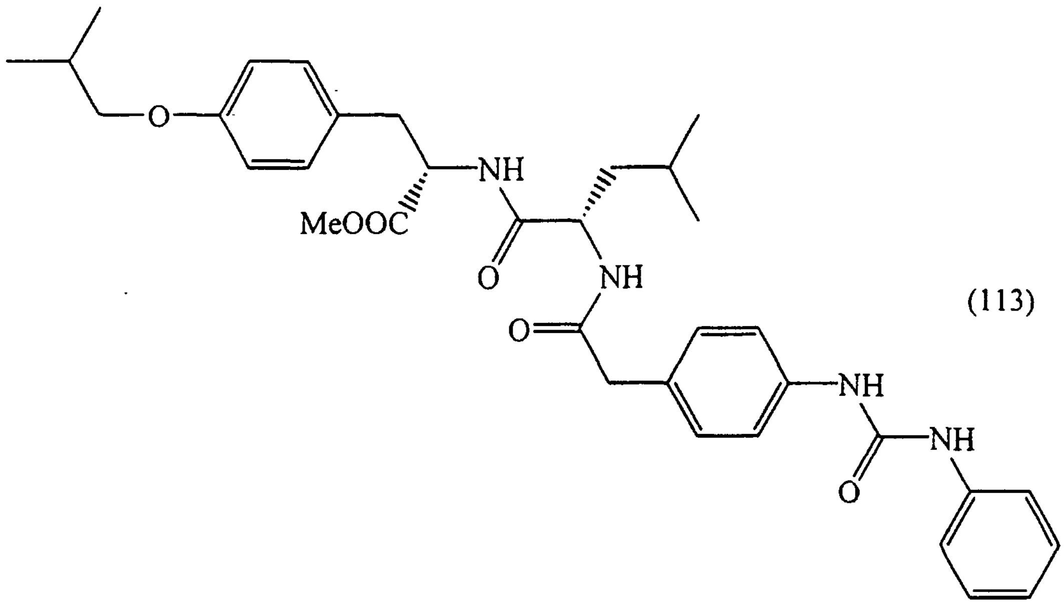

(113)

To methyl 2-(2-amino-4-methylpentanoylamino-3-(4-(2-methylpropoxy)phenyl)propionate (112), 180 mg of N-Boc-leucine, 320 mg of BOP reagent and 0.42 ml of diisopropylethylamine were added, and the mixture was stirred at room temperature for 2 hours. Water was added to the reaction solution and the mixture was extracted with ethyl acetate. Organic layers were combined, washed with 1N hydrochloric acid and saturated aqueous sodium hydrogen carbonate solution, dried over anhydrous magnesium sulfate and concentrated under reduced pressure. The residue was purified by silica gel column chromatography to quantitatively obtain 280 mg of methyl 2-(4-methyl-2-(2-(4-((phenylamino)carbonylamino)phenyl)acetylamino)pentanoylamino)-3-(4-(2-methylpropoxy)phenyl)propionate (113).
LR-MS(m/z):617$(M+H)^+$
$^1$H-NMR(300MHz, DMSO-$d_6$, δppm) : 0.78, 0.86 (6H, d), 0.94 (6H, d), 1.09-1.40 (2H, m), 1.52 (1H, m), 2.70-3.03 (2H, m), 3.31 (2H, s), 3.53 (3H, s), 3.67 (2H, d), 4.25-4.46 (2H, m), 6.75-7.46 (13H, m), 8.02-8.62 (4H, m).

Example 114

2-(4-methyl-2-(2-(4-((phenylamino)carbonylamino)phenyl)acetylamino)pentanoylamino)-3-(4-(2-methylpropoxy)phenyl)propionic acid (114)

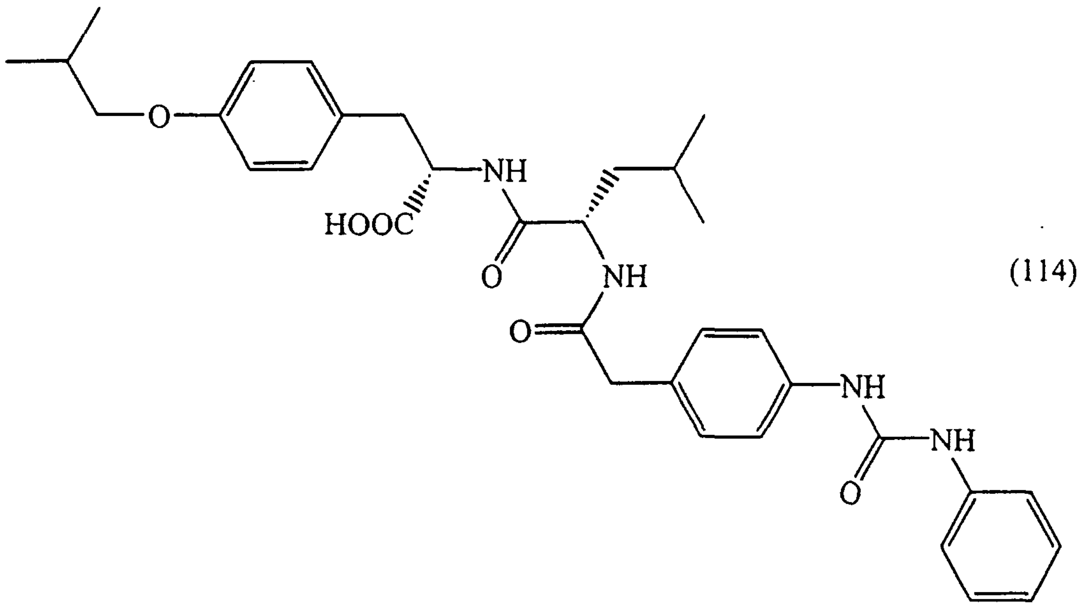

(114)

In 0.5 ml of methanol and 0.5 ml of chloroform, methyl 2-(4-methyl-2-(2-(4-((phenylamino)carbonylamino)phenyl)acetylamino)pentanoylamino)-3-(4-(2-methylpropoxy)phenyl)propionate (113) (23 mg, 0.037 mmol) was dissolved, and 0.5 ml of 1N aqueous sodium hydroxide solution was added, followed by stirring the resulting mixture at room temperature for 12 hours. To the reaction solution, 1N hydrochloric acid was added to adjust the pH to 1, and the mixture was extracted with chloroform. Organic layers were combined, washed with saturated brine, dried over anhydrous magnesium sulfate and concentrated under reduced pressure to obtain 23 mg of 2-(4-methyl-2-(2-(4-((phenylamino)carbonylamino)phenyl)acetylamino)pentanoylamino)-3-(4-(2-methylpropoxy)phenyl)propionic acid (114).
LR-MS(m/z):603(M+H)$^+$
$^1$H-NMR(300MHz, $CDCl_3$, δppm) : 0.68, 0.72 (6H, d), 0.83 (6H, d), 1.19-1.41 (3H, m), 1.88 (1H, m), 2.71-2.98 (2H, m), 3.31 (2H, s), 3.53 (2H, d), 4.27 (1H, m), 4.50 (1H, m), 6.61-7.28 (17H, m).

Example 115

Methyl 3-(4-((trifluoromethyl)sulfonyloxy)phenyl)-2-((tert-butoxy)carbonylamino)propionate (115)

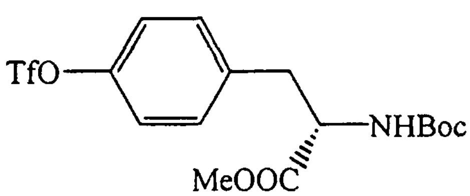

(115)

Under cooling in ice, to 3.00 g (10.2 mmol) of L-Boc-Tyr-OMe, a solution of 4.00 g (11.2 mmol) of N-Phenylbis (trifluoromethane-sulfonimide) in anhydrous dichloromethane (30 ml) and 1.60 ml (11.5 mmol) of $Et_3N$ were added, and the resulting mixture was stirred at 0°C for 1 hour and then at room temperature overnight. The reaction solution was concentrated, and water was added to the residue, followed by extraction of the resulting mixture with ethyl acetate (20 ml x 4). Organic layers were combined, washed with water and saturated brine, dried over anhydrous sodium

sulfate and concentrated. The residue was purified by medium-pressure chromatography (Si-40C/ cyclohexane:ethyl acetate = 80:20) to obtain 4.14 g of methyl 3-(4-((trifluoromethyl)sulfonyloxy)phenyl)-2-((tert-butoxy)carbonylamino) propionate (115) as an oil (yield>99%).
LR-MS(m/z):371 $(M+-C(CH_3)_3+H)^+$
IR(neat):3363, 2980, 1746, 1715, 1502, 1424, 1367, 1250, 1213, 1167, 1141cm$^{-1}$ $^1$H-NMR(300MHz, $CDCl_3$, δppm) : 7.22 (4H, s), 5.03 (1H, d, J=7.14Hz), 4.62-4.59 (1H, m), 3.72 (3H, s), 3.21-3.14 (1H, m), 3.08-3.01 (1H, m), 1.41 (9H, s)

Example 116

Methyl 3-(4-(2-((tert-butoxy)carbonylamino)-2-(methoxycarbonyl)phenyl)acrylate (116)

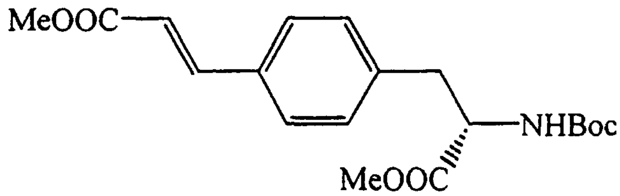

(116)

Under argon atmosphere, to a solution of 3.40 g (7.96 mmol) of methyl 3-(4-((trifluoromethyl)sulfonyloxy) phenyl)-2-((tert-butoxy)carbonylamino)propionate (115) in anhydrous DMF (40 ml), were added 6.8 ml (49.0 mmol) of $Et_3N$, 1.01 g (23.8 mmol) of LiCl, 1.21 g (3.97 mmol) of $P(o\text{-}Tol)_3$, 184 mg (0.819 mmol) of palladium acetate and 1.8 ml (20.1 mmol) of methyl acrylate, and the mixture was stirred overnight at 90°C. The reaction mixture was filtered through Celite, and the filtrate was concentrated. Water was added to the residue and the mixture was extracted with ethyl acetate (50 ml x 4). Organic layers were combined, washed with water and saturated brine, dried over anhydrous sodium sulfate and concentrated. The residue was purified by medium-pressure chromatography (Si-40C/ cyclohexane:ethyl acetate = 80:20) to obtain 2.47 g of methyl 3-(4-(2-((tert-butoxy)carbonylamino)-2-(methoxycarbonyl)phenyl) acrylate (116) as yellow solid (yield: 85%).
LR-MS( m/z):371 $(M+-C(CH3)3+H)^+$

Example 117

Methyl 2-((tert-butoxy)carbonylamino)-3-(4-(2-(methoxycarbonyl)ethyl)phenyl)propionate (117)

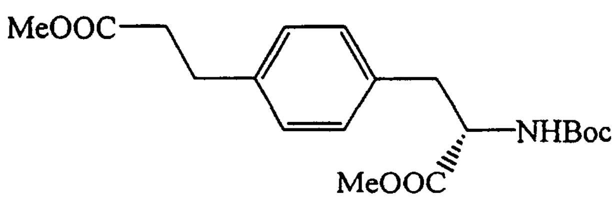

(117)

Under argon atmosphere, 1.78 g of 10% palladium/carbon was added to a solution of 17.78 g (48.93 mmol) of methyl 3-(4-(2-((tert-butoxy)carbonylamino)-2-(methoxycarbonyl)phenyl)acrylate (116) in MeOH (200 ml), and hydrogen replacement was carried out, followed by stirring the resulting mixture overnight at room temperature. The reaction mixture was filtered through Celite, and the filtrate was concentrated. The residue was purified by column chromatography (silica gel/cyclohexane: ethyl acetate = 80:20 - 70:30) to obtain 16.45 g of methyl 2-((tert-butoxy) carbonylamino)-3-(4-(2-(methoxycarbonyl)ethyl)phenyl)propionate (117) as colorless oil (yield 92%).
LR-MS(m/z):366 $(M+H)^+$
IR(neat):3374, 2978, 2953, 1739, 1715, 1515, 1438, 1391, 1366, 1251, 1166cm$^{-1.}$ $^1$H-NMR(300MHz, $CDCl_3$, δppm) : 7.14-7.03 (4H, m), 4.96 (1H, d, J=7.69Hz), 4.60-4.53 (1H, m), 3.72 (3H, s), 3.67 (3H, s), 3.12-2.98 (2H, m), 2.92 (2H, t, J=8.24Hz), 2.61 (2H, t, J=8.24Hz), 1.41 (9H, s)

Example 118

Methyl 2-amino-3-(4-(2-(methoxycarbonyl)ethyl)phenyl)propionate (118)

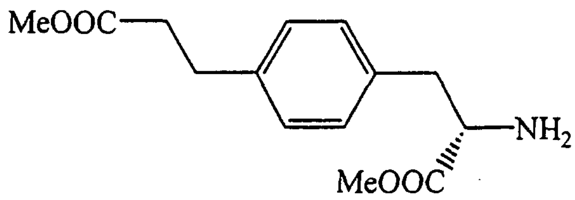

(118)

To a solution of 210 mg of methyl 2-((tert-butoxy)carbonylamino)-3-(4-(2-(methoxycarbonyl)ethyl)phenyl)propionate (117) in dichloromethane (3.0 ml), 2.5 ml of HBr/AcOH was added and the mixture was stirred at room temperature for 1 hour. The reaction solution was concentrated and MeOH was added to the residue to dissolve the same, followed by reprecipitation with ether to obtain 182 mg of methyl 2-amino-3-(4-(2-(methoxycarbonyl)ethyl)phenyl)propionate (118) hydrobromic acid salt as white solid.

Example 119

Methyl 2-(2-(4-acetyloxyphenyl)-4-methylpentanoylamino)-3-(4-(2-(methoxycarbonyl)ethyl)phenyl)propionate (119)

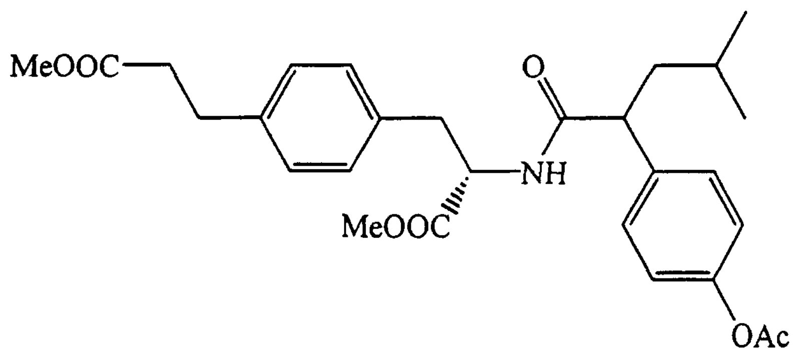

(119)

To 151 mg of methyl 2-amino-3-(4-(2-(methoxycarbonyl)ethyl)phenyl)propionate (118) hydrobromic acid salt, 132 mg of 2-(4-acetyloxyphenyl)-4-methylvaleric acid, 231 mg of BOP reagent, 2.0 ml of anhydrous dichloromethane and 380 μl of diisopropylamine were added and the mixture was stirred overnight at room temperature. Water was added to the reaction solution and the mixture was extracted with ethyl acetate (15 ml x 4). Organic layers were combined, washed with water and saturated brine, dried over anhydrous sodium sulfate and concentrated. The residue was purified by medium-pressure chromatography (Si-40B/ cyclohexane:ethyl acetate = 60:40) to obtain methyl 2-(2-(4-acetyloxyphenyl)-4-methylpentanoylamino)-3-(4-(2-(methoxycarbonyl)ethyl)phenyl)propionate (119) as two diastereomers. (Isomer A: 96.8 mg (yield: 45%); Isomer B: 100 mg (yield: 46%) (on TLC ($SiO_2$/ cyclohexane: ethyl acetate = 2:1), the component of which Rf was 0.33 was defined as Isomer A, and the component of which Rf was 0.18 was defined as Isomer B).

<Isomer A>

LR-MS(m/e):497 ($M^+$)
IR(neat):3319, 2954, 1741, 1655, 1506, 1438, 1369, 1204cm$^{-1}$
$^1$H-NMR(300MHz, $CDCl_3$, δppm) :7.28-7.23 (2H, m), 7.08-7.04 (2H, m), 6.95 (2H, d, J=7.97Hz), 6.62 (2H, d, J=8.24Hz), 5.83 (1H, d, J=7.42Hz), 4.84 (1H, td, J=5.49, 7.69Hz), 3.73 (3H, s), 3.68 (3H, s), 3.40 (1H, t, J=7.42Hz), 2.97-2.94 (2H, m), 2.91-2.85 (2H, m), 2.61-2.56 (2H, m), 2.32 (3H, s), 1.94 (1H, td, J=7.42, 13.46Hz), 1.69-1.60 (1H, m), 1.47-1.34 (1H, m), 0.86 (6H, dd, J=1.92, 6.59Hz)

<Isomer B>

LR-MS(m/e):497 ($M^+$)
IR(neat):3313, 2954, 1740, 1653, 1506, 1438, 1369, 1204cm$^{-1}$
$^1$H-NMR(300MHz, $CDCl_3$, δppm) :7.29-7.24 (2H, m), 7.08-7.01 (4H, m), 6.89 (2H, d, J=8.24Hz), 5.89 (1H, d, J=7.96Hz), 4.78 (1H, td, J=5.77, 7.42Hz), 3.68 (3H, s), 3.68 (3H, s), 3.42 (1H, t, J=7.69Hz), 3.11-2.85 (4H, m), 2.64-2.59 (2H, m), 2.30 (3H, s), 1.94 (1H, td, J=7.42, 13.74Hz), 1.62 (1H, ddd, J=6.87, 7.97, 13.74Hz), 1.47-1.33 (1H, m), 0.87 (6H, dd, J=1.10, 6.59Hz)

Example 120

3-(4-(2-carboxyethyl)phenyl)-2-(2-(4-hydroxyphenyl)-4-methylpentanoylamino)propionic acid (120)

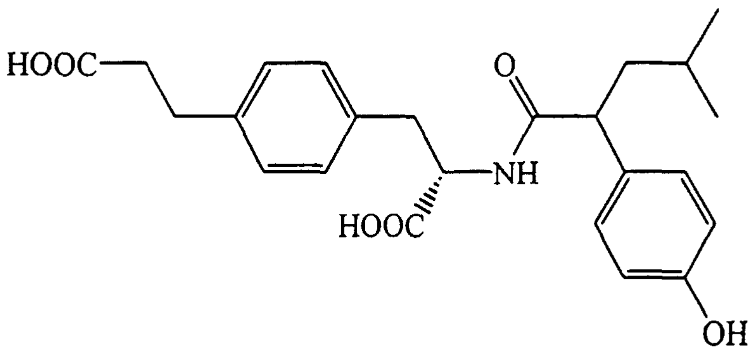

(120)

In 2.0 ml of MeOH, 96.3 mg of methyl 2-(2-(4-acetyloxyphenyl)-4-methylpentanoylamino)-3-(4-(2-(methoxycarbonyl)ethyl)phenyl)propionate (119) (Isomer A) was dissolved, and 1.2 ml of aqueous 1N NaOH solution was added thereto, followed by stirring the mixture at room temperature for 1.5 hours. The reaction solution was concentrated and 5% aqueous citric acid solution was added, followed by extraction of the resulting mixture with ethyl acetate (10 ml x 4). Organic layers were combined, washed with saturated brine (10 ml x 2), dried over anhydrous sodium sulfate and concentrated. The residue was recrystallized from hexane/ethyl acetate to obtain 78.2 mg of 3-(4-(2-carboxyethyl)phenyl)-2-(2-(4-hydroxyphenyl)-4-methylpentanoylamino)propionic acid (120) (Isomer A) (yield: 95%).
In the same manner, 74.8 mg of 3-(4-(2-carboxyethyl)phenyl)-2-(2-(4-hydroxyphenyl)-4-methylpentanoylamino)propionic acid (120) (Isomer B) was obtained from 94.7 mg of methyl 2-(2-(4-acetyloxyphenyl)-4-methylpentanoylamino)-3-(4-(2-(methoxycarbonyl)ethyl)phenyl)propionate (119) (Isomer B) (yield: 92%).

<Isomer A>

mp:89-91°C
LR-MS(m/z):427 ($M^+$)
IR(neat):3422, 2926, 1719, 1655, 1638, 1560, 1543, 1510, 1459, 1440, 1243, 838, 616cm$^{-1}$
$^1$H-NMR(300MHz, $CD_3OD$, δppm) :7.94 (1H, d, J=8.52Hz), 7.05-7.02 (2H, m), 6.93 (2H, d, J=8.24Hz), 6.83 (2H, d, J=8.24Hz), 6.71-6.67 (2H, m), 4.63-4.56 (1H, m), 3.54-3.49 (1H, m), 3.07-3.01 (1H, m), 2.89-2.78 (3H, m), 2.52 (2H, t, J=7.97Hz), 1.90-1.80 (1H, m), 1.55-1.38 (2H, m), 0.88 (6H, t, J=6.04Hz)
HR: Calcd.=427.1995, Found=427.2025
$[\alpha]^{20}_D$= +16.3° (c=0.098, MeOH)

<Isomer B>

mp:186-188°C
LR-MS(m/z):427 ($M^+$)
IR(KBr):2956, 2928, 1707, 1638, 1614, 1515, 1447, 1244, 1016, 835, 643, 583, 540cm$^{-1}$
$^1$H-NMR(300MHz, $CD_3OD$, δppm) :8.06 (1H, d, J=8.52Hz), 7.11-7.01 (6H, m), 6.70-6.67 (2H, m), 4.64-4.58 (1H, m), 3.46 (1H, dd, J=6.59, 9.07Hz), 3.19-3.12 (1H, m), 2.93-2.83 (3H, m), 2.56 (2H, t, J=7.42Hz), 1.82-1.73 (1H, m), 1.41-1.32 (1H, m), 1.23-1.12 (1H, m), 0.79 (6H, d, J=6.59Hz)

HR: Calcd.=427.1995, Found=427.1968

Example 121

Methyl 3-(4-(2(S)-(methoxycarbonyl)ethyl)phenyl)-2-(4-methyl-2(R,S)-(4-((phenylsulfonyl)amino)phenyl) pentanoylamino)propionate (121)

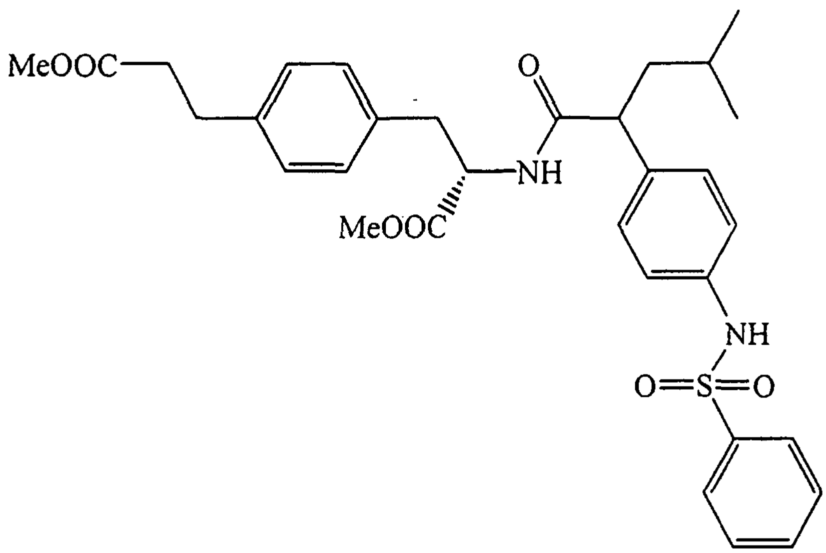

(121)

Under argon atmosphere, 0.090 ml of diisopropylethylamine was added to a suspension of 34.6 mg of methyl 2-amino-3-(4-(2-(methoxycarbonyl)ethyl)phenyl)propionate (118) hydrobromic acid salt, 41.7 mg of 4-methyl-2-(4-((phenylsulfonyl)amino)phenyl)valeric acid and 53.1 mg of BOP reagent in 1.5 ml of dichloromethane while cooling the mixture in ice, and the resulting mixture was stirred overnight at room temperature. To the reaction solution, 4 ml of 0.1N hydrochloric acid and 1 ml of water were added and the mixture was extracted with ethyl acetate (3 x 12 ml). Organic layers were combined, washed with saturated brine, dried over anhydrous sodium sulfate and concentrated under reduced pressure. The residue was purified by column chromatography (silica gel, cyclohexane: ethyl acetate = (80/20) - (1/99)) to quantitatively obtain 59.8 mg of methyl 3-(4-(2(S)-(methoxycarbonyl)ethyl)phenyl)-2-(4-methyl-2 (R,S)-(4-((phenylsulfonyl)amino)phenyl)pentanoylamino)propionate (121) (diastereomer ratio: about 1:1) as colorless oil.

LR-MS(m/z):594($M^+$)

IR (neat):3585, 3278, 1737, 1653, 1510, 1446, 1403, 1342, 1213, 1161, 1092, 1021, 925, 843, 756, 731, 688 $cm^{-1}$.

$^1$H-NMR(300MHz, $CDCl_3$, δppm) :7.81-7.73 (2H, m), 7.50 (1H,m), 7.45-7.35 (2H, m), 7.14-7.07 (2H, m), 7.07-6.94 (3H, m), 6.93-6.83 (2H, m), 6.57 (1H, d, J = 8.0Hz), 5.92 (0.5H, d, J = 7.7 Hz), 5.81 (0.5H, d, J = 8.0 Hz), 4.80 (1H, m), 3.71 (1.5H, s), 3.68 (1.5H, s), 3.67 (1.5H, s), 3.66 (1.5H, s), 3.34 (1H, ddd, J = 10.7, 8.0, 7.7 Hz), 3.09-2.82 (4H, m), 2.62 (1H, d, J = 8.0 Hz), 2.57 (1H, d, J = 7.7Hz), 1.86 (1H, m), 1.56 (1H, m), 1.30 (1H, m), 0.81 (6H, d, J = 6.6 Hz).

Example 122

3-(4-(2(S)-carboxyethyl)phenyl)-2-(4-methyl-2(R,S)-(4-((phenylsulfonyl)amino)phenyl)pentanoylamino)propionic acid (122)

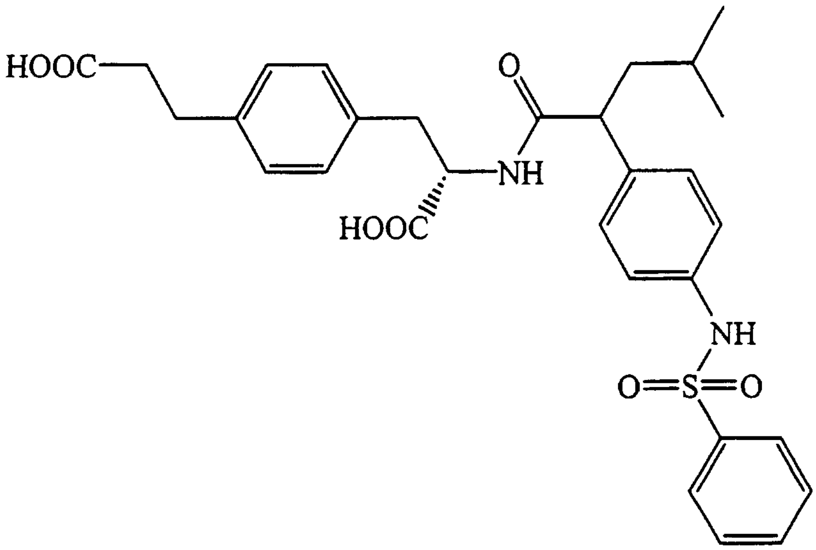

(122)

Under argon atmosphere, 1.0 ml of 1N aqueous sodium hydroxide solution was added to a solution of 57.0 mg of methyl 3-(4-(2(S)-(methoxycarbonyl)ethyl)phenyl)-2-(4-methyl-2(R,S)-(4-((phenylsulfonyl)amino)phenyl)pentanoylamino)propionate (121) in 2.0 ml of methanol, and the mixture was stirred at room temperature for 3 hours. To the reaction solution, 1.1 ml of 1N hydrochloric acid and 5 ml of water were added, and the mixture was extracted with ethyl acetate (3 x 12 ml). Organic layers were combined, washed with 6 ml of saturated brine, dried over anhydrous sodium sulfate and concentrated under reduced pressure. The residue was purified by column chromatography (DIOL, cyclohexane: ethyl acetate = (50/50) - (20/80)) to quantitatively obtain 54.0 mg of 3-(4-(2(S)-carboxyethyl)phenyl)-2-(4-methyl-2(R,S)-(4-((phenylsulfonyl)amino)phenyl)pentanoylamino)propionic acid (122) (diastereomer ratio: about 1:1) as colorless oil (yield: 99%).
LR-MS(m/z):566 ($M^+$).
$^1$H-NMR(300MHz, $CD_3OD$, δppm) :8.21 (0.5H, d, J = 8.5 Hz), 8.08 (0.5H, d, J = 8.2 Hz), 7.78-7.68 (2H, m), 7.52 (1H, m), 7.47-7.38 (2H, m), 7.17-6.95 (6H, m), 6.87 (1H, d, J = 8.2 Hz), 6.77 (1H, d, J = 8.2 Hz), 4.59 (1H, m), 3.54 (0.5H, dd, J = 8.8, 6.8 Hz), 3.47 (0.5H, dd, J = 9.4, 6.2 Hz), 3.16 (0.5H, dd, J = 14.0, 4.5 Hz), 3.01 (0.5H, dd, J = 14.0, 4.7 Hz), 2.91-2.75 (3H, m), 2.59-2.48 (2H, m), 1.80 (1H, m), 1.49-1.24 (1.5H, m), 1.10 (0.5H, m), 0.86 (1.5H, d, J = 6.3 Hz), 0.84 (1.5H, d, J = 6.3 Hz), 0.77 (1.5H, d, J = 6.3 Hz), 0.76 (1.5H, d, J = 6.3 Hz).
HR-MS :$C_{30}H_{34}N_2O_7S$ Calcd.: 566.2087, Found: 566.2051

Example 123

Methyl 3-(4-(2(S)-(methoxycarbonyl)ethyl)phenyl)-2(S)-(4-methyl-2(R,S)-(4-(phenylcarbonylamino)phenyl) pentanoylamino)propionate (123)

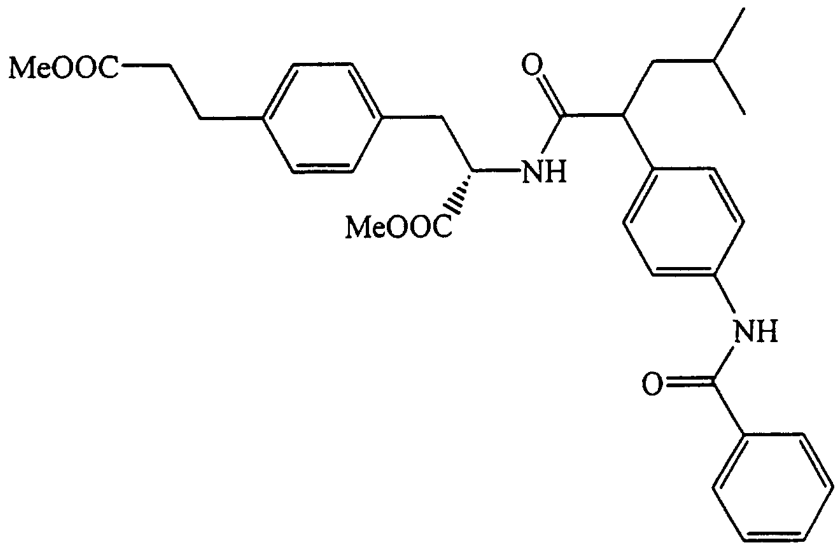

(123)

Under cooling in ice, 46 mg of methyl 2-amino-3-(4-(2-(methoxycarbonyl)ethyl)phenyl)propionate (118) hydrochloric acid salt and 50 mg of 4-methyl-2-(4-(phenylcarbonylamino)phenyl)valeric acid were dissolved in 1.5 ml of dichloromethane, and 71 mg of BOP reagent and 84 mg of diisopropylethylamine were added, followed by stirring the mixture at room temperature for I and half hours. Water and 1N hydrochloric acid were added to the reaction solution, and the mixture was extracted with ethyl acetate. Organic layers were combined, washed with saturated brine, dried over anhydrous magnesium sulfate and concentrated under reduced pressure. The residue was purified by silica gel column chromatography to obtain 33 mg of methyl 3-(4-(2(S)-(methoxycarbonyl)ethyl)phenyl)-2(S)-(4-methyl-2(R,S)-(4-(phenylcarbonylamino)phenyl)pentanoylamino)propionate (123) (yield: 47%).
LR-MS (m/z):558 ($M^+$)
IR(KBr): 3279, 2952, 1739, 1647, 1604, 1534, 1414, 1329, 1262, 710 $cm^{-1}$
$^1$H-NMR(300MHz, $CDCl_3$, δppm) : 7.91 (2H, d), 7.63-7.40 (5H, m), 7.23 (2H, d), 7.05 (2H, d), 6.85 (2H, d), 5.83 (1H, m), 4.81 (1H, m), 3.65 (3H, s), 3.62 (3H, s), 3.43 (1H, m), 3.03-2.87 (3H, m), 2.62 (2H, t), 1.93 (1H, m), 1.68 (1H, m), 1.42 (1H, m), 0.90 (6H, m)

Example 124

3-(4-(2(S)-carbonylethyl)phenyl)-2(S)-(4-methyl-2(R,S)-(4-(phenylcarbonylamino)phenyl)pentanoylamino)propionic acid (124)

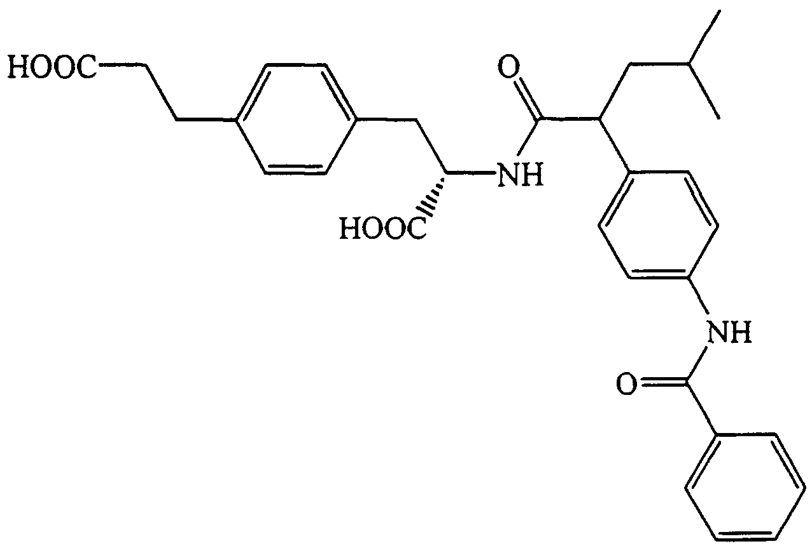

(124)

In 2 ml of methanol, 33 mg of methyl 3-(4-(2(S)-(methoxycarbonyl)ethyl)phenyl)-2(S)-(4-methyl-2(R,S)-(4-(phenylcarbonylamino)phenyl)pentanoylamino)propionate (123) was dissolved, and 1 ml of 1N aqueous sodium hydroxide solution was added thereto, followed by stirring the mixture at room temperature for 4 hours. Water and 1N hydrochloric acid were added to the reaction solution so as to attain a pH of 1, and the mixture was extracted with ethyl acetate. Organic layers were combined, washed with saturated brine, dried over anhydrous magnesium sulfate and concentrated under reduced pressure. The residue was recrystallized from ethyl acetate/methanol to obtain 3.9 mg of a single optical isomer of 3-(4-(2(S)-carbonylethyl)phenyl)-2(S)-(4-methyl-2(R,S)-(4-(phenylcarbonylamino)phenyl)pentanoylamino)propionic acid (124). The mother liquor was purified by diol column chromatography to obtain 20.9 mg of 3-(4-(2(S)-carbonylethyl)phenyl)-2(S)-(4-methyl-2(R,S)-(4-(phenylcarbonylamino)phenyl)pentanoylamino)propionic acid (124) (R:S=1:1, 67%).

<Single Optical Isomer>

m.p.: 226°C
LR-MS (m/z):531(M+H)$^+$
IR(KBr): 3315, 2955, 1706, 1654, 1525, 1414, 1326, 829, 710 cm$^{-1}$
$^1$H-NMR(300MHz, $CDCl_3$, δppm) : 7.95 (2H, d), 7.63-7.48 (5H, m), 7.36 (2H, d), 7.10 (4H, m), 4.61 (1H, m), 3.58 (1H, m), 2.58 (2H, t), 1.83 (1H, m), 1.45 (1H, m), 1.23 (1H, m), 0.90 (6H, m)

<Mixture of Optical Isomers R:S=1:1>

LR-MS (m/z):531 (M+H)$^+$
IR(KBr): 3320, 2956, 1719, 1648, 1527, 1414, 1327, 839, 710 cm$^{-1}$
$^1$H-NMR(300MHz, $CDCl_3$, δppm) : 7.83 (2H, m), 7.56-7.40 (5H, m), 7.22 (1H, d), 7.18-6.99 (3H, m), 6.82 (1H, dd), 4.56 (1H, m), 3.54 (1H, m), 3.18-2.98 (1H, m), 2.86-2.70 (3H, m), 2.55-2.39 (2H, m), 1.80 (1H, m), 1.58-1.05 (3H, m), 0.88 (6H, m)

Example 125

Methyl 3-(4-((trifluoromethyl)sulfonyloxy)phenyl)-2-((tert-butoxy)carbonylamino)propionate (125)

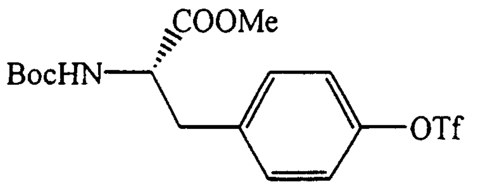

(125)

Under cooling in ice, 3.00 g of L-Boc-Tyr-OMe was dissolved in 30 ml of anhydrous dichloromethane, and 4.00 g of N-Phenylbis (trifluoromethane-sulfonimide) and 1.60 ml of $Et_3N$ were added thereto, followed by stirring the resulting mixture at 0°C for 1 hour and then at room temperature overnight. The reaction solution was concentrated and water was added to the residue, followed by extraction of the resulting mixture with ethyl acetate (20 ml x 4). Organic layers were combined, washed with saturated brine, dried over anhydrous sodium sulfate and concentrated. The residue was purified by medium-pressure chromatography (Si-40C/ cyclohexane:ethyl acetate = 80:20) to obtain 4.41 g of methyl 3-(4-((trifluoromethyl)sulfonyloxy)phenyl)-2-((tert-butoxy)carbonylamino)propionate (125) as an oil (yield: 99%).
LR-MS(m/z):371 ($M^+$-$C(CH_3)_3$+H)
IR(neat):3363, 2980, 1746, 1715, 1502, 1424, 1367, 1250, 1213, 1167, 1141cm$^{-1}$
$^1$H-NMR(300MHz, $CDCl_3$, δppm) :7.22 (4H, s), 5.03 (1H, d, J=7.14Hz), 4.62-4.59 (1H, m), 3.72 (3H, s), 3.21-3.14 (1H, m), 3.08-3.01 (1H, m), 1.41 (9H, s)

Example 126

tert-Butyl 3-(4-(2-((tert-butoxy)carbonylamino)-2-(methoxycarbonyl)phenyl)acrylate (126)

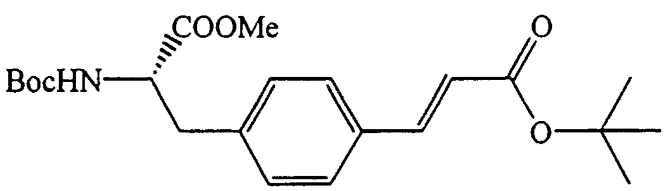

(126)

Under argon atmosphere, to 4.35 g of methyl 3-(4-((trifluoromethyl)sulfonyloxy)phenyl)-2-((tert-butoxy)carbonylamino)propionate (125), 55 ml of anhydrous DMF, 8.5 ml of $Et_3N$, 1.30 g of LiCl, 1.55 g of $P(o\text{-}Tol)_3$, 231 mg of palladium acetate and 3.8 ml of tert-butyl acrylate were added, and the resulting mixture was stirred overnight at 90°C. The reaction mixture was filtered through Celite and the filtrate was concentrated. Water was added to the residue, and the mixture was extracted with ethyl acetate (30 ml x 4). Organic layers were combined, washed with saturated brine, dried over anhydrous sodium sulfate and concentrated. The residue was purified by medium-pressure chromatography (Si-40C/ cyclohexane:ethyl acetate = 70:30) to obtain 3.72 g of tert-butyl 3-(4-(2-((tert-butoxy)carbonylamino)-2-(methoxycarbonyl)phenyl)acrylate (126) as yellow solid (yield: 90%).
LR-MS(m/z):405 ($M^+$)
IR(KBr):3346, 2979, 1748, 1712, 1634, 1514, 1440, 1392, 1367, 1329, 1255, 1215, 1152, 1059, 1022, 984, 818cm$^{-1}$
$^1$H-NMR(300MHz, $CDCl_3$, δppm):7.56 (1H, d, J=16.21Hz), 7.44 (1H, d, J=8.24Hz), 7.14 (1H, d, J=7.97Hz), 6.34 (1H, d, J=15.93Hz), 4.99 (1H, d, J=7.69Hz), 4.61-4.59 (1H, m), 3.72 (3H, s), 3.18-3.11 (1H, m), 3.08-3.01 (1H, m), 1.53 (9H, s), 1.42 (9H, s)

Example 127

Methyl 2-((tert-butoxy)carbonylamino)-3-(4-(2-((tert-butyl)oxycarbonyl)phenyl)propionate (127)

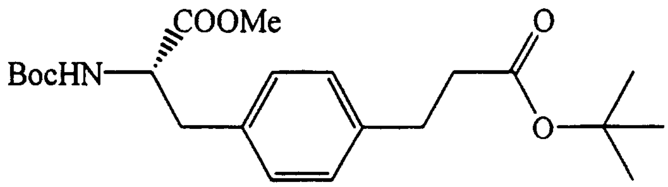

(127)

Under argon atmosphere, 858 mg of tert-butyl 3-(4-(2-((tert-butoxy)carbonylamino)-2-(methoxycarbonyl)phenyl)acrylate (126) was suspended in 6.0 ml of MeOH, 85.9 mg of 10% palladium/carbon was added, and hydrogen replacement was carried out. The reaction mixture was stirred overnight at room temperature and filtered through Celite, followed by concentrating the filtrate to obtain 860 mg of methyl 2-((tert-butoxy)carbonylamino)-3-(4-(2-((tert-butyl)oxycarbonyl)phenyl)propionate (127) as colorless oil (yield: 99%).
LR-MS(m/z):408 $(M+H)^+$
IR(neat):3365, 2978, 1717, 1507, 1455, 1392, 1366, 1252, 1165cm$^{-1}$
$^1$H-NMR(300MHz, $CDCl_3$, δ ppm):7.13 (2H, d, J=7.97Hz), 7.03 (2H, d, J=7.97Hz), 4.95 (1H, d, J=7.97Hz), 4.60-4.53 (1H, m), 3.71 (3H, s), 3.12-2.98 (2H, m), 2.88 (2H, t, J=7.8Hz), 2.52 (2H, t, J=7.8Hz), 1.42 (18H, s)

Example 128

3-(4-(2-((tert-butoxy)carbonylamino)-2-(methoxycarbonyl)ethyl)phenyl)propionic acid (128)

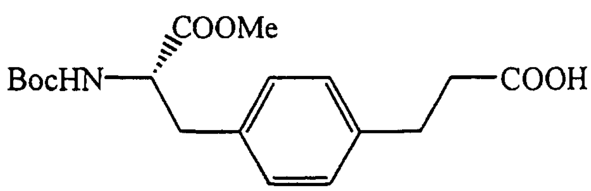

(128)

In 4.0 ml of anhydrous dichloromethane, 439 mg of methyl 2-((tert-butoxy)carbonylamino)-3-(4-(2-((tert-butyl) oxycarbonyl)phenyl)propionate (127) was dissolved, and 2.0 ml of trifluoroacetic acid was added thereto while cooling the mixture in ice. After stirring the reaction solution at 0°C for 2 hours, another 1.0 ml of trifluoroacetic acid was added and the mixture was stirred for 1 hour. The reaction solution was concentrated and subjected to azeotropic distillation with toluene.
To the residue, 2.0 ml of water and 3.0 ml of 1,4-dioxane were added, and 477 mg of (Boc)20 and 0.75 ml of $Et_3N$ were added, followed by stirring the mixture at room temperature for 1 hour. To the reaction solution, 5% aqueous citric acid solution was added and the mixture was extracted with ethyl acetate (10 ml x 4). Organic layers were combined, washed with water and saturated brine, dried over anhydrous sodium sulfate and concentrated. The residue was recrystallized from hexane/ethyl acetate to obtain 223 mg of 3-(4-(2-((tert-butoxy)carbonylamino)-2-(methoxycarbonyl)ethyl)phenyl)propionic acid (128) as colorless crystals. Further, the mother liquor was purified by medium-pressure column chromatography (Diol-40A/ cyclohexane: ethyl acetate = 67:33) to obtain 319 mg of the product (yield: 84%).
LR-MS(m/z):351 $(M^+)$
IR(KBr):3350, 2962, 1733, 1709, 1518, 1440, 1366, 1301, 1247, 1226, 1177, 1062, 1012, 978, 830cm$^{-1}$
$^1$H-NMR(300MHz, $CDCl_3$, δppm):7.16-7.04 (4H, m), 4.98 (1H, d, J=8.24Hz), 4.61-4.56 (1H, m), 3.72 (3H, s), 3.12-3.00 (2H, m), 2.94 (2H, t, J=8.24Hz), 2.67 (2H, t, J=8.24Hz), 1.42 (9H, s)

Example 129

Methyl 3-(4-(2-(N-(1-(4-acetyloxyphenyl)-3-methylbutyl)carbamoyl)ethyl)phenyl)-2-((tert-butoxy)carbonylamino) propionate (129)

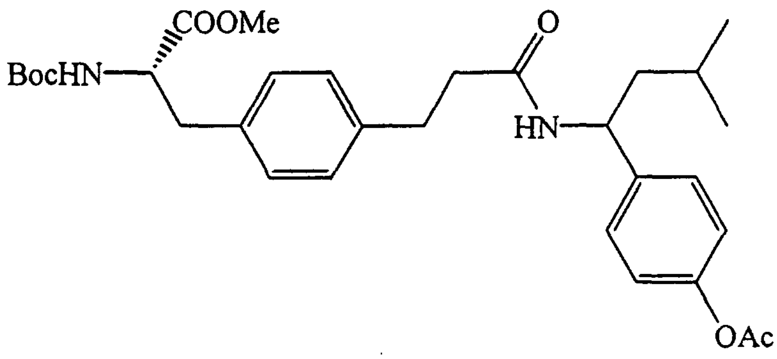

(129)

In 1.0 ml of anhydrous dichloromethane, 194 mg of 3-(4-(2-((tert-butoxy)carbonylamino)-2-(methoxycarbonyl) ethyl)phenyl)propionic acid (128) was dissolved, and 244 mg of BOP (benzotriazole-1-yl-tris(dimethylamino)phosphoniumhexafluoro phosphide salt) was added thereto, followed by stirring the mixture at room temperature for 10 minutes. Thereafter, 214 mg of 4-((1-amino-3-methyl)butyl)phenyl acetate and 0.24 ml of diisopropylamine were added, and the mixture was stirred overnight at room temperature. Water was added to the reaction solution, and the mixture was extracted with ethyl acetate (10 ml x 3). Organic layers were combined, washed with saturated brine, dried over anhydrous sodium sulfate and concentrated. The residue was purified by medium-pressure chromatography (Si-40B/ cyclohexane:ethyl acetate = 50:50) to obtain 189 mg of methyl 3-(4-(2-(N-(1-(4-acetyloxyphenyl)-3-methylbutyl)carbamoyl)ethyl)phenyl)-2-((tert-butoxy)carbonylamino)propionate (129) as colorless crystals (yield: 75%).
LR-MS(m/z):512 ($M^+$-$CO_2CH_3$+H)
IR(KBr):3378, 2956, 1750,1717,1648, 1508, 1367, 1204, 1168, 1019cm$^{-1}$
$^1$H-NMR(300MHz, $CDCl_3$, δppm):7.24-6.94 (8H, m), 5.44 (1H, d, J=7.69Hz), 5.05-4.96 (2H, m), 4.56 (1H, m), 3.72 (3H, s), 3.11-3.01 (2H, m), 2.93-2.84 (2H, m), 2.46-2.41 (2H, m), 2.30 (3H, s), 1.58-1.54 (3H, m), 1.42 (9H, s), 0.91-0.89 (6H, m)

Example 130

Methyl 3-(4-(2-(N-(1-(4-acetyloxyphenyl)-3-methylbutyl)carbamoyl)ethyl)phenyl)-2-(3,3-dimethylbutanoylamino) propionate (130)

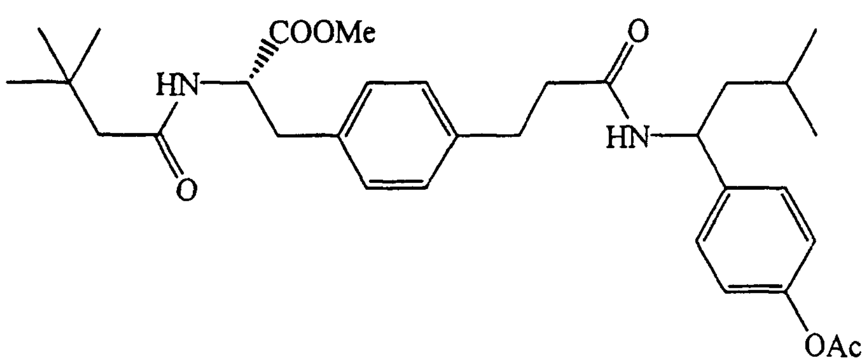

(130)

In 1.0 ml of anhydrous dichloromethane, 85.0 mg of methyl 3-(4-(2-(N-(1-(4-acetyloxyphenyl)-3-methylbutyl) carbamoyl)ethyl)phenyl)-2-((tert-butoxy)carbonylamino)propionate (129) was dissolved, and 0.5 ml of trifluoroacetic acid was added while cooling the mixture in ice, followed by stirring the mixture at 0°C for 1.5 hours. The reaction

solution was concentrated and subjected to azeotropic distillation with toluene.

The residue was dissolved in 1.5 ml of anhydrous dichloromethane, and 0.05 ml of $Et_3N$ and 0.026 ml of tert-butylacetyl chloride were added while cooling the mixture in ice, followed by stirring the mixture at room temperature for 30 minutes. To the reaction solution, 1.0 ml of 1N hydrochloric acid was added and the resulting mixture was extracted with ethyl acetate (10 ml x 3). Organic layers were combined, washed with saturated aqueous sodium hydrogen carbonate solution and saturated brine, dried over anhydrous sodium sulfate and concentrated. The residue was purified by medium-pressure chromatography (Si-40A/ cyclohexane:ethyl acetate = 50:50) to obtain 56.6 mg of methyl 3-(4-(2-(N-(1-(4-acetyloxyphenyl)-3-methylbutyl)carbamoyl)ethyl)phenyl)-2-(3,3-dimethylbutanoylamino)propionate (130) as colorless oil (yield: 67%).
LR-MS(m/z):552 ($M^+$)
IR(neat):3296, 2955, 2869, 1748, 1644, 1542, 1438, 1368, 1202cm$^{-1}$
$^1$H-NMR(300MHz, $CDCl_3$, δppm):7.24-7.21 (2H, m), 7.09-6.97 (6H, m), 5.83-5.79 (1H, m), 5.54 (1H, d, J=8.52Hz), 5.08-5.00 (1H, m), 4.91-4.82 (1H, m), 3.72 (3H, s), 3.14-3.00 (2H, m), 2.90 (2H, t, J=7.56Hz), 2.51-2.36 (2H, m),2.30 (3H, s), 2.05-2.03 (2H, m), 1.65-1.50 (2H, m), 1.47-1.36 (1H, m), 0.97 (9H, s), 0.91-0.89 (6H, m)

Example 131

3-(4-(2-(N-(1-(4-hydroxyphenyl)-3-methylbutyl)carbamoyl)ethyl)phenyl)-2-(3,3-dimethylbutanoylamino)propionic acid (131)

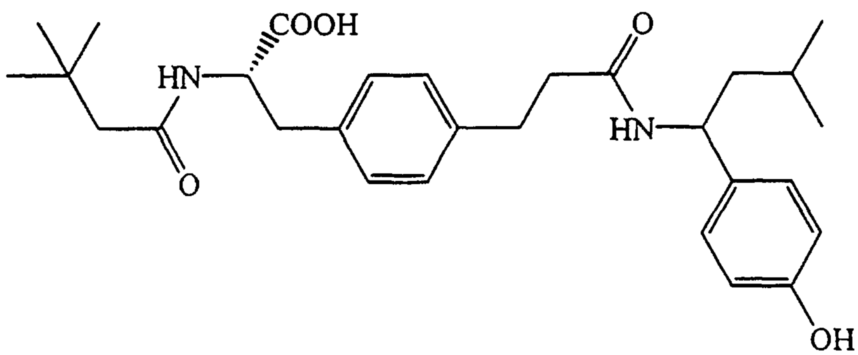

(131)

In 1.0 ml of MeOH, 56.3 mg of methyl 3-(4-(2-(N-(1-(4-acetyloxyphenyl)-3-methylbutyl)carbamoyl)ethyl)phenyl)-2-(3,3-dimethylbutanoylamino)propionate (130) was dissolved, and 0.5 ml of 1N aqueous NaOH solution was added, followed by stirring the mixture at room temperature for 20 minutes. The reaction solution was concentrated, and 5% aqueous citric acid solution was added to the residue, followed by extraction of the resulting mixture with ethyl acetate (10 ml x 3). Organic layers were combined, washed with water and saturated brine, dried over anhydrous sodium sulfate and concentrated. The residue was purified by medium-pressure chromatography (Diol-40A/ cyclohexane: ethyl acetate = 20:80) to obtain 49.4 mg of 3-(4-(2-(N-(1-(4-hydroxyphenyl)-3-methylbutyl)carbamoyl)ethyl)phenyl)-2-(3,3-dimethylbutanoylamino)propionic acid (131) as colorless crystals (yield: 98%).
mp:100-102°C
LR-MS(m/z):496 ($M^+$)
IR(KBr):3314, 2956, 1720, 1647, 1516, 1448, 1367, 1234, 835, 549cm$^{-1}$
$^1$H-NMR(300MHz, $CDCl_3$, δppm):7.18-7.06 (6H, m), 6.76-6.73 (2H, m), 4.91-4.87 (1H, m), 4.69 (1H, dd, J=4.95, 9.62Hz), 3.25-3.19 (1H, m), 2.97-2.85 (3H, m), 2.51-2.45 (2H, m), 2.07 (2H, d, J=1.10Hz), 1.67-1.57 (1H, m), 1.52-1.38 (2H, m), 0.93-0.90 (15H, m)
HR-MS:$C_{29}H_{40}N_2O_5$ Calcd.: 496.2937, Found: 496.2955

Example 132

Methyl 3-(4-(2-(N-(1-(4-acetyloxyphenyl)-3-methylbutyl)carbamoyl)ethyl)phenyl)-2-(2-(4-acetyloxyphenyl)-4-methylpentanoylamino)propionate (132)

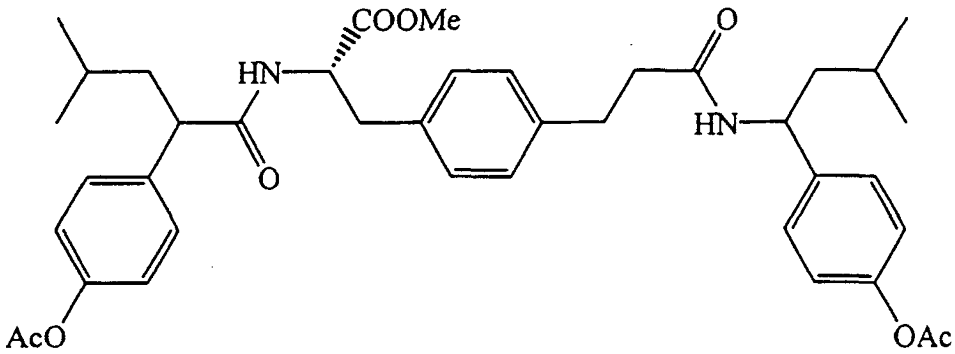

(132)

In 1.0 ml of anhydrous dichloromethane, 47.5 mg of methyl 3-(4-(2-(N-(1-(4-acetyloxyphenyl)-3-methylbutyl) carbamoyl)ethyl)phenyl)-2-((tert-butoxy)carbonylamino)propionate (129) was dissolved, and 0.5 ml of trifluoroacetic acid was added while cooling the mixture in ice, followed by stirring the mixture at 0°C for 2.5 hours. The reaction solution was concentrated and the residue was subjected to azeotropic distillation with toluene to obtain colorless solid.

In 0.5 ml of anhydrous dichloromethane, 28.2 mg of 2-(4-acetyloxyphenyl)-4-methylvaleric acid was dissolved, and 48.0 mg of BOP (benzotriazole-1-yl-tris(dimethylamino)phosphoniumhexafluoro phosphide salt) was added thereto, followed by stirring the mixture at room temperature for 10 minutes. Thereafter, the colorless solid obtained above and 0.06 ml of diisopropylamine were added and the mixture was stirred overnight at room temperature. To the reaction solution, 0.5 ml of 1N hydrochloric acid was added and the mixture was extracted with ethyl acetate (10 ml x 4). Organic layers were combined, washed with water and saturated brine, dried over anhydrous sodium sulfate and concentrated. The residue was purified by medium-pressure chromatography (Si-40A/ cyclohexane: ethyl acetate = 50:50) to obtain 38.5 mg of methyl 3-(4-(2-(N-(1-(4-acetyloxyphenyl)-3-methylbutyl)carbamoyl)ethyl)phenyl)-2-(2-(4-acetyloxyphenyl)-4-methylpentanoylamino)propionate (132) as colorless oil (yield: 65%).
LR-MS(m/z):686 ($M^+$)
IR(neat):3300,2955,2869,1748,1647,1541,1506,1437,1369,1203,1168$cm^{-1}$
$^1H$-NMR(300MHz, $CDCl_3$, δppm):7.26-6.51 (12H, m), 5.92-5.59 (2H, m), 5.07-4.98 (1H, m), 4.84-4.71 (1H, m), 3.72 (1.5H, s), 3.66 (1.5H, s), 3.44-3.36 (1H, m), 3.09-2.80 (4H, m), 2.45-2.36 (2H, m), 2.29-2.27 (6H, m), 1.98-1.87 (1H, m), 1.67-1.30 (5H, m), 0.90-0.84 (12H, m)

Example 133

3-(4-(2-(N-(1-(4-hydroxyphenyl)-3-methylbutyl)carbamoyl)phenyl)-2-(2-(4-hydroxyphenyl)-4-methylpentanoylamino) propionic acid (133)

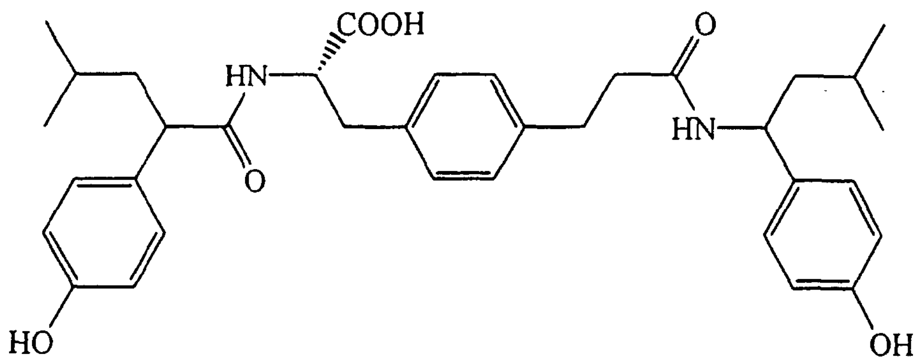

(133)

In 1.0 ml of MeOH, 36.1 mg of methyl 3-(4-(2-(N-(1-(4-acetyloxyphenyl)-3-methylbutyl)carbamoyl)ethyl)phenyl)-2-(2-(4-acetyloxyphenyl)-4-methylpentanoylamino)propionate (132) was dissolved, and 0.3 ml of 1N aqueous NaOH solution was added, followed by stirring the mixture at room temperature for 3 hours. The reaction solution was concentrated, and 5% aqueous citric acid solution was added to the residue, followed by extraction of the resulting mixture with ethyl acetate (10 ml x 3). Organic layers were combined, washed with saturated brine (10 ml x 2), dried over anhydrous sodium sulfate and concentrated. The residue was purified by medium-pressure column chromatography (Diol-40A/cyclohexane: ethyl acetate = 40:60) to obtain 30.0 mg of 3-(4-(2-(N-(1-(4-hydroxyphenyl)-3-methylbutyl)carbamoyl)phenyl)-2-(2-(4-hydroxyphenyl)-4-methylpentanoylamino)propionic acid (133) as white solid (yield: 97%).
mp:107-109°C
LR-MS(m/z):588 ($M^+$)
IR(KBr):3303, 2956, 2869, 1724, 1658, 1641, 1630, 1611, 1549, 1528, 1513, 1443, 1367, 1238$cm^{-1}$
$^1$H-NMR(300MHz, $CD_3OD$, δppm):8.21 (0.5H, d, J=9.34Hz), 8.14 (0.5H. d, J=8.79Hz), 8.03 (0.5H, d, J=8.52Hz), 7.96 (0.5H. d, J=7.69Hz), 7.11-7.00 (6H, m), 6.90-6.86 (1H, m), 6.83-6.79 (1H, m), 6.71-6.65 (4H, m), 4.83 (1H, m), 4.63-4.57 (1H, m), 3.54-3.43 (1H, m), 3.18-3.02 (1H, m), 2.93-2.76 (3H, m), 2.47-2.40 (2H, m), 1.87-1.14 (6H, m), 0.91-0.78 (12H, m)
HR-MS:$C_{35}H_{44}N_2O_6$ Calcd.: 588.3199, Found: 588.3192

Example 134

(S)-2-cyclohexyl-1-(2-(hydroxymethyl)pyrrolidinyl)ethan-1-one (134)

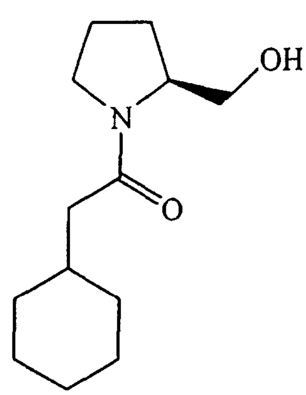

(134)

In dichloromethane, 2-cyclohexyl acetic acid (540mg, 3.65mmol, 1.2eq) was dissolved, and BOP (1.61g, 3.65mmol, 1.2eq) was added thereto, followed by stirring the mixture at room temperature for 10 minutes. After adding (S)-pyrrolidin-2-ylmethan-1-ol (0.31 g, 3.04mmol) to the reaction solution, the mixture was stirred while cooling the mixture in ice and diisopropylethylamine was added, followed by stirring the mixture at room temperature for 24 hours. To the reaction solution, 1N hydrochloric acid was added and the resulting mixture was extracted with dichloromethane. Organic layers were combined, washed with saturated aqueous sodium hydrogen carbonate solution, dried over anhydrous sodium sulfate and concentrated under reduced pressure. The residue was purified by silica gel column chromatography to obtain 701 mg of (S)-2-cyclohexyl-1-(2-(hydroxymethyl)pyrrolidinyl)ethan-1-one (134) (yield: 85%).
LR-MS(m/z):225($M^+$)
$^1$H-NMR(300MHz, $CDCl_3$, δppm): 5.27 (1H, d), 4.23 (1H, m), 3.68-3.42 (4H, m), 2.19 (2H, d), 2.10-1.51 (9H, m), 1.38-0.88 (6H, m)

Example 135

(S)-1-(2-(bromomethyl)pyrrolidinyl)-2-cyclohexylethan-1-one (135)

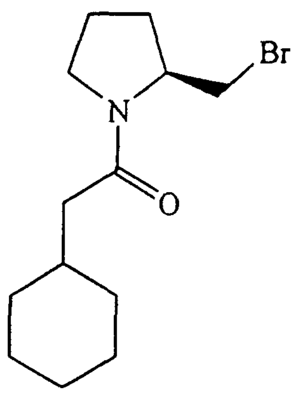

(135)

(S)-2-cyclohexyl-1-(2-(hydroxymethyl)pyrrolidinyl)ethan-1-one (134) (520mg, 2.31 mmol) was dissolved in dichloromethane, and $PPh_3$ (1.21g, 4.62mmol, 2.0eq) and $CBr_4$ (1.15g, 3.47mmol, 1.5eq) were added while cooling the mixture in ice, followed by stirring the resulting mixture at room temperature for 1 hour. Saturated aqueous sodium hydrogen carbonate solution was added to the reaction solution and the resulting mixture was extracted with ethyl acetate. Organic layers were combined, washed with saturated brine, dried over anhydrous magnesium sulfate and concentrated under reduced pressure. The residue was purified by silica gel column chromatography to obtain 565 mg of (S)-1-(2-(bromomethyl)pyrrolidinyl)-2-cyclohexylethan-1-one (135) (yield: 85%).
LR-MS (m/z): 289 ($M^+$)
IR (neat): 3418, 2924, 1740, 1635, 1448 $cm^{-1}$
NMR (300MHz, $CDCl_3$, δppm): 4.34 (1H, m), 3.77-3.46 (4H, m), 2.18 (2H, d), 2.03 (3H, m), 1.96-1.58 (7H, m), 1.38-0.88 (6H, m)

Example 136

Methyl 3-((1-(2-cyclohexylacetyl)pyrrolidin-2(S)-yl)methylthio)-2(S)-((tert-butoxy)carbonylamino)propionate (136)

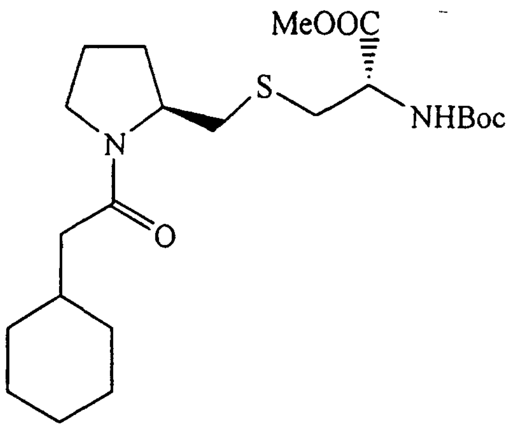

(136)

N-Boc-D-cyctein methyl ester (174mg, 0.74mmol, 1.3eq) was dissolved in 1.5 ml of THF, and potassium tert-butoxide (76mg, 0.68mmol, 1.2eq) was added thereto, followed by stirring the resulting mixture at room temperature for 10 minutes. (S)-1-(2-(bromomethyl)pyrrolidinyl)-2-cyclohexylethan-1-one (135) (130 mg, 0.57 mmol) was dissolved in 1.5 ml of THF and the mixture was stirred at room temperature for 1 hour. Saturated aqueous ammonium chloride solution was added to the reaction solution and the resulting mixture was extracted with ethyl acetate. Organic layers were combined, washed with saturated brine, dried over anhydrous magnesium sulfate and concentrated under reduced pressure. The residue was purified by silica gel column chromatography to obtain 111 mg (yield: 44%).
LR-MS (m/z): 442 ($M^+$)
$^1$H-NMR(300MHz, $CDCl_3$, δppm): 5.51 (1H, bd), 4.55 (1H, m), 4.20 (1H, m), 3.78 (3H, s), 3.54-3.38 (2H, m), 3.18-2.92 (3H, m), 2.42 (1H, dd, J=12.9, 9.6Hz), 2.12 (2H, d, J=7.1Hz), 2.00-1.58 (7H, m), 1.45 (9H, s), 1.38-0.89 (8H, m)
$[\alpha]^{27}_D$ = -60.1° (c=0.65,$CHCl_3$)

Example 137

Methyl 2(S)-amino-3-((1-(2-cyclohexylacetyl)pyrrolidin-2(S)-yl)methylthio)propionate (137)

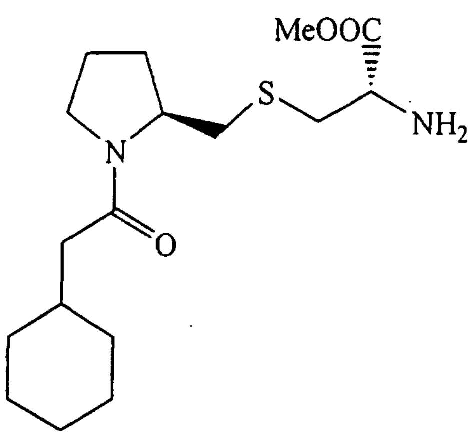

(137)

In 4 ml of dichloromethane, 111 mg of methyl 3-((1-(2-cyclohexylacetyl)pyrrolidin-2(S)-yl)methylthio)-2(S)-((tert-butoxy)carbonylamino)propionate (136) was dissolved and 0.4 ml of TFA was added thereto, followed by stirring

the mixture at room temperature for 2 hours. Saturated aqueous sodium hydrogen carbonate solution was added to the reaction solution and the mixture was extracted with dichloromethane. Organic layers were combined, washed with saturated brine, dried over anhydrous magnesium sulfate and concentrated under reduced pressure to obtain 67 mg of methyl 2(S)-amino-3-((1-(2-cyclohexylacetyl)pyrrolidin-2(S)-yl)methylthio)propionate (137) (78%). The product (137) was used in the next reaction without purification.

Example 138

Methyl 3-((1-(2-cyclohexylacetyl)pyrrolidin-2(S)-yl)methylthio)-2(S)-(4-methyl-2-(S)-(phenylmethoxy)pentanoylamino) propionate (138)

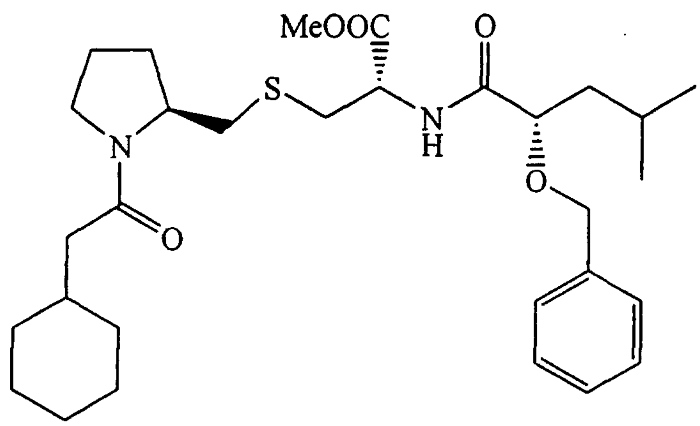

(138)

In 2 ml of DMF, methyl 2(S)-amino-3-((1-(2-cyclohexylacetyl)pyrrolidin-2(S)-yl)methylthio)propionate (137) (67mg, 0.2mmol) and methyl (S)-2-phenylmethoxy-4-methylvalerate (43mg, 0.2mmol) were dissolved, and PyBOP (118mg, 0.23mmol) and N-methylmorpholine (25mg, 0.25mmol) were added thereto, followed by stirring the mixture at room temperature for 2 hours. To the reaction solution, 1N hydrochloric acid was added and the mixture was extracted with ethyl acetate. Organic layers were combined, washed with saturated aqueous sodium hydrogen carbonate solution and saturated brine, dried over anhydrous magnesium sulfate and concentrated under reduced pressure. The residue was purified by silica gel column chromatography to obtain 107 mg of methyl 3-((1-(2-cyclohexylacetyl)pyrrolidin-2(S)-yl)methylthio)-2(S)-(4-methyl-2-(S)-(phenylmethoxy)pentanoylamino)propionate (138) (yield: 98%).
LR-MS (m/z): 546 ($M^+$)
IR(neat): 3410, 3298, 2924, 1747, 1633, 1514, 1415, 1327, 1209, 1089, 1027 $cm^{-1}$ $^1$H-NMR(300MHz, $CDCl_3$, δppm): 7.41-7.26 (5H, m), 4.83 (1H, m), 4.70 (1H, d, J=11.3Hz), 4.45 (1H, d, J=11.3Hz), 4.18 (1H, m), 3.92 (1H, m), 3.76 (3H, s), 3.42 (2H, m), 3.20-3.02 (2H, m), 2.94 (1H, dd), 2.43 (1H, dd, J=12.9, 9.3Hz), 2.08 (2H, m), 2.00-1.50 (11H, m), 1.48-1.04 (8H, m), 1.86 (6H, dd, J=6.6Hz)
$[\alpha]^{27}_D$ = -62.72° (c=0.72, $CHCl_3$)

Example 139

3-((1-(2-cyclohexylacetyl)pyrrolidin-2(S)-yl)methylthio)-2(S)-(4-methyl-2(S)-(phenylmethoxy)pentanoylamino) propionic acid (139)

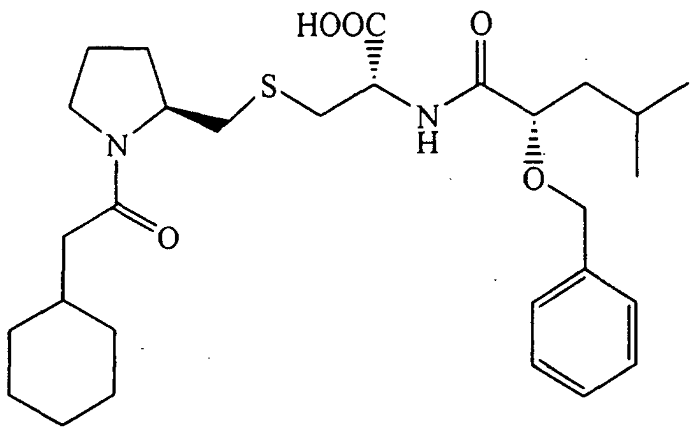

(139)

Methyl 3-((1-(2-cyclohexylacetyl)pyrrolidin-2(S)-yl)methylthio)-2(S)-(4-methyl-2-(S)-(phenylmethoxy)pentanoylamino)propionate (138) (90 mg, 0.16 mmol) was dissolved in 2 ml of methanol, and 1ml of 1N aqueous sodium hydroxide solution was added thereto, followed by stirring the mixture at room temperature for 1 hour. To the mixture, 1N hydrochloric acid was added and the resulting mixture was extracted with ethyl acetate. Organic layers were combined, washed with saturated brine, dried over anhydrous magnesium sulfate and concentrated under reduced pressure. The residue was purified by silica gel column chromatography to obtain 96 mg of 3-((1-(2-cyclohexylacetyl)pyrrolidin-2(S)-yl)methylthio)-2(S)-(4-methyl-2(S)-(phenylmethoxy)pentanoylamino)propionic acid (139) (yield: 81%).
LR-MS (m/z):533 $(M+H)^+$
IR(KBr): 3403, 2925, 1733, 1632, 1516, 1453, 1328, 1208, 1089, 917, 734 $cm^{-1}$
$^1$H-NMR(300MHz, $CDCl_3$, δppm): 7.43-7.28 (5H, m), 5.01-4.82 (1H, m), 4.75 (1H, d), 4.46-4.20 (3H, m), 3.90 (1H, m), 3.58-3.22 (4H, m), 3.03 (2H, m), 2.95 (2H, m), 2.45 (1H, dd), 2.14 (3H, m), 2.00-1.50 (9H, m), 1.36-1.00 (6H, m), 0.86 (6H, dd, J=4.6Hz)
$[\alpha]^{27}_D$ = -82.63° (c=1.09, $CHCl_3$)

Example 140

3-((2(R)-carboxy-2(R)-(3,3-dimethylbutanoylamino)ethyl)disulfanyl)-2(R)-(3,3-dimethylbutanoylamino)propionic acid (140)

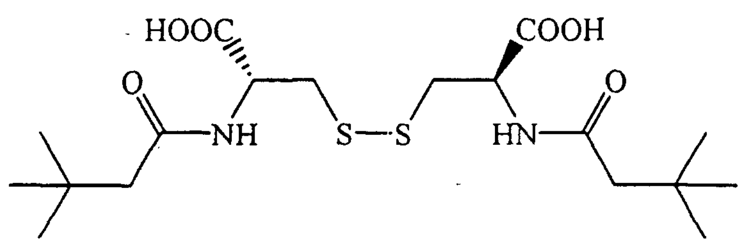

(140)

To 1.00g (4.19mmol) of L-cystine, 10 ml of 1,4-dioxane and 20 ml of 10% aqueous sodium carbonate solution were added to dissolve the L-cystine. Under cooling in ice, 2.4ml(17.3mmol) of t-butylacetyl chcloride dissolved in 15 ml of 1,4-dioxane was added to the mixture and the resulting mixture was stirred at room temperature for 3 hours. Water and ether were added to the reaction solution, and the aqueous layer, after separation, was acidified with 6N

hydrochloric acid, followed by extraction of the resulting mixture with ethyl acetate. Organic layers were combined, washed with saturated brine, dried over anhydrous magnesium sulfate and concentrated under reduced pressure. The residue was suspended in ether, and the obtained suspension was filtered to obtain 914 mg of 3-((2(R)-carboxy-2(R) -(3,3 -dimethylbutanoylamino)ethyl)disulfanyl)-2(R)-(3,3-dimethylbutanoylamino)propionic acid as colorless crystals (yield: 50%).
LR-MS(m/z):437$(M+H)^+$
IR(KBr):3300-2500, 3348, 2858, 1729 $cm^{-1}$
$^1$H-NMR (300MHz, $CDCl_3$ δppm) : 1.05(18H, s), 2.14(4H, s), 3.23(2H, dd, J=4.5,14.0Hz),3.30(2H, dd, J=5.5, 14.0Hz), 4.78-4.84(2H, m), 6.61(2H, d, J=7.5Hz)
HR-MS:$C_{18}H_{33}N_2O_6S_2$ Calcd.: 437.1780, Found: 437.1742
$[\alpha]^{25}_D$: -168° (c=1.06, MeOH)

Example 141

5-N-Boc-amino-2-((2,6-dichlorophenyl)carbonylamino)pentanoic acid methyl ester (141)

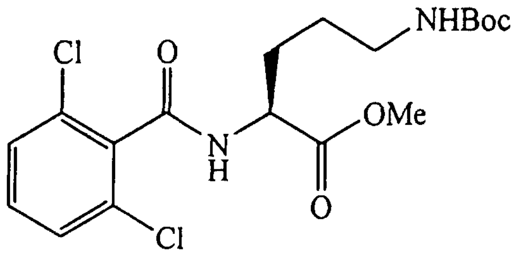

(141)

Under cooling in ice, 0.8 ml (5.73 mmol) of triethylamine and 547 ml (3.82 mmol) of dichlorophenylbenzyl chloride were added to a solution of 472 mg (1.91 mmol) of (S)-2-amino-5-N-Boc-aminopentanoic acid methyl ester in dichloromethane (5 ml), and the mixture was stirred overnight at room temperature. To the reaction solution, 20 ml of 1N hydrochloric acid was added while cooling the mixture in ice and the resulting mixture was extracted with ethyl acetate (70 ml x 2). Organic layers were combined, washed with saturated brine (100 ml), dried over anhydrous sodium sulfate and concentrated. The residue was purified by column chromatography (silica gel 30 g; hexane: ethyl acetate = 2:1→1:2) to obtain 636.2 mg (yield: 79%) of 5-N-Boc-amino-2-((2,6-dichlorophenyl)carbonylamino)pentanoic acid methyl ester as colorless oil.
LR-MS(m/z):418$(M^+)$
IR(neat):3292,3068,2977,2871,1743,1660,1580,1531,1433,1391,1366,1252,1171,10 90,1015,871,785$cm^{-1}$
$^1$H-NMR(300MHz,$CDCl_3$, δppm): 1.43(9H,S), 1.52-1.70(2H,m), 1.73-1.88(1H,m), 2.01-2.12(1H,m), 3.10-3.23(2H,m), 3.81(3H,s), 4.62(1H,brs), 4.82-4.92(1H,m), 6.54(1H,brs), 7.24-7.37(3H,m)

Example 142

2-((2,6-dichlorophenyl)carbonylamino)-5-((2,6-dimethoxyphenyl)carbonylamino)pentanoic acid methyl ester (142)

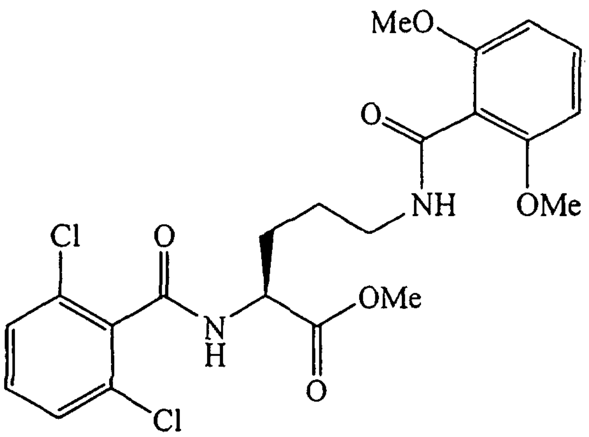

(142)

To a solution of 552.6 mg (1.32 mmol) of 5-N-Boc-amino-2-((2,6-dichlorophenyl)carbonylamino)pentanoic acid methyl ester in dichloromethane (2 ml), 1 ml of trifluoroacetic acid was added and the mixture was stirred at room temperature for 1 hour, followed by concentrating the mixture. The residue was twice subjected to azeotropic distillation with toluene, and dissolved in dichloromethane (4 ml). To the mixture, 288.5 mg (1.58 mmol) of 2,6-dimethoxybenzoic acid, 700 mg (1.58 mmol) of BOP and 0.92 ml (5.28 mmol) of i-$Pr_2NEt$ were added, and the resulting mixture was stirred overnight at room temperature. To the reaction solution, 1N hydrochloric acid was added and the mixture was extracted with ethyl acetate (70 ml x 2). Organic layers were combined, washed with aqueous sodium hydrogen carbonate solution (70 ml) and saturated brine (70 ml), and concentrated. The residue was purified by column chromatography (silica gel 40 g; hexane: ethyl acetate = 1:2→1:4) to obtain 539 mg (yield: 85%) of 2-((2,6-dichlorophenyl) carbonylamino)-5-((2,6-dimethoxyphenyl)carbonylamino)pentanoic acid methyl ester as an amorphous product.
LR-MS(m/z):482($M^+$)
IR(KBr):3409,3279,3070,2948,1739,1653,1595,1 538,1472,1435,1304,1253,1111, 846,788$cm^{-1}$
$^1H$-NMR(300MHz,$CDCl_3$, δppm): 1.70-1.85(2H,m), 1.86-1.99(1H,m), 2.13-2.23(1H,m), 3.50-3.58(2H,m), 3.81(9H,S), 4.85-4.92(1H,m), 5.86(1H,brs), 6.53(2H,d,J=8.5), 6.56(1H,brs), 7.21-7.34(4H,m)

Example 143

2-((2,6-dichlorophenyl)carbonylamino)-5-((2,6-dimethoxyphenyl)carbonylamino)pentanoic acid (143)

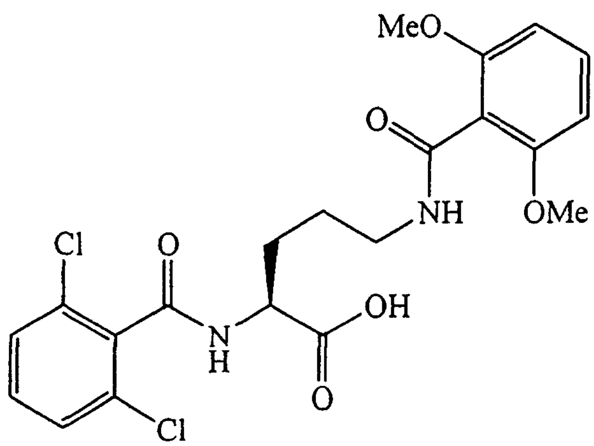

(143)

In 5 ml of a mixed solvent of methanol:THF=1:1, 145 mg (0.3 mmol) of 2-((2,6-dichlorophenyl)carbonylamino)-5-((2,6-dimethoxyphenyl)carbonylamino)pentanoic acid methyl ester was dissolved, and 0.9 ml of 1N aqueous sodium hydroxide solution was added, followed by stirring the mixture overnight at room temperature. To the reaction solution, 1N hydrochloric acid was added and the mixture was extracted with ethyl acetate (50 ml x 2). Organic layers were combined, washed with saturated brine (70 ml), and concentrated. The residue was purified by medium-pressure liquid chromatography (Yamazen Ultrapack DIOL-40A; hexane:ethyl acetate = 25:75→5:95) to quantitatively obtain 140 mg of 2-((2,6-dichlorophenyl)carbonylamino)-5-((2,6-dimethoxyphenyl)carbonylamino)pentanoic acid as an amorphous product.
$[\alpha]_D^{23}$=-107° (c=0.1,MeOH)
LR-MS(m/z):468($M^+$)
IR(KBr):3428,2934,1653,1599,1472,1434,1304,1253,1111,846$cm^{-1}$
$^1$H-NMR(300MHz,$CDCl_3$, δppm): 1.70-1.99(3H,m), 2.11-2.26(1H,m), 3.40-3.62(2H,m), 3.77(6H,S), 4.80-4.90(1H,m), 6.13(1H,brs), 6.50(2H,d,J=8.5), 6.87(1H,brs), 7.18-7.34(4H,m)
HR-MS :$C_{21}H_{22}Cl_2N_2O_6$ Calcd.: 468.0855, Found: 468.0840

Example 144

2,5-di((2,6-dichlorophenyl)carbonylamino)pentanoic acid methyl ester (144)

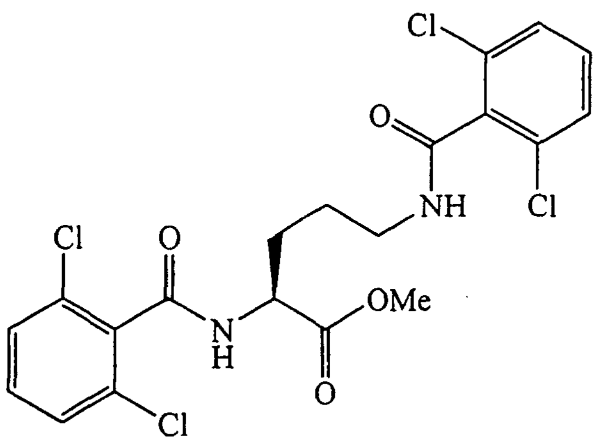

(144)

To a solution of 162.4 mg (0.39 mmol) of 5-N-Boc-amino-2-((2,6-dichlorophenyl)carbonylamino)pentanoic acid methyl ester in dichloromethane (2 ml), 0.5 ml of trifluoroacetic acid was added, and the mixture was stirred at room temperature for 1 hour, followed by concentrating the mixture. The residue was twice subjected to azeotropic distillation with toluene, and then dissolved in dichloromethane (6 ml). To the mixture, 0.14ml (0.98mmol) of triethylamine and 0.07 ml (0.46 mmol) of 2,6-dichlorobenzoyl chloride were added, and the resulting mixture was stirred at room temeprature for 30 minutes. To the reaction solution, 1N hydrochloric acid was added and the mixture was extracted with ethyl acetate (10 ml x 2). Organic layers were combined, washed with aqueous sodium hydrogen carbonate solution (70 ml) and saturated brine (70 ml), and concentrated. The residue was washed with ether (5 ml x 3) to obtain 165.7mg (0.34mmol) of 2-((2,6-dichlorophenyl)carbonylamino)-5-((2,6-dichlorophenyl)carbonylamino)pentanoic acid methyl ester as an amorphous product (yield: 85%).
$[\alpha]_D^{23}$=-28.5 ° (C=0.42,MeOH)
LR-MS(m/z):492($M^+$)
IR(KBr):3270,3079,2954,1740,1652,1555,1432,1363,1294,1195,803$cm^{-1}$
$^1$H-NMR(300MHz,$CDCl_3$,δppm): 1.65-1.94(3H.m), 2.04-2.18(1H,m), 3.39-3.46(2H,m), 3.74(3H,s), 4.68-4.74(1H,m), 6.30(1H,brs), 6.44(2H,d,J=8.5), 7.10-7.28(5H,m)

Example 145

2,5-di((2,6-dichlorophenyl)carbonylamino)pentanoic acid (145)

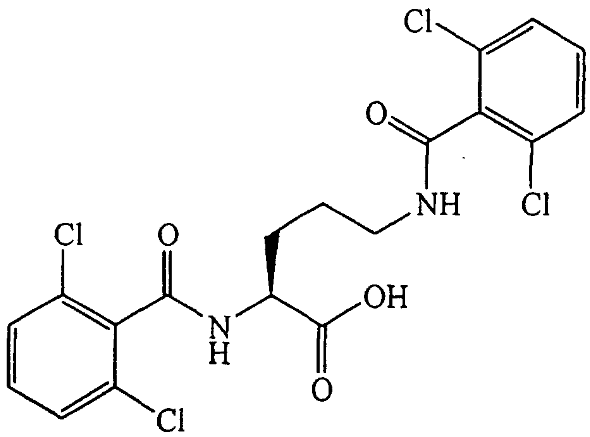

(145)

In methanol (4 ml), 100.7 mg (0.2 mmol) of 2-((2,6-dichlorophenyl)carbonylamino)-5-((2,6-dichlorophenyl) carbonylamino)pentanoic acid methyl ester was dissolved, and 0.6 ml of 1N aqeuous sodium hydroxide solution was added, followed by stirring the mixture at room temperature for 1 hour. To the reaction solution, 1N hydrochloric acid was added and the mixture was extracted with chloroform (10 ml x 2). Organic layers were combined, washed with saturated brine (5 ml), and concentrated to quantitatively obtain 2-((2,6-dichlorophenyl)carbonylamino)-5-((2,6-dichlorophenylphenyl)carbonylamino)pentanoic acid.
$[\alpha]_D^{23}$=-19.3 ° (C=0.30.MeOH)
LR-MS(m/z):477 $(M+H)^+$
IR(KBr):3408,3272,3080,2933,1730,1653,1584,1558,1432,1304,1195,802,782$cm^{-1}$
$^1$H-NMR(300MHz,$CDCl_3$, δppm): 1.80-2.06(3H.m), 2.11-2.23(1H,m), 3.48-3.60(2H,m), 4.77-5.02(1H,m), 7.05(1H,brs), 7.13(1H,brs), 7.21-7.37(6H,m)
HR-MS: $C_{19}H_{16}Cl_2N_2O_4$ Calcd.: 475.9864, Found: :474.9762 $(M-H)^-$

Example 146

6-N-Boc-amino-2-((2,6-dichlorophenyl)carbonylamino)hexanoic acid methyl ester (146)

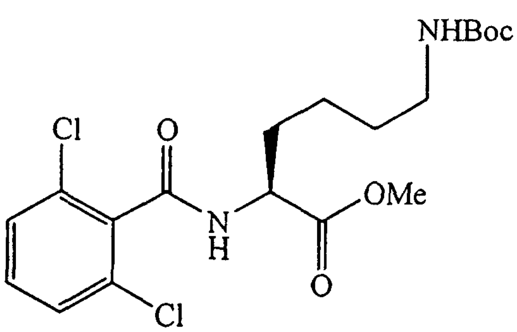

(146)

Under cooling in ice, 0.8 ml (5.76 mmol) of triethylamine and 410 ml (2.88 mmol) of dichlorophenylbenzyl chloride were added to a solution of 797 mg (3.24 mmol) of (S)-2-amino-6-N-Boc-aminohexanoic acid methyl ester in dichloromethane (5 ml), and the mixture was stirred for 2.5 hours. Under cooling in ice, 20 ml of saturated aqueous sodium hydrogen carbonate solution was added to the reaction solution, and the mixture was extracted with ethyl acetate (50 ml x 3). Organic layers were combined, washed with saturated brine (30 ml x 3), dried over anhydrous sodium sulfate and concentrated. The residue was purified by column chromatography (silica gel 50 g, hexane: ethyl

acetate = 2:1) to obtain 1.25 g of 6-N-Boc-amino-2-((2,6-dichlorophenyl)carbonylamino)hexanoic acid methyl ester as colorless oil (yield: 89%).
LR-MS(m/z):432($M^+$)
IR(neat):3284,3066,2952,2866,1740,1660,1580,1531,1433,1365,1251,1171,1012,80 2,782$cm^{-1}$
$^1$H-NMR(300MHz,$CDCl_3$,δppm): 1.41(9H,S), 1.48-1.59(4H,m), 1.78-1.92(1H,m), 1.97-2.08(1H,m), 3.05-3.18(2H,m), 3.80(3H,s), 4.57(1H,brs), 4.82-4.90(1H,m), 6.46(1H,brs), 7.24-7.37(3H,m)

Example 147

2-((2,6-dichlorophenyl)carbonylamino)-6-((2,6-dimethoxyphenyl)carbonylamino)hexanoic acid methyl ester (147)

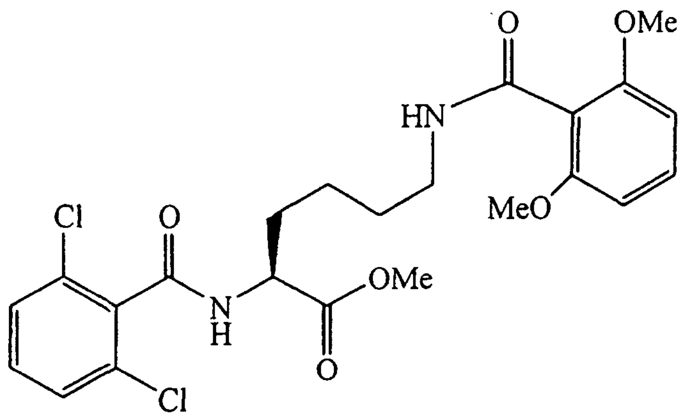

(147)

To a solution of 1.24 g (2.86 mmol) of6-N-Boc-amino-2-((2,6-dichlorophenyl)carbonylamino)hexanoic acid methyl ester in dichloromethane (5 ml), 2 ml of trifluoroacetic acid was added and the mixture was stirred at room temperature for 1 hour, followed by concentration of the resulting mixture. The residue was twice subjected to azeotropic distillation with toluene, and then dissolved in dichloromethane (10 ml). To the mixture, 574 mg (3.15 mmol) of 2,6-dimethoxybenzoic acid, 1.52g (3.43 mmol) of BOP and 1.99 ml (11.4 mmol) of i-$Pr_2$NEt were added, and the resulting mixture was stirred overnight at room temperature. To the reaction solution, 1N hydrochloric acid was added and the mixture was extracted with ethyl acetate (50 ml x 3). Organic layers were combined, washed with saturated aqueous sodium hydrogen carbonate solution (30 ml x 3) and saturated brine (30 ml x 3), and concentrated. The residue was purified by column chromatography (silica gel 60 g, hexane: ethyl acetate = 1:1→1:2→1:3) to obtain 923 mg of 2-((2,6-dichlorophenyl)carbonylamino)-6-((2,6-dimethoxyphenyl)carbonylamino)hexanoic acid methyl ester as an amorphous product (yield: 65%).
$[\alpha]^D_{23}$=-193.8° (c=0.004,MeOH)
LR-MS(m/z):496($M^+$)
IR(KBr):3272,3065,2946,1739,1651,1595,1540,1472,1434,1253,1112,845,787$cm^{-1}$
$^1$H-NMR(300MHz,$CDCl_3$,δppm): 1.23-1.72(4H,m), 1.82-1.95(1H,m), 2.01-2.13(1H,m), 3.42-3.50(2H,m), 3.81(9H,s), 4.82-4.91(1H,m), 5.74(1H,brs), 6.50(1H,brs), 6.53(2H,d,J=8.5), 7.21-7.33(4H,m)

Example 148

2-((2,6-dichlorophenyl)carbonylamino)-6-((2,6-dimethoxyphenyl)carbonylamino)hexanoic acid (148)

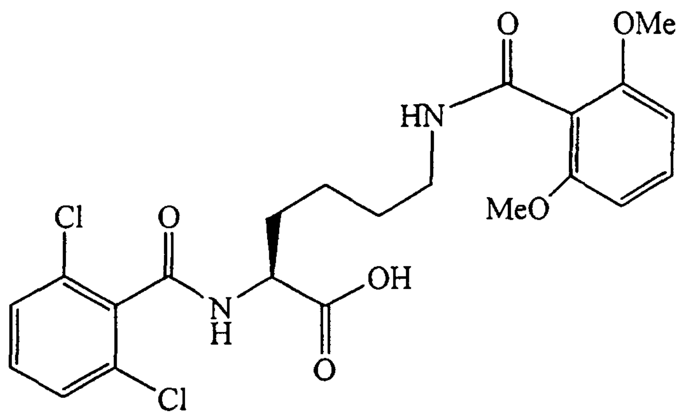

(148)

In 5 ml of methanol, 915 mg (1.84 mmol) of 2-((2,6-dichlorophenyl)carbonylamino)-6-((2,6-dimethoxyphenyl) carbonylamino)hexanoic acid methyl ester was dissolved, and 5.5 ml (5.52 mmol) of 1N aqueous sodium hydroxide solution was added, followed by stirring the mixture overnight at room temperature. To the reaction solution, 1N hydrochloric acid was added and the mixture was extracted with ethyl acetate (50 ml x 3). Organic layers were combined, washed with saturated brine (30 ml x 3), and concentrated. The residue was purified by medium-pressure column chromatography (Yamazen Ultrapack DIOL-40A; hexane: ethyl acetate = 10:90→1:99) to quantitatively obtain 899 mg of 2-((2,6-dichlorophenyl)carbonylamino)-6-((2,6-dimethoxyphenyl)carbonylamino)hexanoic acid as an amorphous product.
$[\alpha]_D^{23}$=-44.1 ° (c=0.018,MeOH)
LR-MS(m/z):482($M^+$)
IR(KBr):3302,2940,1730,1655,1598,1542,1472,1433,1306,1253,1112,787$cm^{-1}$
$^1$H-NMR(300MHz,$CDCl_3$,δppm): 1.38-1.90(4H,m), 1.90-2.18(2H,m), 3.43-3.52(2H,m), 3.78(6H,s), 4.77-4.83(1H,m), 5.98(1H,brs), 6.54(2H,d,J=8.5), 7.00-7.38(5H,m)
HR-MS:$C_{22}H_{24}Cl_2N_2O_6$ Calcd.: 482.1011, Found: 482.1019

Example 149

Ethyl 3-(4-(2-(ethoxycarbonyl)-2-nitroethyl)phenyl)-2-nitropropionate (149)

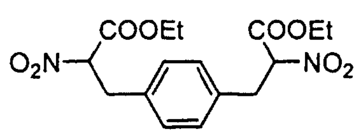

(149)

A solution of 2.26 ml (24.0 mmol) of ethyl nitro acetate in 30 ml of THF was cooled to 0°C, and 2.69 g (24.0 mmol) of potassium tert-butoxide and 264 mg (1.00 mmol) of 18-crown-6-ether were added, followed by stirring the mixture for 30 minutes. To this solution, a solution of 2.64g (10.0 mmol) of 1,4-bis(bromomethyl)benzene in 20 ml of THF was added, and the resulting mixture was heated to reflux for 1 hour. To the reaction solution, 1N hydrochloric acid was added and the mixture was extracted with ethyl acetate. Organic layers were combined, washed with saturated brine, dried over anhydrous sodium sulfate and concentrated. The residue was purified by column chromatography (silica gel; toluene/ethyl acetate = 50/1-10/1) to obtain 475 mg of ethyl 3-(4-(2-(ethoxycarbonyl)-2-nitroethyl)phenyl)-2-nitropropionate (149) as colorless oil (yield: 13%).
LR-MS (m/z):368($M^+$)
IR(KBr) : 3405, 3264, 3074, 2961, 2933, 1740, 1639, 1581, 1561, 1523, 1432, 1406, 1219, 1195, 1117, 1086 $cm^{-1}$

$^1$H-NMR (300MHz, $CDCl_3$, δppm) : 7.86 (2H, d, *J* = 8.0 Hz), 7.41 (2H, d, *J* = 8.0 Hz), 5.41-5.26 (2H, m), 4.36-4.22 (4H, m), 3.71-3.40 (4H, m), 1.33-1.24 (6H, m)

Example 150

Ethyl 2-((2,6-dichlorophenyl)carbonylamino)-3-(4-(2-((2,6-dichlorophenyl)carbonylamino)-2-(ethoxycarbonyl)ethyl) phenyl)propionate (150)

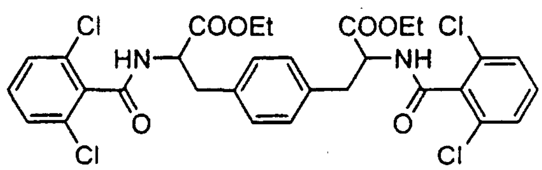

(150)

To a solution of 473 mg (1.28 mmol) of ethyl 3-(4-(2-(ethoxycarbonyl)-2-nitroethyl)phenyl)-2-nitropropionate (149) in ethanol, 100 mg of platinum oxide was added and the mixture was stirred under hydrogen atmosphere at room temperature for 2 hours. After evaporating the solvent, 2.56 ml of 1N hydrochloric acid/methanol solution was added to the residue, and the mixture was concentrated. The residue was dissolved in 5.0 ml of dichloromethane, and then 0.45 ml (3.18 mmol) of triethylamine and 0.182 mmol (1.27 mmol) of 2,6-dichlorobenzoyl chloride were added, followed by stirring the mixture overnight at room temperature. To the reaction mixture, 1N hydrochloric acid was added and the mixture was extracted with ethyl acetate. Organic layers were combined, washed with saturated brine, dried over anhydrous sodium sulfate and concentrated. The residue was purified by column chromatography (silica gel; hexane/ethyl acetate = 2/1-2/3) to obtain 50.2 mg of ethyl 2-((2,6-dichlorophenyl)carbonylamino)-3-(4-(2-((2,6-dichlorophenyl) carbonylamino)-2-(ethoxycarbonyl)ethyl)phenyl)propionate (150) as colorless oil (yield: 15%).
LR-MS (m/z) : 651 [$(M-H)^-$]
$^1$H-NMR (300MHz, $CDCl_3$, δppm) : 7.34-7.16 (10H, m), 6.30 (2H, br.d, *J* = 8.0 Hz), 5.18-5.04 (2H, m), 4.22-4.06 (4H, m), 3.28-3.14 (4H, m), 1.30-1.18 (6H, m)

Example 151

2-((2,6-dichlorophenyl)carbonylamino)-3-(4-(2-((2,6-dichlorophenyl)carbonylamino)-2-carboxyethyl)phenyl)propionic acid (151)

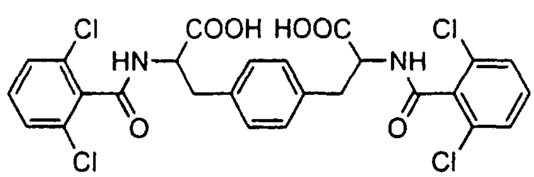

(151)

To a solution of 50.2 mg (0.077 mmol) of ethyl 2-((2,6-dichlorophenyl)carbonylamino)-3-(4-(2-((2,6-dichlorophenyl)carbonylamino)-2-(ethoxycarbonyl)ethyl)phenyl)propionate (150) in 1.5 ml of methanol/THF (2/1), 0.5 ml of 1N aqueous sodium hydroxide solution was added, and the mixture was stirred overnight at room temperature. To the reaction mixture, 1N hydrochloric acid was added and the mixture was extracted with ethyl acetate. Organic layers were combined, washed with saturated brine, dried over anhydrous sodium sulfate and concentrated under reduced pressure. The residue was purified by column chromatography (DIOL; hexane/ethyl acetate = 20/80-5/95) to obtain 35.8 mg of 2-((2,6-dichlorophenyl)carbonylamino)-3-(4-(2-((2,6-dichlorophenyl)carbonylamino)-2-carboxyethyl)phenyl)propionic acid (151) as an amorphous product (yield: 78%).
LR-MS (m/z) : 595 ($(M-H)^-$]
IR (neat) : 3405, 3264, 3074, 2961, 2933, 1740, 1639, 1581, 1561, 1523, 1432, 1406, 1219, 1195, 1117, 1086, 890 $cm^{-1}$
$^1$H-NMR (300MHz, $CDCl_3$+DMSO-d6, δppm) : 7.34-7.20 (10H, m), 7.00 (2H, br.d, *J* = 8.0 Hz), 5.06-4.96 (2H, m), 3.30-3.16 (4H, m)

Example 152

2,7-bis(1-aza-2,2-diphenylvinyl)octanedioic acid dimethyl ester (152)

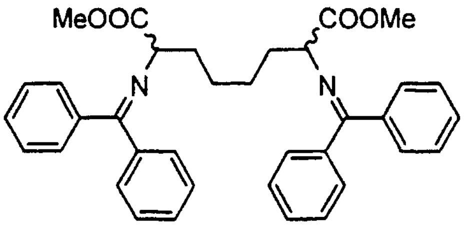

(152)

A mixture of 254 mg (1.00 mmol) of glycine methyl ester diphenylmethylimine, 27.3 mg (0.100 mmol) of benzyltriethyl ammonium bromide and 27.3 mg (0.100 mmol) of cesium hydroxide monohydrate was dissolved in 5.0 ml of dichloromethane, and 0.066 ml (0.500 mmol) of 1,4-diiodobutane was added, followed by stirring the mixture at 0°C for 6 hours. The reaction mixture was diluted with 150 ml of ether, and the resulting mixture was washed with water and saturated brine, followed by drying over anhydrous sodium sulfate and concentration under reduced pressure. The residue was purified by column chromatography (silica gel; hexane/ethyl acetate = 90/10-70/30) to obtain 68.8 mg of 2,7-bis(1-aza-2,2-diphenylvinyl)octanedioic acid dimethyl ester (152) as colorless oil (yield: 24%).
LR-MS (m/z) : 559 $[(M-H)^-]$
IR (neat) : 3058, 2950, 2858, 1739, 1623, 1597, 1577, 1491, 1445, 1315, 1286, 1199, 1157, 1074, 1028 $cm^{-1}$.
$^1H$-NMR (300MHz, $CDCl_3$, δppm): 7.62 (4H, d, *J* = 7.0 Hz), 7.44-7.28 (12H, m), 7.15-7.09 (4H, m), 4.02 (1H, t, *J* = 6.3 Hz), 4.01 (1H, t, *J* = 6.3 Hz), 3.69 (3H, s), 3.69 (3H, s), 1.91-1.81 (4H,m), 1.29-1.01 (4H, m).

Example 153

2,7-bis((2,6-dichlorophenyl)carbonylamino)octanedioic acid dimethyl ester (153)

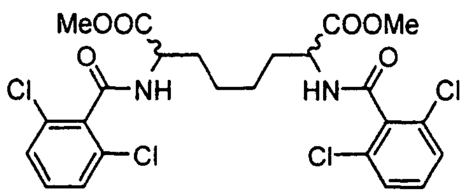

(153)

To a solution of 65.8 mg (0.177 mmol) of 2,7-bis(1-aza-2,2-diphenylvinyl)octanedioic acid dimethyl ester (152) in 2.5 ml of ether, 0.50 ml (0.50 mmol) of 1N hydrochloric acid was added and the mixture was stirred at room temperature for 28 hours. To the reaction solution, 5 ml of water was added and the mixture was shaken with ether. After freeze-drying the aqueous layer, the residue was dissolved in 3.0 ml of dichloromethane, and then 0.15 ml (1.08 mmol) of triethylamine and 0.060 ml (0.421 mmol) of 2,6-dichlorobenzoyl chloride were added, and the mixture was stirred overnight at room temperature. To the reaction mixture, 8 ml of saturated aqueous sodium hydrogen carbonate solution was added and the mixture was extracted with ethyl acetate. Organic layers were combined, washed with water, 1N hydrochloric acid, water and with saturated brine, dried over anhydrous sodium sulfate and concentrated under reduced pressure. The residue was purified by column chromatography (silica gel; cyclohexane/ethyl acetate = 70/30-40/60) to obtain 60.6 mg of 2,7-bis((2,6-dichlorophenyl)carbonylamino)octanedioic acid dimethyl ester (153) as colorless oil (yield: 90%).
LR-MS (m/z) : 575 $[(M-H)^-]$
IR (neat): 3273, 3070, 2952, 2862, 1739, 1655, 1580, 1561, 1542, 1432, 1359, 1269, 1196, 1173,1088, 1015 $cm^{-1}$.
$^1H$-NMR (300MHz, $CDCl_3$, δppm): 7.36-7.23 (6H, m), 6.46-6.38 (2H, m), 4.91-4.82 (2H, m), 3.81 (3H, s), 3.80 (3H, s), 2.10-1.96 (2H,m), 1.90-1.85 (2H,m), 1.62-1.48 (4H, m).

Example 154

2,7-bis((2,6-dichlorophenyl)carbonylamino)octanedioic acid (154)

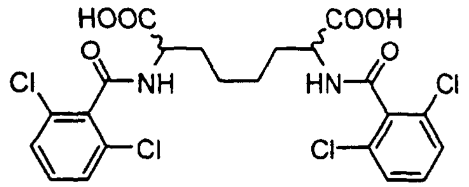

(154)

To a solution of 60.6 mg (0.106 mmol) of 2,7-bis((2,6-dichlorophenyl)carbonylamino)octanedioic acid dimethyl ester (153) in 2.0 ml of methanol, 1.5 ml of 1N aqueous sodium hydroxide solution was added and the mixture was stirred overnight at room temperature. To the reaction mixture, 6.6 ml of 0.25N hydrochloric acid was added and the mixture was extracted with ethyl acetate. Organic layers were combined, washed with saturated brine, dried over anhydrous sodium sulfate and concentrated under reduced pressure. The residue was purified by preparative HPLC [Mighty Sil RP-18 250-20
(5mm) ;TFA/methaol/water=0.1/50/50] to obtain 47.1 mg of 2,7-bis((2,6-dichlorophenyl)carbonylamino)octanedioic acid (154) as colorless oil (yield: 81%).
LR-MS (m/z) : 547 $[(M-H)^-]$
IR (neat) : 3654, 3410, 3273, 3079, 2935, 2863, 1736, 1654, 1583, 1522, 1432, 1312, 1195, 1086, 1020cm$^{-1}$
$^1$H-NMR (300MHz, CD3OD, δppm) : 7.45-7.33 (6H, m), 4.67-4.57 (2H, m), 2.03-1.89 (2H, m), 1.89-1.73 (2H, m), 1.68-1.50 (4H, m)
HR-MS : $C_{22}H_{19}Cl_4N_2O_6$ $[(M-H)^-]$ Calcd.: 546.9997, Found: 547.0026.

Example 155

2,8-bis(1-aza-2,2-diphenylvinyl)nonanedioic acid dimethyl ester (155)

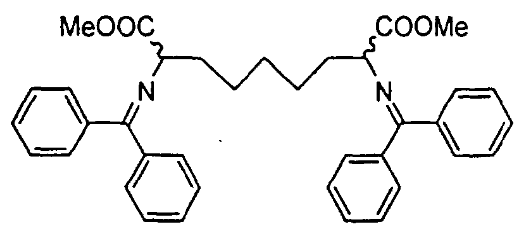

(155)

A mixture of 261 mg (1.03 mmol) of glycine methyl ester diphenylmethylimine, 190 mg (0.698 mmol) of benzyltriethyl ammonium bromide and 190 mg (1.13 mmol) of cesium hydroxide monohydrate was dissolved in 5.0 ml of dichloromethane, and 0.080 ml (0.538 mmol) of 1,5-diiodopentane was added, followed by stirring the mixture overnight at room temperature. The reaction mixture was diluted with 150 ml of ether, and the resulting mixture was washed with water and saturated brine, followed by drying over anhydrous sodium sulfate and concentration under reduced pressure. The residue was purified by column chromatography (silica gel; hexane/ethyl acetate = 90/10-70/30) to obtain 95.3 mg of 2,8-bis(1-aza-2,2-diphenylvinyl)nonanedioic acid dimethyl ester (155) as colorless oil (yield: 32%).
LR-MS (m/z) : 573 $[(M-H)^-]$
IR (neat) : 3057, 3023, 2947, 2856, 1739, 1623, 1597, 1576, 1490, 1445, 1315, 1288, 1201, 1174, 1138, 1074, 1029, 1000 cm$^{-1}$
$^1$H-NMR (300MHz, $CDCl_3$, δppm) : 7.66-7.60 (4H, m), 7.50-7.28 (12H, m), 7.18-7.10 (4H, m), 4.03 (2H, t, $J$ = 6.5 Hz), 3.70 (6H, s), 1.89-1.78 (4H,m), 1.29-1.05 (6H, m)

Example 156

2,8-bis((2,6-dichlorophenyl)carbonylamino)nonanedioic acid dimethyl ester (156)

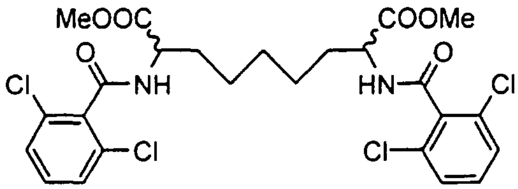

(156)

To a solution of 93.1 mg (0.162 mmol) of 2,8-bis(1-aza-2,2-diphenylvinyl)nonanedioic acid dimethyl ester (155) in 4.0 ml of ether, 0.80 ml (0.80 mmol) of 1N hydrochloric acid was added and the mixture was stirred at room temperature for 24 hours. After diluting the reaction solution with 10 ml of water, the aqueous layer was separated and washed with ether, followed by freeze-drying the resultant. The residue was dissolved in 5.0 ml of dichloromethane, and then 0.18 ml (1.30 mmol) of triethylamine and 0.070 ml (0.491 mmol) of 2,6-dichlorobenzoyl chloride were added, followed by stirring the mixture overnight at room temperature. To the reaction mixture, 10 ml of saturated aqueous sodium hydrogen carbonate solution was added, and the mixture was extracted with ethyl acetate. Organic layers were combined, washed with water, 0.5N hydrochloric acid, water and with saturated brine, dried over anhydrous sodium sulfate and concentrated under reduced pressure. The residue was purified by column chromatography (silica gel; cyclohexane/ethyl acetate = 70/30-40/60) to obtain 82.8 mg of 2,8-bis((2,6-dichlorophenyl)carbonylamino)nonanedioic acid dimethyl ester (156) as colorless oil (yield: 86%).
LR-MS (m/z) : 589 $[(M-H)^-]$
IR (neat) : 3276, 3069, 2951, 2960, 1740, 1655, 1579, 1560, 1542, 1432, 1361, 1300, 1270, 1196, 1172, 1089, 1015 $cm^{-1}$
$^1$H-NMR (300MHz, $CDCl_3$, δppm) : 7.36-7.22 (6H, m), 6.37 (2H, d, *J* = 8.0 Hz), 4.87 (2H, ddd, *J* = 8.0, 7.4, 5.5 Hz), 3.80 (6H, s), 2.09-1.95 (2H,m), 1.87-1.73 (2H,m), 1.59-1.34 (6H, m).

Example 157

2,8-bis((2,6-dichlorophenyl)carbonylamino)nonanedioic acid (157)

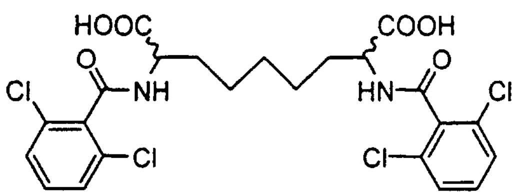

(157)

To a solution of 60.6 mg (0.106 mmol) of 2,8-bis((2,6-dichlorophenyl)carbonylamino)nonanedioic acid dimethyl ester (156) in 2.5 ml of methanol/THF (3/2), 1.5 ml of 1N aqueous sodium hydroxide solution was added and the mixture was stirred overnight at room temperature. To the reaction mixture, 6.6 ml of 0.25N hydrochloric acid was added, and the mixture was extracted with ethyl acetate. Organic layers were combined, washed with saturated brine, dried over anhydrous sodium sulfate and concentrated under reduced pressure. The residue was purified by preparative HPLC [Mighty Sil RP-18 250-20
(5mm) ;TFA/methao1/water=0.1/50/50] to obtain 64.6 mg of 2,8-bis((2,6-dichlorophenyl)carbonylamino)nonanedioic acid (157) as colorless oil (yield: 83%).
LR-MS (m/z) : 563 $[(M-H+2)^-]$
IR (KBr) : 3408, 3274, 3079, 2934, 2860, 1735, 1657, 1584, 1558, 1516, 1432, 1318, 1197, 1174, 1087 $cm^{-1}$
$^1$H-NMR (300MHz, $CDCl_3/CD_3OD$, δppm) : 7.37-7.24 (6H, m), 4.82-4.74 (2H, m), 2.10-1.96 (2H, m), 1.90-1.76 (2H, m), 1.60-1.38 (6H, m)
HR-MS : $C_{23}H_{22}Cl_4N_2O_6$ $[(M-H)^-]$ Calcd.: 561.0154, Found: 561.0153

Example 158

Methyl 2(S)-3-(4-(2-(methoxycarbonyl)ethyl)phenyl)-2-(phenylcarbonylamino)propionate (158)

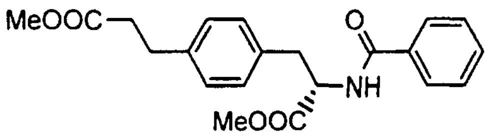

(158)

To a suspension of 43.3 mg (0.125 mmol) of methyl 2(S)-amino-3-(4-(2-(methoxycarbonyl)ethyl)phenyl)propionate (118) hydrobromic acid salt in 2.0 ml of dichloromethane, were added 0.060 ml (0.433 mmol) of triethylamine and 0.030 ml (0.260 mmol) of benzoyl chloride, followed by stirring the mixture at room temperature for 3 hours. To the reaction mixture, 5 ml of aqueous sodium hydrogen carbonate solution was added, and the mixture was extracted with ethyl acetate. Organic layers were combined, washed with saturated brine, dried over anhydrous sodium sulfate and concentrated under reduced pressure. The residue was purified by column chromatography (silica gel; cyclohexane/ethyl acetate = 90/10-60/40) to obtain 46.9 mg of methyl 2(S)-3-(4-(2-(methoxycarbonyl)ethyl)phenyl)-2-(phenylcarbonylamino)propionate (158) as an amorphous product (yield: 100%).
LR-MS (m/z) : 369 ($M^+$)
IR (neat) : 3334, 2951, 1737, 1644, 1602, 1579, 1531, 1488, 1437, 1362, 1215, 1111, 1024, 990 $cm^{-1}$.
$^1$H-NMR (300MHz, $CDCl_3$, δppm) : 7.76-7.69 (2H, m), 7.55-7.39 (3H, m), 7.13 (2H, d, *J* = 8.0 Hz), 7.05 (2H, d, *J* = 8.0 Hz), 6.56 (1H, d, *J* = 7.4 Hz), 5.08 (1H, ddd, *J* = 7.4, 5.6, 5.5 Hz), 3.77 (3H, s), 3.66 (3H, s), 3.27 (1H, dd, *J* = 14.0, 5.6 Hz), 3.19 (1H, dd, *J* = 14.0, 5.5 Hz), 2.92 (2H, t, *J* = 7.8 Hz), 2.61 (2H, t, *J* = 7.8 Hz).

Example 159

2(S)-3-(4-(2-carboxyethyl)phenyl)-2-(phenylcarbonylamino)propionic acid (159)

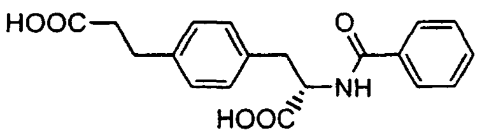

(159)

To a solution of 43.9 mg (0.119 mmol) of methyl 2(S)-3-(4-(2-(methoxycarbonyl)ethyl)phenyl)-2-(phenylcarbonylamino)propionate (158) in 2.0 ml of methanol, 1.0 ml of 1N aqueous sodium hydroxide solution was added, and the mixture was stirred overnight at room temperature. To the reaction mixture, 1.2 ml of 1N hydrochloric acid and 3 ml of water were added, and the mixture was extracted with ethyl acetate. Organic layers were combined, washed with saturated brine, dried over anhydrous sodium sulfate and concentrated under reduced pressure. The residue was purified by recrystallization (toluene/methanol) to obtain 39.2 mg of 2(S)-3-(4-(2-carboxyethyl)phenyl)-2-(phenylcarbonylamino)propionic acid (159) as colorless crystals (yield: 96%).
m.p. : 139.0-140.0°C
LR-MS (m/z) : 341 ($M^+$).
IR (neat) : 3356, 3276, 3029, 2926, 1707, 1644, 1576, 1533, 1488, 1423, 1297, 1235, 1156,1092, 1027 $cm^{-1}$.
$^1$H-NMR (300MHz, $CDCl_3$/DMSO-d6, δppm) : 7.76 (2H, d, *J* = 7.6 Hz), 7.54-7.36 (3H, m), 7.21-7.10 (4H, m), 4.89 (1H, dd, *J* = 7.3, 5.5 Hz), 3.29 (1H, dd, *J* = 13.5, 5.5 Hz), 3.17 (1H, dd, *J* = 13.5, 7.3 Hz), 2.89 (2H, t, *J* = 7.7 Hz), 2.55 (2H, t, *J* = 7.7 Hz).

| Elementary Analysis: $C_{19}H_{19}NO_5$ | | | |
|---|---|---|---|
| Calcd. | C 66.85%, | H 5.61%, | N 4.10% |
| Found | C 66.84%, | H 5.61%, | N 4.04% |

Example 160

Methyl 2(S)-2-((2,6-dichlorophenyl)carbonylamino)-3-(4-(2-(methoxycarbonyl)ethyl)phenyl)propionate (160)

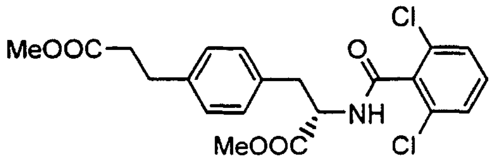

(160)

To a suspension of 62.3 mg (0.180 mmol) of methyl 2(S)-amino-3-(4-(2-(methoxycarbonyl)ethyl)phenyl)propionate (118) hydrobromic acid salt in 3.0 ml of dichloromethane, were added 0.090 ml (0.649 mmol) of triethylamine and 0.050 ml (0.351 mmol) of 2,6-dichlorobenzoyl chloride, and the mixture was stirred at 0°C for 3 hours. To the reaction mixture, 5 ml of saturated aqueous sodium hydrogen carbonate solution was added, and the mixture was extracted with ethyl acetate. Organic layers were combined, washed with saturated brine, dried over anhydrous sodium sulfate and concentrated under reduced pressure. The residue was purified by column chromatography (silica gel; cyclohexane/ethyl acetate = 90/10-60/40) to obtain 81.0 mg of methyl 2(S)-2-((2,6-dichlorophenyl)carbonylamino)-3-(4-(2-(methoxycarbonyl)ethyl)phenyl)propionate (160) as colorless oil (yield: 100%).
LR-MS (m/z) : 438 ($M^+$)
IR (neat) : 3316, 2951,1736, 1664, 1579, 1561, 1516, 1432, 1362, 1267, 1216, 1195, 1114, 1086, 1022 $cm^{-1}$.
$^1$H-NMR (300MHz, $CDCl_3$, δppm) : 7.35-7.21 (3H, m), 7.13 (4H, s), 6.28 (1H, d, *J* = 8.0 Hz), 5.18 (1H, dt, *J* = 8.0, 5.7 Hz), 3.76 (3H, s), 3.66 (3H, s), 3.24 (2H, t, *J* = 5.7 Hz), 2.91 (2H, t, *J* = 7.5 Hz), 2.60 (2H, t, *J* = 7.5 Hz).

Example 161

2(S)-2-((2,6-dichlorophenyl)carbonylamino)-3-(4-(2-carbaxyethyl)phenyl)propionic acid (161)

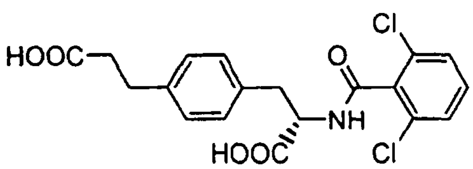

(161)

To a solution of 77.8 mg (0.178 mmol) of methyl 2(S)-2-((2,6-dichlorophenyl)carbonylamino)-3-(4-(2-(methoxycarbonyl)ethyl)phenyl)propionate (160) in 3.5 ml of methanol/THF (6/1), 1.5 ml of 1N aqueous sodium hydroxide solution was added, and the mixture was stirred overnight at room temperature. To the reaction mixture, 1.6 ml of 1N hydrochloric acid and 3 ml of water were added, and the mixture was extracted with ethyl acetate. Organic layers were combined, washed with saturated brine, dried over anhydrous sodium sulfate and concentrated under reduced pressure. The residue was purified by recrystallization (toluene/methanol) to obtain 37.9 mg of 2(S)-2-((2,6-dichlorophenyl) carbonylamino)-3-(4-(2-carboxyethyl)phenyl)propionic acid (161) as colorless powder (yield: 52%).
m.p.: 220.0-221.0°C.
LR-MS (m/z) : 409 ($M^+$)
IR (neat) : 3303, 1711, 1673, 1582, 1536, 1428, 1300, 1274, 1246, 1191.1168, 1116, 1082 $cm^{-1}$
$^1$H-NMR (300MHz, $CDCl_3$/DMSO-d6, δ ppm) : 7.80 (1H, d, *J* = 8.0 Hz), 7.35-7.21 (5H, m), 7.12 (2H, d, *J* = 6.9 Hz), 4.96 (1H, ddd, *J* = 8.0, 7.1, 5.2 Hz), 3.24 (1H, dd, *J* = 14.0, 5.2 Hz), 3.12 (1H, dd, *J* = 14.0, 7.1 Hz), 2.88 (2H, t, *J* = 7.5 Hz), 2.62-2.51 (2H, m).

| Elementary Analysis: $C_{19}H_{17}Cl_2NO_6$ | | | | |
|---|---|---|---|---|
| Calcd. | C 55.37%, | H 4.10%, | N 3.37%, | Cl 17.52% |
| Found | C 55.63%, | H 4.18%, | N 3.41%, | Cl 17.28% |

Example 162

Methyl 2(S)-3-(4-(2-(methoxycarbonyl)ethyl)phenyl)-2-((4-methoxyphenyl)carbonylamino)propionate (162)

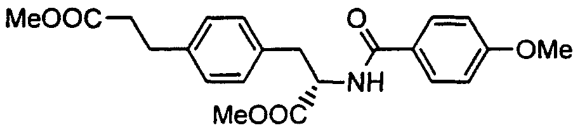

(162)

To a suspension of 137.6 mg (0.397 mmol) of methyl 2(S)-amino-3-(4-(2-(methoxycarbonyl)ethyl)phenyl)propionate (118) hydrobromic acid salt in 2.5 ml of dichloromethane, were added 0.33 ml (2.38 mmol) of triethylamine and a solution of 161.4 mg (0.946 mmol) of 4-methoxybenzoyl chloride in 1.5 ml of dichloromethane, and the mixture was stirred at 0°C for 2 hours. To the reaction mixture, 7 ml of saturated aqueous sodium hydrogen carbonate solution was added, and the resulting mixture was extracted with ethyl acetate. Organic layers were combined, washed with saturated brine, dried over anhydrous sodium sulfate and concentrated under reduced pressure. The residue was purified by column chromatography (silica gel; cyclohexane/ethyl acetate = 90/10-60/40) to obtain 161 mg of methyl 2(S)-3-(4-(2-(methoxycarbonyl)ethyl)phenyl)-2-((4-methoxyphenyl)carbonylamino)propionate (162) as colorless solid (yield: 100%).
LR-MS (m/z) : 399 ($M^+$)
IR (KBr) : 3335, 3008, 2952, 2841, 1737, 1640, 1607, 1537, 1504, 1438, 1362, 1304, 1256, 1216, 1177, 1111, 1027, 987 $cm^{-1}$.
$^1$H-NMR (300MHz, $CDCl_3$, δppm) : 7.70 (2H, d, *J* = 8.8 Hz), 7.12 (2H, d, *J* = 8.0 Hz), 7.05 (2H, d, *J* = 8.0 Hz), 6.92 (2H, d, *J* = 8.8 Hz), 6.47 (1H, d, *J* = 7.1 Hz), 5.06 (1H, ddd, *J* = 7.1, 5.7, 5.1 Hz), 3.85 (3H, s), 3.76 (3H, s), 3.66 (3H, s), 3.25 (1H, dd, *J* = 13.7, 5.7 Hz), 3.18 (1H, dd, *J* = 13.7, 5.2 Hz), 2.92 (2H, t, *J* = 7.8 Hz), 2.61 (2H, t, *J* = 7.8 Hz).

Example 163

2(S)-3-(4-(2-carboxyethyl)phenyl)-2-((4-methoxyphenyl)carbonylamino)propionic acid (163)

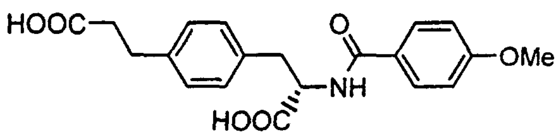

(163)

To a solution of 78.0mg (0.195 mmol) of methyl 2(S)-3-(4-(2-(methoxycarbonyl)ethyl)phenyl)-2-((4-methoxyphenyl)carbonylamino)propionate (162) in 2.0 ml of methanol, 2.0 ml of 1N aqueous sodium hydroxide solution was added, and the mixture was stirred at room temperature for 8 hours. To the reaction mixture, 2.1 ml of 1N hydrochloric acid was added and the resulting mixture was extracted with ethyl acetate. The organic layer was washed with saturated brine, dried over anhydrous sodium sulfate and concentrated under reduced pressure. The residue was purified by recrystallization (cyclohexane/ethyl acetate) to obtain 72.6 mg of 2(S)-3-(4-(2-carboxyethyl)phenyl)-2-((4-methoxyphenyl)carbonylamino)propionic acid (163) as colorless powder (yield: 100%).
m.p.: 121.0-122.0°C
LR-MS (m/z):371 ($M^+$)
IR (neat) : 3302, 3023, 2928, 2848, 1709, 1633, 1537, 1506, 1429, 1305, 1258, 1178, 1111, 1026 $cm^{-1}$
$^1$H-NMR (300MHz, $CDCl_3/CD_3OD$, δ ppm) : 7.69 (2H, d, *J* = 9.1 Hz), 7.12 (4H, s), 6.92 (2H, d, *J* = 9.1 Hz), 4.95 (1H, dd, *J* = 5.8, 5.8 Hz), 3.85 (3H, s), 3.28 (1H, dd, *J* = 14.0, 5.8 Hz), 3.18 (1H, dd, *J* = 14.0, 5.8 Hz), 2.91 (2H, t, *J* = 7.7 Hz), 2.59 (2H, t, *J* = 7.7 Hz)

| Elementary Analysis: $C_{20}H_{21}NO_6$ | | | |
|---|---|---|---|
| Calcd. | C 64.64%, | H 5.70%, | N 3.77% |
| Found | C 64.48%, | H 5.97%, | N 3.58% |

Example 164

Methyl 2(S)-2-((2,6-dimethoxyphenyl)carbonylamino)-3-(4-(2-(methoxycarbonyl)ethyl)phenyl)propionate (164)

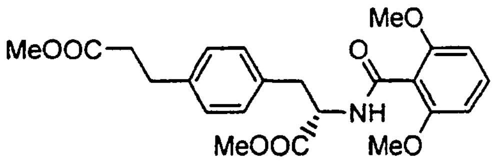

(164)

A mixture of 66.0 mg (0.191 mmol) of methyl 2(S)-amino-3-(4-(2-(methoxycarbonyl)ethyl)phenyl)propionate (118) hydrobromic acid salt, 38.2 mg (0.210 mmol) of 2,6-dimethoxybenzoyl chloride and 92.9 mg (0.210 mmol) of BOP reagent was suspended in 3.0 ml of dichloromethane, and 0.17 ml (0.987 mmol) of diisopropylethylamine was added, followed by stirring the mixture overnight at room temperature. After concentrating the reaction solution, 5 ml of water was added to the residue and the resulting mixture was extracted with ethyl acetate. Organic layers were combined, washed with saturated aqueous sodium hydrogen carbonate solution, water and with saturated brine, dried over anhydrous sodium sulfate and concentrated under reduced pressure. The residue was purified by column chromatography (silica gel; cyclohexane/ethyl acetate = 70/30-40/60) to obtain 92.3 mg of methyl 2(S)-2-((2,6-dimethoxyphenyl)carbonylamino)-3-(4-(2-(methoxycarbonyl)ethyl)phenyl)propionate (164) as an amorphous product (yield: 100%).
LR-MS (m/z) : 429 ($M^+$)
IR (neat) : 3428, 3013, 2953, 2843, 1738, 1652, 1596, 1514, 1474, 1438, 1365, 1254, 1213, 1111, 1025 $cm^{-1}$
$^1$H-NMR (300MHz, $CDCl_3$/$CD_3OD$, δppm) : 7.31 (1H, t, *J* = 8.2 Hz), 7.18-7.08 (4H, m), 6.58 (2H, d, *J* = 8.2 Hz), 5.06 (1H, dd, *J* = 5.5, 5.5 Hz), 3.81 (6H, s), 3.74 (3H, s), 3.66 (3H, s), 3.57 (1H, br.s), 3.27 (1H, dd, *J* = 14.0, 5.5 Hz), 3.17 (1H, dd, *J* = 14.0, 5.5 Hz), 2.91 (2H, t, *J* = 7.8 Hz), 2.62 (2H, t, *J* = 7.8 Hz).

Example 165

2(S)-2-((2,6-dimethoxyphenyl)carbonylamino)-3-(4-(2-carboxyethyl)phenyl)propionic acid (165)

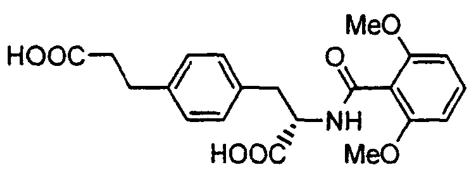

(165)

To a solution of 88.3 mg (0.205 mmol) of methyl 2(S)-2-((2,6-dimethoxyphenyl)carbonylamino)-3-(4-(2-(methoxycarbonyl)ethyl)phenyl)propionate (164) in 2.0 ml of methanol, 2.0 ml of 1N aqueous sodium hydroxide solution was added, and the mixture was stirred at room temperature for 8 hours. To the reaction mixture, 2.1 ml of 1N hydrochloric acid was added, and the mixture was extracted with ethyl acetate. Organic layers were combined, washed with saturated brine, dried over anhydrous sodium sulfate and concentrated. The residue was purified by column chromatography (DIOL; hexane/ethyl acetate = 45/55-1/99) to obtain 69.8 mg of 2(S)-2-((2,6-dimethoxyphenyl)carbonylamino)-3-(4-(2-carboxyethyl)phenyl)propionic acid (165) as an amorphous product (yield: 84%).
LR-MS (m/z) : 402 [$(M+H)^+$]
$^1$H-NMR (300MHz, $CD_3OD$, δppm) : 7.29 (1H, t, *J* = 8.2 Hz), 7.23 (2H, d, *J* = 8.1 Hz), 7.15 (2H, d, *J* = 8.1 Hz), 6.63 (2H, d, *J* = 8.2 Hz), 4.82 (1H, m), 3.74 (6H, s), 3.21 (1H, dd, *J* = 14.0, 5.2 Hz), 3.04 (1H, dd, *J* = 14.0, 5.2 Hz), 2.88 (2H, t, *J* = 7.5 Hz), 2.57 (2H, t, *J* = 7.5 Hz)
HR-MS : $C_{21}H_{23}NO_7$ Calcd.: 401.1475, Found: 401.1447

Example 166

2(S)-3-(4-(2-carboxyethyl)phenyl)-2-((4-hydroxyphenyl)carbonylamino)propionic acid (166)

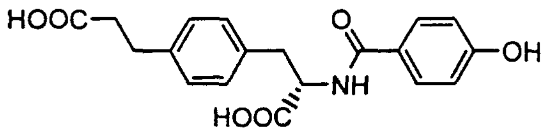

(166)

To a solution of 79.3 mg (0.198 mmol) of methyl 2(S)-3-(4-(2-(methoxycarbonyl)ethyl)phenyl)-2-((4-methoxyphenyl)carbonylamino)propionate (162) in 2.4 ml of dichloromethane, 1.60 ml of 1.0M boron tribromide solution in dichloromethane was added at -40°C, and the mixture was stirred at room temperature for 4 hours. To the reaction mixture, 2.0 ml of 1N hydrochloric acid and 4 ml of water were added, and the resulting mixture was extracted with ethyl acetate. Organic layers were combined, washed with saturated brine, dried over anhydrous sodium sulfate and concentrated. The residue was purified by column chromatography (DIOL; cyclohexane/ethyl acetate = 70/30-1/99) to obtain 26.1 mg of 2(S)-3-(4-(2-carboxyethyl)phenyl)-2-((4-hydroxyphenyl)carbonylamino)propionic acid (166) as an amorphous product (yield: 37%).
LR-MS (m/z) : 357 ($M^+$)
$^1$H-NMR (300MHz, $CD_3OD$, δppm) : 7.61 (2H, d, *J* = 8.8 Hz), 7.18 (2H, d, *J* = 8.4 Hz), 7.13 (2H, d, *J* = 8.4 Hz), 6.78 (2H, d, *J* = 8.8 Hz), 4.78 (1H, dd, *J* = 9.2, 4.9 Hz), 3.27 (1H, dd, *J* = 13.9, 4.9 Hz), 3.07 (1H, dd, *J* = 13.9, 9.2 Hz), 2.85 (2H, t, *J* = 7.7 Hz), 2.54 (2H, t, *J* = 7.7 Hz)

Example 167

Methyl 2(S)-3-(4-(2-(methoxycarbonyl)ethyl)phenyl)-2-(4-methyl-2-(4-((phenylamino)carbonylamino)phenyl) pentanoylamino)propionate (167)

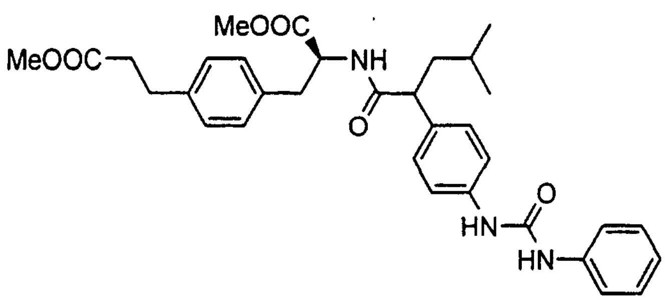

(167)

A mixture of 150 mg (0.460 mmol) of 4-methyl-2-(4-((phenylamino)carbonylamino)phenyl)valeric acid and 203 mg (0.460 mmol) of BOP reagent was dissolved in 4.0 ml of DMF, and then 173 mg (0.500 mmol) of methyl 2(S)-amino-3-(4-(2-(methoxycarbonyl)ethyl)phenyl)propionate (118) hydrobromic acid salt and 0.35 ml (2.0 mmol) of diisopropylethylamine were added, followed by stirring the mixture overnight at room temperature. To the reaction mixture, 1N hydrochloric acid was added, and the resulting mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 1/2-1/9) to obtain 235 mg of methyl 2(S)-3-(4-(2-(methoxycarbonyl)ethyl)phenyl)-2-(4-methyl-2-(4-((phenylamino) carbonylamino)phenyl)pentanoylamino)propionate (167) as an amorphous product (yield: 98%).
LR-MS (m/z) : 574 $[(M+H)^+]$
IR (KBr) : 3297, 3205, 3057,2956,2930,2870, 1712, 1652, 1599, 1546, 1514, 1499, 1422, 1414, 1313, 1233, 1048, 1022 $cm^{-1}$
$^1$H-NMR (300MHz, $CDCl_3$, δ ppm) : 0.85(6H, dd, *J* = 4.4, 6.6 Hz), 1.33-1.45(1H, m), 1.60-1.78(1H, m), 1.90-1.99(1H, m), 2.60(2H, q, *J* = 7.3 Hz), 2.84-2.95(3H, m), 3.00(1H, t, *J* = 6.2 Hz), 3.40(1H, dd, *J* = 9.0, 17.1 Hz), 3.64(3H, dd, *J* = 1.1, 3.9 Hz), 3.69(3H, dd, *J* = 1.0, 11.7 Hz), 4.73-4.95(1H, m), 5.88-6.02(1H, m), 6.66(1H, d, *J* = 8.4 Hz), 6.86(1H, d,

*J* = 8.4 Hz), 6.94-7.06(3H, m), 7.15(1H, dd, *J* = 8.4, 4.8 Hz), 7.22-7.32(2H, m), 7.40(2H, t, *J* = 8.2 Hz), 7.46(2H, dd, *J* = 7.5, 3.9 Hz), 7.86-8.01(1H, m)

Example 168

(2S)-3-(4-(2-carboxyethyl)phenyl)-2-(4-methyl-2-(4-((phenylamino)carbonylamino)phenyl)pentanoylamino)propionic acid (168)

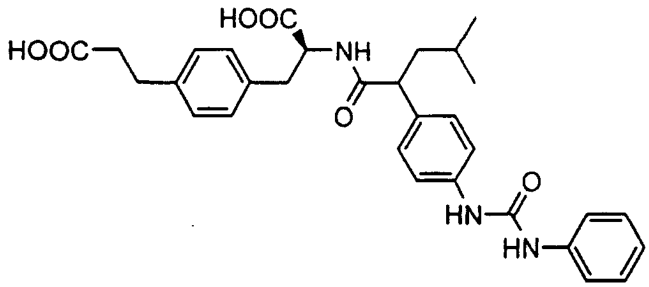

(168)

To a solution of 131 mg (0.23 mmol) of methyl 2(S)-3-(4-(2-(methoxycarbonyl)ethyl)phenyl)-2-(4-methyl-2-(4-((phenylamino)carbonylamino)phenyl)pentanoylamino)propionate (167) in 6.0 ml of methanol/THF (1/1), 1.3 ml of 1N aqueous sodium hydroxide solution was added, and the mixture was stirred overnight at room temperature. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 20/80-1/99) to obtain 115 mg of (2S)-3-(4-(2-carboxyethyl)phenyl)-2-(4-methyl-2-(4-((phenylamino)carbonylamino)phenyl)pentanoylamino)propionic acid (168) as an amorphous product (yield: 92%).
LR-MS (m/z) : 546 [$(M+H)^+$]
$^1$H-NMR (300MHz, $CDCl_3$, δppm) : 0.84-0.87(6H, m), 1.34-1.45(1H, m), 1.58-1.74(1H, m), 1.84-1.96(1H, m), 2.45-2.61 (2H, m), 2.84-2.89(2H, m), 2.92(1H, s), 2.97-3.00(1H, m), 3.35-3.45(1H, m), 4.69-4.77(1H, m), 6.11(1H, dd, *J* = 4.8, 35.1 Hz), 6.69(1H, d, *J* = 2.6 Hz), 6.91-7.06(4H, m), 7.16(2H, t, *J* = 6.9 Hz), 7.28(2H, t, *J* = 7.5 Hz), 7.34-7.48(4H, m), 8.08(1H, d, *J* = 5.2 Hz), 8.14(1H, d, *J* = 13.2 Hz)
$[\alpha]_D^{20}$ = -11.1° (c = 0.018, $CH_3OH$)

Example 169

Methyl (2S)-2-((2S)-2-((tert-butoxy)carbonylamino)-4-methylpentanoylamino)-3-(4-(2-(methoxycarbonyl)ethyl) phenyl)propionate (169)

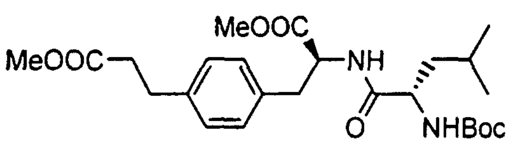

(169)

In 5.0 ml of dichloromethane, 127 mg (0.55 mmol) of N-Boc-L-Leu-OH • H2O was dissolved, and then 173 mg (0.50 mmol) of methyl 2(S)-amino-3-(4-(2-(methoxycarbonyl)ethyl)phenyl)propionate (118) hydrobromic acid salt and 0.35 ml (2.0 mmol) of diisopropylethylamine were added, followed by stirring the mixture at room temperature for 2 hours. To the reaction mixture, 1N hydrochloric acid was added and the resulting mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 3/1-2/1) to obtain 236 mg of methyl (2S)-2-((2S)-2-((tert-butoxy)carbonylamino)-4-methylpentanoylamino)-3-(4-(2-(methoxycarbonyl)ethyl)phenyl)propionate (169) as colorless oil (yield: 98%).

LR-MS (m/z) : 479 [$(M+H)^+$]
IR (KBr) : 3312, 3058, 2958, 2929, 2871, 1728, 1650, 1598, 1547, 1442, 1413, 1314, 1234, 1200, 1112, 1047, 1022, 997 $cm^{-1}$
$^1$H-NMR (300MHz, $CDCl_3$, δppm) : 0.90-0.93(6H, m), 1.44(9H, s), 1.55-1.64(3H, m), 2.61(2H, t, *J* = 8.0 Hz), 2.92(2H, t, *J* = 7.8 Hz), 3.09(2H, q, *J* = 5.6 Hz), 3.67(3H, s), 3.72(3H, s), 4.02-4.15(1H, br), 4.83(2H, dd, *J* = 6.0, 13.5 Hz), 6.46 (1H, d, *J* = 8.1 Hz), 7.07(4H, dd, *J* = 8.4, 26.4 Hz)

Example 170

Methyl (2S)-2-((2S)-4-methyl-2-(2-(4-((phenylamino)carbonylamino)phenyl)acetylamino)pentanoylamino)-3-(4-(2-(methoxycarbonyl)ethyl)phenyl)propionate (170)

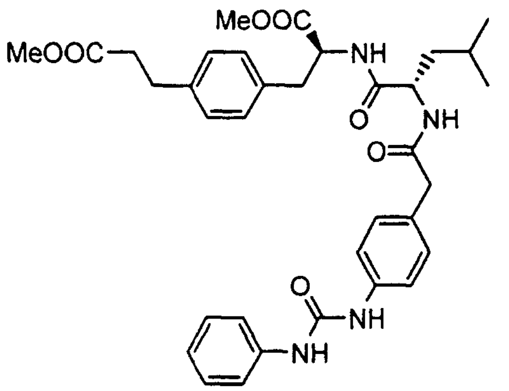

(170)

To a solution of 204 mg (0.43 mmol) of methyl (2S)-2-((2S)-2-((tert-butoxy)carbonylamino)-4-methylpentanoylamino)-3-(4-(2-(methoxycarbonyl)ethyl)phenyl)propionate (169) in 2.0 ml of dichloromethane, 1.0 ml of TFA was added and the mixture was stirred for 30 minutes, followed by concentration of the resulting mixture under reduced pressure. To the residue, 127 mg (0.47 mmol) of 2-(4-((phenylamino)carbonylamino)phenyl)acetic acid, 208 mg (0.47 mmol) of BOP reagent, 4.0 ml of DMF and 0.30 ml (1.7 mmol) of diisopropylethylamine were added, and the mixture was stirred overnight at room temperature. To the reaction mixture, 1N hydrochloric acid was added and the resulting mixture was extracted with ethyl acetate. Organic layers were combined, washed with saturated aqueous sodium hydrogen carbonate solution and saturated brine, dried over anhydrous sodium sulfate and concentrated. The residue was purified by reprecipitation (ether/chloroform/methanol) to obtain 177 mg of methyl (2S)-2-((2S)-4-methyl-2-(2-(4-((phenylamino)carbonylamino)phenyl)acetylamino)pentanoylamino)-3-(4-(2-(methoxycarbonyl)ethyl)phenyl) propionate (170) as an amorphous product (yield: 66%).
LR-MS (m/z) : 631 [$(M+H)^+$]
$^1$H-NMR (300MHz, $CDCl_3$, δppm) : 0.84-0.87(6H, m), 2.61(2H, t, *J* = 7.9 Hz), 2.91(2H, t, *J* = 7.7 Hz), 2.91-3.13(2H, m), 3.50(2H, s), 3.67(3H, d, *J* = 0.8 Hz), 3.71(3H, d, *J* = 0.8 Hz), 4.36-4.44(1H, m), 4.73-4.80(1H, m), 5.88-5.98(1H, br s), 6.56-6.64(1H, br s), 7.05(4H, dd, *J* = 8.0, 24.3 Hz), 7.14(4H, d, *J* = 8.6 Hz), 7.31(2H, d, *J* = 7.7 Hz), 7.42(4H, dd, *J* = 8.4, 9.9 Hz), 7.66-7.82(1H, br s)

Example 171

(2S)-2-((2S)-4-methyl-2-(2-(4-((phenylamino)carbonylamino)phenyl)acetylamino)pentanoylamino)-3-(4-(2-carboxyethyl)phenyl)propionic acid (171)

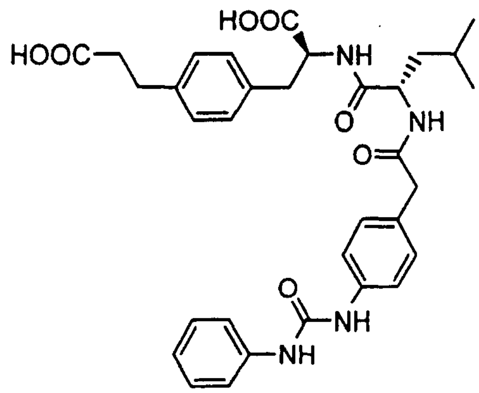

(171)

To a solution of 100 mg (0.16 mmol) of methyl (2S)-2-((2S)-4-methyl-2-(2-(4-((phenylamino)carbonylamino)phenyl)acetylamino)pentanoylamino)-3-(4-(2-(methoxycarbonyl)ethyl)phenyl)propionate (170) in 6.0 ml of methanol/THF (1/1), 0.95 ml of 1N aqueous sodium hydroxide solution was added and the mixture was stirred overnight at room temperature. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 20/80-1/99) to obtain 70 mg of (2S)-2-((2S)-4-methyl-2-(2-(4-((phenylamino)carbonylamino)phenyl)acetylamino)pentanoylamino)-3-(4-(2-carboxyethyl)phenyl)propionic acid (171) as an amorphous product (yield: 73%).
LR-MS (m/z) : 603 [$(M+H)^+$]
$^1$H-NMR (300MHz, $CDCl_3$+DMSO-d6, δppm) : 0.84-0.87(6H, m), 2.61(2H, t, $J$ = 7.9 Hz), 2.91(2H, t, $J$ = 7.7 Hz), 2.91-3.13(2H, m), 3.50(2H, s), 4.36-4.44(1H, m), 4.73-4.80(1H, m), 6.52(1H, d, $J$ = 12.0 Hz), 6.84(1H, d, $J$ = 12.3 Hz), 6.98-7.30(8H, m), 7.37(2H, s), 7.42(4H, dd, $J$ = 8.4, 9.9 Hz), 8.13(2H, d, $J$ = 19.5 Hz)
$[\alpha]_D^{20}$ = -8.2° (c = 0.028, $CH_3OH$)

Example 172

Methyl 4-((2R)-2-(3,3-dimethylbutanoylamino)-2-(methoxycarbonyl)ethylthio)-(2S)-2-(4-methyl-2-(4-((phenylamino)carbonylamino)phenyl)pentanoylamino)butyrate (172)

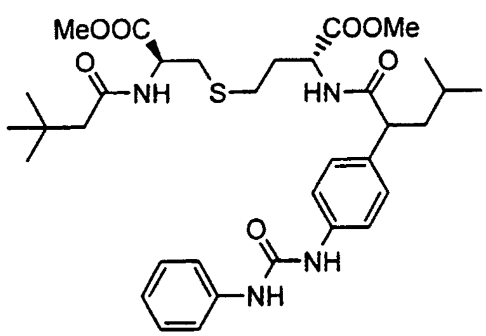

(172)

In 2.0 ml of DMF, 115 mg (0.33 mmol) of methyl 2(S)-amino-4-(2(R)-(3,3-dimethylbutanoylamino)-2(R)-(methoxycarbonyl)ethylthio) butyrate (12), 118 mg (0.36 mmol) of 4-methyl-2-(4-((phenylamino)carbonylamino)phenyl)valeric acid and 177 mg (0.40 mmol) of BOP reagent were suspended, and 0.23 ml (1.32mmol) of diisopropylethylamine was added, followed by stirring the resulting mixture overnight at room temperature. To the reaction solution, 10 ml of

1N hydrochloric acid was added and the resulting mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 2/1-1/1) to obtain methyl 4-((2R)-2-(3,3-dimethylbutanoylamino)-2-(methoxycarbonyl)ethylthio)-(2S)-2-(4-methyl-2-(4-((phenylamino)carbonylamino)phenyl)pentanoylamino)butyrate (172) as two diastereomers (Isomer A: 57.0 mg (yield: 26%), Isomer B 39.2 mg (yield: 18%))(on TLC, the component in the low polarity side was defined as Isomer A, and the component in the high polarity side was defined as Isomer B).

<Isomer A>

LR-MS (m/z) : 656 [$(M+H)^+$]
IR (KBr) : 3315, 3061, 2955, 2869, 1743, 1646, 1600, 1545, 1442, 1312, 1232, 1018 $cm^{-1}$
$^1$H-NMR (300MHz, $CDCl_3/CD_3OD$, δ ppm) : 7.44-7.21 (8H, m), 7.03 (1H, t, *J* = 7.4 Hz), 6.88 (1H, br d), 6.76 (1H, br. d), 4.65 (1H, m), 4.48 (1H, m), 3.73 (3H, s), 3.69 (3H, s), 3.50 (1H, t, *J* = 7.7 Hz), 2.70-2.56 (2H, m), 2.20 (2H, t, *J* = 7.4 Hz), 2.14 (2H, s), 2.06-1.89 (2H, m), 2.00 (1H, m), 1.67 (1H, m), 1.45 (1H, m), 1.04 (9H, s), 0.90 (6H, d, *J* = 6.6 Hz)

<Isomer B>

LR-MS (m/z) : 656 [$(M+H)^+$]
IR (neat) : 3310, 3063, 2954, 2870, 1737, 1643, 1600, 1545, 1442, 1312, 1234, 1018 $cm^{-1}$
$^1$H-NMR (300MHz, $CDCl_3/CD_3OD$, δppm) : 7.89 (1H, br.s), 7.81 (1H, br.s), 7.41-7.19 (8H, m), 7.01 (1H, t, *J* = 7.4 Hz), 6.92 (1H, br d), 6.69 (1H, br.d), 4.67 (1H, m), 4.54 (1H, m), 3.73 (3H, s), 3.65 (3H, s), 3.56 (1H, t, *J* = 7.7 Hz), 2.92-2.69 (2H, m), 2.41 (2H, t, *J* = 7.4 Hz), 2.14 (2H, s), 2.10-1.88 (3H, m), 1.70 (1H, m), 1.46 (1H, m), 1.05 (9H, s), 0.89 (6H, m)

Example 173

4-((2R)-2-(3,3-dimethylbutanoylamino)-2-(carboxyethylthio)-(2S)-2-(4-methyl-2-(4-((phenylamino)carbonylamino)phenyl)pentanoylamino)butyric acid (173)

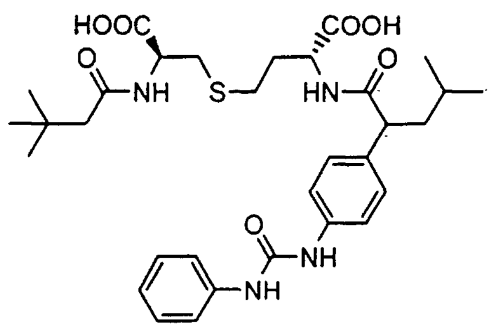

(173)

To a solution of 51.0 mg (0.082 mmol) of methyl 4-((2R)-2-(3,3-dimethylbutanoylamino)-2-(methoxycarbonyl)ethylthio)-(2S)-2-(4-methyl-2-(4-((phenylamino)carbonylamino)phenyl)pentanoylamino)butyrate (172) (Isomer A) in 1.0 ml of methanol, 0.49 ml of 1N aqueous sodium hydroxide solution was added and the mixture was stirred at room temperature for 62 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 30/70-15/85) to obtain 47 mg of 4-((2R)-2-(3,3-dimethylbutanoylamino)-2-(carboxyethylthio)-(2S)-2-(4-methyl-2-(4-((phenylamino)carbonylamino)phenyl)pentanoylamino)butyric acid (173) (Isomer A) (yield: 91%).
In the same manner, from 38.9 mg of methyl 4-((2R)-2-(3,3-dimethylbutanoylamino)-2-(methoxycarbonyl)ethylthio)-(2S)-2-(4-methyl-2-(4-((phenylamino)carbonylamino)phenyl)pentanoylamino)butyrate (29) (Isomer B), 32 mg of 4-((2R)-2-(3,3-dimethylbutanoylamino)-2-(carboxyethylthio)-(2S)-2-(4-methyl-2-(4-((phenylamino)carbonylamino)phenyl)pentanoylamino)butyric acid (173) (Isomer B) was obtained (yield: 86%).

<Isomer A>

$[\alpha]^{20}_D$ = -32.5° (c=0.010, $CH_3OH$)
LR-MS (m/z) : 627 [$(M-H)^-$].
$^1$H-NMR (300MHz, $CDCl_3$/DMSO-d6, δppm) : 8.02 (1H, s), 7.91 (1H, s), 7.44-7.21 (8H, m), 7.01 (1H, t, *J* = 7.4 Hz), 6.64 (1H, br.d), 6.55 (1H, br.d), 4.60 (1H, m), 4.51 (1H, m), 3.79 (1H, m), 2.84 (1H, m), 2.69 (1H, m), 2.27-2.23 (2H, m), 2.14 (2H, s), 2.03 (1H, m), 1.97-1.80 (2H, m), 1.70 (1H, m), 1.45 (1H, m), 1.04 (9H, s), 0.88 (6H, d, *J* = 6.6 Hz)
HR-MS : $C_{32}H_{43}N_4O_7S$ [$(M-H)^-$] Calcd.: 627.2852, Found: 627.2873

<Isomer B>

$[\alpha]^{20}_D$ = -16.1° (c=0.010, $CH_3OH$)
LR-MS (m/z) : 629 [$(M+H)^+$]
$^1$H-NMR (300MHz, $CDCl_3$/DMSO-d6, δppm) : 8.04 (1H, s), 7.93 (1H, s), 7.40-7.15 (8H, m), 6.99 (1H, t, *J* = 7.4 Hz), 6.67 (1H, br.d), 4.61 (1H, m), 4.51 (1H, m), 3.57 (1H, t, *J* = 7.7 Hz), 2.98 (1H, m), 2.85 (1H, m), 2.51-2.46 (2H, m), 2.14 (2H, s), 2.08-1.87 (3H, m), 1.65 (1H, m), 1.45 (1H, m), 1.03 (9H, s), 0.89-0.85 (6H, m)
HR-MS : $C_{32}H_{45}N_4O_7S$ [$(M+H)^+$] Calcd.: 629.3009, Found: 629.3033

Example 174

Methyl 4-((2R)-2-(3,3-dimethylbutanoylamino)-2-(methoxycarbonyl)ethylthio)-(2S)-2-((2S)-2-(tert-butoxy)carbonylamino)-4-methylpentanoylamino)butyrate (174)

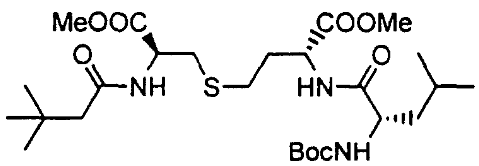

(174)

In 2.0 ml of dichloromethane, 90.6 mg (0.26 mmol) of methyl 2(S)-amino-4-(2(R)-(3,3-dimethylbutanoylamino)-2(R)-(methoxycarbonyl)ethylthio) butyrate (12), 72.3 mg (0.29 mmol) of L-N-Boc-Leu•$H_2O$ and 137 mg (0.31 mmol) of BOP reagent were suspended, and 0.18 ml (1.04mmol) of diisopropylethylamine was added, followed by stirring the mixture overnight at room temperature. To the reaction solution, 5 ml of 1N hydrochloric acid was added, and the mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 2/1-3/2) to obtain 60 mg of methyl 4-((2R)-2-(3,3-dimethylbutanoylamino)-2-(methoxycarbonyl)ethylthio)-(2S)-2-((2S)-2-(tert-butoxy)carbonylamino)-4-methylpentanoylamino)butyrate (174) (yield: 41%).
$[\alpha]^{29}_D$ = -41.5° (c=0.22, $CH_3OH$)
LR-MS (m/z) : 562 [$(M+H)^+$]
IR (neat) : 3311, 2956, 2871, 1745, 1660, 1531, 1438, 1367, 1170, 1112, 1046, 1023 $cm^{-1}$
$^1$H-NMR (300MHz, $CDCl_3$, δppm) : 6.90 (1H, m), 6.39 (1H, br.d), 5.16 (1H, d, *J* = 8.2 Hz), 4.80 (1H, m), 4.69 (1H, m), 4.13 (1H, m), 3.78-3.75 (6H, m), 2.96 (2H, d, *J* = 5.2 Hz), 2.62-2.47 (2H, m), 2.15 (2H, s), 2.12 (1H, m), 1.97 (1H, m), 1.70-1.66 (2H, m), 1.46 (1H, m), 1.45 (9H, s), 1.06 (9H, s), 0.96-0.93 (6H, m)

Example 175

Methyl 4-((2R)-2-(3,3-dimethylbutanoylamino)-2-(methoxycarbonyl)ethylthio)-(2S)-2-((2S)-4-methyl-2-(2-(4-((phenylamino)carbonylamino)phenyl)acetylamino)pentanoylamino)butyrate (175)

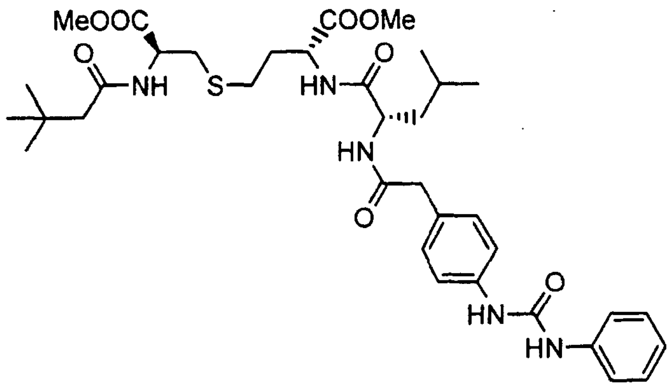

(175)

To a solution of 60.0 mg (0.11 mmol) of methyl 4-((2R)-2-(3,3-dimethylbutanoylamino)-2-(methoxycarbonyl) ethylthio)-(2S)-2-((2S)-2-(tert-butoxy)carbonylamino)-4-methylpentanoylamino)butyrate (174) in 2.0 ml of dichloromethane, 1.0 ml of TFA was added, and the mixture was stirred at room temperature for 1 hour, followed by concentration of the mixture. The residue was dissolved in 2.0 ml of DMF, and then 32.4 mg (0.12 mmol) of 2-(4-((phenylamino) carbonylamino)phenyl)acetic acid, 57.5 mg (0.13 mmol) of BOP reagent and 0.77 ml (0.44 mmol) of diisopropylethylamine were added thereto, followed by stirring the mixture overnight at room temperature. To the reaction solution, 5 ml of 1N hydrochloric acid was added and the mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 3/2-1/1) and by recrystallization (ether/methanol) to obtain 49.0 mg of methyl 4-((2R)-2-(3,3-dimethylbutanoylamino)-2-(methoxycarbonyl)ethylthio)-(2S)-2-((2S)-4-methyl-2-(2-(4-((phenylamino)carbonylamino)phenyl)acetylamino)pentanoylamino)butyrate (175) (yield: 62%).
$[\alpha]^{28}_D$ = -61.1° (c=0.037, $CH_3OH$
LR-MS (m/z) : 714 $[(M+H)^+]$
IR (KBr) : 3303, 3064, 2955, 2870, 1733, 1642, 1547, 1441, 1312, 1234, 1022 $cm^{-1}$
$^1H$-NMR (300MHz, $CD_3OD$, δppm) : 7.34-7.21 (4H, m), 7.19-7.12 (4H, m), 6.92 (1H, t, *J* = 7.3 Hz), 4.53-4.44 (2H, m), 4.33-4.28 (1H, m), 3.62-3.61 (6H, m), 3.41 (2H, m), 2.88 (1H, m), 2.70 (1H, m), 2.53 (1H, m), 2.40 (1H, m), 2.02 (2H, s), 1.96 (1H, m), 1.83 (1H, m), 1:60-1.50 (3H, m), 0.93 (9H, s), 1.06 (9H, s), 0.87 (3H, d, *J* = 6.2 Hz), 0.81 (3H, d, *J* = 6.2 Hz)

Example 176

4-((2R)-2-(3,3-dimethylbutanoylamino)-2-carboxyethylthio)-(2S)-2-((2S)-4-methyl-2-(2-(4-((phenylamino)carbonylamino)phenyl)acetylamino)pentanoylamino)butyric acid (176)

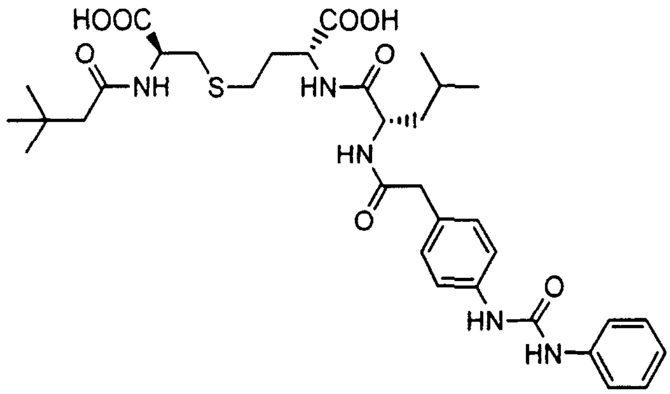

(176)

To a solution of 44.2 mg (0.082 mmol) of methyl 4-((2R)-2-(3,3-dimethylbutanoylamino)-2-(methoxycarbonyl)ethylthio)-(2S)-2-((2S)-4-methyl-2-(2-(4-((phenylamino)carbonylamino)phenyl)acetylamino)pentanoylamino)butyrate (175) in 1.5 ml of methanol, 0.37 ml of 1N aqueous sodium hydroxide solution was added, and the mixture was stirred overnight at room temperature. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = (40/60) - ethyl acetate) to obtain 41.9 mg of 4-((2R)-2-(3,3-dimethylbutanoylamino)-2-carboxyethylthio)-(2S)-2-((2S)-4-methyl-2-(2-(4-((phenylamino)carbonylamino)phenyl)acetylamino)pentanoylamino)butyric acid (176) (yield: 98%).
$[\alpha]^{20}_D$ = -63.5° (c=0.010, $CH_3OH$)
LR-MS (m/z) : 686 $[(M+H)^+]$.
IR (KBr) : 3330, 3065, 2957, 2871, 1726, 1646, 1545, 1442, 1415, 1312, 1232, 984 $cm^{-1}$
$^1$H-NMR (300MHz, $CD_3OD$, δppm) : 7.43-7.36 (4H, m), 7.30-7.21 (4H, m), 7.00 (1H, m), 4.59-4.54 (2H, m), 4.44 (1H, m), 3.52 (2H, s), 3.02 (1H, m), 2.83 (1H, m), 2.68-2.47 (2H, m), 2.14 (2H, s), 2.12 (1H, m), 1.93 (1H, m), 1.70-1.60 (3H, m), 1.03 (9H, s), 0.95 (3H, d, *J* = 6.2 Hz), 0.90 (3H, d, *J* = 6.2 Hz)
HR-MS : $C_{34}H_{48}N_5O_8S$ $[(M+H)^+]$ Calcd.: 686.3224, Found: 686.3188

Example 177

Methyl 4-((2R)-2-(methoxycarbonyl)-2-((phenylmethoxy)carbonylamino)ethylthio) (2S)-2-((tert-butoxy)carbonylamino)butyrate (177)

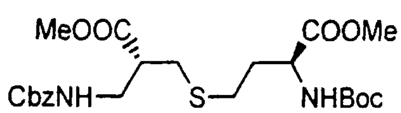

(177)

To a solution of 900 mg (3.3 mmol) of N-Cbz-cystein methyl ester in 15 ml of THF, 340 mg (3.0 mmol) of potassium tert-butoxide was added at 0°C, and the mixture was stirred for 30 minutes. To this solution, 1N hydrochloric acid was added and the mixture was extracted with ethyl acetate. Organic layers were combined, washed with saturated brine, dried over anhydrous sodium sulfate and concentrated. The residue was purified by column chromatography (hexane/ethyl acetate = 3/1-1/1) to obtain 843 mg of methyl 4-((2R)-2-(methoxycarbonyl)-2-((phenylmethoxy)carbonylamino)ethylthio) (2S)-2-((tert-butoxy)carbonylamino)butyrate (177) as colorless oil (yield: 63%).
LR-MS (m/z) : 498 $(M^+)$
IR (KBr) : 3248, 3071, 2923, 1720, 1651, 1581, 1560, 1433, 1315, 1269, 1238, 1195, 1086, 909 $cm^{-1}$

$^1$H-NMR (300MHz, $CDCl_3$, δppm) : 7.39-7.31 (5H, m), 5.66 (1H, br.s), 5.13 (2H, s), 5.13 (1H, m), 4.59 (1H, m), 4.39 (1H, m), 3.77 (3H, br.s), 3.74 (3H, s), 3.02-2.96 (2H, m), 2.60-2.50 (2H, m), 2.06 (1H, m), 1.88 (1H, m), 1.44 (9H, s)

Example 178

Methyl 4-((2R)-2-amino-2-(methoxycarbonyl)ethylthio) (2S)-2-aminobutyrate (178)

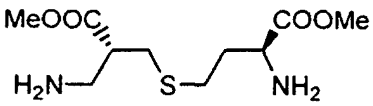

(178)

To a solution of 485 mg (1.00 mmol) of methyl 4-((2R)-2-(methoxycarbonyl)-2-((phenylmethoxy)carbonylamino)ethylthio) (2S)-2-((tert-butoxy)carbonylamino)butyrate (177) in 2.0 ml of dichloromethane, 2.0 ml of 30% hydrogen bromide/acetic acid solution was added at 0°C, and the mixture was stirred for 30 minutes. Water was added to the reaction mixture, and the resulting mixture was shaken with ethyl acetate. Saturated aqueous potassium carbonate solution was added to the aqueous layer and the resulting mixture was extracted with chloroform. Organic layers were combined, washed with saturated brine, dried over anhydrous sodium sulfate and concentrated to obtain 90 mg of methyl 4-((2R)-2-amino-2-(methoxycarbonyl)ethylthio) (2S)-2-aminobutyrate (178). The product (178) was used in the next reaction without purification.

Example 179

Methyl 4-((2R)-2-((2, 6-dichlorophenyl)carbonylamino)-2-(methoxycarbonyl)ethylthio) (2S)-2-((2, 6-dichlorophenyl) carbonylamino)butyrate (179)

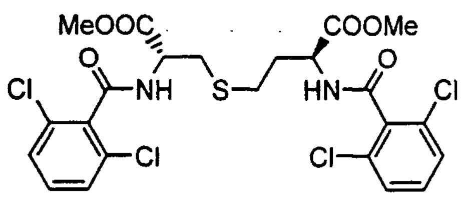

(179)

To a solution of 90 mg of methyl 4-((2R)-2-amino-2-(methoxycarbonyl)ethylthio) (2S)-2-aminobutyrate (178) in 2.0 ml of dichloromethane, were added 0.20 ml (1.44 mmol) of triethylamine and 0.124 ml (0.86 mmol) of 2,6-dichlorobenzoyl chloride, and the mixture was stirred overnight at room temperature. To the reaction solution, 1N hydrochloric acid was added and the mixture was extracted with ethyl acetate. Organic layers were combined, washed with saturated brine, dried over anhydrous sodium sulfate and concentrated. The residue was purified by column chromatography (hexane/ethyl acetate = 2/1-1/2) to obtain 154 mg of methyl 4-((2R)-2-((2, 6-dichlorophenyl)carbonylamino)-2-(methoxycarbonyl)ethylthio) (2S)-2-((2, 6-dichlorophenyl)carbonylamino)butyrate (179) as colorless oil (yield: 26%).
LR-MS (m/z) : 595 $[(M+H)^+]$
$^1$H-NMR (300MHz, $CDCl_3$, δppm) : 7.35-7.19 (6H, m), 6.77 (1H, m), 6.59 (1H, br.d, $J$ = 7.7 Hz), 5.07 (1H, m), 4.95-(1H, m), 3.81 (3H, s), 3.80 (3H, s), 3.18-3.10 (2H, m), 2.80-2.68 (2H, m), 2.31 (1H, m), 2.12 (1H, m)

Example 180

4-((2R)-2-((2, 6-dichlorophenyl)carbonylamino)-2-carboxyethylthio) (2S)-2-((2, 6-dichlorophenyl)carbonylamino) butyric acid (180)

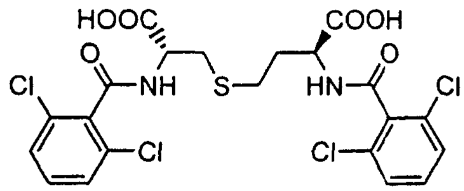

(180)

To a solution of 135 mg (0.23 mmol) of methyl 4-((2R)-2-((2, 6-dichlorophenyl)carbonylamino)-2-(methoxycarbonyl)ethylthio) (2S)-2-((2, 6-dichlorophenyl)carbonylamino)butyrate (179) in 1.5 ml of methanol/THF (2/1) solution, 1.4 ml of 1N aqueous sodium hydroxide solution was added and the mixture was stirred overnight at room temperature. To the reaction solution, 1N hydrochloric acid was added and the mixture was extracted with ethyl acetate. Organic layers were combined, washed with saturated brine, dried over anhydrous sodium sulfate and concentrated. The residue was purified by column chromatography (DIOL; hexane/ethyl acetate = 20/80-5/95) to obtain 112 mg of 4-((2R)-2-((2, 6-dichlorophenyl)carbonylamino)-2-carboxyethylthio) (2S)-2-((2, 6-dichlorophenyl)carbonylamino)butyric acid (180) as an amorphous product (yield: 87%).
LR-MS (m/z) : 566 [$(M+H)^+$]
$^1$H-NMR (300MHz, $CDCl_3$+DMSO-d6, δppm) : 7.36-7.22 (6H, m), 4.96 (1H, m), 4.83 (1H, m), 3.28-3.12 (2H, m), 2.86-2.57 (2H, m), 2.30 (1H, m), 2.15 (1H, m)
$[\alpha]^{20}_D$=-100° (c=0.018, $CH_3OH$)

Example 181

Methyl 4-((2R)-2-(methoxycarbonyl)-2-((phenylmethoxy)carbonylamino)ethylthio) (2S)-2-((2, 6-dichlorophenyl) carbonylamino)butyrate (181)

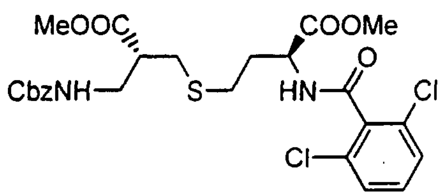

(181)

To a solution of 350 mg (0.72 mmol) of methyl 4-((2R)-2-(methoxycarbonyl)-2-((phenylmethoxy)carbonylamino)ethylthio) (2S)-2-((tert-butoxy)carbonylamino)butyrate (177) in 5.0 ml of dichloromethane, 2.0 ml of TFA was added and the mixture was stirred for 1 hour. After concentrating the reaction mixture, the residue was dissolved in 3.0 ml of dichloromethane, and then 0.39 ml (2.8 mmol) of triethylamine and 0.206 ml (1.44 mmol) of 2,6-dichlorobenzoyl chloride were added, followed by stirring the mixture overnight at room temperature. To the reaction solution, 1N hydrochloric acid was added and the mixture was extracted with ethyl acetate. Organic layers were combined, washed with saturated brine, dried over anhydrous sodium sulfate and concentrated. The residue was purified by column chromatography (hexane/ethyl acetate = 2/1-1/2) to obtain 353 mg of methyl 4-((2R)-2-(methoxycarbonyl)-2-((phenylmethoxy)carbonylamino)ethylthio) (2S)-2-((2, 6-dichlorophenyl)carbonylamino)butyrate (181) as colorless oil (yield: 88%).
LR-MS (m/z) : 571 [$(M+H)^+$]
$^1$H-NMR (300MHz, $CDCl_3$, δppm) 7.38-7.24 (8H, m), 6.73 (0.5H, br.d, *J* = 7.9 Hz), 6.56 (0.5H, br.d, *J* = 7.7 Hz), 5.63 (1H, m), 5.09 (1H, s), 5.08 (1H, s), 4.93 (1H, m), 4.58 (1H, m), 3.79 (3H, s), 3.77 (3H, s), 3.02-2.88 (2H, m), 2.78-2.60 (2H, m), 2.30 (1H, m), 2.10 (1H, m)

Example 182

Methyl 4-((2R)-2-amino-2-(methoxycarbonyl)ethylthio) (2S)-2-((2, 6-dichlorophenyl)carbonylamino)butyrate (182)

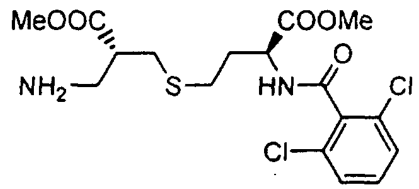

(182)

To a solution of 263 mg (0.47 mmol) of methyl 4-((2R)-2-(methoxycarbonyl)-2-((phenylmethoxy)carbonylamino)ethylthio) (2S)-2-((2, 6-dichlorophenyl)carbonylamino)butyrate (181) in 1.0 ml of dichloromethane, 2.0 ml of 30% hydrogen bromide/acetic acid was added at 0°C, and the mixture was stirred for 30 minutes. Water was added to the reaction mixture, and the resulting mixture was shaken with ethyl acetate. Saturated aqueous potassium carbonate solution was added to the aqueous layer and the resulting mixture was extracted with chloroform. Organic layers were combined, washed with saturated brine, dried over anhydrous sodium sulfate and concentrated to obtain 260 mg of methyl 4-((2R)-2-amino-2-(methoxycarbonyl)ethylthio) (2S)-2-((2, 6-dichlorophenyl)carbonylamino)butyrate (182). The product (182) was used in the next reaction without purification.

Example 183

Methyl 4-((2R)-2-((2, 6-dimethoxyphenyl)carbonylamino)-2-(methoxycarbonyl)ethylthio) (2S)-2-((2, 6-dichlorophenyl) carbonylamino)butyrate (183)

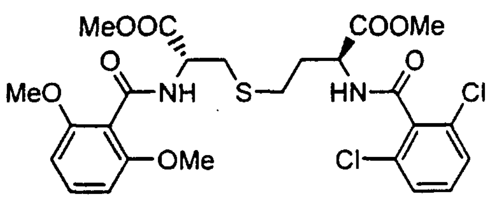

(183)

To a solution of 130 mg of methyl 4-((2R)-2-amino-2-(methoxycarbonyl)ethylthio) (2S)-2-((2, 6-dichlorophenyl) carbonylamino)butyrate (182) in 3.0 ml of dichloromethane, were added 56 mg (0.31 mmol) of 2,6-dimethoxybenzoic acid, 136 mg (0.31 mmol) of BOP reagent and 0.164 ml (0.94 mmol) of diisopropylethylamine, and the mixture was stirred overnight at room temperature. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (hexane/ethyl acetate = 2/1-1/3) to obtain 105 mg of methyl 4-((2R)-2-((2, 6-dimethoxyphenyl)carbonylamino)-2-(methoxycarbonyl)ethylthio) (2S)-2-((2, 6-dichlorophenyl)carbonylamino)butyrate (183) as colorless oil (yield: 76%).
LR-MS (m/z) : 587 [$(M+H)^+$]
$^1$H-NMR (300MHz, $CDCl_3$, δppm) : 7.34-7.20 (4H, m), 6.72 (1H, m), 6.64 (1H, m), 5.07 (1H, m), 4.92 (1H, m), 3.81 (6H, s), 3.80 (3H, s), 3.79 (3H, s), 3.25-3.01 (2H, m), 2.80-2.70 (2H, m), 2.32 (1H, m), 2.15 (1H, m)

Example 184

4-((2R)-2-((2, 6-dimethoxyphenyl)carbonylamino)-2-carboxyethylthio) (2S)-2-((2, 6-dichlorophenyl)carbonylamino) butyric acid (184)

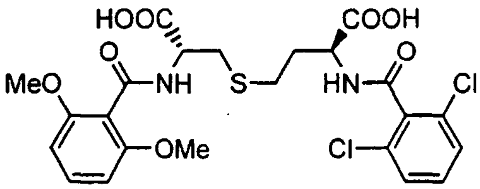

(184)

To a solution of 90 mg (0.15 mmol) of methyl 4-((2R)-2-((2, 6-dimethoxyphenyl)carbonylamino)-2-(methoxycarbonyl)ethylthio) (2S)-2-((2, 6-dichlorophenyl)carbonylamino)butyrate (183) in 4.0 ml of methanol/THF(1/1), 1.0 ml of 1N aqueous sodium hydroxide solution was added, and the mixture was stirred overnight at room temperature. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 20/80-5/95) to obtain 68 mg of 4-((2R)-2-((2, 6-dimethoxyphenyl)carbonylamino)-2-carboxyethylthio) (2S)-2-((2, 6-dichlorophenyl)carbonylamino)butyric acid (184) as an amorphous product (yield: 80%).
LR-MS (m/z) : 559 [$(M+H)^+$)
$^1$H-NMR (300MHz, $CDCl_3$, δppm) : 7.36-7.20 (4H, m), 6.55 (1H, d, *J* = 7.1 Hz). 6.52 (1H, d, *J* = 7.1 Hz), 4.99 (1H, m), 4.85 (1H, m), 3.81 (3H, s), 3.81 (3H, s), 3.34-3.07 (2H, m), 2.84-2.72 (2H, m), 2.32 (1H, m), 2.16 (1H, m)

Example 185

Methyl (2R)-3-((2R)-2-((tert-butoxy)carbonylamino)-2-(methoxycarbonyl)ethylthio) -2-((phenylmethoxy) carbonylamino)propionate (185)

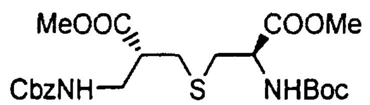

(185)

To a solution of 539 mg (2.0 mmol) of N-Cbz-cystein methyl ester and 607 mg (1.8mmol) of N-((3S)-2-oxooxetan)-3-yl)(tert-butoxy)carboxamide in 10 ml of DMF, 652 mg (2.0 mmol) of cesium carbonate was added, and the mixture was stirred overnight at room temperature. To the reaction mixture, 1N hydrochloric acid was added and the resulting mixture was extracted with ethyl acetate. Organic layers were combined, washed with saturated brine, dried over anhydrous sodium sulfate and concentrated. The residue was dissolved in 5 ml of methanol and 3 ml of trimethylsilyldizaomethane (2.0 M solution in hexane) was added, followed by stirring the mixture for 30 minutes. The reaction solution was concentrated and the residue was purified by column chromatography (silica gel; hexane/ethyl acetate = 3/1-2/1) to obtain 270 mg of methyl (2R)-3-((2R)-2-((tert-butoxy)carbonylamino)-2-(methoxycarbonyl)ethylthio)-2-((phenylmethoxy)carbonylamino)propionate (185) as colorless oil (yield: 32%).
LR-MS (m/z) : 484 ($M^+$)
$^1$H-NMR (300MHz, $CDCl_3$, δppm) : 7.40-7.32 (5H, m), 5.66 (1H, br.s), 5.35 (1H, br.s), 5.13 (2H, s), 4.60 (1H, m), 4.51 (1H, m), 3.78 (3H, br.s), 3.75 (3H, s), 3.10-2.88 (4H, m), 1.44 (9H, s)

Example 186

Methyl (2R)-3-((2R)-2-((2,6-dichlorophenyl)carbonylamino)-2-(methoxycarbonyl)ethylthio)-2-((phenylmethoxy) carbonylamino)propionate (186)

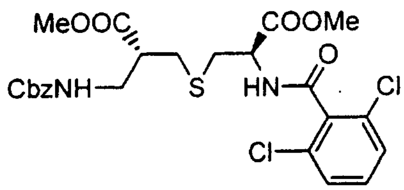

(186)

To a solution of 214 mg (0.46 mmol) of methyl (2R)-3-((2R)-2-((tert-butoxy)carbonylamino)-2-(methoxycarbonyl)ethylthio)-2-((phenylmethoxy)carbonylamino)propionate (185) in 2.0 ml of dichloromethane, 1 ml of trifluoroacetic acid was added and the mixture was stirred for 2 hours, followed by concentration of the mixture under reduced pressure. The residue was dissolved in 3.0 ml of dichloromethane, and then 254 ml (1.8 mmol) of triethylamine and 0.13 ml (0.91 mmol) of 2,6-dichlorophenylbenzoyl chloride were added, followed by stirring the mixture overnight at room temperature. To the reaction mixture, 1N hydrochloric acid was added and the resulting mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 3/2-1/1) to obtain 247 mg of methyl (2R)-3-((2R)-2-((2,6-dichlorophenyl) carbonylamino)-2-(methoxycarbonyl)ethylthio) -2-((phenylmethoxy)carbonylamino)propionate (186) as colorless oil (yield: 100%).
LR-MS (m/z) : 557 [(M+H)$^+$]
$^1$H-NMR (300MHz, $CDCl_3$, δppm) : 7.40-7.24 (8H, m), 6.85 (1H, br.d, *J* = 6.9 Hz), 5.59 (1H, br.d, *J* = 8.8 Hz), 5.12-5.02 (3H, m), 4.60 (1H, m), 3.81 (3H, s), 3.76 (3H, br.s), 3.20-2.95 (4H, m)

Example 187

Methyl (2R)-3-((2R)-2-((2,6-dichlorophenyl)carbonylamino)-2-(methoxycarbonyl)ethylthio)-2-((2,6-dimethoxyphenyl) carbonylamino)propionate (187)

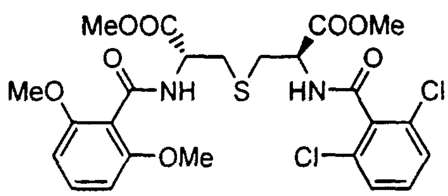

(187)

To a solution of 152 mg (0.28 mmol) of methyl (2R)-3-((2R)-2-((2,6-dichlorophenyl)carbonylamino)-2-(methoxycarbonyl)ethylthio) -2-((phenylmethoxy)carbonylamino)propionate (186) in 1.0 ml of dichloromethane, 2.0 ml of 30% hydrogen bromide/acetic acid solution was added, and the mixture was stirred for 30 minutes. After concentrating the reaction mixture, the residue was dissolved in 3.0 ml of dichloromethane, and then 66 mg (0.36 mmol) of 2,6-dimethoxybenzoic acid, 161 mg (0.36 mmol) of BOP reagent and 0.195 ml (1.12 mmol) of diisopropylethylamine were added, followed by stirring the resulting mixture overnight at room temperature. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (silica gel; hexane/ethyl acetate = 2/1-1/2) to obtain 97 mg of methyl (2R)-3-((2R)-2-((2,6-dichlorophenyl) carbonylamino)-2-(methoxycarbonyl)ethylthio) -2-((2,6-dimethoxyphenyl)carbonylamino)propionate (187) as colorless oil (yield: 60%).
LR-MS (m/z) : 573 [(M+H)$^+$]
$^1$H-NMR (300MHz, $CDCl_3$, δppm) : 7.35-7.24 (4H, m), 6.78 (1H, br.d, *J* = 7.1 Hz), 6.68 (1H, br.d, *J* = 8.0 Hz), 6.55 (2H,

m), 5.16-5.04 (2H, m), 3.82 (9H, s), 3.79 (3H, s), 3.32-3.20 (3H, m), 3.13 (1H, dd, *J* = 14.0, 4.4 Hz)

Example 188

(2R)-3-((2R)-2-((2,6-dichlorophenyl)carbonylamino)-2-carboxyethylthio)-2-((2,6-dimethoxyphenyl)carbonylamino) propionic acid (188)

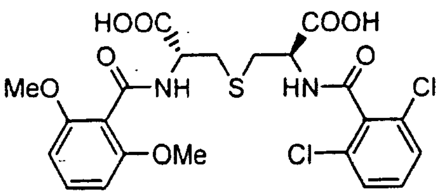

(188)

To a solution of 80 mg (0.14 mmol) of methyl (2R)-3-((2R)-2-((2,6-dichlorophenyl)carbonylamino)-2-(methoxycarbonyl)ethylthio) -2-((2,6-dimethoxyphenyl)carbonylamino)propionate (187) in 4 ml of methanol/THF (1/1), 1 ml of 1N aqueous sodium hydroxide solution was added, and the mixture was stirred overnight at room temperature. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 40/60-20/80) to obtain 47 mg of (2R)-3-((2R)-2-((2,6-dichlorophenyl)carbonylamino)-2-carboxyethylthio) -2-((2,6-dimethoxyphenyl)carbonylamino)propionic acid (188) as an amorphous product (yield: 61%).
LR-MS (m/z) : 555 [$(M+H)^+$]
$^1$H-NMR (300MHz, $CD_3OD$, δppm) : 7.44-7.28 (4H, m), 6.66 (2H, d, *J* = 8.5 Hz), 4.88-4.80 (2H, m), 3.81 (6H, s), 3.28-3.04 (4H, m)

Example 189

((2S)-1-(2-cyclohexylacetyl)pyrrolidin-2-yl)methyl=methane sulfonate (189)

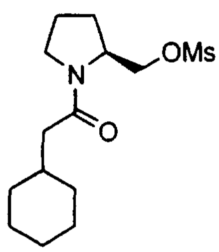

(189)

A solution of 450 mg (2.00 mmol) of (S)-2-cyclohexyl-1-(2-(hydroxymethyl)piperidinyl)ethan-1-one (134) in 10 ml of dichloromethane was cooled to 0°C, and 0.56 ml (4.00 mmol) of triethylamine and 0.31 ml (4.00 mmol) of methanesulfonyl chloride were added, followed by stirring the mixture for 1 hour. Water was added to the reaction mixture and the resulting mixture was extracted with dichloromethane. Organic layers were combined, washed with saturated brine, dried over anhydrous sodium sulfate and concentrated. The residue was purified by column chromatography (silica gel; chloroform) to obtain 488 mg of ((2S)-1-(2-cyclohexylacetyl)pyrrolidin-2-yl)methyl=methane sulfonate (189) (yield: 84%).
LR-MS (m/z) : 303 ($M^+$)
$^1$H-NMR (300MHz, $CDCl_3$, δppm) : 0.87-1.35(6H, m), 1.60-1.88(10H, m), 2.09-2.18(3H, m), 2.72(1H, s), 2.97(1H, s), 3.12(1H, s), 3.36-3.53(2H, m), 4.30(1H, s)

Example 190

Methyl (2R)-3-(((2S)-1-(2-cyclohexylacetyl)pyrrolidin-2-yl)methylthio)-2-((tert-butoxy)carbonylamino)propionate (190)

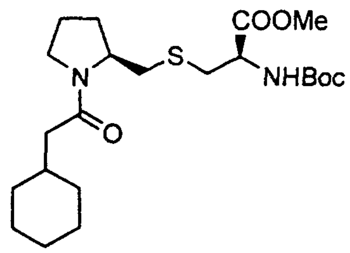

(190)

To a solution of 673 mg (2.86 mmol) of N-Boc-L-cystein methyl ester in 20 ml of DMF, 385 mg (3.43 mmol) of potassium tert-butoxide was added, and the mixture was stirred at 30°C for 30 minutes. Then a solution of 868 mg (2.86 mmol) of ((2S)-1-(2-cyclohexylacetyl)pyrrolidin-2-yl)methyl=methane sulfonate (189) in 10 ml of DMF was added, and the mixture was stirred for 3 hours. Saturated aqueous ammonium chloride solution was added to the reaction mixture and the resulting mixture was extracted with ethyl acetate. Organic layers were combined, washed with saturated brine, dried over anhydrous sodium sulfate and concentrated. The residue was purified by column chromatography (silica gel; hexane/ethyl acetate = 3/1-2/1) to obtain 298 mg of methyl (2R)-3-(((2S)-1-(2-cyclohexylacetyl)pyrrolidin-2-yl)methylthio)-2-((tert-butoxy)carbonylamino)propionate (190) (yield: 24%).
LR-MS (m/z) : 442 ($M^+$)
$^1$H-NMR (300MHz, $CDCl_3$, δppm) : 0.86-1.01(6H, m), 1.44(9H, s), 1.65-1.97(11H, m), 2,11(2H, d, $J$ = 6.6 Hz), 2.55(1H, dd, $J$ = 12.9,9.3 Hz), 2.91-3.10(3H, m), 3.39-3.49(2H, m), 3.75(3H, s), 4.12-4.22(1H, m), 4.47-4.58(1H, m)

Example 191

Methyl (2R)-3-(((2S)-1-(2-cyclohexylacetyl)pyrrolidin-2-yl)methylthio)-2-aminopropionate (191)

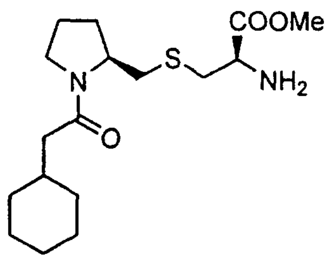

(191)

To a solution of 298 mg of methyl (2R)-3-(((2S)-1-(2-cyclohexylacetyl)pyrrolidin-2-yl)methylthio)-2-((tert-butoxy)carbonylamino)propionate (190) in 5 ml of dichloromethane, 0.15 ml of TFA was added, and the mixture was stirred at room temperature for 2.5 hours. Saturated aqueous sodium hydrogen carbonate solution was added to the reaction mixture and the resulting mixture was extracted with dichloromethane. Organic layers were combined, washed with saturated brine, dried over anhydrous sodium sulfate and concentrated to obtain 195 mg of methyl (2R)-3-(((2S)-1-(2-cyclohexylacetyl)pyrrolidin-2-yl)methylthio)-2-aminopropionate (191). The product (191) was used in the next reaction without purification.

Example 192

Methyl (2R)-3-(((2S)-1-(2-cyclohexylacetyl)pyrrolidin-2-yl)methylthio)-2-(3,3-dimethylbutanoylamino)propionate (192)

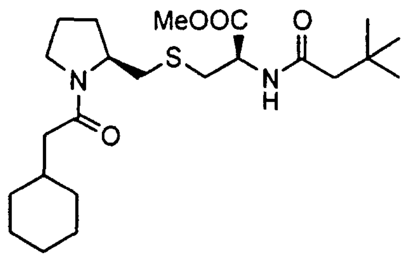

(192)

To a solution of 94 mg (0.27 mmol) of methyl (2R)-3-(((2S)-1-(2-cyclohexylacetyl)pyrrolidin-2-yl)methylthio)-2-aminopropionate (191) in 5 ml of dichloromethane, were added 0.076 ml (0.55 mmol) of triethylamine and 0.076 ml (0.55 mmol) of 3,3-dimethylbutanoyl chloride, and the mixture was stirred at room temperature for 1 hour. Saturated aqueous sodium hydrogen carbonate solution was added to the reaction mixture and the resulting mixture was extracted with ethyl acetate. Organic layers were combined, washed with saturated brine, dried over anhydrous sodium sulfate and concentrated. The residue was purified by column chromatography (silica gel; hexane/ethyl acetate = 2/1) to obtain 55 mg of methyl (2R)-3-(((2S)-1-(2-cyclohexylacetyl)pyrrolidin-2-yl)methylthio)-2-(3,3-dimethylbutanoylamino)propionate (192) (yield: 34%).
LR-MS (m/z) : 440 ($M^+$)
$^1$H-NMR (300MHz, $CDCl_3$, δppm) : 0.90-1.20(13H, m), 1.65-1.94(12H, m), 2.11-2.18(4H, m), 2.47(1H, dd, *J* = 13.5, 9.0 Hz), 2.91-3.00(2H, m), 3.09(1H, dd, *J* = 14.1, 3.9 Hz), 3.34-3.50(2H, m), 3.73(3H, s), 4.13-4.22(1H, m), 4.79-4.86 (1H, m)

Example 193

(2R)-3-(((2S)-1-(2-cyclohexylacetyl)pyrrolidin-2-yl)methylthio)-2-(3,3-dimethylbutanoylamino)propionic acid (193)

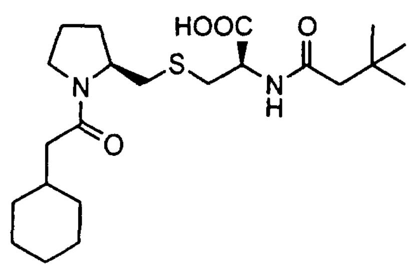

(193)

To a solution of 55 mg (0.13 mmol) of methyl (2R)-3-(((2S)-1-(2-cyclohexylacetyl)pyrrolidin-2-yl)methylthio)-2-(3,3-dimethylbutanoylamino)propionate (192) in 5 ml of methanol, 0.13 ml (0.13 mmol) of 1N aqueous sodium hydroxide solution was added, and the mixture was stirred at room temperature for 1 hour. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 1/3) to obtain 36 mg of (2R)-3-(((2S)-1-(2-cyclohexylacetyl)pyrrolidin-2-yl)methylthio)-2-(3,3-dimethylbutanoylamino)propionic acid (193) (yield: 68%).
LR-MS (m/z) : 420 ($M^+$)
$^1$H-NMR (300MHz, $CDCl_3$, δppm) : 0.90-1.19(13H, m), 1.65-1.94(12H, m), 2.13-2.15(4H, m), 2.54(1H, dd, *J* = 13.5, 9.6 Hz), 2.87-2.96(1H, m), 3.07-3.13(1H, m), 3.27(1H, dd, *J* = 14.3, 3.3 Hz), 3.38-3.52(1H, m), 4.20-4.28(1H, m), 4.81-4.89(1H, m), 8.67-9.15(1H, br)

Example 194

Methyl (2R)-2-((2S)-3,3-dimethyl-2-(phenylmethoxy)butanoylamino)-3-(((2S)-1-(2-cyclohexylacetyl)pyrrolidin-2-yl) methylthio)propionate (194)

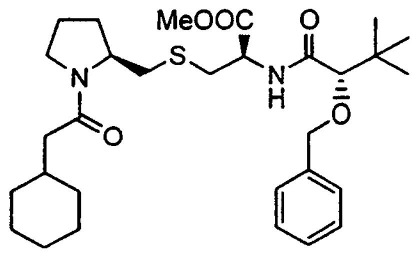

(194)

To a solution of 108 mg of methyl (2R)-3-(((2S)-1-(2-cyclohexylacetyl)pyrrolidin-2-yl)methylthio)-2-aminopropionate (191) in 3 ml of DMF, were added 70 mg of (2S)-3,3-dimethyl-2-(phenylmethoxy)butyric acid, 70 mg of PyBOP and 0.042 ml of *N*-methylmorpholine, and the mixture was stirred at room temperature for 1.5 hours. To the reaction mixture, 1N hydrochloric acid was added and the resulting mixture was extracted with ethyl acetate. Organic layers were combined, washed with saturated aqueous sodium hyrogen carbonate solution and saturated brine, dried over anhydrous sodium sulfate and concentrated. The residue was purified by column chromatography (silica gel; hexane/ethyl acetate = 3/1-1/1) to obtain 136 mg of methyl (2R)-2-((2S)-3,3-dimethyl-2-(phenylmethoxy)butanoylamino)-3-(((2S)-1-(2-cyclohexylacetyl)pyrrolidin-2-yl)methylthio)propionate (194) (yield: 52%).
LR-MS (m/z) : 546 ($M^+$)
$^1$H-NMR (300MHz, $CDCl_3$, δppm) : 0.90-1.01(9H, m), 1.09-1.29(4H, m), 1.65-1.95(11H, m), 2.09(2H, dd, *J* = 6.6, 5.1 Hz), 2.39-2.51(1H, m), 2.87-3.18(3H, m), 3.34-3.47(2H, m), 3.50(1H, d, *J* = 5.5 Hz), 3.75(3H, d, *J* = 6.6 Hz), 4.10-4.22 (1H, m), 4.38(1H, dd, *J* = 10.1, 4.8 Hz), 4.71(1H, dd, *J* = 10.4, 6.9 Hz), 4.80-4.87(1H, m), 7.14-7.19(1H, m), 7.29-7.42 (5H, m)

Example 195

(2R)-3-(((2S)-1-(2-cyclohexylacetyl)pyrrolidin-2-yl)methylthio)-2-(3,3-dimethylbutanoylamino)propionic acid (195)

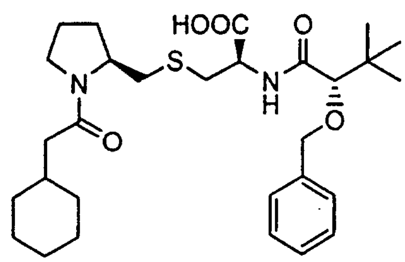

(195)

To a solution of 136 mg (0.25 mmol) of methyl (2R)-2-((2S)-3,3-dimethyl-2-(phenylmethoxy)butanoylamino)-3-(((2S)-1-(2-cyclohexylacetyl)pyrrolidin-2-yl)methylthio)propionate (194) in 5 ml of methanol, 0.50 ml (0.50 mmol) of 1N aqueous sodium hydroxide solution was added, and the mixture was stirred at room temperature for 8 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 2/3) to obtain 109 mg of (2R)-3-(((2S)-1-(2-cyclohexylacetyl)pyrrolidin-2-yl)methylthio)-2-(3,3-dimethylbutanoylamino)propionic acid (195) (yield: 82%).
LR-MS (m/z) : 532 ($M^+$)
$^1$H-NMR (300MHz, $CDCl_3$, δppm) : 1.00(9H, d, *J* = 3.6 Hz), 1.10-1.29(4H, m), 1.60-2.03(11H, m), 2.09-2.16(2H, m), 2.19-2.48(1H, m), 2.84-3.25(3H, m), 3.33-3.46(2H, m), 3.50(1H, d, *J* = 8.0 Hz), 4.18-4.22(1H, m), 4.38(1H, dd, *J* = 13.5, 13.5 Hz), 4.73(1H, dd, *J* = 11.1, 2.1 Hz), 4.86-5.05(1H, m), 7.27-7.45(5H, m)

Example 196

Methyl (2R)-3-(((2S)-1-(2-cyclohexylacetyl)pyrrolidin-2-yl)methylthio)-2-((2S)-4-methyl-2-(phenylmethoxy) pentanoylamino)propionate (196)

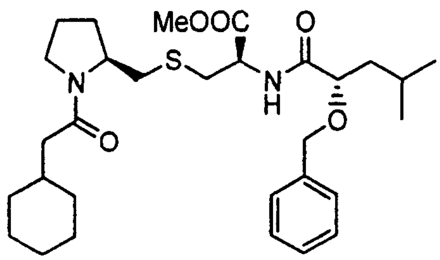

(196)

To a solution of 78 mg (0.23 mmol) of methyl (2R)-3-(((2S)-1-(2-cyclohexylacetyl)pyrrolidin-2-yl)methylthio)-2-aminopropionate (191) in 3 ml of DMF, were added 51 mg (0.23 mmol) of (2S)-4-methyl-2-(phenylmethoxy)valeric acid, 132 mg (0.25 mmol) of PyBOP and 0.030 ml (0.28 mmol) of *N*-methylmorpholine, and the mixture was stirred at room temperature for 1.5 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (silica gel; hexane/ethyl acetate = 3/2-2/1) to obtain 84 mg of methyl (2R)-3-(((2S)-1-(2-cyclohexylacetyl)pyrrolidin-2-yl)methylthio)-2-((2S)-4-methyl-2-(phenylmethoxy)pentanoylamino)propionate (196) (yield: 57%).
LR-MS (m/z) : 546 ($M^+$)
$^1$H-NMR (300MHz, $CDCl_3$, δppm) : 0.82-0.93(6H, m), 1.52-1.93(18H, m), 2.07-2.11(2H, m), 2.40-2.54(1H, m), 2.88-3.15(3H, m), 3.39-3.45(2H, m), 3.75-3.77(3H, m), 3.87-3.93(1H, m), 4.18(1H, s), 4.44(1H, dd, *J* = 3.0, 10.8 Hz), 4.70(1H, dd, *J* = 7.2, 10.8 Hz), 4.80-4.84(1H, m), 7.26-7.39(5H, m), 8.01(1H, s)

Example 197

(2R)-3-(((2S)-1-(2-cyclohexylacetyl)pyrrolidin-2-yl)methylthio)-2-((2S)-4-methyl-2-(phenylmethoxy)pentanoylamino) propionic acid (197)

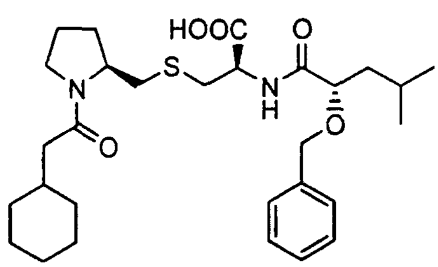

(197)

To a solution of 84 mg (0.15 mmol) of methyl (2R)-3-(((2S)-1-(2-cyclohexylacetyl)pyrrolidin-2-yl)methylthio)-2-((2S)-4-methyl-2-(phenylmethoxy)pentanoylamino)propionate (196) in 5 ml of methanol, 0.30 ml (0.30 mmol) of 1N aqueous sodium hydroxide solution was added, and the mixture was stirred at room temperature for 8 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 1/2) to obtain 64 mg of (2R)-3-(((2S)-1-(2-cyclohexylacetyl)pyrrolidin-2-yl)memylthio)-2-((2S)-4-methyl-2-(phenylmethoxy)pentanoylamino)propionic acid (197) (yield: 78%).
LR-MS (m/z) : 532 ($M^+$)
$^1$H-NMR (300MHz, $CDCl_3$, δppm) : 0.81-0.95(6H, m), 123-1.92(18H, m), 2.04-2.16(2H, m), 2.18-2.51(1H, m), 2.88-3.50 (5H, m), 3.90(1H, dt, *J* = 3.6, 9.6 Hz), 4.22-4.35(1H, m), 4.42(1H, dd, *J* = 7.8, 10.1 Hz), 4.71(1H,t, *J* = 10.4 Hz), 4.82-4.99(1H, m), 7.27-7.45(5H, m), 7.53(1H, d, *J* = 7.8 Hz)

Example 198

((2R)-1-(2-cyclohexylacetyl)pyrrolidin-2-yl)methyl=methanesulfonate(198)

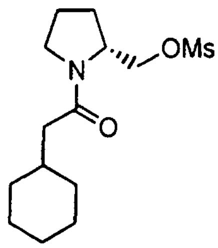

(198)

A solution of 1.13 g (5.00 mmol) of (R)-2-cyclohexyl-1-(2-(hydroxymethyl)piperidinyl)ethan-1-one in 30 ml of dichloromethane was cooled to 0°C, and 1.39 ml (10.00 mmol) of triethylamine and 0.77 ml (10.00 mmol) of methanesulfonyl chloride were added, followed by stirring the mixture for 1 hour. Water was added to the reaction mixture and the resulting mixture was extracted with dichloromethane. Organic layers were combined, washed with saturated brine, dried over anhydrous sodium sulfate and concentrated. The residue was purified by column chromatography (silica gel; hexane/ethyl acetate = 1/1) to obtain 1.04 g of ((2R)-1-(2-cyclohexylacetyl)pyrrolidin-2-yl)methyl=methanesulfonate (198) (yield: 68%).
LR-MS (m/z) : 303 ($M^+$)
$^1$H-NMR (300MHz, $CDCl_3$, δppm) : 0.87-1.35(6H, m), 1.60-1.88(10H, m), 2.09-2.18(3H, m), 2.72(1H, s), 2.97(1H, s), 3.12(1H, s), 3.36-3.53(2H, m), 4.30(1H, s)

Example 199

Methyl (2R)-3-(((2R)-1-(2-cyclohexylacetyl)pyrrolidin-2-yl)methylthio)-2-((tert-butoxy)carbonylamino)propionate (199)

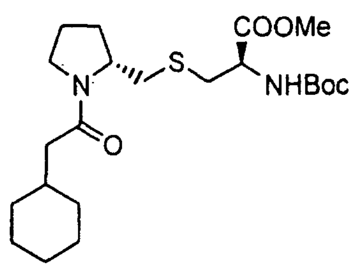

(199)

To a solution of 648 mg (2.75 mmol) of N-Boc-L-cystein methyl ester in 20 ml of DMF, 370 mg (3.30 mmol) of potassium tert-butoxide was added, and the mixture was stirred at 30°C for 15 minutes. To the reaction mixture, a solution of 1.04 g (3.43 mmol) of ((2R)-1-(2-cyclohexylacetyl)pyrrolidin-2-yl)methyl=methanesulfonate (198) in 10 ml of DMF was added, and the resulting mixture was stirred for 2 hours. Saturated aqueous ammonium chloride solution was added to the reaction mixture and the resulting mixture was extracted with ethyl acetate. Organic layers were combined, washed with saturated brine, dried over anhydrous sodium sulfate and concentrated. The residue was purified by column chromatography (silica gel; hexane/ethyl acetate = 3/1-2/1) to obtain 298 mg of methyl (2R)-3-(((2R)-1-(2-cyclohexylacetyl)pyrrolidin-2-yl)methylthio)-2-((tert-butoxy)carbonylamino)propionate (199) (yield: 24%).
LR-MS (m/z) : 442 ($M^+$)
$^1$H-NMR (300MHz, $CDCl_3$, δppm) : 0.87-0.98(6H, m), 1.43(9H, s), 1.65-2.02(10H, m), 2.11(2H, d, *J* = 6.6 Hz), 2.43 (1H, dd, *J* = 12.9, 9.6 Hz), 2.96(1H, dd, *J* = 3.0, 12.9 Hz), 3.04(1H, t, *J* = 2.4 Hz), 3.14(1H, s), 3.38-3.51(2H, m), 3.74 (3H, s), 4.13-4.23(1H, m), 4.47-4.57(1H, m)

Example 200

Methyl (2R)-3-(((2R)-1-(2-cyclohexylacetyl)pyrrolidin-2-yl)methylthio)-2-aminopropionate (200)

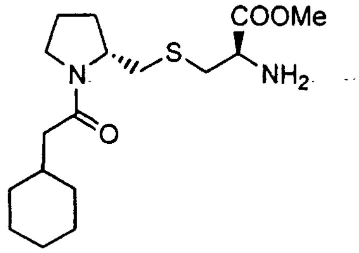

(200)

To a solution of 843 mg of methyl (2R)-3-(((2R)-1-(2-cyclohexylacetyl)pyrrolidin-2-yl)methylthio)-2-((tert-butoxy)carbonylamino)propionate (199) in 20 ml of dichloromethane, 2.0 ml of TFA was added, and the mixture was stirred at room temperature for 1.5 hours. Saturated aqueous sodium hydrogen carbonate solution was added to the reaction mixture and the resulting mixture was extracted with dichloromethane. Organic layers were combined, washed with saturated brine, dried over anhydrous sodium sulfate and concentrated to obtain 536 mg of methyl (2R)-3-(((2R)-1-(2-cyclohexylacetyl)pyrrolidin-2-yl)methylthio)-2-aminopropionate (200). The product (200) was used in the next reaction without purification.

Example 201

Methyl (2R)-3-(((2R)-1-(2-cyclohexylacetyl)pyrrolidin-2-yl)methylthio)-2-(3,3-dimethylbutanoylamino)propionate (201)

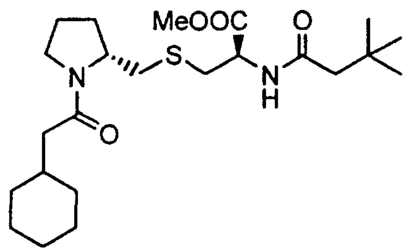

(201)

To a solution of 172 mg (0.50 mmol) of methyl (2R)-3-(((2R)-1-(2-cyclohexylacetyl)pyrrolidin-2-yl)methylthio)-2-aminopropionate (200) in 5 ml of dichloromethane, were added 0.14 ml (1.00 mmol) of triethylamine and 0.14 ml (1.00 mmol) of 3,3-dimethylbutanoyl chloride, and the mixture was stirred at room temperature for 1 hour. Saturated aqueous sodium hydrogen carbonate solution was added to the reaction mixture and the resulting mixture was extracted with ethyl acetate. Organic layers were combined, washed with saturated brine, dried over anhydrous sodium sulfate and concentrated. The residue was purified by column chromatography (silica gel; hexane/ethyl acetate = 2/1-1/1) to obtain 166 mg of methyl (2R)-3-(((2R)-1-(2-cyclohexylacetyl)pyrrolidin-2-yl)methylthio)-2-(3,3-dimethylbutanoylamino) propionate (201) (yield: 62%).
LR-MS (m/z) : 440 ($M^+$)
$^1$H-NMR (300MHz, $CDCl_3$, δppm) : 0.92-1.22(13H, m), 1.66-1.93(12H, m), 2.11-2.24(4H, m), 2.32(1H, dd, $J$ = 13.5, 9.9 Hz), 2.96-3.15(3H, m), 3.41-3.48(2H, m), 3.74(3H, s), 4.15-4.25(1H, m), 4.82-4.87(1H, m)

Example 202

(2R)-3-(((2S)-1-(2-cyclohexylacetyl)pyrrolidin-2-yl)methylthio)-2-(3,3-dimethylbutanoylamino)propionic acid (202)

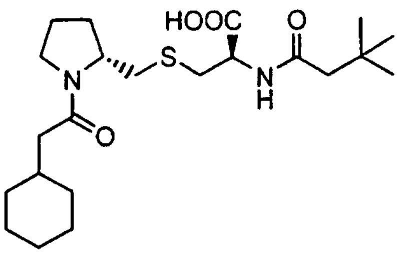

(202)

To a solution of 166 mg (0.38 mmol) of methyl (2R)-3-(((2R)-1-(2-cyclohexylacetyl)pyrrolidin-2-yl)methylthio)-2-(3,3-dimethylbutanoylamino)propionate (201) in 5 ml of methanol, 0.76 ml (0.76 mmol) of 1N aqueous sodium hydroxide solution was added, and the mixture was stirred at room temperature for 4 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 2/3-1/2) to obtain 92 mg of (2R)-3-(((2S)-1-(2-cyclohexylacetyl) pyrrolidin-2-yl)methylthio)-2-(3,3-dimethylbutanoylamino)propionic acid (202) (yield: 57%).
LR-MS (m/z) : 426 ($M^+$)
$^1$H-NMR (300MHz, $CDCl_3$, δppm) : 0.88-1.20(13H, m), 1.67-1.95(12H, m), 2.14-2.55(5H, m), 2.87-3.21(3H, m), 3.38-3.53(2H, m), 4.20-4.29(1H, m), 4.81-4.92(1H, m)

Example 203

Methyl (2R)-2-((2R)-3,3-dimethyl-2-(phenylmethoxy)butanoylamino)-3-(((2S)-1-(2-cyclohexylacetyl)pyrrolidin-2-yl) methylthio)propionate (203)

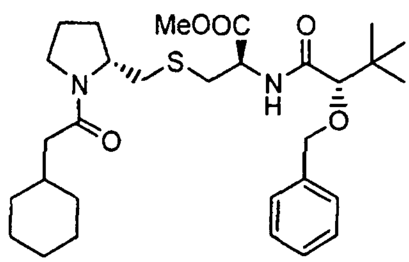

(203)

To a solution of 108 mg (0.31 mmol) of methyl (2R)-3-(((2R)-1-(2-cyclohexylacetyl)pyrrolidin-2-yl)methylthio)-2-aminopropionate (200) in 3 ml of DMF, were added 70 mg (0.31 mmol) of (2S)-3,3-dimethyl-2-(phenylmethoxy)butyric acid, 70 mg (0.35 mmol) of PyBOP and 0.042 ml (0.38 mmol) of N-methylmorpholine, and the mixture was stirred at room temperature for 2 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (silica gel; hexane/ethyl acetate = 3/1-2/1) to obtain 131 mg of methyl (2R)-2-((2R)-3,3-dimethyl-2-(phenylmethoxy)butanoylamino)-3 -(((2S)-1-(2-cyclohexylacetyl)pyrrolidin-2-yl)methylthio)propionate (203) (yield: 63%).
LR-MS (m/z) : 546 ($M^+$)
$^1$H-NMR (300MHz, $CDCl_3$, δppm) : 1.00(9H, d, $J$ = 4.5 Hz), 1.20-1.29(5H, m), 1.65-1.91(11H, m), 2.10(2H, d, $J$ = 6.9 Hz), 2.43(1H, dd, $J$ = 13.2, 12.6 Hz), 2.88-2.95(1H, m), 3.10(2H, d, $J$ = 5.3 Hz), 3.35-3.50(2H, m), 3.49(1H, s), 4.15-4.23 (1H, m), 4.38(1H, d, $J$ = 11.2 Hz), 4.73(1H, d, $J$ = 11.0 Hz), 4.84-4.86(1H, m), 7.30-7.42(5H, m)

Example 204

(2R)-3-(((2R)-1-(2-cyclohexylacetyl)pyrrolidin-2-yl)methylthio)-2-(3,3-dimethylbutanoylamino)propionic acid (204)

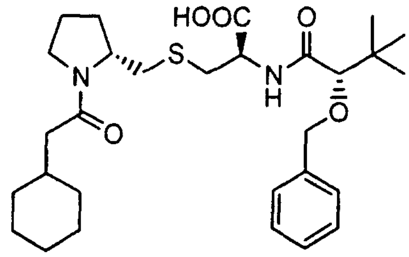

(204)

To a solution of 131 mg (0.24 mmol) of methyl (2R)-2-((2R)-3,3-dimethyl-2-(phenylmethoxy)butanoylamino)-3-(((2S)-1-(2-cyclohexylacetyl)pyrrolidin-2-yl)methylthio)propionate (203) in 5 ml of methanol, 0.72 ml (0.72 mmol) of 1N aqueous sodium hydroxide solution was added, and the mixture was stirred at room temperature for 8.5 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 2/1) to obtain 109 mg of (2R)-3-(((2R)-1-(2-cyclohexylacetyl)pyrrolidin-2-yl)methylthio)-2-(3,3-dimethylbutanoylamino)propionic acid (204) (yield: 82%).
LR-MS (m/z) : 532 ($M^+$)
$^1$H-NMR (300MHz, $CDCl_3$, δppm) : 1.00(9H, d, *J* = 2.2 Hz), 1.04-1.32(5H, m), 1.66-2.01(11H, m), 2.09-2.20(2H, m), 2.24-2.47(1H, m), 2.82-3.01(1H, m), 3.01-3.25(1H, m), 3.33-3.47(4H, m), 4.16-4.26(1H, m), 4.39(1H, dd, *J* = 14.0,13.8 Hz), 4.72(1H, dd, *J* = 11.1, 11.4 Hz), 5.04-5.80(1H, br), 7.29-7.41(5H, m)

Example 205

Methyl (2R)-3-(((2R)-1-(2-cyclohexylacetyl)pyrrolidin-2-yl)methylthio)-2-((2S)-4-methyl-2-(phenylmethoxy) pentanoylamino)propionate (205)

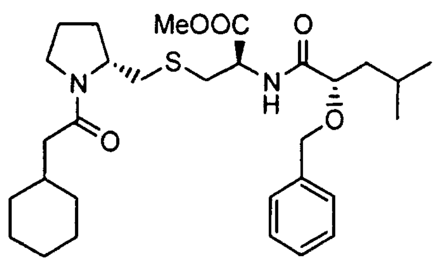

(205)

To a solution of 60 mg (0.175 mmol) of methyl (2R)-3-(((2R)-1-(2-cyclohexylacetyl)pyrrolidin-2-yl)methylthio)-2-aminopropionate (200) in 3 ml of DMF, were added 39 mg (0.175 mmol) of (2S)-4-methyl-2-(phenylmethoxy)valeric acid, 100 mg (0.193 mmol) of PyBOP and 0.023 ml (0.210 mmol) of N-methylmorpholine, and the mixture was stirred at room temperature for 2 hours. To the reaction mixture, 1N hydrochloric acid was added and the resulting mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 2/1) to obtain 35 mg of methyl (2R)-3-(((2R)-1-(2-cyclohexylacetyl)pyrrolidin-2-yl)methylthio)-2-((2S)-4-methyl-2-(phenylmethoxy)pentanoylamino)propionate (205) (yield: 30%).
LR-MS (m/z) : 546 ($M^+$)
$^1$H-NMR (300MHz, $CDCl_3$, δppm) : 0.86(6H, dd, *J* = 6.6, 23.4 Hz), 1.52-1.91(18H, m), 2.09(2H, d, *J* = 6.9 Hz), 2.43 (1H, dd, *J* = 9.3, 15.9 Hz), 2.92(1H, dd, *J* = 3.0, 13.2 Hz), 3.08-3.11(2H, m), 3.38-3.46(2H, m), 3.74-3.78(3H, m), 3.87-3.93(1H, m), 4.15-4.19(1H, m), 4.42(1H, d, *J* = 11.0 Hz), 4.70(1H, t, *J* = 10.5 Hz), 4.81-4.87(1H, m), 7.26-7.41(5H, m)

Example 206

(2R)-3-(((2R)-1-(2-cyclohexylacetyl)pyrrolidin-2-yl)methylthio)-2-((2S)-4-methyl-2-(phenylmethoxy)pentanoylamino) propionic acid (206)

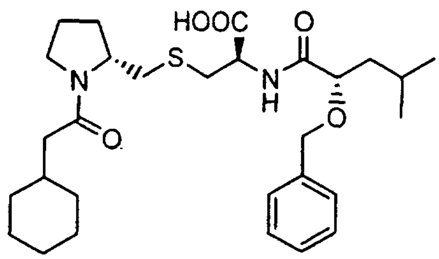

(206)

To a solution of 35 mg (0.064 mmol) of methyl (2R)-3-(((2R)-1-(2-cyclohexylacetyl)pyrrolidin-2-yl)methylthio)-2-((2S)-4-methyl-2-(phenylmethoxy)pentanoylamino)propionate (205) in 3 ml of methanol, 0.18 ml (0.18 mmol) of 1N aqueous sodium hydroxide solution was added, and the mixture was stirred at room temperature for 8 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 2/3) to obtain 24 mg of (2R)-3-(((2R)-1-(2-cyclohexylacetyl)pyrrolidin-2-yl)methylthio)-2-((2S)-4-methyl-2-(phenylmethoxy)pentanoylamino)propionic acid (206) (yield: 71%).
LR-MS (m/z) : 532 ($M^+$)
$^1$H-NMR (300MHz, $CDCl_3$, δppm) : 0.81-0.84(3H, m), 0.88-0.91(3H, m), 1,43-1,94(18H, m), 2.15(2H, d, *J* = 7.2 Hz), 2.24-2.49(1H, m), 2.90-3.15(3H, m), 3.34-3.50(2H, m), 3.88-3.93(1H, m), 4.20-4.32(1H, m), 4.42(1H, dd, *J* = 9.9, 11.1 Hz), 4.71(1H, t, *J* = 10.8 Hz), 4.84-4.89(1H, m), 7.28-7.43(5H, m), 7.51(1H, d, *J* = 2.7 Hz)

Example 207

Methyl (2R)-3-(((2R)-1-(2-cyclohexylacetyl)pyrrolidin-2-yl)methylthio)-2-(4-methylpentanoylamino)propionate (207)

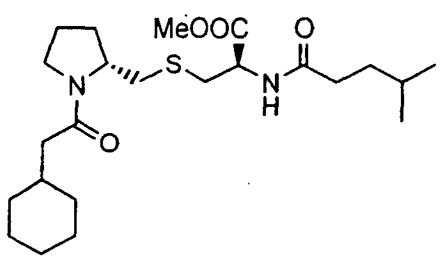

(207)

To a solution of 60 mg (0.175 mmol) of methyl (2R)-3-(((2R)-1-(2-cyclohexylacetyl)pyrrolidin-2-yl)methylthio)-2-aminopropionate (200) in 3 ml of DMF, were added 20 mg (0.175 mmol) of 4-methylvaleric acid, 100 mg (0.193 mmol) of PyBOP and 0.023 ml (0.210 mmol) of N-methylmorpholine, and the mixture was stirred at room temperature for 2 hours. To the reaction mixture, 1N hydrochloric acid was added and the resulting mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 2/1) to obtain 36 mg of methyl (2R)-3-(((2R)-1-(2-cyclohexylacetyl)pyrrolidin-2-yl)methylthio)-2-(4-methylpentanoylamino)propionate (207) (yield: 38%).
LR-MS (m/z) : 440 ($M^+$)

$^1$H-NMR (300MHz, $CDCl_3$, δppm) : 0.88(6H, dd, *J* = 1.5, 6.3 Hz), 1.48-1.97(18H, m), 2.10(2H, d, *J* = 6.6 Hz), 2.16-2.35 (3H, m), 2.86-3.01(2H, m), 3.09(1H, dd, *J* = 6.9, 17.4 Hz), 3.37-3.61(2H, m), 3.73-3.76(3H, m), 4.19(1H, s), 4.81-4.87 (1H, m), 7.06(1H, d, *J* = 7.5 Hz)

Example 208

(2R)-3-(((2R)-1-(2-cyclohexylacetyl)pyrrolidin-2-yl)methylthio)-2-(4-methylpentanoylamino)propionic acid (208)

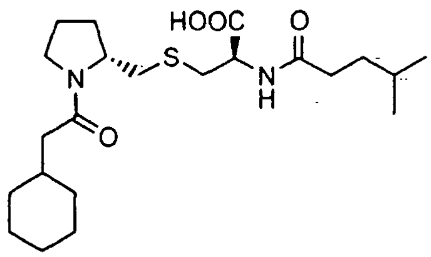

(208)

To a solution of 36 mg (0.081 mmol) of methyl (2R)-3-(((2R)-1-(2-cyclohexylacetyl)pyrrolidin-2-yl)methylthio)-2-(4-methylpentanoylamino)propionate (207) in 3 ml of methanol, 0.24 ml (0.24 mmol) of 1N aqueous sodium hydroxide solution was added, and the mixture was stirred at room temperature for 8 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was washed with hexane to obtain 8 mg of (2R)-3-(((2R)-1-(2-cyclohexylacetyl)pyrrolidin-2-yl)methylthio)-2-(4-methylpentanoylamino) propionic acid (208) (yield: 23%).
LR-MS (m/z) : 426 ($M^+$)
$^1$H-NMR (300MHz, $CDCl_3$, δppm) : 0.88-0.90(6H, m), 1.09-1.96(18H, m), 2.04-2.58(5H, m), 2.85-3.20(3H, m), 3.28-3.53(2H, m), 4.23-4.26(1H, m), 4.82-4.91(1H, m), 6.66(1H, d, *J* = 7.5 Hz)

Example 209

1-((2S)-2-(hydroxymethyl)pyrrolidinyl)-3-methylbutan-1-one (209)

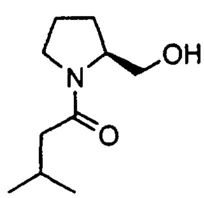

(209)

To a solution of 1.52 g (15.0 mmol) of (S)-pyrrolidin-2-ylmethan-1-ol in 100 ml of dichloromethane, were added 2.5 ml (18.0 mmol) of triethylamine and 1.83 ml (15.0 mmol) of 3-methylbutanoyl chloride, and the mixture was stirred at room temperature for 2 hours. Water was added to the reaction mixture and the resulting mixture was extracted with ethyl acetate. Organic layers were combined, washed with saturated brine, dried over anhydrous sodium sulfate and concentrated to obtain 2.76 g of 1-((2S)-2-(hydroxymethyl)pyrrolidinyl)-3-methylbutane-1-one (209). The product (209) was used in the next reaction without purification.
LR-MS (m/z) : 185($M^+$)
$^1$H-NMR (300MHz, $CDCl_3$, δppm) : 0.97(6H, dd, *J* = 6.6, 0.9 Hz), 1.52-1.61(1H, m), 1.79-2.09(4H, m), 2.18(2H, d, *J* = 2.0 Hz), 3.41-3.68(4H, m), 4.19-4.27(1H, m), 5.25(1H, d, *J* = 6.6 Hz)

Example 210

((2S)-1-(3-methylbutanoyl)pyrrolidin-2-yl)methyl=methanesulfonate (210)

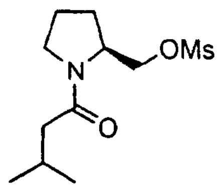

(210)

To a solution of 1.11 g (6.00 mmol) of 1-((2S)-2-(hydroxymethyl)pyrrolidinyl)-3-methylbutane-1-one (209) in 30 ml of dichloromethane, were added 1.67 ml (12.0 mmol) of triethylamine and 0.93 ml (12.0 mmol) of methanesulfonyl chloride at 0°C, and the mixture was stirred at room temperature for 1 hour. Water was added to the reaction mixture and the resulting mixture was extracted with dichloromethane. Organic layers were combined, washed with saturated brine, dried over anhydrous sodium sulfate and concentrated. The residue was purified by column chromatography (silica gel; hexane/ethyl acetate = 2/3-1/2) to obtain 646 mg of ((2S)-1-(3-methylbutanoyl)pyrrolidin-2-yl)methyl=methanesulfonate (210) (yield: 41%).
LR-MS (m/z) : 263 ($M^+$)
$^1$H-NMR (300MHz, $CDCl_3$, δ ppm) : 0.94-0.96(6H, m), 1.89-2.02(5H, m), 2.15(2H, d, *J* = 2.2 Hz), 2.75-3.14(3H, m), 3.41-3.52(2H, m), 4.29-4.40(3H, m)

Example 211

Methyl (2R)-3-(((2S)-1-(3-methylbutanoyl)pyrrolidin-2-yl)methylthio)-2-((tert-butoxy)carbonylamino)propionate (211)

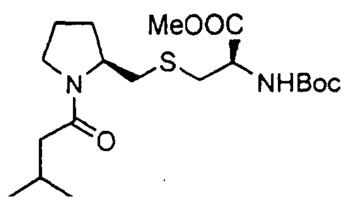

(211)

To a solution of 578 mg (2.46 mmol) of *N*-Boc-L-cystein methyl ester in 20 ml of DMF, 330 mg (2.95 mmol) of potassium tert-butoxide was added, and the mixture was stirred at 30°C for 20 minutes. To the reaction mixture, a solution of 646 mg (2.46 mmol) of ((2S)-1-(3-methylbutanoyl)pyrrolidin-2-yl)methyl=methanesulfonate (210) in 10 ml of DMF was added, and the mixture was stirred for 2 hours. Saturated aqueous ammonium chloride solution was added to the reaction mixture and the resulting mixture was extracted with ethyl acetate. Organic layers were combined, washed with saturated brine, dried over anhydrous sodium sulfate and concentrated. The residue was purified by column chromatography (silica gel; hexane/ethyl acetate = 3/1-2/1) to obtain 195 mg of methyl (2R)-3-(((2S)-1-(3-methylbutanoyl)pyrrolidin-2-yl)methylthio)-2-((tert-butoxy)carbonylamino)propionate (211) (yield: 20%).
LR-MS (m/z) : 402 ($M^+$)
$^1$H-NMR (300MHz, $CDCl_3$, δppm) : 0.95(1H, d, *J* = 6.3 Hz), 1.43(9H, d, *J* = 0.5 Hz), 1.76-2.01(5H, m), 2.09-2.19(2H, m), 2.44(1H, dd, *J* = 13.2, 9.3 Hz), 2.87-3.06(3H, m), 3.36-3.50(2H, m), 3.74(3H, d, *J* = 2.5 Hz), 4.12-4.22(1H, m), 4.51-4.55(1H, m)

Example 212

Methyl (2R)-3-(((2S)-1-(3-methylbutanoyl)pyrrolidin-2-yl)methylthio)-2-aminopropionate (212)

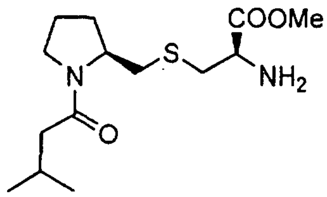

(212)

To a solution of 195 mg of methyl (2R)-3-(((2S)-1-(3-methylbutanoyl)pyrrolidin-2-yl)methylthio)-2-((tert-butoxy)carbonylamino)propionate (211) in 5 ml of dichloromethane, 0.5 ml of TFA was added, and the mixture was stirred at room temperature for 2 hours. Saturated aqueous sodium hydrogen carbonate solution was added to the reaction mixture, and the resulting mixture was extracted with dichloromethane. Organic layers were combined, washed with saturated brine, dried over anhydrous sodium sulfate and concentrated to obtain 107 mg of methyl (2R)-3-(((2S)-1-(3-methylbutanoyl)pyrrolidin-2-yl)methylthio)-2-aminopropionate (212). The product (212) was used in the next reaction without purification.

Example 213

Methyl (2R)-3-(((2S)-1-(3-methylbutanoyl)pyrrolidin-2-yl)methylthio)-2-(3,3-dimethylbutanoylamino)propionate (213)

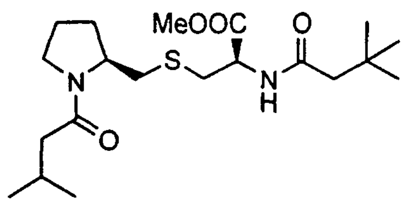

(213)

To a solution of 50 mg (0.165 mmol) of methyl (2R)-3-(((2S)-1-(3-methylbutanoyl)pyrrolidin-2-yl)methylthio)-2-aminopropionate (212) in 5 ml of dichloromethane, were added 0.046 ml (0.33 mmol) of triethylamine and 0.046 ml (0.33 mmol) of 3,3-dimethylbutanoyl chloride, and the mixture was stirred at room temperature for 1.5 hours. Saturated aqueous sodium hydrogen carbonate solution was added to the reaction mixture, and the resulting mixture was extracted with ethyl acetate. Organic layers were combined, washed with saturated brine, dried over anhydrous sodium sulfate and concentrated. The residue was purified by column chromatography (silica gel; hexane/ethyl acetate = 2/1) to obtain 58 mg of methyl (2R)-3-(((2S)-1-(3-methylbutanoyl)pyrrolidin-2-yl)methylthio)-2-(3,3-dimethylbutanoylamino) propionate (213) (yield: 64%).
LR-MS (m/z) : 400 ($M^+$)
$^1$H-NMR (300MHz, $CDCl_3$, δppm) : 0.96(6H, dd, *J* = 6.1, 2.7 Hz), 1.05(9H, s), 1.82-1.98(5H, m), 2.08-2.24(4H, m), 2.30-2.55(1H, m), 2.92-3.16(3H, m), 3.39-3.51(2H, m), 3.74(3H, d, *J* = 2.5 Hz), 4.15-4.23(1H, m), 4.82-4.89(1H, m)

Example 214

(2R)-3-(((2S)-1-(3-methylbutanoyl)pyrrolidin-2-yl)methylthio)-2-(3,3-dimethylbutanoylamino)propionic acid (214)

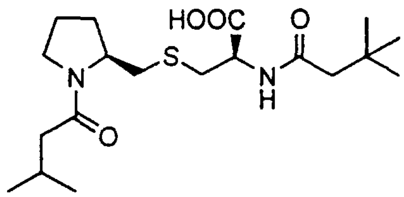

(214)

To a solution of 58 mg (0.145 mmol) of methyl (2R)-3-(((2S)-1-(3-methylbutanoyl)pyrrolidin-2-yl)methylthio)-2-(3,3-dimethylbutanoylamino)propionate (213) in 5 ml of methanol, 0.45 ml of 1N aqueous sodium hydroxide solution was added, and the mixture was stirred at room temperature for 6 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 2/3-1/2) to obtain 41 mg of (2R)-3-(((2S)-1-(3-methylbutanoyl)pyrrolidin-2-yl) methylthio)-2-(3,3-dimethylbutanoylamino)propionic acid (214) (yield: 73%).
LR-MS (m/z) : 386 ($M^+$)
$^1$H-NMR (300MHz, $CDCl_3$, δppm) : 0.94(6H, d, *J* = 6.3 Hz), 1.02(9H, d, *J* = 1.2 Hz), 1.83-2.00(5H, m), 2.09-2.21(4H, m), 2.22-2.57(1H, m), 2.88-2.96(1H, m), 3.08-3.29(2H, m), 3.39-3.52(2H, m), 4.21-4.23(1H, m), 4.80-4.88(1H, m)

Example 215

Methyl (2R)-2-((2S)-3,3-dimethyl-2-(phenylmethoxy)butanoylamino)-3-(((2S)-1-(3-methylbutanoylamino)pyrrolidin-2-yl)methylthio)propionate (215)

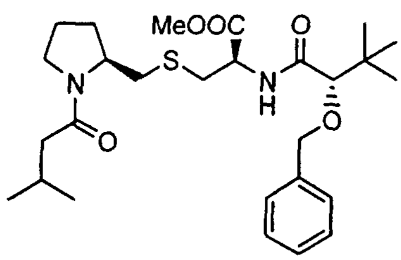

(215)

To a solution of 50 mg (0.165 mmol) of methyl (2R)-3-(((2S)-1-(3-methylbutanoyl)pyrrolidin-2-yl)methylthio)-2-aminopropionate (212) in 3 ml of DMF, were added 37 mg (0.165 mmol) of(2S)-3,3-dimethyl-2-(phenylmethoxy) butyric acid, 95 mg (0.182 mmol) of PyBOP and 0.022 ml (0.198 mmol) of *N*-methylmorpholine, and the mixture was stirred at room temperature for 1.5 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (silica gel; hexane/ ethyl acetate = 2/1) to obtain 74 mg of methyl (2R)-2-((2S)-3,3-dimethyl-2-(phenylmethoxy)butanoylamino)-3-(((2S)-1-(3-methylbutanoylamino)pyrrolidin-2-yl)methylthio)propionate (215) (yield: 64%).
LR-MS (m/z) : 506 ($M^+$)
$^1$H-NMR (300MHz, $CDCl_3$, δppm) : 0.94-1.00(6H, m), 1.01(9H, s), 1.84-1.96(5H, m), 2.07-2.18(2H, m), 2.42-2.54(1H, m), 2.89-3.18(3H, m), 3.35-3.47(2H, m), 3.50(1H, d, *J* = 5.2 Hz), 3.75(1H, d, *J* = 6.4 Hz), 4.13-4.17(1H, m), 4.39(1H, dd, *J* = 11.3, 5.1 Hz), 4.71(1H, dd, *J* = 11.1, 6.6 Hz), 4.81-4.87(1H, m), 7.29-7.42(5H, m)

Example 216

(2R)-2-((2S)-3,3-dimethyl-2-(phenylmethoxy)butanoylamino)-3-(((2S)-1-(3-methylbutanoyl)pyrrolidin-2-yl)methylthio) propionic acid (216)

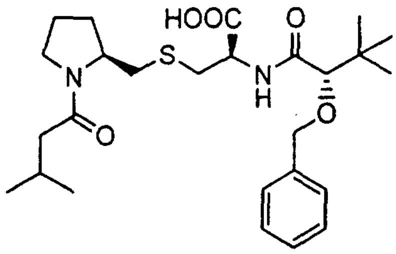

(216)

To a solution of 74 mg (0.146 mmol) of methyl (2R)-2-((2S)-3,3-dimethyl-2-(phenylmethoxy)butanoylamino)-3-(((2S)-1-(3-methylbutanoylamino)pyrrolidin-2-yl)methylthio)propionate (215) in 5 ml of methanol, 0.45 ml of 1N aqueous sodium hydroxide solution was added, and the mixture was stirred at room temperature for 6.5 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 2/3) to obtain 30 mg of(2R)-2-((2S)-3,3-dimethyl-2-(phenylmethoxy)butanoylamino)-3-(((2S)-1-(3-methylbutanoyl)pyrrolidin-2-yl)methylthio)propionic acid (216) (yield: 42%).
LR-MS (m/z) : 492 ($M^+$)
$^1$H-NMR (300MHz, $CDCl_3$, δppm) : 0.94(6H, t, $J$ = 6.0 Hz), 1.00(9H, d, $J$ = 2.1 Hz), 1.82-1.93(5H, m), 2.07-2.15(2H, m), 2.29-2.49(1H, m), 2.85-3.03(2H, m), 3.13-3.16(1H, m), 3.33(1H, dd, $J$ = 3.6, 14.1 Hz), 3.37-3.52(2H, m), 4.20-4.31 (1H, m), 4.37(1H, dd, $J$ = 12.9, 13.2 Hz), 4.71-4.75(1H, m), 4.82-7.99(1H, m), 7.27-7.43(5H, m), 7.45-7.70(1H, br)

Example 217

(2S)-2-((2,6-dichlorophenyl)carbonylamino)-5-((tert-butoxy)carbonylamino)valeric acid methyl ester (217)

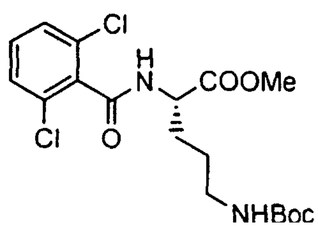

(217)

To a solution of 1.50 g (3.30 mmol) of Fmoc-Orn(Boc)-OH in 16.0 ml of methanol/toluene (1/1) solution, 3.5 ml of 2.0 M trimethylsilyldiazomethane solution in hexane was added. The reaction mixture was concentrated under reduced pressure and the residue was dissolved in 10 ml of DMF. To this solution, 0.35 ml of piperidine was added and the mixture was stirred at room temperature for 4 hours. After diluting the reaction mixture with 80 ml of water, 1 ml of 1N aqueous sodium hydroxide solution was added, and the resulting mixture was extracted with chloroform. Organic layers were combined, washed with saturated brine, dried over anhydrous sodium sulfate and concentrated under reduced pressure. The residue was purified by column chromatography (silica gel; cyclohexane/ethyl acetate = 50/50, chloroform/methanol = 99/1-5/1) to obtain colorless oil. This oily product was then dissolved in 25 ml of dichloromethane, and then 1.50 ml (10.8 mmol) of triethylamine and 0.75 ml (5.26 mmol) of 2,6-dichlorobenzoyl chloride were added, followed by stirring the mixture for 4 hours. To the reaction mixture, 50 ml of saturated aqueous sodium hydrogen carbonate solution was added, and the resulting mixture was extracted with ethyl acetate. Organic layers were combined, washed with 5% aqueous citric acid solution and saturated brine, dried over anhydrous sodium sulfate and concentrated under reduced pressure. The residue was purified by column chromatography (silica gel; cyclohexane/ethyl acetate = 80/20-50/50) to obtain 1.32 g of (2S)-2-((2,6-dichlorophenyl)carbonylamino)-5-((tert-butoxy)carbonylamino)valeric acid methyl ester (217) as an amorphous product (yield: 95%).
LR-MS (m/z) : 418 ($M^+$)
IR (KBr) : 3288, 3059, 2978, 2869, 1739, 1692, 1663, 1580, 1561, 1527, 1433, 1392, 1366, 1270, 1253, 1170, 1089, 1041, 1014, 991 $cm^{-1}$
$^1$H-NMR (300MHz, DMSO-d6, δppm) : 1.43(9H, s), 1.58-1.72(2H, m), 1.76-1.89(1H, m), 2.01-2.10(1H, m), 3.14-3.22 (2H, m), 3.80(3H, s), 4.62(1H, s), 4.84-4.91(1H, m), 6.54(1H, d, *J* = 6.3 Hz), 7.25-7.35(3H, m)

Example 218

(2S)-2-((2,6-dichlorophenyl)carbonylamino)-5-(((2,6-dimethylphenyl)amino)carbonylamino)valeric acid methyl ester (218)

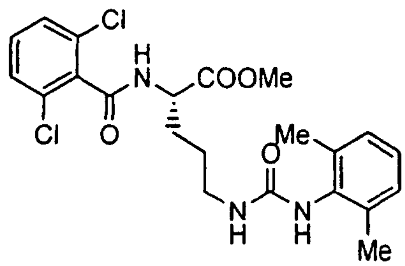

(218)

To a solution of 131.5 mg (0.313 mmol) of (2S)-2-((2,6-dichlorophenyl)carbonylamino)-5-((tert-butoxy)carbonylamino)valeric acid methyl ester (217) in 3.0 ml of dichloromethane, 0.24 ml of TFA was added, and the mixture was stirred at room temperature for 4 hours. After concentrating the reaction mixture, the residue was dissolved in 5.0 ml of dichloromethane, and then 0.090 ml (0.65 mmol) of triethylamine and 0.050 ml (0.36 mmol) of 2,6-dimethylphenylisocyanate were added, followed by stirring the mixture overnight at room temperature. To the reaction mixture, 10 ml of saturated aqueous sodium hydrogen carbonate solution was added, and the resulting mixture was extracted with ethyl acetate. Organic layers were combined, washed with saturated brine, dried over anhydrous sodium sulfate and concentrated under reduced pressure. The residue was washed with ether to obtain 130.2 mg of (2S)-2-((2,6-dichlorophenyl)carbonylamino)-5-(((2,6-dimethylphenyl)amino)carbonylamino)valeric acid methyl ester (218) (yield: 89%).
LR-MS (m/z) : 465 ($M^+$)
IR (KBr) : 3312, 3269, 3077, 2959, 2933, 2856, 1743, 1647, 1571, 1509, 1435, 1364, 1286, 1253, 1227, 1200, 1129, 1091, 1041, 997 $cm^{-1}$
$^1$H-NMR (300MHz, DMSO-d6, δppm) : 1.53-1.82(4H, m), 2.14(6H, s), 3.07(2H, dd, *J* = 6.3, 3.3 Hz), 3.66(3H, s), 4.42-4.49(1H, m), 6.14(1H, s), 7.01(3H, d, *J* = 0.9 Hz), 7.41-7.52(4H, m), 9.16(1H, d, *J* = 7.8 Hz)

Example 219

(2S)-2-((2,6-dichlorophenyl)carbonylamino)-5-(((2,6-dimethylphenyl)amino)carbonylamino)valeric acid (219)

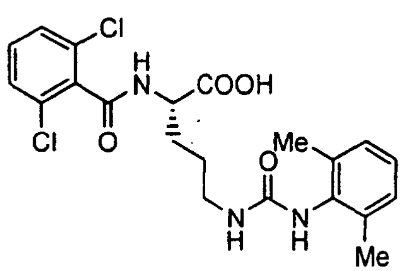

(219)

To a solution of 90.9 mg (0.194 mmol) of (2S)-2-((2,6-dichlorophenyl)carbonylamino)-5-(((2,6-dimethylpohenyl)amino)carbonylamino)valeric acid methyl ester (218) in 2.5 ml of DMF, 2.0 ml of 1N aqueous sodium hydroxide

solution was added, and the mixture was stirred at room temperature for 5 hours. To the reaction mixture, 8 ml of water and 1N hydrochloric acid were added, and the resulting mixture was extracted with ethyl acetate. Organic layers were combined, washed with saturated brine, dried over anhydrous sodium sulfate and concentrated. The residue was washed with ether to obtain 98.5 mg of (2S)-2-((2,6-dichlorophenyl)carbonylamino)-5-(((2,6-dimethylphenyl)amino) carbonylamino)valeric acid (219) (yield: 89%).
LR-MS (m/z) : 450 [$(M-H)^-$].
IR (KBr) : 3298, 3073, 2923, 2859, 1725, 1656, 1634, 1564, 1434, 1252, 1089 $cm^{-1}$
$^1$H-NMR (300MHz, DMSO-d6, δppm) : 1.51-1.82(4H, m), 2.14(6H, s), 3.07(2H, dd, *J* = 2.7, 5.7 Hz), 4.37-4.44(1H, m), 6.09(1H, s), 7.01(3H, d, *J* = 1.2 Hz), 7.39-7.51(4H, m), 9.02(1H, d, *J* = 7.8 Hz), 12.65(1H, s)
HR-MS : $C_{21}H_{22}Cl_2N_3O_4$ [$(M-H)^-$]     Calcd.: 450.0987,     Found: 450.0967

Example 220

(2S)-2-((2,6-dichlorophenyl)carbonylamino)-5-(((2,6-dichlorophenyl)amino)carbonylamino)valeric acid methyl ester (220)

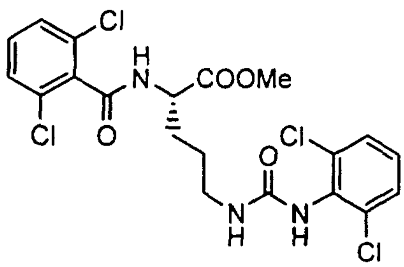

(220)

To a solution of 112.7 mg (0.268 mmol) of (2S)-2-((2,6-dichlorophenyl)carbonylamino)-5-((tert-butoxy)carbonylamino)valeric acid methyl ester (217) in 3.0 ml of dichloromethane, 0.20 ml of TFA was added, and the mixture was stirred overnight at room temperature. After concentrating the reaction mixture, 5.0 ml of dichloromethane was added to the residue, and then 0.075 ml (0.541 mmol) of triethylamine and 50.4 mg (0.268 mmol) of 2,6-dichlorophenylisocyanate were added, followed by stirring the mixture at room temperature for 2.5 hours. To the reaction mixture, 10 ml of saturated aqueous sodium hydrogen carbonate solution was added, and the resulting mixture was extracted with ethyl acetate. Organic layers were combined, washed with 0.1N hydrochloric acid and saturated brine, dried over anhydrous sodium sulfate and concentrated under reduced pressure. The residue was washed with ether to obtain 170.0 mg of (2S)-2-((2,6-dichlorophenyl)carbonylamino)-5-(((2,6-dichlorophenyl)amino)carbonylamino)valeric acid methyl ester (220) (yield: 100%).
LR-MS (m/z) : 505 ($M^+$)
IR (KBr) : 3306, 3246, 3082, 2952, 2915, 2855, 1743, 1654, 1634, 1589, 1565, 1504, 1432, 1352, 1277, 1243, 1216, 1191, 1091, 1004, 931 $cm^{-1}$
$^1$H-NMR (300MHz, DMSO-d6, δppm) : 1.55-1.84(4H, m), 3.07(2H, d, *J* = 5.4 HZ), 3.67(3H, s), 4.41-4.49(1H, m), 6.42 (1H, t, *J* = 6.0 Hz), 7.25(1H, t, *J* = 8.1 Hz), 7.41-7.52(5H, m), 7.99(1H, s), 9.16(1H, d, *J* = 7.8 Hz).

Example 221

(2S)-2-((2,6-dichlorophenyl)carbonylamino)-5-(((2,6-dichlorophenyl)amino)carbonylamino)valeric acid (221)

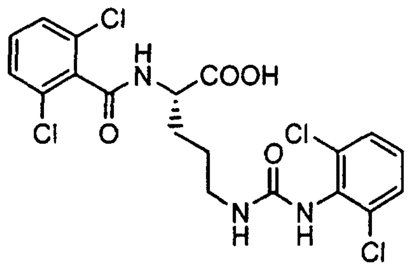

(221)

To a solution of 148.9 mg (0.293 mmol) of (2S)-2-((2,6-dichlorophenyl)carbonylamino)-5-(((2,6-dichlorophenyl)amino)carbonylamino)valeric acid methyl ester (220) in 3.0 ml of DMF, 3.0 ml of 1N aqueous sodium hydroxide solution was added, and the mixture was stirred overnight at room temperature. To the reaction mixture, 4 ml of water and 4 ml of 1N hydrochloric acid were added, and the resulting mixture was extracted with ethyl acetate. Organic layers were combined, washed with water and saturated brine, dried over anhydrous sodium sulfate and concentrated. The residue was washed with hexane/ether to obtain 79.0 mg of (2S)-2-((2,6-dichlorophenyl)carbonylamino)-5-(((2,6-dichlorophenyl)amino)carbonylamino)valeric acid (221) (yield: 59%).
LR-MS (m/z) : 490 [$(M-H)^-$].
IR(KBr) : 3295, 3077, 2930, 2862, 1736, 1651, 1557, 1431, 1270, 1240, 1195, 1092 $cm^{-1}$
$^1$H-NMR (300MHz, DMSO-d6, δppm) : 1.56-1.83(4H, m), 3.08(2H, d, $J$ = 6.0Hz), 4.36-4.43(1H, m), 6.36(1H, t, $J$ = 5.7 Hz), 7.25(1H, dd, $J$ = 7.8,8.4 Hz), 7.40-7.51(5H, m), 7.92(1H, s), 9.02(1H, d, $J$ = 7.8 Hz), 12.65(1H, s)
HR-MS : $C_{19}H_{16}Cl_4N_{34}$ [$(M-H)^-$] Calcd.: 489.9895, Found: 489.9958

Example 222

(2S)-2-((2,6-dichlorophenyl)carbonylamino)-6-((tert-butoxy)carbonylamino)hexanoic acid methyl ester (222)

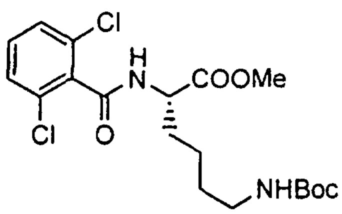

(222)

To a solution of 1.40 g (2.99 mmol) of Fmoc-Lys(Boc)-OH in 5.0 ml of methanol, 2.0M trimethylsilyldiazomethane solution in hexane was added. The reaction mixture was concentrated under reduced pressure and the residue was dissolved in 10 ml of DMF. To this solution, 5.0 ml of piperidine was added and the mixture was stirred at room temperature for 5 minutes. To the reaction mixture, 80 ml of water was added and the resulting mixture was extracted with ethyl acetate. Organic layers were combined, washed with saturated brine, dried over anhydrous sodium sulfate and concentrated under reduced pressure. The residue was purified by column chromatography (silica gel; hexane/ethyl acetate = 50/50 - chloroform/methanol = 10/1) to obtain colorless oil.

This oily product was then dissolved in 5 ml of dichloromethane, and then 0.80 ml (5.76 mmol) of triethylamine and 0.41 ml (2.88 mmol) of 2,6-dichlorobenzoyl chloride were added, and the mixture was stirred for 2.5 hours. Saturated aqueous sodium hydrogen carbonate solution was added to the reaction mixture, and the resulting mixture was extracted with ethyl acetate. Organic layers were combined, washed with 10% aqueous citric acid solution and saturated brine, dried over anhydrous sodium sulfate and concentrated under reduced pressure. The residue was purified by column chromatography (silica gel; hexane/ethyl acetate = 2/1) to obtain 1.24 g of (2S)-2-((2,6-dichlorophenyl)carbonylamino)-6-((tert-butoxy)carbonylamino)hexanoic acid methyl ester (222) (yield: 89%).
LR-MS (m/z) : 432 ($M^+$)

IR (KBr) : 3284, 3066, 2952, 2866, 1740, 1660, 1580, 1531, 1433, 1391, 1365, 1251, 1172, 1012 $cm^{-1}$
$^1H$-NMR (300MHz, $CDCl_3$, δppm) : 7.36-7.26(3H, m), 6.47(1H, br.d, *J* = 7.1 Hz), 4.86(1H, td, *J* = 7.5, 5.0 Hz), 4.56(1H, br.s), 3.80(3H, s), 3.18-3.04(2H, m), 2.03(1H, m), 1.85(1H, m), 1.60-1.44(4H, m), 1.41(9H, s)

Example 223

(2S)-2-((2,6-dichlorophenyl)carbonylamino)-6-((2,6-dimethoxyphenyl)carbonylamino)hexanoic acid methyl ester (223)

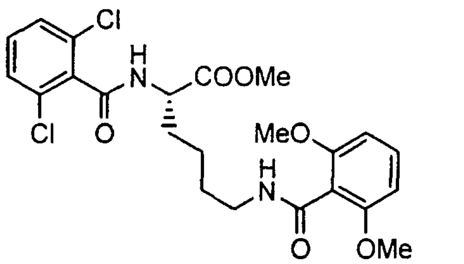

(223)

To a solution of 1.24 g (2.86 mmol) of (2S)-2-((2,6-dichlorophenyl)carbonylamino)-6-((tert-butoxy)carbonylamino)hexanoic acid methyl ester (222) in 5.0 ml of dichloromethane, 2.0 ml of TFA was added and the mixture was stirred at room temperature for 1 hour. After concentrating the reaction mixture, the residue was dissolved in 10 ml of dichloromethane, and then 1.52 g (3.43 mmol) of BOP reagent, 574 mg (3.15 mmol) of 2,6-dimethoxybenzoic acid and 2.0 ml (11.4 mmol) of diisopropylethylamine were added, followed by stirring the mixture overnight at room temperature. To the reaction mixture, 1N hydrochloric acid was added and the resulting mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 1/1-1/3) to obtain 928 mg of (2S)-2-((2,6-dichlorophenyl)carbonylamino)-6-((2,6-dimethoxyphenyl)carbonylamino)hexanoic acid methyl ester (223) (yield: 65%).
LR-MS (m/z) : 496 ($M^+$)
IR (KBr) : 3272, 3065, 2946, 2866, 2845, 1739, 1651, 1595, 1540, 1472, 1434, 1304, 1253, 1199, 1178, 1112, 1031 $cm^{-1}$
$^1H$-NMR (300MHz, $CDCl_3$, δppm) 7.32-7.20(4H, m), 6.53(2H, d, *J* = 8.5 Hz), 6.50(1H, d, *J* = 8.2 Hz), 5.74(1H, m), 4.85 (1H, td, *J* = 7.7, 5.0 Hz), 3.80(6H, s), 3.79(3H, s), 3.46(2H, td, *J* = 6.3, 6.3 Hz), 2.07(1H, m), 1.89(1H, m), 1.73-1.46 (4H, m)
$[\alpha]_D^{20}$ = -194° (c=0.004, $CH_3OH$)

Example 224

(2S)-2-((2,6-dichlorophenyl)carbonylamino)-6-((2,6-dimethoxyphenyl)carbonylamino)hexanoic acid (224)

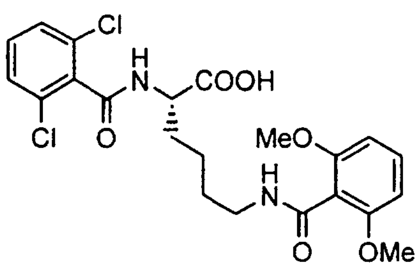

(224)

To a solution of 915 mg (1.84 mmol) of (2S)-2-((2,6-dichlorophenyl)carbonylamino)-6-((2,6-dimethoxyphenyl) carbonylamino)hexanoic acid methyl ester (223) in 5.0 ml of methanol, 5.5 ml of 1N aqueous sodium hydroxide solution was added and the mixture was stirred overnight at room temperature. To the reaction mixture, 1N hydrochloric acid was added and the resulting mixture was extracted with ethyl acetate. Organic layers were combined, washed with

saturated brine, dried over anhydrous sodium sulfate and concentrated. The residue was purified by column chromatography (DIOL; hexane/ethyl acetate = 10/90-1/99) to obtain 899 mg of(2S)-2-((2,6-dichlorophenyl)carbonylamino)-6-((2,6-dimethoxyphenyl)carbonylamino)hexanoic acid (224) (yield: 100%).
LR-MS (m/z) : 482 ($M^+$)
IR (KBr) : 3395, 3302, 3073, 2940, 2861, 2841, 1730, 1655, 1598, 1542, 1472, 1433, 1306, 1253, 1193, 1112, 1033 $cm^{-1}$
$^1$H-NMR (300MHz, $CDCl_3$, δppm) : 7.42-7.20(4H, m), 7.02(1H, d, *J* = 7.5 Hz), 6.62-6.44(2H, m), 5.98(1H, br.s), 4.81 (1H, m), 3.78(6H, s), 3.55-3.44(2H, m), 2.18-1.90(2H, m), 1.72-1.54(4H, m)
HR-MS : $C_{22}H_{24}Cl_2N_2O_6$ ($M^+$) Calcd.: 482.1011, Found: 482.1019
$[\alpha]_D^{20}$ = -44.1° (c=0.018, $CH_3OH$)

Example 225

(5S)-5-((tert-butoxy)carbonylamino)-2-hexene acid tert-butyl methyl ester (225)

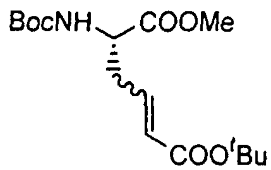

(225)

To a solution of 1.04 g (8.73 mmol) of L-homoserine in 30 ml of 1,4-dioxane/water (2/1), were added 8.7 ml of 1N aqueous sodium hydroxide solution and 3.81 g (17.5 mmol) of $(Boc)_2O$, and the mixture was stirred overnight at room temperature. To the reaction mixture, 10% aqueous citric acid solution was added, and the resulting mixture was extracted with ethyl acetate. Organic layers were combined, washed with saturated brine, dried over anhydrous sodium sulfate and concentrated under reduced pressure to obtain 3.24 g of an oily product. In 7 ml of methanol, 1.08 g of this oily product was dissolved, and 2.0M trimethylsilyldiazomethane solution in hexane was added. After concentrating the reaction solution under reduced pressure, the residue was dissolved in 7 ml of dichloromethane, and 1.2 g of Dess-Martin reagent was added, followed by stirring the resulting mixture for 10 minutes. After diluting the reaction solution with ether, saturated aqueous sodium carbonate solution was added, and the obtained precipitates were separated by filtration. Aqueous sodium chloride solution was added to the filtrate, and the resulting mixture was extracted with ethyl acetate. Organic layers were combined, washed with saturated brine, dried over anhydrous sodium sulfate and concentrated under reduced pressure. A solution of the residue in 4 ml of THF was added to a suspension of 1.5 g (5.82 mmol) of diethylphosphonoacetic acid tert-butyl ester and 233 mg (5.82 mmol) of sodium hydride (60% dispersion in mineral oil) in 6 ml of THF, and the resulting mixture was stirred overnight at room temperature. Saturated aqueous ammonium chloride solution was added to the reaction mixture, and the resulting mixture was extracted with ethyl acetate. Organic layers were combined, washed with saturated brine, dried over anhydrous sodium sulfate and concentrated under reduced pressure. The residue was purified by column chromatography (silica gel; hexane/ethyl acetate = 10/1-7/1) to obtain 131 mg of (5S)-5-((tert-butoxy)carbonylamino)-2-hexenedioic acid tert-butyl methyl ester (225) (yield: 14%).
LR-MS (m/z) : 329 ($M^+$)
$^1$H-NMR (300MHz, $CDCl_3$, δppm) : 6.71 (1H, dt, *J* = 15.3, 7.6Hz), 5.82 (1H, d, *J* = 15.3Hz), 5.07 (1H, br.d, *J* = 7.8Hz), 4.46 (1H, m), 3.76 (3H, s), 2.71 (1H, m), 2.58 (1H, m), 1.48 (9H, s), 1.45 (9H, s)

Example 226

(2S)-2-((tert-butoxy)carbonylamino)-5-(N-(2,6-dimethylphenyl)carbamoyl)-4-pentenoic acid methyl ester (226)

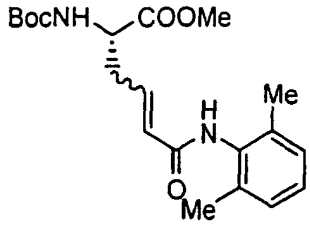

(226)

To a solution of 130 mg (0.395 mmol) of (5S)-5-((tert-butoxy)carbonylamino)-2-hexenedioic acid tert-butyl methyl ester (225) in 2.0 ml of dichloromethane, 1.0 ml of TFA was added, and the mixture was stirred at room temperature for 1 hour. After concentrating the reaction mixture under reduced pressure, the residue was dissolved in 2.0 ml of 1,4-dioxane and 1.0 ml of water, and then 0.4 ml of 1N aqueous sodium hydroxide solution and 172 mg (0.789 mmol) of $(Boc)_2O$ were added, followed by stirring the resulting mixture overnight at room temperature. To the reaction mixture, 10% aqueous citric acid solution was added, and the resulting mixture was extracted with ethyl acetate. Organic layers were combined, washed with saturated brine, dried over anhydrous sodium sulfate and concentrated under reduced pressure. To a solution of the residue in 2.0 ml of DMF, were added 175 mg (0.395 mmol) of BOP reagent, 0.058 ml (0.474 mmol) of 2,6-dimethylaniline and 0.21 ml (1.18 mmol) of diisopropylethylamine, and the mixture was stirred overnight at room temperature. To the reaction mixture, 10% aqueous citric acid solution was added, and the resulting mixture was extracted with ethyl acetate. Organic layers were combined, washed with saturated aqueous sodium hydrogen carbonate solution and saturated brine, dried over anhydrous sodium sulfate and concentrated under reduced pressure. The residue was purified by column chromatography (silica gel; hexane/ethyl acetate = 20/1-1/1) to obtain 29.1 mg of (2S)-2-((tert-butoxy)carbonylamino)-5-(N-(2,6-dimethylphenyl)carbamoyl)-4-pentenoic acid methyl ester (226) (yield: 20%).
LR-MS (m/z) : 376 ($M^+$)
$^1$H-NMR (300MHz, $CDCl_3$, δppm) : 7.71 (1H, br.s), 7.10-6.94 (3H, m), 6.02 (1H, m), 5.72 (1H, dd, *J* = 15.6, 6.3Hz), 4.47 (1H, m), 3.71 (3H, s), 2.62 (1H, m), 2.17 (1H, m), 2.13 (6H, s), 1.30 (9H, s)

Example 227

(2S)-2-((2,6-dichlorophenyl)carbonylamino)-5-(N-(2,6-dimethylphenyl)carbamoyl)-4-pentenoic acid methyl ester (227)

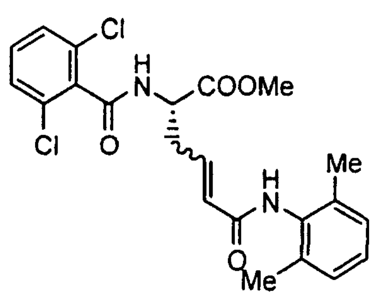

(227)

To a solution of 29.1 mg (0.077 mmol) of (2S)-2-((tert-butoxy)carbonylamino)-5-(N-(2,6-dimethylphenyl)carbamoyl)-4-pentenoic acid methyl ester (226) in 2.0 ml of dichloromethane, 1.0 ml of TFA was added, and the mixture was stirred at room temperature for 30 minutes, followed by concentration of the mixture. The residue was dissolved in 2.0 ml of dichloromethane, and then 0.080 ml (0.57 mmol) of triethylamine and 0.040 ml (0.28 mmol) of 2,6-dichlorobenzoyl chloride were added, followed by stirring the mixture overnight at room temperature. Saturated aqueous sodium hydrogen carbonate solution was added to the reaction mixture, and the resulting mixture was extracted with ethyl acetate. Organic layers were combined, washed with 10% aqueous citric acid solution and saturated brine, dried

over anhydrous sodium sulfate and concentrated under reduced pressure. The residue was purified by column chromatography (silica gel; hexane/ethyl acetate = 1/1) to obtain 26.0 mg of (2S)-2-((2,6-dichlorophenyl)carbonylamino)-5-(N-(2,6-dimethylphenyl)carbamoyl)-4-pentenoic acid methyl ester (227) (yield: 75%).
LR-MS (m/z) : 448 ($M^+$)
IR (KBr) : 3424, 3245, 3019, 2948, 1722, 1654, 1580, 1562, 1523, 1476, 1433, 1377, 1353, 1265, 1194, 1164, 1072 $cm^{-1}$
$^1$H-NMR (300MHz, $CDCl_3$, δppm) : 7.90 (1H, s), 7.40-6.74 (9H, m), 3.82 (3H, s), 2.85-2.54 (3H, m), 2.26-2.12 (6H, m)

Example 228

(2S)-2-((2,6-dichlorophenyl)carbonylamino)-5-(N-(2,6-dimethylphenyl)carbamoyl)-4-pentenoic acid (228)

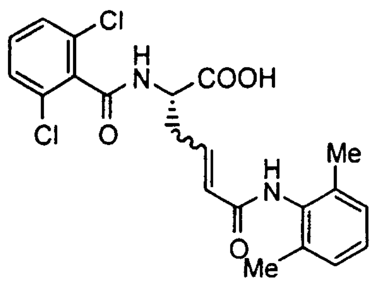

(228)

To a solution of 26.0 mg (0.058 mmol) of (2S)-2-((2,6-dichlorophenyl)carbonylamino)-5-(N-(2,6-dimethylphenyl)carbamoyl)-4-pentenoic acid methyl ester (227) in 1.5 ml of methanol, 0.29 ml of 1N aqueous sodium hydroxide solution was added, and the mixture was stirred overnight at room temperature. To the reaction mixture, 1N hydrochloric acid was added and the resulting mixture was extracted with ethyl acetate. Organic layers were combined, washed with saturated brine, dried over anhydrous sodium sulfate and concentrated. The residue was purified by column chromatography (DIOL; hexane/ethyl acetate = 40/60) to obtain (2S)-2-((2,6-dichlorophenyl)carbonylamino)-5-(N-(2,6-dimethylphenyl)carbamoyl)-4-pentenoic acid (228) as two geometric isomers ((4E) isomer: 3.2 mg (yield: 13%); (4Z) isomer: 14.4 mg (yield: 57%)).

<(4E) Isomer>

LR-MS (m/z) : 434 ($M^+$)
IR (KBr) : 3395, 3260, 3068, 2925, 2856, 1736, 1721, 1658, 1581, 1561, 1523, 1476, 1432, 1267, 1197, 1090, 977 $cm^{-1}$
$^1$H-NMR (300MHz, $CDCl_3$, δppm) : 7.66-6.80 (8H, m), 6.30 (0.75H, d, *J* = 12.1Hz), 5.72 (0.25H, d, *J* = 14.3Hz), 4.95 (1H, m), 3.35-2.71 (2H, m), 2.25-2.07 (6H, m)
HR-MS : $C_{21}H_{22}Cl_2N_2O_4$ ($M^+$) Calcd.: 434.0800, Found: 434.0815
$[\alpha]_D^{20}$ = -10.0° (c = 0.010, $CH_3OH$)

<(4Z) Isomer>

LR-MS (m/z) : 434 ($M^+$)
IR (KBr) : 3420, 3264, 3067, 2963, 2927, 1738, 1673, 1581, 1562, 1545, 1524, 1511, 1475, 1432, 1291, 1197, 1170, 1117, 1089 $cm^{-1}$
$^1$H-NMR (300MHz, $CDCl_3$, δ ppm) : 8.16 (1H, br.s), 7.37-7.19 (3H, m), 7.13-6.95 (3H, m), 6.40 (0.5H, d, *J* = 7.7Hz), 6.38 (0.5H, d, *J* = 7.7Hz), 4.86-4.74 (1.5H. m), 4.50 (0.5H, m), 2.74 (1H, m), 2.60-2.14 (8H, m)
HR-MS : $C_{21}H_{22}Cl_2N_2O_4$ ($M^+$) Calcd.: 434.0800, Found: 434.0782
$[\alpha]_D^{20}$ = -23.8° (c = 0.040, $CH_3OH$)

Example 229

Methyl 2-((tert-butoxy)carbonylamino)-3-(2-oxo-4-((phenylmethoxy)carbonylamino)hydropyrimidinyl)propionate (229)

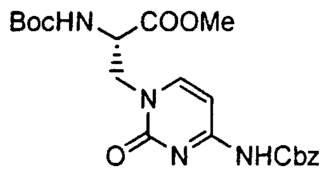

(229)

To a suspension of 4.86 g (19.8 mmol) of N-(2-oxohydropyrimidin-4-yl)(phenylmethoxy)carboxamide in 50 ml of DMF, 0.76 g (19 mmol) of sodium hydride (60% dispersion in mineral oil) was added, and the mixture was stirred at room temperature for 40 minutes. To the reaction mixture, a solution of 3.37 g (18 mmol) of (tert-butoxy)-N-(2-oxooxetan-3-yl)carboxamide in 10 ml of DMF was. added, and the resulting mixture was stirred overnight at room temperature. Water was added to the reaction mixture and the resulting mixture was filtered. To the filtrate, 1N hydrochloric acid was added and the resulting mixture was extracted with chloroform. Organic layers were combined, washed with saturated brine, dried over anhydrous sodium sulfate and concentrated. The residue was dissolved in 20 ml of methanol, and 20 ml of 2.0N trimethylsilyldiazomethane solution in hexane was added, followed by stirring the mixture for 30 minutes. The reaction solution was concentrated and the residue was purified by silica gel column chromatography (hexane:ethyl acetate = 1:1) to obtain 4.02 g of methyl 2-((tert-butoxy)carbonylarnino)-3-(2-oxo-4-((phenylmethoxy) carbonylamino)hydropyrimidinyl)propionate (229) (yield: 50%).
LR-MS(m/z) : 446 ($M^+$)
$^1$H-NMR(300MHZ,$CD_3OD$,δppm) : 3.75 (3H, s), 3.79-3.93 (1H, m), 4.46-4.55 (1H, m), 4.62-4.68 (1H, m), 5.21 (2H, s), 7.07-7.28 (2H, m), 7.32-7.43 (4H, m), 7.80(1H, m)

Example 230

Methyl 3-(4-amino-2-oxohydropyrimidinyl)-2-((tert-butoxy)carbonylamino)propionate (230)

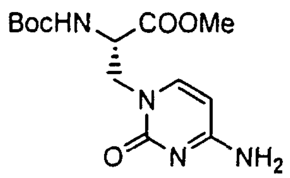

(230)

To a solution of 4.02 g (9.0 mmol) of methyl 2-((tert-butoxy)carbonylamino)-3-(2-oxo-4-((phenylmethoxy)carbonylamino)hydropyrimidinyl)propionate (229) in 20 ml of methanol, 400 mg of 10% palladium/carbon was added, and the mixture was stirred at room temperature for 1 hour under hydrogen atmosphere. The reaction mixture was filtered through Celite, and the filtrate was concentrated. The residue was purified by silica gel column chromatography (chloroform:methanol = 4:1) to obtain 2.53 g of methyl 3-(4-amino-2-oxohydropyrimidinyl)-2-((tert-butoxy)carbonylamino) propionate (230) (yield: 90%).
LR-MS(m/z) : 312 ($M^+$)
$^1$H-NMR(300MHz, $CD_3OD$, δppm) : 1.39(9H, s), 2.31(2H, s), 3.73(3H, s), 3.75-3.82(1H, m), 4.33-4.40(1H, m), 4.53-4.60(1H, m), 5.80(1H, m), 7.05-7.21(1H, m), 7.41(1H, m)

Example 231

Methyl 3-(4-((2,6-dichlorophenyl)carbonylamino)-2-oxohydropyrimidinyl)-2-((tert-butoxy)carbonylamino)propionate (231)

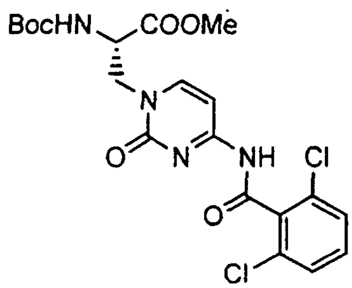

(231)

In 15 ml of pyridine and 10 ml of dichloromethane, 2.96 g (9.48 mmol) of methyl 3-(4-amino-2-oxohydropyrimidinyl)-2-((tert-butoxy)carbonylamino)propionate (230) was dissolved, and 2.7 ml (19.0 mmol) of 2,6-dichlorobenzoyl chloride was added, followed by stirring the mixture at 50°C for 2 hours. Methanol was added to the reaction solution and the resulting mixture was concentrated. To the residue, 3N hydrochloric acid was added and the resulting mixture was extracted with chloroform. The organic layer was washed with saturated brine, dried over anhydrous sodium sulfate and concentrated. The residue was purified by silica gel column chromatography (chloroform:methanol = 20:1) to obtain 3.35 g of methyl 3-(4-((2,6-dichlorophenyl)carbonylamino)-2-oxohydropyrimidinyl)-2-((tert-butoxy)carbonylamino)propionate (231) (yield: 73%).
LR-MS(m/z) : 484 ($M^+$)
IR(KBr):3442, 3222, 2962, 1701, 1627, 1562, 1492, 1435, 1369, 1334, 1303, 1250, 1162, 788 $cm^{-1}$
$^1$H-NMR(300MHz, $CD_3OD$,δppm) : 1.38 (9H, s), 3.77 (3H, s), 3.83-3.94 (1H, m), 4.55-4.63 (1H, m), 4.68-4.77 (1H, m), 7.41-7.55 (4H, m), 7.93-7.98 (1H, m)

Example 232

Methyl 2-amino-3-(4-((2,6-dichlorophenyl)carbonylamino)-2-oxohydropyrimidinyl)propionate (232)

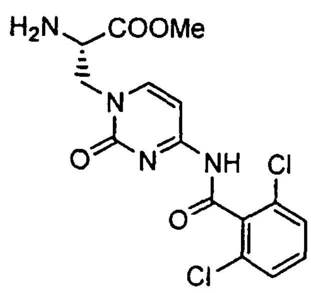

(232)

To a suspension of 202 mg of methyl 3-(4-((2,6-dichlorophenyl)carbonylamino)-2-oxohydropyrimidinyl)-2-((tert-butoxy)carbonylamino)propionate (231) in 9 ml of dichloromethane, 1 ml of trifluoroacetic acid was added, and the mixture was stirred at room temperature for 3 hours. The reaction solution was concentrated to obtain 156 mg of methyl 2-amino-3-(4-((2,6-dichlorophenyl)carbonylamino)-2-oxohydropyrimidinyl)propionate (232) TFA salt (yield: 97%). The TFA salt of the product (232) was used in the next reaction without purification.

Example 233

Methyl (2S)-3-(4-((2,6-dichlorophenyl)carbonylamino)-2-oxohydropyrimidinyl)-2-(phenylcarbonylamino)propionate (233)

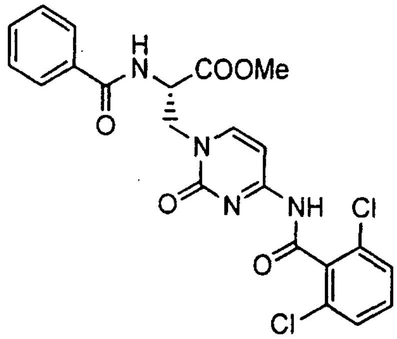

(233)

To a solution of 100 mg (0.200 mmol) of methyl 2-amino-3-(4-((2,6-dichlorophenyl)carbonylamino)-2-oxohydropyrimidinyl)propionate (232) TFA salt in 4 ml of dichloromethane, were added 0.15 ml (1.08 mmol) of triethylamine and 0.050 ml (0.434 mmol) of benzoyl chloride, and the mixture was stirred at room temperature for 1.5 hours. Saturated aqueous sodium hydrogen carbonate solution was added to the reaction mixture, and the resulting mixture was extracted with ethyl acetate. Organic layers were combined, washed with 0.5N hydrochloric acid and saturated brine, dried over anhydrous sodium sulfate and concentrated. The residue was purified by column chromatography (silica gel; chloroform/methanol = 100/1-60/1) to obtain 34.2 mg of methyl (2S)-3-(4-((2,6-dichlorophenyl)carbonylamino)-2-oxohydropyrimidinyl)-2-(phenylcarbonylamino)propionate (233) (yield: 33%).
LR-MS (m/z) : 487 [$(M-H)^-$]
IR (KBr) : 3421, 1648, 1560, 1491, 1433, 1369, 1338, 1304, 1243, 1196, 1159, 1134, 1057, 1009 $cm^{-1}$
$^1$H-NMR (300MHz, $CDCl_3$, δppm) : 3.80(3H, s), 4.52(2H, d, *J* = 5.4 Hz), 5.02(1H, dd, *J* = 5.4, 11.7 Hz)7.35(3H, dd, *J* = 3.3, 3.9 Hz), 7.39-7.52(4H, m), 7.74(1H, d, *J* = 7.2 Hz), 7.82-7.85(2H, m), 8.03(1H, d, *J* = 6.3 Hz)
$[\alpha]_D^{20}$ = -51.8° (c=0.021, $CH_3OH$)

Example 234

(2S)-3-(4-((2,6-dichlorophenyl)carbonylamino)-2-oxohydropyrimidinyl)-2-(phenylcarbonylamino)propionic acid (234)

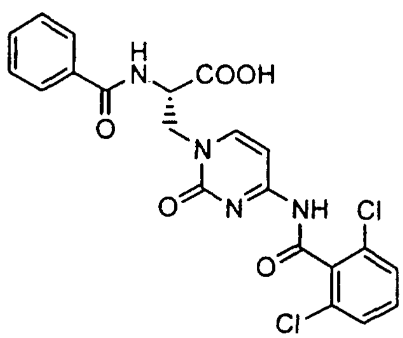

(234)

To a solution of 27.8 mg (0.061 mmol) of methyl (2S)-3-(4-((2,6-dichlorophenyl)carbonylamino)-2-oxohydropyrimidinyl)-2-(phenylcarbonylamino)propionate (233) in 1.0 ml of methanol, 1.0 ml of 1N aqueous sodium hydroxide solution was added, and the mixture was stirred at room temperature for 1 hour. To the reaction mixture, 0.2N hydrochloric acid was added and the resulting mixture was extracted with ethyl acetate. Organic layers were combined, washed with saturated brine, dried over anhydrous sodium sulfate and concentrated under reduced pressure. The

residue was reprecipitated from hexane/chloroform/methanol to obtain 15.0 mg of (2S)-3-(4-((2,6-dichlorophenyl)carbonylamino)-2-oxohydropyrimidinyl)-2-(phenylcarbonylamino)propionic acid (234) (yield: 55%).
LR-MS (m/z) : 473 [$(M-H)^-$]
IR (KBr) : 3307, 3126, 3026, 2969, 1718, 1659, 1568, 1493, 1430, 1405, 1363, 1308, 1273, 1249, 1189, 1131, 1087, 976 $cm^{-1}$
$^1H$-NMR (300MHz, $CD_3OD$, δppm) : 4.22(1H, dd, *J* = 9.3, 13.5 Hz), 4.74(1H, dd, *J* = 4.4, 13.5 Hz), 5.09(1H, dd, *J* = 4.4, 9.3 Hz), 7.40-7.60(7H, m), 7.80(2H, dd, *J* = 1.6, 6.9 Hz), 7.99(1H, d, *J* = 7.4 Hz)
$[\alpha]_D^{20}$ = -169° (c=0.010, $CH_3OH$)

Example 235

Methyl (2S)-2-((2,6-dichlorophenyl)carbonylamino)-3-(4-((2,6-dichlorophenyl)carbonylamino)-2-oxohydropyrimidinyl) propionate (235)

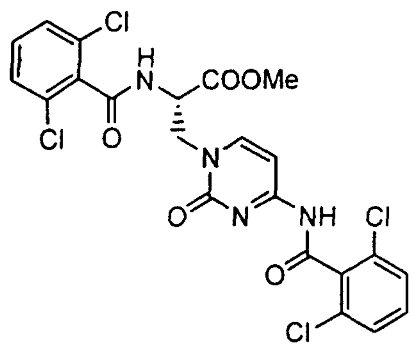

(235)

To a solution of 100 mg (0.200 mmol) of methyl 2-amino-3-(4-((2,6-dichlorophenyl)carbonylamino)-2-oxohydropyrimidinyl)propionate (232) TFA salt in 4 ml of dichloromethane, were added 0.15 ml (1.08 mmol) of triethylamine and 0.050 ml (0.434 mmol) of 2,6-dichlorobenzoyl chloride, and the mixture was stirred at room temperature for 1.5 hours. Saturated aqueous sodium hydrogen carbonate solution was added to the reaction mixture and the resulting mixture was extracted with ethyl acetate. Organic layers were combined, washed with 0.5N hydrochloric acid and saturated brine, dried over anhydrous sodium sulfate and concentrated. The residue was purified by column chromatography (silica gel; chloroform/methanol = 100/1-60/1) to obtain 34.2 mg of methyl (2S)-2-((2,6-dichlorophenyl)carbonylamino)-3-(4-((2,6-dichlorophenyl)carbonylamino)-2-oxohydropyrimidinyl)propionate (235) (yield: 33%).
LR-MS (m/z) : 555 [$(M-H)^-$]
$^1H$-NMR (300MHz, $CD_3OD$, δppm) : 8.06(1H, d, *J* =7.4Hz), 7.54(1H, d, *J* =6.9Hz), 7.49-7.38(6H, m), 5.32(1H, dd, *J* =9.1, 5.1Hz), 4.89(1H, dd, *J* =13.6, 5.1Hz), 4.19(1H, dd, *J* = 13.6, 9.1Hz), 3.81(3H,s)

Example 236

(2S)-2-((2,6-dichlorophenyl)carbonylamino)-3-(4-((2,6-dichlorophenyl)carbonylamino)-2-oxohydropyrimidinyl) propionic acid (236)

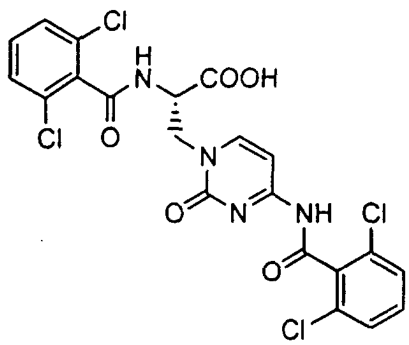

(236)

To a solution of 27.8 mg (0.061 mmol) of methyl (2S)-2-((2,6-dichlorophenyl)carbonylamino)-3-(4-((2,6-dichlorophenyl)carbonylamino)-2-oxohydropyrimidinyl)propionate (235) in 1.0 ml of methanol, 1.0 ml of 1N aqueous sodium hydroxide solution was added, and the mixture was stirred at room temperature for 1 hour. To the reaction mixture, 0.2N hydrochloric acid was added, and the resulting mixture was extracted with ethyl acetate. Organic layers were combined, washed with saturated brine, dried over anhydrous sodium sulfate and concentrated under reduced pressure. The residue was reprecipitated from hexane/chloroform/methanol to obtain 15.0 mg of (2S)-2-((2,6-dichlorophenyl)carbonylamino)-3-(4-((2,6-dichlorophenyl)carbonylamino)-2-oxohydropyrimidinyl)propionic acid (236) (yield: 55%).
LR-MS (m/z) : 541 $[(M-H)^-]$
IR (KBr) : 3396, 3259, 3078, 1719, 1657, 1579, 1562, 1493, 1432, 1367, 1306, 1246, 1196, 1135, 1087, 901 $cm^{-1}$
$^1H$-NMR (300MHz, $CD_3OD$, δppm) : 8.07(1H, d, *J* =5.5Hz), 7.52(1H, d, *J* =8.8Hz), 7.49-7.36(6H, m), 5.29(1H, br.s), 4.61(1H, m), 4.15(1H, dd, *J* =13.8, 11.3Hz)
$[\alpha]^{20}_D$=-130° (c=0.010, $CH_3OH$)

Example 237

Methyl (2S)-3-(4-((2,6-dichlorophenyl)carbonylamino)-2-oxohydropyrimidinyl)-2-((2,6-difluorophenyl)carbonylamino) propionate (237)

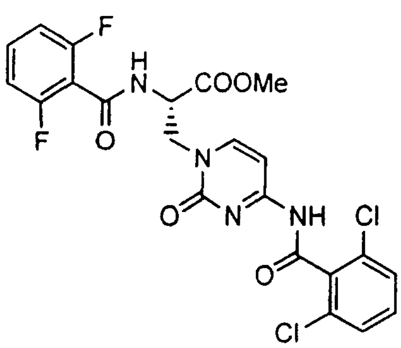

(237)

To a solution of 100 mg (0.200 mmol) of methyl 2-amino-3-(4-((2,6-dichlorophenyl)carbonylamino)-2-oxohydropyrimidinyl)propionate (232) TFA salt in 6 ml of dichloromethane, were added 0.28 ml (2.00 mmol) of triethylamine and 0.050 ml (0.40 mmol) of 2,6-difluorobenzoyl chloride, and the mixture was stirred at room temperature for 1.5

hours. Saturated aqueous sodium hydrogen carbonate solution was added to the reaction mixture, and the resulting mixture was extracted with ethyl acetate. Organic layers were combined, washed with 1N hydrochloric acid and saturated brine, dried over anhydrous sodium sulfate and concentrated. The residue was purified by column chromatography (silica gel; chloroform/methanol = 60/1-40/1) to obtain 97 mg of methyl (2S)-3-(4-((2,6-dichlorophenyl)carbonylamino)-2-oxohydropyrimidinyl)-2-((2,6-difluorophenyl)carbonylamino)propionate (237) (yield: 92%).
LR-MS (m/z) : 523 [$(M-H)^-$]
IR (KBr) : 3267, 3073, 2956, 1666, 1626, 1562, 1493, 1433, 1369, 1342, 1304, 1242, 1195, 1158, 1133, 1057, 1008 $cm^{-1}$
$^1$H-NMR (300MHz, $CDCl_3$, δppm) : 3.83 (3H, S), 4.43-4.57 (2H, m), 4.98 (1H, dd, *J* = 5.4, 11.4 Hz), 6.93 (2H, t, *J* = 8.1 Hz), 7.34-7.37 (4H, m), 7.48 (2H, d, *J* = 6.9 Hz), 7.74 (1H, d, *J* = 7.5Hz), 8.51 (1H, s)
$[\alpha]_D^{20}$ = -109° (c=0.088, $CH_3OH$)

Example 238

(2S)-3-(4-((2,6-dichlorophenyl)carbonylamino)-2-oxohydropyrimidinyl)-2-((2,6-difluorophenyl)carbonylamino) propionic acid (238)

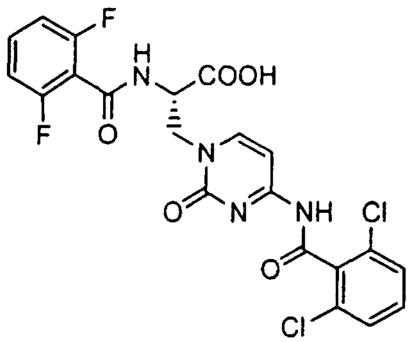

(238)

To a solution of 86 mg (0.16 mmol) of methyl (2S)-3-(4-((2,6-dichlorophenyl)carbonylamino)-2-oxohydropyrimidinyl)-2-((2,6-difluorophenyl)carbonylamino)propionate (237) in 1.0 ml of methanol, 3.0 ml of 1N aqueous sodium hydroxide solution was added, and the mixture was stirred at room temperature for 1.5 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was reprecipitated from hexane/chloroform/methanol to obtain 44.7 mg of (2S)-3-(4-((2,6-dichlorophenyl)carbonylamino)-2-oxohydropyrimidinyl)-2-((2,6-difluorophenyl)carbonylamino)propionic acid (238) (yield: 55%).
LR-MS (m/z) : 509 [$(M-H)^-$]
IR (KBr) : 3250, 3073, 1720, 1660, 1626, 1563, 1494, 1468, 1432, 1367, 1305, 1242, 1195, 1158, 1134, 1057, 1008 $cm^{-1}$
$^1$H-NMR (300MHz, $CDCl_3$, δppm) : 4.51(2H, d, *J* = 5.4 Hz), 4.91-4.94(1H, m), 6.96(2H, t, *J* = 8.1 Hz), 7.31-7.42(4H, m), 7.45(1H, s), 7.60(1H, d, *J* = 7.2 Hz), 7.64(1H, s), 7.81(1H, d, 7.5 Hz)
$[\alpha]_D^{20}$ = -46.3° (c=0.040, $CH_3OH$)

Example 239

Methyl (2S)-3-(4-((2,6-dichlorophenyl)carbonylamino)-2-oxohydropyrimidinyl)-2-((2-bromo-6-methylphenyl) carbonylamino)propionate (239)

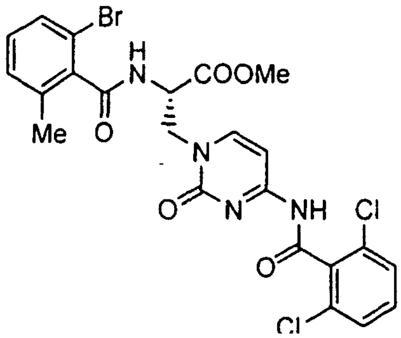

(239)

To a solution of 63.9 mg (0.128 mmol) of methyl 2-amino-3-(4-((2,6-dichlorophenyl)carbonylamino)-2-oxohydropyrimidinyl)propionate (232) TFA salt in 4 ml of dichloromethane, were added 0.15 ml (1.08 mmol) of triethylamine and 2 ml of a solution of 2-bromo-6-methylbenzoyl chloride (prepared from 42.0 mg (0.195 mmol) of 2-bromo-6-methylbenzoic acid and 0.020 ml of thionyl chloride) in dichloromethane, and the mixture was stirred overnight at room temperature. Saturated aqueous sodium hydrogen carbonate solution was added to the reaction mixture, and the resulting mixture was extracted with chloroform. Organic layers were combined, washed with 1N hydrochloric acid and saturated brine, dried over anhydrous sodium sulfate and concentrated. The residue was purified by column chromatography (silica gel; chloroform/methanol = 100/1-30/1) to obtain 65.1 mg of methyl (2S)-3-(4-((2,6-dichlorophenyl) carbonylamino)-2-oxohydropyrimidinyl)-2-((2-bromo-6-methylphenyl)carbonylamino)propionate (239) (yield: 87%).
LR-MS (m/z) : 579 $[(M-H)^-]$
IR (KBr) : 3268, 3015, 2957, 1748, 1713, 1662, 1625, 1561, 1493, 1433, 1371, 1338, 1305, 1243, 1196, 1182, 1136, 1061, 1004 $cm^{-1}$
$^1$H-NMR (300MHz, $CD_3OD$, δppm) : 8.34(1H, br.s), 7.89(1H, d, *J* = 7.1Hz), 7.52(1H, br.d, *J* = 5.5Hz), 7.40-7.22(5H, m), 7.15-7.05(2H, m), 5.06(1H, ddd, *J* = 6.9, 5.5, 5.5Hz), 4.52(1H, dd, *J* = 13.7, 6.9Hz), 4.43(1H, dd, *J* = 13.7, 5.5Hz), 3.82(3H, s), 2.36(3H, s)
$[\alpha]_D^{20}$ = -77.0° (c=0.10, $CH_3OH$)

Example 240

(2S)-3-(4-((2,6-dichlorophenyl)carbonylamino)-2-oxohydropyrimidinyl)-2-((2-bromo-6-methylphenyl)carbonylamino) propionic acid (240)

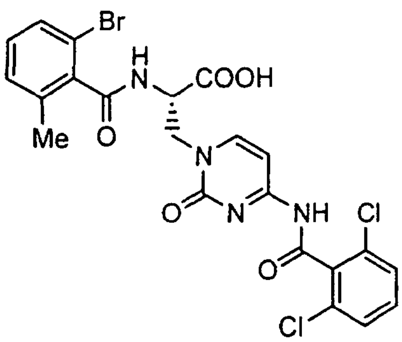

(240)

To a solution of 62.3 mg (0.107 mmol) of methyl (2S)-3-(4-((2,6-dichlorophenyl)carbonylamino)-2-oxohydropyrimidinyl)-2-((2-bromo-6-methylphenyl)carbonylamino)propionate (239) in 2.4 ml of methanol/THF (1/1), 0.6 ml of 1N aqueous sodium hydroxide solution was added, and the mixture was stirred at room temperature for 5 hours. To the reaction mixture, 0.1N hydrochloric acid was added and the resulting mixture was extracted with ethyl acetate. Organic layers were combined, washed with saturated brine, dried over anhydrous sodium sulfate and concentrated. The residue was reprecipitated from ether/methanol to obtain 41.0 mg of (2S)-3-(4-((2,6-dichlorophenyl)carbonylamino)-2-oxohydropyrimidinyl)-2-((2-bromo-6-methylphenyl)carbonylamino)propionic acid (240) (yield: 67%).
LR-MS (m/z) : 565 $[(M-H)^-]$
IR (KBr) : 3653, 3234, 3060, 2975, 2847, 1709, 1645, 1563, 1499, 1432, 1384, 1307, 1250, 1193, 1179, 1132, 975 $cm^{-1}$
$^1$H-NMR (300MHz, $CD_3OD$, δppm) : 8.08(1H, d, *J* = 7.1Hz), 7.60-7.37(5H, m), 7.27-7.15(2H, m), 5.35(1H, dd, *J* = 9.9, 4.9Hz), 4.69(1H, dd, *J* = 13.5, 4.9Hz), 4.14 (1H, dd, *J* = 13.5, 9.9Hz), 2.37(3H, s)
$[\alpha]_D^{20}$ = -62.5° (c=0.10, $CH_3OH$)
HR-MS: $C_{22}H_{16}BrCl_2N_4O_4$ $[(M-H)^-]$ Calcd.: 564.9681, Found: 594.9745

Example 241

Methyl (2S)-3-(4-((2,6-dichlorophenyl)carbonylamino)-2-oxohydropyrimidinyl)-2-((2,6-dimethylphenyl)carbonylamino) propionate (241)

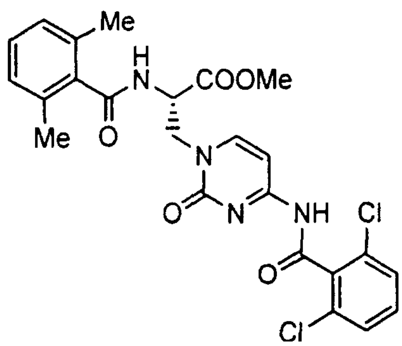

(241)

To a solution of 100 mg (0.200 mmol) of methyl 2-amino-3-(4-((2,6-dichlorophenyl)carbonylamino)-2-oxohydropyrimidinyl)propionate (232) TFA salt in 3 ml of dichloromethane, were added 0.28 ml (0.400 mmol) of triethylamine and 3 ml of a solution of 2,6-dimethylbenzoyl chloride (prepared from 60.1 mg (0.400 mmol) of 2,6-dimethylbenzoic acid and 0.035 ml of thionyl chloride) in dichloromethane, and the mixture was stirred at room temperature for 3.5 hours. Saturated aqueous sodium hydrogen carbonate solution was added to the reaction mixture, and the resulting mixture was extracted with chloroform. Organic layers were combined, washed with 1N hydrochloric acid and saturated brine, dried over anhydrous sodium sulfate and concentrated. The residue was purified by column chromatography (DIOL; hexane/ethyl acetate = 50/50-1/99) to obtain 62.9 mg of methyl (2S)-3-(4-((2,6-dichlorophenyl)carbonylamino)-2-oxohydropyrimidinyl)-2-((2,6-dimethylphenyl)carbonylamino)propionate (241) (yield: 69%).
LR-MS (m/z) : 515 $[(M-H)^-]$
IR (KBr) : 3275, 3073, 3017, 1954, 1743, 1714, 1658, 1624, 1561, 1491, 1433, 1368, 1341, 1305, 1244, 1196, 1161, 1133, 1052, 1002 $cm^{-1}$
$^1$H-NMR (300MHz, $CDCl_3$, δppm) : 2.29(6H, s), 3.82(3H, s), 4.40-4.50(2H, m), 5.08(1H, dd, *J* = 7.2, 12.9 Hz), 6.91 (1H, d, *J* = 7.5 Hz), 7.08(1H, t, *J* = 7.8 Hz), 7.30-7.33(1H, m), 7.36(3H, dd, *J* = 3.3, 4.5 Hz), 7.52(1H, d, *J* = 7.2 Hz)
$[\alpha]_D^{20}$ = -222° (c=0.012, $CH_3OH$)

Example 242

(2S)-3-(4-((2,6-dichlorophenyl)carbonylamino)-2-oxohydropyrimidinyl)-2-((2,6-dimethylphenyl)carbonylamino) propionic acid (242)

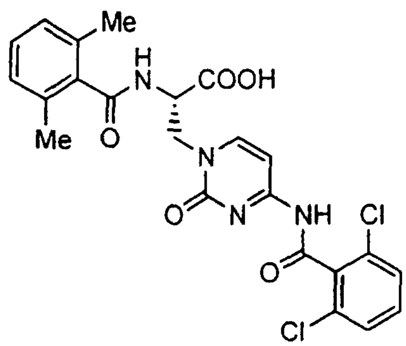

(242)

To a solution of 59.3 mg (0.115 mmol) of methyl (2S)-3-(4-((2,6-dichlorophenyl)carbonylamino)-2-oxohydropyrimidinyl)-2-((2,6-dimethylphenyl)carbonylamino)propionate (241) in 3.0 ml of methanol/THF(2/1), 1.0 ml of 1N aqueous sodium hydroxide solution was added, and the mixture was stirred at room temperature for 5 hours. To the reaction mixture, 0.2N hydrochloric acid was added, and the resulting mixture was extracted with ethyl acetate. Organic layers were combined, washed with saturated brine, dried over anhydrous sodium sulfate and concentrated. The residue was reprecipitated from ether/methanol to obtain 42.4 mg of (2S)-3-(4-((2,6-dichlorophenyl)carbonylamino)-2-oxohydropyrimidinyl)-2-((2,6-dimethylphenyl)carbonylamino)propionic acid (242) (yield: 73%).
LR-MS (m/z) : 501 [(M-H)$^-$].
IR (KBr) : 3510, 3232, 3053, 2979, 2927, 1710, 1636, 1566, 1496, 1429, 1380, 1304, 1247, 1190, 1162, 1131, 972 cm$^{-1}$
$^1$H-NMR (300MHz, $CDCl_3$, δppm) : 2.22(6H, s), 4.12(1H, dd, *J* = 9.9, 13.6 Hz), 5.35(1H, dd, *J* = 5.0, 9.9 Hz), 7.01(2H, d, *J* = 7.7 Hz), 7.15(1H, dd, *J* = 7.0, 8.0 Hz), 7.42-7.60(4H, m), 8.06 (1H, d, *J* = 7.4 Hz)
$[\alpha]_D^{20}$ = -92.0° (c=0.010, $CH_3OH$)

Example 243

Methyl (2S)-3-(4-((2,6-dichlorophenyl)carbonylamino)-2-oxohydropyrimidinyl)-2-((2,6-dimethoxyphenyl) carbonylamino)propionate (243)

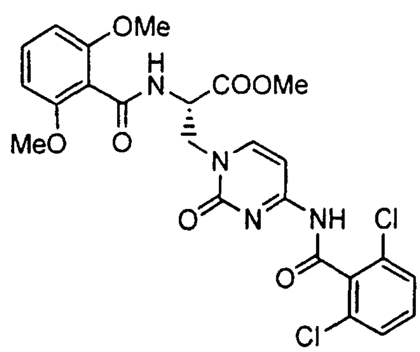

(243)

To a solution of 100 mg (0.200 mmol) of methyl 2-amino-3-(4-((2,6-dichlorophenyl)carbonylamino)-2-oxohydropyrimidinyl)propionate (232) TFA salt in 3 ml of dichloromethane, were added 0.28 ml (0.400 mmol) of triethylamine and 3 ml of a solution of 2,6-dimethylbenzoyl chloride (prepared from 73 mg (0.40 mmol) of 2,6-dimethoxybenzoic acid and 0.042 ml of oxalyl chloride) in dichloromethane, and the mixture was stirred at room temperature for 3.5 hours. Saturated aqueous sodium hydrogen carbonate solution was added to the reaction mixture, and the resulting mixture

was extracted with chloroform. Organic layers were combined, washed with 1N hydrochloric acid and saturated brine, dried over anhydrous sodium sulfate and concentrated. The residue was purified by column chromatography (silica gel; chloroform/methanol = 60/1-40/1) to obtain 94 mg of methyl (2S)-3-(4-((2,6-dichlorophenyl)carbonylamino)-2-oxohydropyrimidinyl)-2-((2,6-dimethoxyphenyl)carbonylamino)propionate (243) (yield: 86%).
LR-MS (m/z) : 547 [(M-H)$^-$]
IR (KBr) : 3248, 3075, 3012, 2954, 2840, 1739, 1715, 1661, 1595, 1564, 1494, 1433, 1370, 1340, 1304, 1253, 1195, 1112, 1032 cm$^{-1}$
$^1$H-NMR (300MHz, $CDCl_3$, δppm) : 3.83(6H, s), 3.86(3H, s), 4.45(1H, dd, *J* = 5.7, 13.5 Hz), 4.67(1H, d, 12.9 Hz), 4.92 (1H, t, *J* = 5.1 Hz), 6.58(2H, d, *J* = 8.4 Hz), 7.29-7.41(6H, m), 7.57(1H, d, *J* = 7.5 Hz), 7.89(1H, d, *J* = 7.2 Hz)
$[\alpha]_D^{20}$ = -34.1° (c = 0.044, $CH_3OH$)

Example 244

(2S)-3-(4-((2,6-dichlorophenyl)carbonylamino)-2-oxohydropyrimidinyl)-2-((2,6-dimethoxyphenyl)carbonylamino) propionic acid (244)

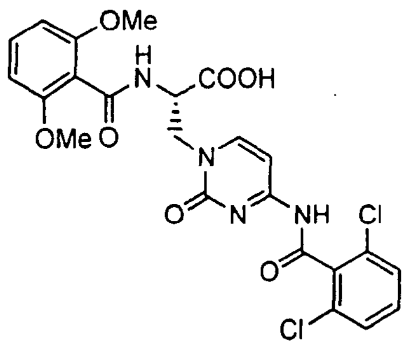

(244)

To a solution of 84 mg (0.15 mmol) of methyl (2S)-3-(4-((2,6-dichlorophenyl)carbonylamino)-2-oxohydropyrimidinyl)-2-((2,6-dimethoxyphenyl)carbonylamino)propionate (243) in 3.0 ml of methanol, 0.75 ml of 1N aqueous sodium hydroxide solution was added, and the mixture was stirred at room temperature for 2 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was reprecipitated from hexane/chloroform/methanol to obtain 73.9 mg of (2S)-3-(4-((2,6-dichlorophenyl)carbonylamino)-2-oxohydropyrimidinyl)-2-((2,6-dimethoxyphenyl)carbonylamino)propionic acid (244) (yield: 92%).
LR-MS (m/z) : 533 [(M-H)$^-$].
IR (KBr) : 3354, 3154, 3072, 2938, 2840, 1718, 1652, 1594, 1515, 1475, 1433, 1373, 1324, 1298, 1253, 1193, 1156, 1115, 1032, 1001, 962 cm$^{-1}$
$^1$H-NMR (300MHz, $CDCl_3$, δppm) : 3.80(6H, s), 4.12(1H, dd, *J* = 13.2, 13.5 Hz), 4.67(1H, dd, *J* = 4.2, 13.5 Hz), 5.12 (1H, dd, *J* = 4.8, 9.9 Hz), 6.65(2H, d, *J* = 8.7 Hz), 7.32(1H, t, *J* = 8.4 Hz), 7.47(3H, s), 7.56(1H, d, *J* = 7.5 Hz), 7.85(1H, s), 8.63(1H, d, *J* = 7.5 Hz)
$[\alpha]_D^{20}$ = -22.7° (c = 0.044, $CH_3OH$)

Example 245

Methyl (2S)-3-(4-((2,6-dichlorophenyl)carbonylamino)-2-oxohydropyrimidinyl)-2-((2-methyl-5-nitrophenyl) carbonylamino)propionate (245)

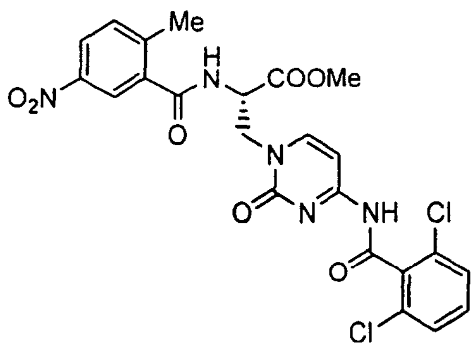

(245)

To a solution of 100 mg (0.200 mmol) of methyl 2-amino-3-(4-((2,6-dichlorophenyl)carbonylamino)-2-oxohydropyrimidinyl)propionate (232) TFA salt in 3 ml of dichloromethane, were added 0.28 ml (0.400 mmol) of triethylamine and 3 ml of a solution of 2,6-dimethylbenzoyl chloride (prepared from 72 mg (0.40 mmol) of 2-methyl-5-nitrobenzoic acid and 0.042 ml of oxalyl chloride) in dichloromethane, and the mixture was stirred at room temperature for 1.5 hours. Saturated aqueous sodium hydrogen carbonate solution was added to the reaction mixture, and the resulting mixture was extracted with chloroform. Organic layers were combined, washed with 1N hydrochloric acid and saturated brine, dried over anhydrous sodium sulfate and concentrated. The residue was purified by column chromatography (silica gel; chloroform/methanol = 60/1-40/1) to obtain 107 mg of methyl (2S)-3-(4-((2,6-dichlorophenyl)carbonylamino)-2-oxohydropyrimidinyl)-2-((2-methyl-5-nitrophenyl)carbonylamino)propionate (245) (yield: 98%).
LR-MS (m/z) : 546 $[(M-H)^-]$
IR (KBr) : 3263, 3078, 2956, 1719, 1663, 1561, 1491, 1433, 1348, 1304, 1243, 1194, 1135, 1079, 1042, 1007, 902 $cm^{-1}$
$^1$H-NMR (300MHz, $CD_3OD$, δppm) : 2.53(3H, s), 3.79(3H, s), 4.38-4.57(2H, m), 5.01-5.08(1H, m), 7.33-7.37(4H, m), 7.54(1H, d, *J* = 7.5 Hz), 7.82(1H, d, *J* = 7.5 Hz), 8.12(1H, dd, *J* = 2.4, 8.1 Hz), 8.26(1H, s), 8.28(1H, d, *J* = 2.7 Hz), 8.87(1H, s)
$[\alpha]_D^{20}$ = -120° (c = 0.048, $CH_3OH$)

Example 246

(2S)-3-(4-((2,6-dichlorophenyl)carbonylamino)-2-oxohydropyrimidinyl)-2-((2-methyl-5-nitrophenyl)carbonylamino) propionic acid (246)

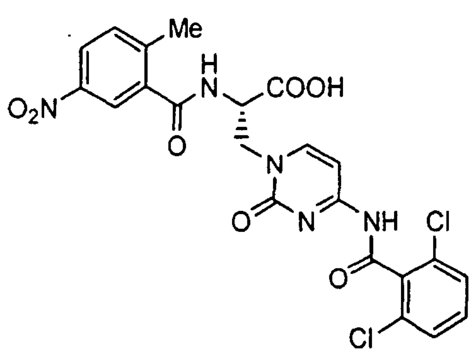

(246)

To a solution of 84 mg (0.15 mmol) of methyl (2S)-3-(4-((2,6-dichlorophenyl)carbonylamino)-2-oxohydropyrimidinyl)-2-((2-methyl-5-nitrophenyl)carbonylamino)propionate (245) in 3.0 ml of methanol, 0.9 ml of 1N aqueous sodium hydroxide solution was added, and the mixture was stirred at room temperature for 1.5 hours. To the reaction mixture, 1N hydrochloric acid was added and the resulting mixture was extracted with ethyl acetate. Organic layers were combined, washed with saturated brine, dried over anhydrous sodium sulfate and concentrated. The residue was reprecipitated from hexane/chloroform/methanol to obtain 84.2 mg of (2S)-3-(4-((2,6-dichlorophenyl)carbonylamino)-2-oxohydropyrimidinyl)-2-((2-methyl-5-nitrophenyl)carbonylamino)propionic acid (246) (yield: 88%).
LR-MS (m/z) : 532 [$(M-H)^-$].
IR (KBr) : 3256, 3076, 1717, 1658, 1563, 1521, 1494, 1432, 1350, 1308, 1245, 1195, 1136, 1081, 978 $cm^{-1}$
$^1$H-NMR (300MHz, $CDCl_3$+$CD_3OD$, δppm) : 2.54(3H, s), 4.33-4.40(1H, m), 4.62(1H, dd, *J* = 4.8, 13.8 Hz), 4.98(1H, dd, *J* = 5.1, 7.2 Hz), 7.34-7.43(4H, m), 7.63(1H, d, *J* = 7.5 Hz), 7.87(1H, d, *J* = 6.9 Hz), 8.19(1H, d, *J* = 8.1 Hz), 8.30(1H, s)
$[\alpha]_D^{20}$ = -29.5° (c = 0.044, $CH_3OH$)

Example 247

Methyl (2S)-3-(4-((2,6-dichlorophenyl)carbonylamino)-2-oxohydropyrimidinyl)-2-((2-chlorophenyl)carbonylamino) propionate (247)

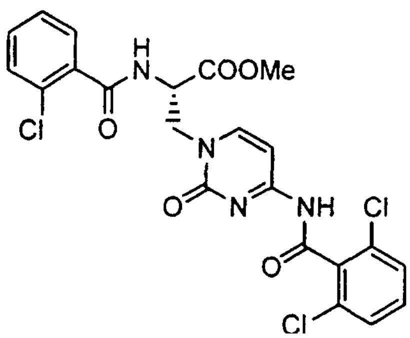

(247)

To a solution of 105 mg (0.210 mmol) of methyl 2-amino-3-(4-((2,6-dichlorophenyl)carbonylamino)-2-oxohydropyrimidinyl)propionate (232) TFA salt in 3 ml of dichloromethane, were added 0.293 ml (2.10 mmol) of triethylamine and 0.053 ml (0.42 mmol) of 2-chlorobenzoyl chloride, and the mixture was stirred at room temperature for 3 hours. To the reaction mixture, saturated aqueous sodium hydrogen solution was added and the resulting mixture was extracted with ethyl acetate. Organic layers were combined, washed with 1N hydrochloric acid and saturated brine, dried over anhydrous sodium sulfate and concentrated. The residue was purified by column chromatography (silica gel; chloroform/methanol = 60/1-40/1) to obtain 60 mg of methyl (2S)-3-(4-((2,6-dichlorophenyl)carbonylamino)-2-oxohydropyrimidinyl)-2-((2-chlorophenyl)carbonylamino)propionate (247) (yield: 55%).
LR-MS (m/z) : 521 [$(M-H)^-$]
IR (KBr) : 3275, 3145, 3080, 2956, 2851, 1722, 1650, 1562, 1529, 1491, 1433, 1363, 1334, 1308, 1244, 1195, 1134, 1054, 1004, 929, 899 $cm^{-1}$
$^1$H-NMR (300MHz, $CD_3OD$, δppm) : 8.01(1H, d, *J*=7.4Hz), 7.55(1H, d, *J*=7.1Hz), 7.50-7.34(7H, m), 5.19(1H, dd, *J*=9.6, 4.6Hz), 4.73(1H, dd, *J*=13.4, 4.6Hz), 4.17(1H, dd, *J*=13.4, 9.6Hz), 3.84(3H, s)
$[\alpha]_D^{20}$ = -109° (c = 0.024, $CH_3OH$)

Example 248

(2S)-3-(4-((2,6-dichlorophenyl)carbonylamino)-2-oxohydropyrimidinyl)-2-((2-chlorophenyl)carbonylamino)propionic acid (248)

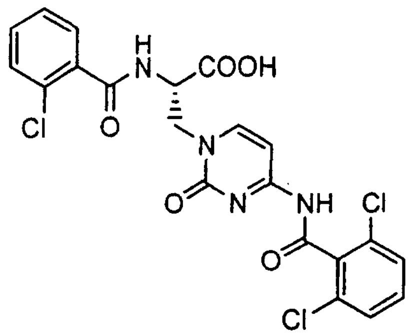

(248)

To a solution of 58.0 mg (0.11 mmol) of methyl (2S)-3-(4-((2,6-dichlorophenyl)carbonylamino)-2-oxohydropyrimidinyl)-2-((2-chlorophenyl)carbonylamino)propionate (247) in 2 ml of methanol, 0.6 ml of 1N aqueous sodium hydroxide solution was added, and the mixture was stirred at room temperature for 1 hour. To the reaction mixture, 1N hydrochloric acid was added, and the resulting mixture was extracted with ethyl acetate. Organic layers were combined, washed with saturated brine, dried over anhydrous sodium sulfate and concentrated. The residue was reprecipitated from hexane/chloroform/methanol to obtain 48.6 mg of (2S)-3-(4-((2,6-dichlorophenyl)carbonylamino)-2-oxohydropyrimidinyl)-2-((2-chlorophenyl)carbonylamino)propionic acid (248) (yield: 87%).
LR-MS (m/z) : 507 [$(M-H)^-$].
IR (KBr) : 3388, 3257, 3072, 2958, 2927, 1719, 1657, 1562, 1493, 1432, 1367, 1306, 1244, 1195, 1134, 1053, 1000, 977, 901 $cm^{-1}$
$^1$H-NMR (300MHz, $CDCl_3$, δppm) : 8.01(1H, d, *J*=7.4Hz), 7.55(1H, d, *J*=7.1Hz), 7.50-7.34(7H, m), 5.19(1H, dd, *J*=9.6, 4.6Hz), 4.73(1H, dd, *J*=13.4, 4.5Hz), 4.17(1H, dd, *J*=13.4, 9.6Hz)
$[\alpha]_D^{20}$ = -51.7° (c = 0.030, $CH_3OH$)

Example 249

Methyl (2S)-3-(4-((2,6-dichlorophenyl)carbonylamino)-2-oxohydropyrimidinyl)-2-((2-bromophenyl)carbonylamino) propionate (249)

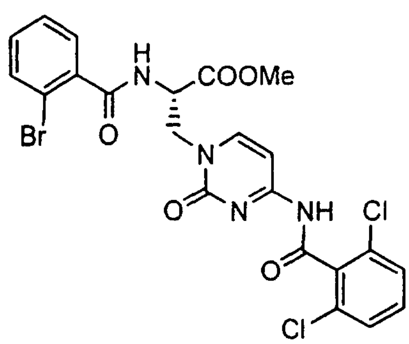

(249)

To a solution of 105 mg (0.210 mmol) of methyl 2-amino-3-(4-((2,6-dichlorophenyl)carbonylamino)-2-oxohydropyrimidinyl)propionate (232) TFA salt in 3 ml of dichloromethane, were added 0.29 ml (2.10 mmol) of triethylamine and 0.055 ml (0.420 mmol) of 2-bromobenzoyl chloride, and the mixture was stirred at room temperature for 3 hours. Saturated aqueous sodium hydrogen carbonate solution was added to the reaction mixture, and the resulting mixture

was extracted with chloroform. Organic layers were combined, washed with 1N hydrochloric acid and saturated brine, dried over anhydrous sodium sulfate and concentrated. The residue was purified by column chromatography (silica gel; chloroform/methanol = 60/1) to obtain 64 mg of methyl (2S)-3-(4-((2,6-dichlorophenyl)carbonylamino)-2-oxohydropyrimidinyl)-2-((2-bromophenyl)carbonylamino)propionate (249) (yield: 54%).
LR-MS (m/z) : 565 $[(M-H)^-]$.
IR (KBr) : 3262, 3079, 2956, 1721, 1659, 1626, 1562, 1492, 1433, 1367, 1307, 1244, 1217, 1179, 1136, 1054, 1029, 937, 902 $cm^{-1}$
$^1$H-NMR (300MHz, $CD_3OD$, δppm) : 8.00(1H, d, *J*=7.1Hz), 7.64-7.32(8H, m), 5.03(1H, dd, *J*=8.7, 5.1Hz). 4.65(1H, dd, *J*=14.0, 5.1Hz), 4.19(1H, dd, *J*=14.0, 8.7Hz), 3.81(3H, s)
$[\alpha]_D^{20}$ = -57.0° (c = 0.010, $CH_3OH$

Example 250

(2S)-3-(4-((2,6-dichlorophenyl)carbonylamino)-2-oxohydropyrimidinyl)-2-((2-bromophenyl)carbonylamino)propionic acid (250)

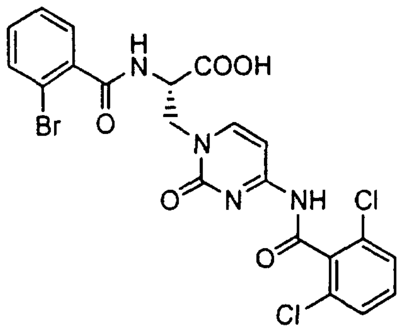

(250)

To a solution of 63.0 mg (0.11 mmol) of methyl (2S)-3-(4-((2,6-dichlorophenyl)carbonylamino)-2-oxohydropyrimidinyl)-2-((2-bromophenyl)carbonylamino)propionate (249) in 2 ml of methanol, 0.6 ml of 1N aqueous sodium hydroxide solution was added, and the mixture was stirred at room temperature for 1 hour. To the reaction mixture, 1N hydrochloric acid was added, and the resulting mixture was extracted with ethyl acetate. Organic layers were combined, washed with saturated brine, dried over anhydrous sodium sulfate and concentrated. The residue was reprecipitated from hexane/chloroform/methanol to obtain 40.4 mg of (2S)-3-(4-((2,6-dichlorophenyl)carbonylamino)-2-oxohydropyrimidinyl)-2-((2-bromophenyl)carbonylamino)propionic acid (250) (yield: 66%).
LR-MS (m/z) : 551 $[(M-H)^-]$.
IR (KBr) : 3249, 3079, 2959, 2928, 1719, 1655, 1627, 1562, 1493, 1433, 1367, 1309, 1243, 1217, 1186, 1137, 1055, 1029, 999, 978, 902 $cm^{-1}$
$^1$H-NMR (300MHz, $CD_3OD$, δppm) : 7.99(1H, d, *J*=7.5Hz), 7.63-7.32(8H, m), 5.01(1H, dd, *J*=9.0, 4.5Hz), 4.68(1H, dd, *J*=14.1, 4.5Hz), 4.15(1H, dd, *J*=14.1, 9.0Hz)
$[\alpha]_D^{20}$ = -61.5° (c = 0026., $CH_3OH$)

Example 251

Methyl (2S)-3-(4-((2,6-dichlorophenyl)carbonylamino)-2-oxohydropyrimidinyl)-2-((2-methylphenyl)carbonylamino) propionate (251)

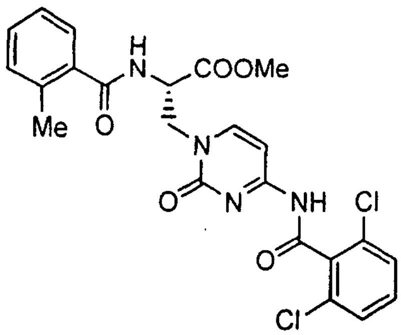

(251)

To a solution of 105 mg (0.210 mmol) of methyl 2-amino-3-(4-((2,6-dichlorophenyl)carbonylamino)-2-oxohydropyrimidinyl)propionate (232) TFA salt in 3 ml of dichloromethane, were added 0.293 ml (2.10 mmol) of triethylamine and 0.055 ml (0.42 mmol) of 2-methylbenzoyl chloride, and the mixture was stirred at room temperature for 3 hours. Saturated aqueous sodium hydrogen carbonate solution was added to the reaction mixture, and the resulting mixture was extracted with ethyl acetate. Organic layers were combined, washed with 1N hydrochloric acid and saturated brine, dried over anhydrous sodium sulfate and concentrated. The residue was purified by column chromatography (silica gel; chloroform/methanol = 60/1) to obtain 74 mg of methyl (2S)-3-(4-((2,6-dichlorophenyl)carbonylamino)-2-oxohydropyrimidinyl)-2-((2-methylphenyl)carbonylamino)propionate (251) (yield: 70%).
LR-MS (m/z) : 501 [$(M-H)^-$].
IR (KBr) : 3260, 3026, 2956, 1720, 1658, 1626, 1562, 1493, 1459, 1433, 1368, 1341, 1306, 1244, 1194, 1135, 1084, 1053, 1008, 903 $cm^{-1}$
$^1$H-NMR (300MHz, $CD_3OD$, δppm) : 7.94(1H, m), 7.60-7.17(8H, m), 5.15(1H, m), 4.65(1H, m), 4.18(1H, m), 3.80(1H, s), 3.82(2H, s), 2.57(1H, s), 2.34(2H, s)
$[\alpha]_D^{20}$ = -105° (c = 0.014, $CH_3OH$)

Example 252

(2S)-3-(4-((2,6-dichlorophenyl)carbonylamino)-2-oxohydropyrimidinyl)-2-((2-methylphenyl)carbonylamino)propionic acid (252)

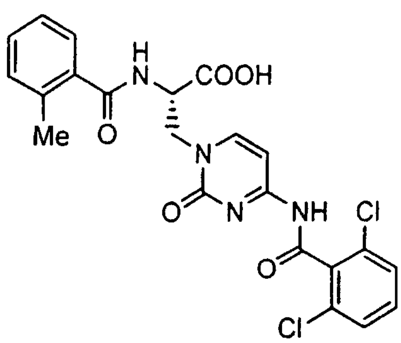

(252)

To a solution of 72.0 mg (0.14 mmol) of methyl (2S)-3-(4-((2,6-dichlorophenyl)carbonylamino)-2-oxohydropyrimidinyl)-2-((2-methylphenyl)carbonylamino)propionate (251) in 2 ml of methanol, 0.7 ml of 1N aqueous sodium hydroxide solution was added, and the mixture was stirred at room temperature for 1 hour. To the reaction mixture, 1N hydrochloric acid was added, and the resulting mixture was extracted with ethyl acetate. Organic layers were combined, washed with saturated brine, dried over anhydrous sodium sulfate and concentrated. The residue was reprecipitated from hexane/chloroform/methanol to obtain 52.4 mg of (2S)-3-(4-((2,6-dichlorophenyl)carbonylamino)-2-oxohydropyrimidinyl)-2-((2-methylphenyl)carbonylamino)propionic acid (252) (yield: 76%).
LR-MS (m/z) : 487 [$(M-H)^-$].
IR (KBr) : 3249, 3146, 3071, 2961, 1720, 1655, 1562, 1493, 1432, 1367, 1307, 1244, 1194, 1167, 1136, 1085, 1055, 997, 974, 902 $cm^{-1}$
$^1$H-NMR (300MHz, $CD_3OD$, δppm) : 8.00(1H, d, *J*=7.4Hz), 7.57(1H, d, *J*=6.8Hz), 7.47-7.29(5H, m), 7.26-7.18(2H, m), 5.16(1H, dd, *J*=9.4, 4.6Hz), 4.73(1H, dd, *J*=13.5, 4.6Hz), 4.16(1H, dd, *J*=13.5, 9.4Hz)
$[\alpha]_D^{20}$ = -70.0° (c = 0.020, $CH_3OH$)

Example 253

Methyl (2S)-3-(4-((2,6-dichlorophenyl)carbonylamino)-2-oxohydropyrimidinyl)-2-((2-methoxyphenyl)carbonylamino) propionate (253)

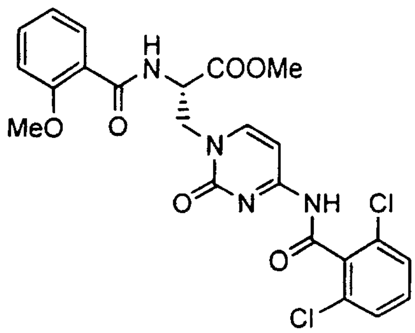

(253)

To a solution of 105 mg (0.210 mmol) of methyl 2-amino-3-(4-((2,6-dichlorophenyl)carbonylamino)-2-oxohydropyrimidinyl)propionate (232) TFA salt in 3 ml of dichloromethane, were added 0.293 ml (2.10 mmol) of triethylamine and 0.055 ml (0.42 mmol) of 2-methoxylbenzoyl chloride, and the mixture was stirred at room temperature for 3 hours. Saturated aqueous sodium hydrogen carbonate solution was added to the reaction mixture, and the resulting mixture was extracted with ethyl acetate. Organic layers were combined, washed with 1N hydrochloric acid and saturated brine, dried over anhydrous sodium sulfate and concentrated. The residue was purified by column chromatography (silica gel; chloroform/methanol = 60/1) to obtain 53 mg of methyl (2S)-3-(4-((2,6-dichlorophenyl)carbonylamino)-2-oxohydropyrimidinyl)-2-((2-methoxyphenyl)carbonylamino)propionate (253) (yield: 49%).
LR-MS (m/z) : 517 [$(M-H)^-$].
IR (KBr) : 3357, 3079, 3017, 2952, 2844, 1740, 1716, 1658, 1627, 1601, 1562, 1523, 1490, 1434, 1368, 1340, 1301, 1246, 1182, 1160, 1133, 1046, 1019, 903 $cm^{-1}$
$^1$H-NMR (300MHz, $CDCl_3$, δppm) : 8.76(1H, d, *J*=6.3Hz), 8.09(1H, dd, *J*=7.5, 1.7Hz), 7.71-6.98(9H, m), 4.93(1H, m), 4.58-4.44(2H, m), 3.97(3H, s), 3.83(3H, s)
$[\alpha]_D^{20}$ = -43.8° (c = 0.024, $CH_3OH$)

Example 254

(2S)-3-(4-((2,6-dichlorophenyl)carbonylamino)-2-oxohydropyrimidinyl)-2-((2-methoxyphenyl)carbonylamino) propionic acid (254)

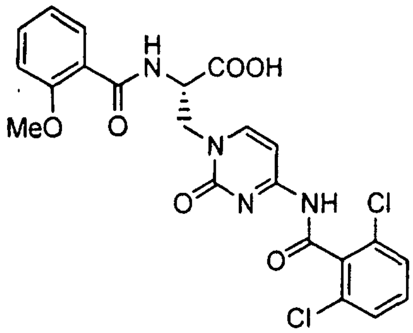

(254)

To a solution of 51 mg (0.10 mmol) of methyl (2S)-3-(4-((2,6-dichlorophenyl)carbonylamino)-2-oxohydropyrimidinyl)-2-((2-methoxyphenyl)carbonylamino)propionate (253) in 0.50 ml of methanol, 2 ml of 1N aqueous sodium hydroxide solution was added, and the mixture was stirred at room temperature for 1 hour. To the reaction mixture, 1N hydrochloric acid was added, and the resulting mixture was extracted with ethyl acetate. Organic layers were combined, washed with saturated brine, dried over anhydrous sodium sulfate and concentrated. The residue was reprecipitated from hexane/chloroform/methanol to obtain 40.2 mg of (2S)-3-(4-((2,6-dichlorophenyl)carbonylamino)-2-oxohydropyrimidinyl)-2-((2-methoxyphenyl)carbonylamino)propionic acid (254) (yield: 80%).
LR-MS (m/z) : 503 $[(M-H)^-]$.
IR (KBr) : 3350, 3084, 3018, 2945, 2844, 1743, 1715, 1639, 1601, 1563, 1523, 1486, 1433, 1370, 1352, 1306, 1248, 1198, 1182, 1157, 1135, 1108, 1049, 1018, 907 $cm^{-1}$
$^1$H-NMR (300MHz, $CD_3OD$, δppm) : 7.96-7.88(2H, m), 7.51-7.42(5H, m), 7.13-7.02(2H, m), 5.06(1H, dd, *J*=7.7, 5.0Hz), 4.66(1H, dd, *J*=13.2, 5.0Hz), 4.36(1H, dd, *J*=13.2, 7.7Hz), 3.99(3H, s)
$[\alpha]_D^{20}$ = -31.3° (c = 0.010, $CH_3OH$)

Example 255

Methyl (2S)-3-(4-((2,6-dichlorophenyl)carbonylamino)-2-oxohydropyrimidinyl)-2-((3-chlorophenylphenyl) carbonylamino)propionate (255)

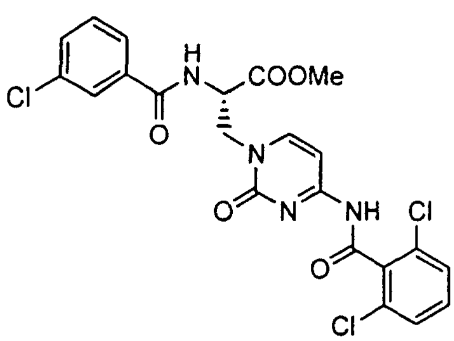

(255)

To a solution of 105 mg (0.210 mmol) of methyl 2-amino-3-(4-((2,6-dichlorophenyl)carbonylamino)-2-oxohydropyrimidinyl)propionate (232) TFA salt in 3 ml of dichloromethane, were added 0.293 ml (2.10 mmol) of triethylamine and 0.053 ml (0.42 mmol) of 2-methoxybenzoyl chloride, and the mixture was stirred at room temperature for 3 hours. Saturated aqueous sodium hydrogen carbonate solution was added to the reaction mixture, and the resulting mixture

was extracted with ethyl acetate. Organic layers were combined, washed with 1N hydrochloric acid and saturated brine, dried over anhydrous sodium sulfate and concentrated. The residue was purified by column chromatography (silica gel; chloroform/methanol = 60/1) to obtain 13 mg of methyl (2S)-3-(4-((2,6-dichlorophenyl)carbonylamino)-2-oxohydropyrimidinyl)-2-((3-chlorophenylphenyl)carbonylamino)propionate (255) (yield: 12%).
LR-MS (m/z) : 521 [(M-H)$^-$].
IR (KBr) : 3266, 3070, 2953, 2845, 1740, 1719, 1658, 1625, 1562, 1491, 1432, 1367, 1341, 1304, 1243, 1195, 1132, 1052, 1000, 900 cm-1
1H-NMR (300MHz, CDCl3, δppm): 8.90(1H, br.s), 8.32(1H, d, J=6.3Hz), 7.84(1H, t, J=1.8Hz), 7.78(1H, d, J=7.1Hz), 7.70(1H, m), 7.52(1H, d, J=7.4Hz), 7.45(1H, m), 7.40-7.25(4H, m), 5.05(1H, m), 4.59-4.41(2H, m), 3.78(3H, s)
$[\alpha]_D^{20}$ = -150° (c = 0.010, $CH_3OH$)

Example 256

(2S)-3-(4-((2,6-dichlorophenyl)carbonylamino)-2-oxohydropyrimidinyl)-2-((3-chlorophenyl)carbonylamino)propionic acid (256)

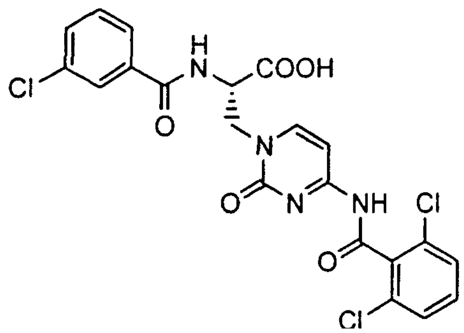

(256)

To a solution of 8.0 mg (0.015 mmol) of methyl (2S)-3-(4-((2,6-dichlorophenyl)carbonylamino)-2-oxohydropyrimidinyl)-2-((3-chlorophenyl)carbonylamino)propionate (255) in 2 ml of methanol, 0.1 ml of 1N aqueous sodium hydroxide solution was added, and the mixture was stirred at room temperature for 1 hour. To the reaction mixture, 1N hydrochloric acid was added, and the resulting mixture was extracted with ethyl acetate. Organic layers were combined, washed with saturated brine, dried over anhydrous sodium sulfate and concentrated. The residue was reprecipitated from hexane/chloroform/methanol to obtain 6.8 mg of (2S)-3-(4-((2,6-dichlorophenyl)carbonylamino)-2-oxohydropyrimidinyl)-2-((3-chlorophenyl)carbonylamino)propionic acid (256) (yield: 67%).
LR-MS (m/z) : 507 [(M-H)$^-$].
IR (KBr) : 3396, 3075, 2959, 1719, 1655, 1563, 1493, 1432, 1366, 1339, 1306, 1245, 1196, 1134, 1082, 1001, 979, 903 cm-1
$^1$H-NMR (300MHz, $CD_3OD$, δppm) 7.88(1H, d, *J*=7.1 Hz), 7.72(1H, s), 7.62(1H, d, *J*=7.7Hz), 7.47-7.31(6H, m), 4.99 (1H, dd, *J*=9.3, 4.9Hz), 4.63(1H, dd, *J*=13.7, 4.9Hz), 4.13(1H, dd, *J*=13.7, 9.3Hz)
$[\alpha]_D^{20}$ = -60.0° (c = 0.008, $CH_3OH$)

Example 257

Methyl (2S)-3-(4-((2,6-dichlorophenyl)carbonylamino)-2-oxohydropyrimidinyl)-2-((4-chlorophenyl)carbonylamino) propionate (257)

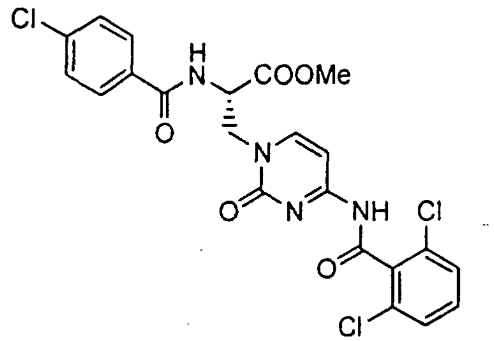

(257)

To a solution of 105 mg (0.210 mmol) of methyl 2-amino-3-(4-((2,6-dichlorophenyl)carbonylamino)-2-oxohydropyrimidinyl)propionate (232) TFA salt in 3 ml of dichloromethane, were added 0.293 ml (2.10 mmol) of triethylamine and 0.053 ml (0.42 mmol) of 4-chlorobenzoyl chloride, and the mixture was stirred at room temperature for 3 hours. Saturated aqueous sodium hydrogen carbonate solution was added to the reaction mixture, and the resulting mixture was extracted with chloroform. Organic layers were combined, washed with 1N hydrochloric acid and saturated brine, dried over anhydrous sodium sulfate and concentrated. The residue was purified by column chromatography (silica gel; chloroform/methanol = 60/1) to obtain 35 mg of methyl (2S)-3-(4-((2,6-dichlorophenyl)carbonylamino)-2-oxohydropyrimidinyl)-2-((4-chlorophenyl)carbonylamino)propionate (257) (yield: 32%).
LR-MS (m/z) : 521 $[(M-H)^-]$.
IR (KBr) : 3265, 3070, 2953, 2851, 1719, 1655, 1625, 1594, 1562, 1488, 1433, 1369, 1340, 1304, 1243, 1196, 1160, 1133, 1092, 1053, 1014, 903 $cm^{-1}$
$^1$H-NMR (300MHz, $CDCl_3$, δppm) : 9.24(1H, br.s), 8.30(1H, d, *J*=6.3Hz), 7.82-7.73(3H, m), 7.53(1H, d, *J*=7.4Hz), 7.41-7.29(5H, m), 5.03(1H, ddd, *J*=6.6, 6.6, 4.4Hz), 4.53(1H, dd, *J*=13.7, 4.4Hz), 4.44(1H, dd, *J*=13.7, 6.6Hz), 3.77 (3H, s)
$[\alpha]_D^{20}$ = -157° (c = 0.022, $CH_3OH$)

Example 258

(2S)-3-(4-((2,6-dichlorophenyl)carbonylamino)-2-oxohydropyrimidinyl)-2-((4-chlorophenyl)carbonylamino)propionic acid (258)

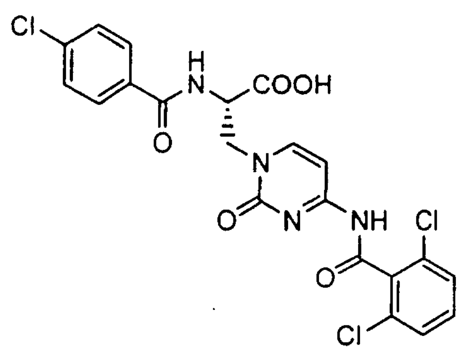

(258)

To a solution of 33 mg (0.063 mmol) of methyl (2S)-3-(4-((2,6-dichlorophenyl)carbonylamino)-2-oxohydropyrimidinyl)-2-((4-chlorophenyl)carbonylamino)propionate (257) in 2 ml of methanol, 0.3 ml of 1N aqueous sodium hydroxide solution was added, and the mixture was stirred at room temperature for 1 hour. To the reaction mixture, 1N hydrochloric acid was added, and the resulting mixture was extracted with ethyl acetate. Organic layers were combined, washed with saturated brine, dried over anhydrous sodium sulfate and concentrated. The residue was reprecipitated from hexane/chloroform/methanol to obtain 26.9 mg of (2S)-3-(4-((2,6-dichlorophenyl)carbonylamino)-2-oxohydropyrimidinyl)-2-((4-chlorophenyl)carbonylamino)propionic acid (258) (yield: 88%).
LR-MS (m/z) : 507 $[(M-H)^-]$.
IR (KBr) : 3370, 3294, 3083, 2960, 1719, 1654, 1595, 1563, 1489, 1432, 1366, 1340, 1305, 1244, 1196, 1161, 1133, 1093, 1058, 1013, 975, 902 $cm^{-1}$
$^1$H-NMR (300MHz, $CD_3OD$, δppm) : 7.98(1H, d, *J*=7.4Hz), 7.81-7.73(2H, m), 7.52-7.38(7H, m), 5.07(1H, dd, *J*=9.1, 4.4Hz), 4.72(1H, dd, *J*=13.0, 4.4Hz), 4.24(1H, dd, *J*=13.0,9.1Hz)
$[\alpha]_D^{20}$ = -44.2° (c = 0.012, $CH_3OH$)

Example 259

Methyl (2S)-2-((tert-butoxy)carbonylamino)-3-(4-((2,6-dimethylphenyl)carbonylamino)-2-oxohydropyrimidinyl) propionate (259)

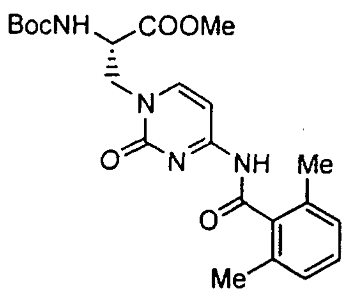

(259)

To a solution of 300 mg (2.00 mmol) of 2,6-dimethylbenzoic acid in 3 ml of dichloromethane, 0.26 ml (3.00 mmol) of oxalyl chloride was added, and the mixture was stirred at room temperature for 1 hour. After concentrating the reaction mixture, the residue was suspended in 1 ml of dichloromethane, and then 312 mg (1.00 mmol) of methyl 3-(4-amino-2-oxohydropyrimidinyl)-2-((tert-butoxy)carbonylamino)propionate (230) and 1.5 ml of pyridine were added, followed by stirring the mixture at 50°C for 2.5 hours. Methanol was added to the reaction solution and the resulting mixture was concentrated. To the residue, 1N hydrochloric acid was added and the resulting mixture was extracted with ethyl acetate. The organic layers were combined, washed with saturated brine, dried over anhydrous sodium sulfate and concentrated. The residue was purified by silica gel column chromatography (hexane:ethyl acetate = 1: 5-0:1) to obtain 308 mg of methyl (2S)-2-((tert-butoxy)carbonylamino)-3-(4-((2,6-dimethylphenyl)carbonylamino)-2-oxohydropyrimidinyl)propionate (259) as an amorphous product (yield: 69%).
LR-MS(m/z) : 444 $(M^+)$
IR(KBr):3442, 3234, 3152, 3082, 2966, 2929, 1695, 1626, 1567, 1488, 1443, 1420, 1369, 1334, 1306, 1242, 1163, 1118, 1065, 1031, 1011, 935, 904 $cm^{-1}$
$^1$H-NMR(300MHz, $CDCl_3$,δppm) : 8.33(1H, br.s), 7.63(1H, d, *J*=7.4Hz), 7.57(1H, d, *J*=7.1Hz), 7.22(1H, d, *J*=8.0Hz), 7.07(2H, d, *J*=7.7Hz), 5.58(1H, br.s), 4.60(1H, m), 4.44-4.31(2H, m), 3.81(3H, s), 2.35(6H, s), 1.43(9H, s)

Example 260

Methyl (2S)-2-((2,6-dichlorophenyl)carbonylamino)-3-(4-((2,6-dimethylphenyl)carbonylamino)-2-oxohydropyrimidinyl) propionate (260)

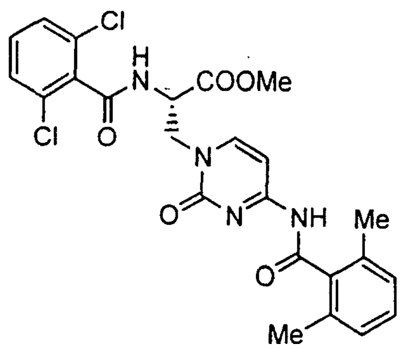

(260)

To a solution of 66.7 mg (0.15 mmol) of methyl (2S)-2-((tert-butoxy)carbonylamino)-3-(4-((2,6-dimethylphenyl) carbonylamino)-2-oxohydropyrimidinyl)propionate (259) in 2 ml of dichloromethane, 1 ml of trifluoroacetic acid was added, and the mixture was stirred at room temperature for 1 hour. After concentrating the reaction mixture, the residue was dissolved in 2 ml of dichloromethane, and then 0.17 ml (0.12 mmol) of triethylamine and 0.043 ml (0.30 mmol) of 2,6-dichlorobenzoyl chloride were added, and the mixture was stirred overnight at room temperature. To the reaction mixture, 1N hydrochloric acid was added, and the resulting mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 1/5-0/1) to obtain 66.0 mg of methyl (2S)-2-((2,6-dichlorophenyl)carbonylamino)-3-(4-((2,6-dimethylphenyl)carbonylamino)-2-oxohydropyrimidinyl)propionate (260) as an amorphous product (yield: 85%).
LR-MS (m/z) : 515 [$(M-H)^-$].
IR (KBr) : 3356, 3273, 3065, 2954, 2852, 1744, 1713, 1665, 1622, 1552, 1481, 1431, 1365, 1335, 1307, 1255, 1235, 1195, 1177, 1118, 1086, 1068, 1004, 898 $cm^{-1}$
$^1$H-NMR (300MHz, $CDCl_3$, δppm) : 8.03(1H, d, *J*-7.4Hz), 7.59(1H, d, *J*=7.1Hz), 7.43-7.38(3H, m), 7.24(1H, dd, *J*=8.4, 7.1Hz), 7.11(2H, d, *J*=7.7Hz), 5.32(1H, dd, *J*=9.3, 5.3Hz), 4.59(1H, dd, *J*=13.5, 5.3Hz), 4.18(1H, dd, *J*=13.5, 5.3Hz), 3.81(3H, s), 2.32(6H, s)

Example 261

(2S)-2-((2,6-dichlorophenyl)carbonylamino)-3-(4-((2,6-dimethylphenyl)carbonylamino)-2-oxohydropyrimidinyl) propionic acid (261)

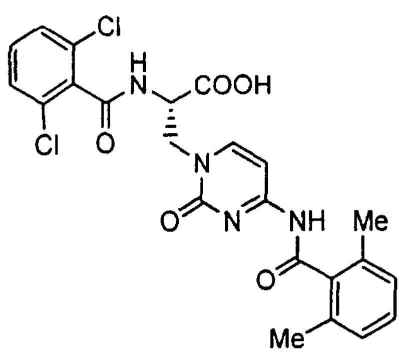

(261)

To a solution of 53.6 mg (0.10 mmol) of methyl (2S)-2-((2,6-dichlorophenyl)carbonylamino)-3 -(4-((2,6-dimethylphenyl)carbonylamino)-2-oxohydropyrimidinyl)propionate (260) in 2 ml of methanol/THF (1/1), 0.4 ml of 1N aqueous sodium hydroxide solution was added, and the mixture was stirred overnight at room temperature. To the reaction mixture, 1N hydrochloric acid was added, and the resulting mixture was extracted with ethyl acetate. Organic layers were combined, washed with saturated brine, dried over anhydrous sodium sulfate and concentrated. The residue was washed with ether to obtain 36.0 mg of (2S)-2-((2,6-dichlorophenyl)carbonylamino)-3-(4-((2,6-dimethylphenyl)carbonylamino)-2-oxohydropyrimidinyl)propionic acid (261) as an amorphous product (yield: 69%).
LR-MS (m/z) : 501 [$(M-H)^-$].
IR(KBr) : 3257, 3142, 3067, 2970, 1702, 1672, 1616, 1579, 1558, 1496, 1428, 1386, 1365, 1315, 1261, 1237, 1195, 1176, 1119, 1076, 997, 969 $cm^{-1}$
$^1$H-NMR (300MHz, $CD_3OD$, δppm) : 8.05(1H, d, *J*=7.1 Hz), 7.58(1H, br.d, *J*=7.4Hz), 7.44-7.32(3H, m), 7.24(1H, dd, *J*=8.1, 7.0Hz), 7.11(2H, d, *J*=7.4Hz), 5.30(1H, dd, *J*=10.0, 4.7Hz), 4.60(1H, dd, *J*=13.5, 4.7Hz), 4.14(1H, dd, *J*=13.5, 10.0Hz), 2.32(6H, s)

Example 262

Methyl (2S)-3-(4-((2,6-dimethoxyphenyl)carbonylamino)-2-oxohydropyrimidinyl)-2-((tert-butoxy)carbonylamino) propionate (262)

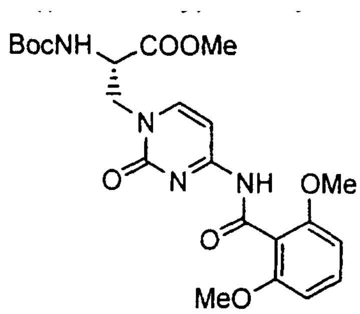

(262)

To a solution of 364 mg (2.00 mmol) of 2,6-dimethoxybenzoic acid in 3 ml of dichloromethane, 0.26 ml (3.00 mmol) of oxalyl chloride was added, and the mixture was stirred at room temperature for 1 hour. After concentrating the reaction mixture, the residue was suspended in 1 ml of dichloromethane, and then 312 mg (1.00 mmol) of methyl 3-(4-amino-2-oxohydropyrimidinyl)-2-((tert-butoxy)carbonylamino)propionate (230) and 1.5 ml of pyridine were added, followed by stirring the mixture at 50°C for 40 minutes. Methanol was added to the reaction solution and the resulting mixture was concentrated. To the residue, 1N hydrochloric acid was added and the resulting mixture was extracted with ethyl acetate. The organic layers were combined, washed with saturated brine, dried over anhydrous sodium sulfate and concentrated. The residue was purified by silica gel column chromatography (hexane:ethyl acetate = 1: 5-0:1) to obtain 275 mg of methyl (2S)-3-(4-((2,6-dimethoxyphenyl)carbonylamino)-2-oxohydropyrimidinyl)-2-((tert-butoxy)carbonylamino)propionate (262) as an amorphous product (yield: 58%). LR-MS(m/z) : 476 ($M^+$)
IR(KBr) : 3294, 3147, 2976, 2843, 1708, 1664, 1626, 1596, 1561, 1478, 1434, 1368, 1341, 1308, 1255, 1161, 1112, 1059, 1027, 1001, 900 $cm^{-1}$
$^1$H-NMR(300MHz, $CDC_3$,δppm) : 8.39(1H, br.s), 7.56-7.53(2H, m), 7.36(1H, t, *J*=8.5Hz), 6.60(2H, d, *J*=8.2Hz), 5.59 (2H, d, *J*=6.8Hz), 4.57(1H, m), 4.40-4.30(2H, m), 3.85(6H, s), 3.80(3H, s), 1.44(9H, s)

Example 263

Methyl (2S)-2-((2,6-dichlorophenyl)carbonylamino)-3-(4-((2,6-dimethoxyphenyl)carbonylamino)-2-oxohydropyrimidinyl)propionate (263)

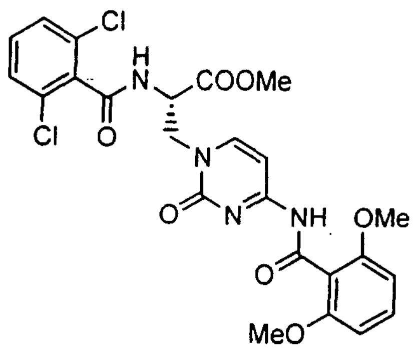

(263)

To a solution of 71.5 mg (0.15 mmol) of methyl (2S)-3-(4-((2,6-dimethoxyphenyl)carbonylamino)-2-oxohydropyrimidinyl)-2-((tert-butoxy)carbonylamino)propionate (262) in 2 ml of dichloromethane, 1 ml of trifluoroacetic acid was added, and the mixture was stirred at room temperature for 1 hour. After concentrating the reaction mixture, the residue was dissolved in 2 ml of dichloromethane, and then 0.17 ml (0.12 mmol) of triethylamine and 0.043 ml (0.30 mmol) of 2,6-dichlorobenzoyl chloride were added, followed by stirring the mixture overnight at room temperature. To the reaction mixture, 1N hydrochloric acid was added, and the resulting mixture was extracted with ethyl acetate. Organic layers were combined, washed with saturated aqueous sodium hydrogen carbonate solution and saturated brine, dried over anhydrous sodium sulfate and concentrated. The residue was purified by column chromatography (silica gel; hexane/ethyl acetate = 1/5-0/1) to obtain 68.7 mg of methyl (2S)-2-((2,6-dichlorophenyl)carbonylamino)-3-(4-((2,6-dimethoxyphenyl)carbonylamino)-2-oxohydropyrimidinyl)propionate (263) as an amorphous product (yield: 83%).
LR-MS (m/z) : 547 [$(M-H)^-$].
IR (KBr) : 3596, 3556, 3493, 1295, 3045, 3009, 2976, 2946, 2841, 1739, 1711, 1661, 1628, 1598, 1561, 1478, 1433, 1371, 1349, 1349, 1310, 1290, 1254, 1194, 1174, 1112, 1069, 1031, 995 $cm^{-1}$
$^1$H-NMR (300MHz, $CD_3OD$, δppm) : 7.99(1H, d, *J*=7.2Hz), 7.53(1H, d, *J*=7.4Hz), 7.45-7.35(4H, m), 6.72(2H, d, *J*=8.5Hz), 5.29(1H, dd, *J*=9.0, 5.3Hz), 4.57(1H, dd, *J*=13.5, 5.3Hz), 4.18(1H, dd, *J*=13.5, 9.0Hz), 3.83(6H, s), 3.80(3H, s)

Example 264

(2S)-2-((2,6-dichlorophenyl)carbonylamino)-3-(4-((2,6-dimethoxyphenyl)carbonylamino)-2-oxohydropyrimidinyl) propionic acid (264)

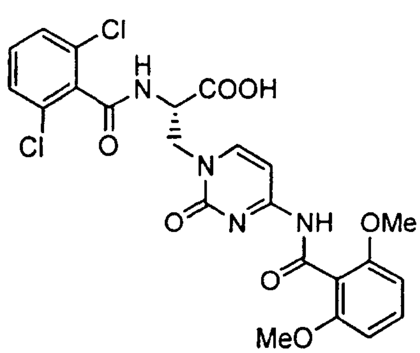

(264)

To a solution of 59.2 mg (0.11 mmol) of methyl (2S)-2-((2,6-dichlorophenyl)carbonylamino)-3-(4-((2,6-dimethoxyphenyl)carbonylamino)-2-oxohydropyrimidinyl)propionate (263) in 2 ml of methanol/THF (1/1), 0.4 ml of 1N aqueous sodium hydroxide solution was added, and the mixture was stirred overnight at room temperature. To the reaction mixture, 1N hydrochloric acid was added, and the resulting mixture was extracted with ethyl acetate. Organic layers were combined, washed with saturated brine, dried over anhydrous sodium sulfate and concentrated. The residue was washed with ether to obtain 40.0 mg of (2S)-2-((2,6-dichlorophenyl)carbonylamino)-3-(4-((2,6-dimethoxyphenyl)carbonylamino)-2-oxohydropyrimidinyl)propionic acid (264) as an amorphous product (yield: 70%).
LR-MS (m/z) : 533 [$(M-H)^-$].
IR (KBr) : 3423, 3262, 3151, 3071, 2978, 2939, 2843, 1712, 1675, 1617, 1594, 1561, 1499, 1476, 1432, 1388, 1365, 1321, 1255, 1195, 1175, 1113, 1075, 1030, 1005, 972, 894 $cm^{-1}$
$^1$H-NMR (300MHz, $CD_3OD$, δppm) : 8.01(1H, d, *J*=7.1 Hz), 7.52(1H, d, *J*=7.1Hz), 7.44-7.32(4H, m), 6.72(2H, d, *J*=8.5Hz), 5.26(1H, dd, *J*=10.0, 5.0Hz), 4.58(1H, dd, *J*=13.7, 5.0Hz), 4.14(1H, dd, *J*=13.7, 10.0Hz), 3.83(6H, s)

Example 265

Effects of Compounds for Inhibition of Binding Between CS-1 Peptide and VLA-4-IgG

A conjugate between a peptide (Gys Leu His Gly Pro Glu Ile Leu Asp Val Pro Ser Thr) containing CS-1 sequence and rabbit IgG (Sigma) was prepared by the reported method (Humphries, M.J. et al. J. Biol.Chem., 262, 6886-6892 (1987)). The obtained conjugate was diluted with phosphate buffer (hereinafter referred to as "PBS(-)", NISSUI) and the resulting solution was placed in wells of a 96-well immunoplate (NUNC) in an amount of 100 μl/well, followed by leaving the solution to stand at 4°C for 16 hours to immobilize the conjugate.

Then each well was washed twice with PBS(-), and a PBS solution containing 1% BSA heat-treated at 80°C for 10 minutes was placed in each well in an amount of 300 μl/well. The immunoplate was left to stand at 4°C for 3 hours, and then the solution in each well was removed by aspiration.

After preliminarily allowing each compound to react with VLA-4-IgG chimeric protein (100 μl) at room temperature for 20 minutes, the resulting solution was allowed to react with CS-1 peptide in each well at 30°C for 3 hours. Thereafter, non-bound VLA-4-IgG chimeric protein was removed by aspiration and each well was washed twice with 0.1% BSA-containing TBS buffer (150 mM NaCl,25 mM Tris-HCl, 1 mM $MnCl_2$ pH 7.4). To the bound VLA-4-IgG chimeric protein, biotin-labeled anti-human IgG antibody (Vector) as an primary antibody was added, and then avidin-labeled horse radish peroxidase (Sigma) as a secondary antibody was added, and the reaction was allowed to occur. After the reaction, *o*-phenylenediamine as a substrate was added to color the solution, and the absorbance at 490 nm was measured. From the absorbance, the binding-inhibition activity of each test compound was determined. The inhibition activities of 9 compounds are shown in Table 1.

Table 1

| Compound No. | Inhibition Activity($IC_{50}$ : nM) |
|---|---|
| 8 | 200 |
| 14 | 500 |
| 19 | 500 |
| 62 | 90 |
| 74 | 120 |
| 102 | 500 |
| 120 | 160 |
| 131 | 1800 |
| 140 | 1800 |
| 143 | 13 |
| 154 | 430 |
| 161 | 64 |
| 171 | 780 |
| 176 | 13 |
| 180 | 38 |
| 193 | 2200 |

Example 266

Effect of Compound in Mouse Peritonitis Induced by Ragweed Pollen

Ragweed pollen extract 1:100 (TORII PHARMACEUTICAL) was diluted by 10-fold with physiological saline, and the resulting solution was used as an antigen for sensitization. This antigen solution was subcutaneously administered to each BLAB/c mouse (female, 6 weeks old, JAPAN CHARLES RIVER) in an amount of 100 µl each on DAY-0 and DAY-1, and 200 µl each on DAY-6, DAY-8 and DAY-14 to obtain sensitized mice, DAY-0 being the day of the initial sensitization. On DAY-20, 200 µl of the antigen solution was intraperitoneally administered to induce peritonitis. After 48 hours from the induction, each mouse was sacrificed by bleeding to death, and 3 ml of heparin-containing PBS(-) was intraperitoneally injected. Celiotomy was performed and washing solution of the abdominal cavity was recovered. The leukocytes contained in the recovered washing solution were subjected to site spin and a smear specimen was prepared. After staining the specimen by May-Giemsa staining method, the leukocytes were grouped and counted under microscope to determine the number of eosinophils. Compound No. 140 used as the test compound was dissolved in physiological saline and the solution was intravenously administered totally 5 times, i.e., at 16 hours before induction, at the time of induction, at 8 hours, 24 hours and 32 hours after induction, at a dose of 400 mg/kg per each time. The experiment was carried out using 7 mice in each group.

As a result, Compound No. 140 inhibited accumulation of leukocytes, especially eosinophils, to abdominal cavity, and the inhibition rate at 400 mg/kg was 31.1%. This result shows that this compound is effective for therapy of allergic diseases.

Example 267

Effect of Compound in Auricular Edema Model

To each BLAB/c mouse (female, 7 weeks old, JAPAN CHARLES RIVER), mouse IgE anti-DNP antibody (5 mg/kg, SEIKAGAKU CORPORATION) was intravenously administered to passively sensitize the mouse. Twenty four hours later, 20 µl of 0.38% 2,4- dinitrofluorobenzene (DNFB, Aldrich) dissolved in acetone:olive oil (4:1) was applied to the auricle of the sensitized mouse to induce inflammation. The thickness of the auricle was measured until 24 hours after the induction with Digimatic Indicator (Mitutoyo) and the ear swelling rate was calculated according to the following equation:

$$\text{Ear Swelling Rate (\%)} = (A - B)/B \times 100$$

A: Thickness of right auricle at 24 hours after induction
B: Thickness of right auricle before induction

Twenty four hours after the induction, the auricle was cut off and fixed with formalin. After the fixation, continuous sections of the auricle were prepared and stained by Luna staining. The number of eosinophils in each section was counted and the total of them was defined as the number of eosinophils accumulated in the auricle.

Compound No. 140 used as the test compound was dissolved in physiological saline and the solution was intravenously administered totally 3 times, i.e., at the time of sensitization, at 8 hours and 24 hours after sensitization, at a dose of 400 mg/kg per each time. Further, to examine the effect of the compound when applied to the auricle, the compound was dissolved in ethanol, and the solution was applied to the auricle totally twice, i.e., at 8 hours after sensitization and at 1 hour before the induction, at a dose of 0.3 mg/ear. To the background group and the control group, physiological saline was intravenously administered and ethanol was applied, respectively. The experiment was carried out using 4 to 6 mice in each group. The results are shown in terms of mean ± standard error.

As a result, it was proved that Compound 140 inhibited swelling of ear and accumulation of eosinophils to auricle, induced by application of the antigen (Table 2, Fig. 1). These results suggest that the compound is effective for the therapy of allergic dermatitis.

Table 2

| Effect of Compound No. 140 for Swelling of Ear Induced by Antigen | | |
|---|---|---|
| | Ear Swelling Rate (%) | Inhibition Rate (%) |
| Background | 10.08 | 100 |

Table 2 (continued)

| Effect of Compound No. 140 for Swelling of Ear Induced by Antigen | | |
|---|---|---|
| | Ear Swelling Rate (%) | Inhibition Rate (%) |
| Control | 36.96 | 0 |
| Compound 140(applied to auricle) | 26.12 | 40.3 |

Example 268

Effect of Compound in Mouse Asthma Model

Equivolume of 10% $KAl(SO_4)_2 \cdot 12H_2O$ (WAKO) solution and ovalbumin (hereinafter "OVA") solution (500 µg/ml) were mixed, and 10N NaOH was slowly added to neutralize the solution to obtain $Al(OH)_3$ gel (OVA 500 µg/ml) on which OVA was adsorbed. The obtained product was intraperitoneally administered to each BLAB/c mouse (female, 7 weeks old, JAPAN CHARLES RIVER) at a dose of 200 µl (100 µg) per mouse on DAY-0 and DAY-14. Thereafter, on DAY-14, 50 µl of 2 mg/ml solution of OVA in physiological saline was intranasally administered, and on DAY-25, 26 and 27, 50 µl of 1 mg/ml solution of OVA in physiologically saline was intranasally administered to induce inflammation. Compound No. 140 used as the test compound was dissolved in physiological saline and the solution was intranasally administered at 30 minutes before induction with OVA, at 8 hours after induction, and on DAY-25, 26 and 27, twice a day, at a dose of 750 µg per time. To the background group and control group, physiological saline was intranasally administered.

On DAY-28, bronchus of each mouse was incised and the bronchus and alveoli were washed (BAL) 4 times with 0.7 ml of physiological saline. After the washing, the washing fluid (BALF) of the bronchus and alveoli was recovered and centrifuged. The leukocytes contained in the BALF were subjected to site spin to prepare a smear specimen. After staining the specimen by May-Giemsa staining method, the leukocytes were grouped and counted under microscope to determine the number of eosinophils. The experiment was carried out using 7 mice in each group. The results are shown in terms of mean ± standard error.

As a result, it was proved that Compound 140 inhibited accumulation of leukocytes, especially eosinophils, in the lung (Fig. 2). These results suggest that the compound is effective for the therapy of allergic inflammatory diseases.

Example 269

Effect of Compound in Guinea Pig Rhinitis Model

Cyclophosphamide (SIGMA) was dissolved in physiological saline to a concentration of 25 mg/ml, and the solution was intraperitoneally administered to each Harley guinea pig (male, 2 weeks old, JAPAN SLC) at a dose of 50 mg/kg on DAY-0. Equivolume of OVA (2.0 mg/ml) dissolved in physiological saline and 12 ml of aqueous 9% $AlK(SO_4)_2$ solution were mixed, and then 10% aqueous $Na_2CO_3$ solution was added thereto to adjust the pH to 6.5, thereby obtaining $Al(OH)_3$ gel (OVA 500 µg/ml) on which OVA was adsorbed. The obtained product was centrifuged and the precipitated gel was washed with PBS(-), followed by addition of *Bordetella pertussis* cell suspension (WAKO PURE CHEMICALS). The OVA concentration was adjusted to 100 µg/ml, and 1 ml (100 µg) of the resulting product was intraperitoneally administered to each guinea pig. On DAY-22, each mice was made to inhale 1% OVA dissolved in physiological saline for 10 minutes. On DAY-29, 40 µl (40 µg) of 0.1% OVA dissolved in physiological saline was intranasally administered to carry out induction by the antigen. The increase in the respiratory resistance from 10 minutes after the induction to 8 hours after the induction was measured by a respiratory resistance-measuring apparatus (body plethysmograph method; SHIZUME MEDICAL), and the result was expressed in terms of the ratio of the measured resistance based on the respiratory resistance measured before the induction by the antigen. The experiment was carried out using 6 to 10 mice in each group. The results are shown in terms of mean ± standard error.

Compound No. 140 was dissolved in physiological saline and 10 mg was intranasally administered before induction by the antigen. To the background group and the control group, physiological saline was intranasally administered.

As a result, it was proved that Compound 140 inhibited increase in the respiratory resistance observed in the control group (Fig. 3). These results suggest that the compound is effective for the therapy of allergic inflammatory diseases such as bronchial asthma, allergic rhinitis and pollinosis.

Example 270 Radical Scavenger Action

The radical scavenger action of the compounds was measured using the amount of the superoxide as an index, which is produced when xanthine and xanthine oxidase are reacted. First, 1 mM of xanthine (SIGMA) dissolved in Hanks buffer solution (GIBCO), 2 mM dietylenetriamine-N,N,N',N"-pentaacetic acid (DOJIN) and 40 μM 2-methyl-6-phenyl-3,7-dihydroimidazo[1,2-a]pyrazin-3-one (TOKYO KASEI KOGYO) dissolved in distilled water when use were mixed. Further, 0.3 U/ml xanthine oxidase (BMY) dissolved in Hanks buffer when use was added. Luminescence intensity at 380 nm from 10 seconds to 60 seconds after the addition was measured by Lumat (Berthold, LB9501), and the measured amount was defined as the amount of produced superoxide. The test compound was added to the xanthine-containing solution before addition of the xanthine oxidase, and the resulting mixture was preincubated at 25°C for 5 minutes to examine its influence.

As a result, Compound Nos. 240 and 236 exhibited radical scavenger activities 2 to 5 times higher than that of 3-(4-((2,6-dichlorophenyl)carbonylamino)phenyl)-2-((6-bromo-2-methylphenyl)carbonylamino)propanoic acid. It is known that radicals produced by activated leukocytes and the like promote inflammation. Thus, it was suggested that Compound Nos. 240 and 236 suppress the progress of inflammatory symptoms by the radical scavenger actions.

Example 271 Influence by Compound on Cell Survival Rate

Jurkat cells (ATCC TIB-152) passaged on 10% fetal calf serum-containing RPMI medium (10F-RPMI, GIBCO) were suspended in the same medium to a cell population of 5 x $10^5$ cells/ml, and the suspension was placed in each well of a 96-well plate (COSTAR) in an amount of 100 μl/well, followed by culturing the cells at 37°C under 5% $CO_2$ for 24 hours. Then alamarBlue®(BioSource) was added in an amount of 10 μl/well and the cells were cultured for another 4 hours. Thereafter, the absorbance at 570 nm and 600 nm was measured with a microplate reader (BioRad model 3550). The test compound dissolved in 10F-RPMI was mixed with the Jurkat cells and the cells were cultured for 24 hours to examine its influence.

As a result, the influences of Compound Nos. 240 and 236 on the survival rate of the cells were not more than 1/4 of that of 3-(4-((2,6-dichlorophenyl)carbonylamino)phenyl)-2-((6-bromo-2-methylphenyl)carbonylamino)propanoic acid. Thus, it was proved that both of these compounds are less toxic than 3-(4-((2,6-dichlorophenyl)carbonylamino)phenyl)-2-((6-bromo-2-methylphenyl)carbonylamino)propanoic acid.

Example 272 Effect of Compound in Auricular Edema Model

To each BLAB/c mouse (female, 7 weeks old, JAPAN CHARLES RIVER), mouse IgE anti-DNP antibody (5 mg/kg, SEIKAGAKU CORPORATION) was intravenously administered to passively sensitize the mouse. Twenty four hours later, 20 μl of 0.38% 2,4- dinitrofluorobenzene (DNFB, Aldrich) dissolved in acetone:olive oil (4:1) was applied to the auricle of the sensitized mouse to induce inflammation. The thickness of the auricle was measured until 24 hours after the induction with Digimatic Indicator (Mitutoyo) and the ear swelling rate was calculated according to the following equation:

$$\text{Ear Swelling Rate (\%)} = (A - B)/B \times 100$$

A: Thickness of right auricle at 24 hours after induction
B: Thickness of right auricle before induction

Twenty four hours after the induction, the auricle was cut off and fixed with formalin. After the fixation, continuous sections of the auricle were prepared and stained by Luna staining. The number of eosinophils in each section was counted. Compound No. 143 used as the test compound was dissolved in physiological saline and the solution was topically or systemically administered totally 3 times, i.e., at the time of sensitization, at 8 hours and 24 hours after sensitization. The experiment was carried out using 4 to 6 mice in each group. The results are shown in terms of mean ± standard error.

As a result, Compound No. 143 inhibited swelling of ear and accumulation of eosinophils to auricle, induced by application of the antigen, at a dose of not more than 150 mg/kg. These results suggest that the compound is effective for the therapy of allergic diseases such as allergic dermatitis, and of chronic diseases.

Example 273 Effect of Compound in Mouse Asthma Model

A mixed solution containing aluminum hydroxide gel (Alum) on which ovalbumin (hereinafter "OVA") was

adsorbed (20 ∝g OVA/2mg Alum/200 ∝l physiological saline) was intraperitoneally administered to each BLAB/c mouse (female, 7 weeks old, JAPAN CHARLES RIVER) at a dose of 200 ∝l (100 ∝g) per time on DAY-1 and DAY-14 to sensitize the mouse. Then each mice was made to aspire 2% OVA dissolved in physiological saline on DAY-28, 29 and 30 to induce inflammation. Compound Nos. 143 and 236 used as the test compounds were dissolved in physiological saline and each solution was topically or systemically administered at 30 minutes before induction by OVA, and twice a day after induction.

On DAY-31, development of airway anaphylaxis was measured using a non-restrictive chamber (BUXCO). Then the bronchus of each mouse was incised and the bronchus and alveoli were washed (BAL) with 0.7 ml of physiological saline. After the washing, the washing fluid (BALF) of the bronchus and alveoli was recovered and centrifuged. The leukocytes contained in the BALF were subjected to site spin to prepare a smear specimen. After staining the specimen by May-Giemsa staining method, the leukocytes were grouped and counted under microscope to determine the number of eosinophils. The experiment was carried out using 6 to 8 mice in each group. The results are shown in terms of mean ± standard error.

As a result, Compound Nos. 143 and 236 inhibited progress of airway anaphylaxis and accumulation of eosinophils to the lung, at a dose of not more than 150 mg/kg, when topically or systemically administered. These results suggest that the compounds are effective for the therapy of allergic diseases such as bronchial asthma.

## Claims

1. A carboxylic acid derivative of the Formula I:

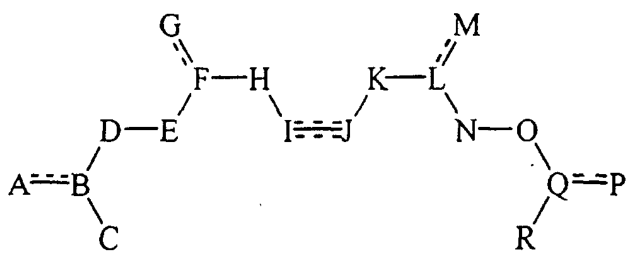

I

[In Formula I, A, C, P and R may or may not exist, and may be the same or different, in cases where A, C, P or R exists, they respectively represent (i) hydrogen, (ii) $C_1$-$C_6$ straight alkyl, (iii) $C_3$-$C_8$ branched alkyl, (iv) Formula II:

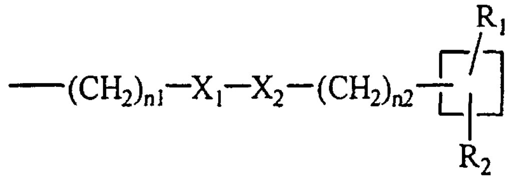

II

(wherein n1 and n2 represent numbers of 0 to 3, $X_1$ and $X_2$ may or may not exist, in cases where $X_1$ or $X_2$ exists, $X_1$ represents an oxygen atom, sulfur atom or nitrogen atom, $X_2$ represents carbonyl group or sulfonyl group; □ represents cyclohexane ring, benzene ring, naphthalene ring, indole ring, imidazole ring, furan ring, thiophen ring, pyrrole ring or pyridine ring; $R_1$ and $R_2$ may or may not exist, and may be the same or different, in cases where $R_1$ or $R_2$ exists, they respectively represent $C_1$-$C_6$ straight alkyl, $C_3$-$C_8$ branched alkyl, hydroxyl, methoxy, chloro, bromo, fluoro, nitro, amino, Formula III:

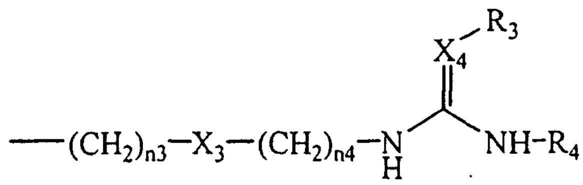

III

(wherein n3 and n4 represent numbers of 0 to 3; $X_3$ may or may not exist, in cases where $X_3$ exists, $X_3$ represents a nitrogen atom or oxygen atom; $X_4$ represents a carbon atom, oxygen atom, nitrogen atom or sulfur atom; in cases where $X_4$ is a carbon atom or nitrogen atom, $R_3$ exists and represents nitro, cyano, methylsulfonyl or phenylsulfonyl group; $R_4$ represents hydrogen, $C_1$-$C_6$ straight alkyl, $C_3$-$C_8$ branched alkyl, $C_6$-$C_{10}$ non-substituted aryl or $C_6$-$C_{10}$ aryl substituted with 0 to 2 hydroxyl groups, methoxy groups, nitro groups, amino groups, chloro, bromo and/or fluoro) or Formula IV:

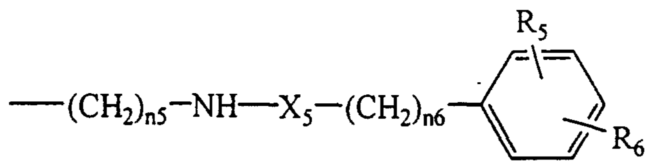

IV

(wherein n5 and n6 represent numbers of 0 to 3; $X_5$ represents carbonyl group or sulfonyl group; $R_5$ and $R_6$ represent hydroxyl, methyl, methoxy, chloro, bromo, fluoro, nitro or amino groups)

B and Q may or may not exist, and may be the same or different, in cases where B or Q exists, they represent (i) nitrogen atom, (ii) carbon atom, (iii) $C_6$-$C_{10}$ aryl or cyclohexyl each of which is bound through 0 to 3 methylene groups, each of which is substituted with 0 to 3 methyl groups, ethyl groups, hydroxyl groups, methoxy groups, chloro, bromo, fluoro, nitro groups, tetrazole groups or amino groups, (iv) Formula V:

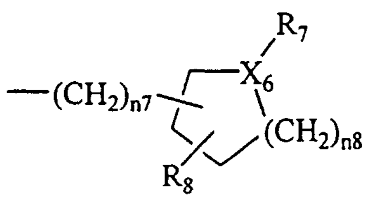

V

(wherein n7 represents number of 0 to 3; n8 represents number of 1 to 3; $X_6$ represents a carbon atom, nitrogen atom, oxygen atom or sulfur atom; in cases where $X_6$ is a carbon atom or nitrogen atom, $R_7$ exists and represents $C_1$-$C_6$ straight alkyl, $C_1$-$C_6$ straight acyl, $C_3$-$C_8$ branched alkyl, $C_3$-$C_8$ branched acyl, $C_6$-$C_{10}$ aryl or benzoyl group bound through 0 to 3 methylene groups, which aryl or benzoyl group is substituted with 0 to 2 hydroxyl groups, methoxy groups, chloro, bromo, fluoro, nitro groups or amino groups; $R_8$ represents Formula VI:

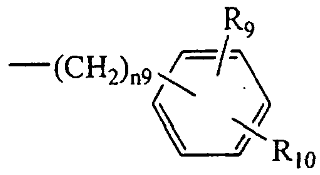

VI

(wherein n9 represents number of 0 to 3; $-(CH_2)_{n9}-$ is bound to an arbitrary position on the benzene ring; $R_9$ and $R_{10}$ may or may not exist, and may be the same or different, in cases where $R_9$ or $R_{10}$ exists, $R_9$ and $R_{10}$ are bound at ortho-, meta- or para-position to $-(CH_2)_{n9}-$, and represent $C_1$-$C_6$ straight alkyl, $C_3$-$C_8$ branched alkyl, hydroxyl, methoxy, nitro, amino, chloro, bromo, fluoro, Formula III:

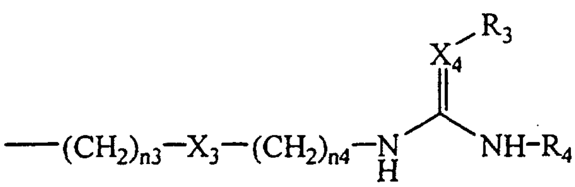

III

(wherein n3, n4, $X_3$, $X_4$, $R_3$ and $R_4$ represent the same meanings as described above), or Formula IV:

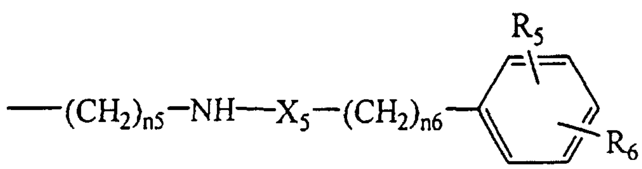

IV

(wherein n5, n6, $X_5$, $R_5$ and $R_6$ represent the same meanings as described above)
(v) Formula VII

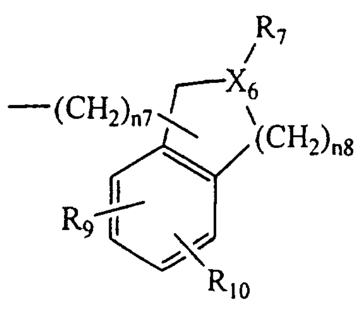

VII

(wherein n7, n8, $X_6$, $R_7$, $R_9$ and $R_{10}$ represent the same meanings described above);

D and O may or may not exist, and may be the same or different, in cases where D or O exists, D and O represent carbonyl group, sulfonyl group, $C_1$-$C_6$ methylene chain, nitrogen atom, oxygen atom or sulfur atom;

E and N may or may not exist, and may be the same or different, in cases where E or N exists, E and N

represent $C_1$-$C_6$ methylene chain, nitrogen atom, oxygen atom or sulfur atom;

F and L may or may not exist, and may be the same or different, in cases where F or L exists, F and L represent carbon atom or nitrogen atom;

G and M may or may not exist, and may be the same or different, in cases where G or M exists, G and M respectively represent (i) Formula VIII:

$$—(CH_2)_{n9}COOR_{11} \quad \text{VIII}$$

(wherein n9 represents number of 0 to 3; $R_{11}$ represents hydrogen, or $C_1$-$C_6$ straight alkyl);
(ii) hydrogen; (iii) hydroxyl group; (iv) amino group; (v) when F or L are carbon atoms and the bonds between F and G, or between L and M are double bonds, they respectively represents an oxygen atom; (vi) E, F and G, or M, L and N cooperatively form Formula IX:

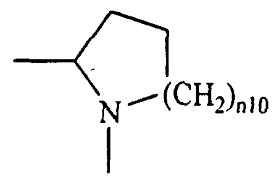

IX

(wherein n10 represents number of 1 to 3); or
(vii) tetrazole group;

H and K may or may not exist, and may be the same or different, in cases where H or K exists, they represent Formula X:

$$—(CH_2)_{n11}—X_7—(CH_2)_{n12}— \quad \text{X}$$

(wherein n11 and n12 represent numbers of 0 to 3; $X_7$ may or may not exist, in cases where $X_7$ exists, it represents a nitrogen atom, oxygen atom or sulfur atom);

I and J may or may not exist, and may be the same or different, in cases where I or J exists, they respectively represent (i) carbon atom; (ii) nitrogen atom; (iii) sulfur atom (with the proviso that the compounds represented by Formula I wherein both I and J are sulfur atoms, both D and O are carbonyl groups, both E and N are nitrogen atoms, all of F, L, K and H are carbon atoms, both G and M are carboxyl groups or ester groups, none of A, B, Q and P exists, and C and R respectively are hydrogen, $C_1$-$C_6$ straight alkyl, $C_3$-$C_8$ branched alkyl or those represented by Formula II wherein none of $X_1$, $X_2$, $R_1$ and $R_2$ exists, or $X_1$ is an oxygen atom and $X_2$ does not exist, □ is benzene ring, n1 = 1 to 3 and n2 = 0, are excluded); (iv) oxygen atom; (v) Formula XI (with the proviso that the compounds represented by Formula I wherein none of A, C, P, R, K, L and M exist, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom, G is a carboxyl group or an ester group, and H is methylene, are excluded)

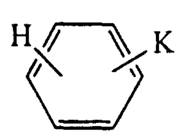

XI

(wherein H and K represent the same meanings as described above and are bound to the benzene ring at ortho-, meta- or para- positions)

(vi) Formula XII (with the proviso that the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom, G is a carboxyl group or an ester group, and H is methylene, are excluded),

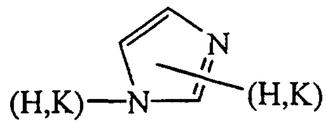

XII

(wherein H and K represent the same meanings as described above, one of H and K is bound to the nitrogen atom at the 1-position, and the other is bound to 2-, 4- or 5-position of the imidazole ring);

(vii) Formula XIII (with the proviso that the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom G is a carboxyl group or an ester group, and H is methylene, are excluded)

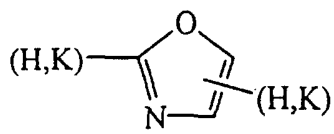

XIII

(wherein H and K represent the same meanings as described above, one of H and K is bound to the 2-position and the other is bound to the 4- or 5-position of the oxazole ring);

(viii) Formula xiv

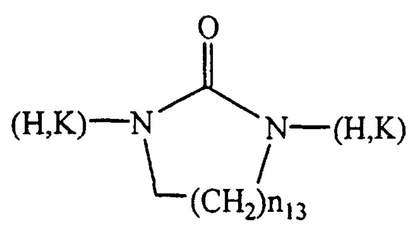

xiv

(wherein n13 represents number of 1 to 3; H and K represent the same meanings as described above with the proviso that one of H and K is bound to one of the nitrogen atoms and the other is bound to the other nitrogen atom); or

(ix) Formula xv:

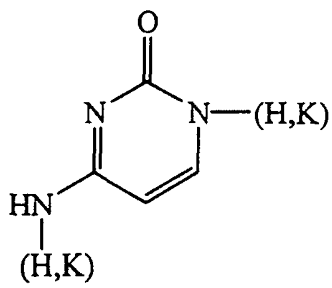

XV

(wherein H and K represent the same meanings as described above with the proviso that one of H and K is bound to one of the nitrogen atoms in the ring and the other is bound to the nitrogen atom outside the ring);
— — —represents single bond or double bond; and
- - - - -
———
- - - - -
represents single bond, double bond or triple bond]
and pharmaceutically acceptable salts thereof.

**2.** The carboxylic acid derivative or the pharmaceutically acceptable salt thereof according to claim 1, wherein A, C, P, R, H and K represent the same meanings as described above; I and J may or may not exist, and may be the same or different, in cases where I or J exists, I and J represent (i) carbon atom; (ii) sulfur atom (excluding the compounds represented by Formula I wherein both I and J are sulfur atoms, both D and O are carbonyl groups, both E and N are nitrogen atoms, all of F, L, K and H are carbon atoms, both G and M are carboxyl groups or ester groups, none of A, B, Q and P exists, and C and R respectively are hydrogen, $C_1$-$C_6$ straight alkyl, $C_3$-$C_8$ branched alkyl or those represented by Formula II wherein none of $X_1$, $X_2$, $R_1$ and $R_2$ exists, or $X_1$ is an oxygen atom and $X_2$ does not exist, □is benzene ring, n1 = 1 to 3 and n2 = 0); (iii) nitrogen atom; (iv) oxygen atom; (v) Formula XI (excluding the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is carbon atom, G is carboxyl group or ester group, and H is methylene); (vi) Formula XII (excluding the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is carbon atom, G is carboxyl group or ester group, and H is methylene); (vii) Formula XIII (excluding the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is carbon atom, G is carboxyl group or ester group, and H is methylene); (viii) Formula xiv; or (ix) Formula xv; B and Q may or may not exist, and may be the same or different, in cases where B or Q exists, B and Q represent carbon atom, $C_6$-$C_{10}$ aryl group bound through 0 to 2 methylene groups, which aryl group is substituted with 0 to 3 methyl groups, ethyl groups, hydroxyl groups, methoxy groups, nitro groups, amino groups, chloro, bromo, fluoro or tetrazole groups, Formula V or Formula VII; D and O may or may not exist, and may be the same or different, in cases where D or O exists, D and O represent carbonyl group, sulfonyl group, $C_1$-$C_6$ methylene chain; E and N may or may not exist, and may be the same or different, in cases where E or N exists, E and N represent $C_1$-$C_3$ methylene chain, nitrogen atom, oxygen atom or sulfur atom; F and L may or may not exist, and may be the same or different, in cases where F or L exists, F and L represent carbon atom; G and M may or may not exist, and may be the same or different, in cases where G or M exists, G and M respectively represent hydrogen, tetrazole group or $-(CH_2)_{n9}COOH$, E, F and G, or M, L and N may or may not cooperatively form Formula IX; in cases where B, Q, I or J are carbon atoms, the bonds between A and B, or between Q and P are single bonds or double bonds, or the bond between I and J is single bond, double bond or triple bond; the definitions other than mentioned above being the same as those recited in claim 1.

**3.** The carboxylic acid derivative or the pharmaceutically acceptable salt thereof according to claim 1, wherein A, C, P, R, H and K represent the same meanings as described above; I and J may or may not exist, and may be the same or different, in cases where I or J exists, I and J represent (i) carbon atom; (ii) sulfur atom (excluding the compounds represented by Formula I wherein both I and J are sulfur atoms, both D and O are carbonyl groups, both E and N are nitrogen atoms, all of F, L, K and H are carbon atoms, both G and M are carboxyl groups or ester

groups, none of A, B, Q and P exists, and C and R respectively are hydrogen, $C_1$-$C_6$ straight alkyl, $C_3$-$C_8$ branched alkyl or those represented by Formula II wherein none of $X_1$, $X_2$, $R_1$ and $R_2$ exists, or $X_1$ is an oxygen atom and $X_2$ does not exist, □is benzene ring, n1 = 1 to 3 and n2 = 0); (iii) nitrogen atom; (iv) oxygen atom; (v) Formula XI (excluding the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is carbon atom, G is carboxyl group or ester group, and H is methylene); (vi) Formula xiv; or (vii) Formula xv; B and Q may or may not exist, and may be the same or different, in cases where B or Q exists, they represent carbon atom, $C_6$-$C_{10}$ aryl group bound through 0 to 2 methylene groups, which aryl group is substituted with 0 to 3 methyl groups, ethyl groups, hydroxyl groups, methoxy groups, nitro groups, amino groups, chloro, bromo, fluoro or tetrazole groups, Formula V or Formula VII; D and O may or may not exist, and may be the same or different, in cases where D or O exists, D and O represent carbonyl group, sulfonyl group, $C_1$-$C_6$ methylene chain; E and N may or may not exist, and may be the same or different, in cases where E or N exists, E and N represent $C_1$-$C_3$ methylene chain, nitrogen atom, oxygen atom or sulfur atom; F and L may or may not exist, and may be the same or different, in cases where F or L exists, F and L represent carbon atom; G and M may or may not exist, and may be the same or different, in cases where G or M exists, G and M respectively represent hydrogen, tetrazole group or $-(CH_2)_{n9}COOH$, E, F and G, or M, L and N may or may not cooperatively form Formula IX; in cases where B, Q, I or J are carbon atoms, the bonds between A and B, or between Q and P are single bonds or double bonds, or the bond between I and J is single bond, double bond or triple bond; the definitions other than mentioned above being the same as those recited in claim 1.

**4.** The carboxylic acid derivative or the pharmaceutically acceptable salt thereof according to claim 1, wherein A, C, P, R, H and K represent the same meanings as described above; I and J may or may not exist, and may be the same or different, in cases where I or J exists, I and J represent (i) carbon atom; (ii) sulfur atom (with the proviso that the compounds represented by Formula I wherein both I and J are sulfur atoms, both D and O are carbonyl groups, both E and N are nitrogen atoms, all of F, L, K and H are carbon atoms, both G and M are carboxyl groups or ester groups, none of A, B, Q and P exists, and C and R respectively are hydrogen, $C_1$-$C_6$ straight alkyl, $C_3$-$C_8$ branched alkyl or those represented by Formula II wherein none of $X_1$, $X_2$, $R_1$ and $R_2$ exists, or $X_1$ is an oxygen atom and $X_2$ does not exist, □is benzene ring, n1 = 1 to 3 and n2 = 0, are excluded); (iii) nitrogen atom; (iv) oxygen atom; B and Q may or may not exist, and may be the same or different, in cases where B or Q exists, they represent carbon atom, $C_6$-$C_{10}$ aryl group bound through 0 to 2 methylene groups, which aryl group is substituted with 0 to 3 methyl groups, ethyl groups, hydroxyl groups, methoxy groups, nitro groups, amino groups, chloro, bromo, fluoro or tetrazole groups, Formula V or Formula VII; D and O may or may not exist, and may be the same or different, in cases where D or O exists, D and O represent carbonyl group, sulfonyl group, $C_1$-$C_6$ methylene chain; E and N may or may not exist, and may be the same or different, in cases where E or N exists, E and N represent $C_1$-$C_3$ methylene chain, nitrogen atom, oxygen atom or sulfur atom; F and L may or may not exist, and may be the same or different, in cases where F or L exists, F and L represent carbon atom; G and M may or may not exist, and may be the same or different, in cases where G or M exists, G and M respectively represent hydrogen, tetrazole group or $-(CH_2)_{n9}COOH$, E, F and G, or M, L and N may or may not cooperatively form Formula IX; in cases where B, Q, I or J are carbon atoms, the bonds between A and B, between Q and P, or between I and J are single bonds or double bonds; the definitions other than mentioned above being the same as those recited in claim 1.

**5.** The carboxylic acid derivative or the pharmaceutically acceptable salt thereof according to claim 1, wherein in Formula I, A, C, P, R, H and K represent the same meanings as described above; I and J may or may not exist, and may be the same or different, in cases where I or J exists, they respectively represent Formula XI (with the proviso that the compounds represented by Formula I wherein none of A, C, P, R, K, L and M exist, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom, G is a carboxyl group or an ester group, and H is methylene, are excluded); (vi) Formula xiv or (vii) Formula xv; B and Q may or may not exist, and may be the same or different, in cases where B or Q exists, they represent carbon atom, $C_6$-$C_{10}$ aryl group bound through 0 to 2 methylene groups, which aryl group is substituted with 0 to 3 methyl groups, ethyl groups, hydroxyl groups, methoxy groups, nitro groups, amino groups, chloro, bromo, fluoro or tetrazole groups, Formula V or Formula VII; D and O may or may not exist, and may be the same or different, in cases where D or O exists, D and O represent carbonyl group, sulfonyl group, $C_1$-$C_6$ methylene chain; E and N may or may not exist, and may be the same or different, in cases where E or N exists, E and N represent $C_1$-$C_3$ methylene chain, nitrogen atom, oxygen atom, carbon atom or sulfur atom; F and L may or may not exist, and may be the same or different, in cases where F or L exists, F and L represent carbon atom; G and M may or may not exist, and may be the same or different, in cases where G or M exists, G and M respectively represent hydrogen, tetrazole group or

$-(CH_2)_{n9}COOH$, in cases where B, Q, I or J are carbon atoms, the bonds between A and B, between Q and P, or between I and J are single bonds or double bonds; the definitions other than mentioned above being the same as those recited in claim 1.

**6.** A pharmaceutical comprising the carboxylic acid derivative or the pharmaceutically acceptable salt thereof according to any one of claims 1 to 3 as an effective ingredient.

**7.** An adhesion molecule inhibitor comprising as an effective ingredient a carboxylic acid derivative of the Formula I:

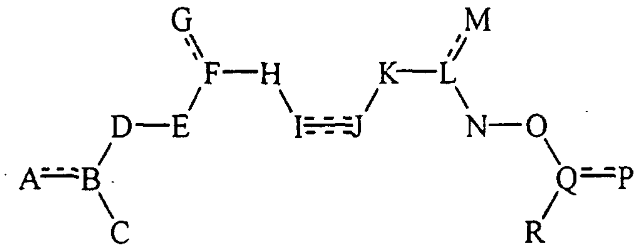

I

[In Formula I, A, C, P and R may or may not exist, and may be the same or different, in cases where A, C, P or R exists, they respectively represent (i) hydrogen, (ii) $C_1$-$C_6$ straight alkyl, (iii) $C_3$-$C_8$ branched alkyl, (iv) Formula II:

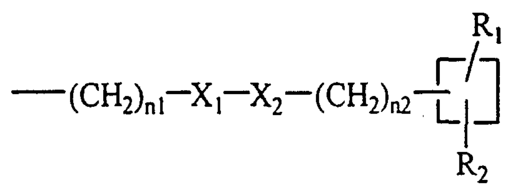

II

(wherein n1 and n2 represent numbers of 0 to 3, $X_1$ and $X_2$ may or may not exist, in cases where $X_1$ or $X_2$ exists, $X_1$ represents an oxygen atom, sulfur atom or nitrogen atom, $X_2$ represents carbonyl group or sulfonyl group; □ represents cyclohexane ring, benzene ring, naphthalene ring, indole ring, imidazole ring, furan ring, thiophen ring, pyrrole ring or pyridine ring; $R_1$ and $R_2$ may or may not exist, and may be the same or different, in cases where $R_1$ or $R_2$ exists, they respectively represent $C_1$-$C_6$ straight alkyl, $C_3$-$C_8$ branched alkyl, hydroxyl, methoxy, chloro, bromo, fluoro, nitro, amino, Formula III:

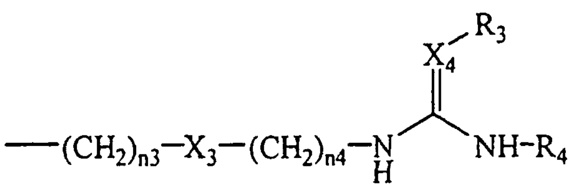

III

(wherein n3 and n4 represent numbers of 0 to 3; $X_3$ may or may not exist, in cases where $X_3$ exists, $X_3$ represents a nitrogen atom or oxygen atom; $X_4$ represents a carbon atom, oxygen atom, nitrogen atom or sulfur atom; in cases where $X_4$ is a carbon atom or nitrogen atom, $R_3$ exists and-represents nitro, cyano, methylsulfonyl or phenylsulfonyl group; $R_4$ represents hydrogen, $C_1$-$C_6$ straight alkyl, $C_3$-$C_8$ branched alkyl, $C_6$-$C_{10}$ non-substituted aryl or $C_6$-$C_{10}$ aryl substituted with 0 to 2 hydroxyl groups, methoxy groups, nitro groups, amino groups, chloro, bromo and/or fluoro) or Formula IV:

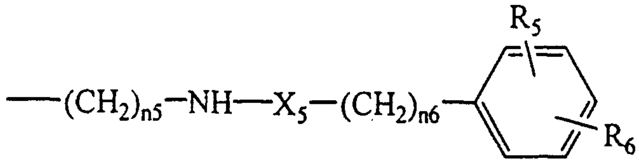

IV

(wherein n5 and n6 represent numbers of 0 to 3; $X_5$ represents carbonyl group or sulfonyl group; $R_5$ and $R_6$ represent hydrogen, methyl, methoxy, chloro, bromo, fluoro, nitro or amino groups)

B and Q may or may not exist, and may be the same or different, in cases where B or Q exists, they represent (i) nitrogen atom, (ii) carbon atom, (iii) $C_6$-$C_{10}$ aryl or cyclohexyl each of which is substituted with 0 to 2 hydroxyl groups, methoxy groups, chloro, bromo, fluoro, nitro, amino, or $C_6$-$C_{10}$ aryl or cyclohexyl each of which is bound through 1 to 3 methylene groups, each of which is substituted with 0 to 2 methoxy groups, chloro, bromo, fluoro, nitro, tetrazole or amino groups, (iv) Formula V:

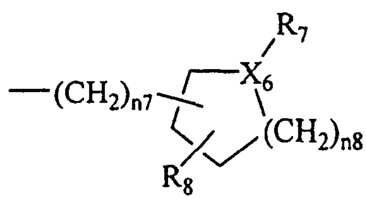

V

(wherein n7 represents number of 0 to 3; n8 represents number of 1 to 3; $X_6$ represents a carbon atom, nitrogen atom, oxygen atom or sulfur atom; in cases where $X_6$ is a carbon atom or nitrogen atom, $R_7$ exists and represents $C_1$-$C_6$ straight alkyl, $C_1$-$C_6$ straight acyl, $C_3$-$C_8$ branched alkyl, $C_3$-$C_8$ branched acyl, $C_6$-$C_{10}$ aryl or benzoyl group bound through 0 to 3 methylene groups, which aryl or benzoyl group is substituted with 0 to 2 hydroxyl groups, methoxy groups, chloro, bromo, fluoro, nitro groups and/or amino groups; $R_8$ represents Formula VI:

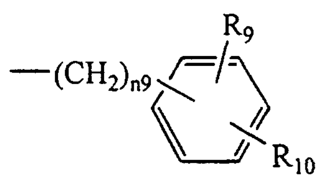

VI

(wherein n9 represents number of 0 to 3; $-(CH_2)_{n9}-$ is bound to an arbitrary position on the benzene ring; $R_9$ and $R_{10}$ may or may not exist, and may be the same or different, in cases where $R_9$ or $R_{10}$ exists, $R_9$ and $R_{10}$ are bound at ortho-, meta- or para-position to $-(CH_2)_{n9}-$, and represent $C_1$-$C_6$ straight alkyl, $C_3$-$C_8$ branched alkyl, hydroxyl, methoxy, nitro, amino, chloro, bromo, fluoro, Formula III:

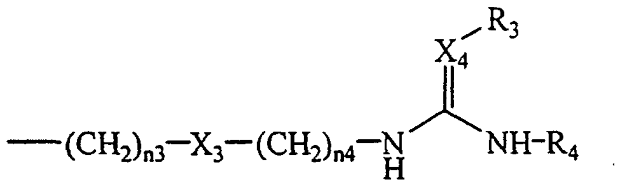

III

(wherein n3, n4, $X_3$, $X_4$, $R_3$ and $R_4$ represent the same meanings as described above), or Formula IV:

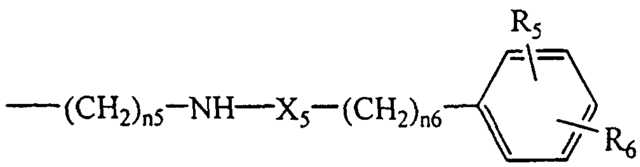

IV

(wherein n5, n6, $X_5$, $R_5$ and $R_6$ represent the same meanings as described above) (v) Formula VII

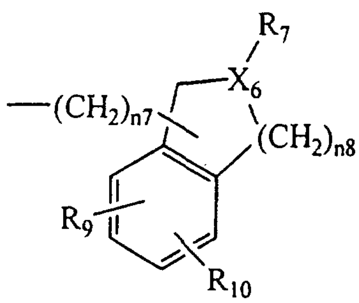

VII

(wherein n7, n8, $X_6$, $R_7$, $R_9$ and $R_{10}$ represent the same meanings as described above);

D and O may or may not exist, and may be the same or different, in cases where D or O exists, D and O represent carbonyl group, sulfonyl group, $C_1$-$C_6$ methylene chain, nitrogen atom, oxygen atom or sulfur atom;

E and N may or may not exist, and may be the same or different, in cases where E or N exists, E and N represent $C_1$-$C_6$ methylene chain, nitrogen atom, oxygen atom or sulfur atom;

F and L may or may not exist, and may be the same or different, in cases where F or L exists, F and L represent carbon atom or nitrogen atom;

G and M may or may not exist, and may be the same or different, in cases where G or M exists, G and M respectively represent (i) Formula VIII:

$$-(CH_2)_{n9}COOR_{11} \qquad \text{VIII}$$

(wherein n9 represents number of 0 to 3; $R_{11}$ represents hydrogen or $C_1$-$C_6$ straight alkyl);
(ii) hydrogen; (iii) hydroxyl group; (iv) amino group; (v) when F or L are carbon atoms and the bonds between F and G, or between L and M are double bonds, they respectively represents an oxygen atom; (vi) E, F and G, or M, L and N cooperatively form Formula IX:

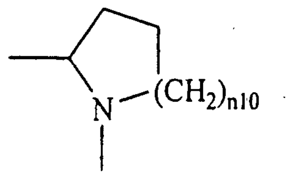

IX

(wherein n10 represents number of 1 to 3); or
(vii) tetrazole group;

H and K may or may not exist, and may be the same or different, in cases wherein H or K exists, they represent Formula X:

$$—(CH_2)_{n11}—X_7—(CH_2)_{n12}— \quad X$$

(wherein n11 and n12 represent numbers of 0 to 3; $X_7$ may or may not exist, in cases where $X_7$ exists, it represents a nitrogen atom, oxygen tom or sulfur atom);

I and J may or may not exist, and may be the same or different, in cases where I or J exists, they respectively represent (i) carbon atom; (ii) nitrogen atom; (iii) sulfur atom; (iv) oxygen atom; (v) Formula XI (with the proviso that the compounds represented by Formula I wherein none of A, C, P, R, K, L and M exist, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom, G is a carboxyl group or an ester group, and H is methylene, are excluded)

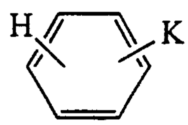

XI

(wherein H and K represent the same meanings as described above and are bound to the benzene ring at ortho-, meta- or para- positions)
(vi) Formula XII (with the proviso that the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom, G is a carboxyl group or an ester group, and H is methylene, are excluded)

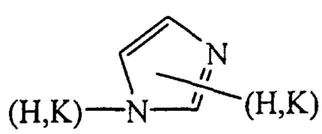

XII

(wherein H and K represent the same meanings as described above, one of H and K is bound to the nitrogen atom at the 1-position, and the other is bound to 2-, 4- or 5-position of the imidazole ring);
(vii) Formula XIII (with the proviso that the compounds represented by Formula I wherein none of A, C, P, R, K, L and M exists, one of I and J does not exist, B and Q are aryl groups substituted with methyl, ethyl, hydroxyl, methoxy, nitro, amino, chloro, bromo or fluoro, D and O are carbonyl groups, E and N are nitrogen atoms, F is a carbon atom G is a carboxyl group or an ester group, and H is methylene, are excluded)

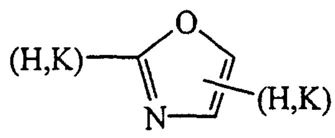

XIII

(wherein H and K represent the same meanings as described above, one of H and K is bound to the 2-position and other is bound to the 4- or 5-position of the oxazole ring);
(viii) Formula xiv

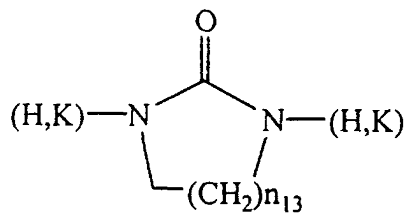

xiv

(wherein n13 represents number of 1 to 3; H and K represent the same meanings as described above with the proviso that one of H and K is bound to one of the nitrogen atoms and the other is bound to the other nitrogen atom); or
(ix) Formula xv:

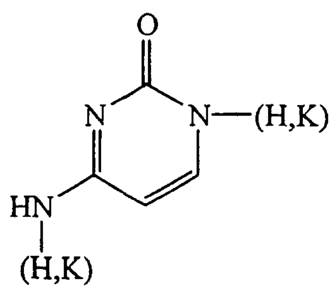

xv

(wherein H and K represent the same meanings as described above with the proviso that one of H and K is bound to one of the nitrogen atoms in the ring and the other is bound to the nitrogen atom outside the ring);
— — — represents single bond or double bond; and
- - - - -
represents single bond, double bond or triple bond]
or a pharmaceutically acceptable salt thereof.

**8.** The adhesion molecule inhibitor according to claim 7, wherein said adhesion molecule belongs to an integrin family.

**9.** The adhesion molecule inhibitor according to claim 8, wherein said integrin family is VLA-4.

**10.** The adhesion molecule inhibitor according to claim 7, wherein the disease to be treated is an inflammatory disease.

**11.** The adhesion molecule inhibitor according to claim 8, wherein the inflammatory disease is an allergic disease.

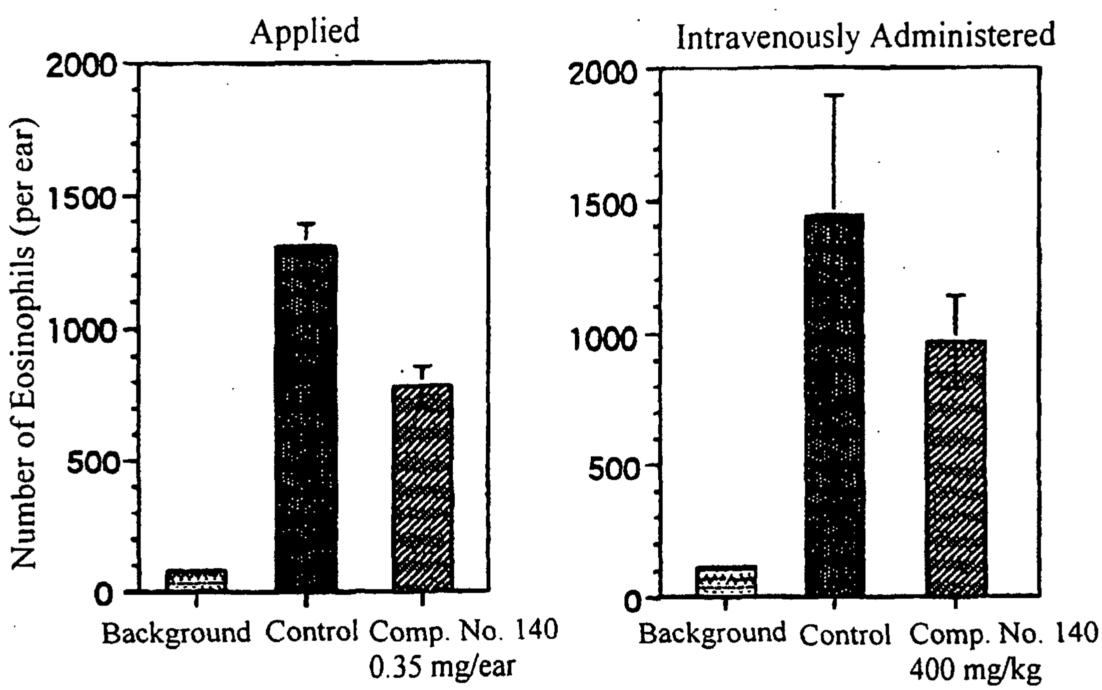
Applied
Intravenously Administered
Number of Eosinophils (per ear)
2000
1500
1000
500
0
Background
Control
Comp. No. 140
0.35 mg/ear
400 mg/kg

Fig. 1

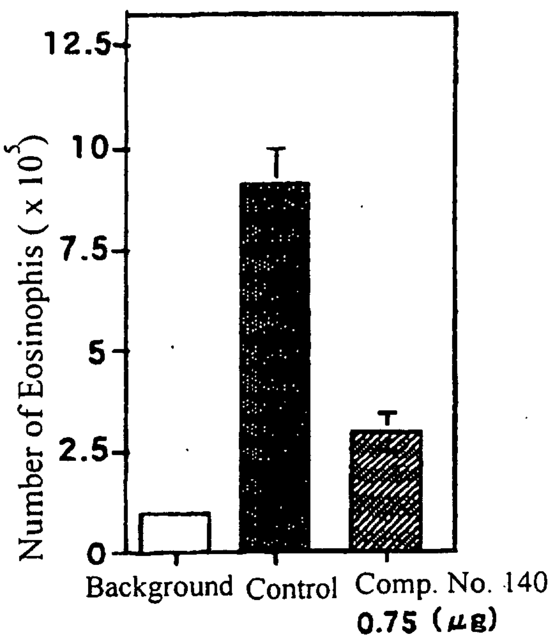
12.5
10
7.5
5
2.5
0
Number of Eosinophis ( x 10^5)
Background
Control
Comp. No. 140
0.75 (μg)

Fig. 2

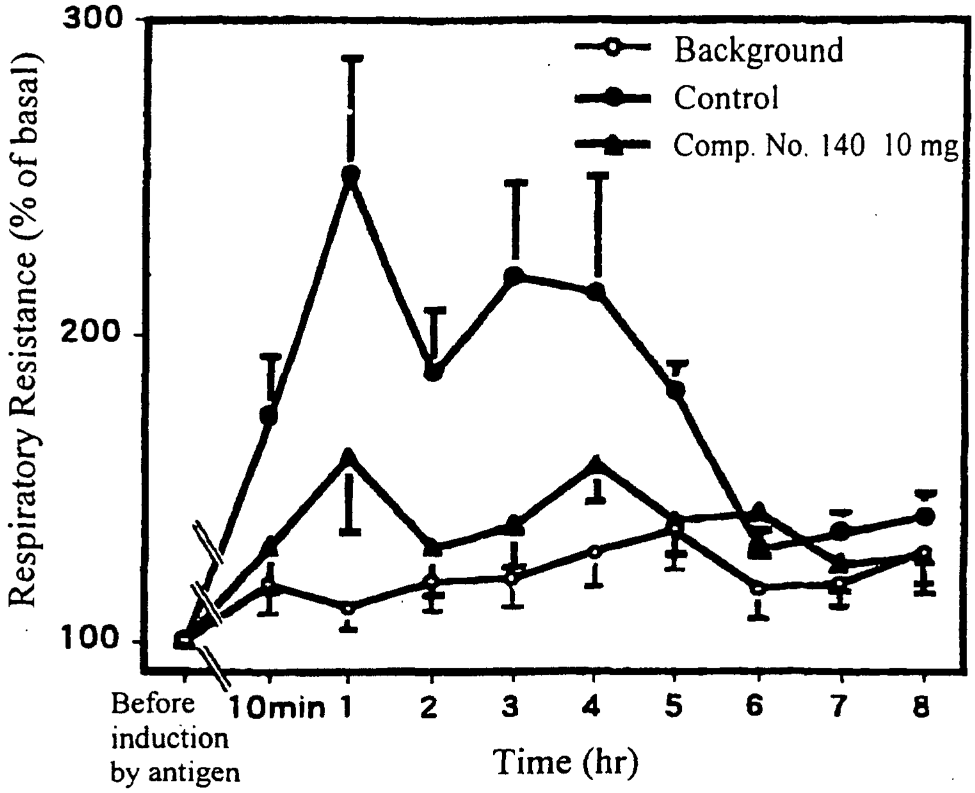
300
200
100
Respiratory Resistance (% of basal)
Background
Control
Comp. No. 140 10 mg
Before induction by antigen
10min 1 2 3 4 5 6 7 8
Time (hr)

Fig. 3

## INTERNATIONAL SEARCH REPORT

International application No.
PCT/JP00/04965

**A. CLASSIFICATION OF SUBJECT MATTER**

Int.Cl$^7$ C07C233/51, 233/87, 235/12, 235/66, 237/22, 275/42, 311/21, 323/59, 329/06, C07D207/08, 239/47, C07K5/062, A61K31/198, 31/216, 31/22, 31/222, 31/235, 31/27, 31/277, 31/40, 31/505, 38/00, A61P29/00, 43/00

According to International Patent Classification (IPC) or to both national classification and IPC

**B. FIELDS SEARCHED**

Minimum documentation searched (classification system followed by classification symbols)

Int.Cl$^7$ C07C233/51, 233/87, 235/12, 235/66, 237/22, 275/42, 311/21, 323/59, 329/06, C07D207/08, 239/47, C07K5/062, A61K31/198, 31/216, 31/22, 31/222, 31/235, 31/27, 31/277, 31/40, 31/505, 38/00, A61P29/00, 43/00

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO, 96/22966, A1 (BIOGEN,INC.), 01 August, 1996 (01.08.96), Claims; page 4, line 28 to page 5, line 13<br>& AU, 9649115, A & NO, 9703384, A<br>& EP, 805796, A1 & FI, 9703087, A<br>& BR, 9606778, A & CZ, 9702340, A3<br>& SK, 9700987, A3 & HU, 9702461, A2<br>& MX, 9705569, A1 & JP, 10-513160, A<br>& KR, 98701672, A | 1-11 |

☐ Further documents are listed in the continuation of Box C.  ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 September, 2000 (13.09.00) | 26 September, 2000 (26.09.00) |
| Name and mailing address of the ISA/<br>Japanese Patent Office | Authorized officer |
| Facsimile No. | Telephone No. |

Form PCT/ISA/210 (second sheet) (July 1992)